# EUROPEAN PATENT SPECIFICATION

(11) **EP 4 306 041 B1**
(45) Date of publication and mention of the grant of the patent: **25.11.2026**
(21) Application number: 23196226.7
(22) Date of filing: 06.01.2016
(51) Int. Cl.: A61B 5/00, A61B 5/0205, A61B 5/024, A61B 5/11, A61B 5/1455, A61B 5/291, A61B 5/369, A61B 5/389, A61B 5/398, A61N 2/00

(54) **MOBILE WEARABLE MONITORING SYSTEM**
MOBILE, AM KÖRPER TRAGBARE ÜBERWACHUNGSSYSTEM
SYSTÈME DE SURVEILLANCE POUVANT ÊTRE MOBILES ET PORTES

(30) Priority: 06.01.2015 AU 2015900015
(43) Date of publication of application: 17.01.2024
(60) Divisional application: 25155431.7 / 4 523 734; 26206219.3 / 4 834 420
(62) Document number(s) of the earlier application(s) in accordance with Art. 76 EPC: 16701198.0 / 3 242 587
(73) Proprietor: Burton, David, Abbotsford, Victoria 3067 (AU)
(72) Inventor: Burton, David, Abbotsford, Victoria 3067 (AU)
(74) Representative: Handsome I.P. Ltd

(56) References cited:
- WO-A1-2013/169838
- WO-A1-2014/164926
- US-A1- 2011 307 029
- US-A1- 2012 289 761
- US-A1- 2013 231 553
- US-A1- 2013 281 759
- US-B1- 6 488 617
- SANTIAGO-RODRIGUEZ E ET AL: "Repetitive transcranial magnetic stimulation decreases the number of seizures in patients with focal neocortical epilepsy", SEIZURE, BAILLIERE TINDALL, LONDON, GB, vol. 17, no. 8, 1 December 2008 (2008-12-01), pages 677 - 683, XP025642370, ISSN: 1059-1311, [retrieved on 20080520], DOI: 10.1016/J.SEIZURE.2008.04.005
- JOO ET AL: "Antiepileptic effects of low-frequency repetitive transcranial magnetic stimulation by different stimulation durations and locations", CLINICAL NEUROPHYSIOLOGY, ELSEVIER, AMSTERDAM, NL, vol. 118, no. 3, 7 February 2007 (2007-02-07), pages 702 - 708, XP005896489, ISSN: 1388-2457, DOI: 10.1016/J.CLINPH.2006.11.008
- MUSTAFA EFKAN COLPAN ET AL: "Proportional Feedback Stimulation for Seizure Control in Rats", EPILEPSIA, RAVEN PRESS LTD, NEW YORK , US, vol. 48, no. 8, 18 April 2007 (2007-04-18), pages 1594 - 1603, XP071209988, ISSN: 0013-9580, DOI: 10.1111/J.1528-1167.2007.01073.X
- BOSCH-BAYARD J. ET AL: "3D Statistical Parametric Mapping of EEG Source Spectra by Means of Variable Resolution Electromagnetic Tomography (VARETA)", vol. 32, no. 2, 27 April 2001 (2001-04-27), US, pages 47 - 61, XP093103630, ISSN: 0009-9155, Retrieved from the Internet <URL:http://journals.sagepub.com/doi/full-xml/10.1177/155005940103200203> DOI: 10.1177/155005940103200203

## Description

### Overview

The present invention is defined by the claims appended hereto.

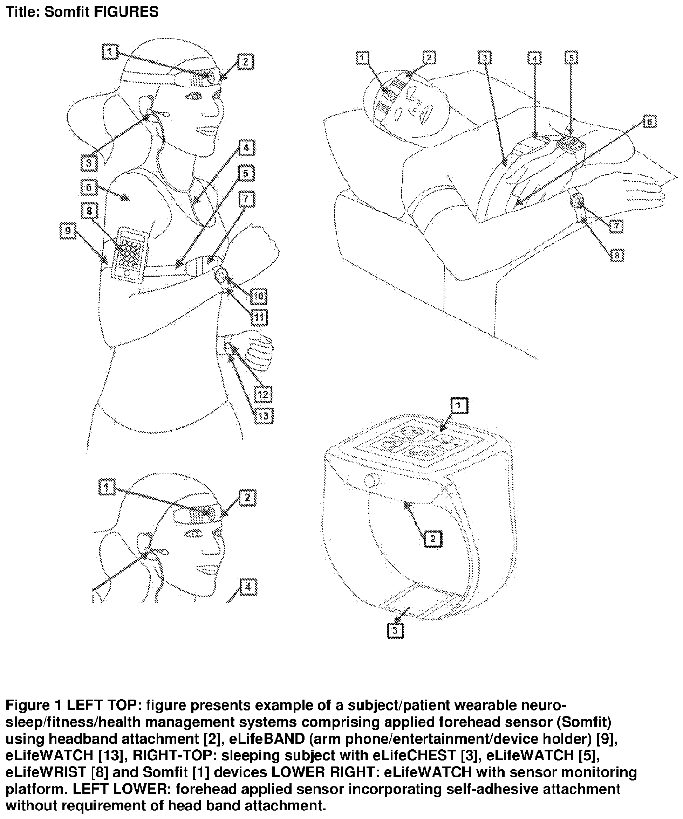

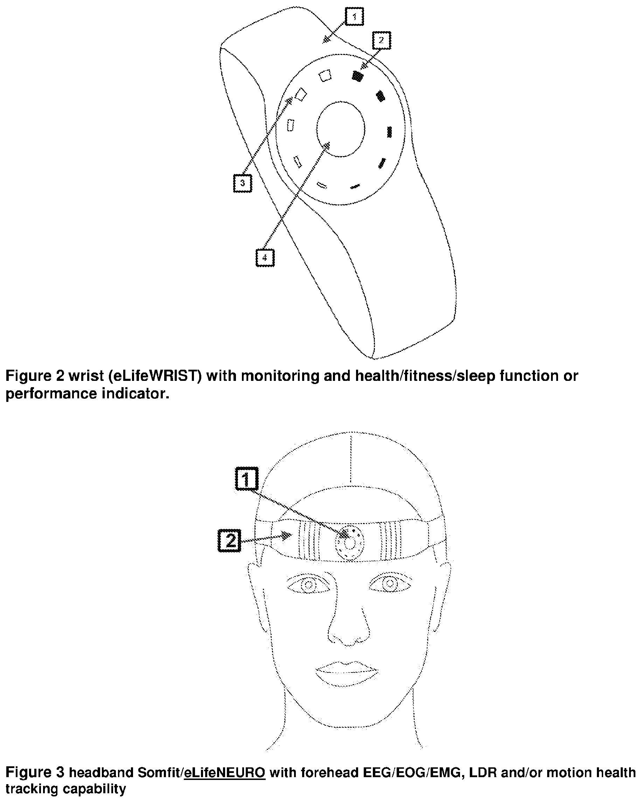

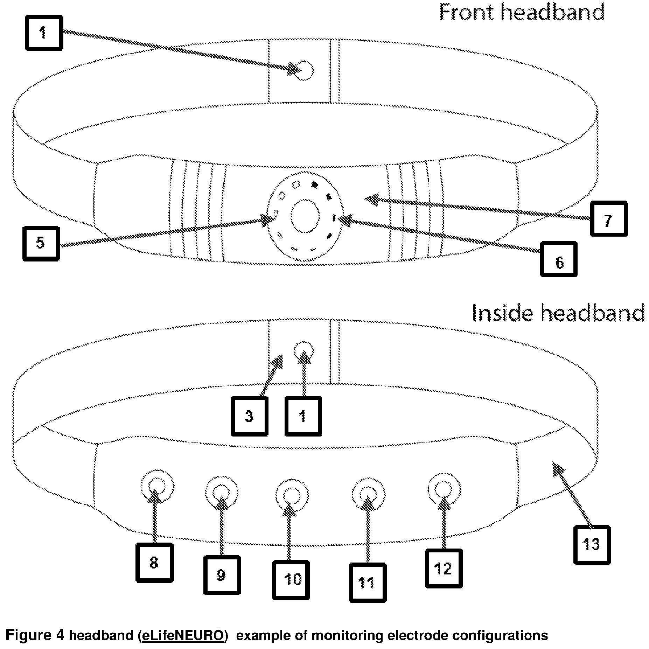

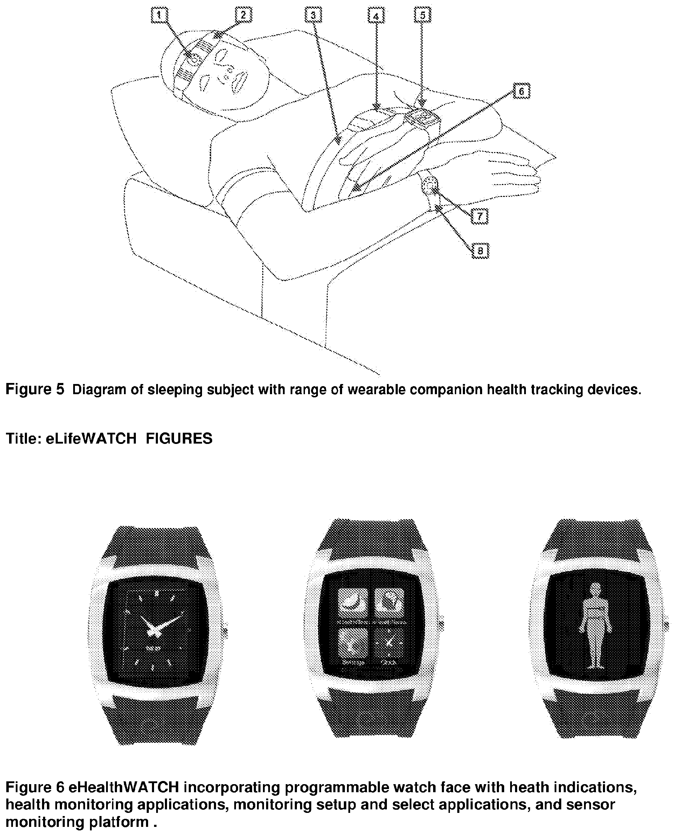

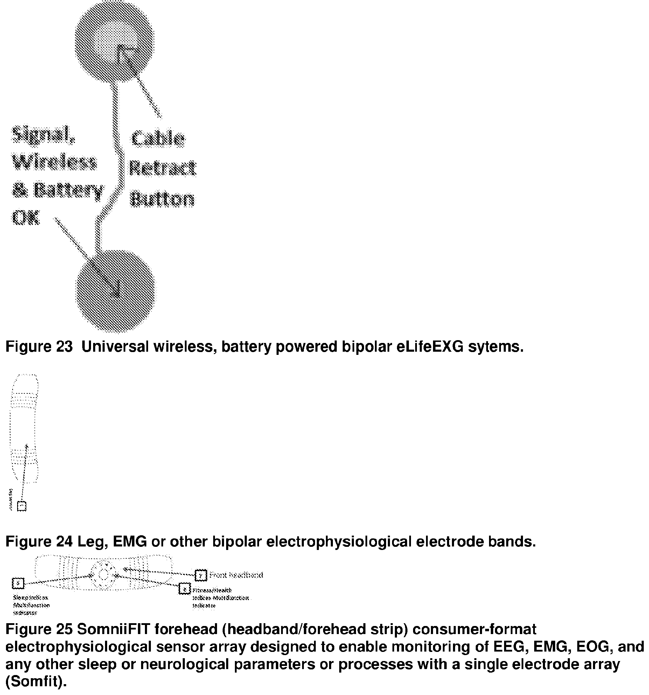

The application additionally presents a number of example methods and devices incorporating or enabling communication interface (including one or more wearable devices, or connectivity options (such as WWW, IP, LAN, WAN, supplementary/companion monitoring/sensing or computing systems, SAAS including Cloud-computing services or NAS, peer to peer connections etc.) with the means of sensing, monitoring, tracking, storing and/or analysing any of or any combination of a subject's physiological parameters, pathological conditions, psychological states, wake, sleep, activity, fitness, health, other sentient states, associated transitions, and/or neurological parameters including option of automatic determination of the prediction, onset or incidence of health conditions/disorders or events of interest by way of incorporating characterisation and determination of processing capabilities or combinations (i.e. multivariate analysis) or clusters or ensembles applicable to any of or any combination of the following;
a) ***A patient-wearable sleep, fitness, health, sentient state monitoring device*** (***Somfit***);
b) An forehead applied sensor system incorporating a wrist-forehead interchangeable or exchangeable active electronic module (**Figure 1** RHS, [1], [7]) with option of display capabilities (any of sleep measure and/or or health tracking measure) (***Somfit***);
c) The capability of ***continuously transmitted sleep parameter information enabling reconstitution of the analysis of contiguous 20 or 30 seconds data epochs*** in order to enable online sleep staging based on these continuous epoch periods. The monitored sleep parameter data (**Figure 1** LHS, [1]) can be transferred to a secondary wearable device for sleep status display purposes such as a smart watch device (**Figure 1** RHS UPPER, [5]), interconnected mobile, clock or other device etc. The monitored sleep parameter data (**Figure 1** LHS, [1]) can also be simultaneously transferred to additional communication networks, systems or other interconnectivity options (such as WWW, IP, LAN, WAN, supplementary/companion monitoring/sensing or computing systems, SAAS including Cloud-computing services or NAS, peer to peer connections etc.) in order to enable personalised or remote tracking, reporting or surveillance or an individual sleep and associated outcomes.
d) ***Automatic data exchange is possible between a first applied forehead monitoring system*** and a ***second information indicator*** (computer based wrist watch system, mobile device, clock, bangle with indicator such as **Figure 1** LHS [8] or [10] or **Figure 1** RHS [5]), whereby user/patient can track sleep progress during any stage of sleep and also as a means of ***tracking sleep deprivation or sleep quality*** (based on prior sleep or wake measures and/or **circadian** cycle **offset**s factors).
e) The monitored and exchanged parameters can include ***sleep, health and fitness measures*** and indicator display capabilities, comprising one or more light-detection and applied forehead electrophysiological sleep-parameter(s) (**EEG**, EOG and EMG) monitoring capability, along with measures or associated indices including (but not limited to) sleep efficiency (SE), wake after sleep onset (WASO);
f) The **sensing, monitoring and analysis** of any of or any combination of wake and sleep events, measures, states or health and environmental conditions of interest,
g) ***One electronic part of a wearable monitoring system* (**i.e. in case of an interchangeable module for day activity and night sleep monitoring, for example) ***or a plurality of electronic parts of wearable monitoring systems* (**i.e. in case of separate modules such as a wrist device containing a separate module, or a microprocessor based wrist watch incorporating the day-time activity and other health tracking and/or monitoring functions, etc.) containing the for an interchangeable module for day activity and night sleep monitoring, for example**)** can be deployed in order to provide both day activity and health monitoring and tracking, as well as night homeostatic sleep monitoring capabilities, as part of an overall wake/sleep health monitoring and tracking system;
h) The said *"**electronic part of a wearable monitoring system" (***Figure **1** **LHS UPPER, [1] and [10]**) or **Figure 1** **LHS LOWER, [2]** or **Figure 1** **RHS UPPER, [1] and [7]**) can comprise of an electronic module containing any of or any combination of functions for physiological monitoring, ***analysis*** of monitored physiological parameters, ***storage*** of monitored physiological parameters, means of ***information flow interconnectivity, health measures or health status indications,*** including ***sleep*/*wake information*** and/or ***activity information*** (i.e. movement, motion, steps, activity etc.) and/or other health information,
i) Said "electronic module" (***Somfit***) incorporating means (i.e. any of or any combination of magnetic, mechanical, and/or interlocking) of being **interchanged between a plurality of wearable** health or environmental monitoring devices;
j) Said ***Somfit*** electronic module (**Figure 1** **LHS UPPER [1]) or** **Figure 1** **LHS LOWER [1]**) can be **attached or applied to a subject's forehead** via a compatible forehead applied sensor device (i.e. **Figure 1** **LHS UPPER [2] or** **Figure 1** **LHS LOWER [2]**).
k) Additionally in order to enable the same ***Somfit*** electronic module device also be used for daytime or wake activity (i.e. user can transfer more expensive electronic module to wrist band holder device this enabling sleep/wake or day/night 24 hours 7 days a week monitoring from a ***single Somfit module*** with different wearable devices such as forehead system for sleep and wrist band for daytime fitness or health tracking.
l) The ***Somfit*** electronic module can also incorporate display or indicator capabilities so that the module provides users indication of measures or related indices of sleep/wake as well as daytime fitness or general health tracking capabilities.
m) The ***Somfit*** electronic module indicator can be monochrome or graphic displays or alpha numeric type. Alternatively the display can be simple bar-graph or other graphic or numeric indicator types presenting a range of measures of daytime fitness or general health tracking capabilities, including the analysis measures further detailed elsewhere, throughout this document.
n) In one example of indicator the **Somfit module** can incorporate real **sleep parameters** by way of the forehead applied monitoring of **sleep parameters** (including any of or any combination of one or more of or combined channels of any of or any combination of **EEG,** EMG and/or EOG signals) (i.e. **Figure 2 [3] and [2****]** whereby sleep quality or **sleep parameters** (including those of homeostatic sleep and/or **circadian** clock factors) as well as fitness or other general health or daytime parameters can be displayed in a single wearable indicator system for 24 hour, 7 day sleep/wake health tracking capabilities.)
o) The said ***Somfit*** electronic module (i.e. **Figure 1** **RHS UPPER [1])** can also be attached (i.e. interlocked, magnetically coupled, clipped, press-stud attached, or otherwise mechanically engaged or clipped into etc.) into or as part of a plurality of wearable devices (including but not limited to) compatible wrist band device (i.e. **Figure 1** **RHS UPPER [8] wrist-band).** Additionally, wireless or other connectivity capabilities can manually or automatically transfer the ***Somfit module*** sleep monitoring parameters from the forehead ***Somfit monitoring module*** (i.e. **Figure 1** **RHS UPPER [1])** to the smartwatch system (i.e. **Figure** 1 **RHS UPPER [5]).**
p) A "Forehead applied sensor" incorporating **at least one bipolar electrophysiological** forehead electrophysiological signal (**Figure 4****; [8], [9], [10], [11], [12]**);
q) A "Forehead applied sensor" comprising of a **reusable sensor;**
r) A "Forehead applied sensor" comprising of a **disposable sensor;**
s) A "Forehead applied sensor" comprising of a sensor with or without a partial or total circumference headband (**Figure 4****; [13]**);
t) A "Forehead applied sensor" which can comprise on one **side of a sensor with self-adhesive surface and embedded self-gelled electrophysiological electrodes,** whereby these said "electrode" can be exposed with the **removal of backing paper;**
u) A "Forehead applied sensor" which can comprise on **non-electrode side** of said "forehead applied sensor" a **means of interfacing** (i.e. self-adhesive, press-stud, magnetic, mechanical interlocking or other means) to said "**electronic module**" or a device containing or holding said "electronic module" (**Figure 4****; [7]**);
v) A "Forehead applied sensor" The said "Forehead applied sensor" can monitor at least one forehead sleep-parameter signal including any of or any combination of **EEG,** EOG **and EMG;**
w) The deployment of a subject **applied forehead-sensor capable of monitoring principle sleep-parameters *(EEG, EOG, EMG)*** combined with interconnectivity with a secondary wearable communication/indicator device enables a range of sleep and wake events or disorders to be tracked and managed, including the option of corresponding countermeasures by way of therapeutic device or other forms of intervention;
x) The ***deployment of compatible and interconnected companion patient worn devices*** (i.e. subject worn or related; placement of sound monitoring or environmental monitoring devices further enables monitoring and automatic ***tracking of wake or sleep and associated breathing disorders.*** Information between a group of compatible (i.e. wireless communication and information) devices (i.e. as covered elsewhere in this document:; eLife**WATCH (**Figure 5, **[5]),** eLife**WRIST (**Figure 5, **[8]**); eLife**CHEST (**Figure 5, **[4]**); eLife**EXG (****Figure 23****),** eLife**NEURO** (**Figure 4****, [2]**) which can automatically and dynamically interchange data between one of more of the said devices, enabling determination and associated indication of any combination of measures or associated indices;
y) Where said *"**measures or associated indices**"* can include any of or any combination of this for fitness, health and/or sleep-parameters (***personalised sleep quality, efficiency*/*SE, wake after sleep onset*/*WASO,*** deep-sleep body recovery period, REM sleep brain restoration period, sleep-in-progress tracking, sleep-quality progress, sleep-journal, comparative population or personalised sleep function, sleep-debt, sleep-disturbance, respiratory-disturbance, sleep-disturbance causation along with corresponding sleep improvement hints or recommendations, sleep-architecture, sleep structure, sleep fragmentation etc.).
z) The deployment of a ***somnilink*** system comprising of an internet or other wireless or **interconnectivity means of interfacing signals or derived measures from the *Somfit* system with medical therapeutic or diagnostic Internet Of Medical Devices (IOMD) whereby the *Somfit* can form a biofeedback (closed loop or other control incorporating *Somfit* measures as part of decision making processes responsible for controlling therapy administration).** Where said therapy control can include sleep therapeutic devices such oral mouth adjustment systems, patient positioning devices or trainers, PAP, NIPPV and other devices. Where said therapy control can include relaxation or meditation vidual outputs or controls (i.e. massage chair control) or music or room lighting or video or 3D projections etc.). Where said therapy control can include magnetic or electrical stimulation devices.
aa) The ***Somnisync*** system incorporates a means of enabling *"**dynamic data-exchange" of sleep parameters*** or other health or fitness parameters between two or more wearable devices or associated mobile wireless communication devices or computer systems.
bb) Whereby said *"**dynamic data-exchange"*** can enable personalised management of wrist-bangle or other wearable device sleep and/or fitness and/or other health conditions or status;
cc) This ***dynamic data-exchange*** can be via wireless interconnection between ***two or more wearable devices*** enabling a means for sleep monitored parameter data and/or associated sleep measures to be automatically displayed on a wearable display device, such as mobile phone **(****Figure 1****, LHS, [8]**), smart watch **(****Figure 1****, RHS, [5]**), wrist bangle **(****Figure 1** **RHS, [8]**) or other wearable system;
dd) This ***dynamic data-exchange*** can be via wireless interconnection between ***two or more wearable devices*** enabling a means for sleep monitored parameter data and/or associated sleep measures to be automatically displayed on a wearable display device, such as mobile phone **(****Figure 1****, LHS, [8]**), smart watch **(****Figure 1****, RHS, [5]**), wrist bangle **(****Figure 1****, RHS, [8]**) or other wearable system;
ee) An electronic monitoring transfer electronic module from (with applied forehead **EEG,** EOG, EMG monitoring part), for example, to remove forehead applied sensor and device and transfer electronic module to a wearable wrist device wrist augment conventional daytime wrist pedometer or motion-based fitness devices with sleep measures.
ff) The ***Somnilink forehead applied, wrist device*** (i.e. watch or bangle) or ***other wearable or attachable devices*** can incorporate a means of detecting room or environmental light conditions (i.e. such ***means include light dependent resistor*** or other photo-sensors essential for internationally accepted standard sleep indices such as sleep efficiency or sleep after wake onset). The output information of the light sensors can be linked to online and automatic measures applicable to sleep measures such as sleep efficiency, sleep after wake onset, sleep time, REM sleep (during sleep time), deep-sleep (during sleep-time), NON-REM sleep etc. These said light detections sensor derived measures can be displayed as part of another wearable device to provide a subject instantaneous measures or indicators of sleep function and performance. These sleep measures can be displayed in conjunction with other fitness (such as accelerometer and/or motion sensor and/or pedometer sensor measures) in order to provide a sleep and fitness tracking capability applicable to the said subject via a wearable information indicator device (such as watch or bangle) or other subject wearable or attachable device.
gg) A system automatically computing a subjects ***intra-sleep and inter sleep progress and associated outcomes*** and related information (disorders, measures, indices, quality or severity of sleep and associated disorders or concerns). The system enables distillation of information using an information ***triage means*** (i.***e*. *triage means*** can include an information dissemination process, whereby such a process is in accordance to a predefined (i.e. but not limited to empirical prior data studies, normative or disease/disorder state populations data or studies) or dynamically (i.e. but not limited to determination and/or adaptation and/or adjustment based on preceding monitored or sensed information) computed user access ***authority*** and ***rights*** covering ***role, qualifications, security*** and ***privacy*** aspects as well as appropriate ***user interface and information access or information content complexity levels.** Said "information content" can include* sleep architecture, including REM sleep and deep-sleep amount, disruptions or arousals.
*hh)* A system ***automatically computing and advising a subject when they should be having or need additional sleep, the quality of sleep, the improvement recommendations*** based on ongoing tracking and computation of a subject's ongoing sleep tracking outcomes, as well as these said outcomes comparable to a subject's normal sleep requirements or normative population database comparatives. The system incorporates a means of referencing said data bases.
ii) A questionnaire or ***sleep** survey* ***such* as *a validated sleep-scale or validated drowsiness scale to be deployed in conjunction with self-assessment*** in order to **establish a set of criteria corresponding to a subject's normal, levels or indices** (i.e. REM sleep, sleep time, deep-sleep, arousal index, AH respiratory index, RERA index, overall sleep quality, and/or sleep deprived status, enabling a subject or subject's health carer ***enhanced information access for improved sleep management.*** A further ***means of comparing such information*** with current inter-sleep and intra-sleep progress reports or related trends in order to establish recommendations based on these outcomes and recommendations of hints for subject or associated health carer (means of ***means of comparing such information*** can include reference to a subject's personal model of sleep as established with said personal survey assessments and ongoing calibration of related "criteria" as well as comparison to patient normative data bases.
***jj)* *Sleep-stage-linked synchronisation of* a *clock or alarm* system** or other clock or alarm system to choose the more optimal alarm time for awakening based on user's need to awaken versus sleep cycles of minimal adverse impact during awakening (i.e. avoid awakening during deep-sleep when longer sleep recovery may be evident, if prediction or eventuation of REM sleep stages are close-by that can enable less disruptive awakening without compromising overall sleep or awakening time requirement).
***kk)*** A ***minimal configuration comprising*** of a single headband sensor (***Somfit***) capable of monitoring brain signals capable continuous monitoring of **sleep *parameters (EEG, EOG, EMG)*** and automatic online processing to enable the determination of sleep stages (i.e. REM, non-REM stage 1, non-REM stage 2, non-REM stage 3 and stage 1).
***ll)*** The options of ***monitoring room light detection (i.e. LDR)*** and/or ***breathing sounds (i.e. snoring)*** monitoring and/or sleep hypopnoea monitoring via a range of other optional sensors (including any of or any combination of RIP, PVDF, piezo-electric sensor or other thoracic and/or abdomen belt; nasal cannula sensor; airflow sensor)
mm) The the option to have the single sensor strip incorporating an embedded ***reflective oximeter sensor (LED with LDR),*** whereby the oximeter sensor can be attached or embedded in the forehead sensor providing plethysmography and oximetry with associated outputs (including any of or any combination of PTT, pulse-wave oscillatory amplitude autonomic markers of obstructive apnoea, pulse wave amplitude, pulse arterial tone). Whereby other sensors can include a "drop-down" (i.e. connects to ***Somfit***) airflow sensor (i.e. PVDF, thermo-coupler, thermistor, nasal cannula etc.), whereby said sensor(s) can enable monitoring of sleep disordered breathing including apnoea, hypopnoea, mixed apnoea/hypopnoea.

WO2013169838A1 discloses systems and methods for use in diagnosing and treating 'centralized pain', including a stimulation system comprising electrodes, arranged in a wearable cap, connected to a signal generation module.

### eLifeWATCH overview

A ***wearable wrist-based monitoring device incorporating a gyro-meter or positional tracking system capable of inputting to automatic incorporating means of computing gait, walking characteristics (including Parkinson's onset)*** including automatic analysis of long-term trending of automatic gait analysis capable of detecting ***fluidity of walking and manoeuvring, along with predictive assessment of associated outcomes*** (i.e. hint to see GP or specialist based on detected trends that may have further implications) of individual's walking (gait) such as inability to naturally swing arms with walking stride, short or shuffling steps and difficulties (i.e. change of motion of limbs and stride associated with manoeuvring corners). Any combination of measures such as GPS, gyro-meter, motion, location data can be analysed as a marker of predefined events or health condition onset, or incidence (**Figure 1** **RHS, [5]**).

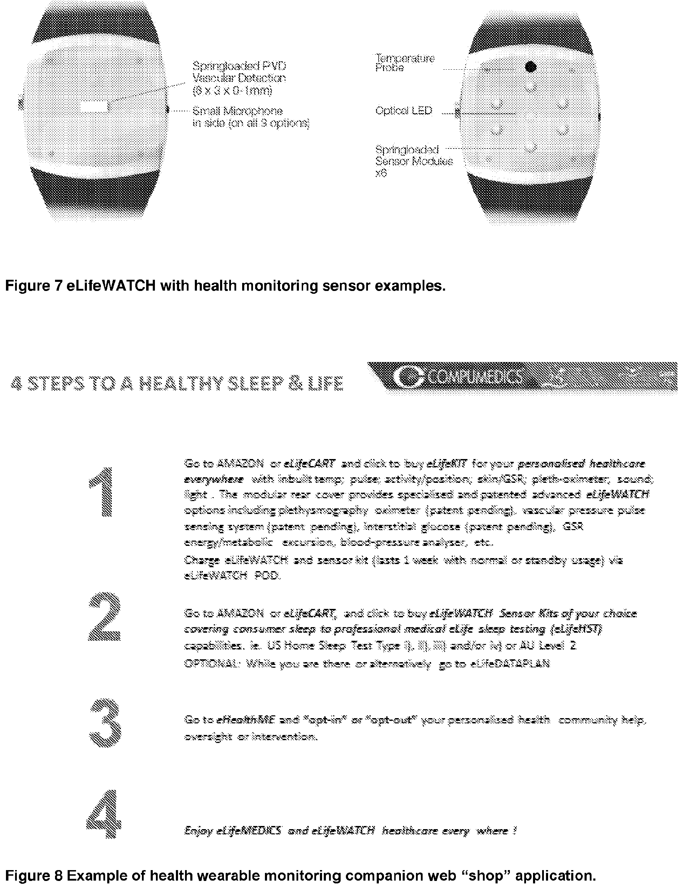

***An example of watch-body modular sensor platform*** system incorporating any of or any combination of photo-plethysmography, oximetry plethysmography, temperature, spring-pressure-loaded or fixed sensor electrophysiological monitoring (i.e. conductive rubber), means of galvanic skin resistance (GSR) monitoring, Doppler ultrasound monitoring, light detection monitoring, microphone monitoring **(*****Figure 7***), a smart-phone system [1] with SAAS including Cloud-computing services interface and other connectivity options (i.e. including simultaneously interconnecting with additional communication networks, systems or other interconnectivity options including WWW, IP, LAN, WAN, supplementary/companion monitoring/sensing or computing systems, SAAS including Cloud-computing services or NAS, peer to peer connections etc.) and range of embedded sensors, applanation tonometry monitoring, Doppler blood-flow [3], PPG, temperature, GSR, pedometer/accelerometer, position, metabolism/calorie-burn tracking (***Figure 7******,*** ***Figure 9******,*** ***Figure 13***).
- Whereby said means of GSR can include incorporation of a plurality of non-polarising electrodes applied to skin surface with a small constant current (i.e. 3 to 5uV), whereby electrical resistance of palmar skin is proportional to the voltage potential developed between said electrodes.
- The resistance is mainly due to the semi-permeable nature of the sweat glands and associated epidermis electrical properties.
- Sleep and wakefulness periods generate different electrical properties which can be tracked as a surrogate or contributory measure for sleep and wake state determination.
- An option of the GSR approach includes switching or alternating the direction of the small constant current between the electrodes in order to minimise electrode polarisation effects.

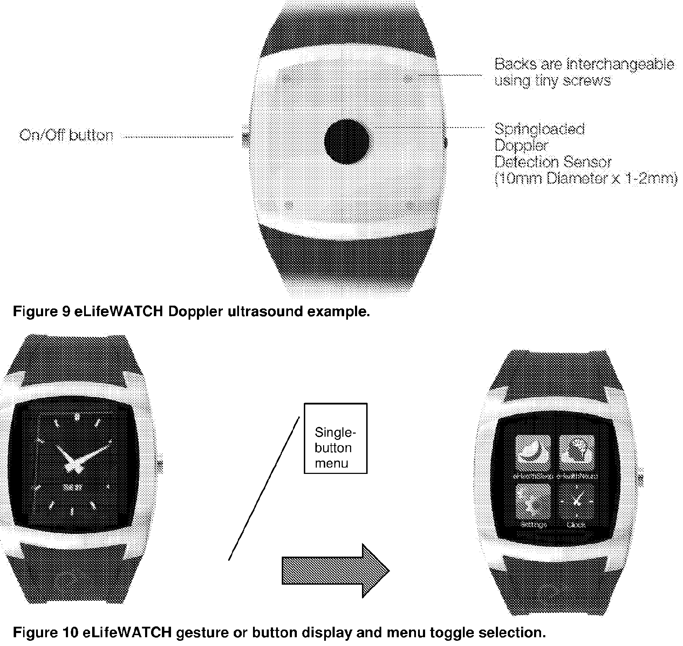

Example of watch-body modular sensor platform system incorporating DOPPLER ULTRASOUND MONTORING and/or TONONOMETER MONITORING detection system. Interchangeable back section (screws, pressure fits or rotational screw rear-cover fit; rubber seals can ensure waterproofing or water-resistance capability) (**Figure 9****).**

### eLifeBUDS overview

***Ear-bud (earphones) worn monitoring device*** with combined sleep, health and fitness incorporating a range of physiological parameter monitoring sensors including GSR, temperature, pulse, motions and/or energy/heat profile ***characterisation for enhanced calorie burn*** determination (**Figure 1** **LHS, [3]**).

### eLifeKIT overview

- eLifeKIT provides a range of wearable or applied monitoring systems capable of working individually or as part of a cluster of interconnected systems across a common framework and health management system platform, enabling personalised health management system framework, based on a compatible health tracking and management technology;

### MULTIPOINT TIME-SYNCH MONITORING (MTM) SYSTEM overview

***Incorporating a multipoint time-synchronisation monitoring (MTM)*** system capable of automatic online characterization (clock drift, **offset,** stability) of clock properties (i.e. drift, **offset,** stability) along with the calibration of the said timing characteristics by way of continuous tracking and compensation where required of all associated clock-synchronisation systems, using any combination or phase locked or open-loop control approaches. MTM incorporates ***calibration modes and corresponding compensation*** modes to ***minimise online or data reconstruction time-alignment errors across disparate transmission medium or multiple, simultaneous monitoring devices or systems, of a subject at any time or under varying monitoring*/ *communication conditions.*** MTM can be deployed across a disparate group of monitoring systems and communication networks with the adoption of ***free-running,*** ***master-slave or multi-modality timing reference approaches**,* in order to achieve minimal data acquisition misalignment with precision of time-clock synchronisation of more interrelated monitoring systems ranging from ***atomic-clock accuracy*** or as required in accordance to accuracy ranging from and reduction of associated phase or data alignment errors applicable to special-purpose or ***specific applications or requirements.***

### eLifeSLEEP overview

- the present example enables true sleep monitoring and tracking with the incorporation of a head applied (i.e. Somfit) system capable of monitoring principal **sleep parameters** for the investigation of sleep and sleep related disorders with a single small electronic device easily applied (i.e. magnetically) and disposable self-adhesive (avoids cross infection or need for pressure application to an individual's head or forehead).

### eLifeCHEST/eLifeSCOPE overview (chestband) (Figure 5[3];[4]; Figure 32; Figure 33)

***Chest-worn monitoring device*** with stethoscope sleep and wake breathing sound, central versus obstructive apnoea/hypopnoea and other automatic sleep disorder tracking capabilities (**Figure 1** **LHS UPPER [7]**). ***Chest-worn monitoring device*** with ***combined sleep, health and fitness*** incorporating a range of physiological parameter monitoring sensors including ***stethoscope sound monitoring*** with online automatic breathing disorder determination and tracking, reflective plethysmography oximeter and related outputs, photo-pulse, energy/heat profile characterisation for enhanced calorie burn determination (with option of monitoring spatiotemporal dynamics of body-heat emissions using time-gated NFIR analysis) for enhanced calorie burn determination.

The present example further comprises of ***obstructive versus central apnoea discrimination*** by way of ***correlating respiratory movements with respiratory effort**,* whereby respiratory effort can be determined by ***EMG and*/*or pulse transient oscillatory amplitude measures*** and/using thoracic and/or abdominal respiration circumference movement) (**Figure 1** **LHS UPPER [7]**).

### Adaptive Physiological-Body Network (APM) overview

The incorporation of an ***adaptive physiological-body monitoring (APM) system,*** enabling a group of mobile companion (i.e. interconnection compatibility) monitoring systems to be automatically reconfigured in terms of data-acquisition, ***physiological parameter data-acquisition-linked system or network properties or intercommunication properties, resources and*/*or parameters,*** information, communication and associated data-prioritisation, and other available processing system or communication system resource(s) allocation, ***usage and*/*or shared resources*** across devices, methods, systems and networks or interconnectivity arrangements (i.e. memory storage, buffering, etc.) in accordance to available resources or monitoring and/or communication conditions and/or available communication mediums, networks or other interconnectivity options (including simultaneously interconnecting with additional communication networks, systems or other interconnectivity options such as WWW, IP, LAN, WAN, supplementary/companion monitoring/sensing or computing systems, SAAS including Cloud-computing services or NAS, peer to peer connections etc.) and the and/or communication pathways (i.e. wireless connectivity) at any point in time, in order to accommodate minimal monitoring study type requirements (i.e. professional medical level or consumer level study type, format and criteria) in order to minimise risk of data loss (such as during wireless connectivity degradation or disruption).

The APM system adopts a substantially enhanced degree of data-acquisition/monitoring/sensing system resource and communications "elasticity" for improved system reliability and data dependability by enabling adaptation of automatic compensation of changing or unpredictable monitoring conditions such as subject's body shrouding of wireless communication pathways, associated with wearable-monitoring devices.

### eLifeBAND (MOBILE DEVICE & INTEGRATED SENSOR ARMBAND) overview

**An armband-worn metabolism-monitoring device** such as a mobile phone-case with combined health and fitness monitoring capabilities, incorporating a range of sensors particularly useful for determination of an individual's energy exertion associated with estimating or predicting an individual's metabolic rate (calorie burn) related to exercise effort, ***incorporating measures based on characterisation of the spatiotemporal dynamics of body-heat emissions (i.e. time-gate NFIR analysis) for enhanced calorie burn determination, whereby such means can include any number and combination of temperature sensor physiological sensing or 3-dimensional infrared heat-mapping imaging capabilities. Heat dissipation*** can be measured using more sophisticated means such as infrared sensor enabling body-heat dispersion and emission characterisation enabling physiological temperature as well as temporal special dynamic heat imaging for ***more comprehensive and precise modelling of metabolism and associated calorie burn rates* (****Figure 1** **LHS UPPER [9]).**

**A phone-case incorporating metabolism-monitoring capability comprising *range of sensors*** and ***associated multivariate analysis*** to automatically compute and indicate online (i.e. via mobile App or wearable device indicator ***with wireless, dynamic-data exchange or exchangeable electronic module compatibility or interface capabilities**.*) metabolism or calorie burn measures (**Figure 1** **LHS UPPER [9]**) including spatiotemporal dynamics of body-heat/energy emissions (i.e. time-gated NFIR analysis);
**eLifeDOPPLER overview**

The present example provides the deployment of Doppler-watch tracking (**DWT**) system comprising of monitoring any of or any combination of a subject's wrist, ankle, arm, and/or other subject limb or body extremity for any of or any combination of the following Doppler and/or ultrasonic vascular or cardiac characteristics, based on periodic or continuous monitoring of any of or any combination of:
- Dual channel - radial artery and ulnar artery;
- Single channel - radial artery and ulnar artery;
- Single channel - ulnar artery;
- Single channel - radial artery;

### eLifePULSE (applanation tonometry) overview

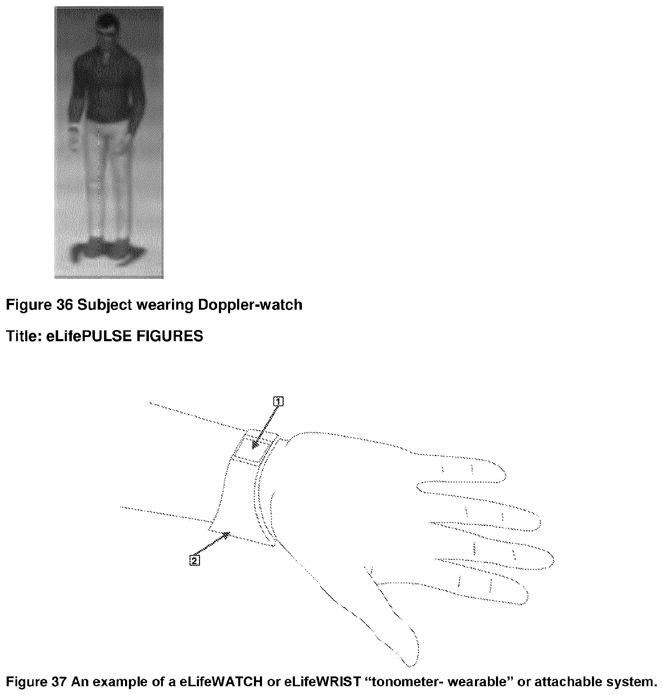

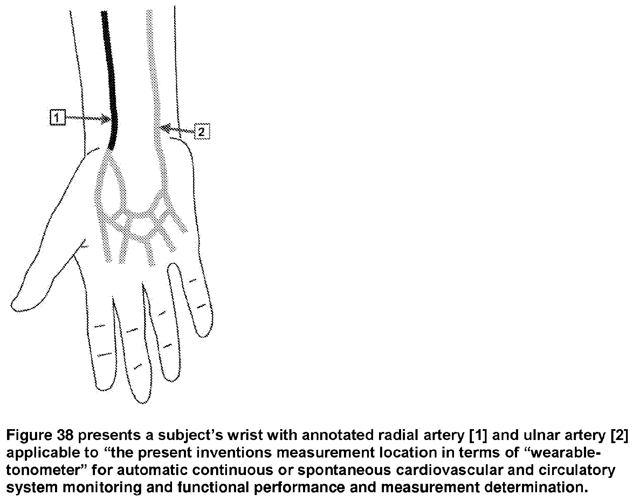

The present example comprises of the deployment of an ***attachable*/*wearable*/*applanation tonometry (AAT)*** which can comprise of a watch, bangle or other device incorporating a means of periodic **or continuous applanation tonometry** (**Figure 37**) whereby said means applies a **pressure sensor capable of measuring and/or characterisation of radial** and/or ulnar artery (**Figure 38**) or other body artery pulsation characteristics (pulse wave-shape). Moreover the present example can further computes the pulse wave analysis (PWA) comprising recording a period (10 seconds for example) of arterial pressure **in order to derive the associated ascending aortic pressure wave, from which a number cardiovascular measurements such as central aortic systolic pressure, aortic augmentation index, and the central pulse pressure can be derived, and/or other cardiac functional measures.**

### Tite: Somfit/Somfit

### Patent Background: Circadian Rhythm Background

The human **circadian** rhythm is an endogenous (*self-sustained* or *"in-built")* biological process with an entrainable physiological oscillation (rhythm) of approximately 24 hours.

*A typical **circadian** clock could be represented by the following 24-hour cyclic sequence:*
00:00 Midnight - start of sleep period
02:00 - deepest sleep period
04:30 - minimal body temperature
06:45 - blood pressure undergoes most rapid pressure increase
07:30 - cessation of melatonin secretion
08:30 - likely bowel movement
08:30 - testosterone secretion at maximum
09:00 - likely bowel movement
10:00 - alertness peak
12:00 noon
14:30 - coordination at premium
15:30 - reaction time most responsive
17:00 - cardiovascular efficiency and must strength at maximum
18:30 - blood pressure peak
19:00 - body temperature peak
21:00 - commencement of melatonin secretion
22:30 - suppressed bowel movements
00:00 Midnight - start of sleep period
02:00 - repeat of typical **circadian** cycle per above

**The circadian clock cycle comprises of the following 3 aspects:**
1. A free-running period (referred to as tau or the Greek letter "τ ") with an approximate period of 24 hours;
2. An entrainment characteristic which can be reset or adapted (entrainment) by way of exposure to external stimuli such as light or temperature changes. Examples of a person's entrainment or adjustment of their **circadian** clock are when a person suffers unexpected or unanticipated sleep urges resulting from jet-lag or other disruptions of conventional sleep routines. For example in cases where a person's biological clock (**circadian** clock or rhythm) body has not yet adjusted or synchronised with the local time or current routine sleep/wake cycle this can occur following international travel, across different time zones, or examples such as adjusting during or following to shiftwork, or as a result of late nights during demanding study period or other events;
3. A "temperature compensation" characteristic, where the body maintains a certain cyclic nature over a range of physiologic temperatures, regardless of changing kinetic (temperatures and different thermal energy of molecular processes in cells).¹

### The circadian clock has a profound impact upon metabolism, general well-being and sleep/wake regulation

➢ An association has been demonstrated between shift work and metabolic disease ²;
➢ Studies have associated sleep time and **circadian** disruption with a wide range of disorders such as type 2 diabetes, cancer and gastrointestinal disorder ²;
➢ "Social Jet Lag" (i.e. the habit of altering sleeping times on weekends has been associated with increased body weight) ²;
➢ The **circadian** clock regulates energy homeostasis, and its disruption, similar to social jetlag, can contribute to weight-related pathologies ²;
➢ **circadian** factor plays an important role in sleep quantity; i.e. length is not dependent of on the homeostatic sleep factor, but dependent on whether you sleep in accordance to your own **circadian** sleep cycle (i.e. the **circadian** sleep cycle factor is more predominant than sleep homeostasis (sleep-urge) ²;
➢ therefore from a practical or productivity perspective, an understanding and guidance in terms of the interaction and relationship between an individual's homeostatic and **circadian** processes can be an important aspect as it is important to remain awake for a significant amount of time in order to achieve high quality sleep, and also to have regular bed and rise times to achieve stable sleep duration (i.e. more time in bed to sleep or more sleep is not necessarily better quality sleep but working with versus against your **circadian** clock can result in the most efficient and effective use of sleep time) ²;
➢ the homeostatic sleep factors accumulate according to prior wakefulness, i.e. the homeostatic factor (sleep urge) increases the longer you are awake. Consequently this factor is believed to be of major importance for sleep quality. For example, the longer you remain awake the deeper the following sleep episode will be, as marked by an increase in slow wave **EEG** activity ²;
➢ In contrast the **circadian** rhythm fulfills an important role in terms of sleep quantity. For example, the duration of sleep is mainly determined by when you go to bed.

***Method or device*** whereby a subject can control a system's indications or associated display information (i.e. ***tap or gesture or touch*** via capacitive surface detection or touch via resistive surface detection etc.) ***to toggle of switch between display modes reflective of brain-based sleep parameter markers or associated indices representative of a subject's monitored sleep status and measures reflective of other fitness and health*** monitored measures or environmental sensing.

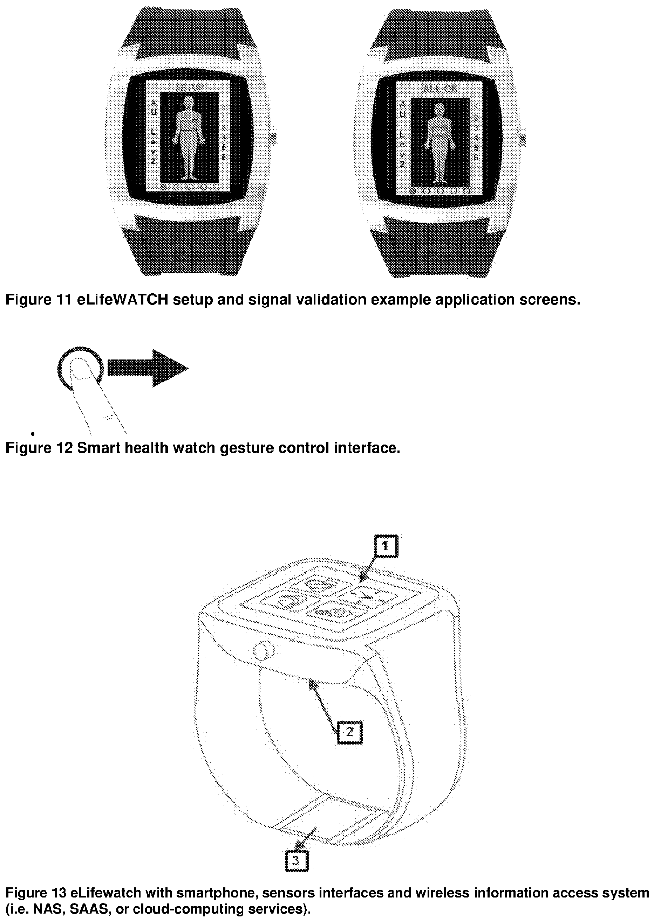

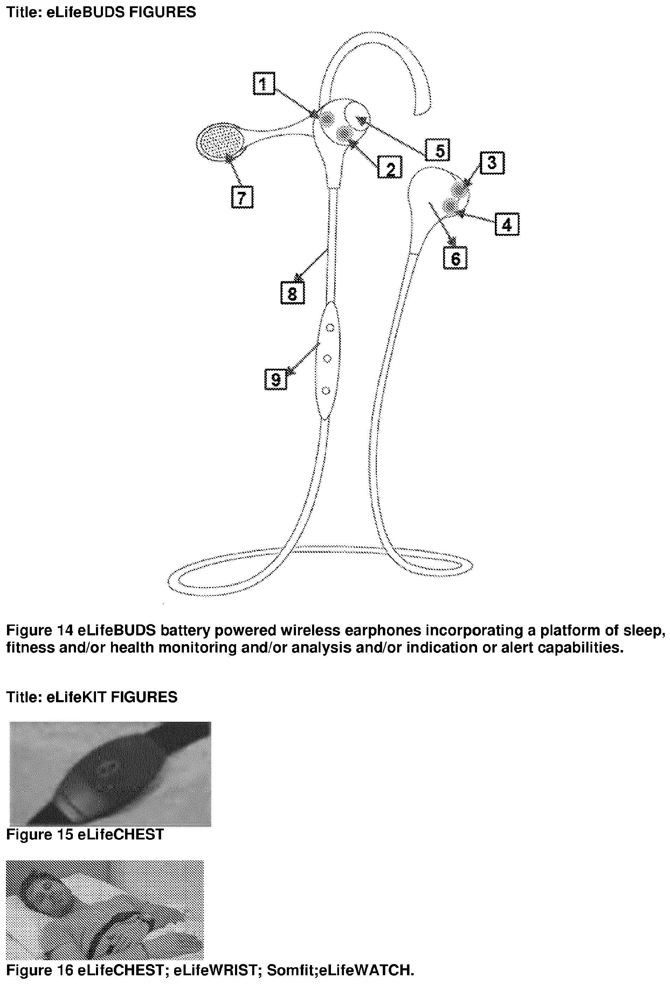

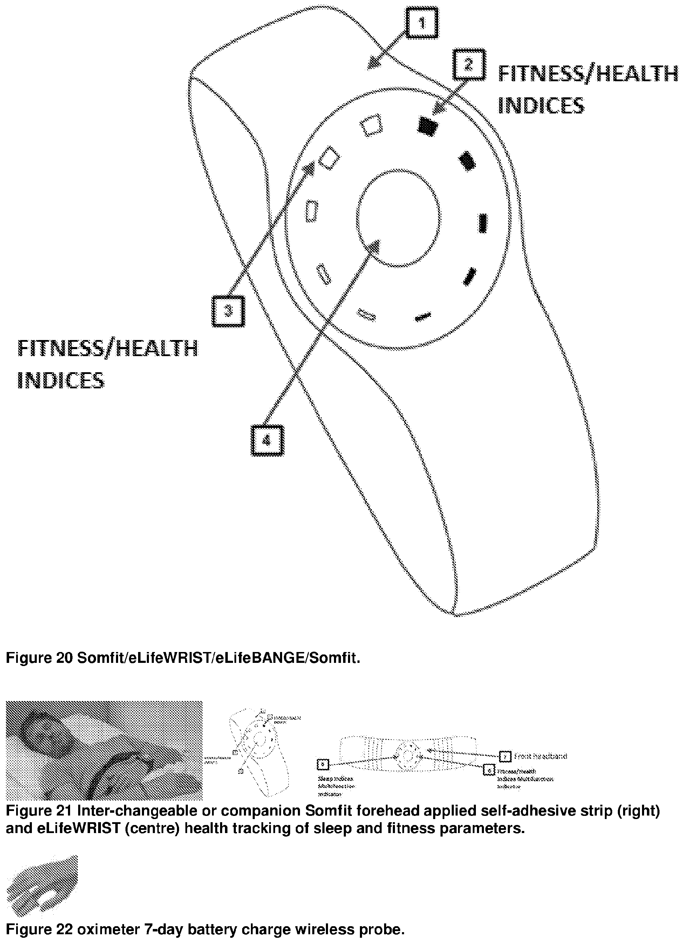

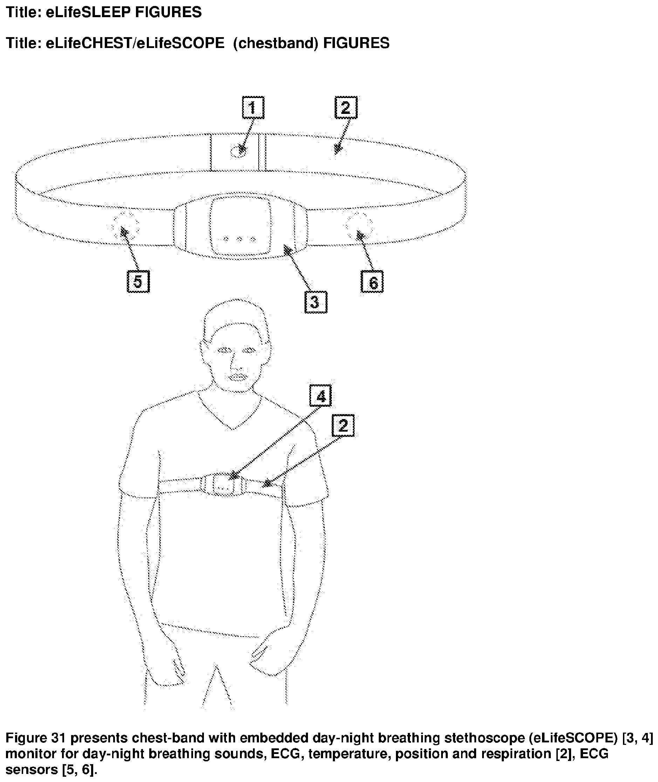

The present example provides a method or device whereby a subject can control a system indications or associated display information to ***toggle** of switch **between*** display modes reflective of ***monitored sleep measures or indices*** (***i.e. brain-signal linked or EEG-signal*** based on any of or any combination of **EEG,** EOG and/or EMG signal-based sleep parameter measures, as well as health and fitness measures (such as accelerometer or motion-based measures and/or as also further detailed elsewhere in this document including ***"**physiological or psychological monitoring including" and*/*or "Environmental sensing"*** as detailed elsewhere in this document and below;
- ***Where in one example*** the subject with a ***wearable device (such as but not limited to headband* (****Figure 25** **[7]); *universal -bipolar sensor (******Figure 23******); oximeter (******Figure 22******); legband (******Figure 24******); wristband (******Figure 2******), ankle-band (******Figure 2******), armband (******Figure 2******), earphone(s) (******Figure 14******),*** chest-band ***(******Figure 5******[3];[4];*** ***Figure 31******),*** other attachable device or watch) can be used to measure and enable ***rhythm, synchrony, fluidity, motion, degree of various motions,*** and also interrelationship with other limbs such as wrist or arm motion characteristics;
- Where in one example the subject with a wearable device (such as but not limited to wristband, ankle-band, armband, earphone(s), chest-band, other attachable device or watch) can ***automatically or manually exchange data*** with a data-interface compatible device (i.e. but not limited to wireless subject-applied head **EEG,** EOG and/or EMG signal-based sleep parameter monitoring system) in a way that enables a subject to automatically ***track sleep measures, indices and overall sleep quality*** (i.e. including measures such as wake after sleep onset, sleep efficiency, REM sleep amount, deep-sleep amount, sleep architecture fragmentation or normality, along with other optionally and user or health-carer programmable accessible prognostic, diagnostic and subject personal-care management measures enabling sleep-debt, sleep architecture, sleep requirements to be easily accessible and trackable based on each user's degree of required simplicity, complexity and sophistication;
- Where in one example the subject with a wearable device (such as but not limited to ***headband (******Figure 25******;*** ***Figure 3******;*** ***Figure 4******), wristband (******Figure 2******), ankle-band (******Figure 2******), armband (******Figure 2******;*** ***Figure 32; Figure 33******), earphone(s)*/*earbuds (******Figure 14******), chest-band (******Figure 31******),*** other ***attachable device or watch (Fiqure 6; Fiqure 7;*** ***Figure 9******;*** ***Figure 13******)*** can ***automatically or manually exchange data*** with a data-interface compatible device including an alarm clock or mobile phone or other communication-compatible device. For example, information that can be exchanged includes information relating to user/patient coaching (recommendations or guide for improved health or current health status), including indicators capable of ***recommending sleep requirements*** based on any combination of daily health, fitness/activity and sleep-monitored parameters (including but not limited to **EEG,** EOG and/or EMG signal-based sleep parameter measures), and/or environmental monitoring inputs (i.e. temperature, humidity, air pollutants, airborne pollen or other air contaminants etc.).

### Somfit minimum configuration

- the present example provides monitoring capabilities capable of any of or any combination of ***physiological channels*** including, but not limited to, **EEG**/P1, **EEG**/P2, **EEG**/Pz. EOG/L/P7, EOG/R/P8, patient posture or if possible multi-axis combined patient position and patient motion, infra-red breathing detection, microphone sound breathing monitoring, light detection (i.e. light detection resistor), ***optional reflective oximeter,*** optional wireless interconnectivity with any of or any combination of sensors required for any of categorisation formats applicable to ***type 1 to 4 study*** types or levels as further detailed herein.

**The present example comprises of physiological or environmental monitoring including any of or any combination of:**
- Sound monitoring; Stethoscope auscultation sensor, monitoring and automatic analysis, classification, tracking and detection capabilities; Acoustic noise cancellation system; Motion detection; REM sleep behaviour disorder (RBD); Pulse sensor integrated watch of other wrist worn device (band or bangle); Pulse wave analysis (PWA) and pulse wave velocity (PWV) monitoring and analysis capability; PWA and PWV sensors; Pulse wave analysis (PWA) sensor measures; Ballistocardiograph; Position, locational and movement sensing and monitoring; Gait or movement tracking and characterisation of events of interest; Movement and locational information; ECG sensor(s) and monitoring; Light sensor(s) and monitoring; Breathing band sensors and monitoring; EMG sensors and monitoring; GSR; Cardiac function; Heart rate; Sleep training system; Plethysmography Oximetry; Pulse transient oscillation amplitude measures; Temperature; Energy-exertion/metabolism-monitoring (EM) as a surrogate calorie-burn measure; **Sleep parameters;** Physiological and/or Sleep and/or Wake Markers; **Sleep parameters;** Sleep Architecture Measures; Environmental sensing; "dynamically-linked capabilities"; Psychological states; Whereby the present example comprises sensing, monitoring, data-acquisition, signal processing, analysis, storage, and information access including the online automatic characterisation of a subject/individual's physiological, neurological, nervous system, movement system, muscular system, psychological, pathological, states, events of interest and/or health conditions including any of or any combination of the following:
   Rapid Eye Movement (REM) Sleep Characteristics ; Sleep Disorder Classifications; Select Sleep disorders; Dreaming States; HALLUCINATION STATES; Dissociated States; Hypnosis States ; and as further outlined below and elsewhere in this patent application document.

The present example provides ***environmental monitoring or sensing comprising any of or any combination of:***
- ***Environmental sensing*** (with alarm or alert or indicator or interface to mobile device associated or messaging, email, phone automated voice message and other information or communication systems), weather elements, wind, humidity, temperature, ionisation monitoring, ionisation smoke alarm, methane monitoring, toxic gas monitoring, toxic chemical monitoring, C02 gas monitoring, methane gas monitoring, other toxic gases, other toxic chemicals and/or thermometer, and/or ***"**physiological or psychological monitoring including" and*/*or "Environmental sensing",*** as further detailed below and elsewhere in this document;
- Where in one example the subject with a wearable device (such as but not limited to headband ***(******Figure 25******;*** ***Figure 4******),*** wristband ***(******Figure 2******)**,* ankle-band ***(******Figure 2******)**,* armband ***(******Figure 2******;*** ***Figure 32; Figure 33******)**,* earphone(s) ***(******Figure 14******),*** chest-band ***(******Figure 31******)**,* , other attachable device or watch) can ***automatically or manually exchange data*** with a data-interface compatible device including a subject's room thermostat (or other communication-compatible device) capable of optimising sleep environmental conditions in accordance to a subject's preferences and/or also automatically based on environmentally monitored and/or subject-specific or biological-synchronisation characterisation (i.e. delineation of snoring partner where sound is not biologically synchronised to partner, and where option exists for automatic sleep-training feedback or notification (i.e. recommendations, hints, prognostic or diagnostic supporting data access for health-carers and the like) to offending snorer (for example only) of sleep disruption incidence(s))
- Where in one example the subject with a wearable device (such as but not limited to wristband, ankle-band, armband, earphone(s), chest-band, other attachable device or watch) can ***automatically or manually exchange data*** with a data-interface compatible device based on subject-specific or biological-synchronisation characterisation and determination, such as the delineation of snoring partner where sound is ***not biologically synchronised to partner,*** and where option exists for automatic sleep-training feedback or ***notification or adaptation, or adjustment is possible as it relates* to *causation of sleep disruption;***
- Where in one example the subject with a wearable device (such as but not limited to wristband, ankle-band, armband, earphone(s), chest-band, other attachable device or watch) can ***automatically or manually exchange data*** with a data-interface compatible device based on subject-specific or biological-synchronisation characterisation and determination as it relates to another person snoring (for example only) and causing sleep disruption to the user or wearer, whereby ***notification includes automatic alarms*** (i.e. mobile phone messages, alarms, calendar entries, events and/or sleep-trainer system and the like) or other sleep disruption deterrents applicable to recommendations, hints, prognostic or diagnostic supporting data access for health-carers or therapeutic adjustment (i.e. any of or any combination of automatic; biofeedback or manual adaptation, adjustment or reconfiguration) of user or subject or other nearby person(s);
- Where in one example the subject with a wearable device (such as but not limited to wristband, ankle-band, armband, earphone(s), chest-band, other attachable device or watch) can ***automatically or manually exchange data*** with a data-interface can include any network connection and/or available communication mediums, networks or other interconnectivity options (including simultaneously interconnecting with additional communication networks, systems or other interconnectivity options such as WWW, IP, LAN, WAN, supplementary/companion monitoring/sensing or computing systems, SAAS including Cloud-computing services or NAS, peer to peer connections etc.).

### Full-disclosure online physiological parameters

The present example comprises of a **portable device** (i.e. mobile wireless system, wrist band, smart watch, phone, PDA, headband, head-worn apparatus, attachable or pocket device etc.) incorporating means of indicating and/or tracking any of or any combination of a ***subject's sleep performance*/*function, sleep parameters, fitness or motion parameters, fitness or motion performance*/*function, health parameters and*/*or health*** performance/function, the present example further comprising any of or any combination of:
- ***gesture activated*** (i.e. tap motion, switch, finger slide, wrist shake etc.) as a means of toggling between any said parameters or performance/function measures or indices;
- wireless monitoring capability **enabling online monitoring of sleep parameters** (i.e. **EEG,** EMG, EOG etc.);
- full disclosure capacity of online monitoring of **sleep parameters** (i.e. **EEG,** EMG, EOG etc.); >whereby full disclosure includes means of displaying any of or any combination of **primary monitored raw data** (i.e. physiological waveform data), **secondary monitored data** (i.e. summaries of compressed data) and/or **tertiary data** (i.e. analysis transformation(s) of primary or secondary data such as, but not limited to, indices or spectral analysis, signal dynamics analysis (i.e. non-linear dynamic analysis), correlation analysis, coherence analysis, multivariate analysis, FFT and associated outputs, etc.);
- incorporation of MTM capability;
- incorporation of APM capability;
- incorporation of HDCM capability;
- synch somnisync (covered elsewhere in this patent application document);
- a wearable headband device incorporating **means of enabling full-disclosure (i.e. raw data such as any of EEG, EMG, and or EOG electrophysiological signal** fluctuations, including capability to enable bandwidth of all sleep and other neurological events including HFOs, spikes, spindles, vertex sharp waves, or other events or health conditions covered elsewhere in this document.
- incorporating means **"spectral compensation"** and "other compensation" in such a manner whereby **EEG** signals undergo analysis transformation applicable to a standardised (i.e. AASM or K&K - expand recommendations or standards) or any specific or conventional **EEG** electrode locations;
- **"spectral compensation"** and **"other compensation"** can comprise of a spectral transfer characteristics, phase transfer characteristics, signal amplitude, signal distortion, multiple signal superposition, capable of compensating for neural source locations otherwise attenuated by non-obtrusive monitoring constraints (i.e. forehead positions below hair- line such as Fp1, Fp2, F7, F8 and/or Fz) versus standardised positions such as (but not limited to) **EEG** locations traditionally utilised for **EEG** sleep monitoring parameters (i.e. including F4 - M1; C4 - M1; O2 - M2; with backup monitoring electrodes including F3 - M2; C3 - M2; o1 - M2 sleep monitoring electrode locations).
- whereby said **EEG signal compensation transfer characteristics** incorporate a means of emulating the **EEG** signal characteristics similar to another "designated alternative location" or "traditional location",
- whereby said determination of a **"designated alternative location"** can comprise of ***forward or inverse source localisation computations*;**
- whereby said determination of a "designated **alternative location**" can comprise of transfer characteristics based on empirical data studies investigating the comparative **EEG** signal characteristics of different **EEG** locations during sleep states in order to enable the relationship and associated transfer function required to convert;
- whereby the **EEG** signal of a **first monitored location** can be processed by a transfer function capable of generating (modelling) a data set applicable to the **approximate data set values** of a **second monitored EEG location;**
- whereby said transfer function is modelled in order **to most precisely simulate** data of the second location applicable to difference sleep stages (i.e. **AASM or R&K sleep scoring recommendations**);
- The present example enables a means of **full disclosure online signal monitoring,** sleep stage analysis, ongoing (whereby means includes the capability to access real-time sample by sample or regularly ***sampled real-time monitored information in order to compute measures indicative of continuous, uninterrupted, raw-data,*** versus only summaries or compressed versions of data, in order to enable diagnostic quality and industry standard (i.e. ASSM and/or R&K scoring recommendations of human sleep, respiration and other related aspects) and along with the capability to generate accurate measures of monitored sleep variables, derive measure of sleep progress, performance, and status (i.e. and other sleep measure, but not limited to, as further outlined in section ***"Physiological and*/*or Sleep and*/*or Wake Markers;"*** and other sections detailed elsewhere in this document) and also enable capability for online remote or local online analysis.
- whereby said analysis and monitoring capabilities can be updated online or in virtual real-time **so that an individual can read a display indicator** (including wireless linked to sleep parameter (i.e. **EEG,** EMG and/ or EOG) monitoring system) at any time during the subject's sleep or associated wake states using a patient worn or mobile or remote computer device or information access system;

The present example enables a wearable mobile monitoring system comprising of a means of ***wake and*/*or sleep health monitoring*** and management, the said means comprising any of or any combination of (but not limited to) descriptions presented elsewhere in this patent application document including **a) Forehead applied physiological monitoring sensors, b) Re-usable or disposable sensors, c) Reusable or disposable sensors automatic reload dispenser device, d) Continuous online sleep parameter (EEG, EOG, EMG) monitoring and sleep analysis, e) Wearable monitoring, f) Exchangeable/interchangeable part with dynamic data exchange, g) Infrared respirology or body heat flux monitoring, h) Body position and/or position and/or movement, i) Movement and/or motion, j) Oximeter, k) PPG,** as further outlined here:
**a) Forehead applied physiological monitoring sensors**
   - the present example enables the monitoring of 1 or more forehead applied physiological monitoring sensors with option of deriving a plurality of **sleep parameters** from any one or more said sensor(s);
   - option whereby said **"sleep parameters"** monitoring includes any of or any combination of **EEG,** EOG, EMG, and/or ECG;
   - option whereby said **"sleep parameters"** includes any of or any combination of those further detailed under sub-headings section headed "
      ***Title:*** eLifeKIT:" and ***"**Title: eLifeCHEST*/*eLifeSCOPE (chestband) (******Figure 5******[3];[4];*** ***Figure 16******;*** ***Figure 31******),"*** described elsewhere in this patent application document;
   - option of forehead applied electrodes which can comprise of partial or total head, forehead and or face coverage;
   - option of self-adhesive forehead applied strip, whereby pressure interface in order to attain a quality electrode connection for monitoring of **sleep parameters** from patient/subject's forehead is not required;
   - option of disposable self-adhesive forehead applied strip;
**b) Re-usable or disposable sensors**
   - the present example provides the option of disposable self-adhesive forehead applied strip with electrode dispenser device capable of automatically discarding old sensor and replacing with new sensor;
**c) Reusable or disposable sensors automatic reload dispenser device**
   - the present example provides the option of disposable self-adhesive forehead applied strip with electrode dispenser device capable of automatically discarding old sensor and replacing with new sensor, whereby said sensor dispensing device is part of packaging system of a pack of new self-adhesive sensors;
   - the present example provides the option of **disposable self-adhesive forehead applied strip with electrode dispenser** device capable of automatically discarding old sensor and replacing with new sensor, whereby said sensor dispensing device is part of packaging system of a pack of new self-adhesive sensors, and whereby said dispensing device comprises any of or any combination of (but is not limited to) a) enabling replacement of monitoring sensor from forehead monitoring strip, b)
   - sensor injector and eject process that removes and safely stores or contains used sensor while or subsequently applying new sensor, with option of single action push, discard old sensor and reload new sensor;
   - where, for example **forehead sensor strip can be push loaded unto and then retracted** from the said sensor dispenser device as a means ( i.e. a spring tensioned dispenser mechanism can push new replacement sensor onto forehead sensor holder device, while used sensor at the same time is peeled off and discarded. The reloaded sensor and forehead sensor can then be ejected with single depression of ejection button /lever in order to access reloaded sensor system.

   > In this way a package of **disposable electrodes can be loaded into a sensor reloading device which in a single push action** enables a used sensor to be peeled away from Somfit device and discarded, while new sensor's backing paper can be peeled away ready for adhesion to Somfit device, followed by a further spring-loading mechanism being activated at the downward extremity during insertion of the Somfit insertion into sensor dispenser device, in order for the new sensor (backing paper now peeled back) to now be pressure applied via a sensor spring-loaded mechanism, and finally ejection of the Somfit from the sensor dispenser device can result in a loaded sensor with the option of a quality control code transfer from the dispenser device verifying operation and transmitting a warranty violation and risk code where counterfeit sensors are being used;
   >The present example provides **continuous, uninterrupted sleep parameter monitoring including** (but not limited to) any of or any combination of **EEG,** EMG, EOG monitoring for online accurate, continuous, uninterrupted sleep/wake hypnogram and associated sleep quality determination and coaching, and/or intelligent clock interface (i.e. wrist watch or alarm clock settings);
**d) Continuous online sleep parameter (EEG, EOG, EMG) monitoring and sleep analysis**
   > the present example provides continuous **EEG** monitoring with associated analysis capable of the ***determination of epoching*** (time period segmentation) and ***epoch-based sleep stages*** (i.e. such as but not limited to wake, N1, N2, N3, REM) and/or sleep events (i.e. such as but not limited to spindles, k-complexes, vertex-waves, alpha-bursts, body movements, arousals etc.), and/or sleep measures (i.e. not limited to measures covered elsewhere in section including but not limited to) or indices (i.e. but not limited to sleep efficiency/SE, wake after sleep onset (WASO), respiratory event related arousals/RERA, therapeutic event related arousals/TERA, apnoea/hypopnoea index/AHI, sleep disturbance index/SDI, respiratory disturbance index/RDI, sleep fragmentation, percentage and amount of each sleep stage, total sleep time/TST, sleep hypopnoea, sleep delay syndrome delay factors, residual daytime sleepiness/RDS, arousal index/Al, degree **and other sleep** measures such as but not limited to sleep disorders or other events of interest (i.e. including but not limited to events of interest "**DENIFITION**", as described elsewhere in this document);

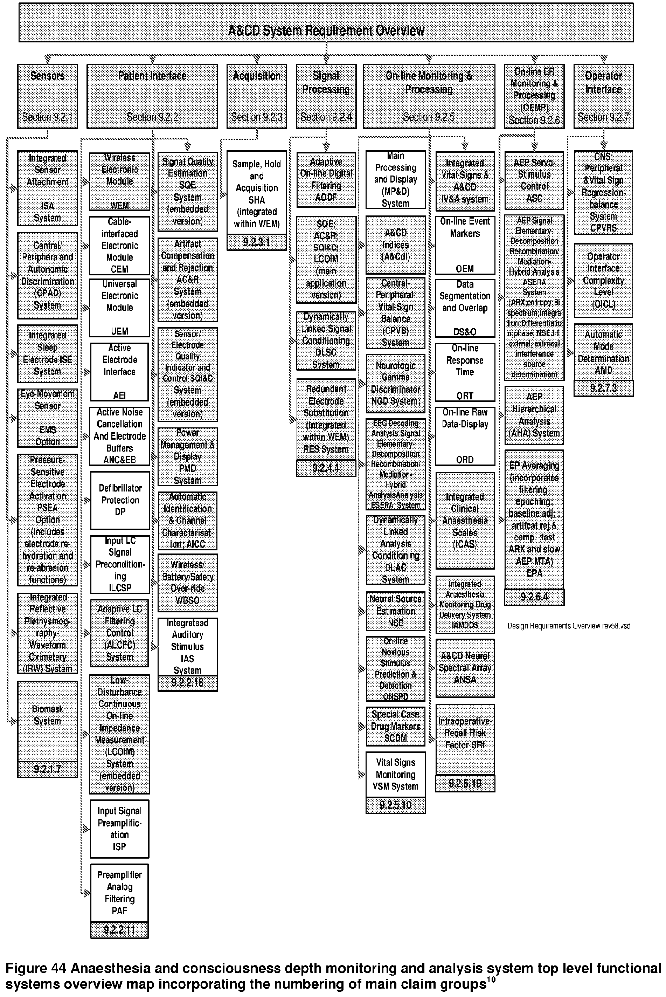

   - whereby said ***determination of epoching*** comprises of segmenting data in blocks of time such as 30 seconds;
   - whereby said ***epoch-based sleep stages*** determination can comprise of local (micro-processing device or DSP system forming part of monitoring sensor or device) and/or available communication mediums, networks or other interconnectivity options (including simultaneously interconnecting with additional or supplementary communication networks, systems or other interconnectivity options such as WWW, IP, LAN, WAN, supplementary/companion monitoring/sensing or computing systems, SAAS including Cloud-computing services or NAS, peer to peer connections etc.).
**e) Wearable monitoring**
   > the present example wearable monitoring (including option of mobile wireless interconnected) devices or associated system van comprise of any of or any combination of example monitoring systems per ***Figure 1*** to ***Figure 94*****;**
**f) Exchangeable/interchangeable part with dynamic data exchange**

The present example incorporates a ***means of connectivity*** and/or ***exchangeable parts*** between two or more wearable devices/systems as a ***means on enabling information access applicable to sleep health* as *well* as** ***general health**,* and further comprises any or any combination of:
- the said *"**means of connectivity**"* can include any of or any combinations of connectivity with available communication mediums, networks or other interconnectivity options (including simultaneously interconnecting with additional or supplementary communication networks, systems or other interconnectivity options such as WWW, IP, LAN, WAN, supplementary/companion monitoring/sensing or computing systems, SAAS including Cloud services or NAS, peer to peer connections etc.), various data-communication channels and/or different mediums, cellular network, optical communications network, Wi-Fi, blue-tooth, satellite, SMS, copper communications network, Wi-Fi, pager alerts, automatic phone alerts, calendar updates, social or business information interfaces, etc.
- the said *"****exchangeable*/*****interchangeable part**"* can comprise of an electronic element or component of a first wearable device which has means of attachment (i.e. but not limited to magnetic interlocking, mechanical interlocking, adhesive interlocking, material or matter interlocking etc.) or interconnectivity with a second wearable device and/or other nearby device,
- the said *"**means of enabling information access applicable to sleep health as well as general health**"* can include combining, sharing, exchanging indications, displays, data storage, data processing, deriving indices, based on ***sleep monitored parameters*** or associated measurement derivations (i.e. such as **EEG,** EOG, EMG, breathing sound, room or environmental sound, room light conditions, airflow, reflective plethysmography oximetry and associated outputs (pulse wave amplitude/PWA, pulse arterial tone/PAT, Pulse arterial tone/PTT, pulse-wave oscillatory amplitude autonomic markers of obstructive apnoea to enable central versus obstructive discrimination (i.e. the increased activation of breathing muscles evoke additional blood-flow, which in turn appear as discernible oscillatory or amplitude fluctuations which can be detected using signal morphological, spectral and other events extraction techniques), along with other monitored **sleep parameters** or associated measures outlined elsewhere in this document) and other ***health monitored parameters or associated measurements** ;*
- whereby the present example can enable "separate or combined sleep and/or other health and/or fitness measures and/or information" to be indicated or displayed as part of any wearable devices (i.e. wrist device, watch, clock etc.);
- whereby said *"**means on enabling information access applicable to sleep health as well as general health"*** can include dynamically exchanging (i.e. **dynamic data exchange/DDE**) data comprising of any said "separate or combined sleep and/or other health and/or fitness measures and/or information", or other health status or progress;

### g) Infrared respirology/thermal airflow or body heat flux monitoring

The present example can incorporate one or more "attached infrared" breathing monitoring sensor(s) and/or skin heat flux monitoring centre(s) (body energy dissipation via skin surface, enabling measure of energy or calories burning/metabolic-activity of subject);

### IR sensor/lens

The present example can comprise of one or more infrared sensors with option of associated lens in a manner whereby the sensor is incorporated as part of a forehead sensor that can be located and the sensor directed to detect a subject's breathing and associated breathing disorders;

### IR lens

The said "infrared sensors" can be combined with one or more lens systems capable of focussing respiration heat change associated with subject's oral and/or nasal breathing inspiration and expiration towards direction of infrared sensor;

### IR thermal

The said "infrared sensor" can comprise of thermal sensors including any of or any combination of (but not limited to) photo diode, photo conductive, photovoltaic, pyro-electric types;

### Photonic

The said "infrared sensor" can comprise of photonic (photo-detectors) including any of or any combination of (but not limited to) charge coupled device or active pixel sensors (CMOS);

### h) Body position and/or position and/or movement

The present example can incorporate one or more subject/patient body/position sensors;

### i) Movement and/or motion

The present example can incorporate one or more movement and/or motion sensors (i.e. but not limited to one or more axis accelerometers) as detailed elsewhere in sections titled "Position, locational and movement sensing and monitoring";

### j) Oximeter and/or PPG

- The present example can incorporate one or more attached photo-plethysmography (PPG) sensor(s), and/or reflective oximeter(s) with the option of plethysmography function, as detailed elsewhere in sections titled "Plethysmography Oximetry and/or photo-plethysmography (PPG)";

### k) Forehead or, head or body attached thermal airflow monitoring

- wearable sound monitoring (i.e. but not limited to ***forehead or, head or body attached microphone***) incorporating combination of breathing sound (i.e. but not limited to microphone) and other physiological signal (i.e. ***thermal airflow monitoring*** including, but not limited to infrared sensor capable of tracking airflow temperature changes related to breathing subject/patient) as a means of discriminating between subject/patent/user of interest versus other person (i.e. but not limited to sleeping partner snoring);
   - the present example combines one or more wearable monitoring systems (i.e. but not limited to head attached or applied device with sound or thermal monitoring sensor(s))) and/or one or more ***microphone sensors capable of distinguishing of differentiating two or more sound sources*** in order to ***detect breathing sounds of one or more subject(s)*/*patient(s)*/*individual(s)*/*user(s);***
   - whereby means of monitoring breathing sounds (i.e. a measure of monitoring any of or any combination of sound, airflow, thermal breathing characteristics related to inspiration and/or expiration and/or aspirating and/or nasal aspirating and/or oral aspirating of a subject/patent/user) can incorporate ***tracking breath for variations in magnitude or cessation of breathing*** in a manner where hypopnoea (i.e. such as but not limited to reduced breathing for a period) and/or ***where apnoea*** (cessation of breathing for a period) and or ***hypoxia*** (i.e. such as but not limited to sustained reduction in breathing), along with other sleep breathing disorders;
- whereby such monitoring can incorporate a means of determining one or more sleep/wake states or stages of said subject/patent/user;

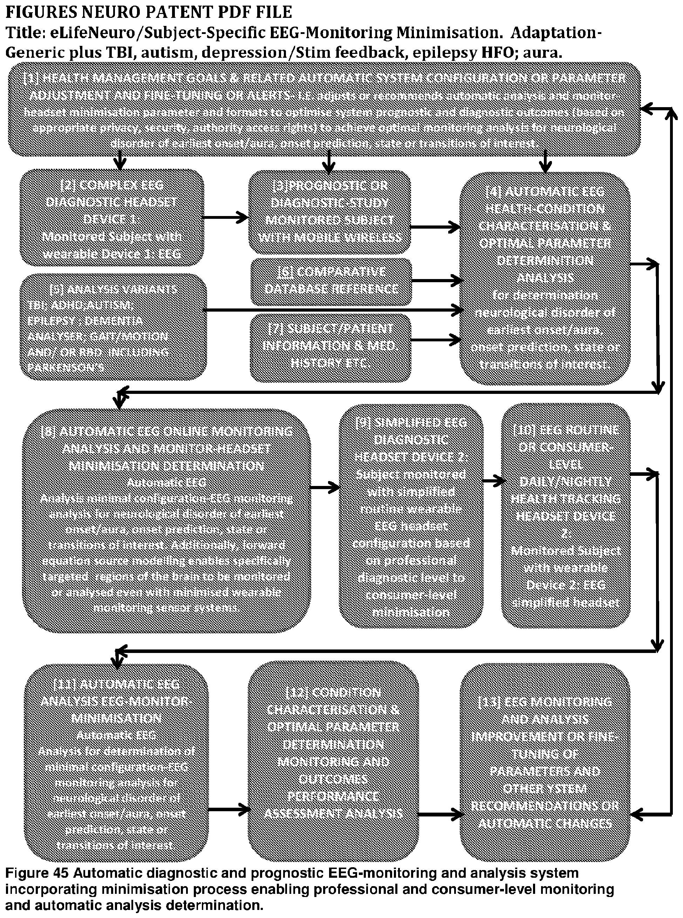

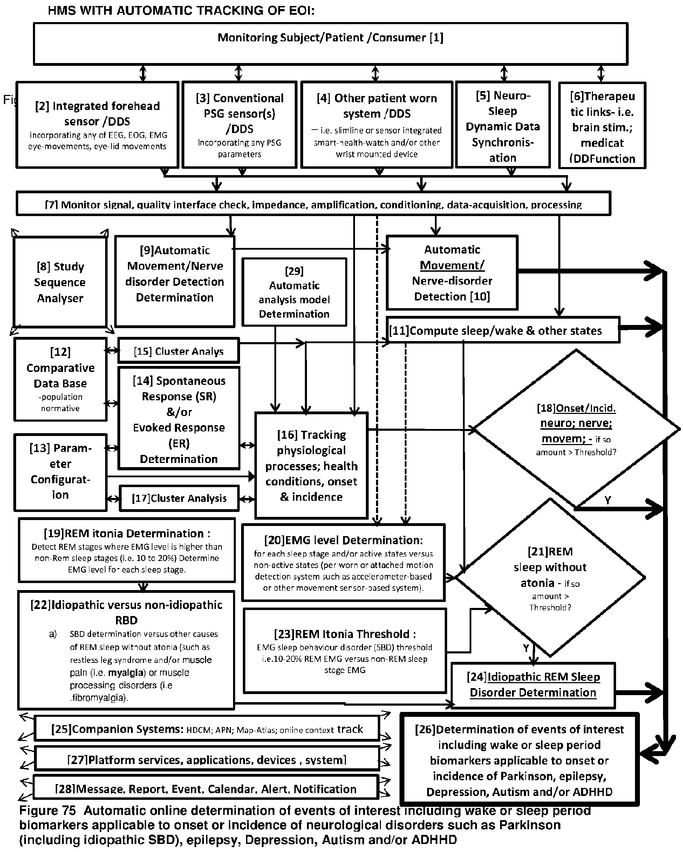

**1) Forehead sensor with multifunctional physiological parameters applicable** to **diagnosis and prognosis of a subject/patient's true homeostatic sleep/wake, circadian clock and associated sleep disorder factors**
- the present example provides a ***wearable forehead sensor*** (one or more sensors and/or electrophysiological electrodes) strip sleep monitoring system incorporating means of monitoring any of or any combination of **sleep parameters** (i.e. ***EEG, EOG, EMG***), along with any of or any combination of (but not limited to) monitoring, determination and/or tracking of:
   - ***sleep disordered breathing*** monitoring and associated event determination (i.e. microphone and/or thermals respiration determination - i.e. infrared breathing detection), and/or environment light sensing (i.e. light dependent resistor enabling computation of sleep efficiency and other measures),
   - ***body temperature measures*** applicable to estimation or determination or contribution to **circadian** clock cycle computation;
   - ***one or more "clock" determinations (*** including **circadian** clock (CC) factors (as well as any of or any combination of a subject/individual's travel, work, social, relation, schedule (i.e. clock, calendar, itinerary, agenda, requirements for any of or any combination of (but not limited to) sleep/wake, work, social activities, leisure, relaxation, sports or exercise activities; whereby "clock" refers to daily or weekly schedules according to an individual's social schedule (i.e. "social clock) or work schedule (i.e. work clock) or travel agenda/schedule (travel clock) etc.;
   - ***environmental time determination*** (i.e. via information access to inbuilt GSM, GPS or interconnectivity with mobile device, clock, clock settings, clock or time applications etc.);
   - monitoring, determination and/or tracking of activity or motion detection as a measure of actigraphy and/or patient position;
   - ***circadian clock cycle or offset and other factors*** with relation to environmental clock, time-zone conditions, and/or other applicable subject/user time factors (i.e. via calendar or schedule or itinerary information access to enable determination of social clock, work clock, leisure clock, sleep/wake clock and outcomes ***including offset with CC*** along lack or synchronisation or implications of ***sleep quality risk*** (i.e. due to asynchronous relationship with CC), ***sleep urge risk*** (i.e. result of previous sleep/wake historical information), sleep duration versus sleep quality risk as a result of CC **offset** factor, etc.);
   - ***sound envelope analogue signal or digital data processing capabilities*** (i.e. ability to track sound envelope where memory storage and processing requirements are otherwise prohibitive in terms of processing higher bandwidth sound waveform signals, for example, (i.e. as a data reduction mechanism for sleep breathing disorder monitoring whereby sound envelope can be extracted before or after data acquisition, signal processing or memory storage;
   - ***with further option of the incorporation of* a *forehead oximeter*** (including option of ***plethysmography reflective*** with capability of deriving oximeter outputs including any of or any combination of pulse wave amplitude (PWA), pulse arterial tone (PAT), characterisation of plethysmography amplitude as a means of distinguishing obstructive (i.e. generation of oximeter plethysmography waveform oscillation corresponding to autonomic disruption evident during obstructive sleep apnoea autonomic disturbances),
   - ***Wearable forehead strip sleep monitoring system incorporating an active circuit element*** able to be interchanged between wrist mounted monitoring system (i.e. containing motion, position, stride (including interaction with mobile phone motion detection as a measure of stride versus arm motion symmetry and/or synchronicity applicable to movement or neural disorders such as Parkinson gait dysfunction);
   - ***characterisation of subject motion, body movements, body vibrations, by using multi-axis accelerometer*** (i.e. whereby any one (i.e. spectral segmentation in accordance to primary energy band of various monitoring goals such as gait/fall/cardioballistogram; stride symmetry or synchronicity between and of subjects extremities or limbs and body) or plurality of single or multi-axis accelerometer sensors can monitor and determine any of or any combination of subject/ patient physiological or movement monitoring including ***any of or any combination of capable of*:**
      1) cardioballistogram such as via sensing of cardiac pulse variations;
      2) subject/patient body position/posture;
      3) subject gait or stride characteristics including but not limited to stride symmetry or synchronicity between and of subjects extremities or limbs and body applicable to activity or disorder tracking ( i.e. Parkinson's movement disorder);
      4) determination of subject/patient walking or jogging or running steps;
      6) fall/trip/stumble determination;
   - whereby means of "monitoring and/or characterisation of subject motion, body movements, body vibrations, by using multi-axis accelerometer" can comprise any of any combination of:
      *>**FIRST STEP*** COMPRISING SUBJECT MONITORING with one or more physiological signal channels, including those sensor monitoring channels for motion detection (i.e. accelerometer with one or more axis,), pressure sensor channels such as measurement of cardioballistogram, pulse or tonometry vascular pressure changes;
      **>*SECOND PROCESS STEP (SENSOR SIGNAL PROCESSING)*** involving the signal processing (i.e. amplification and/or filtering of accelerometer) of one or more signals from a single or multi-axis accelerometer (i.e. 3 or more axis but not limited to);
      **>*THIRD PROCESS STEP (ACQUISITION)*** involving acquisition (i.e. by way of analogue to signal acquisition or acquisition of digital data in accordance to sensor format or requirements);
      **>*FOURTH PROCESS STEP(S)* comprises any of or any combination of analyses including (but not limited to) the analysis options present elsewhere in this document including but not limited to provisions further detailed under** *Figure 1 to Figure 99*, *but particularly as it applies top examples of ANALYSIS VARIANTS INCLUDING THOSE APPLICABLE TO GAIT OR MOTION ANALYSER INCUDING PARKINSON ONSET SYMTOMS AND*/*OR COMBINED SBD SLEEP BEHAVIOUR DISORDER DETERMINATION (****Figure 45*** *[5]);excessive motion associated with REM without atonia* as *with during RBD, or associated with restless legs syndrome, or with muscle processing disorders such as fibromyalgia per* ***Figure 75*** *[20];[22]);* also as further detailed under heading **"Motion detection and/or actigraphy", "REM sleep behaviour disorder (RBD)", "Pulse sensor integrated watch of other wrist worn device (band or bangle)", "Pulse wave analysis (PWA) and pulse wave velocity (PWV) monitoring and analysis capability", "PWA and PWV sensors, "Pulse wave analysis (PWA) sensor measures", "Ballistocardiograph", "Position, locational and movement sensing and monitoring", "Movement and locational information", "Gait or movement tracking and characterisation of events of interest",** covered elsewhere in the **"eLifeCHEST/eLifeSCOPE" section of** this document.
      ***>ADDITIONAL FOURTH PROCESS STEP*** (ANALYSIS - SPECTRAL OPTION) **incorporating FFT or other monitored spectral, morphological/pattern, signal dynamics (i.e. NLDB), coherence (or other analysis correlation or variance approaches)** between two or more sensor outputs, amplitude or power analysis designed to characterise **characterisation of subject motion, body movements, body vibrations,** in terms of signal sources (i.e. in terms of the segmenting movement signals according to motion characterisation along with associated **motion source association, such as stride versus arm motion symmetry and/or synchronicity** applicable to movement or neural disorders such as Parkinson gait dysfunction; fall-detection; activity versus **idiopathic RBD with associated muscle tonicity changes (i.e.** REM sleep without atonia); steps; movement; hyperkinesia (exaggeration of unwanted motion), such as twitching or writhing in Huntington's disease or Tourette's Syndrome; tremor, or other movements; diagnosis and treatment of movement disorders, including Parkinsonian Tremor, Restless Legs Syndrome, Dystonia, Wilson's Disease or Huntington's disease; bradykinesia (i.e. slowness of movement) and dyskinesia (i.e. diminished voluntary movements; involuntary movements), whereby any of or any combination of analysis techniques covered elsewhere in this document including but not limited to Figure 1 to Figure 99, but particularly as it applies top examples of ANALYSIS VARIANTS INCLUDING THOSE APPLICABLE TO GAIT OR MOTION ANALYSER INCUDING PARKINSON ONSET SYMTOMS AND/OR COMBINED SBD SLEEP BEHAVIOUR DISORDER DETERMINATION (***Figure 45*** ***[5]);***excessive motion associated with REM without atonia as with during RBD, or associated with restless legs syndrome, or with muscle processing disorders such as fibromyalgia per ***Figure 75*** ***[20];[22]);*** also as further detailed under headings "Motion detection and/or actigraphy", "REM sleep behaviour disorder (RBD)", "Pulse sensor integrated watch of other wrist worn device (band or bangle)", "Pulse wave analysis (PWA) and pulse wave velocity (PWV) monitoring and analysis capability", "PWA and PWV sensors, "Pulse wave analysis (PWA) sensor measures", "Ballistocardiograph", "Position, locational and movement sensing and monitoring", "Movement and locational information", "Gait or movement tracking and characterisation of events of interest", covered elsewhere in the "eLifeCHEST/eLifeSCOPE" section of this document.
      **>*FOURTH PROCESS STEP*** whereby motion and/or patient position analysis techniques including any of or any combination of analysis techniques covered elsewhere in this document including but not limited to ***"Position, locational and movement sensing and monitoring", "Movement and locational information ", "Gait or movement tracking and characterisation of events of interest",* covered elsewhere in the** "eLifeCHEST/eLifeSCOPE" section of **this document,** and whereby these analyses can be applied to any of or any combination of sensor outputs (i.e. including a plurality of accelerometer axes);
      **>*FOURTH PROCESS STEP*** can include gyro sensor positional (based on vertical or horizontal positioning degree) outputs as a measure of a subject/patient's gait and/or smoothness and/or staggered stride or walk, whereby motion and/or patient position analysis techniques including any of or any combination of analysis techniques covered elsewhere in this document including but not limited to provisions further detailed under heading "Position, locational and movement sensing and monitoring", **covered elsewhere in the** "eLifeCHEST/eLifeSCOPE" section of **this document;**
      ***>FIFTH PROCESS STEP*** whereby analysis outcomes can be deployed as part of prognosis or diagnosis determination for a subject/individual;
      ***>SIXTH PROCESS STEP*** whereby said analysis outcomes and/or associated results can be disseminated in terms of messages, events, calendar information or data access, information networks (social, work, professional etc.);

### CIRCADIAN TEMPERATURE CYCLE DETERMINATION VERSUS LOCAL ENVIRONMENT AND/OR SLEEP/WAKE/ACTIVITY/WORK

- A nub of the personal health management system is to ***automatically determine and indicate, coach, alert, message, CC entrainment stimulation applicable to subject*/*patient*/*user based on CC input factors, a range of* scenarios** , ***covering sleep quality and length relating to the interrelationship or the manner the said subject works with or against their natural CC***;
- Moreover, the present example can provide a number of ***entrainment scenarios such* as *the intervention with subject-wearable device or environmental lighting*** as a means various degrees of advancing or delaying a subject's phase response curve (i.e. a subjects inbuilt **circadian** curve phase relationship with external clock factors, including social, time-zone, work, work-shift, study requirements etc.) applicable to ***minimising delayed sleep phase disorder (DSPD) or advanced sleep phase disorder (ASPD)*** according to a subject's healthcare oversight or intervention and/or an individual's personal preferences or requirements and/or occupational hazard and safety considerations;
- The present example can automatically or via manual assistance ***activate light intensity and type*** (i.e. visible blue light with short wavelength, and stronger melatonin suppression affect can be deployed as part of an automatically computed CC entrainment treatment regime, versus longer wavelength light) as well as the timing functions of such light therapy (i.e. light-therapy in the evening can enable CC phase delay, while light therapy in the day can product CC phase advancement);
- The present example can ***automatically (or with manual intervention option) control entrainment factors*** (i.e. lighting timing and/or lux intensity and/or melatonin dosage and administration timing or recommendation), as well as the option of recommending or setting bed-times or alarm clock settings, in accordance to a subject/patient (or healthcare advisor) social, work, travel requirements or environmental factors;
- The present example can advise/coach ***and*/*or automatically adjust said CC entrainment*** in accordance to subject/patient/user preferences, selections or personalised scenario choices (i.e. more aggressive adjustment over a shorter period of days or more moderate CC adjustment over a longer period of days);
- The present example can ***automatically access a travel agenda*** and based on any of or any combination of these or other CC and sleep homeostatic factors:
   >sleep duration;
   >sleep time;
   >bed-time;
   >sleep quality;
   >sleep urge or deprivation;
   >work-clock, social-clock;
   >CC phase **offset** with any other said clock:
      >alarm clock settings:
      >map application visual annotation showing various travel agendas and likely CC adjustment consequences such as the east-west travel versus west-east varying travel jet-lag factors;
      >various travel schedule can automatically access or interface travel information relating to one or more travel itineraries as a means of generating optimal travel itineraries in accordance to CC entrainment options or optimal subject/patient/user performance outcomes in terms of subject's peak energy CC levels or a subject's optimal sleep bed-times and/or sleep duration times;
      >agenda interface to a travel scenarios based on user's preferences in terms of prioritising these said factors, various alarm clock schedule scenarios based on subject/patient/user prioritising these said factors;
      >various ***map application including annotations or associated information annotation*** of CC versus other environmental clock/jet-lag/social/work/sleep deprivation-urge/sleep duration/sleep-quality etc.;
      > alarm clock schedule scenarios based on subject/patient/user prioritising these said factors) sleep taking into account known etc., applicable to personalised subject/patient/user preferences for CC versus social-clock, versus work-clock, versus solar clock, versus time-zone clock, versus sleep duration versus sleep quality versus bed times versus sleep times;
         the present example can measure environmental lighting conditions applicable to subject/patient/user (i.e. via wearable device such as watch, mobile device etc.) in order to provide coaching or guidance to treat winter depression or other forms of depression or ***delayed sleep phase disorder (DSPD),*** or compensate for **offset** between CC and environmental (i.e. time zone or solar clock factors or behavioural clock properties (i.e. social clock, work-clock, shift-works, travel/jet-lag clock, clock and associated requirements or planning/scheduling preferences), whereby said coaching can include CC **offset** therapy (i.e. light therapy, melatonin medication, adaptation of homeostatic sleep factors (i.e. optimal increasing of a subject/patient/user's awake period to enable higher quality sleep and alignment of CC with sleep patterns or vice versa);
         the present example can ***automatically incorporate all CC entrainment factors, indication aspects, alarm clock functions, light detection functions, coaching and*/*or messaging and*/*or alert functions, into a single application as part of a wearable or mobile device*;**
         The present example can determine ***circadian clock nadir factors*** (i.e. body temperature and/or interval from body temperature nadir to sleep **offset)** including subject/patient/user's with ***delayed sleep phase syndrome (DSPS)*** in order to optimise CC entrainment (i.e. light therapy including glasses with blue light projected towards subject/patient/user retina as a stimulus - can be blocked form forward projection based on shaded or blocked upper section of glasses in order to minimise obtrusive or obvious nature of such treatment), whereby light exposure prior to the nadir of the core body temperature rhythm can produce phase delay, whilst light therapy (bright light therapy) administered after nadir can result in advancement of phase;
         The present example can track sleep-wake rhythms and characterise a lack of clearly discernible **circadian** patterns of sleep-wake time, as a marker or ***potential prognosis of irregular sleep-wake rhythm***;
         The present example can in incorporate sleep-wake rhythms and characterise a lack of clearly discernible **circadian** patterns of sleep-wake time, and/or questionnaire outcomes relating to excessive sleepiness , unrefreshing sleep, and/or insomnia that vary in accordance to work schedule as a marker or ***potential prognosis of shift work disorder (SWD)** ;*

### Circadian algorithm: circadian-automatic regressive analysis modelling with context analysis

Whilst conventional scientific laboratory measures of **circadian** clock temperature cycles implicate core body measures generally not suited to routine or **circadian** monitoring (rectal temperature probe monitoring), in contrast a nub of the present example is to enable the derivation of an individual's (subject/patient/user's) natural inbuilt (endogenous) **circadian** temperature by way of **circadian-automatic regressive analysis modelling** with one or more **external inputs** (CRX).

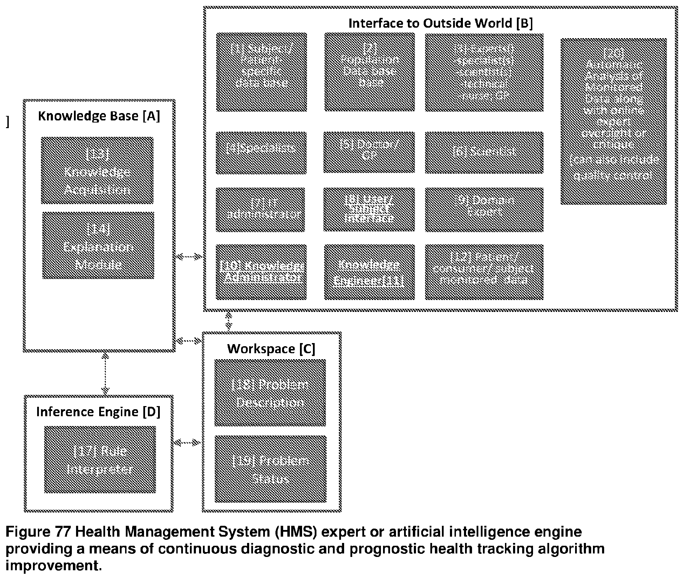

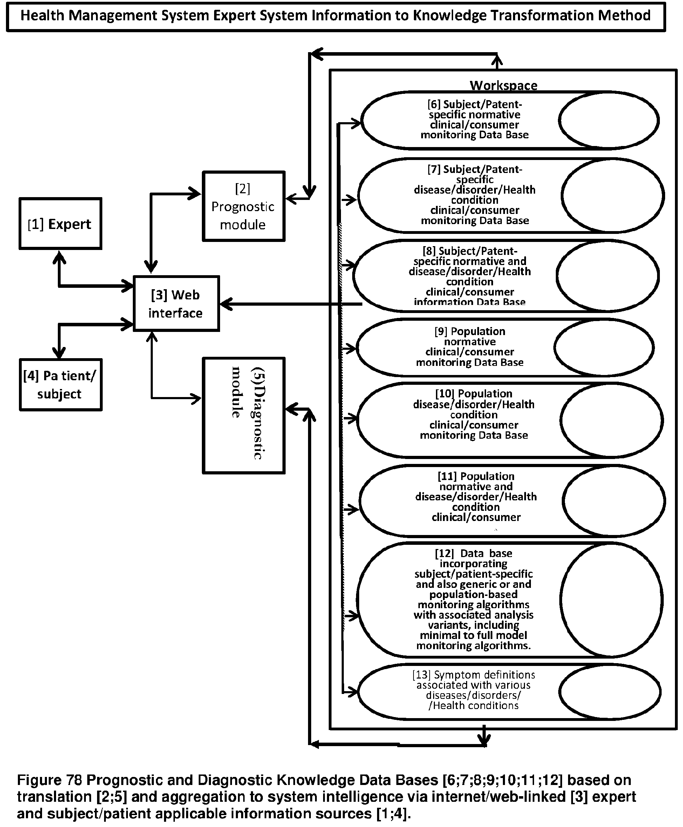

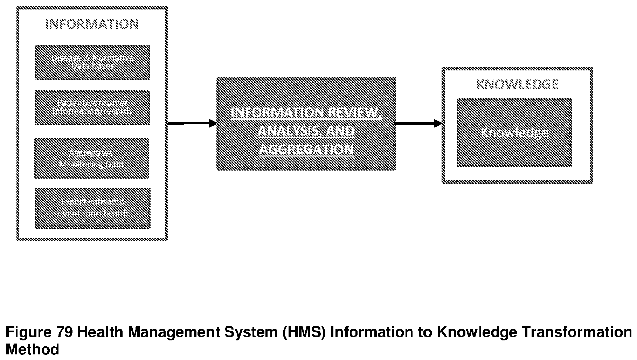

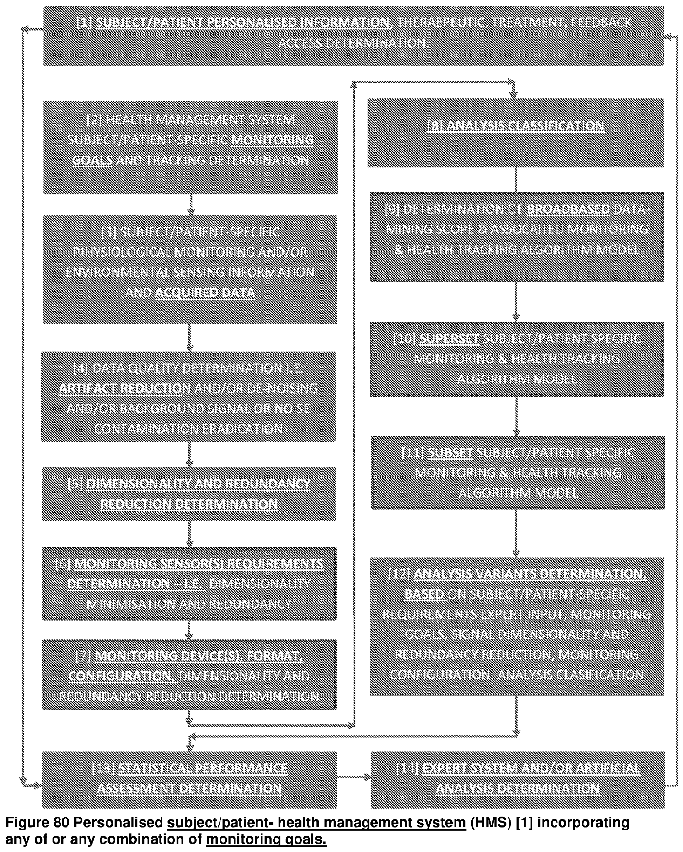

The said **circadian-automatic regressive analysis modelling** can incorporate **context analysis;**
**a) Circadian algorithm processing: context analysis**
   whereby said **context analysis** (not limited to) in one example of a model only can include **inputs to the said model comprising of an individual's inbuilt circadian rhythm clock information** can include any of or any combination of (but not limited to):
**b) Circadian algorithm input: survey(s) or tracking subject/patient information**
   - input data via patient or other person based on **survey(s) or tracking subject/patient information,** such as sleep propensity or sleepiness information such as with Epworth Sleepiness scale or other survey/scales/measures, or routine sleep, wake, work, recreation and/or other active routines, similar data derived from applications such as health apps or calendars, schedulers, health applications, wearable or mobile devices etc.;
**c) Circadian algorithm input: sleep study information**
   - data accessed or input automatically, manually or via computer-assistance relating to **sleep study information;**
**d) Circadian algorithm input:** sleep monitoring studies or applications
   - data accessed or input automatically, manually or via computer-assistance relating to **sleep monitoring studies or applications** associated with the monitoring, system, applications, or other application capabilities (i.e. such as, but not limited to, those further outlined elsewhere this document);
**e) Circadian algorithm input: information relating to fitness, health monitoring and/or related applications**
   - **information relating to fitness, health monitoring and/or related applications** associated with the monitoring, system, applications, or other application capabilities (i.e. such as, but not limited to, those further outlined elsewhere this document);data accessed, input automatically, manually or via computer-assistance or derived relating to
**f) Circadian algorithm input: information or information derivations based on sleep, fitness or other health applications, devices and/or systems**
   - information or derivation from any combination of information such as (but not limited to) a clock or mobile phone or other software applications or systems containing information relating to subject/patient's activity, sports, work, sleep, wake, alarm clock, schedules or routines or timing data;
**g) Circadian algorithm input:** local or new time zone information
   - **local or new time zone information** (i.e. but not limited to GSM, GPS, radio clock or other timing source);
**h) Circadian algorithm output:** phase shift between inbuilt **circadian** clock **and local environment time-zone**
   whereby outputs of said **context analysis model** (not limited to) in one example of a model only can include any of or any combination of (but not limited to):
   - **phase shift between inbuilt circadian clock** rhythms and local environment time-zone properties or related subject/patient schedules or required routines and/or time cycles;
**i) Circadian algorithm output:** coaching or recommendation of optimal alarm clock or scheduling
   - **coaching or recommendation of optimal alarm clock or scheduling** or time-management aspects based on any of or any combination of said model inputs;
**j) Circadian algorithm processing:** auto-regression estimation of natural or inbuilt estimation of local or new environment **circadian** clock/**rhythm**
   - **auto-regression estimation of natural or inbuilt** subject/patient's **circadian clock**/rhythm;
   - **auto-regression estimation of local or new environment** subject/patient's **circadian clock**/rhythm;
**k) Circadian algorithm processing options:** auto-regression estimation/determination of **subject wearable or attachable temperature sensors/probes whereby a low pass filtering function capable of emphasising low frequency cyclic changes** for derivation of natural/inbuilt or new environment **circadian** clock/**rhythm status or requirements**
   The automatic regressive analysis can be subject wearable or attachable temperature sensors/probes whereby a low pass filtering function capable of emphasising low frequency cyclic changes (i.e. 24 hour **circadian** core temperature changes versus external environment or shorter term activity or exercise mainly independent of **circadian** core temperature changes);
**l) Circadian algorithm inputs:** "external inputs" **including** derivation of natural **circadian** clock
   The said "**external inputs**" can include any of or any combination of (but not limited to):
   - **natural or derivation of natural circadian clock** (i.e. via any of or any combination of temperature measures and/or **EEG** measures);
**m) Circadian algorithm processing inputs:** new environment or time zone 24 hour cycle
   - **new environment or time zone 24 hour cycle** (i.e. based on GSM, GPS, radio clock, mobile phone or watch determination etc.);
**n) Circadian algorithm inputs:** actual sleep monitored sleep/wake cycle
   - subject/patient **actual sleep monitored sleep/wake cycle** (including but not limited to sleep parameter based (i.e. any of or any combination of but not limited to **EEG,** EOG, EMG measures and associated sleep stage or sleep cycle or sleep hypnogram derivations);
**o) Circadian algorithm inputs:** existent known knowledge based on the association between sleep/wake
   - **existent known knowledge based on the association between sleep/wake** and the natural cyclic **circadian** clock and/or sleep cycles and/or core body temperature cycles (i.e. low core temperature on REM sleep etc. ).
   - aggregated knowledge (i.e. per accumulated knowledge and transition of information to knowledge per artificial intelligent or expert system self-learning capability examples in ***Figure 77*** ***knowledge base, real-world inputs, inference engine and workspace*:** ***Figure 78******;*** ***Figure 79******, middle-block***; to ***Figure 80******, [12],[13,[14]*)** based on subject/ patient's association between sleep/wake and the natural cyclic **circadian** clock and/or sleep cycles and/or core body temperature cycles (i.e. low core temperature on REM sleep etc. ).
**p) Circadian algorithm inputs:** "subject wearable or attachable temperature sensors/probes"
   The said **"subject wearable or attachable temperature sensors/probes"** can include any of or any combination one or more the temperature sensors attached or embedded as part of the earbuds/earplugs with combined audio monitoring function described elsewhere in this patent. Moreover the present example can further delineate between external temperature with thermistors positioned to detect current environmental temperature (i.e.one or more temperature sensors incorporated as part of any of or any combination of wearable devices/probes (i.e. watch, mobile phone, earbuds, chest-wall monitor, armband monitor, head, body extremity, body-orifice etc. monitoring).
**q) Circadian algorithm processing options:** delineate between body transient or short term fluctuations
   Additionally, in order to further **delineate between body transient or short term fluctuations** (such as due to physical exercise) and core **circadian** related temperature the CRX analysis can compare and contrast heat-flux measures versus external environment versus core body temperature versus skin temperature measures (i.e. temperature probes just above skin surface can reflect heat flux emitted from skin surface, versus deeper earbud measures being more reflective of body temperature, versus external environment temperature sensors not as closely associated with body temperature changes, for example only).

### Circadian temperature

### Circadian algorithm processing options: wearable health monitoring and/or tracking system/device

- The present example provides a **circadian** function within a **wearable health monitoring and/or tracking system/device,** whereby such function includes any of or any combination of:
   **a) Circadian algorithm:** *monitoring physiological variable(s)* **related to** *natural (inbuilt)* wearable health monitoring, tracking, *time-management factors, determination and*/*or coaching of a subject*/*patient's local time and*/*or current or required routine sleep*/*wake*/*work*/*recreation cycle*
      means of ***monitoring physiological variable(s)*** related to ***natural (inbuilt)*** or associated factors (i.e. sleep/wake) and/or required ***time-management factors*** (external; such as wake, sleep, work, recreation etc. schedules and associated time or performance management demands) influencing said "inbuilt" **circadian** cycle (i.e. **EEG,** temperature, sleep/wake staging or associated cycles; noise, activity etc.),
   **b) Circadian algorithm outputs: *"free-running" or "in-built* circadian *rhythm cycle;*** means of determination of a ***subject*/*patient's "free-running" or "in-built circadian rhythm cycle*** (i.e. via analysis of monitored **circadian** rhythm physiological parameters for an underlying cyclic trend nature;
   **c) Circadian algorithm processing options:** *determination and*/*or coaching of a subject*/*patient's local time and*/*or current and*/*or required and*/*or optimal routine sleep*/*wake*/*work*/*recreation cycle*
      means of ***determination and*/*or coaching of* a *subject*/*patient's local time and*/*or current or required routine sleep*/*wake*/*work*/*recreation cycle*** (i.e. via clock, watch, subject/patient or other accessible or entered information relating to such factors - i.e. alarm clock, time-zone clocks, GPS or GSM locational information, sleep questionnaires/surveys; etc.) ,
   **d) Circadian algorithm processing options: *comparing and*/*or contrasting synchronisation of* a *subject*/*person's natural "in-built" circadian cycle versus environment time zone factors***
      ***means of comparing and*/*or contrasting synchronisation of a subject*/*person's natural "in-built" circadian cycle at any time with the said subject*/*patient's required or current or new environment sleep*/*wake cycle*** requirements or expectations at any time,
   **e) Circadian algorithm processing options:** *analysing the phase difference associated with the said "in-built" natural versus current or required sleep*/*wake cycle time*
      means of ***analysing the phase difference associated with the said "in-built" natural versus current or required sleep*/*wake cycle time*** (i.e. in any of or any combination of tabular or graphic, or user interface, or interactive user interface, vibration or sound or other notification or alert means, via clock or watch-face overlaid or incorporating links or association with **circadian** "inbuilt" clock and/or any other clock or timing information), with in order to produce a measure of ***circadian "inbuilt" versus "required" offset time factor,***
   **f) Circadian algorithm output:** *providing external **circadian** stimulus*
      means of ***providing external stimulus*** (i.e. temperature change of wearable, environmental, bed or bedding material or bed room etc., or light along with associated power and/or colour and/or frequency of lighting,
   **g) Circadian algorithm output:** ***"circadian offset** time factor"*
      means of incorporating measure of ***"circadian offset time factor"*** as part of the biofeedback or control decision matrix of said external stimulus application of subject/patient, in order to minimise said ***"circadian offset time factor"**,*
   **h) Circadian algorithm inputs:** *attached temperature sensors for **circadian** rhythm determination*
      - The present example can incorporate one or more ***attached temperature sensors for circadian rhythm determination*** and/or (but not limited to) other physiological measures with option of plethysmography formats as further detailed in eLifeCHEST/eLifeSCOPE chapter under section "Temperature" or eLifeBUDS chapter, and elsewhere in this document describing temperature monitoring and analyses.

### Circadian EEG

**Forward equation targeted EEG brain region monitoring including circadian clock function** The present example incorporates ***forward equation source localisation*** such as (but not limited to) monitoring and derivation of associated measures relating to the subject/patient's natural ***circadian rhythm,*** including (but not limited to monitoring associated brain regions (i.e. ***suprachiasmatic nucleus (circadian clock) regions***) applicable to ***circadian EEG cycle signals*** (such as for determination of **circadian** clock cycle for subject/patient),
Whereby in one example based on configuration of **EEG** sensor monitoring system (i.e. but not limited to examples per ***Figure 1 to Figure 3******,*** ***Figure 5******,*** ***Figure 14, Figure 16******,*** ***Figure 21******,*** ***Figure 23, Figure 25******,*** ***Figure 27******,*** ***Figure 28******,*** ***Figure 46 to Figure 55***) coupled with the monitoring goals and associated wearable monitoring minimisation computation (***Figure 45***), the present example can compute and determine sensitivity and/or filtering and/or other processing formats (including any of or combinations of NLDBTV, STV, SR, ER, clusters of ERs, clusters of SRs, spectral EOI, interconnectivity of any of same - i.e. coherence and /or Dipole sequences and/or associated clusters, sequences and/or ensembles) and/or neurological amplitude, power, morphological signals or values) in the context of the optimal ***forward equation*** coupled with corresponding neurology channel (or body physiological channels where applicable) in order to most effectively "pin-point" or localise the anatomical sources of interest (i.e. **circadian** clock **EEG** signals in order to contribute to determination of human phasic sleep-wake clock for example, such as could be used as part of therapeutic medication, light treatment or other sleep coaching or recommendation hints to re-along a subject/patient's **circadian** clock or sleep-wake phasic cycle)

### Circadian source localisation based on pre-diagnostic

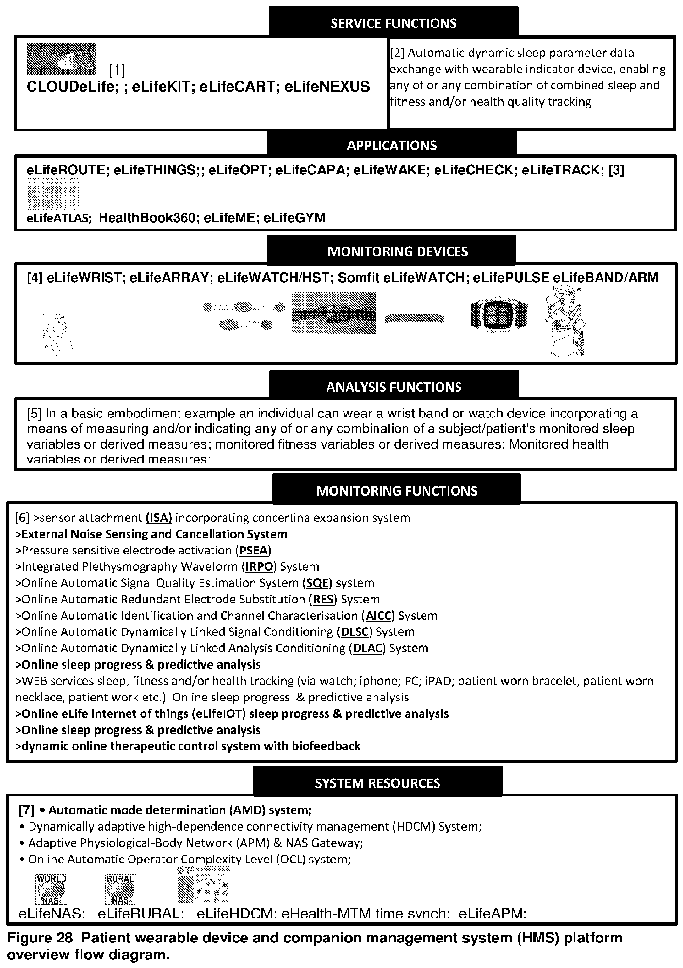

The present example incorporates a means of **EEG** monitoring and ***source localisation.*** The said source localisation comprises of source reconstruction is based on ***pre-diagnostic*** subject/patient studies or general population data as a basis of determining signal source or brain sources of interest. In this manner the spectral and sensitivity characteristics of signal processing applicable to a plurality of sensors (including but not limited to ***Figure 3******; Fiqure 4;*** ***Figure 21******;*** ***Figure 23******;*** ***Figure 46; Figure 47******;*** ***Figure 48******;*** ***Figure 49******;*** ***Figure 50******;*** ***Figure 51******;*** ***Figure 52******;*** ***Figure 53******;*** ***Figure 54******;*** ***Figure 55******;*** ***Figure 56***). For example, based on the modelling of the **EEG** signal attenuation of skin layer, skull and brain matter, combined with the **EEG** electrode positions and specific distances from the targeted brain regions of monitoring interest, each **EEG** sensor can be designated a sensitivity (amplification) and spectral (filtering) properties in accordance to EEF forward source reconstructions where the neural origins are known but the head surface electrode signals can be computed (using forward equation modelling). In this way the present example can provide optimal compensation as it relates to electrode locations designed for subject/patient convenience (i.e. per wearable monitor minimisation formats, (such as in ***Figure 45*** or sensor configurations such as ***Somfit*** forehead sensor per ***Figure 16******;*** ***Figure 28*** [4]).

Using this forward equation source reconstruction analysis approach the present example can determine optimal **EEG** signal processing applicable to sensor monitoring systems specific electrode location (i.e. per ***Somfit***) in order to emulate standardised AASM sleep monitoring manual recommendations (i.e. F4 - M1; C4 - M1; O2 - M2; with backup monitoring electrodes including F3 - M2; C3 - M2; o1 - M2 versus Somfit Fp1, Fp2, F7, F8 and/or Fz sleep monitoring electrode locations).

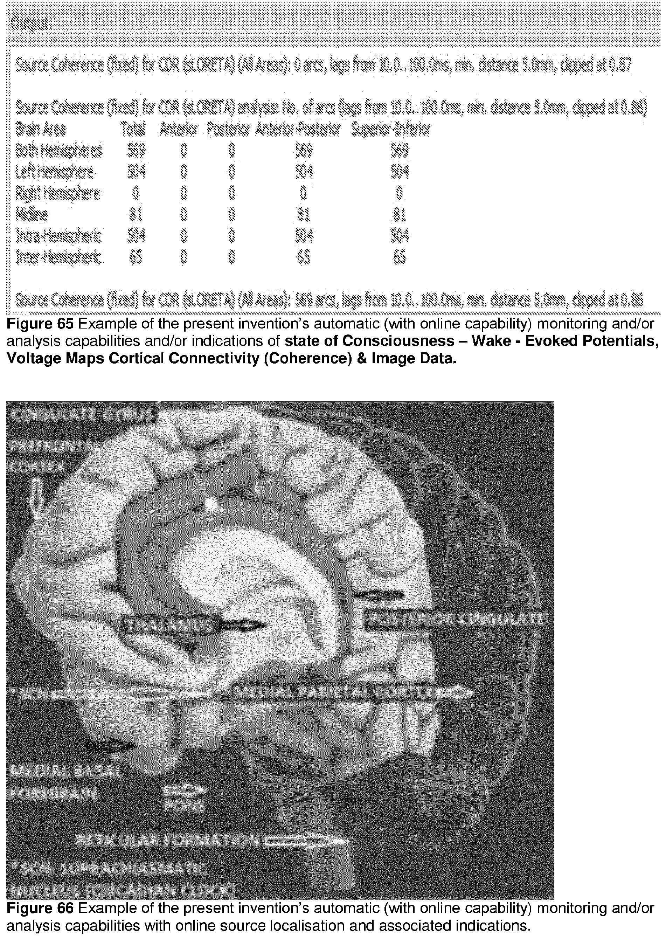

Using this forward equation source reconstruction analysis approach the present example can determine optimal **EEG** signal processing (i.e. adapting frequency, phase and/or amplitude of sensor signals) applicable to sensor monitoring system's specific electrode location (i.e. per ***Somfit***) in order to emulate location of brain regions (i.e. ***suprachiasmatic nucleus (circadian clock) regions***) applicable to ***circadian EEG cycle signals*** (such as for determination of **circadian** clock cycle for subject/patient), and/or consciousness switch region (i.e. thalamus), or other brain regions (i.e. but not limited to ***Figure 66*** ***source localised regions***).

### Circadian clock monitoring and tracking

### Circadian-based sleep deprivation determination

The present example provides a means of monitoring and indicating as part of a wearable or mobile wireless system one or more measures relating to a subject's **circadian** rhythm including the incorporation of a subject's brain or temperature measures, and further comprises any of or any combination of (but not limited to):

### a) Temperature circadian clock measures

>>means of monitoring a subject's temperature and by way or analyses (such as regression analyses but not limited to) determine the 24 hour cyclic nature of a subject's body temperature, whereby shorter term temperature time changes such as those applicable to a subject's motion or exercise (such spontaneous or shorter term measures, for example, can otherwise distort slower changing body temperature trends applicable to **circadian** cycles. Therefore low pass filtering of temperature changes over a 24 hour cycle, coupled with exclusion of temperature measures not fitting within the typical **circadian** cycle can be compensated for in order to derive a subject's underling **circadian** temperature cycle);

### b) Brain/EEG circadian clock measures

>>means of enabling **EEG** linked **circadian** clock synchchronisation determination, whereby means incorporates determination of phasic or rhythmic pulses of signals from the **circadian** clock brain region via 1 or more monitored **EEG** signals, whereby in a first process step the frequency or periodic nature of the **circadian** brain region (i.e. ***suprachiasmatic nucleus (circadian clock) regions***) applicable to ***circadian EEG cycle signals*** such as for determination of **circadian** clock cycle for subject/patient, and/or consciousness switch region (i.e. thalamus), or other brain regions (i.e. but not limited to ***Figure 66*** ***source localised regions***).

Whereby the brain region can be monitored, and/or analysed as a means of computing any measure subject's sleep propensity (i.e. sleep deprivation; sleep delay syndrome; sleep recovery recommendations). Whereby, the present example further enables a means of analysing the periodic nature of **EEG** signals evolving from the human brain or body **circadian** clock. so that only small samples versus continuous or uninterrupted **circadian** clock output signals need to be monitored, in order to accurately compute or estimate the cyclic or phasic position at any time or over any period of the human body or brain **circadian** clock (body clock).

### c) Circadian clock cycle determination and integration with mobile or other map, calendar, messaging, community applications

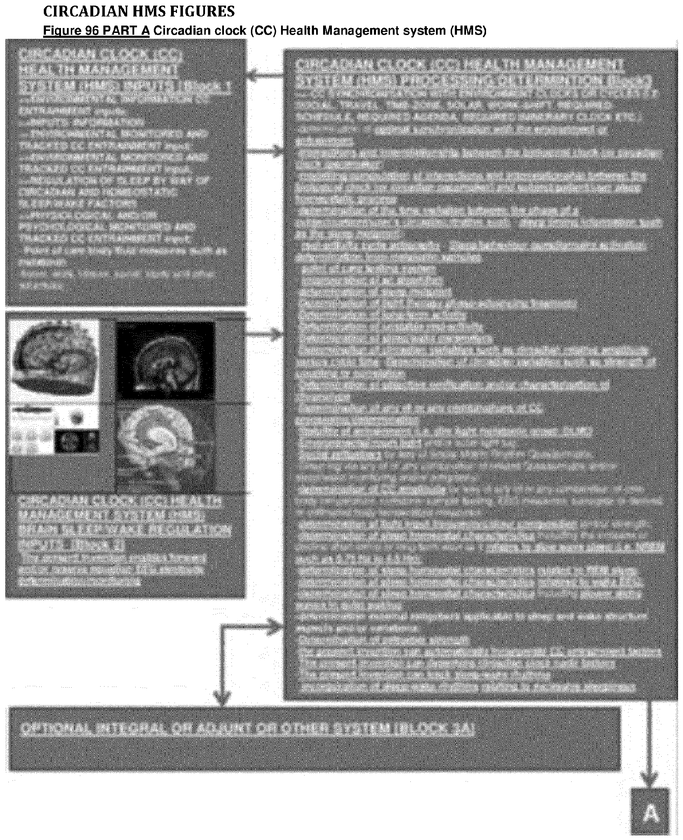

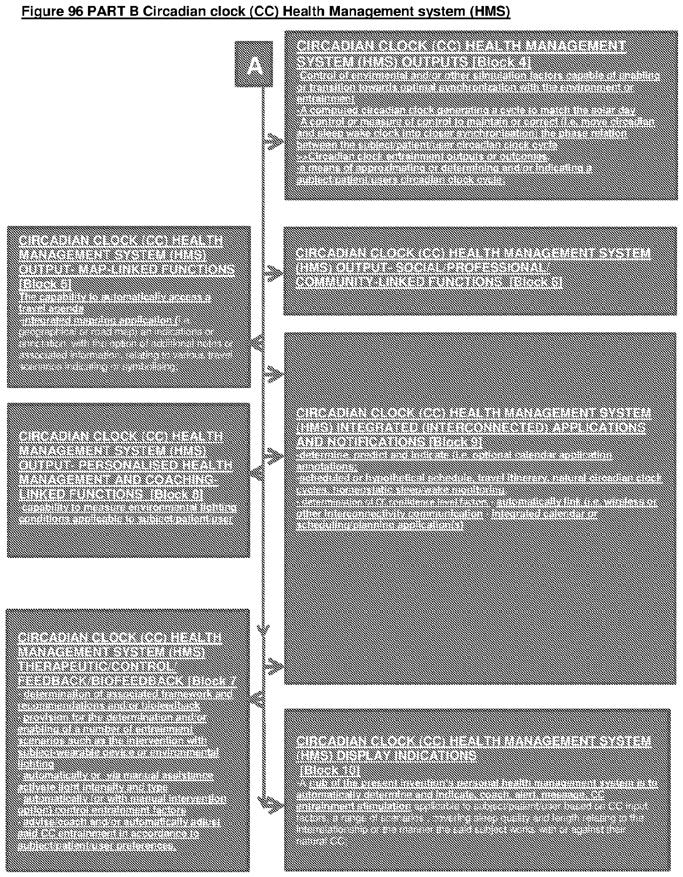

The present example can automatically **determine, predict and indicate offsets** between an individual's natural **circadian** cycle, sleep/wake patters and proposed travel, social, work, recreation. Leisure or other proposed schedule.
i.e. **via calendar application annotations** can incorporate an indication of an individual's **offset** between their natural **circadian** cycle versus their proposed travel agenda and scheduled activities/events. For example, if the individual has to attend a business meeting at a certain time in the future, following a bout of travel, the present example can take into account the time-zone changes, the travel schedule (i.e. automatic linking to travel websites or personally managed flight schedules, or travel agent flight data etc.) and then provide a measure of sleep urge based on a range of assumptions or an individual's data entries or selections or default factors. For example, if an 8 hour flight is followed by an important business meeting, and the assumption is that the individual achieves no sleep on the flight prior to the business meeting, then the present example can estimate the likely sleep urge of the subject in meaningful terms (i.e. based on no or little sleep between departure and scheduled meeting you are likely to have a sleep propensity similar to 3 hours after your normal sleep-time or saying up to 3AM based on your normal sleep/wake cycle.) ***(******Figure 96*** ***output blocks [4] to [10]).***

Similarly the present example allows more complex scenarios to be computed and presented (i.e. map settings or annotations, calendar settings or annotations, clock settings, alarms or annotations) in terms of optimal scheduling of an individual's performance or mood factors versus time efficiency factors, based on the determination of any of or any combination of an individual's a) **scheduled or hypothetical schedule (*****Figure 96*** ***[1]),* b) travel itinerary (*****Figure 96*** ***[1]),* c) natural circadian clock cycles (*****Figure 96*** ***[1])*, d) homeostatic sleep/wake monitoring** (i.e. including automatic access to the routine sleep monitoring capabilities and the associated normal homeostatic sleep factors of an individual, and/or actigraphy, and/or environment light conditions, as covered elsewhere in this document) **(*****Figure 96*** ***[1]),*** e) an individual's preferences in terms of maintaining optimal sleep quality (i.e. taking into consideration previous wake period and the current or prediction such as new environment time-zone adjustment), f) or **entrainment factors** (***Figure 96*** ***[1])*** as the background in terms of time-zone shifts and various adjustment periods etc.), g) individual preferences in terms of Jet-lag minimisation versus social clock factors versus work-clock factors, individual **circadian** clock factors (phase delay, phase advance, delay stability and **confidence level in terms of the accuracy of the determination of the individual's previous and up-to-date circadian clock cycle status, along with other factors impacting the determination of current circadian clock status of an individual) (*****Figure 96*** ***[3])***;

The present example enables all these functions and capabilities to be incorporated into one or more wearable or mobile devices (i.e. smartwatch, mobile phone, Somfit sleep monitoring headband and/or other covered elsewhere at any section within this patent application document such as (but not limited to) wearable device examples presented in ***Figure 1*****;**

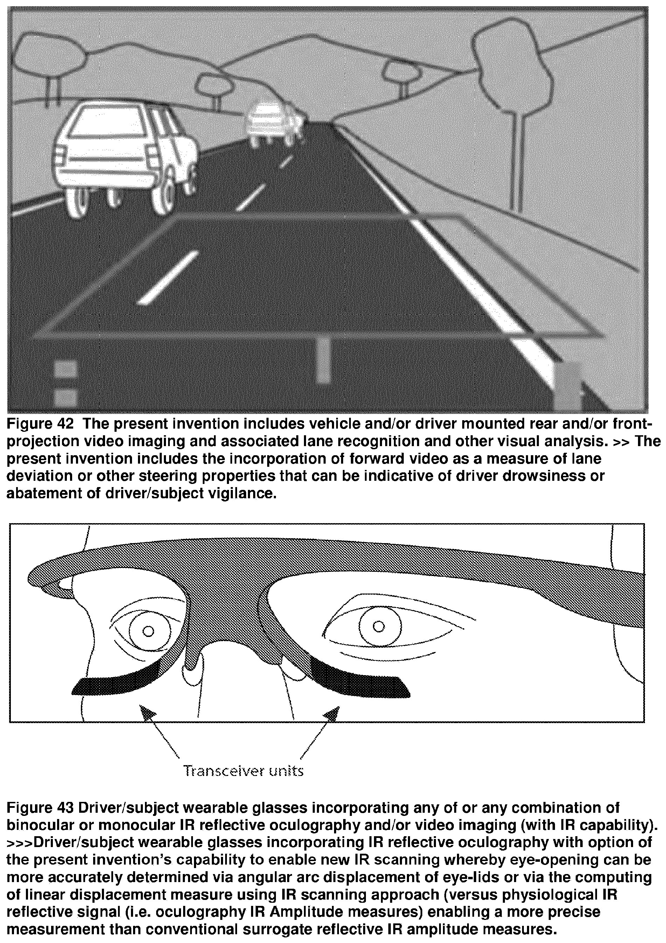

In another example an individual (i.e. traveller) incorporation of CC computed parameters as part of automatic **entrainment (i.e. per** ***Figure 96*** ***[1],[7]***) **programming of CC treatment systems** (i.e. such as **bright light therapy including glasses or sunglasses** (i.e. half-shielded glasses, for example only, tinted in upper section of glass lenses only), **whereby said glasses can include reflective oculography** (i.e. per ***Figure 43***) **capable of both entrainment light-therapy** and/or detection of **eye-lid movements** and/or opening as a **marker of drowsiness** in order to enable biofeedback entrainment capabilities in order to adjust for CC cycle **offset** factors and/or sleep propensity and/or sleep urge factors. For example, the present example can incorporate a series of blue LEDS or other blue lighting arrangement, which can be controlled automatically via wireless interconnect to provide an individual's selected entrainment treatment regime designed to adjust for **circadian** phase advancement or delay based on the computation of the individual's current **circadian** clock versus social, travel, time-zone and or work or recreation schedule/clock requirements **(i.e. per** ***Figure 96*** ***[1]*);**

Similarly the present example can **automatically link** (i.e. wireless or other interconnectivity communication and information access means) **to messaging systems** (such as mobile phone SMS, emails, calendar, applications and the like) in **order to track and/or comment/health-coach and/or enable sleep scheduling** as it relates to an individual's current **circadian** clock versus social, travel, time-zone and or work or recreation schedule/clock requirements, in terms of optimal performance, energy, sleep-urge, occupational-health sleepiness risks, fatigue implications and **other factors an individual may be interested in activating *(i.e. per*** ***Figure 96*** ***[7]).***

One example enables **integrated mapping applications (i.e. geographical or road map) or related indications or annotation, to incorporate additional notes or associated information, relating to various travel scenarios indicating or symbolising** (i.e. single sine-wave cycle with normal sleep periods marked versus new environment time-clock with respectively positioned such as equivalent start and end times corresponding to new time zone environment) along with indication of CC phase lag or lead factors so that an individual can associate travel plans along with associated travel itinerary with CC and jet-lag factors.

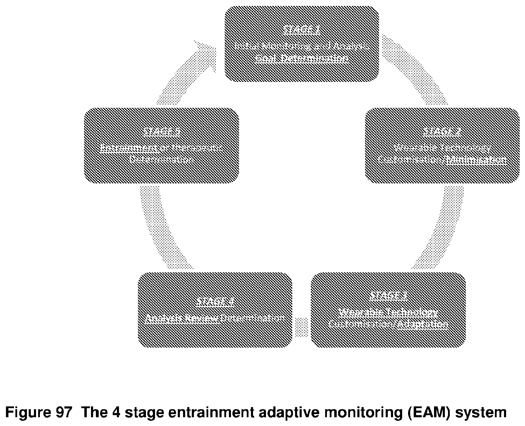

Additionally, CC entrainment suggestions/health-coaching along with projected or estimated sleep propensity factor or sleep quality aspects (i.e. difficulty based on phase relationship of CC's prior wake period and homeostatic sleep patterns. In this way an individual can visually, automatically, instantly and in an integrated or seamless (i.e. mobile personal planning applications **including mapping or routing related functions or applications or processes,** wearable devices, etc.) associate travel plans with the related health management impact and precautions or countermeasures to optimise an individual sleep-quality, sleep duration, sleep-timing, daytime energy, sleep-propensity, mood and other elements which clearly can be managed and enhanced in terms of information access, understanding and control **via the circadian health management system *(i.e. per*** ***Figure 96*** ***block [3] health management system with associated inputs [1] and[2], adjunct system options [3A], and outputs [4] to [10];*** ***Figure 97******, 4-stage entrainment adaptive monitoring system).***

The present example enables **integrated calendar or scheduling/planning application(s) (i.e. geographical or road map) an indications or annotation, with the option of additional notes or associated information, relating to various travel scenarios indicating or symbolising** (i.e. single sine-wave cycle with normal sleep periods marked versus new environment time-clock with respectively positioned (i.e. equivalent start and end times corresponding to new time zone environment) along with indication of current individuals CC inbuilt, along with various (i.e. different travel itinerary or different schedules for travel, social events, work events, study, etc.) CC phase-lead, CC phase-lag or adjustment/entrainment (i.e. melatonin dosage and dosage timing and/or bright-light dosage and timing of therapy etc.) strategies to align these conflicting clock cycles (i.e. inbuilt CC versus working against social-clock (i.e. based on natural CC wake/sleep requirements versus conflicting clock time-cycle requirements) versus attempting to synchronous CC phase-lead or phase-lag factors. In this way an individual can visually, automatically, instantly and in an integrated or seamless (i.e. **integrated calendar or planning or scheduling related applications or applications or processes,** mobile personal planning applications, wearable devices, etc.) associate travel plans with the related health management impact and precautions or countermeasures to optimise an individual sleep-quality, sleep duration, sleep-timing, daytime energy, sleep-propensity, mood and other elements which clearly can be managed and enhanced in terms of information access, understanding and control **via the circadian health management system *(i.e. per*** ***Figure 96*** ***block [3] health management system with associated inputs [1] and[2], adjunct system options [3A], and outputs [4] to [10];*** ***Figure 97*** ***4*** ***stage entrainment adaptive monitoring system).***

The present example enables watch or clock application to be programmed so that information relating to the CC clock cycle and/or individual's required scheduled clock requirements (i.e. travel-clock, time-zone changes, social-clock, work-clock, leisure-clock, special-event clock) along with strategies or scenarios capable of providing trip programs or sequences designed to minimise the disruption of quality sleep or an individual performance in work, sport, play, leisure etc. can be structured and stored in a library or easily recallable such each or clock display face setups, along with various CC examples, entrainments, strategies, personalised preferences, planning capabilities and the like, in order to enable user to establish an ideal library of personalise configurations for CC HMS ***(i.e. per*** ***Figure 96*** ***block [3] health management system with associated inputs [1] and[2], adjunct system options [3A], and outputs [4] to [10];*** ***Figure 97*** ***4*** ***stage entrainment adaptive monitoring system).***

***Additionally the CC HMS enables a community or private select grouping function whereby healthcare specialists can coach, guide, assist and intervene in tracking, diagnosing and supporting an individual's occupational safety aspects, sports performance aspects, general health aspects, depression and other psychological disorders greatly impacted by appropriate CC management (i.e. per*** ***Figure* 96** ***block [3] health management system with associated inputs [1] and[2], adjunct system options [3A], and outputs [4] to [10];*** ***Figure 97*** ***4*** ***stage entrainment adaptive monitoring system).***

Additionally, the present example can **automatically link** (i.e. wireless or other interconnectivity communication and information access means) **to an individual time reference** (i.e. alarm clock, watch, mobile phone clock or other application) in **order to automatically set or suggest alarm settings and/or enable sleep scheduling track and/or comment/health-coach** as it relates to an individual's current **circadian** clock versus social, travel, time-zone and or work or recreation schedule/clock requirements, in terms of optimal performance, energy, sleep-urge, occupational-health sleepiness risks, fatigue implications and **other factors an individual may be interested in activating.**

For example, one said projected or predicted scenario could be based on the assumption that subject/patient continues their **sleep patterns or behaviour/quality** (i.e. time of sleep, sleep time , sleep fragmentation, sleep architecture, sleep arousals, sleep disturbance, respiratory disturbance, REM sleep amount and structure, deep sleep amount and structure) unabated, or with various degrees of correction such as ***mild correction*** (i.e. gradual increase in sleep quality or decrease in sleep deficit), ***moderate correction or severe correction*** (i.e. ***any of or any combination of medication,*** light-therapy, sleep hygiene or environmental improvements (i.e. reduce external arousals related to audible noise, temperature, humidity, air pollution or other breathing or asthmas antagonists etc.).

Additionally, the present example provides a **"means of computing circadian cycles"** based on projecting or predicting a sleep-**circadian**-cycle scenario based on assumption that the subject/patient reduces or compromises their sleep for a period (i.e. period of study or to allow for a special event, travel etc.). Said means can also provide a typical measure of sleepiness or propensity based on comparative circumstances (i.e. you will be at extreme risk of falling asleep within 10 seconds (i.e. period of say 1 second to 30 seconds subject to specific scenario) of shutting eyes and you should not drive, undertake decisions of financially material nature, undertake any task where occupational work risks to yourself or others are relevant etc. Additionally, (for example only) the predicted sleep deprivation scenario could be related to other equivalent reaction time or alertness or sleepiness measures such as the predicted equivalent Epworth sleepiness scale outcomes or blood-alcohol readings etc.
- Whereby "means of computing **circadian** cycles" including for example, but not limited to, examining **information of prior monitored circadian physiological variables** (i.e. temperature, **EEG** suprachiasmatic brain region, etc.) and also examining such variables along with other factors influencing the shift of the **circadian** clock cycle (i.e. **sleep parameters** and associated sleep measures of subject/patient) in order to determine, based on historical or prior changes versus consequential sleep and **circadian** clock shift measures. i.e. per algorithm subject/patient-specific learning (ASL) system or wearable-device minimisation (WM) system the subject/patient's prior and/or current and/or predicted (with indicated scenarios) and/or training (i.e. to improve or optimise sleep management based on circumstances and/or scenarios and/or assumptions and or users selection of various desired sleep quality versus work/play schedule preferences - i.e. per ***Figure 45*** ***where this example demonstrates*** the capability to examine the goals of monitoring **(*****Figure 45*** ***[1]***) and then in this context advise user of the more complex (***Figure 45*** ***[2]*)** monitoring configuration(s) (subject to clinical oversight or requirements or considerations consumer or GP oversight guidance, for example), followed by enabling a monitoring, analysis, determination of outcomes and then statistical assessment of accuracy of outcomes diagnosis or prognosis, in order to determine reconfiguration requirements and related wearable monitoring adaptation for both monitoring sensor configuration(s) or wearable monitoring sensor(s) and related devices **(*****Figure 45*** ***10******,*** ***11, 12*** ***- not in the context of circadian*** ***monitoring the same principles apply* as *neurology minimisation except that the broad range of physiological parameters and related wearable monitoring configurations are taken into account in order to cover relevant monitoring measures including any of (but not limited to) direct or surrogate measures of body temperature monitoring, homeostatic sleep measures, and*/*or daytime activity measures )*** but also the minimal or streamlined/minimalized **(*****Figure 45*** **[8], [9], [10])** sensor and wearable configurations based on achieving relevant and appropriate accuracy of diagnostic and/or prognostic aims and outcomes, applicable to the specific or personalised requirements of user and user's health community (as personalised based on user's privacy, security and preferences or allowance of opt in capabilities and intervention).

### d) Inbuilt (natural) circadian clock

- the present example provides ***natural* circadian *clock device means* and/or *application-linked means* and/or application-linked means of characterising, determining, and/or accommodating a subject/patient's natural circadian rhythm;**

Whereby said ***circadian clock*** characterising can comprise of monitoring slow changing, or typically 24 hour cycles with typical sleep and wake phases of cycle, of a subject/patient's temperature, **EEG** associated with **circadian** clock (i.e. associated or directly implicated with ***suprachiasmatic nucleus (circadian clock) brain regions, per*** ***Figure 66***), as further detailed elsewhere in this document;

Whereby said ***natural circadian clock device means* or *application-linked means*** includes (but is not limited to) one or more ***time management systems*** and/or ***sleep*/*wake management systems***, along with the interrelationship between such aspects;

Whereby said **natural circadian clock device and application-linked *time management system means*** includes (but is not limited to) any of or any combination of:
- alarm clock settings, mobile phone clock settings, map applications, calendar applications, any scheduling applications, any project management applications, any travel planning applications, wrist watch settings, computing devices, online applications, social media applications, social network applications or other systems applicable to an individual's sleep/wake management;

Whereby said **natural circadian clock device or application-linked *sleep*/*wake therapy health management system* means** includes (but is not limited to) any of or any combination of:
- health or personal planning applications, any occupational health management planning applications, any health insurance occupational risk and health applications, 3D or other glasses, lighting systems, curtain control systems, room temperature or environmental control systems, subject glasses with light therapy function (i.e. to enable light therapy for adjustment of sleep delay or sleep quality), sleep/wake coaching, sleep/wake recommendations, sleep/wake alarm clock or subject/patient clock recommendations or hints or other systems applicable to adapting, adjusting therapeutic intervention of an individual's sleep/wake planning or intervention of timing schedules or cycles (including natural **circadian** clock aspects);

Whereby the present example can enable an individual to adjust and/or optimise ***time management systems*** and/or ***sleep*/*wake management systems,*** along with the interrelationship between such aspects in accordance to optimising an individual health, including (but not limited to) adjustment for sleep delay syndrome, undesirable or unsafe sleep propensity or sleep-urges and/or adjusting and/or accommodating a subject/patient/user's natural **circadian** clock;

Whereby the present example can include a computer system programmed with steps comprising of a decision matrix incorporating adjustable properties relating to **natural circadian clock device or application-linked means** associated with ***time management systems*** and/or ***sleep*/*wake management systems, along with interrelating factors***;

### e) Further circadian clock aspects

The present example further enables
i) **natural circadian clock cycle parameters;**
ii) **users essential awakening time parameters** (i.e. work, study, travel commitments etc.);
iii) **calendar, travel and other schedule applications with associated commitments or planning details;**
iv) **travel itinerary long with automatic world time-zone computational implications** in terms of association, comparison and contrast with other time-related (i.e. per a) to c) herein;
v) **map applications in terms of world travel routes** or routing as a mean of planning travel routes to optimise adjustment or readjustment of a subject/patient/user's natural (**circadian**) time clock in order to adapt back to a timing structure capable of reducing or most appropriately recovering sleep deprivation over a defined (i.e. user preference or essential needs) period of time;
vi) **messaging or other mobile phone, watch or other user** (or other designated or authorised location or party - i.e. enabling a transportation driver/pilot or shift-worker or shift-worker safety carer to **better manage occupational vigilance or safety or risk day-time sleep deprivation/propensity and real-world solutions or coaching assistance, for example);**
vii) **Clock alert or alarm system** - i.e. alarm clock setting, mobile phone clock settings, wrist watch settings, application forming part of existing mobile phone, alarm clocks or other devices settings and functionality;
viii) **Personal rhythm or clock health management system** - i.e. the ability to enable subject/patient/user to adjust timing indicators and/or alarm clock functions and/or calendar planning coaching or hints and/or messaging system coaching or hints in accordance to optimising schedules or adaptation/adjustment (i.e. **recovery from or avoidance of more adverse sleep deprivation, for example**) of personal **circadian** rhythm in accordance to **mild, moderate, significant, or severe realignment to optimal sleep wake cycle** (i.e. whereby user can configure their optimal sleep/wake cycle for the immediate sleep/wake periods or any future period, and thereby enabling adjustment from sleep deprivation or unconventional (i.e. departure from standard sleep/wake cycle times due to travel, recreation activities, work demands, exam studies, jet-lag, night-shirt etc.);
ix) health or sleep coaching application capable of advising, adjusting or controlling a range of devices or systems (i.e. internet of things or medical internet of things or other wire, wireless means of interconnecting systems) subject/patient/user different options or programs of clock and timing schedules (i.e. via calendar appointments and alarm clock awakenings, along with option of adjusting room temperature, room lighting or associated lighting adjustments and other sleep/wake environmental influences);

### f) Forward equation source analysis

>>>> the present example provides capability of **forward equation source analysis** based on the know
n typical **circadian** clock brain anatomical ***location or other brain functional or structural locations*** such as the ***suprachiasmatic region (circadian clock) and*/*or associated control or interconnectivity regions*** of the brain (i.e. as well as options for (but not limited to locations outlined in ***Figure 66*****, *Talairach Atlas;*** Current atlas tools; Harvard Whole Brain Atlas, MNI Template, The standard template of SPM and International Consortium for Brain Mapping; Atlas of the Developing Human Brain; All Functional including Brodmann areas, Gyri, Sulci etc.
   - where the present example can provide compensation for electrode location based on compensating signals in accordance to the amplification or attenuation, filtering, phase adjustment and any other factors in order to simulate or emulate the conditions applicable to targeting the monitoring of ***location or other brain functional or structural locations*** any of or any combination of brain regions or connectivity aspects (i.e. coherence or Dipole measures); For example, in one of the simplest modelling scenarios the present example can:
      - assume ***in the first step*** that all **EEG** electrodes are presented with exactly the same signal,
      - ***in a second step*** define a ***location or other brain functional or structural locations*** of interest:
      - ***in a third step*** determine the likely attenuation, spectral filtering characteristics, and phase shifts most probable (i.e. based on skin, skull, brain matter attenuation factors applicable between each electrode and the brain region of interest);
      - ***in a fourth step*** apply the determined attenuation, spectral filtering characteristics, and phase shifts characteristics in term of signal processing for each respective **EEG** electrode;
      - ***in a fifth step*** - the determined attenuation, spectral filtering characteristics, and phase shifts characteristics can be further improved by way of contrasting and comparing with actual subject/patient imaging and diagnostic assessment data;

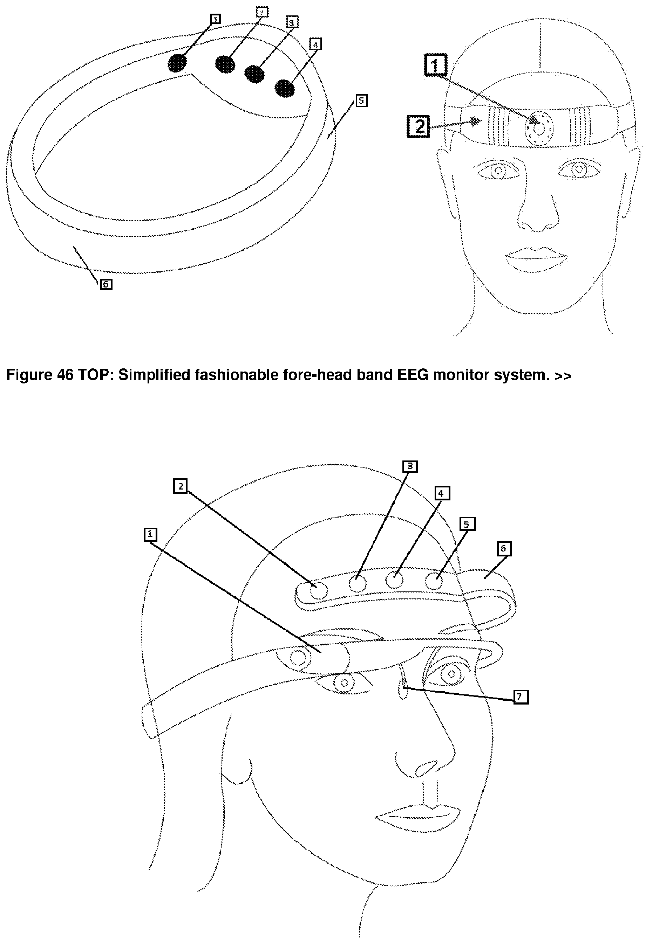

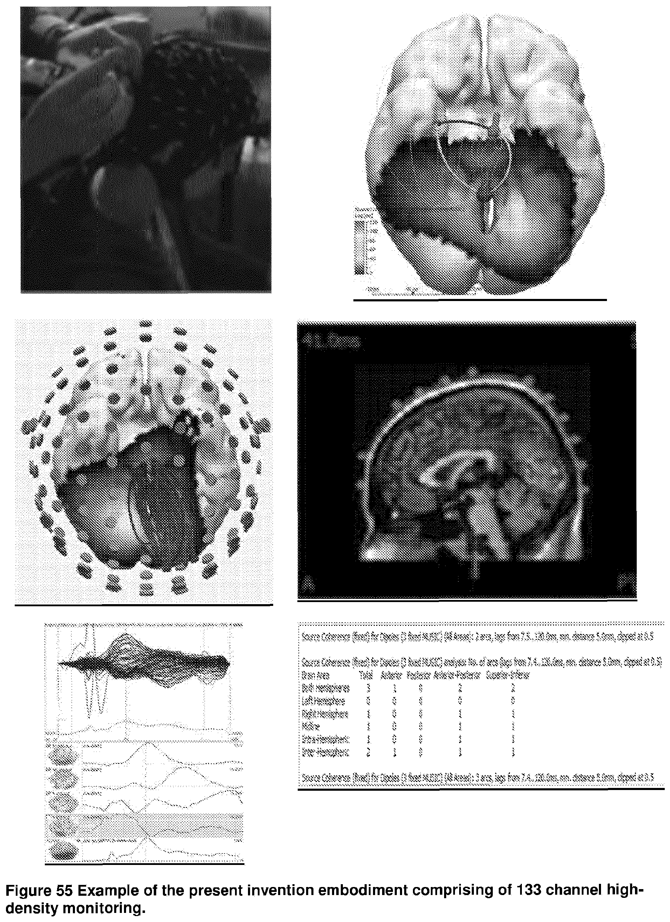

In this way forward equation source modelling enables specifically targeted regions of the brain to be monitored or analysed even with minimised wearable monitoring sensor systems (per example process in ***Figure 45*** ***demonstrating example of minimisation of sensors capable of targeting brain regions of interest with minimal possible sensors or electrodes***). Moreover, more complex monitoring sensor systems (per example ***Figure 55***) can still be streamlined or minimised (per ***Figure 46*** to ***Figure 54*** ***providing example (but not limited to) different wearable monitoring formats ranging from the simplest consumer-level monitoring options to more complex clinical diagnostic options)*** with improved source localisation capabilities using these ***forward analysis modelling*** and ***minimisation processes.***

### g) Intelligent circadian-based, clock, alarm, scheduling, coaching, biofeedback, control system)

> the present example provides ***intelligent circadian-based measures with interlinking to clock, alarm, scheduling, coaching, biofeedback, and associate control system*** whereby intelligent wrist or alarm clock settings can include any of or any combination of (but not limited to):
   a) actual subject/patient's **circadian** clock-based alarm settings or clock time indicator capabilities;,
   b) conventional (i.e. time-zone adjusted) time or alarm indications);
   c) sleep coaching recommendations whereby a subject's sleep/wake behaviour modification recommendations in terms of, for example, adjusting sleep cycle to compensate or minimise time phase sift between a subject/patient's current natural **circadian** clock cycle and a subject/patient's desired sleep/wake phasic requirements (i.e. adjusting for jet-lag, night-shift, late study or recreational nights and the like);
   d) interlinking (wireless communication, for example) with other devices applicable to adjusting or compensating or indicating ((i.e. displaying, comparing or contrasting between **circadian** phase and cyclic nature versus an individual's required or desired sleep/wake cycle) an individual's sleep cycle and or **circadian** cycle and such as, but not limited to light therapy (i.e. activation or adjustment of conventional room lighting or special (i.e. any power, brightness, lighting colour, light frequency radiation spectrum etc.) lighting; mobile device or wearable device interface (i.e. smart watch or mobile phone or intelligent alarm clock etc.); room temperature or electric blanket and other environmental controls that can also impact and contribute to adjustment or correction of **circadian** rhythm;
   e) the present example provides a means of administering, coaching or medication determination, along with online automatic or assisted (including regulatory, security, privacy and personal authorisation and access requirements) medication ordering based on any of or any combination of (but not limited to):
      i) schedule of medication,
      ii) online access for ordering dosage,
      iii) determination of dosage based on any of sleep/wake monitoring outcomes, subject/patient medical information, subject/patient personalised **circadian** rhythm, subject/patient sleep/wake monitoring outcomes, subject/patient HMS outcomes or related information, subject/patient health survey and other health information records, subject/patient natural **circadian** versus ongoing sleep/wake schedule requirements, subject/patient **circadian** monitoring outcomes (i.e. natural **circadian** rhythm based on **EEG circadian** clock, temperature and other **circadian** physiological monitored measures and associated analysis or derived outcomes);
      iv) dosage and medication or ***medication-dispenser*** or medication-coaching (i.e. guidance on pharmaceutical such as recommended dosage and dosage schedule automatically programmed and/or control linked with an automated medication dispenser system (i.e. programmed to best compensate shifts between natural **circadian** rhythm/clock and new environment (i.e. change in time-zone or day-night activities) or sleep/wake/work requirements);
      v) user interface to clock or alarm system (i.e. smart watch, alarm clock or mobile phone application that can enable user to select between optimal **circadian** rhythm synchronised time indication (i.e. for most natural alarm setting or calendar scheduling etc.) versus actual world time-zone based clock, alarm or scheduling means).

### h) Wearable mobile health tracking and indicator means

- the present example provides a means (i.e. means including integrating into a smart watch device any measures or combination of measures including but not limited to temperature sensing of subject, galvanic skin resistance of subject, photo-plethysmography, ECG, oximetry or plethysmography oximetry measures, all of which have a very slow underlying **circadian** cycle component which can be derived using any such measures, with further option of incorporating other wearable or mobile wireless monitoring systems local/distributed analyses outlined elsewhere in this document) of monitoring, computing and tracking, indicating a subject's/patient's **circadian** rhythm.

Such means can also include a means to display any of or any combination of conventional 24 hour time clock and/or date/and or calendar and/or sleep schedule means (i.e. such means can include calendar, messaging, clock display, or other means of indicating any of or any combination of actual subject/patient's actual **circadian** cycle, one or more predicted or projected **circadian** cycles, inclusion of the indication of normal variance or the range of sleep deficit and/or surplus that can be accommodated without mild, moderate or severe consequences (moreover this information can be based on comparative population data bases or aggregated subject/patient-specific monitored or other input information (i.e. per artificial intelligence or expert system analysis methods per ***Figure 77*** **[A]** whereby a **Knowledge Base** is accumulated based on the evaluation of inputs from the **Interface to the Real World [8]** and the associated of the rule interpreter which is based on standardised scoring rules applicable to disease, disorders or ***health conditions of interest.***

The said ***"health conditions of interest"*** can include (for example but not limited to) sleep disorders this can refer to the American Academy of Sleep Medicine rules for staging sleep scoring sleep-related respiratory disorders **(i.e. per section eLifeCHEST/eLifeSCOPE section titled** " **Monitoring, Determination and Tracking of Sleep, Wake and other Mental States, Events of Interest or Health Conditions of Interest"),** epilepsy prognosis or diagnosis including detection of events or clusters of events such as HFOs, ripples, spikes, waves, K-complexes or spindles as further details in ***Figure 75*** **[*29]* Automatic analysis model Determination.**

The said ***"health conditions of interest"*** can include (for example but not limited to) **the** diagnosis or prognosis of Parkinson's, whereby the determination of the said The said ***"health conditions of interest"*** can include (for example but not limited to) monitoring and analysis identification of biomarkers and related "expert system rules" (i.e. per ***Figure 77*** ***[D]***) associated with analysis of events of interest (i.e. symptoms corresponding to diagnosis or prognosis or disorders, health conditions or diseases of interest). These said ***"expert system rules"*** are described elsewhere, including sleep behaviour disorder combined with movement (i.e. motion or activity characteristics) as further covered under prognostic and/or diagnostic movement disorder marker monitoring and analysis associated with Parkinson's or other movement, muscle or nervous system disorders markers (i.e. per vibration or uncontrolled, jerky movements, symmetry of movement, flow or movement, synchronisation of movement between two or more body parts or as further **details in section eLifeCHEST/eLifeSCOPE section including (but not limited to) section titled** "Gait or movement tracking and characterisation of events of interest" (with automatic analysis option)combined with (or assessed as independent factors) the monitoring and analysis or per example (but not limited to) ***Figure 75*** **[21], [24] and associated features enabling the determination of** sleep behaviour disorder (with automatic analysis option) as a diagnostic or prognostic marker of Parkinson's. Similarly, these artificial intelligent or expert system analysis processes can be deployed in order to automatically analyse **circadian offset** factors (**i.e. but not limited to section headed Somfit including (but not limited to) section titled "CIRCADIAN** TEMPERATURE CYCLE DETERMINATION VERSUS LOCAL ENVIRONMENT AND/OR SLEEP/WAKE/ACTIVITY/WORK".

The said ***"health conditions of interest"*** can include (for example but not limited to) **the** diagnosis or prognosis of other neural disorders such as ASD as further described in section headed "PATENT TITLE: DIMENTIA /ALZHEIMER'S /ASD /ASP" as well as combined measures covered in section headed "eLifeALERT".

The said *"****health conditions of interest"*** can include (for example but not limited to) **the** diagnosis or prognosis of other neural disorders such as ASD as further described in section headed "eLifeALERT".

Additionally, as part of the input factors to the expert system or artificial intelligence process **concordance analysis** (i.e. means of assessing and rating the accuracy of the expert or artificial system analysis accuracy, compared to comparative expert analysis outcomes in order to both quality control and also continually improve the prognostic and diagnostic analysis outcomes in terms of validation with professional medical or scientific experts at all times and ultimately provide the highest quality medical and health tracking for subject/user) can be automatically deployed in order to validate and where required continually modify and improve the interpreter rules ***Figure 77*** **[D]** to enhance the accuracy of the Knowledge Base [A] and ultimately the quality of the subject/patient's diagnosis or prognosis ***Figure 78*** ***[2]; [5].***

The present example can incorporate **minimisation** rules as part of the system's artificial intelligence or expert system self-learning capability so that based on the **broad-scoped** or **narrow-scope monitoring criteria** or **monitoring goals** the present example can both continually validate and improve the monitoring algorithms personalised or specific to the subject/patient as well as streamline and **adapt the most appropriate** and minimalized wearable monitoring sensors and associated **prognostic and diagnostic analyses,** once again tailored specific to the patient/subject. The said artificial analysis or expert system analysis self-learning capabilities can be part of one or more mobile and/or wearable monitoring system or supplemented by interconnecting software network or associated services or resources (i.e. SAAS including Cloud-computing services , LAN, WAN, peer to peer, WWW, NAS etc.).

Additionally as outlined in ***Figure 78*** inputs from experts (including diagnostic review or expert oversight observations per ***Figure 78*** blocks **[1]),** patient survey or monitoring data **[2],** can be examined within prognostic ***Figure 78*** **[2]** or diagnostic ***Figure 78*** **[5]** modules, for example, in the context of artificial intelligence or expert analysis (as outlined in ***Figure 77*** as described above.

In one example, conventional clock face time keeping can be supplemented with a series of **circadian** clock overlays indicating subject/patient's actual **circadian** cycle status and/or one or more projected **circadian** sleep cycle scenarios and/or interlinking with local smart watch and/or interlinking with a remote (i.e. wireless or mobile wireless linked alarm clock application designed to enable recommended sleep alarm recommendations for minimal to maximal sleep quality and/or minimal to maximal work scheduling and/or any compromise or balanced position. Additionally, one or more subject/ patient's room or house lights and/or wireless linked lights can be controlled in the context of light therapy to help adjust or optimise a subject/patent's **circadian** cycle.

### i) Determination of optimal circadian sleep cycle

The present example **enables current or new environment** (i.e. world cock time zone) **time, along with current sleep/wake** (and option of comparison with work and/or recreation time cycles - i.e. via calendar and/or clock settings of subject/patient/user's mobile device and/or sleep/wake monitoring) as well comparison and contrasting analysis with normal rhythm or typical cycle or phasic nature of subject/patient/user individual during healthy or peak sleep performance or health condition (i.e. via ESS or other sleep propensity or sleep scale performance, sleep/wake studies, sleep study outcomes indicating normal or healthy sleep propensity or daytime performance of an individual);
>>> indicator means such as watch face (i.e. shows clock time of body versus actual clock and/or phase lag or gain and/or recommendation or coaching go catch up with sleep or potential amount to sacrifice sleep without excessive adverse consequences (i.e. modest or minor sleep deprivation/sleep propensity) , mobile, alarm clock - linkage, alarm clock etc.
>>in a manner whereby the periodic nature of the **circadian** clock of the human body can be determined and the elapsed cycle or time of the human **circadian** clock can be computed and compared to the typical **circadian** clock cycle for the particular subject/patient or the typical normalised population example of **circadian** clock cycle in a health individual.
>> interlinking to lighting/glasses/pharmaceutical timing and dose recommendations or administration in order to correct or **realign natural body clock (circadian) versus desired clock** and scheduling requirements;
>>The present example can incorporate into a ***triaged health-watch, or scheduling* /*activity*/*calendar application*** or shared social or business media information to enable on a personal or community/group level explicit health data or coaching and/or associated treatment (i.e. light therapy **circadian** clock adjustment entrainment linked to internet of thins or various lights and other associated controls) in order to assist with the management of crucial sleep stages such as deep-sleep (body recovery) or REM sleep (brain restoration), **circadian** clock alignment (i.e. ***offset and management of healthy realignment between homeostatic sleep*/*wake stages*/*stages' local versus travel clocks, work schedules, leisure schedules, relaxation schedules, of a subject and conflicting or other social or business or travel agendas*/*itineraries***) whereby user/subject can preference or weighted in order to ***influence and*/*or impact and*/*or modify and*/*or adapt*** upon the on scheduling or alarm clock setting or ***calendar functions and availability*** can be segmented such as colour coded in accordance to impact various ***schedule or commitment will have on homeostatic or* circadian *sleep alignment, sleep-debt, sleep-urge and other sleep-health factors***) the importance from their private or personalised perspective in terms of sleep propensity or sleep urge risks, sleep debt factors, sleep urge factors, work time priority, work productivity or performance factors, leisure time or leisure enjoyment, sleep time, quality sleep and other sleep, wake, **circadian**-clock, sleep health and general health associated factors. For example, in one example a mobile wireless personalised mobile device or ***wearable devices (i.e. watch, phone, computer etc.)*** can incorporate a watch function with ability of user to toggle through related watch-face or scheduling modes applicable to any of or any combination of ***homeostatic sleep*/*wake stages*/*stages' local versus travel clocks, work schedules, leisure schedules, relaxation schedules, of a subject and conflicting or other social or business or travel agendas*/*itineraries.*** Moreover, the present example can coach, guide and recommend calendar entries, activity scheduling, clock alerts or alarms, shift-work schedules or sleep/wake-planning in accordance to the personalised preference of ***work-productivity and*/*or focus*/*attention*** (i.e. work-mode), personalised occupational work hazard and risk factors (i.e. incorporates a means of monitoring, analysing, correlating and alerting of health conditions such as excessive sleep/wake disruptions including crucial aspects of quality sleep such as amount of REM sleep, amount of deep-sleep, and also the **circadian**-clock **offset** factors ***versus*** (for example only) ***population normative requirements along with personalised requirements*** etc. versus ***also the level or risk or responsibility of job* -*i.e. truck driving with indications of snoring or OSA sleep disruptions could be indicated on a personalised and personal safety management level to allow private information but potentially critical personal health guidance and support***) and ***cognitive performance*** (i.e. mind-mode), leisure time (fun without consideration of sleep deprivation or fun with various degrees of sleep deprivation or sleep-urge consideration or weighting factors in terms of the associated fitness or training coaching, sleep training or coaching, work schedules or coaching, leisure schedules or coaching and/or relaxation schedules or coaching

### j) Sleep survey and artificial intelligence

>> the present example enables a means of self-assessment of a subject/patient via sleep or other health surveys (i.e. but not limited to ***Epworth sleepiness scale***), whereby sleep urge or sleep propensity can be tracked in accordance to sleep patterns of an individual in order to determine (i.e. artificial intelligence or expert system means per examples ***Figure 77******,*** ***Figure 78******,*** ***Figure 79*****,** but not limited to) sleep patterns and/or sleep start and end times most conducive to minimising sleep urge day-time sleepiness; the present example can further associate these measures with monitored sleep measures along with associated sleep scoring (scoring of human sleep via sleep stage analysis) and/or respiratory scoring (i.e. detection of sleep disordered breathing) in order to determine, indicate, therapeutic device control (i.e. biofeedback or configuration or pressure dynamics of pressure ranges or dynamical changes of pressure associated with APAP/CPAP/PAP/NIPPV in order to minimise sleep disruptions or sleep fragmentation, maximise sleep architecture quality and/or minimise sleep disordered breathing while talking into account improved sleep propensity or daytime sleepiness (or residual daytime sleepiness). This can involve ***minimising or eliminating therapeutic event related arousals and*/*or respiratory event related arousals (TERA and RERA) events.***
>>computation of an individual's **circadian** clock cycle can be based on any of or any combination of:
   - temperature of the subject/patient and/or cycle **EEG** signals indicative of the brains **circadian** clock and/or activity or motion of subject/patient and/or homeostatic sleep monitored characteristics and/or sleep/wake or other health survey information;
   - the present example enables wearable ***mobile wireless monitoring of information and derivation of* a *subject*/*patient's natural circadian rhythm***, whereby such measures can be based on measurement sensors attached or forming part of smart watch, wristband, forehead sensor, armband or other wearable monitoring sensor system incorporating measures that can be analysed in a manner where slow (typically 24 hour slow changing cyclic measures can be derived based on monitoring temperature and/or galvanic skin resistance, and/or heat-flux, and photo-plethysmographic (PPG) measures such as pulse rate, and/or heart rate variability etc., which are physiologically aligned with the natural 24 hour **circadian** clock function.
   - The present example can monitor and compute a measure of **residual excessive sleepiness (RES)** in addition to computation of, comparing, and contrasting measure(s) of any of or any combination of the following as a means of determining association or causation of RES (i.e. TERA versus other factors as a mechanism to optimise automatic positive airway pressure titration in order to maximise obliteration of sleep disordered breathing, optimise cardiac function and optimise sleep quality) with CC or homeostatic factors (i.e. sleep propensity versus **circadian** clock (CC) asynchronous factors - i.e. delay or advanced CC phase): 1) a subject/patient's **sleep parameters** (i.e. any of or any combination of but not limited to **EEG,** EMG, EOG), 2) sleep breathing disorders, 3) therapeutic event related arousals (TERA) ³⁻⁵, 4) respiratory event related arousals (RERA) arousals, 5) **circadian** clock factors, 6) previous sleep duration(s), 7) previous wake period, 8) previous waking periods, 9) previous sleep periods in terms of sleep architecture, 10) previous sleep periods in terms of deep sleep (i.e. N3) and/or REM sleep, 11) previous sleep period versus CC, 12) current sleep duration(s), 13) current wake period, 14) current waking periods, 15) current sleep periods in terms of sleep architecture, 16) current sleep periods in terms of deep sleep (i.e. N3) and/or REM sleep, 17) current sleep in terms of sleep architecture;⁶
**>>>Somfit or other forehead monitoring device with sleep posture training function**
   with inbuilt (self-contained) training system capable of detecting snoring (i.e. via inbuilt breathing sound or snoring monitoring function (i.e. accelerometer vibration or microphone sensor) in a manner whereby subject/patient can be alerted or awoken (including headband attached vibration or sound alarm device) based on determination of any of or any combination of:
   1) sleep alarm setting (i.e. watch, mobile phone, alarm clock etc.),
   2) sleep stage determination including wake, N1, N2. N3, REM, Non-REM, arousal, spindle, k-complex, alpha bursts, EMG bursts, EMG atonia status etc.,
   3) subject/patient posture,
   4) subject/patient posture preference where snoring is least evident (i.e. side versus back position etc.),
   5) subject/patient biological synchronisation with breathing cycle (such as derived from any of monitoring sensors including infrared heat flux or subject/patient breathing signals, in order to mitigate against risk of detection of sleep partner breathing measures (or snoring) versus actual subject/patient snoring measures;
   6) in one example the present example can (for example only):
      - in a ***first process step*** determine from the users selection of alarm request the amount of time before the alarm activates;
      - in a ***second process step*** track sleep stages (i.e. wake, N1, N2, N3, REM), and sleep events (i.e. spindles, arousals, k-complexes), while continually assessing the time before the alarm is activated;
      - in a ***third process step*** and in circumstance when a subject/patient is entering a sleep state which is prone to or has been defined as being prone to cause "grogginess" or "weariness" when interrupted or awakened from, the present example can activate alarm in a more favourable sleep state that is less prone to cause such "weariness upon awakening. For example, if a subject/patient sets alarm for a daytime 1 hour power nap to try and overcome jet-lag or other forms of sleep-urge or sleep propensity, and the present example determines or tracks (via online sleep analysis capability) that the user/subject is in REM sleep (less adverse recovery impact than N3 deep-sleep state, for example) then the alarm will sound at the selected alarm setting.
      - in a ***fourth process step*** and if (for example), the present example determines or tracks (via online sleep analysis capability) that the user/subject appears to be transitioning from REM to NI/deep-sleep stage (prone to result in high adverse recovery impact than REM stage, for example) and also there is only say 10 minutes time before the alarm set time is activated, along with the condition that the ***maximum pre-wake alarm time*** is set (or defaulted) to greater than 10 minutes, then the present example will sound the alarm and allow subject/patient to be awakened in the preferred REM state versus the potential N3 deep-sleep state.
   The ***maximum pre-wake alarm time*** is the setting related to the maximum time allowed before awakening that the present example can use its processing capabilities to optimise awakening event based on subject/patient sleep stage.

***Maximum pre-wake alarm time*** can be set in accordance to sleep time (i.e. hours and minutes) or selected in terms of sleep time (i.e. percentage of total sleep time, where ***typical start and end sleep times*** or ***typical total sleep time periods*** are entered or set as default by system) or time prior to alarm 10% of total sleep period, for example.

### Circadian drug delivery clock patent

The present example includes the capability for a smart watch with attachable or integrated (embedded or on-board as part of smart watch and/or wireless interconnected processing system(s)) a biofeedback feedback drug delivery (BFDD) system and or "associated therapy" system, whereby said biofeedback drug system can comprise of any of or any combination of: a) a drug delivery dispenser system, b) an automatic analysis system and/or c) a drug delivery control system;

The said "drug delivery dispenser system" can comprise of any attachable drug delivery system including a manually or automatically controlled drug dispenser capable of "optimal medication dispensation" including any of or any combination of:
A) dispensing medication at an optimal time, and/or rate,
B) and/or compound (I.e. Drug type or drug compound mixture including but not limited to melatonin) and/or
C) concentration and/or delivery concentration and/or
D) delivery rate,

- whereby said in order to minimise the effects of sleep delay syndrome (I.e. **Offset** between a subject's sleep/wake cycle and/or work cycle/schedule/clock/calendar (i.e. shift work) and/or social cycle/schedule/clock/calendar and/or travel cycle/schedule/clock/calendar (i.e. itinerary), whereby said "optimal medication dispensation" can be applicable to any physiological and/or neurological and/or sleep disorder and/or other adverse health condition;
- whereby said "biofeedback" system and/or "associated therapy" system can include any of or any combination of:
   i) **circadian** rhythm entrainment system;
   ii) **circadian** rhythm entrainment system including light radiation control or light radiation;
   iii) Internet or other interconnected **circadian** rhythm entrainment system including light radiation control or light radiation;
- whereby the said "automatic analysis system" can comprise of determination of a subjects sleep/eke patterns and/or homeostatic sleep characterisation and/or determination of an individual's **circadian** clock cycle of an individual as outlined elsewhere in this document;
- whereby the said "drug delivery control system" can comprise of a drug dispensing mechanism (including, but not limited to, a cartridge loaded drug dispenser which can dispense medication/drugs in order to minimise sleep delay or sleep **offset** syndrome in a manner which can be configured to be moderate, severe, or other graduated entrainment (I.e. Compensating for an individual's sleep delay or **circadian** clock **offset** factor);
- whereby the said "biofeedback feedback drug delivery system" can be a part of any of or any combination of: a) any wireless mobile system; b) any wearable monitoring system; c) any telemedicine system;

### AUTOMATIC SLEEP/WAKE /CIRCADIAN MEDICATION AND/OR THERAPY SYSTEM

***The present*** example ***provides a mobile wireless phone or wearable device*** or separate mobile device ***PLUS*** option for automatic or manual medication dispenser system (i.e. sleep suppression or antagonist medications such as melatonin etc.) ***PLUS*** option for sleep/wake cycle analysis system (automatic or manual via local or on-broad processing and/or interconnected processing such as network or cloud-computing or other wireless interconnectivity format) ***PLUS*** option for **circadian** rhythm analysis system (automatic or manual) PLUS option for light therapy entrainment (i.e. internet of things including light and/or alarm or music and/or audio controls designed to adjust an individual's **circadian** rhythm cycle) ***PLUS*** option for meditation and/or relation music and/or audio ***PLUS*** option for linkage of said one or more therapy types to be linked to biofeedback including processed brain signals (i.e. **EEG**) and/or other physiological signals (i.e. temperature, oxygen saturation, heart-rate, perspiration or galvanic skin resistance, ECG, EMG, EOG, room light status, subject motion, subject location, subject position, and/or subject activity;

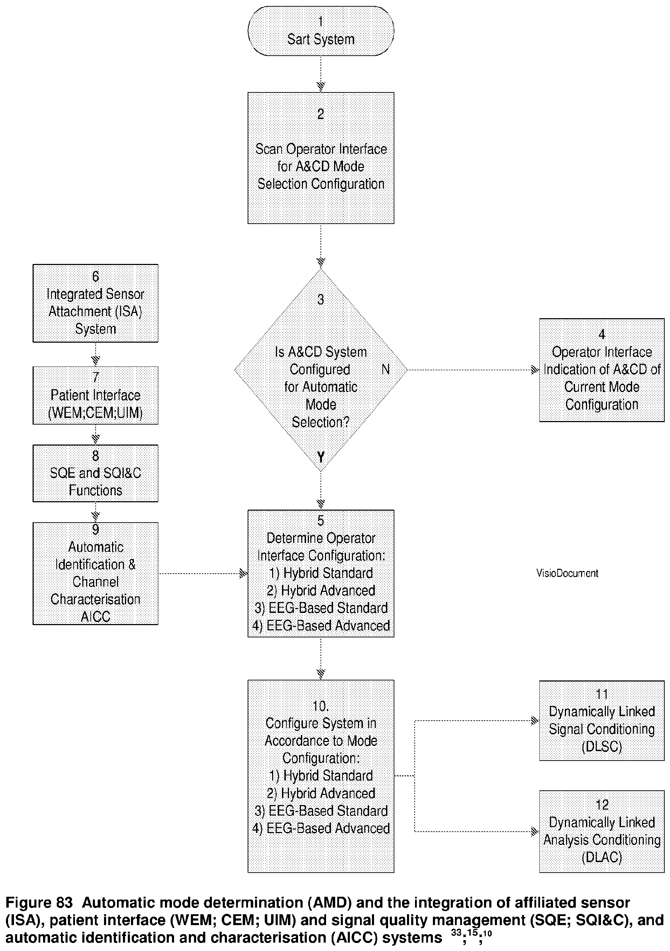

### (automatic monitoring and model configuration capability; Figure 83)

- The present example provides a **means** of determining, ***selecting, configuring*** , whereby said means includes the capability of any of or any combination of (but not limited to): automatic means) of ***sleep monitoring study type*** and/or ***sleep study configuration*** and/or ***indication of required sleep monitoring kit*** (including option of single select options of sleep study device(s) monitoring kit and/or consumable sensor kits), ***adjustment of computer and other related local or remote device and computer resources*** applicable to selected or required study format (can also include consideration of "high dependence connectivity monitoring (***HDCM***)" or "adaptive physiological-Body Network (***APN*/*APM***) *", **automatic sensor connectivity*** requirements and connection or quality status, ***automatic pre-acquisition signal processing*** (i.e. filtering, sensitivity etc.) configurations, ***automatic data-acquisition configurations*** (i.e. analogue to digital sampling rates, sample resolution/steps for each respective monitored, analysed, transmitted, stored and/or displayed channel), ***automatic sensor or electrode location information*** registered for each monitored channel, ***automatic post-acquisition signal processing*** (i.e. filtering, sensitivity etc.), ***automatic interconnectivity and*/*or intercommunication relating to data characteristics*** (i.e. but not limited to sampling rates, sample resolution/steps for each respective monitored, analysed, transmitted, stored, locally displayed, remote displayed, reports, alerts, alarms and/or other communication access or transmission),
- the present example provides a **means** of determining ***configuring or enabling configuration (with automatic configuration option)*** of any of or any combination of (but not limited to): automatic wireless of or wire interface interconnectivity means whereby the present example can automatically detect ***subject*/*patient*/*user* *study type,*** including a ***predefined series or monitoring protocols which can be classified*** in accordance to both ***physiological monitoring parameters*** and quality or specific properties associated with each said ***physiological channel and specific signal characteristics monitored*** (including any of, but not limited to automatic pre-acquisition signal processing (i.e. filtering, sensitivity etc.) configurations, automatic data-acquisition configurations (i.e. analogue to digital sampling rates, sample resolution/steps for each respective monitored, analysed, transmitted, stored and/or displayed channel), automatic sensor or electrode location information registered for each monitored channel, automatic post-acquisition signal processing (i.e. filtering, sensitivity etc.), automatic interconnectivity and/or intercommunication relating to data characteristics), consideration of "high dependence connectivity monitoring (HDCM)" or "adaptive physiological-Body Network (APN/APM) ", and/or automatic sensor connectivity requirements and connection or quality status;

- Whereby said ***predefined series or monitoring protocols which can be classified*** include automatically adjusted or upgraded monitoring types and/or levels and/or categorisation and/or **scope** (or "**SCOPER**" per in clinic and out of clinic study approaches and/or recommendations and/or health insurance requirements or guidelines and/or government health rebaits etc.) along with associated sleep monitoring study scope including type and categorisation of sleep, cardiovascular, oximetry, position, effort, and/or respiratory monitoring requirements);
- the present example provides automatic determination and corresponding configuration of the sleep monitoring system configuration;
   >Whereby said determination can comprise of detecting ***consumer*/*non-regulatory*** versus ***professional*/*regulated*** (i.e. China-FDA, USA-FDA etc.);
   >whereby said determination can comprise of detecting actual study criteria or local regulatory (i.e. health insurance reimbursement or government reimbursement or market clearance body (i.e. USA, China FDA etc.) in contrast or comparison to actual sleep study configuration details;
   >whereby indications, hints, automatic prompting, are provided in terms of any of or any combination of:
      - compliance of ***professional*/*regulated*** (i.e. China-FDA, USA-FDA etc.) requirements;
      - actual progress or **steps of process** in terms of required (outstanding) versus completed progress of study status or study setup/configurations, professional oversight and authorisation versus China-FDA, USA-FDA etc. oversight and authorisation;
      - determination of authorised completion by board certified sleep specialist (BCSS) **evaluation of suspected subject/patient obstructive sleep apnoea** (OSA);
      - BCSS determination of whether subject/patient has symptoms or **signs of comorbid medical disorders**;
      - BCSS determination of whether subject/patient has symptoms or **signs of comorbid sleep disorders;**
      - compliance of ***professional*/*regulated*** (i.e. China-FDA, USA-FDA etc.) sleep or other subject/patient health questionnaire(s) requirements;

### (sleep scope)

- whereby said ***predefined series or monitoring protocols which can be classified*** includes, any of or any combination of (but not limited to) sleep, cardiovascular, oximetry, position, breathing effort and/or respiration measures;
   - where said ***Sleep categorisation 1*** (for example only) can include the monitoring of **sleep parameters** such as (but not limited to) sleep by using 3 active **EEG** channels, at least one EOG channel and chin EMG channel;
   - where said ***Sleep categorisation 2*** (for example only) can include the monitoring of **sleep parameters** such as (but not limited to) sleep by using at least 2 active **EEG** channels (plus separate reference **EEG** channel), with or without EOG channel or chin EMG channel;
   - where said ***Sleep categorisation 3*** (for example only) can include (but not limited to) the monitoring of sleep surrogate channels such as actigraphy;
   - where said ***Sleep categorisation 4*** (for example only) ***based on other*** sleep measures than those detailed in Sleep categorisation 1, 2 and 3;

### Cardiovascular scope

- whereby said ***Cardiovascular categorisation 1*** (for example only) can include the monitoring of cardiovascular parameters such as (but not limited to) more than 1 ECG lead with option of deriving events;
- whereby said ***Cardiovascular categorisation 2*** (for example only) can include the monitoring of cardiovascular parameters such as (but not limited to) peripheral arterial tonometry (such as described in ***Figure 37******;*** ***Figure 38***);
- whereby said ***Cardiovascular categorisation 3*** (for example only) can include the monitoring of cardiovascular parameters such as (but not limited to) a standard (1 lead) ECG measure ;
- whereby said ***Cardiovascular categorisation 4*** (for example only) can include the monitoring of cardiovascular parameters such as (but not limited to) derived pulse, such as oximetry;
- whereby said ***Cardiovascular categorisation 5*** (for example only) can include the monitoring of cardiovascular parameters (but not limited to) based on other cardiac measures than those detailed in Sleep categorisation 1, 2, 3 and 4;

### Oximetry scope

- whereby said ***Oximetry categorisation 1*** (for example only) can include the monitoring of oximetry (but not limited to) finger or ear sensing with sampling of 3 s averaging and at least a 10 Hz sampling rate but preferably 25 Hz;
- whereby said ***Oximetry categorisation 1a*** (for example only) can include the monitoring of oximetry (but not limited to) finger or ear sensing with sampling properties less than 3 s averaging and 10 Hz sampling rate;
- whereby said ***Oximetry categorisation 2*** (for example only) can include the monitoring of oximetry (but not limited to) located at an alternative site such as forehead;
- whereby said ***Oximetry categorisation 3*** (for example only) can include the monitoring of other oximetry formats;

### Position scope

- whereby said ***Position categorisation 1*** (for example only) can include the monitoring of video or visual position measurement;
- whereby said ***Position categorisation 2*** (for example only) can include the monitoring of nonvisual position measurement;

### Breathing Effort scope

- whereby said ***Breathing effort categorisation 1*** (for example only) can include the monitoring of two respiratory inductive plethysmography measures (abdominal and thoracic measures);
- whereby said ***Breathing effort categorisation 2*** (for example only) can include the monitoring of one respiratory inductive plethysmography measures (either abdominal or thoracic measure);
- whereby said ***Breathing effort categorisation 3*** (for example only) can include the monitoring of one respiratory inductive plethysmography measures a derived breathing effort measure, such as forehead forehead venous pressure (FVP);
- whereby said ***Breathing effort categorisation 4*** (for example only) can include the monitoring of other breathing effort measures, such as piezo respiration belt measures;

### Respiratory scope

- whereby said Respiratory ***Breathing effort categorisation 1*** (for example only) can include the monitoring of nasal pressure and thermal sensor device;
- whereby said Respiratory ***Breathing effort categorisation 2*** (for example only) can include the monitoring of nasal pressure sensor device;
- whereby said Respiratory ***Breathing effort categorisation 3*** (for example only) can include the monitoring of thermal sensor;
- whereby said Respiratory ***Breathing effort categorisation 4*** (for example only) can include the monitoring of end-tidal C02 (ETC02) sensor;
- whereby said Respiratory ***Breathing effort categorisation 5*** (for example only) can include the monitoring of another respiratory measure;

- whereby any said or any combination of **sleep *categorisation* formats** (including an of or any categorisation 1-to 5 types or levels) and/or associated monitored signal channels (including any or any combination of **sleep, *Cardiovascular, Oximetry, Position, Breathing Effort, Respiratory scopes per above***) can comprise of automatically meshing one or more distributed wireless interconnected monitoring sensors with *time- **synchronisation*** (i.e. as described in but not limited to Title: MULTIPOINT TIME-SYNCH MONITORING (MTM) SYSTEM and associated "INVETION DECRIPTION").
- The present example provides an automatically connected and configured consumer or professional **wearable mobile HMS** (including device, corresponding online services, sensors, **therapeutic intervention** and control with option of **biofeedback** capabilities) activation linked in accordance to any of or any combination of (but not limited to) : a) monitoring study or HMS goal requirements, b) monitoring or HMS "categorisation" format, c) monitoring study or HMS "type or level" requirements, d) sensor-kit, monitoring-kit, e) professional-authorised and secure opt-in healthcarer, f) consumer services provider, g) professional services provider, h) personal-care-management platform, i) application , j) opt-in practitioner (regulatory - i.e. China FDA; USA FDA; CE etc.), k) online-buy-kit (i.e., Amazon, AppleStore, Samsung, Microsoft, Telco, MobileCo, Xiaomi, Baidu, Alibaber, Huawei, etc.), China Mobile, k) HMS eHealth online shop selection, l) HMS eLife online shop selection, k) HMS eHealth App selection or functions, k) HMS eLife App selection or functions, l) HMO, m) health insurance organisation, n) Government health rebait linked-system) AMD, n) AICC, o) SQE, p) SQI&C;
   - where said ***Sleep monitoring* study type (level) 1 *(most comprehensive level)*** (for example only) includes the following monitored channels: **EEG,** EOG, ECG/Heart rate, Chin EMG, Limb EMG, Respiratory effort at thorax and abdomen, air flow via nasal cannula thermistor and/or breathing effort, (respiratory plethysmography), pulse oximetry, additional channels for CPAP/BiPAPA levels, pressure, CO2, pH etc.;
   - where said ***Sleep monitoring* study type (level) 2** (for example only) includes the following monitored channels: **EEG,** EOG, ECG/heart rate, Airflow, Respiratory effort, oxygen saturation;
   - where said ***Sleep monitoring* study type (level) 2 AU** (for example only) includes the following monitored channels: **EEG,** ECG, airflow, thoracic-abdominal movement, oxygen saturation, body position, and either EOG or chin EMG);
   - where said ***Sleep monitoring* study type (level) 3** (for example only) includes the following monitored channels: 2 respiratory movement/airflow, 1 ECG/heart rate, 1 oxygen saturation;
   - where said ***Sleep monitoring* study type (level) 4** (for example only) includes the following monitored channels: minimum of 3 channels including Arterial oxygen saturation, airflow, optionally thoracic-abdominal movement, along with the direct calculation of AHI or RDI measure;
   - The present example further provides a ***means of Automatic Mode Determination (AMD),***
   whereby the AMD function works in conjunction with the ***AICC, SQE and SQI&C*** functions to enable the system configuration to be set up in accordance with the specific electrode configurations and quality status during the system operation. The AICC, SQE and SQI&C functions are detailed in the patient interface section. Specifically, the SQE system tracks the status of the electrode connections and quality. The corresponding control functions are relayed by the SQI&C function to the AMD system. The A&CD monitoring mode in then adjusted in accordance to the validity and connections status of the sensors and electrodes at any point in time.

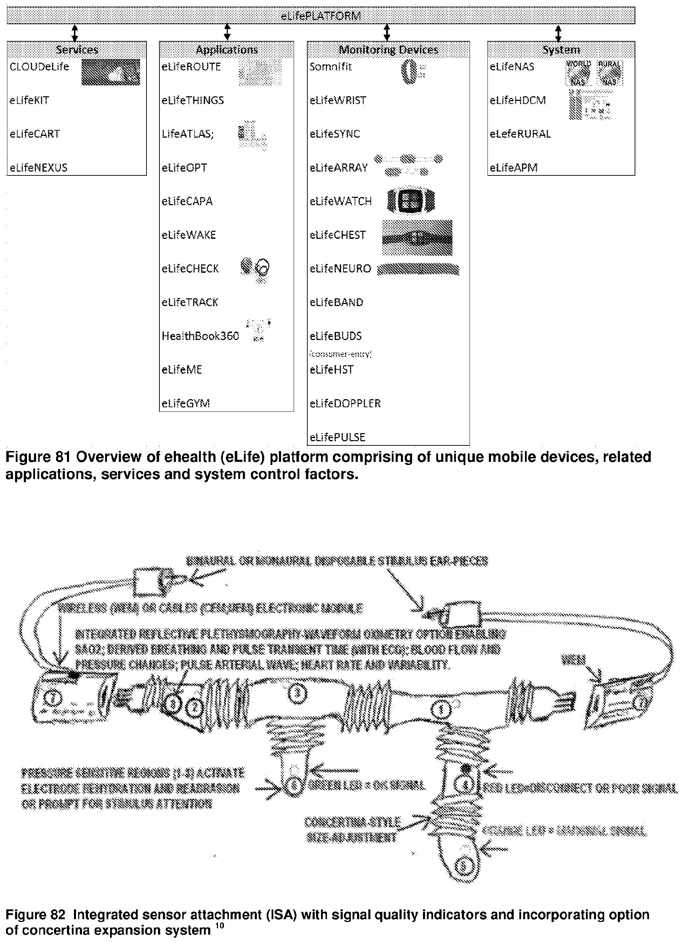

As shown in ***Figure 83*** (below) the activation of automatic mode determination (***Figure 83*** **;[3:Y]**) if activated (***Figure 83*** **;[5]**) prompts the A&CD system to configure the monitoring operational mode in accordance to the ISA format and signal quality. However, of the AMD function is not activated (***Figure 83******;* [3:N]**) the operator interface is configured in accordance to the operator manual mode selection (***Figure 83*** **;[4]**). The integrated attachment sensor (***Figure 83*** **; [6]**) is connected to the patient interface module **[7]** which detects and determines the ISA format, along with the sensor attachment and quality status (***Figure 83******;* [8]**). This information is input as part of the decision matrix involved with the automatic identification and channel characterisation (***Figure 83*** **; [9]**; AICC) system. The mode determination (***Figure 83***; 5) then enables the various affiliated systems including the dynamically linked signal conditioning **(*****Figure 83******;* [11]**) and the dynamically linked analysis conditioning (***Figure 83******;* [12]**) system to configure or adapt the A&CD systems corresponding to operational, environment and signal quality conditions. The combination of on-line signal quality tracking and the AMD enables special on-line adaptation such as when the AEP click stimulus is disconnected, whereupon the system can revert from the a hybrid (AEP and **EEG-**based) to an **EEG**-based monitoring mode.
- The present example further provides a ***means of automated Identification and Channel Characterisation (AICC),***
   whereby the ***AICC system*** is designed to automatically detect the integrated sensor attachment (ISA) system configuration (i.e. ***Figure 82***) and also per (but not limited to ***Figure 1******;*** ***Figure 28******;*** ***Figure 31******;*** ***Figure 38*****;** ***Figure 46******;*** ***Figure 55******)*** other monitoring system examples (i.e. but not limited to) the wearable mobile or other monitoring system monitoring modes (hybrid, **EEG** based etc.) and format (standard or advanced). Additionally, the specific signal filtering and processing characteristics required for each respective channel can be automatically configured. In this way a single quick snap ISA connection (for example) can automatically prepare the system for monitoring.
- The present example further provides a ***means of Signal Quality Estimation (SQE),*** whereby the said Signal Quality Estimation (SQE) System enables continuous tracking of the input signal's integrity and overall quality. Additionally, such measure can be continually compared to predetermined and acceptable signal ranges, limits, and other important characteristics.

The ***SQE*** function works in conjunction with ***on-line impedance measurement functions (OIM).***
- The present example further provides a ***means of Sensor*/*Electrode Quality Indicator and Control (SQI&C),***

Whereby the said ***SQI&C*** system can be integrated within the patient interface (such as Somfit or other wearable monitoring devices (i.e. but not limited to ***Figure 1*** ***wearable device examples**)* or monitoring head-box (i.e. for clinical or laboratory monitoring) provides a number of key functions including visual user-prompts indicating the connection and sensor quality status at all times.

### Low-disturbance Continuous On-line Impedance Measurement (LCOIM) System

The LCOIM system generally refers to the use of active on-line impedance measurements undertaken in such a manner that the measurement periods are flagged and excluded from the downstream analysis results. Furthermore the impedance measurement periods can be minimised by measuring a transient response signal versus conventional steady state waveform measurement methods. The present example enables a combination of passive signal quality tracking (i.e. examination of monitored signal in terms of typical and expected signal characteristics versus signal characteristics typical of interference and other artefact sources (i.e. main, sweat artefact, electrode popping noise etc.).

The present example further enables active impedance measures to be interleaved with monitored signals is such a manner that measurement periods (which can cause monitoring signal disturbances) can be limited to "non-critical" or "less-critical" monitoring periods (i.e. not during periods that are critical to the determination of sleep staging epochs (i.e. not during transitional sleep stages and the like). Non-critical periods can also be predefined as periods when vital measures such as spindles or K-complexes marking sleep stage changes are not evident.

### Artifact Compensation and Rejection (AC&R) System

The AC&R system incorporates a series of algorithms capable of eliminating or minimising noise or artifact. Automatic online artifact routines can identify the specific severity level, interval and classification of artifact. Reduction or removal of the effects of unwanted background physiological artifacts including EMG signal intrusion, eye-blinks, EOG intrusion, arousals (various neural and autonomic categories to be included), body movements, movement time, and unwanted PAMR signal intrusion can be automatically and , continuously tracked and implemented online. The present example enables high tolerance and quality sustained signal monitoring during severe interference from electrical, EMF and other body movement artifact and other signal interference sources. The present example can incorporate a high impedance ***environmental noise-sampling input channel*** configured to operate like an antenna for noise and sample interference in a manner that the signal characteristics or environmental interference can be monitored and/or stored. In this way the environmental noise can be characterised in terms of determining filtering types and characteristics required to minimise or eliminate noise form monitored signals channels. For example, ***environmental noise-sampling sampled input channel*** can be used to help cancel out noise in input channel of interest, by way of phase shift, magnitude adjustment and ultimate unwanted cancellation of unwanted environmental noise. i.e. a noise cancellation channel can be created and then phase shifted to be precisely equal but opposite phase (180 degree phase shift) to noise present in other signal channels, in order to null out or cancel out unwanted noise.

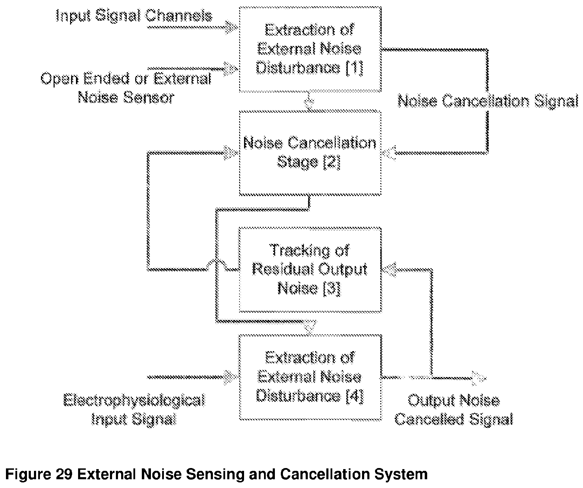

The AC&R incorporation of an open-ended "noise-sampling channel" as a means to cancel out unwanted signals is illustrated in ***Figure 29******.***

### healthpal

The present example enables a **health payment system which "appropriately" (**i.e. compliance with relevant privacy, security, government, medical, patient-care, safety aspects, which can be linked in or programmed in as a function in accordance to the location or patient specific details and health information) incorporates a means of separating patient or consumer information from health services or products supplier but while still enabling a means of accessing a personalised medical data -base service that enables access to important information such as **health insurance compliance** or approval data, **medical prescription data** including verification of therapeutic type and/or adjustments (i.e. PAP. NIPPV devices), in addition to conventional means of enabling online payment transaction;
- further examples include the capability to **"appropriately" assess contradictions, side-effects,** checks against contradictions between patient medical history (i.e. allergies, reaction with certain drugs etc.) and prescribed medications) and alert or notify subject/patient and relevant health-carers as required;
- further examples include the capability to **automatically handle Government/Insurance reimbursement** by way of advice, coaching, simple steps, GP, practitioner, advice/steps, HMOs, etc.
- further examples include the capability utilise **two-way separated or buffered data access** so that a guaranteed disconnect and both privacy security of information exists between subject/ patient and supplier to conceal private ailments and medical history or privacy in general (i.e. private medications or the like with "healthpal" with health-sensitive and health specialist security).

### Patent---Elife gait or motor-neuron (including Parkinson's) process or apparatus

The present example provides a means of ***characterising an individual's gait or motion characteristics,*** including any measure of the ***interrelationship of motion between any plurality of body extremities or limbs,*** and further includes any of or any combination of (but not limited to): **(walking, running, striding motion characteristics)**
The present example provides a means of ***analysing the motion characteristics*** of a subject/patient by way of analysing (i.e. ***spectral analysis such as FFT and segmentation*** of specified frequency bands applicable to different components of a subject's motion, including analysing the ***frequency band characteristics associated with arm swings*** and ***correlating these arm swings with leg steps in terms of phasic nature and synchronisation*** between signals. Said motion characterisation can include resolving motion properties such as any of or any ***combinations*** of the ***linear length*** (i.e. millimetre), ***cyclic time*** (i.e. seconds), ***regularity*** (i.e. ***deterministic or regularity of arm swing or leg stride*** motion characteristics;

### (body extremities motion interrelationship)

Said ***synchronisation and phasic interrelationship between arm swing and corresponding leg motion*** characteristics will change over time with the progression of movement and neural disorders, and particularly during the navigation by subject of more challenging motor control circumstances associated with corners, changing direction and the like.

### (NLDB motion analyses)

Similarly, analyses of motion sensor out puts (i.e. accelerometer) can comprise of the deployment of ***non-linear dynamic based (NLDB) analyses transformations*** whereby, for example only, the regularity (i.e. deterministic or regularity of arm swing or leg stride motion characteristics can be measured in terms of ***non-linear dynamic characteristics*** indices such as (but not limited to) complexity or entropy;

### (motion pattern or signal morphology analyses)

Similarly, analyses of ***motion sensor outputs*** (i.e. accelerometer) can comprise of the deployment of pattern recognition analyses transformations whereby the ***leg stride and arm swing motions can mapped and recorded with signal characterisation*** in order to characterise ***gradual changes in pendulum action, fluency, flow of motion, synchronisation*** with any other limb or extremity as a measure of subtle or more severe deterioration or improvement applicable to reversal/recovery or degradation of movement, neural or nervous disorders including any of "events of interest/EOI" (per "EOI DEFINITION" detailed" in abbreviation table at rear of this document);

### (motion correlation with location/GPS analyses)

The present example further incorporates means of combining ***locational tracking*** such as ***GPS information*** to map out an individual's motion characteristics in a manner whereby progressive deterioration over a period of time and as it relates to ***different category of movements*** (i.e. ***walk, jog, stair climbing, sports or gym exercise systems such as treadmills, running***) can be analysed in context of ***navigational or gait difficulty*** (i.e. mild, moderate or severe manoeuvring difficulty, straight line pathway, etc.) along with corresponding gait or balance output measures (i.e. such as but not limited to any or any combination of:
>>measures of ***interrelationship*** (degree of ***phasic synchronisation between any two or more body limbs or extremities or main body motion sensing locations***);
>> ***cyclic and*/*or phasic interrelationship between any two or more signal or data outputs*** of two or more motion sensing systems (i.e. can be a part of or attached to a wearable device or subject's mobile phone or mobile phone case incorporating sensing device (i.e. single or multiple axis accelerometer measurement sensor),
   - whereby motion analysis or characterisation of ***any one or limbs or extremities and*/*or main body section(s)*** comprises any of or any combination of (but not limited) to any length of arm swing (linear or arc millimetres) ***walking fluency; walking arm movement; swinging arm pattern; swinging arm movement*** (i.e. via wrist watch or bangle such as (but not limited to) single multi-axis accelerometer; degree of walking leg synchrony with arm synchrony; terms of phasic nature and synchronisation between signals. Said motion characterisation can include resolving motion properties such as any of or any combinations of the linear length (i.e. millimetre), cyclic time (i.e. seconds), regularity (i.e. deterministic or regularity of arm swing or leg stride motion characteristics; and ***pendulum motion of each arm swing or step; symmetry factor being correlation between arm and leg movements*** during walking, the present example further comprising of analyses of association, comparison or other phase, or cyclic or other means of determining the interrelationship between a plurality of said extremities, limbs or body section motion of a subject/individual;
      >>>the present example provides measurement assessment and characterisation of combined ***SBD REM atonia*** and motion analyses applicable to prognosis or diagnosis of movement or neurological disorders including "health conditions of interest" or "events of interest/EOI" (per "EOI DEFINITION" detailed in abbreviation table at rear of this document);
   - the present example incorporates a ***means of correlating SBD*** (per ***Figure 75***) ***and*/*or motion analysis means*** such as described elsewhere in this document;

### Description of the Figures

### CIRCADIAN HMS FIGURE DESCRIPTIONS

### Figure 96.

The present example provides a process as part of microprocessor programmed within one or more of any of or any combination of software application(s), mobile device(s), wearable technology, or interconnected communication systems(s) (i.e. SAAS including Cloud-computing services , LAN, WAN, NAS etc.) capable of providing **circadian** clock health management by way of inputs such as but not limited to any of or any combination of inputs functions, application or systems covered here in Figure 96.

**BLOCK 1** AND **BLOCK 2** or processing/determination per BLOCK 3 or respective outputs in **BLOCKS 4 to 10, or other inputs, determinations or outputs covered elsewhere in this document.**
**[Block 1] CIRCADIAN CLOCK (CC) HEALTH MANAGEMENT SYSTEM (HMS) INPUTS >>ENVIRONMENTAL INFORMATION CC ENTRAINMENT inputs;**
   - **Block 1** incorporates inputs including new and current environments time-zones and/or solar daylight conditions, temperature changes, information on population studies or health information such as typical sleep deprivation or sleep urge/propensity associated with various degrees of **circadian** clock asynchrony (including incorporation as part of self-learning algorithm processes (i.e.. but not limited to self-learning processes per examples .);
**>>INPUTS/ INFORMATION**
   - Questionnaire activation with interaction via subject/patient/user and information or related derived information or associated outcomes as they relate to characterisation of an individual's sleep/wake cycle and/or **circadian** clock cycles; i.e. example (but not limited to) questionnaires include Horne-Ostberg Morning-Eveningness Questionnaire (MEQ); Epworth Sleepiness Scale (ESS); Munich Chronotype Questionnaire (MCTQ) etc. (see also CC HMS processing/determinations);
   - endogenous **circadian** cycle;
   - subject/patient/user target or desirable **circadian** cycle entrainment outcome;
   - subject/patient/user target (desirable) **circadian** cycle;
   - actual versus target **circadian** cycle;
**>>ENVIRONMENTAL MONITORED AND TRACKED CC ENTRAINMENT input;** i.e. but not limited to any of or any combination of:
   - Entrainment stimulus or environmental condition changes (i.e. light-therapy controlled inputs; magnetic stimulation controlled targeting and dosage and property adjustment of inputs; temperature controlled inputs etc.;
   - Zeitgebers;
**>>ENVIRONMENTAL MONITORED AND TRACKED CC ENTRAINMENT input;** i.e. but not limited to any of or any combination of:
   >>Entrainment stimulus or environmental condition changes (i.e. light-therapy controlled inputs; magnetic stimulation controlled targeting and dosage and property adjustment of inputs; temperature controlled inputs etc.;
**>>REGULATION OF SLEEP BY WAY OF CIRCADIAN AND HOMEOSTATIC SLEEP/WAKE FACTORS**
   i.e. but not limited to any of or any combination of:
   - **actual sleep-wake cycle** (i.e. timing and structure of sleep and waking such as derived from monitored **sleep parameters** and/or associated measures including (but not limited to ***Figure 77******;*** ***Figure 78******;*** ***Figure 79*** as covered elsewhere in this document);
   - subject/patient/user target (desirable) sleep-wake cycle;
   - actual versus target sleep-wake cycle -temperature measures;
**>>PHYSIOLOGICAL AND/OR PSYCHOLOGICAL MONITORED AND TRACKED CC ENTRAINMENT input;**
   i.e. but not limited to any of or any combination of:
   - **EEG** measures;
   - retina light measures;
   - GSR measures;
   - activity measures;
   - position measures;
   - pulse measures;
   - measures of blood-pressure;
   - brain activation measures;
   - sleep/wake measures;
   - light measures;
   - Objective measurement of rest-activity cycle timing can be made with actigraphy;
   - **Point of care body fluid measures such as melatonin** (i.e. the rhythm of the melatonin concentration provides an optimal **circadian** phase marker for humans) or cortisol (i.e. measures via periodic testing of samples of saliva, blood/plasma or urine or associated blood/plasma, saliva or urine sampling test trips and related point of care testing systems, with option of automatic integration or interface with mobile health monitoring or tracking systems);

### [Block 2] CIRCADIAN CLOCK (CC) HEALTH MANAGEMENT SYSTEM (HMS) BRAIN SLEEP/WAKE REGULATION INPUTS

- **The present** example **enables forward and/or reserve equation EEG electrode minimisation/monitoring** (i.e. see also ***Figure 45*** minimisation process) and/or source localisation applicable to an individual's health management and associated markers of consciousness, sleep/wake homeostatic or **circadian** rhythm determination/monitoring, brain centres regulating wakefulness and sleep (including neuromodulators and neurotransmitters producing **EEG** activation, such as histamine, acetylcholine, norepinephrine, hypocretin and glutamate),
further including any of or any combination of brain regions (but not limited to):
- the thalamus or associated areas;
- posterior cingulate or associated areas; medial parietal cortex or associated areas;
- medial basal forebrain or associated areas;
- occipital cerebral cortex regions or associated areas;
- cingulate gyrus or associated areas;
- prefrontal cortex or associated areas;
- reticular formation or associated areas;
- pons or associated areas;
- visual cortex (visual cortex part of cerebral cortex;
- located in occipital lobe) or associated areas;
- hypothalamus or associated areas: anterior (ventrolateral preoptic nucleus, responsible for promoting sleep) or associated areas;
- posterior (tuberomammillar nucleus, responsible for generating histamine; medulla oblongata; orexin [A/B], relating to wake-promoting neural activity);
- midbrain and pons (central point of the generation of coeruleus (norepinephrine; NE), raphe nuclei (serotonin; 5-HT), and
- pedonculopontine) or associated areas;
- olfactory bulb or associated areas;
- cerebellum or associated areas; and/or
- suprachiasmatic nucleas (**circadian** clock) regions or associated areas;
- along with related coherence or Dipole modelled connectivity interrelationships (including thalamus-cortical interrelationship);

### [Block3] CIRCADIAN CLOCK (CC) HEALTH MANAGEMENT SYSTEM (HMS) PROCESSING/DETERMINATION

### >>-CC SYNCHRONISATION WITH ENVIRONMENT CLOCKS OR CYCLES (I.E SOCIAL, TRAVEL, TIME-ZONE, SOLAR, WORK-SHIFT, REQUIRED-SCHEDULE, REQUIRED AGENDA, REQUIRED ININERARY CLOCK ETC.)

- determination of **optimal synchronization with the environment or entrainment,** which can be mediated by way of controlling steady state or periodic **stimuli (also referred to as zeitgebers)** to stimulate or act upon the subject/patient/user's **circadian** clock (see also CC HMS inputs);
- **interactions and interrelationship between the biological clock (or circadian clock (pacemaker)** and subject/patient/user sleep homeostatic process (i.e. dependent on prior sleep/wake and associated characteristics)
- **modelling/computation of interactions and interrelationship between the biological clock (or circadian pacemaker) and subject/patient/user sleep homeostatic process** as a means of determining a range of sleep disorders or sleep disturbances, such as (but not limited to the interpretation of possible rhythmic **circadian** clock abnormalities implicated with depression, along with the determination of associated framework and recommendations and/or biofeedback and/or control for associated treatment via therapeutic treatment or related **circadian** rhythm adjustment;
- **determination of the time variation between the phase of a subject/patient/user's circadian rhythm such** as sleep onset and the phase of a zeitgeber such as the solar clock cycle (i.e. dusk or dawn);
- **sleep timing information such as the sleep midpoint** (i.e. between sleep onset time and wakeup time);
- Objective analysis of **rest-activity cycle actigraphy** i.e. FFT and frequency analysis of accelerometer outputs followed by segmentation of likely source or causation of activity and then association of said categorisation of movements with sleep, wakes and other activities as a means of deriving final sleep/wake/activity cycle of subject/patient/user;
- **Sleep/behaviour questionnaire activation** with interaction via subject/patient/user and information or related derived information or associated outcomes as they relate to characterisation of an individual's sleep/wake cycle and/or **circadian** clock cycles; i.e. example (but not limited to) questionnaires include Horne-Ostberg Morning-Eveningness Questionnaire (MEQ); Epworth Sleepiness Scale (ESS); Munich Chronotype Questionnaire (MCTQ) etc. (see also CC HMS outputs);
- **determination from melatonin samples** of the "Dim light melatonin onset" (DLMO), which is the marker of body clock time which can be measured in saliva or urine or blood/plasma before a person goes to sleep. Moreover, during 24-hour rhythm measurements the points including peak, midline crossing point, **offset** of secretion, and other points can be determined as a derivations of subject/patient/user **circadian** clock;
- the present example can provide a **point of care testing system** which is automatically interfaced to a mobile or wearable device or via interconnectivity to a communication network or system in order to enable saliva, blood/plasma, urine sampling (i.e. melatonin assays including (but not limited to) chemically impregnated test strips designed to react in accordance for the presence or concentration of melatonin and then have option for said test-strips to be automatically scanned and analysed as a means of outcomes to be indicated or communicated);
- the **incorporation of an algorithm** capable of providing a **clinical utility or personalised subject/patient/user health monitoring or tracking capabilities** including the use of (but not limited) the sleep midpoint (by way of sleep/wake monitoring capability, and so long as sleep is not too disturbed, as detailed anywhere in this document) deployed as a surrogate but reasonable measure of approximated **circadian** phase cycle determination;
- the **incorporation of an algorithm capable of providing the calculation of the time of therapy usage (i.e. bright light therapy)** should be applied in accordance to a subject/patient/user's internal **circadian** versus external environment clock time conditions. In this manner automatic, patient-wearable technology and/or mobile device integrated systems can contribute to the incorporation of **circadian** principles in order to enable the determination of important therapeutic treatment (i.e. alignment or adjustment between **circadian** (internal) and environment (external) clock cycles for optimal health, safety and occupational hazard minimisation. The said therapy can extend to optimising subject/patient/user room, alarm clock settings, or other environment or temperature conditions to personalised versus general conditions;
- **determination of sleep midpoint** on a single or routine basis by way of analysing sleep midpoint computation based on sleep/wake monitoring and/or MEQ, MCTQ questionnaires and/or subject/patient/user sleep journals (i.e. start sleep period time, lights off for sleep time, awakening sleep time) in order to determine an approximation of internal **circadian** cycle clock time;
- **Determination of light therapy phase-advancing treatment** or adjustment/adaptation strategy based in phase relationship and correction requirements I order to shift internal **circadian** clock to synchronise with external environment (time zone and and/or solar clock factors and/or required sleep/wake cycle), such as advancing time of **circadian** clock cycle by way of applying light therapy (i.e. activation of bright lights in room) in co-incidence with alarm clock awakening time in an automatic manner (i.e. wireless linked light activation or light controller deployment). Such process can be deployed to counter disorders such as "cabin" or winter "depression" in winter or snow-bound regions or in the incidence of non-seasonal depression etc.;

In this manner the present example incorporates an **alarm and/or sleep coaching determination** capability and, along with an algorithm designed to assist or guide people with notification of optimal rest periods (i.e. naps or recovery sleeps) the user can set preference (i.e. for a driver or pilot for the purpose of drowsiness occupational-risk aversion or mitigation, for example) in order to enhance vigilance or performance capabilities, whereby the present example enables subject/user to enter desired sleep period and selection of "best sleep recovery strategy" means (said means can associate any CC determination and/or wake, sleep, REM or arousal state with determination of wake function (i.e. alarm via audio, vibration, including vibration function within monitoring wearable device (i.e. forehead Somfit/forehead- strip etc.);
- **Determination of long-term activity (**i.e. actigraphy), sleep/wake (i.e. monitoring capabilities and/or sleep journal entries) applicable to causation and/or treatment and/or coaching applicable to depression;
- **Determination of unstable rest-activity** cycle as a marker of potential poor **circadian** entrainment and potential contribution to adverse physiological or psychological health status;
- **Determination of sleep/wake parameters** such as (but not limited to) sleep onset, wake-up time, wake bouts, sleep efficiency, mid-sleep time etc.) based on any of or any combination of sleep/wake monitoring data or associated outcomes, and/or actigraphy (i.e. accelerometer outcomes measures);
- **Determination of circadian variables such as circadian relative amplitude versus cycle time** and/or CC maximum amplitude and/or CC minimum amplitude, CC day by day stability (inter-day stability or related fragmentation factor) and/or CC daily stability (intra-day stability or related fragmentation factor);
- **Determination of circadian variables such as strength of coupling or correlation** between external CC environmental stimulus factors (zeitgebers) versus CC cycle;
- **Determination of objective verification and/or characterisation of chronotype** (time subject/patient/user goes to bed and awaken up and/or optimally requires to do same),
- Determination of objective verification and/or characterisation of **chronotype** (time subject/patient/user goes to bed and awaken up and/or optimally requires/desires to go to bed and awaken) as it relates to **special period such as exams,** celebrations, illness, associated with or following treatment; associated with or following medication; depression etc.;
- **Determination of any of or any combinations of CC processing/determination** aspects based on longer term measures or trends (.i.e. but not limited to weekly, monthly, yearly summaries or overviews) and/or shorter term measures or trends (such hourly etc.) and/or in correlation with work periods, recreation periods, relation periods etc., along with the option of indications, coaching, entrainment determinations, etc. in order to stabilise CC clock cycle and/or synchronise internal CC cycle with external environmental clock factors (time-zone; solar-clock; zeitgeber factors);
- **Stability of entrainment (i.e. dim light melatonin onset; DLMO** (marker of body clock time based on measuring saliva prior to subject/patient/user going to sleep) over successive days, week, months etc.) by way of any of or any combination of measures of environmental environmental/room light, solar light, temperature and/or physiological subject/patient/user light, temperature, actigraphy, **EEG;**
- **Environmental/room light** and/or solar light log;
- **Social zeitgebers** by way of Social Metric Rhythm Questionnaire;
- Sleep log via any of or any combination of related Questionnaire and/or sleep/wake monitoring and/or actigraphy;
- **determination of CC amplitude** by way of any of or any combination of core body temperature, melatonin sample testing, **EEG** measures, surrogate or derived or estimated body temperature measures;
- **determination of light input frequency/colour composition** and/or strength;
- **determination of sleep homeostatic characteristics** including the increase or decline short-terms or long-term wise as it **relates to slow wave sleep (i.e. NREM such as 0.75 Hz to 4.5 Hz);**
- **determination of sleep homeostatic characteristics** including the increase or decline long-term or short-term-wise **as it relates to REM sleep**;
- **determination of sleep homeostatic characteristics** including the increase or decline long-term or short-term-wise **as it relates to wake EEG;**
- **determination of sleep homeostatic characteristics** including the increase or decline long-term or short-term-wise as it relates to faster beta waves in active waking and/or **slower alpha waves in quiet waking;**
- **determination external zeitgebers applicable to sleep and wake structure aspects and/or variations;**
- **Determination of zeitgeber strength** based on degree of light subject/patient/user receives. along with associated time of day as it relates to entrainment requirements and/or current entrainment determination;
- **the present** example **can automatically incorporate all CC entrainment factors,** indication aspects, alarm clock functions, light detection functions, coaching and/or messaging and/or alert functions, into a single application as part of a wearable or mobile device;
- **The present** example **can determine circadian clock nadir factors** (i.e. body temperature and/or interval from body temperature nadir to sleep **offset**) including subject/patient/user's with delayed sleep phase syndrome (DSPS) in order to optimise CC entrainment (i.e. light therapy including glasses with blue light projected towards subject/patient/user retina as a stimulus - can be blocked form forward projection **based on shaded or blocked section of glasses** in order to minimise obtrusive or obvious nature of such treatment);
- **The present** example **can track sleep-wake rhythms** and characterise a lack of clearly discernible **circadian** patterns of sleep-wake time, as a marker or potential prognosis of irregular sleep-wake rhythm;
- **The present** example **can in incorporate sleep-wake rhythms** and characterise a lack of clearly discernible **circadian** patterns of sleep-wake time, and/or questionnaire outcomes **relating to excessive sleepiness** , unrefreshing sleep, and/or insomnia that vary in accordance to work schedule as a marker or potential prognosis of shift work disorder (SWD) ;

### [BLOCK 3A] - OPTION AN INTEGRAL OR ADJUST SPECIAL WEARBLE TECHNOLOGY, MOBILE OR OTHER SYSTEM

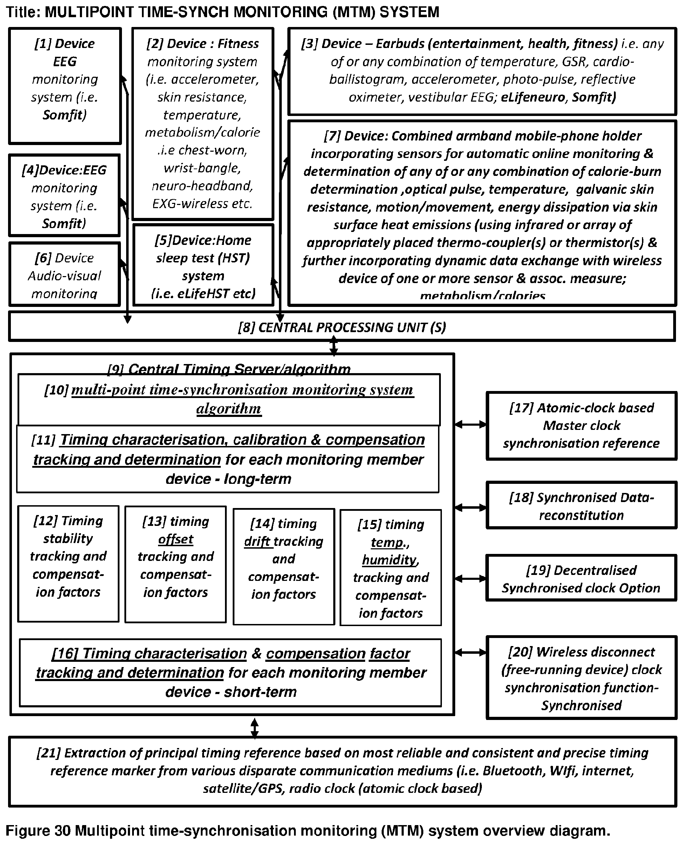

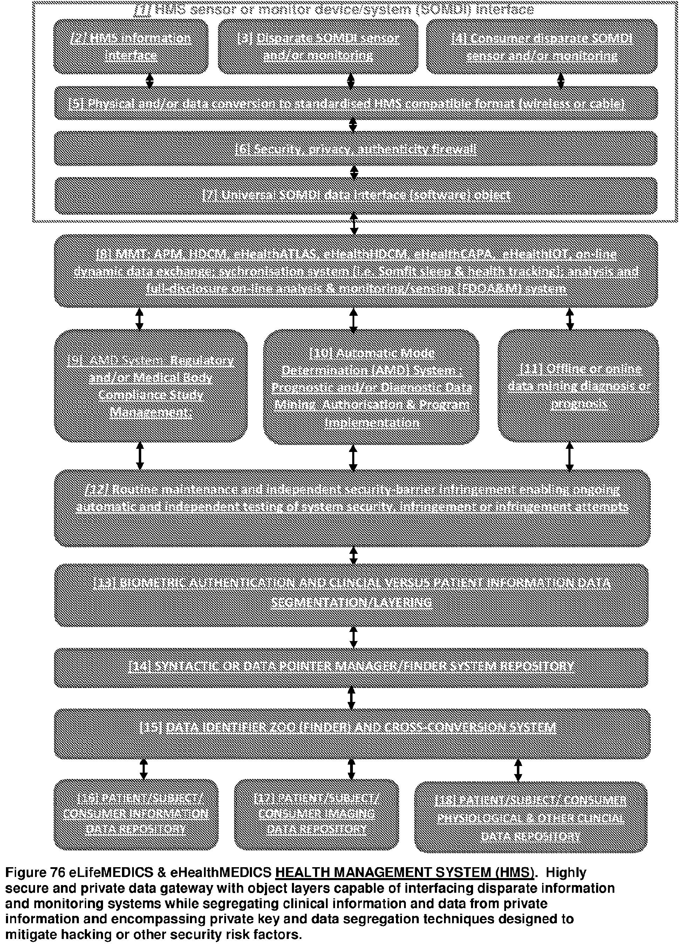

- MULTIPOINT TIME-SYNCH MONITORING (MTM) SYSTEM : ***Figure 30***
- IR reflective oculography and/or video imaging (with IR capability) and/or light therapy integrated and/or mobile CC HMS application interfaced systems ((i.e. glasses): ***Figure 43***
- **Automatic diagnostic and prognostic EEG-monitoring and analysis system incorporating minimisation process enabling professional and consumer-level monitoring and automatic analysis determination (MINIMISATION SYSTEM) per example** ***Figure 45******;***
- HMS WITH AUTOMATIC TRACKING OF EOI per example ***Figure 75******;***
- HMS sensor or monitor device/system (SOMDI) interface per example ***Figure 76******;***
- Driver/subject wearable glasses incorporating any of or any combination of binocular or monocular
- Self-learning and Personalised Self-Adaptation System AI; ES per example ***Figure 77******;*** ***Figure 78******;*** ***Figure 79******; and*/*or***
- Personalised subject/patient- health management system (HMS) [1] incorporating any of or any combination of monitoring goals per example ***Figure 80******.***

### [Block 4] CIRCADIAN CLOCK (CC) HEALTH MANAGEMENT SYSTEM (HMS) OUTPUTS INPUTS

- **Control of environmental and/or other stimulation factors capable of enabling or transition towards optimal synchronization with the environment or entrainment.** This can be mediated by way of controlling steady state or periodic stimuli (also referred to as zeitgebers) to stimulate or act upon the subject/patient/user's **circadian** clock (see also CC HMS inputs);
- **Circadian** sleep-wake cycle,
- Neurobehavioral performance,
- mood,
- cortisol,
- melatonin,
- temperature,
- heart rate, etc.

### -A computed circadian clock generating a cycle to match the solar day

- **A control or measure of control to maintain or correct (i.e. move circadian and sleep wake clock into closer synchronisation) the phase relation between the subject/patient/user circadian clock cycle (i.e.** prior sleep/wake cycle) and the current environment clock cycle (i.e. solar clock, time-zone or schedule requirements of subject/patient/individual) and/or target clock (i.e. desired subject/patient/user and/or the subject/patient/user desired or preference clock or schedule (i.e. any of or any combination of considerations including wake cycle, sleep cycle, work cycle, recreation cycle, fitness cycle, relaxation cycle, travel cycle, work-shift cycle, study-cycle, exam-peak-performance-cycle, jet-lag cycle, current time-zone cycle, one or more new time-zone cycles etc.);
i.e. but not limited to any of or any combination of:
**>>Circadian clock entrainment outputs or outcomes.** i.e. but not limited to **circadian** clock interactive (or one way information access) options (CCIO);
   - whereby said CCIO include any combination of (but not limited to) - map display or application with map functions or indications or associated annotations or information, with automatic and/or dynamic data exchange or update options;
   - clock or watch indications, with automatic and/or dynamic data exchange or update options;
   - clock or watch alarm indications or settings, with automatic and/or dynamic data exchange or update options;
   - as part of **circadian** clock entrainment stimulus or environmental adjustment decision matrix or control of environmental or therapy deployment (i.e. any of but not limited to light-therapy, with automatic and/or dynamic data exchange or update options;
   - temperature or environmental changes, with automatic and/or dynamic data exchange or update options;
   - vibration or other stimulus, magnetic stimulus, steady state or flashing "light stimulus", with automatic and/or dynamic data exchange or update options;
   - whereby said "light stimulus" can include (but is not limited to room or glasses or other head mounted/applied system including concealed steady state light therapy means using forward light blocking means and/or less obvious subject/patient steady state light and light colour selection or light-colour control properties;

   >>the present example includes any combination of or any of motion tracking (such as actigraphy or accelerometer measures), body temperature, GSR measures, pulse measures, light measures as a **means of approximating or determining and/or indicating a subject/patient/users circadian clock cycle;**

### [BLOCK 51- CIRCADIAN CLOCK (CC) HEALTH MANAGEMENT SYSTEM (HMS) OUTPUT- MAP-LINKED FUNCTIONS

**The capability to automatically access a travel agenda**
- **integrated mapping application** (i.e. geographical or road map) an indications or annotation, with the option of additional notes or associated information, relating to various travel scenarios indicating or symbolising;

### [Block 6] CIRCADIAN CLOCK (CC) HEALTH MANAGEMENT SYSTEM (HMS) OUTPUT-SOCIAL/PROFESSIONAL/COMMUNITY-LINKED APPLICATIONS OR FUNCTIONS

The present example enables a *range of clock or watch face programmable functions,* including a sequence of watch displays which can be *toggled through and selected* as *required.* For example, any combination of solar-cycle, sleep-wake cycle, time-zone, social-clock, and associated phase shift with **circadian** clock can be programmed by users, presented as default library of displays and/or programmed display formats shred amongst different users.

The CC HMS enables a community or private select grouping function whereby healthcare specialists can coach, guide, assist and intervene in tracking, diagnosing and supporting an individual's occupational safety aspects, sports performance aspects, general health aspects, depression and other psychological disorders greatly impacted by appropriate CC management *(**Figure* 96.; *Figure 97**).*

### [Block 7] - CIRCADIAN CLOCK (CC) HEALTH MANAGEMENT SYSTEM (HMS) THERAPEUTIC/CONTROL/FEEDBACK/BIOFEEDBACK

- determination **of associated framework and recommendations and/or biofeedback** and/or control for associated treatment via therapeutic treatment or related **circadian** rhythm adjustment including neural magnetic stimulation targeting and dosage determination and/or light therapy stimulation targeting and dosage determination and/or room temperature targeting and dosage determination
- **provision for the determination and/or enabling of a number of entrainment scenarios such as the intervention with subject-wearable device or environmental lighting** as a means various degrees of advancing or delaying a subject's phase response curve (i.e. a subjects inbuilt **circadian** curve phase relationship with external clock factors, including social, time-zone, work, work-shift, study requirements etc.) applicable to minimising delayed sleep phase disorder (DSPD) or advanced sleep phase disorder (ASPD) according to a subject's health- care oversight or intervention and/or an individual's personal preferences or requirements and/or occupational hazard and safety considerations;
- **automatically or via manual assistance activate light intensity and type** (i.e. visible blue light with short wavelength, and stronger melatonin suppression affect can be deployed as part of an automatically computed CC entrainment treatment regime, versus longer wavelength light) as well as the timing functions of such light therapy (i.e. light-therapy in the evening can enable CC phase delay, while light therapy in the day can product CC phase advancement);
- **automatically (or with manual intervention option) control entrainment factors** (i.e. lighting timing and/or lux intensity and/or melatonin dosage and administration timing or recommendation), as well as the option of recommending or setting bed-times or alarm clock settings, in accordance to a subject/patient (or healthcare advisor) social, work, travel requirements or environmental factors;
- the present example can **advise/coach and/or automatically adjust said CC entrainment in accordance to subject/patient/user preferences,** selections or personalised scenario choices (i.e. more aggressive adjustment over a shorter period of days or more moderate CC adjustment over a longer period of days);

### [Block 8] CIRCADIAN CLOCK (CC) HEALTH MANAGEMENT SYSTEM (HMS) OUTPUT-PERSONALISED HEALTH MANGEMET AND COACHING-LINKED FUNCTIONS

- **capability to measure environmental lighting conditions applicable to subject/patient/user** (i.e. via wearable device such as watch, mobile device etc.) in order to provide coaching or guidance to treat winter depression or other forms of depression or delayed sleep phase disorder (DSPD), or compensate for **offset** between CC and environmental (i.e. time zone or solar clock factors or behavioural clock properties (i.e. social clock, work-clock, shift-works, travel/jet-lag clock, clock and associated requirements or planning/scheduling preferences), whereby said coaching can include CC **offset** therapy (i.e. light therapy, melatonin medication, adaptation of homeostatic sleep factors (i.e. optimal increasing of a subject/patient/user's awake period to enable higher quality sleep and alignment of CC with sleep patterns or vice versa);

### [Block 9] CIRCADIAN CLOCK (CC) HEALTH MANAGEMENT SYSTEM (HMS) INTEGRATED (INTERCONNECTED) APPLICATIONS AND NOTIFICATIONS

- **determine, predict and indicate (i.e. optional calendar application annotations;**
- **scheduled or hypothetical schedule, travel itinerary, natural circadian clock cycles, homeostatic sleep/wake monitoring;**
- **determination of accuracy or confidence level in terms of the accuracy** of computing individual's previous and up-to-date **circadian** clock cycle status, along with other factors impacting the **determination of current circadian clock status** of an individual (i.e. based on prior sleep/wake study data quality and availability, along with atigraphy and other availability of **circadian** clock related measures;
- incorporation of CC computed parameters as part of automatic **entrainment programming of CC treatment systems** (i.e. such as bright light therapy including glasses or sunglasses (i.e. half-shielded glasses, for example only, tinted in upper section of glass lenses only), **whereby said glasses can include reflective oculography capable of both entrainment light-therapy** and/or detection of **eye-lid movements** and/or opening as a **marker of drowsiness** in order to enable biofeedback entrainment capabilities in order to adjust for CC cycle **offset** factors and/or sleep propensity and/or sleep urge factors;
- **determination of CC confidence level factors (i.e. accuracy - i.e. error factor)** in terms of the accuracy of the determination of the individual's previous and up-to-date **circadian** clock cycle status, along with other factors impacting the determination of current **circadian** clock status of an individual); The present example enables all these functions and capabilities to be incorporated into one or more wearable or mobile devices (i.e. smartwatch, mobile phone, Somfit sleep monitoring headband and/or other covered elsewhere at any section within this patent application document ***(******Figure 1******);***
- **automatically link (i.e. wireless or other interconnectivity communication** and information access means) to messaging systems (such as mobile phone SMS, emails, calendar, applications and the like) in order to track and/or comment/health-coach and/or enable sleep scheduling;
- **integrated calendar or scheduling/planning application(s)** (i.e. geographical or road map) an indications or annotation, with the option of additional notes or associated information, relating to various travel scenarios indicating or symbolising;

### [Block 10] CIRCADIAN CLOCK (CC) HEALTH MANAGEMENT SYSTEM (HMS) DISPLAY INDICATIONS

- A **nub of the present personal health management system is to automatically determine and indicate, coach, alert, message, CC entrainment stimulation** applicable to subject/patient/user based on CC input factors, a range of scenarios , covering sleep quality and length relating to the interrelationship or the manner the said subject works with or against their natural CC;

### Figure 97

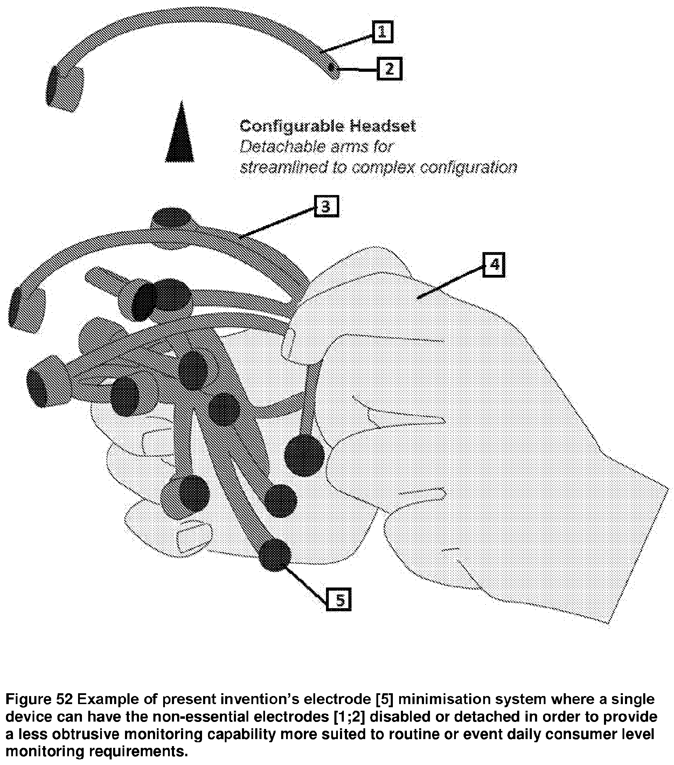

The present example provides entrainment adaptive monitoring (EAM) system comprises of 4 stages including: **STAGE 1** providing initial monitoring and analysis goals (i.e. Goal determination of ***sleep*/*wake process (SWP)* versus** ***circadian process (CP)** applicable to* ***subject*/*patient*/*user Work* and *Lifestyle Personalised-Preferences (W&LP; see also monitoring aims per minimisation process in*** **Figure 45*****)*** and any applicable ***therapeutic*/*biofeedback Personalised-Preferences (TP)*), STAGE 2** including wearable technology customisation/minimisation, **STAGE 3** Wearable technology customisation/adaptation (see also **EEG** monitoring sensor adaptation per **Figure 52****), STAGE 4** incorporating analysis review determination (see also self-learning algorithm with artificial intelligence or expert system analysis processes per **Figure 77****;** ***Figure 78******;*** **Figure 79****)** Entrainment or therapeutic determination.

The Sleep/wake/**circadian** entrainment adaptive monitoring (SEAM) system comprises of 4 stages including: Stage 1 providing initial monitoring and analysis goals (i.e. Goal determination of ***sleep*/*wake process (SWP)* versus** ***circadian process (CP)** applicable* ***to* *subject*/*patient*/*user Work* and *Lifestyle Personalised-Preferences (W&LP)*** and any **applicable *therapeutic*/*biofeedback Personalised-Preferences (TP)*),** Stage 2 including wearable technology customisation/minimisation, Stage 3 Wearable technology customisation/adaptation, Stage 4 incorporating analysis review determination, 5) Entrainment or therapeutic determination.

The upper section presents a **circadian** and homeostatic integrated
sleep/wake/work/recreation/relaxation management (CHASM) system, the middle section presents a fitness health management system and the lower section presents a neurological health management system.

***The basic entrainment adaptive monitoring (BEAM) system comprises of:***
1) Initial Monitoring and Analysis Goal Determination (i.e. per **Figure 45** aims & initial stages) , followed by;
**2) Wearable Technology Customisation/Minimisation** (i.e. per **Figure 45**) for the establishment of monitoring and analysis parameter configurations (i.e. self-learning per experts system and Al per figures **Figure 77****;** ***Figure 78******;*** **Figure 79****),** followed by;
**3) Adaptation Determination (i.e. per** **Figure 45** **showing example of adapting wearable technology EEG electrode system) , followed by;**
**4) Analysis Review Determination (i.e. self-learning per experts system and Al per** **Figure 77****;** ***Figure 78******;*** **Figure 79****), followed by;**
**5)** Entrainment or therapeutic Determination **(****Figure 96****.;** **Figure 97** therapy/biofeedback examples);
**6)** a return to step 1) or step 2) subject to effectiveness of process outcomes (i.e. statistical assessment per **Figure 45****)** and so on.

***Figure* 1** **LEFT TOP:** figure presents example of a subject/patient wearable neuro-sleep/fitness/health management systems comprising applied forehead sensor (Somfit) using headband attachment [2], eLifeBAND (arm phone/entertainment/device holder) [9], eLifeWATCH [13], **RIGHT-TOP:** sleeping subject with eLifeCHEST [3], eLifeWATCH [5], eLifeWRIST [8] and Somfit [1] devices **LOWER RIGHT:** eLifeWATCH with sensor monitoring platform. **LEFT LOWER:** forehead applied sensor incorporating self-adhesive attachment without requirement of head band attachment.
***Figure 2*** wrist (eLifeWRIST) with monitoring and health/fitness/sleep function or performance indicator.
   In this example the display indicator can be programmed to indicate, for example, sleep/wake factors on the left-hand side of display dial (i.e. between the typical 6 o'clock and 12 clock dial range, whilst the right hand side of the 6 to 12 o'clock range can be programmed to indicate daytime fitness and other health parameters. In one example, the system could be programmed to indicate overall sleep quality based on, for example, the subject/user achieving at least say within 20% of the total REM sleep time and non-REM deep-sleep time (say total of N2, N3) compared to the normal quality sleep requirements (i.e. can be based on any of or any combination of: a) normalised population averages and associated comparisons, 2) subject-specific outcomes using sleep quality tracking surveys/questionnaires, 3) monitored sleep/wake parameters, 4) monitored **circadian,** 5) local environmental factors such as tie zones or related shifts, **circadian offset** factors. Additionally, user may toggle through indicator modes such as sleep/wake goals (i.e. sleep/wake target for quality sleep tracking) or actual outcomes (i.e. actual sleep/wake outcomes including sleep-deprivation, **circadian** delay factors etc.) versus goals. Similarly user/subject can toggle through indicator modes such as fitness goals (i.e. steps, motion, activity etc.) versus relates goals. The toggling or switching through display modes can be activated via gesture or tapping/shaking means i.e. detected by ***Somfit module*** on-board accelerometer). Similarly all these functions can be integrated as part of a smart or computer-based watch system. A high level user interface graphic drag and click type application can allow user to program their ***somfit module indicator system*** and/or the compatible computer based watch-face indicators functions or cover these and other measures.
***Figure 3*** headband Somfit/eLifeNEURO with forehead **EEG**/EOG/EMG, LDR and/or motion health tracking capability
***Figure 4*** headband (eLifeNEURO) example of monitoring electrode configurations
***Figure 5*** Diagram of sleeping subject with range of wearable companion health tracking devices.

### Title: eLifeWATCH

**Patient wearable smart health watch device incorporating any combination of:**

### STANDARD eLifeWATCH MODEL SPECIFICATION

- Integrated ***environmental light*** detection sensor
- Integrated ***microphone sensor* (RHS:**
- **Figure** 7) with option for enhanced watch lens coupled sound dish function;
- Integrated **pulse pressure sensor;**
- Integrated ***photo plethysmography*** system with oximetry plethysmography option
- **Integrated temperature sensor; (****Figure 1****;**
- **Figure 7****);**
- ***Motion*/*tremor detection system*** with, fall-detection and capability patient posture detection capability;
- ***GSR sensors*** with accompanying second wrist band option (for wrist to wrist GSR function)
- Unique ***configurable monitoring sensor platform*** whereby modular rear watch panel supports a vast range of embedded monitoring smart watch sensors and systems

### OTHER eLifeWATCH MODEL SPECIFICATIONS

- Configurable display parameters
- Waterproof rated
- 1 week charge capability with standard smart watch mode
- 3 day 24-7 day-sleep eLifeWATCH all-channel acquisition mode

### eLifeWATCH PLATFORM SPECIFICATIONS

- Community shareable eHealthMEDICS App free or purchased App shop display options and reporting programs
- Scientific, developer SDK and technical community shareable eHealthMEDICS App free or purchased App shop display and reporting programs
- Personalised "opt-in" health community Apps for Android, Apple, PC or mobile wireless devices
- Sleep 360 SAAS including SAAS including cloud-computing services or NAS
- Neuro 360 SAAS including SAAS including cloud-computing services or NAS
- Ultrasonic Doppler bloodflow **(****Figure 9****)**
- 360 SAAS including cloud-computing services or NAS
- Cardio 360 SAAS including cloud-computing services or NAS

***Patient wearable smart health watch device incorporating any combination of:***

### • OTHER SPECIFICATIONS

- interface to wireless network such as (but not limited to network application services (NAS), SAAS including cloud-computing services or other network or point to point interconnection;
- temporary and/or removable storage capabilities;
- wireless gateway/interconnection-capability with wearable chest or abdomen band
- wireless gateway/interconnection-capability with wearable device (such as but not limited to band or watch);
- One or more motion or movement detection sensors or systems, incorporating capability for ***linear (i.e. spectral analysis) and non-linear (i.e. spectral entropy or associated complexity analysis) for enhanced distinguishing and classifying of neurological, nervous and*/*or muscular system disorders*** and associated symptomatic vibration or movement "footprints";
- ***Psychological state-linked tremor and*/*or vibration and*/*or motion analysis*** to enable enhanced diagnostic classification of sleep, wake, cardiac, respiratory, neurological, nervous and/or muscular system disorders and associated symptomatic vibration or movement "footprints";
- Integrated function (per above motion detection) or separate posture detection and/or ***fall-detection and*/*or step or run status and*/*or other gate parameters;***
- Unique option for ***Gyrometer*** to enhance posture/position/fall-detection;/gate tracking capabilities;
- Option for ***geopositioning*** system (**GPS**) capable of assisting with geographical locational determination;
- Option for One or more integral ***electrocardiograph (ECG) sensors*** (such as carbonised rubber sensors);
- ***One or more light sensors*** with option of ***smart watch light combined light analysis function*** for enhanced environmental light determination (i.e. sleep statistical analysis outcomes);
- One or more ***respiratory*** inductive plethysmography and/o piezoelectric and/or PVD respiratory sensors or sensor bands;
- Option for body ***EMG sensor and monitoring*** via ECG or separate sensors, with option to provide effort measures associated with breathing effort and delineation of central versus obstructive monitoring and event determination;
- Option for ***plethysmography oximeter*** module and associated analysis enabling cardiac functions including PTT, pulse wave amplitude, Pulse arterial tine, pulse transient oscillation amplitude, oximetry ***(******Figure 6******;******Figure 7******;******Figure 9******;*** ***Figure 13******);***

***Increase Acquisition and Analysis and Complexity without Conventional Data and Analysis Shortcomings:***
- ***The present*** example ***incorporates*** processing capabilities whereby extensive or additional processing requirements can be undertaken by means of distributed or parallel processing systems including (but not limited to) accompanying processing systems, interface to wireless network such as (but not limited to) ***network application services (NAS),*** cloud-computing services or other network or point to point interconnection **(****Figure 13****).**
- Unique processes have been developed to ensure ***data remains synchronised*** with other related ***simultaneously monitored information*** (i.e. subject's physiological parameters; audio and video of subject) , interrupted or misaligned time-synchronised or time critical monitoring data can be constituted automatically and data is time stamped in such a manner that data integrity issues reconstitution status are always evident to user so that incomplete records or misaligned data, along with associated diagnostic ambiguity and risk of misinterpretation can be avoided.

***Essential oversight and health-community involvements to ensure the accuracy and diagnostics of such processes the potential risks of misdiagnosis:***
- The present example incorporates health-carer "opt in" functionality.

- whereby "Opt in" Refers to the capability to allow access by other parties, subject to special medical authorisation. I.e. said "special medical authorisation" can, for example, involve online verification or clearance and confirmation that a medical practitioner is legitimately registered register in order to confirm the legitimacy and qualifications, registration etc. of a medical practitioner's current and acceptable registration status with the official relevant register(s).

In this manner the present example enables the system user to opt-in (enables approved health-care workers to be designated and authorised by the system user), in order to ensure only user selects who can access what data, thus ensuring privacy and data security. I.e. users can request medical "op-in" or "Link-in" status for their personalised "health-network" (i.e. general practitioners, dentists, chiropractors, osteopath, chiropodist etc.).

Once subject/patient/user has "opted" in their personalised health-network group, "opted" in where and who health alerts and messages should be transmitted, opted in what calendar appointments should be automatically configured in personalised calendar, opted in what scheduling should be automatically configured in personalised schedules, opted in what medical record systems are authorised for interface or data access (i.e. personally controlled electronic health record (PCEHR) and opted in what other management systems (mobile phone, smart-watch etc.) can be accessed or configured as part of the present example's interconnectivity options.
- Wearable band (for head, body, any body extremity etc.) with integrated fitness motion and genuine sleep diagnostic monitoring capability
- An example of the screen displays includes a watch mode display screen with single eLifeWATCH menu button activation including 4 application display screen and a home sleep test (HST) set-up display screen **(****Figure 6****;** **Figure 10****).**
- Example of watch-body modular sensor platform system incorporating PHOTO-PLETHYSMOGRAPHY and/or OXIMETERY PLETHYSMOGRAPHY and/or TEMPERATURE and/or spring-pressure-loaded or fixed ELECTROPHYSIOLOGICAL (i.e. conductive rubber) or SKIN GALVONIC SESNOR (GSR) monitoring electrodes and/or DOPPLER ULTRASOUND MONTORING and/or TONONOMETER MONITORING (vascular monitoring) sensor system and/or LIGHT DETECION SENSOR and/or MICROPHONE sensor **(*****Figure* 7****).**
- Example of 3-step eLifeWATCH HST study process **(****Figure 8****):**
   - **STEP 1:** Go to AMAZON or ***eHealthSHOPCART*** and click to buy ***eLifeWATCH*** for your ***personalised healthcare everywhere*** with inbuilt temp; pulse; activity/position; skin/GSR; pleth-oximeter; sound; light. The modular rear cover provides specialised ***eLifeWATCH*** options including Doppler/ultrasound vascular functions (patent pending), plethysmography oximeter (patent pending), vascular pressure pulse sensing system (patent pending), interstitial glucose (patent pending), blood-pressure analyser, etc. eLifeWATCH covers eHealthMEDICS current and future generation applications and services.
   - **STEP 2:** Go to AMAZON or ***eHealthSHOPCART , eHealthSENSORS ,** or **eHealth DATAPLAN*** and click to buy ***eLifeWATCH Sensor Kit** or* your special ***eHealthMEDICS*** service requirements.
   - le. US Home Sleep Test Type i), II), iii) and/or iv) or AU Level 2
   - OPTIONAL: While you are there or alternatively go to eHealth DATAPLAN
   - **STEP 3:** Go to ***eHealthYOU* or *eHealthMEDICS*** and **"opt-in"** your personalised health community.
- **STEP 4:** Charge eLifeWATCH and sensor kit (lasts 1 week with normal or standby usage) via eLifeWATCH POD **(****Figure 8****):**

### Watch-based sleep monitoring system:

- **Study Type is automatically detected and eLifeWATCH system configured for YOU**
- Simple translucent sensor kits with disposable self-adhesive electrodes (like "magic-tape" band-aids) kits are available (ie AMAZON click and buy) for different sleep, cardiac and neurology studies **(****Figure 8****).**
- 2 CM, 4CM and 8CM spaced electrode pairs come in wide (6mm) medium (4mm) and narrow (3mm) options, enabling the magnetic-aligned sensor electronic module to easily align and faultlessly snap connect, virtually invisible and with minimal obtrusion.
- Peel-backing paper off sensors, attach fully charged magnetic-aligned (patent pending) sensor modules and watch the eLifeWATCH indictors stop blinking.
- The green circle around eLifeWATCH sensor display when charged > 24hrs or red when not.
- Unique use of eLifeWATCH display (or select animated video guide) using number and colour codes locational guide system.
- Unique eLifeWATCH automatically detects sensor type and configures system (patent pending) so that the study type and system screens you need are automatically presented.
- Unique eLifeWATCH ***display SETUP mode* (****Figure 11****; LEFT)** is displayed and sensors will blink until all sensors and signal quality is acceptable and then OK mode is displayed (see **Figure 8****).**
- Scroll through screens **(****Figure 12****)** for animated video guides, helpful hints, troubleshooting, diagnosis, more detailed status etc.

### REAR WATCH MODULE SENSOR PLATFORM

The example a subject wearable device for body, head, limbs or body extremity attachment or application Including but not limited to wristband, watch or mobile monitoring, sensing or communication devices with interface capability to monitored subject and/or interface capability between wearable devices and a ***"watch-case modular sensor platform" and*/*or "watch-face modular sensor platform" and*/*or "watch-body modular sensor platform"* (****Figure 13****).**

In one example a ***"watch*/*wristband body"*** and/or ***"watch*/*wristband face"*** and/or ***"watch*/*wristband -back"*** and/or ***"watch*/*wristband strap*** and/or ***"watch*/*wristband buckle" detachable or removable modular sensor platform*** system includes a watch device containing processing capability whereby an interface between the said ***"watch-body modular sensor platform"*** enables any of or any combination of analogue, power, digital or wireless interface in order to enable a range of configurable smart watch devices with a spectrum of environmental or health monitoring characteristics, including (but not limited to) embedded, attached and/or integrated sensors (via watch-back) along with option of automatic processing capabilities as further detailed under sub-headings listed in this document including any ***health monitoring aspects*** such as (but not limited to) of sound monitoring; Stethoscope auscultation sensor, monitoring and automatic analysis, classification, tracking and detection capabilities; Acoustic noise cancellation system; Motion detection; REM sleep behaviour disorder (RBD); Pulse wave analysis (PWA) and pulse wave velocity (PWV) monitoring and analysis capability; PWA and PWV sensors; Pulse wave analysis (PWA) sensor measures; Ballistocardiograph; Position, locational and movement sensing and monitoring; Movement and locational information; ECG sensor(s) and monitoring; Light sensor(s) and monitoring; Breathing band sensors and monitoring; EMG sensors and monitoring; GSR; Cardiac function; Sleep training system; Photo-plethysmography (PPG) ( **Figure** 7**);** Plethysmography Oximetry; Pulse transient oscillation amplitude measures; temperature (

**Figure** 7**),** Energy-exertion/metabolism-monitoring (EM) as a surrogate calorie-burn measure; Physiological and/or Sleep and/or Wake measures or markers; along with other psychological states (i.e. sleep, wake).

***Environmental sensing*** (with alarm or alert or indicator or interface to mobile device associated or messaging, email, phone automated voice message and other information or communication systems, ionisation monitoring, ionisation smoke alarm, methane monitoring, toxic gas monitoring, toxic chemical monitoring and/or C02 gas monitoring, methane gas monitoring and/or thermometer. Multivariate analysis capabilities enable any combination of environmental or health variables to be analysed and generate indicators, alarms and messaging capabilities based on exceeding baseline normal or safe operational regions or any pre-determined combinations or clusters or events or health conditions or environmental conditions of interest or concern.

### Detailed Description of the Figures

***Figure 6*** eHealthWATCH incorporating programmable watch face with heath indications, health monitoring applications, monitoring setup and select applications, and sensor monitoring platform .

***Figure 7*** eLifeWATCH with health monitoring sensor examples.

***Figure 8*** Example of health wearable monitoring companion web "shop" application.

***Figure 9*** eLifeWATCH Doppler ultrasound example.

***Figure 10*** eLifeWATCH gesture or button display and menu toggle selection.

***Figure 11*** eLifeWATCH setup and signal validation example application screens.

***Figure 12*** Smart health watch gesture control interface.

***Figure 13*** eLifewatch with smartphone, sensors interfaces and wireless information access system (i.e. NAS, SAAS, or cloud-computing services).Figure 13 eLifewatch with smartphone, sensors interfaces and wireless information access system (i.e. NAS, SAAS, or cloud-computing services).

### Title: eLifeBUDS

- The present example provides a incorporates into a patient wearable device such as ear phone(s) one or more integrated (embedded or attached) sensors ***(******Figure 14******)*** capable of monitoring one or more physiological parameters, enabling the present example to function as a physiological monitor and mobile wireless device holder.
- The present example provides a sensor capable of detecting fine tremor movements ranging from cardioballistogram or tremors to coarser vibrations or movements. Such a sensor can comprise of a membrane or sensor (such as accelerometer) capable of detecting movement or motion and generating a signal or measure associated with said "movement or motion".
- The present example enables access to said information related to "signal or measure associated with said movement or motion" in order to generate directly access "information" or further "transformations or transpositions (linear or non-linear)" of said "information" in order to associate such "information" with sleep states, including REM sleep state in order to identify possible phases of physiologically generated tremor associated with incidences of rem behaviour disorder.
- The present example provides a incorporates said information relating to "signal or measure associated with said movement or motion" or RBD in the decision process or control process applicable to optimise the administration of appropriate drug therapy in order to achieve predetermined outcomes in terms of any of or any combination of minimising RBD and/or tremor conditions during pre-specified sleep or wake states.
- EMG via carbonised rubber sections of earphone inserts where conductive carbon sections of the earbuds can be positioned in a manner where skin contact and conduction of small electrical signals is possible between two or more of these carbon conductive regions (i.e. galvanic skin resistance can be determined form the measurement of constant current transmitted between two said conductive sensors as a means of determining the impedance/resistance between the two sensors, which changes with different sleep states, subject perspiration and other physiological changes);
- **EEG** including vestibular signals via carbonised rubber sections earphone inserts, where a conductive electrode such as carbonised rubber or other electrical conductive material can detect signals around the brain cochlear region (i.e. PAMR as a measure of auditory muscle responding to sound levels (for example), which can be deployed as a measure of excessive volume and potential auditory damage of a subject).
- PAMR via carbonised rubber sections earphone inserts (as described above);
- Galvanic skin resistance via carbonised rubber sections earphone inserts (as described above);
- ECG via carbonised rubber sections earphone inserts (i.e. conductive sensors as a means of determining small but detectable signal measures evident throughout the skin surface as a marker of cardiology function and heart rate variability);

**Audiology acoustic testing capabilities comprising** any of or any combination of **hearing assessments** (i.e. useful with earplug usage as continued high volume usage of earplugs, and particularly with sensitive hearing physiology amongst younger children or adults, van lead to permanent hearing damage if left unchecked or undiagnosed (i.e. the present example provides a **means of automatically enabling hearing screen tests** and audiology tracking of same within a mobile device, so that a parent or individual can be recommended for further medical assistance where signed may suggest hearing deterioration or possible risk.

The present example enables **hearing tests to be contained within a mobile phone or music or hearing aid** or any combination of same system(s), in order to provide automatic and hearing screening tests, coaching and elevated awareness, as a means of mitigating the potential of more serious unchecked hearing damage amongst children and adults, alike. The present example also has the means of utilising such hearing test outcomes as measure of auditory volume sensitivity, spectral auditory sensitivity, auditory conductive characteristics, auditory directional hearing characteristics (i.e. multiple spatially distributed speakers within earbuds can change and compensate for spatial directivity disorders) in order to automatically compensate each subject/patient-specific optimal auditory processing requirements in accordance to user's preferred mode (i.e. speech intelligibility, music listening, noisy room conversation focus, classroom audibility etc.).

The present example can provide an **audiometer function** as part of a mobile device and earplugs or headphones, whereby the **headphone or earplugs can generate a series** of sounds including frequencies bursts, frequency pips, MMN, odd-ball responses, auditory steady-state responses (ASSR) of other AEP test paradigms capable of contributing towards the assessment of an individual's hearing or attention/awareness (i.e. sedation, alertness, concentration (such as applicable to diagnosing autism spectral disorders, ADHD etc.).

A companion forehead or other **EEG head monitoring system (i.e. per, but not limited to, monitoring examples including any of or any combination of examples presented in** ***Figure 2; Figure 3******;*** ***Figure 4******;*** ***Figure 16******;*** ***Figure 21******;*** ***Figure 23; Figure 24; Figure 25******;*** ***Figure 27******;*** ***Figure 28******;*** ***Figure 45******;*** ***Figure 46; Figure 47******;*** ***Figure 48******;*** ***Figure 49******;*** ***Figure 50******;*** ***Figure 51******;*** ***Figure 52******;*** ***Figure 53******;*** ***Figure 54******;*** ***Figure 55******)*** can be used to monitor myogenic (i.e. PAMR) or neurogenic responses to auditory sound or stimuli (i.e. such as but not limited to presence of tone pips or other frequency pips, tone bursts or frequency generated test sequences etc.), as a means of assessing a **subject's auditory evoked potential (AEP) hearing performance (i.e. hearing conductivity, directional determination, sensitivity, spectral response etc.).**

The present example can provide an **audiometer function** as part of a mobile device and earplugs or headphones, the **headphone or earplugs can generate a series** of test sequence sounds including frequencies bursts or frequency pips.

The present example can include **electrophysiological sensors** attached or embedded (i.e. conductive material) to or as part of the **earplugs,** in a manner whereby the neurological and myogenic signals in the proximity of ear-plug or he cortical region of the subject can be monitored as part of an **AEP test paradigm.**

The present example can include one or more **vibration (i.e. speaker)** vibration sensor capable of vibrating at a range of frequencies in order to emulate the characteristics of a tuning fork whereby the individual can be tested sound conductivity (i.e. a measure or screening assessment of **conductive hearing loss)** versus the sensor function of the subject's auditory physiology.

The present example can include one or more vibrating probe(s) (i.e. speaker or other vibrating element embedded or attached to earplug in a manner where the probe can **vibrate similar to a tuning fork used to evaluate conductive (hearing loss)** be on sensor capable of vibrating at a range of frequencies in order to emulate the characteristics of a tuning fork whereby the individual can be tested sound conductivity (i.e. a measure or screening assessment of **conductive hearing loss)** versus the sensor function of the subject's auditory physiology.

The present example can include one or more separate and/or the **standard speaker transducer(s) used for generating** sound (i.e. embedded or attached to earplug or headphones) whereby any **combination of AEP testing** and also voluntary responses (i.e. subject taps or indicates with a user interface when they can or cannot hear certain sounds in order for ***sensorineural (i.e. caused by a problem in the auditory nerve or auditory pathways*/*cortex of the brain) hearing loss*** to be evaluated).

The present earbud or headphone system can include a means of deploying **hearing in noise (HINT)** within said system, whereby means can comprise of incorporating separate and/or the **standard speaker transducer(s) used for generating** sound (i.e. embedded or attached to earplug or headphones) whereby test paradigms comprising any of generating sound sequences in situations with both **quiet and noisy surrounding audio conditions** (i.e. competing sounds or audio sequences) can be simulated as part of current system. Additionally, the present example comprises of a plurality of speakers strategically positioned within the earplug or headphones in a manner earphone that directional sound can be simulated in order to evaluate a subject's **ability to distinguish sound from different directions** (i.e. the location of speakers at different positions with respect to the ear canal via earbuds or ear structure with headphones can tend to simulate changes in sound direction (i.e. speakers at the top, left, front, back, bottom, etc. can tend to simulate sound direction and assess an individual's neurological processing of sound direction (i.e. with aid of user interface prompting user to indicate perceived changing sound direction based on activating different speaks at different locations in terms of a **subject's auditory spatial orientation)** .

The present example can **enable HINT testing** where a number of conditions including (but not limited to) the assessment of a subject's hearing performance under a number of conditions including any of or combination of: generating sentences or sound sequences **without competing background noise,** generating sentences or sound sequences **with competing background noise,** generating sentences or sound sequences with competing background noise **with a directivity** (i.e. simulated with multiple **strategically located or position speakers within earplug or headphone system) aspect** (i.e. sound perceived as being evolving from in front of subject by way of activating similar sounds and speaker direction (i.e. same sound and speaker front positioned activation) in subject's left and right ears to simulate "centred" sound from subject's perspective), generating sentences or sound sequences with a **90 degrees left or 90 degrees right directivity** (i.e. simulated with multiple **strategically located or position speakers within earplug or headphone system) aspect** (i.e. sound can be steered from left to right by adjusting which speakers and what levels of sound are generated by **strategically positioned speakers within earbuds or headphones).** The present example provides a **means of computing the signal to noise ratio** for different conditions based on the determination of the level of loudness required to playback sentences above background noise before a subject can repeat sentences correctly at least 50% of the time. The present example can use a microphone such as the standard **in-built or earbud microphone to record and analyse correctness of subject's ability to repeat sentences** (buried or distinguished from the generation of competing background noise).

The present earbud or headphone system can include a means of incorporating ***tympanogram hearing assessment*** within said system, whereby means can comprise of incorporating (i.e. embedded within or as part of or attached to earbud(s)) one or more pressure sensors in both or either of the earbuds or headphones, in a manner that a speaker or other device (i.e. part of earbud or headphones, such as calibrated speaker transducer and/or tiny valve arrangement to generate and measure (i.e. a **calibrated pressure sensor** capable or measuring pressure in ear canal in a manner where the generated pressure and/or corresponding air generated air volume versus the resulting pressure and/or **resulting ear canal air volume and/or pressure** help to describe **tympanic membrane characteristics** such as ear drum perforation) desired pressure and pressure variation) can generate and vary a pressure in the ear canal and measures corresponding pressure or leakage of pressure in order to determine the ear canal volume and determine (for example) the function of the tympanic membrane (i.e. perforation in the eardrum).

The present earbud or headphone system can include a means of incorporating
**acoustic reflex test capability** within said system, whereby means can comprise of (i.e. embedded within or as part of or attached to earbud(s) 3 main elements (each element can include a separate tube directed at the subject's ear canal and the earbud forms a tight seal with the subject ear canal. The present earbud can contain (for example only but not limited to) any of or any combination of an air-coupling tube where said tubes can transmit sound via a speaker, another can connect the ear canal to a microphone, another can be a pressure generation pump (i.e. miniature pump within earbud) capable of generating a range of pressures typically ranging between -200 daPa to +400 daPa (1 pascal is equal to 0.1 dekapascal (daPa)), and/or another said tube can couple the ear-canal pressure to a pressure measurement transducer (whereby said tubes can be one or more combined tubes). A series of tones can be generated via the speaker and resulting impedance (via acoustic reflectometery) can be measured at the microphone. The resulting values can be used to generate a graph referred to as a tympanogram comprising of compliance or acoustic impedance corresponding to a range of pressure values. In this way the present example can include one or more separate and/or the **standard speaker transducer(s) used for generating** sound (i.e. embedded or attached to earplug or headphones) whereby one or more said speaker transducers are calibrated to generate a known sound pressure level, and where a tone (i.e. such as but not limited to a tine greater than 70 Decibel sound pressure level (DBSPL ) can be presented to subject in order to **measure of the subject's stapedius muscle** (protects the ear from loud noises, including the subjects own voice which can be 90 DBSPL or more at the subject's eardrum, for example).

The present example comprises of any of or any combination of ***sensorineural (i.e. caused by problems in the cochlear" the sensory organ or hearing),* conductive (i.e. caused by problems in outer or middle ear) hearing loss, hearing in noise (HINT), tympanogram (determination of how well the eardrum and other structures in middle ear are working), acoustic reflex test (to evaluate a subject's hearing thresholds, as well as provide information about vestibular and facial nerve function).**
- Pulse plethysmography (PPG) and outputs (see A&CD patent) ; ear ***reflectance-based pulse oximetery*** contributing to non-invasive measurement of oxygen saturation (SpO2) and pulse rate (PR), along with outputs and contribution to measures including pulse-wave amplitude, pulse arterial tone, pulse transient oscillation amplitude, PTT arousal, surrogate or qualitative blood-pressure measures, sleep stage confidence levels or probability based on vascular tonicity and autonomic disturbances
- Pressure pulse signal
- Temperature measures
- Metabolism, effort or energy exertion monitoring of individual (see also **An armband-worn metabolism-monitoring device)**
- The present example provides a incorporates a gyro-meter system capable of determining an individual tilt or angular position with reference to gravity or horizon position as a measure of health (i.e. gait, Parkinson's onset, fall-detection) or a fitness (optimal performance motion, performance, behavioural physiological mechanics, efficiency, improvement etc.) as also further detailed in this document under health conditions or monitoring detail sub-headings.
- Locational information based on GPS or derived from communications systems including (but not limited to) any of or any combination of CDMA/Code Division Multiple Access, GSM/Global System for Mobiles, Wifi, satellite, LAN, WAN and/or Bluetooth systems;
- The present example provides a position sensor system (such as "metal ball in switch cage device capable of determining an individual's posture at any time);
- The present example provides a incorporates a ***photo-plethysmography pulse sensor;***
- The present example provides a incorporates as part of the body of ear-buds (such as wireless linked music or communication earphones) any sensors including (but not limited to) sensor for the monitoring of cardioballistogram (i.e. sensitive membrane sensor system such as accelerometer), temperature (i.e. thermistor, thermo-coupler, PVDF, infrared LDR, infrared LDR and interfaced infrared LED (including LED switching for 3-dimensional thermal-imaging characterisation or mapping capabilities) capable of deploying near-field energy heat characterisation associated with an individual's effort or energy exertion or related metabolism or calorie burning rate, as detailed elsewhere in this document).
- The present example provides a enables a means of signifying to an individual wearing ear-buds of precursors to health conditions or events of interest such as cardiac events or thresholds, whereupon automated auditory (which can override music or ongoing phone audio as warranted) in circumstances when respiratory rates or oxygen saturation, body-temperature or other factors potentially detrimental to safe and reasonable physiological conditions.
- The present example provides a enables a means of signifying to an individual wearing ear-buds of precursors to health or safety conditions including determination of rail-crossings, raids or detection of on-coming vehicles which otherwise may not be noticed due to reduced sensory perception related to factors such as phone calls or music, whereby the present example can override music or ongoing phone audio as warranted in order to notify individual of pending or possibly pending danger. Such processes or devices can be used in conjunction with glasses or other wearable or mobile camera or audio monitoring devices.

The present example provides a **wireless-linked stereo or mono ear-buds to incorporate health management systems capable of any combination of:**
- Audio sound
- Integrated glasses with superimposed audio-synchronised video capability
- Integrated temperature sensor(s)

### Integrated oximeter

- Integrated plethysmography oximeter
- Integrated plethysmography oximeter with any information outputs including :
- Pulse wave amplitude
- Pulse transit time
- Pulse arterial tone
- pulse oximeter with plethysmography waveform
- One or more ECG signals
- ballistogram motion detection
- movement detection
- GPS system
- Gyro position detection
- Patient position detection

### Electrophysiological sensor including any combination of :

- **EEG** sensor
- **EEG** including vestibular detection
- EMG sensor
- PAMR sensor
- Light pulse reflectance detection oximeter system
- Light reflectance oximeter system
- Audio noise cancellation system
- Auditory echo monitoring system
- ER stimulation capability
- ER auditory response measurement capability
- ER auditory testing echo measurement system
- IP wireless interface capability
- IP wireless interface capability with video glasses superposition capability
- Wireless data modem capability
- Wireless mobile phone capability
- Wireless video and mobile phone capability with synchronises video superposition glasses capability

**The present example provides PHONE-HOLDER of combined phone, entertainment, health-tracking and/or hearing aid ear-buds or headphones**
The present example incorporates within **earphone buds** the capability of one or microphones in one or two subject-worn earphones whereby said **microphones can measure environmental sounds** including speech various orientations, and by way of analysing phase, amplitude level, spectral composition and **comparative characteristics between two or more said microphones** from earphones place in one or both subject's ears (as well as the option of other locations).

Thereby enabling a means of reconstituting sound in a manner where speech of interest can be processed in order to de-noise (including unwanted or background noise cancellation) and then steer focus of auditory reception (i.e. weighting different microphone sound-sources based on steering ultimate "sound source of interest" driving earphone speakers. Where driving earphone speakers of interest can include driving a plurality of speakers in each earphone and collectively across both earphones in order to **maximise spatial information** for subject and/or speech audio focus and/or spectral filtering and/or background or unwanted background noise in accordance to subjects specific auditory hearing requirements as well as personalised audio options (i.e. speech focus, music enjoyment, speech tuning in crowded or noise environments, etc.).

The present example further provides a means of **augmenting conventional entertainment** and/or health sensing ear-buds or earphones wireless or wire connected versions enabling augmentation to conventional mobile phone or entertainment audio or audio-visual applications.

Said example can be calibrated and compensated for subject-specific hearing performance using online application or in specialised acoustic environment.

**The present example provides EAR-BUDS HEALTH MONITORING INCLUDE PAMR HEARING FUNCTION)** -
The present example describes wearable audio ear or headphone(s) method or devices incorporating health monitoring capability comprising one or more electrophysiological sensors (electrodes) capable of neural and/or myogenic monitoring capabilities.

The said **neural monitoring** can include monitoring electroencephalography (**EEG**) signals, **via electrodes embedded or attached** as part of **earphone** or headphone devices, including the capability to monitor **EEG** signals, in the cochlear or vestibular brain regions. Other **EEG** monitoring can include the monitoring proximity head or scalp regions via additional wire connected electrodes.

The said **myogenic monitoring** can include monitoring **post-auricular muscle responses (PAMR),** via electrodes **embedded or attached** as part of earphone or headphone devices, including the capability to monitor electromyography (EMG) signals, in the cochlear or vestibular brain regions. Other EMG monitoring can include the monitoring proximity head or scalp regions via additional wire connected electrodes.

The present example records and analyses the post-auricular muscle response (PAMR) as a biometrics signal applicable to loud sound level responses and relevant to potentially regulating or minimising dangerous sound levels in children's or adults earphones or ear-buds or headphones and thus mitigate potential hearing damage otherwise applicable (especially to younger children with more sensitive auditory sensory systems).

The present example further incorporates as part of a mobile or wireless mobile processing device or phone incorporating a series of audiology tests via an application software and audiology test paradigms. Said ***"audiology tests"*** can comprise of any of or any combination of:
- **Pure Tone Audiometry;**
   - Air conduction;
   - Bone conduction;
   - High frequency;
   - May need to use modified pure tones (e.g., warble, pulsed) around frequencies affected by tinnitus;
   - Masking may exacerbate tinnitus, so should not be performed until after tinnitus perception assessments, if at all;
- **Tinnitus matching;**
   - Pitch;
   - Loudness;
- **Minimum masking level;**
- **Total/partial residual inhibition;**
- **Speech audiometry, which may involve;**
   - Detection;
   - Recognition;
   - Identification;
   - Discrimination;
   - Masking if required (judgment required on likelihood of tinnitus exacerbation);
- **Tympanometry;**
- **Otoacoustic Emissions;**
   - Transient Evoked;
   - Distortion Product; and/or
      - **Auditory Brainstem Response (ABR) ⁷.**

### Ear-phone PAMR monitoring background:

**The problem:** Prolonged high-level audio via earphones can adversely impact hearing and lead to permanently hearing dysfunction, particularly amongst younger infants and children.

**The Solution:** Health monitoring in the form of continuous audiology Audiology measures incorporated in the wires, attachment straps or actual earphones, ear-buds or head-phones can provide useful tracking information to prevent hearing damage due to excessive volume or excessive amplitude specific to an individual hearing response and hearing function.
The present example provides a number of audiology measures capable of minimising risk such hearing damage by way of incorporating with a hearing device (earphone(s), ear-bud(s), headphone(s)) microphone capable of measuring dB sound pressure levels (SPL) or various said ***"audiology tests"*** or related surrogate measures.

By presenting a continuous click sound to a subject (i.e. via earphones) the PAM response can be evoked and measures with electrophysiological sensors. Traditionally this signal has been discarded as unwanted artefact. The PAM response can arguably be monitored as a measure of muscle tonicity. For example, as a subject approaches states of deeper sleep or anaesthesia the overall muscle activity of the body relaxes and therefore EMG responses are suppressed. By "evoking" the post-auricular muscle signal (i.e. loud click sounds to the ear) and then monitoring the resulting "evoked response" one can further gauge a subject's depth of sleep or depth of anaesthesia. For example, as a subject transitions into deeper stages of sleep such as REM sleep or anaesthesia, the tonicity of the subject's muscles are progressively suppressed and similarly the evoked PAM response progressively diminishes. Thus evoking the PAM with an auditory click and then measuring the amplitude of the resulting PAM response ("evoked post auricular response") can provide a useful measure of sleep or anaesthesia depth. Consequently, the responsiveness and associated amplitude of the PAMR can be used as a marker of sound pressure level presented to a subject.

### Description of the Figures

***Figure* 14** eLifeBUDS battery powered wireless earphones incorporating a platform of sleep, fitness and/or health monitoring and/or analysis and/or indication or alert capabilities.

### Title: eLifeKIT

### BACKGROUND

### The Importance of Sleep

- Quality sleep is essential for all aspects of health, wellbeing, lifestyle and even zest for life.
- The **most damaging** impacts of **sleep deprivation** are from **inadequate deep sleep.**
- During **deep sleep** the body repairs itself and **restores energy** for the day ahead.
- It is the **quality** of the **sleep** time and **not** just the **number of hours** in bed that is most important.
- Sleep is made up of different stages and **each stage** in the sleep cycle offers **different benefits.**
- **Deep sleep** (Stage N3) and **REM sleep** are the **most important stages of sleep.**
- About 50% of total sleep time is spent in Stage 2 sleep, **20% in REM sleep,** and **30% in the remaining stages,** including **deep sleep,** for the normal adult.
- **Sleep debt** is the difference between the amount and quality of sleep you get versus your needs.
- Sacrificing sleep adds to your sleep debt.
- Eventually, **sleep debt** must be **"repaid"** in order to rebalance **"sleep-account".**
- **Sleep debt** contributes to sleep urges during the day, leading to lapses in attention and reduction in daytime performance including transport **accidents,** medical errors, falls and other mistakes, mishaps and safety risks..
- Reliable or effective sleep monitoring cannot be achieved using traditional mainly wrist monitoring system, but rather requires monitoring of the brain, muscle tonicity and eyes.
- Insufficient deep sleep adversely impacts metabolism and **weight, memory** recall, **energy** levels, **occupational risks, immune** system including the body's ability to suppress dangerous cells or even fight **cancer,** and indeed our overall state of health and **life-quality.**
- **Amongst children,** poor quality sleep has been linked to **low IQ and behavioural disorders,** whilst amongst women with sleep disorders the unborn foetus health and even life is at risk with serious disorders such as hypertension or **preeclampsia.**
- Sleep deprivation leads to **moodiness, impatience, irritability, lack of concentration** and feeling or tiredness and general apathy.
- Sleep disorders have been linked to cardiovascular health, such as increased stress hormone levels, hypertension, irregular heartbeat, and congestive heart failure.

In terms of sleep disorder co-morbidities the **majority of sufferers of drug resistant hypertension [1], obesity [2], congestive heart failure [3], diabetes type 2 [4], stroke and transient ischemic attack [5]** also have sleep disordered breathing.

### Problem with Traditional consumer-level health monitors

- **Quality or healthy sleep** is dependent on sufficient **deep-sleep and rapid eye movement (REM) sleep** (a.k.a. dream sleep) and not just time the time spent sleeping.
- Other consumer monitoring devices such as Fitbit **"is not a reliable device for the estimation of sleep-wake patterns and sleep quality, significantly overestimating wake and underestimating sleep-efficiency."** ⁸.
- Previous attempts such as ZEO relied upon unreliable and uncomfortable head-band pressure fit sensors and un- validated indices.
- Traditional health trackers **claim** sleep monitoring and **sleep quality tracking** but simply stated this cannot be achieved without monitoring **brain, muscle and eye measures.**
- Without **routine deep-sleep and REM sleep tracking** we cannot track the consistency or enduring effects of **sleep quality.**
- Moreover, without user-friendly and simplified consumer-accessible sleep tracking the average consumer cannot reliably access or manage their own **personalised sleep** and arguably **life-quality, in general.**
- **Without effective sleep quality monitoring** we have little idea of the **quality of our sleep,** let-alone the impact this is having on our **daytime performance, mood, occupational risks and** overall lifestyle **and health status.**
- **Without effective sleep quality monitoring** we cannot effectively track our personalised **sleep-debt** or the **depth or daytime sleep urges,** let-alone understand the associated **risk to ourselves and others.**
- Wit⁹hout **effective sleep quality monitoring** we cannot track the **cause and effects of avoidable sleep disturbances,** such as environmental noise or other conditions.
- **Without effective sleep quality monitoring** we may miss early signs of sleep disorders which could be reported to our doctor for the **early intervention to potentially avoid more severe health conditions.**
- Without **effective sleep quality monitoring** we risk not tracking the causes and effects of sleep quality changes with **age, sleeping environment, health conditions and stress.**
- ***eHealthMEDICS*** solution incorporates a ***eLifeCHEST*** chest-worn band with a "companion" ***eLifeWATCH*** smart health watch, unique network application services (NAS) high-dependence data management (HDCM) system ⁹(RFM14935), and a special ***eLifeSLEEP*** forehead-attached, self-adhesive wireless electrode array capable of enabling traditional fitness or activity tracking and professional-level sleep monitoring, alike.
- Monitoring of **day-time activity and breathing** together with night-time diagnostic sleep and breathing is now possible via a single integrated platform suitable for both consumer-level and professional-level health management.
- **Night-time, day-time and sleep-breathing** can be monitored continuously and seamlessly via a single eHealthMEDICS chest-worn fitness/sleep system.

### eLifeSLEEP

- Enables **routine deep-sleep and REM sleep monitoring to** allow ongoing tracking and investigation of the enduring effects of **sleep quality.**
- **Novel** integration of online tracking of wrist-based **sleep quality and sleep debt indices (1 to 10)** together with **conventional fitness level** and goal measures. Sleep quality and sleep debt based on validated "gold-standard" sleep measures and indices.
- Simple but sophisticated to enable consumer-friendly **consumer and professional-level** monitoring suitable for tracking not only the amount of time spent sleeping but most importantly the quality of sleep - i.e. adequate **deep-sleep and rapid eye movement (REM) sleep** (a.k.a. dream sleep) .
- Uses professional-level ("gold-standard") medically-proven format monitoring approach and associated self-adhesive, disposable-sensors (minimises risk of cross-infection).
- Sensor system enables continuous monitoring of "gold-standard" **sleep parameters** covering **brain, muscle and eye measures.** (i.e. **EEG, EMG, EOG)** enabling automatic online determination and display of **deep-sleep** and **REM sleep,** along with associated **sleep architecture** and **validated indices** such as sleep **efficiency.**
- Enables the determination of **sleep quality,** applicable for achieving optimal levels of **daytime performance, mood control, occupational risk mitigation, and an overall high quality lifestyle and health status.**
- Enables **sleep quality monitoring** and incorporates an associated **personalised sleep journal** with **automated sleep-debt tracker** to help manage the **depth or daytime sleep urges** and contribute to associated occupational safety aspects.
- Incorporates a novel means of tracking the **cause and effects** of **avoidable sleep disturbances,** such as environmental noise or other conditions, including online automated **sleep disturbance event cause, effect and preventative action (CAPA) analysis.**
- **Enables effective sleep quality monitoring** to help your personalised health-management and also assist your doctor or health specialist with **early intervention to potentially avoid more severe health conditions.**
- Enables **effective routine sleep quality monitoring** to help your personalised health-management and also assist your doctor or health specialist in concert with sleep quality variations due to changing factors such as **age, weight, fitness, sleep position, alcohol consumption, sleeping environment, health conditions and stress.**
- **Unique biological synchronised sound discrimination capability whereby** respiratory and sleep disturbances can be automatically analysed and classified online and presented as a simple **"sleep progress" CAPA** personalised sleep tracking. Provides sleep disturbance journal complete with suggested or likely causes with informative hints for improving sleep. i.e. i.e. one can track personal versus partner's snoring disruption impact and enable online CPAP traceability (i.e. **online sound disturbance playback)** enabling the ability to identify disruption source (i.e. abnormal events such as external noise including cars, doors banging, street noise, or personal disruption such as snoring or partner disruption such as snoring or coughing etc)⁹(RFM14935).

### CAPA determination and countermeasures

The present example provides a enables a user to ***view their sleep patterns,*** hypnogram, architecture or other sleep summary with the incorporation or association of event markers representative of sleep or breathing disturbances, whereby the user can activate event marker in order to establish corrective action and preventative action (CAPA) information in order to assist with the management and ultimate reduction of sleep disordered or disturbances or improvement of sleep quality. Said "activation" includes (but is not limited to) replay of sound segments (i.e. corresponding to sleep disturbances) presenting monitored individual or health-carer to understand whether sound was of likely biological nature (i.e. biologically synchronised with subject monitored signals or of biological nature but related to other individual's such as snoring partner) or other (street or house noises etc.).

***CAPA includes the*** means of automatically or manually deployed means of optimising or adapting settings of a therapeutic device or changing environmental conditions (i.e. temperature, room curtains or blinds can automatically be adjusted to block or reduce sound noise interference etc.) in order to improve sleeping or treatment conditions (controlled at a later time or online automatically, or controlled via health-carer remote intervention).

The present example (Somfit bangle) incorporate ***measures, goals*** (i.e. target or normal functional range) ***and*/*or indications of any of or any combination of sleep*** (i.e. ***sleep debt, sleep efficiency, sleep architecture, REM sleep, deep-sleep, wake after sleep onset) and*/*or fitness (steps, movement, mapped pathways, etc.)*** and/or the onset or incidence of health events or clusters of events (i.e. idiopathic RBD, Parkinson's, Epilepsy, seizures, Alzheimer's, Autism and other neurological, nervous system, and muscular or sleep disorders) of interest whereby a) same band can be interchangeable as head or wrist attached system, b) separate attachable systems can be deployed, c) automatic dynamical data synchronisation between head and/or other body monitored physiological processes or **sleep parameters** so that watch, wrist, mobile device or other related information access system can be continuously and efforts updated incorporating total cluster/group of physiologic monitoring systems and related data.

The corrective action preventative action eCAPA function provides a means of adapting or adjusting any of or any combination of:
- means of medication advice or recommendation based on CPAP outcomes - i.e. based on determination of a subject/patient's natural **circadian** clock/rhythm versus the current or required subject/patent's **circadian** clock/rhythm versus the **offset** between these said different **circadian** clock/rhythms, along with coaching and/or recommendation/advice and/or alarm clock or other awakening/arousal stimulus and/or light therapy/room-light-adjustment/room-curtain adjustment (i.e. increased light can suppress the brains natural secretion of ***melatonin, via the pineal region,*** while darkness can stimulate the release of melatonin. Medication capable of supressing or stimulating melatonin can be used to resynchronise or adapt the natural **circadian** clock/rhythm to the required wake/sleep or local time zone requirements. The present example enables any of or any combination of **circadian** clock linked (i.e. based on determinations based on adjusting natural **circadian** clock when it is out of synchronisation with new environment) adjustment or adaptation by of:
   - ***Medication coaching,*** recommendations and/or ***administration*/*dispensation,*** appropriate (i.e. any of or any combination of but not limited to medical, regulatory, health insurance, prescriptive and/or advisory requirements/recommendations) medication options is terms of adapting to new environment time and schedule requirements or conditions;
   - ***Medication online informational or educational access and*/*or ordering*/*supply management capabilities-*** incorporates these said capabilities subject to regulatory requirements (i.e. any of or any combination of but not limited to data security, data privacy, medical, regulatory, health insurance, prescriptive and/or advisory requirements/recommendations) in accordance to providing subject/patient options for medication options (i.e. medication to modify melatonin generation or sleep urge suppression or sleep promotion capabilities) in order to compensate for jet-lag and other causation of shifts in natural **circadian** rhythm/clock versus new environment or sleep/wake/work requirements etc. The ***online medication ordering or supply arrangement capabilities*** include, but are not limited to, **circadian** clock/rhythm modification by way of medications. For example, by way of coaching or recommending dosage and/or types and/or controlling administration (i.e. automatic drug dispensing systems) promote sleep with hypnotic medications; non-sedative hypnotics (non-benzodiazepines - i.e. zolpidem etc.), or where required a benzodiazepine (i.e. short acting types to avoid over-sedation, such as temazepan, along with the coaching hints, messaging, notifications etc. in terms of avoiding mixing alcohol with medication etc.) or otherwise recommendations such as daytime stimulants such a caffeine (along with the coaching hints, messaging, notifications etc. in terms of avoiding such stimulants after midday);
      The present example can enable automatic drug administration or vending via a dispenser device can which can alert and/or recommend and/or dispense and/or manage via tablet draw with dispensation capabilities, along with other appropriate (i.e. with option of online oversight or authorisation using appropriate regulatory, medical and or health insurance approval means) medication administration methods;
   - ***Recovery nap scheduling and management-*** the present example can_help to manage short naps (i.e. 20-30 mins) which can increase energy but not undermine night-sleep, where said management can include any of or any combination of coaching, recommendations or control changes for controls to (including wearable devices such as watches, mobile phones, alarm clocks, lighting conditions, temperature and other environmental (including electric blanket temperature etc.) and/or sleep cycle/stage awakening optimisation (i.e. as covered elsewhere in this document) in order or best manage sleep nap periods based on subject/patient required parameters (i.e. available time, preferred prioritisation of **circadian** synchronisation with new environment versus time or schedule demands, versus cognition or energy demands etc.);
   - ***means of cognitive evaluation*** (i.e. in order to track alertness and cognitive health status via approaches such as testing an individual's behavioural responsiveness, AEP/MMN etc. responses, visual responses, such as camera tracking and video analysis, of head position and/or eyes and/or blinking eyes based on interaction with audio-vidual material and comparative cognitive response measures (i.e. means of comparing subject/patient response to a specific audio-visual sequence compared to various population studies representative of calibrated mild, moderate, severe cognitive deficiency or alertness or attention or responsiveness etc.);
   - ***alarm clock, wearable vibration device*** (i.e. forehead, head, wrist, body, body extremities etc.), with ***goals and coaching recommendations*** to help adjust for travel plans and implication of natural **circadian** clock versus new environment sleep/wake and scheduled requirements (i.e. avoiding critical performance or meeting decision exposure directly after travel where natural **circadian** rhythm/clock is significantly out of synch and not appropriately compensated for, when compared to new environment sleep/wake and time zone conditions);
   - ***geographical map views*** incorporating integrated natural **circadian** clock versus asynchrony with respective time zone local environments;
   - ***exercise plans and tracking with goals and coaching recommendations*** to help adjust for travel plans and implication of natural **circadian** clock versus new environment sleep/wake and scheduled requirements;
   - ***calendar or other scheduling integration*** where recommendations, hints, warning, adjustment etc. can be presented as part of calendar notifications or recommendations/hints/messages etc. in order to help compensate for potential adverse **circadian** clock shift effects;
   - ***personal health tracking and advisory support (and linkage or association with any other circadian rhythm*/*cycle factors or control linked aspects covered here or elsewhere in this document*)** where the conventional clock, watch, mobile phone clock or alarm functions incorporate information of the asynchronous nature of the natural **circadian** clock/rhythm in order for subject/patient to be provided or to otherwise gauge based on available information (i.e. GPS, travel schedules, sleep or drowsy surveys, typical effects of **circadian** rhythm/clock shift etc.) and/or monitored information (i.e. subject/patient sleep studies, sleep patterns or related information) and/or environmental information (i.e. temperature, lighting conditions) and/or exercise (i.e. activity sensing from accelerometer and other motion or movement detection systems) and/or brain function (i.e. **EEG** monitoring of any signals related to the suprachiasmatic nucleus or associated regions providing any measures or markers indicative of the natural **circadian** clock/rhythm);
   - ***lighting control*** (i.e. light therapy) or direct generation (i.e. lamp, clock, watch etc.) related to changing environmental lighting conditions of subject/patient;
   - ***PAP or NIPPV CPAP eCPAP or other therapeutic device control*** (biofeedback) where by device can change or adapt pressure automatically eHealth based or linked Corrective action preventative action (CAPA) control or feedback (i.e. online PAP/NIPPV control adaptation for minimal sleep fragmentation, RERA arousals, and/or sleep disordered breathing and/or maximal sleep efficiency and/or maximal sleep quality and/or optimal **circadian** rhythm/clock synchronisation (i.e. with local environment).
   - ***room (or bed) temperature** -* i.e. reduced environmental temperatures can contribute to awakening or alertness versus higher temperatures which are implicated during subject/patient drowsiness or sleepiness conditions);
   - ***therapeutic means*** of adapting or adjusting-for or attempting to synchronise natural **circadian** rhythm/clock versus actual or required **circadian** rhythm/clock - i.e. an automatic drug disperser device can alert and recommend and/or dispense via tablet draw or dispensation means the recommended (i.e. with option of online oversight or authorisation using appropriate regulatory, medical and or health insurance approval means;
   - see also figures ***Figure 96*****;** ***Figure 97******;***

### (ENVIRONMENTAL SENSING COMMUNITY SHARING MAP-LINKED APPLICATION) - wearable system for automated prognostic and/or diagnostic method or device subject-wearable system monitoring environmental information

This disclosure describes a number of methods, apparatus, systems and computer applications/programs or products applicable to an environmental and health sensing and alert user-community information sharing application incorporating associated mapping and map-routing information links.

The present example enables a mobile communication device (i.e. wireless mobile phone "user device") or the first device to deploy a location application, receiving an indication and authorisation to commence sharing a plurality of location system (i.e. GPS tracking) and other relevant information from the first device, along with the transmission of such information in a format which is compatible and usable by one or more other devices incorporating an application and operator interface capability whereby one or more instances (i.e. community sharing application) of the application can indicate the location, travel routes, and "other relevant information" of the first device.

Whereby the said "other relevant information" can include monitored health information, sensed environmental information (interfaced/associated with or monitored by the first device) or other reports or user-entry/selected information or independent information reports or data dealing with any of or any combination of 1) "travel conditions and potential hazards or risk levels", 2) "Environmental monitored/sensed information", 3) "Respiratory conditions and potential hazards or risk levels" as further detailed below.

The present example enables environmental sensing information monitored via a plurality of mobile device-users to be shared so that a collective or collaborative map (i.e. a community of authorised users or application ambassadors) can share/exchange information relating to monitored or multiple user-reported ***"travel conditions and potential hazards or risk levels", "Environmental monitored*/*sensed information",*** and/or ***"Respiratory conditions and potential hazards or risk levels"*** including risks to the general population and also more vulnerable population groups such as infants and asthmatics (present example includes means of data-mining, sorting an filtering data of subject-specific health concerns and relevance).
An **automated prognostic and/or diagnostic method or subject-wearable system** (such as but not limited to watch, wristband, chest-band, headband, forehead sensor etc., earbuds) and further comprising of means of sensing signal(s), acquisitioning data, **monitoring,** storing, analysing, one or more **electrophysiological (EEG)** signals.

Said method or system further incorporates **information data-networking** (i.e. means including but not limited to LAN, WAN, IP, WWW etc.) or information telecommunication capabilities (i.e. means can be wireless mobile devices, other interconnectivity compatible wearable or companion devices, other wearable devices, wireless transmission, NAS, cloud-computing services, etc.).

Whereby said environmental monitoring can include any of or any combination of: ***Environmental sensing*** (with alarm or alert or indicator or interface to mobile device associated or messaging, email, phone automated voice message and other information or communication systems), weather elements, wind, humidity, temperature ionisation monitoring, ionisation smoke alarm, methane monitoring, toxic gas monitoring, toxic chemical monitoring, C02 gas monitoring, methane gas monitoring and/or thermometer, air pollution measures, air smoke measures., air-particles measures; radiation measures,

Whereby said "ENVIRONMENTAL AND HEALTH SENSING AND ALERT SHARING APPLICATION" includes a means of sharing information with other people with compatible application;
Whereby said ""ENVIRONMENTAL SENSING SHARING APPLICATION" can include peer to peer data sharing, data transfer, linkage to other system with compatible application loaded in terms of a mapping or navigation application indicating any of or any combination of : 1) ***"travel conditions and potential hazards or risk levels",** 2)* ***"Environmental monitored*/*****sensed information",** 3) **"Respiratory conditions and potential hazards or risk levels"*** including smog, smoke, pollution and other breathing risks to the general population including more vulnerable population groups such as infants and asthmatics.

Where ***"travel conditions and potential hazards"*** or risk levels include (but are not limited to) information from various agencies or sources or subject-monitored or community-monitored environments of a defined or authorised community of mobile or wireless mobile devices and associated users, whereby ***"travel conditions and potential hazards"*** monitoring includes any of or any combination of:
- Police alerts and safety warnings or alerts
- Department of trade warnings or concerns
- War zones and travel risk regions (including flight paths etc.)
- Flight path of proposed flight versus risk zones

Where ***"environmental monitored*/*sensed information"*** or risk levels include (but are not limited to) information from various agencies or sources or subject-monitored or community-monitored environments of a defined or authorised community of mobile or wireless mobile devices and associated users, whereby ***"environmental monitored*/*sensed information"*** monitoring includes any of or any combination of:
- Temperature
- Humidity
- Wind conditions
- Rain conditions
- Storm and other weather elements

Where ***"travel conditions and potential hazards"*** or risk levels include (but are not limited to) information from various agencies or sources or subject-monitored or community-monitored environments of a defined or authorised community of mobile or wireless mobile devices and associated users, whereby ***"travel conditions and potential hazards"*** monitoring includes any of or any combination of:
- Visibility road conditions
- Rod slippage conditions
- Road ice conditions
- Fog conditions
- Bike travel conditions
- Jogging or running track conditions

Where ***"Respiratory conditions and potential hazards or risk levels"*** or risk levels include (but are not limited to) information from various agencies or sources or subject-monitored or community-monitored environments of a defined or authorised community of mobile or wireless mobile devices and associated users, whereby ***"Respiratory conditions and potential hazards or risk levels"*** monitoring includes any of or any combination of:
- Allergies
- Smog
- Smoke
- Pollution
- Air particle concentration
- breathing risks
- Asthma risk

Where ***"Health Conditions and potential hazards or risk levels"*** or risk levels include (but are not limited to) information from various agencies or sources or subject-monitored or community-monitored environments of a defined or authorised community of mobile or wireless mobile devices and associated users, whereby ***"Health Conditions and potential hazards or risk levels"*** monitoring includes any of or any combination of:
- Disease or virus alerts
- Flu warnings
- Other validated or authorised health reports or warnings

### Healthy map routing or travel planning for cyclist, jogger or activity, comprising map-linked views with validated or authorised community user sensed or monitored data combined with associated up to date reports:

In one example is for airlines or other travel organisations to provide validation upon request of flight or travel paths so that passengers can have this cross-checked against risk factors such as updated information feeds relating to war zones etc. and present excessive risk or safe travel validation to passengers (otherwise "kept in the dark" information access wise). Similarly airline safety status and risks can be fed into safety travel model to enable individual a determining and control of their proposed travel plans.

### Healthy map routing or travel planning for travellers, comprising map-linked views with relevant, validated and authorised community user-health, environmental monitored data, along with associated up to date reports:

The present example further enables a navigational mapping or direction routing application (i.e. road, car, train, air etc.) to take into account the or environmental
Moreover, the monitored and sensed data of the said "user-device" can be shared with a number of authorised other "user-devices" and visa-versa. In this manner a so that can be transmitted , and the location data including a plurality of locations of the first device, and transmitting the location data in a form usable to enable a user interface of a second instance of a location application executing on a second device to indicate the path travelled by the first device.

### Detailed Description of the Figures

***Figure* 15** eLifeCHEST
***Figure* 16** eLifeCHEST; eLifeWRIST; Somfit;eLifeWATCH.

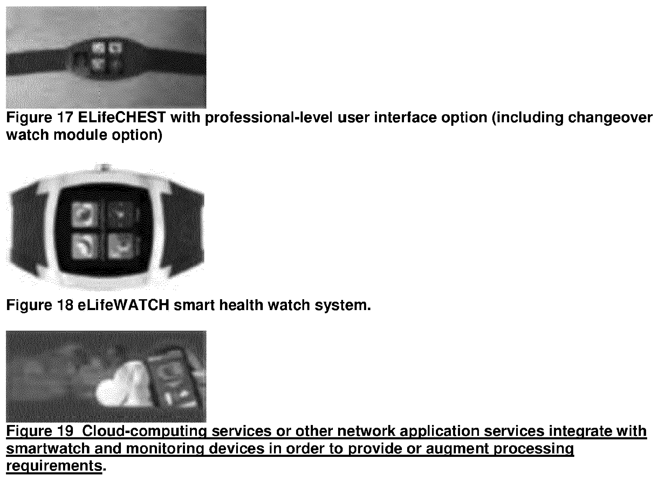

***Figure 17*** ELifeCHEST with professional-level user interface option (including changeover watch module option)
***Figure 18*** eLifeWATCH smart health watch system.
***Figure 19*** Cloud-computing services or other network application services integrate with smartwatch and monitoring devices in order to provide or augment processing requirements.
***Figure 20*** Somfit/eLifeWRIST/eLifeBANGE/Somfit.
***Figure 22*** oximeter 7-day battery charge wireless probe.
***Figure 23*** Universal wireless, battery powered bipolar eLifeEXG sytems.
***Figure 24*** Leg, EMG or other bipolar electrophysiological electrode bands.
***Figure 25*** SomniiFIT forehead (headband/forehead strip) consumer-format electrophysiological sensor array designed to enable monitoring of **EEG,** EMG, EOG, and any other sleep or neurological parameters or processes with a single electrode array (Somfit).

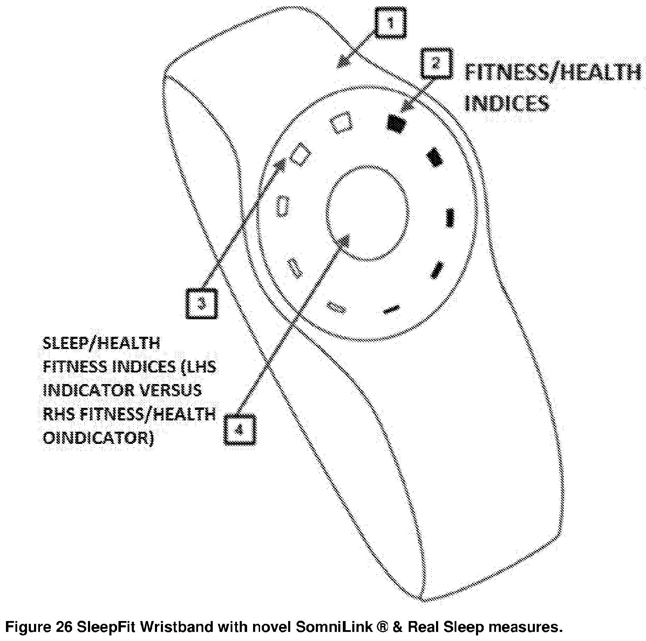

***Figure 26*** SleepFit Wristband with novel SomniLink ^{®} & Real Sleep measures.
   AS outlined in this figure the present example enables any combination of monitored or information related sleep homeostatic sleep/wake and/or **circadian** and/or fitness or activity measures, drowsiness /fatigue and/or vigilance/attention measures, in the context of pre-defined display modes covering actual performance, targets or goals versus actual measures etc.

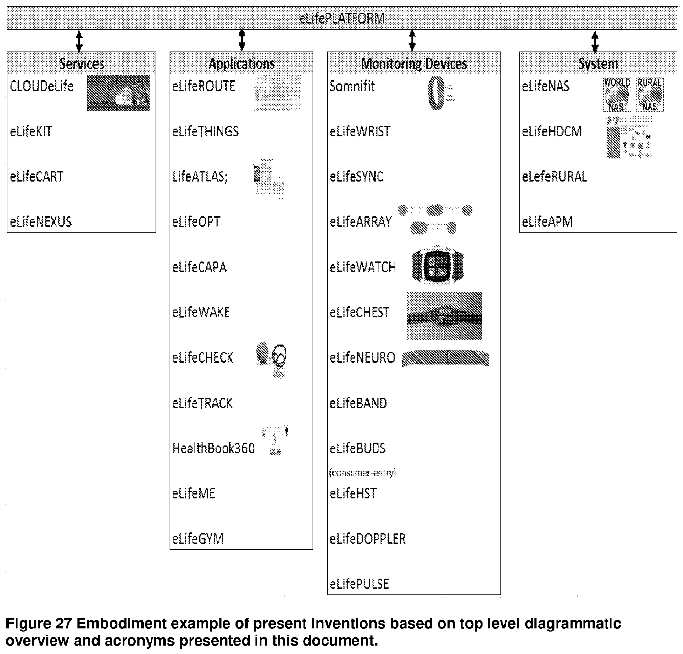

***Figure 27*** Example based on top level diagrammatic overview and acronyms presented in this document.
***Figure 28*** Patient wearable device and companion management system (HMS) platform overview flow diagram
   The eLifeMEDICS (professional medical or scientific diagnostic or prognostic services) and eHealthMEDICS (consumer level monitoring and environmental sensing with information access applicable to a unique health management system approach) comprises of and number of devices and methods deployed across a platform structured to incorporate the following attributes: service functions ***[1]*** (i.e. SAAS;cloud-computing services), applications ***[3]*,** monitoring devices ***[4]*,** analysis functions ***[5]*,** monitoring functions ***[6]*** and system resources ***[7],*** as further outlined here.
**Figure 29** External Noise Sensing and Cancellation System .

### SERVICE FUNCTIONS [1]

[1]CLOUDeLife; ; eLifeKIT; eLifeCART; eLifeNEXUS
[2]Automatic dynamic sleep parameter data exchange with wearable indicator device, enabling any of or any combination of combined sleep and fitness and/or health quality tracking

### APPLICATIONS [3]

eLifeROUTE; eLifeTHINGS;; eLifeOPT; eLifeCAPA; eLifeWAKE; eLifeCHECK; eLifeTRACK; eLifeATLAS; HealthBook360; eLifeME; eLifeGYM;

### MONITORING DEVICES [4]

eLifeWRIST; eLifeARRAY; eLifeWATCH/HST; Somfit eLifeWATCH; eLifePULSE eLifeBAND/ARM

**[4] FIG DESCRIPTION** Single device able to be forehead attached or wrist attached with sleep (i.e. **EEG,** EOG, EMG etc.), fitness or health parameters, including any or any combination of the following:

### Monitoring, Determination and Tracking of Sleep, Wake and other Mental States, Events of Interest or Health Conditions of Interest

-- The present example further deploys one or more said "subject worn ***system(s)"*** with ***device attributes*** as presented in this document incorporating one or more integrated (embedded or attached) sensors capable of sensing and monitoring one or more environmental parameters or physiological parameters, along with the determination, prediction, onset or incident of ***"events*/*measures*/*states"*** or health conditions of interest, where said examples further comprise any of or any combination of analysis or monitoring capabilities including any of or any combination of **"sleep parameters"** and/or **"neurology parameters",** incorporating means of automatic online determination of consciousness or psychological states or associated events/markers including any of or any combination of events or conditions detailed under following headings (as further detailed elsewhere in this patent application document) including under sub-headings: **Whereby the present example** comprises **sensing, monitoring, data-acquisition, signal processing, analysis, storage, and information access including the online automatic characterisation of a subject/individual's physiological, neurological, nervous system, movement system, muscular system, psychological, pathological, states, events of interest and/or health conditions including any of or any combination of the following:** Rapid Eye Movement (REM) Sleep Characteristics; Sleep Disorder Classifications; Select Sleep disorders Dreaming States; Dissociated States; Hypnosis States,
**Whereby the present example** comprises **sensing, monitoring, data-acquisition, signal processing, analysis, storage, and information access including the online automatic characterisation of a subject/individual's physiological, neurological, nervous system, movement system, muscular system, psychological, pathological, states, events of interest and/or health conditions including any of or any combination of the following:**
Rapid Eye Movement (REM) Sleep Characteristics, Sleep Disorder Classifications, Select Sleep disorders, Dreaming States, Dissociated States, Hypnosis States, SCORING STAGE WAKE (Wakefulness), SCORING STAGE N1, SCORING STAGE N2, SCORING STAGE N3, SCORING STAGE R, (any of or any combination of these states or conditions and associated detail below are part of the ***"events of interest:**"* or ***"health conditions of interest"*** referred to throughout this document), as further outlined here :
**Whereby The present example enables in terms of monitoring; sensing, monitoring, data-acquisition, signal processing, analysis, storage, information, and access includes the online automatic characterisation of a subject/individual's physiological, neurological, nervous system, movement system, muscular system, psychological, pathological, states, events of interest and/or health conditions including any of or any combinations of analysis or events or conditions of interest or further objects detailed elsewhere this patent application.**

### ANALYSIS FUNCTIONS [5]

The present example incorporates on-board (i.e. within wireless mobile device or wearable monitoring or sensing system) along with automatic adjunct (i.e. network application services, cloud-computing services, peer to peer supplementary networked processing power, LAN, WAN, internet or other access to augmented online or off-line (i.e. data-mining)) diagnostic or prognostic interconnected processing capabilities, whereby events of interest or conditions of interest outlined in this document outlined in this patent application document can be enabled along with personalised health management information reports providing access to subject/patient or other authorised or nominated (i.e. opt-in) organisations or individuals.

***In a basic example an individual can wear a wrist band*** or watch device incorporating a means of measuring and/or indicating any of or any combination of a subject/patient's:
- Monitored sleep variables or derived measures;
- Monitored fitness variables or derived measures;
- Monitored health variables or derived measures;

- whereby said means of ***"Monitored sleep variables or derived measures"*** can comprise any of or any combination of monitoring one or more **sleep parameters** vi embedded or attached sensors or via wireless connected companions sensors, for example;
- whereby said means of ***"Monitored fitness variables or derived measures"*** can comprise of one or more axis accelerometer sensor and associated analysis;
- whereby said means of ***"Monitored health variables or derived measures"*** can comprise of **measuring ECG** from one device such as a wearable watch or wrist band and then incorporating the send pair of the ECG sensing signal to be formed by way of the user touching any electrode conductive region of the second device, or in another example by way of extracting surface ECG signals from a single wrist band or other wearable device containing a pair of electrodes in contact with the subject/patient's skin and then utilising any combination of combination filtering, signal pattern and signal dynamic (i.e. non-linear dynamic) analysis techniques to extract ECG signal components from the subject/patient's skin surface. In another example where ECG signals are not discernible from background signals or noise an automatic substitution technique enabling a hierarchical analysis process whereby any number of pulse or heart rate signal sources are scanned in order to determine at any time the post reliable signal source in order to compute at least pulse rate, but heart rate variability when suitable signals quality exists, and more comprehensive ECG cardiac waveform analysis when ECG signals are available or discernible).

Alternatively ECG signal monitoring can be derived from a companion device such as chest band or wireless electrodes appropriately placed on the subject's body.
- whereby some **example of events or conditions of interest monitored,** analysed and indicated by system include any of or any combination of including measures such as gaols (i.e. target or normal functional range), sleep debt, sleep efficiency, sleep architecture, REM sleep, deep-sleep, wake after sleep onset with option of combining such measures, analysis and/or indications with fitness measures and goals (steps, movement, mapped pathways, etc.) and/or the onset or incidence of health events or clusters of events (i.e. idiopathic RBD, Parkinson's, Epilepsy, seizures, Alzheimer's, Autism and other neurological, nervous system, and muscular or sleep disorders) of interest Monitoring, Determination, and detection of onset and incidence of fitness, health, and/or associated events or clusters of events.
- whereby any of or combination of combination sleep, and/or fitness and/or health monitored and/or analysed events or health conditions of interest can be any of or any combination further detailed in **this patent document.**

### MONITORING FUNCTIONS [6]

>Online sleep progress & predictive analysis
>WEB services sleep, fitness and/or health tracking (via watch; iphone; PC; iPAD; patient worn bracelet, patient worn necklace, patient work etc.) Online sleep progress & predictive analysis
>Online eLife internet of things (eLifelOT) sleep progress & predictive analysis
>Online sleep progress & predictive analysis
>dynamic online therapeutic control system with biofeedback

### Additionally, the present example can include any combination of the following systems described in this application -

### Monitoring System Capabilities

- Adaptive Physiological-Body Network (APM) & NAS Gateway;

### Monitoring Capabilities

- *Multipoint time-synchronisation monitoring (MTM);*
- *Adaptive physiological-body monitoring (APM) system*

### Expanded details.

[6] The present example comprises of ***online sleep progress & predictive analysis:*** i.e. a method incorporating means of online **sleep progress review** whereby deep-sleep (body recovery), REM-sleep (brain restoration); sleep quality, breathing disorders (i.e. snoring, apnoea, hypopnoea, sleep disturbances, sleep arousals can all be viewed in real-time in terms of **causation** or performance.

**The present** example **further comprises of automatic online diagnosis and prognosis** of said "causation" providing immediate and on a **time-contextual** basis. For example, accessible information dealing with sleep state or progress can relate current sleep state or time in the context of normal measures for time of information access so that quality of sleep or sleep architecture (i.e. progressive deep and REM sleep performance indices **(amount of REM sleep or deep sleep based** on sleep time **elapsed versus typical or high-quality** (can be accessed via sleep survey performance assessments (such as **Epworth Sleepiness Scale (ESS))** or diagnostic assessments sleep sessions for a subject).
i.e. automatic and personalised (calibrated to an individual sleep requirements at any time and stage (i.e. elapsed sleep architecture versus optimal sleep architecture for that person at a certain time can provide a guide as to the REM sleep progress (i.e. accounts for brain recovery and memory consolidation) or deep-sleep (i.e. stage 3 and 4 sleep can project body recovery for an individual based on the actual versus **nominal deep-sleep at any point during a subject/patient's sleep** when the subject/patient chooses to access such information or when an associated and authorised health-worker or carer accesses such information ) sleep architecture stage in the subject/patient's life **(i.e. sleep requirements change with age, medication, sleep deprivations, health status, stress etc.).**

The present example **provides the capability to** assess the progress of an individual's sleep progress in the context of sleep-cycles or stages, sleep architecture, an individual's normal or typical sleep patterns, the normative population (i.e. via a data base normalised for factors such as age, sex, BMI etc.). Moreover the present example incorporates a means (i.e. based on sleep period to time and based on a continued pattern or sleep or sleep disruptions versus normal sleep and normal disruptions an estimate of the resulting sleep architecture or sleep **disturbance, RERA, AHI, RDI, SDI, sleep efficiency, WASO, and other indices** can be projected and this information made available to user along with recommended or hints of countermeasures (including sleep specialist or GP intervention) to circumvent ongoing sleep deterioration or associated adverse health sequelae.

**Other sleep contextual examples include determination of the severity of any physiological measures (cardiology function, ECG, HRV, respiratory disorders, neurological disorders, blood-pressure etc.)** in the context of current time or elapsed sleep period versus normal values for the specific stage or **cycle of sleep a subject/patient** is in or **elapsed- sleep time.**

For example, blood pressure changes typically have different meaning or ramifications in terms for alarming outcomes **or functional performance subject to sleep stage (i.e. REM blood-pressure variation etc.)** and similarly the stage of sleep and the time period elapsed can all be factored into the accessible information reporting to a user/subject or associated health-worker. The relevant sleep-health progress based on the sleep stage and sleep time and even sleep-patient-historical information (i.e. diagnostic reports; cardiac, respiratory, neurological and other safe functional ranges or limits and thresholds warranting intervention or notification/reporting) context.

Another example relates to shift workers, travellers or sufferers of **circadian** disorders relating to irregular sleeping patterns, for example. In these cases the present example can track sleep regularity for an individual and determine information measures of sleep progress or sleep period outcomes in the context of regularity or disordered sleep patterns and hence factor in likely **circadian** sleep cycle impacts or sleep debt due to **circadian driven sleep propensity,** for example.

Further examples, in terms of factoring time, elapsed sleep period or sleep stage in the context of performance outcomes include taking into account other time contextual factors such as sleep architecture, **sleep-disruptions, cardiac activity (i.e. ratio or high and low frequency ECG power as a measure of autonomic activity or dysfunction based on sleep cycle and normative data comparisons) or sleep disruptions, sleep fragmentation information etc.**

**The present** example **enables information access to sleep disruption or sleep disorder events of interest at any time during or post sleep monitoring-** i.e. in one example a simple touch, gesture or tap/shake-motion gesture could enable selection of current or recent sleep disruptions issues along with likely causes and countermeasures (i.e. sound recording and recall associated with disruption from street noise, car noise or temperature or other environmental changes). Predictive analysis projects likely end of sleep period outcome and suggested changes or countermeasures along with projected likely outcomes in terms of sleep performance improvements based on current trajectory with ongoing conditions versus corrective or preventative hints or actions (i.e. ***on-line automatic analysis incorporating sleep or sleep disorder corrective and preventative action*** (SD CAPA) via root mean causation analysis enabling information determination and information access in terms of the most probable causes and countermeasure recommendations or hints in the context of abating continued sleep or related disorder health degradation or related adverse health sequelae reoccurrence. Performance ***assessment can be computed online automatically in terms of the period and phase in the current or any past sleep sleeping session*** whereby said performance measures can be based on personalised patient/consumer specific typical outcomes, outcomes compared to normative population base or other goals or targets established personally or via special online "opt" in sleep coached or other health, fitness or personalised carers. In another example biological synchronisation capabilities enable determination of breathing disorder disturbances associated with a subject being monitored versus other individual's breathing sounds.
□ ***Online eLife internet of things (eLifelOT)*** sleep progress & predictive analysis:one example enables automatic transfer of any relevant study data, patient information and settings for special cardiac-programmed, ventilator servo controlled, positive airway treatment devices or other non-invasive ventilator service positive pressure ventilation (NIPPV) system. Said settings can include thresholds, ranges, normal operating conditions and automatic alarm conditions related to physiological measures (i.e. heart, oxygen, C02 levels, respiratory waveform measures etc.) indicative of health condition or other events of interest. Directly linked device programming, buy, delivery along with health insurance and any other regulatory compliance documentation or processes can be incorporated into the one loan, hire, buy, test and evaluate process selection options.
□ ***Online eLife internet of things (eLifelOT) biofeedback*** and dynamic data exchange capability. sleep progress & predictive analysis:one example enables automatic transfer of any relevant study data, patient information and settings for special cardiac-programmed, ventilator servo controlled, positive airway treatment devices or other non-invasive ventilator service positive pressure ventilation (NIPPV) system. Said settings can include thresholds, ranges, normal operating conditions and automatic alarm conditions related to physiological measures (i.e. heart, oxygen, C02 levels, respiratory waveform measures etc.) indicative of health condition or other events of interest. Directly linked device programming, buy, delivery along with health insurance and any other regulatory compliance documentation or processes can be incorporated into the one loan, hire, buy, test and evaluate process selection options.

### SYSTEM RESOURCES [7]

eLifeNAS; eLifeRURAL; eLifeHDCM; eHealth-MTM time synch; eLifeAPM;
- Adaptive Physiological-Body Network (APM) & NAS Gateway

### Figure 29 External Noise Sensing and Cancellation System

### Title: MULTIPOINT TIME-SYNCH MONITORING (MTM) SYSTEM

**Precision time-synchronisation methods for internet and network based monitoring and data-acquisition systems.**

**Previous state of the art limitations:** One of the key impediments as it relates to monitoring multiple or distributed device or system in an internet, network, wireless communication or wireless mobile device environment is the ability to achieve sufficiently accurate time-synchronisation, capable of avoiding the many pitfalls otherwise evident.

These pitfalls associated with inadequate time-synchronisation between two or more devices can be particularly disconcerting when it applies to monitoring applications.

In one example, the monitoring of a subject's physiological parameters from two or more separate devices and then reconstituting the separate recordings so that meaningful or precise analysis (i.e. spectral analysis comparing FFT phase and spectral outputs in terms of coupling relationships between multiple brain regions or neural sources), for example, of a subject's online health conditions is possible can be fraught with inaccuracies if the data acquisition of each said separate device is not sufficiently accurate.

For example, in the case where neurological biomarkers such as high-frequency oscillations are being tracked as a precursor to a possible seizure event and such high-frequency activity is being analysed in terms of degrees of coupling with other spectral brain activity then time synchronisation can greatly influence computational outcomes.

For example, if a high frequency oscillation of 500Hz is being examined for phase coherence with a simultaneous appearance of 500Hz within the human brain (i.e. say as a marker of seizure onset) then a complete 500 Hz cycle has a time period of .002 or 2 milliseconds (1/500), while each degree of phase shift represents a time-period of .00000556 seconds or 5.56 microseconds.

Therefore, a time-synchronisation accuracy between 2 or more simultaneous data acquisition systems (example only) should be time-synchronised with an accuracy of at least 10 times the minimal measurement component of interest or preferably 100 times or more accuracy. This represents time-synchronisation accuracy in this example of between .000000556 and .0000000556 min or 556 nanoseconds to 5.6 nanoseconds.

Even based with what is claimed to be the most accurate of internet timing functions (based on an atomic clock which is the most accurate timing source in the world) the displayed time typically has a precision of between 0.02 to 0.10 seconds, subject to your internet traffic or your computer processing demands at any given time (RFM:14983)¹⁶.

Moreover, despite the atomic clock has a maximal shift per year of about 25-12 billionths of a second shift (more than adequate in this example) the typical quartz crystal in our watch or computer system can drift a few second per month (RFM:14984)¹⁷.

Taken in the context of this above example the few seconds per month can translate to be very significant when monitoring a subject continuously for an hour let-alone the typical application of monitoring uninterrupted for days or even weeks at a time.

Moreover, when multiple systems are all simultaneously monitoring another time-synchronisation problem occurs in that each system has an inherent drift and therefore in order to time-synchronise the data of all systems in a manner that minimises all combinations of delays or phase shifts between each respective system, and particularly as it applies to physiological data representation and precision analysis, we need a unique time-synchronisation process capable of the following attributes:
Consequently, the nubs of the presents are to provide an accurate time referencing system suitable for precision simultaneous data acquisition amongst a plurality of monitoring or data acquisition systems, where such a system comprises any of or any combination of:
MASTER DATA-ACQUISITION TIME SYNCHRONISTION
   ➢ Means of ***recognising a common Master time-synchronisation reference*** (i.e. utilisation of the **Global Positioning System** (GPS), capable of an accuracy of ±10 ns for master time-synchronisation reference (RFM:14986).
   ➢ Means of ***recognising a common time-synchronisation marker*** (i.e. utilisation of **radio transmitted time reference** (i.e. DCF77 (Germany) in Mainflingen in Hessen; MSF (Great Britain) in Anthorn; JJY (Japan); WWV (USA) in Fort Collins; WWVH (USA) on Hawaii; BPC (China) in Shangqiu) (RFM:14984).
MASTER DATA-ACQUISITION TIME RESYNCHRONISATION
   ➢ Means of both online and offline data reconstitution relating to absolute **time shift** between multiple devices/systems (i.e. utilising ***calibration and compensation applicable to "free-running"*** periods of inaccessible master-time-synchronisation reference.)
   ➢ Means of both online and offline data reconstitution relating to **time stability** (i.e. synchronisation drift) between multiple devices/systems (i.e. utilising ***calibration and compensation applicable to "free-running"*** periods of inaccessible master-time-synchronisation reference.)
   ➢ Means of both online and offline time stability determination (drift-factor over time) ***calibration and compensation applicable to "free-running"*** periods of inaccessible master-time-synchronisation reference.
   ➢ Means of determining maximal time shift between a plurality of monitoring or data acquisition devices;
   ➢ Means of **determining maximal time shift between a plurality** of monitoring or data acquisition devices and warning system users should such conditions or predetermined thresholds or ranges of time-synchronisation requirements be exceeded or approached;
   ➢Means of **determining maximal drift between** a **plurality** of monitoring or data acquisitions devices and warning system users should such conditions or predetermined thresholds or ranges of time-synchronisation stability requirements be exceeded or approached;
   ➢Means of enabling data **time-synchronisation** between a **plurality of communication mediums** or multitude of transmission mediums (i.e. Bluetooth, Wifi, Ethernet, optical, radio, satellite, GSM, etc.).
   ➢Means of enabling **Master clock** reference and multiple devices with **slave time referencing capabilities;**
   ➢Means of enabling data synchronisation across a disparate group of communications systems (i.e. multitude of transmission mediums), mediums, devices and/or systems;
   ➢Means of enabling data time-synchronisation between a plurality of devices or systems;
   ➢Means of enabling data time-synchronisation via a unified and commonly accessible mater-synchronisation signals across a plurality of wireless devices or monitoring systems within a network or group of monitoring/data-acquisition systems where the reconstitution of the total data record is reliant upon precision phase-synchronisation of each said monitoring system or device's data;
   ➢Means of ***enabling Periodic time stamping*** of successive acquisition samples of said simultaneously monitored wireless or mobile device data;
   ➢Means of ***deriving precision time-synchronisation between a multitude of separate monitoring devices*** or systems and/or communication systems, and then defining a master timing reference and a relationship between the said separate devices and computing a relationship between all timing references of master and slave devices or systems so that the overall monitoring data can be time-synchronised in terms of time-**OffSet,** time-alignment (i.e. no periods of monitoring or data acquisition are stretched or shrunk with reference to all monitoring devices or systems), time-stability (i.e. time relationship between any devices does not wax and wane due to relating timing stability factors).
   ➢Means of deriving timing or clock synchronisation via ***Network Timing Protocol NTP);*** This system typically involves user-definable policy control in the design of time synchronisation and evidence model. Additionally, this model can support inline and meshed operating models (i.e. clearly defined master source of time or where no penultimate master reference clocks is required).
      Means establishing time synchronisation relating to multiple system monitoring or data acquisition devices whereby support inline and meshed operating models whereby clearly defined master source of time or models where no penultimate master reference clocks is required;
   ➢Means of deriving master or slave clock synchronisation of a plurality of devices, whereby a master clock is nominated in order for all devices to reference and synchronise independent devices clock timing for data acquisition and other functions.
   ➢Means of augmenting conventional (i.e. NTP; simplifies NTP/SNTP; internal or local clock reference - i.e. quartz crystal) time or clock synchronisation of a plurality of data-acquisition or monitoring systems, incorporating the deployment of an **automatic calibration of compensation** method whereby said calibration and compensation comprises of periodically assessing a more accurate time source (i.e. radio or GPS Atomic based time reference) than **conventional** time reference (i.e. ) NTP.
      Whereby **calibration** can comprise of the determination of time-stability factors, time-**offset** factors associated with the **conventional** time clock, and whereby **compensation** can comprise of adjusting the time **offsets** and stability as required to improve the overall time-synchronisation accuracy between multiple data acquisition or monitoring systems. **(Problem addressed:** While NTP is considered robust, and used widely across the Internet, and generally regarded as state of the art in terms of distributed time synchronization protocols for unreliable networks, the NTP is still typically restricted to synchronization **offsets** over the public Internet of the order of a few milliseconds or in the case of local area networks sub-milliseconds.) (RFM: 14986)
   ➢ Means of deploying the combination of NTP time or clock synchronisation (applicable to multiple data acquisition systems) and **automatic calibration of compensation** method whereby said calibration and compensation comprises of periodically assessing a more accurate time source than NTP (i.e. radio or GPS Atomic based time reference) in order to implement calibration procedures comprising of determination of time stability factors, time-**offset** factors and correspondingly optimal compensation factors in order to further compensate and consequently enhance conventional NTP time synchronisation characteristics.
   ➢ Means of deploying **Berkeley algorithm, or similar format,** or similar format, in **combination** with said **calibration** and **compensation** NTP clock synchronisation (applicable to multiple data acquisition systems) as a means of reducing clock skew between multiple systems.
      **Berkeley algorithm:** A process where a time server periodically fetches the time from all the time clients, computes an average, and then reports back to each clients with the adjustment required to allow local clocks to achieve the computed average, so that all systems can be synchronised. Clocks exceeding predefined limits could be discarded to minimise effects of one extremely skewed clock). (RFM: 14986)
   ➢ Means of deploying **Clock Sampling Mutual Network Synchronization (CS-MNS),** or similar format, in **combination** with said **calibration** and **compensation** NTP clock synchronisation (applicable to multiple data acquisition systems) as a means of reducing clock skew between multiple systems.
      **CS-MNS:** Generally used in distributed and mobile applications. Compatible with IEEE 802.11 and similar standards. Can be scaled over mesh networks with indirectly-linked non-adjacent nodes, and compatible to IEEE 802.11 and similar standards. Accuracy can be in the order of few microseconds, but requires direct physical wireless connectivity with negligible link delay (<1 microsecond) on links between adjacent nodes- this limits to a few hundred meter the distance between neighboring nodes.) (RFM: 14986)
   ➢ Means of deploying **Cristian's algorithm,** or similar format, in **combination** with said **calibration** and **compensation** NTP clock synchronisation (applicable to multiple data acquisition systems) as a means of reducing clock skew between multiple systems.
      **Cristian's algorithm:** This system uses a time server which maintains its clock via by referencing a radio clock or other accurate time source. Time is maintained by all other computers (clients) in the system staying synchronised to the time server, via procedural calls from these clients. Variations of this algorithm can enable more precise time calculations by factoring in network radio propagation time (RFM: 14986).

### EXAMPLE OF MULTIPOINT TIME-SYNCH MONITORING (MTM) SYSTEM : DETERMINATION OF SHORT & LONG-TERM MULTI-SYSTEM MONITORING AND DATA-ACQUISITION CLOCK SYNCHRONISATION DRIFT, DELAY AND STABILITY MEASURES AND COUNTERMEASURES (see Figure 30)

In one example MTM system (or eLifesyc) an further capability is to enable automatic calibration of time-sync between different monitoring systems (i.e. wireless mobile devices) based on compensating for internal clock (typically quartz clocks) by way of comparing any of or any combination of the local clock sources in a network with a precision reference (such as local network or directly connected installed Atomic based clock, radio clock, GPS clock etc.). The resulting comparison can incorporate the use of local (can be located near crystal quartz clocks providing data acquisition timing reference for each respective monitoring system). In this way a comparison between an external accurate clock timing (such as atomic based network or radio or satellite linked clock) can be compared to the current local quartz clock-locality temperature, Atomic referenced clock timing, derived local data-acquisition timing (referenced to local quartz crystal) in order to determine ***timing characterisation*** of all separate monitoring systems on the basis of ***short-term*** and ***longer term*** : a) ***stability*** (i.e. wavering of clock precision, b) clock **offset** (absolute time difference between real Atomic-clock time and local clock data-acquisition time stamping) time, c) clock ***drift*** (progressive change in relationship between Atomic-clock time and local data-acquisition clock), and d) information technology or geographical weather related factors (i.e. ***temperature, humidity)*** environmental conditions including (i.e. associated with local quartz crystal which can be impacted in terms of drift by temperature).

By enabling this type of ongoing characterisation the overall management of time synchronisation between all separate monitoring systems and each individual monitoring system can be managed, even when the master or precision timing source (such as local network or directly connected installed Atomic based clock, radio clock, GPS clock etc.) is not available or connection is disrupted.

For example, the special timing characterisation enables each system to produce accurate predictions based on previously determined factors (i.e. stability, **offset,** drift, temperature-related changes, long-term changes, short-term changes etc.) in terms of compensating overall system time synchronisation between all separate monitoring or data acquisition systems and also algorithms designed to minimise time inaccuracies when local or free-running timing synchronisation is required due to disruption or disconnection of more accurate time referencing means (i.e. wireless disruption etc.).

Moreover, in circumstances like this, a **central timing server** within a network of monitoring/data-acquisition systems can utilise an appropriate (i.e. subject to factors such as application and precision of timing requirements, affordability etc.) **timing master clock timing reference** (such as local network or directly connected/installed Atomic based clock, radio clock, GPS clock etc.) for the network or group of monitoring or data-acquisition systems or devices.

The further incorporation of a **multi-point time-synchronisation monitoring system** algorithm which enables periodic sampling of each member of the group of monitoring systems (i.e. disparate devices or systems monitoring physiological parameters of a subject) enables the computation of an adjustment factor for any of or any combination of the group of systems, where required, in order to achieve the most accurate clock-synchronisation and time alignment between all subject monitoring systems.

Additionally, and particularly as it relates to the **multi-point time-synchronisation monitoring** system, the ability to analyse each monitoring system within a defined or dynamically determined group of monitoring system, associated with a specific subject (consumer; patient etc.) of interest, enables each separate monitoring device or system to continue in a free-running mode but with the incorporation of the most accurate predetermined (during system calibration analysis) independent or free-running (i.e. when disconnected from timing server management) clock-synchronisation circumstances, based on long-term and short-term clock drift, **offset,** and stability adjustments as required.

### EXAMPLE OF MULTIPOINT TIME-SYNCH MONITORING (MTM) SYSTEM : DETERMINATION OF SHORT & LONG-TERM MULTI-SYSTEM MONITORING AND DATA-ACQUISITION CLOCK SYNCHRONISATION DRIFT,

**(see** **Figure 30****)**

### DELAY AND STABILITY MEASURES AND COUNTERMEASURES APPLICABLE TO MONITORING PHYSIOLOGICAL AN D AUDIO-VISUAL PARAMETER (see Figure 30)

In one example any combination of audio monitoring, video monitoring and physiological monitoring corresponding to a subject could be deployed. For example, if a subject if the long-term monitoring is required of an epileptic subject, then audio-visual monitoring of seizure events including the determination of timing synchronisation between seizures, subtle body activity such as, movements, arousals, sounds (i.e. breathing, coughing, voice, or environmental) or even very fast eye-blinks or muscle twitches may be required. Furthermore, such audio-visual monitoring may need to be synchronised or even phase-locked in terms of time-synchronisation with related physiological recordings (including high frequency bursts such as high frequency oscillation neural activity, for example only).

In this example enables multi-point time-synchronisation incorporating any of or any combination of time-synchronisation **characterisation, calibration,** and/or **compensation** for any mixture of disparate monitoring mediums including audio, video and/or physiological parameter monitoring systems.

### EXAMPLE OF MULTIPOINT TIME-SYNCH MONITORING (MTM) SYSTEM WITHOUT CENTRALISED OR MASTER TIME-REFERENCE (see Figure 30)

In one example the **multi-point time-synchronisation monitoring** system device clock-synchronisation monitoring (MCM) system could comprise of many separate devices with individual timing references but rather than a single or central master clock a timing algorithm capable averaging the time of all systems in a group of monitors could be deployed in a manner whereby the average time difference between all clocks is computed and each separate device or system is then provided a clock-synchronisation adjustment factor associated with the compensation factors of each respective separate device or system, in order to achieve synchronisation between all systems or devices. Clocks exceeding predefined or dynamically computed limits could be discarded to minimise effects of one extremely skewed clock). This approach can still incorporate the **calibration process** whereby the long and short-term timing characteristics can be determined as a factor in establishing the optimal time adjustment or compensation factors of different monitoring devices or systems in circumstances where a centralised algorithm is not possible due to interconnectivity disruption (i.e. wireless connection is unreliable and central algorithm approach is not feasible or effective).

### Radio capability to connect to Atomic clock for timing synchronisation applicable to one or more wearable health or environmental monitoring systems or devices

The present example incorporates a radio tuner capability in order to tune into an Atomic clock radio channel for purpose of enhanced timing synchronisation applicable to one or more wearable or mobile (including wireless mobile, PC, tablet or other information or communication system) systems or devices with health or environmental monitoring capabilities, and whereby said synchronisation includes alignment of multiple simultaneously monitored channels or said health or environmental signals.

### Description of the Figures

***Figure 30*** Multipoint time-synchronisation monitoring (MTM) system overview diagram.
**[1]** Device **EEG** monitoring system (i.e. Somfit)
***[2]*** ***Device** : **Fitness** monitoring system (i.e. accelerometer, skin resistance, temperature, metabolism*/*calorie .i.e. chest-worn, wrist-bangle, neuro-headband, EXG-wireless etc.*
**[3]** Device: Earbuds(function: entertainment, health, fitness) I.e. any of or any combination of temperature ,galvanic skin resistance, GSR, cardio ballistogram, accelerometer, photo-pulse, reflective oximeter, vestibular **EEG;** eLifeneuro, Somfit)
**[4]** Device: **EEG** monitoring system (i.e. Somfit)
**[5]** Device: Home sleep test (HST) system
   (i.e. eLifeHST etc)
**[6]** Device: Audio-visual recording systemDevice: Combined armband mobile-phone holder incorporating sensors for automatic online monitoring & determination of any of or any combination of calorie-burn determination ,optical pulse, temperature, galvanic skin resistance, motion/movement, energy dissipation via skin surface heat emissions (using infrared or array of appropriately placed thermo-coupler(s) or thermistor(s) & further incorporating dynamic data exchange with wireless device of one or more sensor & assoc. measure; metabolism/calories.

In one example, a phone arm-band holder can include one or more infrared temperature LED(s) and LDR(s) designed with time gate light activation of LED and associated receiver sensor (infrared LDR) in order to provide a degree of measurement-depth (i.e. able to be determined by way of time between gating on infrared LED and detection of return signals referenced to this time), enabling the characterisation of the heat transmission through and from the skin surface as a measure of body-heat expulsion. The infrared-LED transmitter and infra-red LRD receiver can comprise of near-field infrared (NFIR) monitoring sensors and/or associated systems.

Other examples can additionally include any of or any combination of pulse-measurement (i.e. via using the mobile phone's light generation and camera tracking capabilities), internal accelerometer or movement sensor within the said mobile phone, GPS within the said mobile phone device or arm-band phone-case, temperature sensor(s) (i.e. any combination or mobile-phone sensors or strategically placed thermo-couplers, thermistors, and/or polyvinylidene difluoride (PVDF), NFIR sensors etc.), galvanic skin resistance (GSR) sensor(s) (as part of phone-case or mobile phone), companion wireless devices (i.e. Bluetooth, Wifi ear-phones etc.), other mobile-phone related case health or fitness monitoring sensors, humidity sensor(s) (within case or phone) airflow sensor(s) (i.e. also have an impact on effort or subject energy exertion as well as perspiration) enabling the determination of surrounding weather and also airflow conditions associated with movement of activity of monitored subject.
CENTRAL PROCESSING UNIT (S)
Central Timing Server/algorithm
multi-point time-synchronisation monitoring system algorithm
Timing characterisation, calibration & compensation tracking and determination for each monitoring member device - long-term
Timing stability tracking and compensation factors
timing **offset** tracking and compensation factors
timing drift tracking and compensation factors
timing temperature, humidity, tracking and compensation factors
Timing characterisation & compensation factor tracking and determination for each monitoring member device - short-term
Atomic-clock based Master clock synchronisation reference
Synchronised Data-reconstitution
Decentralised Synchronised clock Option
Wireless disconnect (free-running device) clock synchronisation function-Synchronised
Extraction of principal timing reference based on most reliable and consistent and precise timing reference marker from various disparate communication mediums (i.e. Bluetooth, Wlfi, internet, satellite/GPS, radio clock (atomic clock based)

### Title: eLifeSLEEP

### CONSUMER & PROFESSIONAL LEVEL SLEEP-FITNESS PLATFORM

The present example deploys one or more patient worn devices as presented in this document incorporating one or more integrated (embedded or attached) sensors capable of sensing and monitoring one or more physiological parameters psychological parameters or environmental parameters, further comprising analysis or monitoring capabilities including any of or any combination of:
A single or "cluster" of patient worn system for monitoring physiological process or **sleep parameters,** comprising of one or more patient worn head-attached device(s) where the said device(s) comprise any combination of :
- A part of the said device according to above example incorporating one or more microprocessor system(s) capable of tracking physiological processes, events, or health conditions or interest;
- A part of the said device according to above example incorporating one or more microprocessor system(s) with a means of **monitoring** one or more "**Sleep parameters" and/or "other physiological parameters";**
- A part of the said device according to above example incorporating one or more microprocessor system(s) with a means of **processing** one or more "**Sleep parameters" and/or "other physiological parameters";**
- A part of the said device according to above example incorporating one or more microprocessor system(s) with a means of processing one or more **"physiological and/or sleep or wake markers";**
- A part of the said device according to above example incorporating one or more microprocessor system(s) with a means of **processing** one or more **"Sleep Architecture Measures";**
- A part of the said device according to above example incorporating one or more microprocessor system(s) with a means of **processing** one or more **Physiological and/or Sleep and/or Wake Markers;**
- A part of the said device according to above example with a means of incorporating one or more **"Patient worn system(s) capabilities";**
- A part of the said device according to above example with a means of incorporating one or more **"communication interconnect capabilities";**
- A part of the said device according to above example with a means of incorporating one or more **"dynamically-linked capabilities";**
- A part of the said device according to above example incorporating one or more **sensor *monitoring systems;***
- A part of the said device according to above example incorporating one or more **sensor *signal processing systems;***
- A part of the said device according to above example and incorporating means of ***data acquisition* of one or more channels of information;**
- A part of the said device according to above example incorporating ***online*** monitoring capability of any **sensor monitored channels or other information;**
- A part of the said device according to above example incorporating ***online*** monitoring capability of any **sensor monitored channels or other information;**
- A part of the said device according to above example and incorporating a **means** of enabling one or more **communication interconnect capabilities;**
- A part of the said device according to above example and incorporating a **means** of monitoring one or more "**sleep parameters"** and/or **"neurology parameters";**
- A part of the said device according to above example and incorporating a means of monitoring a combination of "**sleep parameters"** and/or **"neurology parameters";**
- A part of the said device according to above example and incorporating the means of monitoring a combination of "**sleep parameters"** and/or **"neurology parameters"** and/or **"activity or motion parameters";**
- A part of the said **"patient worn head-attached device(s)"** device according to above example incorporating a) **remote monitoring,** b) means of analysis including embedded device and/or remote location **analysis,** c) means of information **display including display** specific to monitored individual and/or local display observation and/or remote display location, d) means of **information access including information access** specific to monitored individual and/or local observation and/or remote location, e) means of data storage including **embedded storage** device or **remote data storage,** f) means of **data archive including** embedded device archiving or remotely located data archiving, g) means of **data transfer including** data transfer specific to monitored individual and/or other user and/or remotely located data transfer, h) means of accessing information via portable **memory removal** device and/or accessing information via **EMF interface** and/or accessing information via **wireless and/or wire interface,** i) means of **data access** specific to monitored individual and/or other user and/or via remote location, j) means of **notification** specific to monitored individual and/or other user and/or remote location , k) means of **"information distribution"** specific to monitored individual and/or other user and/or remote location , I) means of **information reporting** specific to monitored individual and/or other user and/or remote location, m) means of **generating alarm(s)** specific to monitored individual and/or other user and/or remote location, o) means of signalling or notifying for remote intervention notification specific to monitored individual and/or other user and/or remote location, p) means of reviewing any current or past monitored information and/or associated measures specific to monitored individual and/or other user and/or via other remote location, g) means of battery power capability including removable batteries and/or power transfer via **EMF** and/or **wireless and/or wire transfer,**

**Other physiological parameters; Physiological and/or Sleep and/or Wake Markers; Sleep parameters; Sleep Architecture Measures; Attachable/detachable parts; Patient worn system(s) capabilities;**

### Sleep parameters

The present example deploys one or more said "subject worn ***system(s)"*** with ***device attributes*** as presented in this document incorporating one or more integrated (embedded or attached) sensors capable of sensing and monitoring one or more environmental parameters or physiological parameters, along with the determination, prediction, onset or incident of ***"events*/*measures*/*states"*** or health conditions of interest, where said examples further comprise any of or any combination of analysis or monitoring capabilities including any of or any combination of "**sleep parameters"** and/or **"neurology parameters"** includes any of or any combination of: **-EEG,** EOG, EMG, eye lid movements or measures; leg movements; patient position; ECG;

Whereby said ***"events*/*measures*/*states"*** of interest include parameters detailed here under the following headings:
- **Other physiological parameters;**
- **Physiological and/or Sleep and/or Wake Markers;**
- **Sleep parameters;**
- **Sleep Architecture Measures;**
   - herein under sub-heading **Physiological and/or Sleep and/or Wake Markers** and/or "**Sleep parameters" and/or "other physiological parameters"** can be displayed on said "patient worn" device;

- Whereby said any sleep-related information can be monitored online/real time;
- Whereby said any sleep-related information, measures and/or indices can be determined online/real time;
- Whereby said any sleep-related information, measures and/or indices can be determined online/real time within said "forehead attached device";

The present example provides in terms of said ***"events*/*measures*/*states"*** or health conditions of interest including any of or any combination of **"other physiological parameters"** (this section is repeated under eLifeCHEST section and can include only heading with "see detail under heading elsewhere herein" to avoid repetition.

**The present example incorporates a means of wearable/attachable applanation tonometery (WAT;** **Figure 37****) whereby said means applies a pressure sensor capable of measuring radial (****Figure 38****) or other body artery pulsation characteristics (pulse wave-shape). Moreover an the present example further computes the pulse wave analysis (PAW) comprising recording a period (10 seconds for example) of arterial pressure in order to derive the associated ascending aortic pressure wave, from which a number cardiovascular measurements such as central aortic systolic pressure, aortic augmentation index, and the central pulse pressure.**
- Whereby said **means** of ***monitoring*** one or more **other physiological parameters** includes any of or any combination of the following (further detailed elsewhere in this document): Sound monitoring; Stethoscope auscultation sensor, monitoring and automatic analysis, classification, tracking and detection capabilities; Acoustic noise cancellation system; Motion detection; REM sleep behaviour disorder (RBD); Pulse wave analysis (PWA) and pulse wave velocity (PWV) monitoring and analysis capability; PWA and PWV sensors; Pulse wave analysis (PWA) sensor measures; Ballistocardiograph; Position, locational and movement sensing and monitoring; Gait or movement tracking and characterisation of events of interest; Movement and locational information; ECG sensor(s) and monitoring; Light sensor(s) and monitoring; Breathing band sensors and monitoring; EMG sensors and monitoring; GSR; Cardiac function; Sleep training system; Plethysmography Oximetry; Pulse transient oscillation amplitude measures; temperature, Energy-exertion/metabolism-monitoring (EM) as a surrogate calorie-burn measure; Physiological and/or Sleep and/or Wake measures or markers; along with other psychological states (i.e. sleep, wake).

**Physiological and/or Sleep and/or Wake Markers;**
The present example deploys one or more said "subject worn ***system(s)"* with *device attributes*** where said device(s) can incorporate one or more microprocessor system(s) with a means of **processing** one or more **Physiological and/or Sleep and/or Wake Markers** comprising the determination and/or monitoring of one or more of any of or any combination of;
- EOG signal(s), eye-lid signals(s), eye-lid movement(s), eye blinks, eye blink frequency, eye blink velocity, eye blink acceleration, reading eye movements, slow eye movements (SEM), irregular, **EEG** signal(s). Neurology signal(s), conjugate rapid eye movements associated with normal or high chin muscle tone alpha **EEG** rhythm, low-amplitude-mixed-frequency **EEG** activity, mixed-frequency **EEG** activity in general, alpha rhythm, vertex sharp waves, K complex, muscle excursion(s), EMG signal(s), low chin EMG tone, sleep onset (the start of the first epoch scored as any stage other than stage W or in most cases the first epoch of stage N1), sleep architecture, sleep hypnogram, Stage W (Wakefulness), Stage N1 (NREM 1), Stage N2 (NREM 2), Stage N3 (NREM 3), Stage R (REM), stage 1; Rechtschatten and Kales (R&K), stage 2 (R&K), stage 3 (R&K, stage 4 (R&K), **EEG** sawtooth waves, low chin EMG tone, low EMG tone, low motion indicative of low EMG tone, transient muscle activity (i.e. short irregular bursts of EMG activity usually with duration <0.25 seconds superimposed on low EMG tone. The activity may be seen in the chin or anterior tibial EMG derivations, as well as in **EEG** or EOG deviations, the latter indicating activity of cranial nerve innervated muscles such as the facial muscles and scalp. The activity is maximal in association with rapid eye movements.), **EEG** activity in range of 4-7 Hz combined with slowing of background frequencies (i.e. by ≥1 Hz from those of stage wake), sleep spindle, drowsiness, vigilance, attention, sleep;
- **"health conditions"** relating to any sleep parameter related measures or associated indices can include (but is not limited to) sleep efficiency, wake after sleep onset, delayed sleep measures, sleep-architecture measures, deep-sleep measures, REM sleep measures, and other sleep indices;
- **"health conditions"** relating to any sleep parameter related measures or associated indices can include (but is not limited to) other physiological measures including (but not limited to) activity (or movement or motion or accelerometer), number of steps taken, walking and/or running characteristics; any characteristics of activities, any location or directions associated with activities, any performance outcomes associated with activities;
- "health conditions of interest" applicable to any of or any combination of sleep, psychological state or related sleep disorders and/or including other sleep or wake state
- comprising any of or any combination of sleep measures including (but not limited to) any of or any combination of brain signals, muscle, eye physiological signals including but not limited to any
- whereby means of monitoring sensor for **"monitoring one or more physiological signals"** can monitor one or more **"sleep parameters";**
- A part of the said device according to above example incorporating one or more microprocessor system(s) with a means of **monitoring** one or more **"Sleep parameters";**
- whereby said **"neurology"** signals can include one **or more electrophysiological signals;**

### Sleep parameters

The present example deploys one or more said "subject worn ***system(s)"*** with ***device attributes*** as presented in this document incorporating one or more integrated (embedded or attached) sensors capable of sensing and monitoring one or more environmental parameters or physiological parameters, along with the determination, prediction, onset or incident of
***"events*/*measures*/*states"*** or health conditions of interest, where said examples further comprise any of or any combination of
**"sleep parameters"** can include any of or any combination of **neurology, EOG, eye-lid, eye-movement, muscle excursion, EMG, body motion/movement; body- position signal(s);**
- whereby said **"neurology"** signals can include **one or more electrophysiological signals;**
- whereby said **"electrophysiological signals"** can include one or more combined or separate sensors to monitor any of or any combination of **EOG, EMG and/or EEG signals;**
- whereby said **"monitored" "sleep parameters"** can be by way of a **"forehead applied part'"**

### Sleep Architecture Measures

The present example, incorporates where said ***"events*/*measures*/*states"*** or health conditions of interest comprise any of or any combination of:
- sleep architecture, sleep-debt, sleep hypnogram, sleep efficiency, deep-sleep, Stage W (Wakefulness), Stage N1 (NREM 1), Stage N2 (NREM 2), Stage N3 (NREM 3), Stage R (REM), REM sleep, stage 1 sleep, stage 2 sleep, stage 3 sleep, stage 4 sleep,
- sleep after wake onset (SAWO);
- sleep quality progress;
- sleep quality;

The present example **incorporates,** said ***"device attributes"*** can include any attributes presented under the following headings:
- **Attachable/detachable parts;**
- **Patient worn system(s) capabilities;**
- **Forehead part(s)**

### Attachable/detachable parts

The ***"device attributes"*** of the said "subject worn system(s)" can comprise of attached an forehead monitoring applied part for monitoring any said **"sleep parameters"** and said **attachable/detachable "parts"** can interlock and/or "interconnect" with a **"wearable wrist band";**

### Patient worn system(s) capabilities

The present example deploys one or more subject worn said ***"system(s)*** with ***device attributes"*** including any of or any combination of:
- "patient worn system(s)" "part(s)" comprising of one part capable of being **detached and re-attached** with other part;
- "patient worn system(s)" "part(s)" comprising **attached forehead monitoring applied part** for monitoring any said **"sleep parameters";**
- **"monitored" "sleep parameters"** by way of **"forehead applied part"'**
- "patient worn system(s)" comprising of **one or more parts;**
- "patient worn system(s)" comprising of a **plurality** of **parts;**
- "patient worn system(s)" comprising of one or more **interlocking and/or attachable/detachable "part(s)";**
- "patient worn system(s)" comprising of **one or more interconnecting part(s);**
- "patient worn system(s)" can include **head-attached device** incorporating sensors, signal processing, data acquisition, processing and communication capabilities including any of or any combination of: "patient worn system(s)" comprises a one or more wearable forehead attached device headband device for sleep parameter monitoring
- interconnecting parts includes a polarity of ***companion*** (compatible data communication and/or wireless communication capabilities and interconnectivity) devices or a plurality of devices where one or more of these plurality of devices incorporates a means of enabling ***interchangeable monitored or sensed information via a universally compatible option of interlocking*/*interconnecting, detachable monitoring or processing part and*/*or related device (i.e. a removable electronic module which is common to two or more devices thereby minimising the cost of multiple devices such as a sleep-time information monitoring strip with a removable monitoring device that can be attached as part of* a *daytime fitness of health wearable device (i.e. wrist-bangle, chest-band;*** watch etc.) an in doing so provides a means of sleep and wake continuous monitoring with minimal cost or inconvenience. Moreover, dynamic data exchange via wireless interconnect enables a sleeping subject (with applied forehead **EEG,** EOG, EMG monitoring part, for example, to augment conventional daytime wrist pedometer or motion-based fitness devices with credible and validated sleep measures.

The said ***"events*/*measures*/*states"*** or health conditions of interest comprise any of or any combination of:
- means of **processing** one or more **"Sleep parameters"** and/or "other physiological **parameters"** can include any of or any combination of "sleep-related information comprising" (but not limited to) any of or any combination of sleep states; sleep stages ; REM sleep: EMG levels ; eye-movements ; **EEG** spindles; **EEG** K-complexes; and/or EOG signals and/or any related measures including (but not limited to sleep hypnogram ;sleep architecture; sleep efficiency; sleep quality, deep-sleep amount; REM sleep amount; sleep disturbance index; determination of other events as detailed under sub-heading **"Physiological and/or Sleep and/or Wake Markers" detailed elsewhere** herein;
- whereby said **"patient worn system(s)" "part(s)"** can comprise of one part capable of being **detached and re-attached with other part;**
- whereby said one part **of "patient worn system(s)" "part(s)"** can comprise **attached forehead monitoring applied part** for **monitoring** any said **"sleep parameters";**
- Whereby said "patient worn" device can display both **"fitness"** and **"sleep-related information";**
- Whereby said forehead sensor enables a smaller part or sub-section of the said "forehead sensor" to incorporate an "electronics module";
- Whereby said "electronics module" capable of wireless interconnection in real-time to other mobile wireless or wearable device;
- Whereby said "electronics module" capable of wireless interconnection in real-time to other network application services;
   - Whereby said "electronics module" capable of monitoring any of or any combination of events and/or measures detailed herein under sub-heading Physiological and/or Sleep **and/or Wake Markers** and/or **"Sleep parameters" and/or "other physiological parameters"** can be displayed on said "patient worn" device;

### Environmental sensing

The said ***"device attributes"*** can include any attributes comprising any of or any combination of:
- alarm or alert or indicator or interface to mobile device associated or messaging, email, phone automated voice message and other information or communication systems of any health conditions, environmental conditions of interest or concern;
- ionisation monitoring, ionisation smoke alarm, methane monitoring, toxic gas monitoring, toxic chemical monitoring and/or Co2 gas monitoring, methane gas monitoring and/or thermometer.

### Forehead part(s)

The said ***"device attributes"*** can include any attributes comprising any of or any combination of:
- "patient worn part" which can comprise of one or more **"forehead applied part(s)";**
- whereby said "forehead applied part(s)" can include one or more **"monitoring sensors;**
- whereby said **"monitoring sensors"** can monitor one or more electrophysiological signals from a single sensor;
- whereby said **"monitoring sensors"** can monitor one or more physiological signals from a single sensor;
- whereby said "patient worn part" can include a **single** electronic module or a **plurality** of electronics **module(s);**
- whereby said "patient worn part" can include a **single** or a plurality of **applied part(s);**
- whereby said "patient worn part" can include **applied part(s);**
- whereby said **"applied part(s)"** can include can be **disposable or reusable;**
- whereby said **"applied part(s)"** can comprise of a self-adhesive applied part;
- whereby said **applied part(s)** can include a **pressure attached sensor applied** to an individual's forehead;
- whereby said **applied part(s)** can include a microphone;
- whereby said "microphone" can include electronic directional targeting to region of interest (i.e. breathing sounds) applied forehead part with at least one electrophysiological sensor;
- whereby said "applied forehead part" including any of or any combination of:
   at least one **universal pair or** a plurality of sensors are capable of EMG monitoring (includes sensing) and/or **EOG** and/or **EMG** monitoring and/or **eye movement** and/or **blink-movement** and/or **facial squint** or **facial skin movement** and/or **facial muscle EMG** and/or **facial muscle movement** and/or **tremor** and/or motion and/or **acceleration** and/or **body-movement** and/or **room light** monitoring and/or **sound monitoring** and/or **sound cancellation** of unwanted sounds and/or **microphone electronic directional** targeting to region of interest (i.e. breathing sounds) and/or **microphone passive directional** targeting to region of interest (i.e. breathing sounds) and/or temperature monitoring and/or integrated reflective plethysmography **oximeter** with associated outputs and/or cardiobalistogram monitoring;
   - whereby said "forehead sensor" can incorporate **"body wireless network elasticity functions";**
   - whereby said "forehead sensor" incorporating means of **wireless interconnecting** to any worn device to indicate current psychological state;
   - whereby said "forehead sensor" incorporating means of **wireless interconnecting** to any worn device to indicate sleep quality and/or sleep efficiency and/or deep sleep and/or sleep disturbance index and/or RERA disturbance index and/or and measure or index relating to any stages of sleep;
   - whereby said "forehead sensor" incorporating means of **wireless interconnecting** to any worn or other device to indicate current sleep architecture and/or REM sleep;
      - whereby said "forehead sensor" incorporating means of **wireless interconnecting** to any wrist worn device to "indicate" current **sleep architecture** and/or **REM sleep** and/or any measures or indices and/or said events and/or measures detailed herein under sub-heading **Physiological and/or Sleep and/or Wake Markers** and/or **"Sleep parameters" and/or "other physiological parameters"** can be displayed on said "patient worn" device;
   - whereby said **"indicate"** can include (but is not limited to) a **target value versus actual value;**
   - whereby said **"indicate"** can include (but is not limited to) a **values for any desirable period;**
   - **"body wireless network elasticity functions"** (see elsewhere functions;
   - whereby said device(s) incorporates sleep parameter **data replication** and **synchronisation** functions;
   - whereby said device(s) incorporates means of **buffering data** during wireless lost data packets or other errors such as wireless transmission problems;
   - whereby said device(s) incorporates means of indicating to **signals quality status** of monitoring signals at any time including interconnectivity with wireless network and/or patient worn device and/or other wireless device and/or as part of said "forehead device" or associated system;
   - whereby said device(s) incorporates means of monitoring sensors **signal quality online/real-time;**
   - whereby said device(s) incorporates means of monitoring sensors electrode **sensor impedance online/real-time;**

### "communication interconnect capabilities"

The said ***"device attributes"*** can include any of or any combination of **"communication interconnect capabilities" comprising any of or any combination of:**
- means of enabling one or more **communication interconnect capabilities** includes "dynamically-linking" any of or any combination of: a) 2 or more patient worn devices, and/or b) 1 or more patient worn devices and/or other wireless connected information systems, and/or c) 1 or more internet protocol communication interfaced devices or information systems;
- means to **"interconnect" which** can include dynamical and/or online exchange of data so that an individual can "observe" any "display" of sleep parameter related measures or associated indices and/or combined with other physiological measures including (but not limited to) activity (or movement or motion or accelerometer) measures or associated indices;
   - whereby any said **"interconnect"** can include dynamical and/or online exchange of data so that an individual is alerted via audio, visual or mechanical (such as vibration) notification or alarm relating to any sleep parameter related measures or associated indices and/or combined with other physiological measures including (but not limited to) activity (or movement or motion or accelerometer) measures or associated indices;
   - whereby any said **"interconnect"** can include dynamical and/or online exchange of data so that an individual is alerted via audio, visual or mechanical (such as vibration) notification or alarm relating to any thresholds and/or operational ranges and/or detected "health **conditions"** relating to any sleep parameter related measures or associated indices and/or combined with other physiological measures including (but not limited to) activity (or movement or motion or accelerometer) measures or associated indices;
   - whereby any said **"interconnect"** incorporates means of communicating such information indirectly with information system and/or directly and/or companion wrist band or other wearable or nearby device;
   - whereby said **"interconnect"** can include (but is not limited to battery charging capabilities including and of wireless, EMF, EMC/magnetic, connector, and/or wire;

### "dynamically-linked capabilities"

The said ***"device attributes"*** can include any of or any combination of **"dynamically-linked capabilities"** comprising any of or any combination of:
- whereby said **"dynamically-linked"** comprises of automatic, online dynamic wireless linking between a brain-monitoring system and a patient worn device (whereby can be wrist mounted device or smart health watch);
- whereby said **"interconnect"** can comprise of **intercommunication** between one or more brain monitoring sensor system(s) and a patient worn device (whereby can be wrist mounted device or smart health watch) incorporating dynamically linked information exchange whereby "sleep-progress" can be determined and indicated online at any time (whereby said "sleep-progress" comprises of a number of **"indicator"** options ranging from singular sleep-state or overall status determination to more complex indications;
   - whereby said **"dynamically-linked" and/or "indicator" and/or whereby any of or any combination of sleep parameters, Sleeplwake States, Physiological and Sleep or Wake Markers and/or sleep Indices can incorporate any of or any combination of automatic determination and/or indications and/or online sleep progress indication and/or any of or any combination of the following online indices and measures in accordance to any of or any combination of population normative database comparison, personal normative data base comparison, personal goals comparison and/or other target or goals comprising any of or any combination of** (but are not limited to) :
      - Online Automatic Sleep Disturbance Analysis;
      - **Sleep disturbance** index with **"context-linked"** (progressive online sleep journal with event list of sleep disturbances) and **"dynamically-linked" (i.e. sleep disturbance CAPA and associated instant replay, data review and/or event verification)** capabilities;
      - Sleep Efficiency; Sleep debt; Sleep journal; Sleep architecture,

      - whereby said **"interconnect"** can include dynamical and/or online exchange of data so that an individual can **"observe"** any **"display"** any information detailed under other sub-heading in this document (i.e. Sound monitoring; Stethoscope auscultation sensor, monitoring and automatic analysis, classification, tracking and detection capabilities; Acoustic noise cancellation system; Motion detection; REM sleep behaviour disorder (RBD); Pulse wave analysis (PWA) and pulse wave velocity (PWV) monitoring and analysis capability; PWA and PWV sensors; Pulse wave analysis (PWA) sensor measures; Ballistocardiograph; Position, locational and movement sensing and monitoring; Gait or movement tracking and characterisation of events of interest; Movement and locational information; ECG sensor(s) and monitoring; Light sensor(s) and monitoring; Breathing band sensors and monitoring; EMG sensors and monitoring; GSR; Cardiac function; Sleep training system; Plethysmography Oximetry; Pulse transient oscillation amplitude measures; temperature, Energy-exertion/metabolism-monitoring (EM) as a surrogate calorie-burn measure; Physiological and/or Sleep and/or Wake measures or markers; along with other psychological states (i.e. sleep, wake)) , relating to **Physiological and/or Sleep and/or Wake Markers** and/or **Sleep Architecture Measures Sleep parameters** and/or **"sleep parameters"** and/or **"neurology parameters"** and/or **Other physiological parameters** via **wist mounted-device** and/or **headband** mounted device and/or **chest mounted** device and/or **other patient worn device** and/or **other associated device.**
The said ***"device attributes"*** can include any of or any combination of **"dynamically-linked capabilities";**
- whereby said **"dynamically-linked"** comprises of **dynamic information exchange** between two **sensing and/or monitoring** and/or **health tracking** of "events, disorders or health conditions of interest" applicable to action activities/sports or general fitness or health conditions and/or "disorders" of interest;

The present example enables deployment of one or more said "subject worn ***system(s)"*** with ***device attributes*** as presented in this document incorporating one or more integrated (embedded or attached) sensors capable of sensing and monitoring one or more environmental parameters or physiological parameters, along with the determination, prediction, onset or incident of ***"events*/*measures*/*states"*** or health conditions of interest, where said examples further comprise any of or any combination of analysis or monitoring capabilities including any of or any combination of **"sleep parameters"** and/or **"neurology parameters"** includes any of or any combination of:
- A Smart Watch device with wire-linked or wireless plethysmography oximeter, inbuilt data modem Cloud-computing services interface, integral watch 24-7 mobile phone assistance; Deployment of any of or any combination of eHealthAtlas; HealthBook360; eHealthCAPA; Dynamically adaptive high-dependence connectivity management system (HDCMS). Smart Chestband with single "return to main menu" button, intuitive high resolution" colour gesture-touch control screen, novel stethoscope breathing sounds function and option of dual RIP bands. (covered elsewhere in this document).
- Optional consumer-level fitness & real-sleep tracking system; Neuro monitoring, detection of precursors to prediction and remote intervention-support-link 24-(including epilepsy, Parkinson's-SBD; Parkinson's disorders, including bradykinesia (i.e. slowness of movement) and support services for dyskinesia (i.e. diminished voluntary movements; involuntary movements), Autism, depression, anxiety, vigilance/drowsiness, ADHD, internet of medical devices (i.e. SPAP ^{®} CPAP & APAP systems; SleepFit wristband with real-sleep & fitness actual versus goal display; LinkNeuro Headband with SleepFit Link; smart watch with SleepFit Link; wireless red-dot lead with IDscan (auto-config & quality-control functions) (covered elsewhere in this document).
- All wireless components include ease of use and worry-free 7-day rechargeable battery life.
- Neuro headband, unique Smart Watch and Smart Chestband with high-dependence adaptive wireless and cache buffering functions, Neuro head band with unique "SomniLink"^{®} consumer retractable lead bipolar EXG wireless red-dot lead with IDscan (patent pending)
- Configurable diagnostic sleep study formats compliant with AASM Type ii) to type iv) and AU level 1 to level 4 sleep studies.

**The present example comprises of the determination and tracking of said "event/measures/states" including any of or any combination of Hypnosis Phenomena; age regression phenomena,; Observer role; re-vivification; age progression; time distortion; anaesthesia; analgesia; Rapid eye Movements; classified sleep disorders; select sleep disorders; dreaming states; hallucination states; dissociated states; and/or hypnosis states.**

The present example enables deployment of one or more said "subject worn ***system(s)"*** with ***device attributes*** as presented in this document incorporating one or more integrated (embedded or attached) sensors capable of sensing and monitoring one or more environmental parameters or physiological parameters, along with the determination, prediction, onset or incident of ***"events*/*measures*/*states"*** or health conditions of interest, where said examples further comprise any of or any combination of analysis or monitoring capabilities including any of or any combination of **"sleep parameters"** and/or **"neurology parameters",** incorporating means of automatic online determination of consciousness or psychological states including any of or any combination of:

### Psychological states

- Hypnosis Phenomena;
- Age regression;
- Observer role (the person goes back in time as an observer);
- Re-vivification (the person both remembers and relives events);
- Age progression (person imagining an event in the future);
- Time distortion (e.g. via hypnosis events or experiences can be slowed down), and/or
- Anaesthesia and Analgesia (loss of sensation and pain can be achieved via hypnosis).

### Monitoring, Determination and Tracking of Sleep, Wake and other Mental States, Events of Interest or Health Conditions of Interest

The present example enables deployment one or more said "subject worn ***system(s)"*** with ***device attributes*** as presented in this document incorporating one or more integrated (embedded or attached) sensors capable of sensing and monitoring one or more environmental parameters or physiological parameters, along with the determination, prediction, onset or incident of ***"events*/*measures*/*states"*** or health conditions of interest, where said examples further comprise any of or any combination of analysis or monitoring capabilities including any of or any combination of **"sleep parameters"** and/or **"neurology parameters",** incorporating means of automatic online determination of consciousness or psychological states or associated events/markers including any of or any combination of events or conditions detailed under following headings (as further detailed elsewhere in this patent application document) including under sub-headings:
**Whereby the present example comprises sensing, monitoring, data-acquisition, signal processing, analysis, storage, and information access including the online automatic characterisation of a subject/individual's physiological, neurological, nervous system, movement system, muscular system, psychological, pathological, states, events of interest and/or health conditions including any of or any combination of the following:** Rapid Eye Movement (REM) Sleep Characteristics; Sleep Disorder Classifications; Select Sleep disorders Dreaming States; Dissociated States; Hypnosis States,
**Whereby the present example comprises sensing, monitoring, data-acquisition, signal processing, analysis, storage, and information access including the online automatic characterisation of a subject/individual's physiological, neurological, nervous system, movement system, muscular system, psychological, pathological, states, events of interest and/or health conditions including any of or any combination of the following:**
   Rapid Eye Movement (REM) Sleep Characteristics, Sleep Disorder ClassificationsSelect Sleep disordersDreaming StatesDissociated StatesHypnosis States, SCORING STAGE WAKE (Wakefulness), SCORING STAGE N1, SCORING STAGE N2, SCORING STAGE N3, SCORING STAGE R, (any of or any combination of these states or conditions and associated detail below are part of the ***"events of interest:**"* or ***"health conditions of interest"*** referred to throughout this document), as further outlined here :
   **Whereby the present example comprises sensing, monitoring, data-acquisition, signal processing, analysis, storage, and information access including the online automatic characterisation of a subject/individual's physiological, neurological, nervous system, movement system, muscular system, psychological, pathological, states, events of interest and/or health conditions including any of or any combination of the following:**

### Rapid Eye Movement (REM) Sleep Characteristics

- Incidence of rapid-eye-movements;
- Incidence of dreaming;
- Increased autonomic nervous system activity;
- Similar **EEG** to awake state (beta activity), and/or
- Motor paralysis (excluding diaphragm).

### Sleep Disorder Classifications

Classified sleep disorders including 84 or more sleep-related disorders grouped in accordance to the following categories:
1. The insomnias : inability to sleep;
2. The sleep-related breathing disorders;
3. The hypersomnias of central origin (excessive sleepiness);
4. The **circadian** rhythm sleep disorders, and/or
5. The parasomnias.

### Select Sleep disorders

- Nightmares;
- Night terrors;
- Sleep Apnoea;
- Delayed sleep-phase syndrome (DSPS): **circadian** rhythm disorder affecting the timing of sleep, and/or
- Residual Excessive Sleepiness.

### Dreaming States

- Psychodynamics opinion - representation of the unconsciousness where meaning can be implied from dream content;
- Cognitive opinion - dreams created based on daytime experiences, and/or
- Biological view - dreams represent the cortex attempting to interpret the random firing of the brain during sleep.

### Dissociated States

- Daydreaming
- Lucid Dreaming
- Hallucinations
- Sleepwalking
- REM Sleep Behaviour Disorder
- Narcolepsy and Cataplexy

### Hypnosis States

- A state of consciousness which is characterised by deep relaxation and suggestibility, and/or
- Hypnosis (considered a different state to that of sleep)

### Hypnosis Phenomena

- Age regression
   - Observer role (the person goes back in time as an observer)
- Re-vivification (the person both remembers and relives events)
- Age progression (person imagining an event in the future)
- Time distortion (e.g. via hypnosis events or experiences can be slowed down)
- i.e. Anaesthesia and Analgesia (loss of sensation and pain can be achieved via hypnosis).
   - Whereby said "state" can include but is not limited to altered states of consciousness including but not limited to anaesthesia, wake, sleep, epilepsy, hypnosis. Sleep states includes but are not limited to a. Stage W (Wakefulness), Stage N1 (NREM 1), Stage N2 (NREM 2), Stage N3, (NREM 3), Stage R (REM). Note that Stage N3 represents slow wave sleep and replaces the Rechtschatten and Kales nomenclature of stage 3 and stage 4 sleep.

### SCORING STAGE WAKE (Wakefulness)

- **-Alpha rhythm (EEG)** tracking including automatic determination of trains of sinusoidal 8-13 Hz activity recorded over the occipital region with eye closure, attenuating with eye opening;
- **Eye** blinks tracking including automatic determination of **Automatic analysis capable of scoring stage REM stage** movements typically comprising of frequencies of 0.5-2 Hz evident during wakefulness with the subject/patient's eyes closed or open;
- **Reading eye movements** tracking including automatic determination of trains of conjugate eye movements comprising of a slow phase followed by a **rapid phase of opposite direction activity,** as the subject/patient reads;
- **Rapid eye movements (REM)** tracking including automatic determination of conjugate (motor coordination of eyes enabling **bilateral fixation on a specific object),** along with irregular, sharply peaked eye movements incorporating an initial signal deflection that typically lasts **<500 msec.**
- **REM eye movements** tracking including automatic determination of during wakefulness when subject/patient's eyes are open as they view their surrounding environment;
- **Slow eye movements (SEM)** tracking including automatic determination of conjugate, reasonably regular, sinusoidal movements of the eyes incorporating an **initial deflection** which typically lasts for **>500 msec.**
- **-Automatic analysis capable of scoring epochs as stage wake** when greater than 50% of the epoch contains alpha rhythm over the occipital region;
- **-Automatic analysis capable of scoring epochs as stage Wake** when there is an absence of visually discernible stage N3. Score epochs without visually apparent alpha rhythm so long as any of or any combination of the following conditions are also evident:
   - a. **Eye blinks** where signals have a frequency between 0.5-2 Hz
   - b. **Reading eye movements**
   - c. **Irregular, conjugate REM** related to normal or high chin muscle activity

### SCORING STAGE N1

- **-Automatic analysis capable of scoring epochs as stage N1 (non-REM sleep stage 1) based on the following discernible conditions:**
   - **1. Automatic analysis capable of scoring N1** according to the following definitions:
      - Tracking including automatic determination of **slow eye movements (SEM)** comprising of conjugate, reasonably regular, sinusoidal eye movements with an initial deflection typically lasting >500 msec;
      - Tracking including automatic determination of **low-amplitude, mixed-frequency EEG activity** comprising of low-amplitude, containing activity predominantly in the 4-7 Hz range;
      - Tracking including automatic determination of vertex **sharp waves (V waves)** comprising of sharply contoured waves with duration <0.5 seconds maximal over the central region and distinguishable from the background activity.
      - Tracking including automatic determination of **sleep onset** comprising of the start of the first epoch scored as any stage other than stage wake. (i.e. this is typically the first N1 epoch)
   - 2. **Automatic analysis capable of scoring stage N1 where subject/patient** generates **alpha rhythm EEG,** subject to the **alpha rhythm being attenuated and then replaced by low-amplitude,** mixed-frequency activity for greater that 50% of the epoch.
   - 3. **Automatic analysis capable of scoring stage N1 where subject/patient** g in subject/patients who do not generate alpha rhythm, commencing with the earliest of any of or any combination of the following conditions:
      - a. Tracking including automatic determination of **EEG** activity in frequency range of **4-7 Hz accompanied by the slowing of background frequencies by ≥1 Hz** from those of stage wake
      - b. Tracking including automatic determination of **EEG** activity **vertex sharp waves**
      - c. Tracking including automatic determination of **EEG** activity **slow eye movements**

### SCORING STAGE N2

- **1. Automatic analysis capable of scoring stage N2 in** accordance with the following definitions:
   - Tracking including automatic determination of **K complex** comprising of a well- delineated, negative, **sharp wave directly followed by a positive component** discernible from the background **EEG,** with total a duration of ≥0.5 seconds. This waveform will usually be maximal in amplitude when it is recorded using frontal electrode locations. For an **arousal to be associated with a K-complex,** the arousal must either occur **simultaneously with the K complex** or commence less than **1 second** following the termination of the **K complex.**
   - Tracking including automatic determination of **sleep spindle comprising of a** train of distinct waves with a frequency range of 11-16 Hz **(typically 12-14 Hz)** with a duration ≥0.5 seconds, usually maximal in
   - amplitude in the **central electrode derivations;**
- 2. **Automatic analysis capable of scoring stage N2** (with absence of criteria for N3) **if either or both** of the following conditions occur within the first half of that epoch or the last half of the prior epoch:
   - a. Tracking including automatic determination of **one or more K complexes not associated** with **arousals;**
   - b. Tracking including automatic determination of **one or more trains of sleep spindles;**
- 3. **Automatic analysis capable of continued scoring stage N2** epochs comprising **low-amplitude, mixed-frequency EEG activity** with an **absence of K complexes** or **sleep spindles** if they are preceded by epochs containing EITHER of the following conditions:
   - a. Tracking including automatic determination of **K complexes not associated with arousals;**
   - b. Tracking including automatic determination of **Sleep spindles;**

### SCORING STAGE N3

- **1. Automatic analysis capable of scoring stage N3** according to the following definition:
   - Tracking including automatic determination of **slow wave activity** comprising of waves of frequency ranging between 0.5 Hz and 2 Hz and **peak-to-peak amplitude >75 µV,** measured over the **frontal electrode placement regions;**
- 2. **Automatic analysis capable of scoring stage N3** when ≥20% of an epoch comprises of **slow wave activity,** regardless of age;

### SCORING STAGE R

- **1. Automatic analysis capable of scoring stage REM stage** according to the following definitions:
   - Tracking including automatic determination of **rapid eye movements (REM)** comprising of conjugate, irregular, sharply peaked eye movements with an initial deflection typically lasting **<500 msec;**
   - Tracking including automatic determination of **low chin EMG tone** comprising of **baseline chin EMG activity** derivation no greater than in any other sleep stage and typically occurring at the lowest level of the entire recording;
   - Tracking including automatic determination of **sawtooth waves** comprising trains of **sharply contoured or triangular,** often serrated, in frequency range of 2-6 Hz waves with maximum amplitude over the central head electrode location regions and often, but not in all cases, preceding a **burst of rapid eye movements;**
   - Tracking including automatic determination of **transient muscle activity** comprising of short **irregular bursts of EMG activity** typically with duration <0.25 seconds **superimposed on low EMG tone.** The **EMG activity** may be seen in the **chin** or **anterior tibial EMG derivations, in addition** to **EEG or EOG deviations. EMG signal in EOG derivations indicate** activity of **cranial nerve innervated muscles** (facial and scalp muscles and). The activity is maximal when association with rapid eye movements;
- 2. **Automatic analysis capable of scoring stage REM stage** in epochs with **all** of the following conditions:
   - a. Tracking including automatic determination of **low-amplitude, mixed-frequency EEG;**
   - b. Tracking including automatic determination of **low chin EMG tone;**
   - c. Tracking including automatic determination of **rapid eye movements;**
- 3. **Automatic analysis capable of continued scoring of stage REM sleep stage ,** regardless of an **absence of rapid eye movements,** for epochs following one or more epochs of **stage REM** according to the **above rule 2, if** the **EEG continues** to show **low-amplitude, mixed-frequency activity in the absence of K-complexes** or **sleep spindles and** the **chin EMG tone** remains low **for the majority of the epoch;**

### Title: eLifeCHEST/eLifeSCOPE (chestband) (Figure 5[3];[4]; Figure 16; Figure 31),

### ABSTRACT

### Problem with Traditional consumer-level health monitors

- Quality or healthy sleep deep is dependent on appropriate sleep architecture and in particular sufficient deep-sleep, including rapid eye movement sleep (REM-sleep; a.k.a dream sleep).
- Reliable or effective sleep monitoring cannot be achieved using traditional mainly wrist monitoring system, but rather requires proper monitoring of the brain, muscle tonicity and eyes.
- Insufficient deep sleep adversely impacts our daytime performance, energy levels, immune system, memory, occupational risks, and indeed our overall state of health and life-quality.
- Amongst children, poor quality sleep has been linked to low IQ and behavioural disorders, whilst amongst women with sleep disorders the unborn foetus health and even life is at risk with serious disorders such as hypertension or preeclampsia.
- Traditional professional-level sleep monitoring required laboratory-based or complex home-based monitoring in contrast to the present example which enables consumer-level monitoring with professional-level outcomes.

**The consumer-level with professional-level outcomes :**
- The solution comprises of a chest-worn band with a "companion" smart health watch system, NAS, and a special forehead-attached, self-adhesive wireless electrode array capable of enabling both traditional fitness or activity tracking and professional-level sleep monitoring, alike.
- Both daytime breathing and sleep disordered breathing is possible using a patent-pending chest-worn fitness/sleep chest-worn device.
- **Progressive-sleep** and **sleep-journal tracking** for your personal benefit or as a record to discuss with your doctor. ***eLifeSLEEP*** record includes clinical sleep and respiratory data accessible with online private-security "opt-in" health-data access function (RFM14935).
- Intra-sleep or fitness or health sleep progress, condition or tracking, along with corrective and preventative action recommendations (or hints; eLifeCAPA). i.e. REM, deep-sleep, sleep architecture, sleep goals, actual versus sleep goals. Measures can be measured as index, absolute values or comparative values (i.e. actual versus goals). Values can be referenced of comparatively presented in accordance to normative or disease population data base or normative or disease personal data base.
- Sleep disruptions or events can be tracked progressively with hints such as external noise disruptions such as yours or your partners snoring or other abnormal sound events.
- Sleep disruptions can be validated (i.e. events such as snoring or external noise can be instantly played back.
- **Professional and consumer-level sleep monitoring augmented with health tracking capability.**
- Professional-level (i.e. AASM or ASA) online sleep staging and respiratory analysis is enabled, in concert with feedback to clinicians, technicians via biofeedback or remote intervention including the optimisation of monitoring parameters or 3^{rd} party intervention, as required.
- Professional-level sleep monitoring (i.e. American Academy of Sleep Medicine/AASM study types i) to iv) and/or Australian Sleep Association/ASA levels 1 to 4).
- Diagnostic-level wake and sleep disordered breathing monitoring as well as professional-level medical surveillance, interpretation and reporting
- Traditional consumer-level fitness and health tracking capability
- **Online determination, monitoring, detection, tracking and alert of the onset or incidence of a subject's health status, health condition and/or fitness status.**
- Online onset notification (i.e. watch alarm/vibration) of daytime or nocturnal epilepsy, Parkinson's, enabling pre-warning, early intervention (such as medication) and potential avoidance.

### BACKGROUND

The present example comprises of patient wearable chest ***(******Figure 5******[3];[4];*** *Figure 16**;* ***Figure 31******)*** watch, wrist bangle, arm band, forehead sensor or other body worn device incorporating means of monitoring, detecting and alerting corresponding to the determination, prediction, onset computation, or incidence of psychological, physiological, pathological states and other biological events or health conditions of interest by way of utilisation of any of or any combination of the following subject monitoring sensors, physiological monitoring parameters, sleep/wake and other state determination and/or analysis capabilities comprising any of or any combination of the following:
**Physiological or environmental monitoring comprising any of or any combination of:** Sound monitoring; Stethoscope auscultation sensor, monitoring and automatic analysis, classification, tracking and detection capabilities; Acoustic noise cancellation system; Motion detection; REM sleep behaviour disorder (RBD); Pulse sensor integrated watch of other wrist worn device (band or bangle); Pulse wave analysis (PWA) and pulse wave velocity (PWV) monitoring and analysis capability; PWA and PWV sensors; Pulse wave analysis (PWA) sensor measures; Ballistocardiograph; Position, locational and movement sensing and monitoring; Gait or movement tracking and characterisation of events of interest; Movement and locational information; ECG sensor(s) and monitoring; Light sensor(s) and monitoring; Breathing band sensors and monitoring; EMG sensors and monitoring; GSR; Cardiac function; Heart rate; Sleep training system; Plethysmography Oximetry; Pulse transient oscillation amplitude measures; Temperature; Energy-exertion/metabolism-monitoring (EM) as a surrogate calorie-burn measure; **Sleep parameters;** Physiological and/or Sleep and/or Wake Markers; **Sleep parameters;** Sleep Architecture Measures; Environmental sensing; "dynamically-linked capabilities"; Psychological states; Whereby the present example comprises sensing, monitoring, data-acquisition, signal processing, analysis, storage, and information access including the online automatic characterisation of a subject/individual's physiological, neurological, nervous system, movement system, muscular system, psychological, pathological, states, events of interest and/or health conditions including any of or any combination of the following:
Rapid Eye Movement (REM) Sleep Characteristics ; Sleep Disorder Classifications; Select Sleep disorders; Dreaming States; HALLUCINATION STATES; Dissociated States; Hypnosis States ; and as further outlined here and elsewhere in this patent application document:

### Sound monitoring

The present example further comprising any of or any combination of:
- Integrated patient worn monitoring system incorporating **chest-coupled stethoscope sound monitoring sensor;**
- Stethoscope sound monitoring sensor incorporates a soft pliable chest to sound sensor coupling interface to enable monitoring of breathing sounds or associated pathological events;
- Sound sensor is designed to provide an **acoustic passive parabolic dish or other means of reflecting and focussing** (concentrate) sound waves towards sound monitoring sensor;
- Respiratory sounds contain significant information on physiology and pathology of the lungs and airways (RFM289).
- The present example is to produce an online or offline auscultogram with the option of computer-assisted or fully automatic event detection. automatic detection of normal and abnormal breathing sounds applicable to:
- **Fitness, action-based activities or other associated breathing or physiological changes in conjunction with breathing disorders,** such as (but not limited to) asthma, chronic obstructive lung disease (COPD), crackles, wheezes, snoring, stridor, cough, as well as sleep disordered breathing events including obstructive sleep apnoea (OSA), central sleep apnoea (CSA), hypopnoea (mixed combination of both central and obstructive sleep apnoea);

**Stethoscope auscultation sensor, monitoring and automatic analysis, classification, tracking and detection capabilities**
The present example further comprising any of or any combination of:
- Respiratory sounds contain significant information on physiology and pathology of the lungs and airways (RFM289).
- The present example provides an online or offline auscultogram with the option of computer-assisted or fully automatic event detection. Automatic detection of normal and abnormal breathing sounds applicable to:
   - **Asthma;**
   - **Chronic obstructive lung disease (COPD);**
   - **Crackles:** Discontinuous adventitious lung sounds, explosive and transient in character occurring frequently in cardiorespiratory disease), crackle detection as a possible sign of early respiratory disease in patients, assessing the effects of treatment of fibrosing alveolitis and monitoring the progression of asbestosis, squawks in patients with interstitial lung diseases, crackles, crackles followed by short inspiratory musical sounds referred to as squawks (a squawk rarely exceeds 400 ms and requires similar analysis to wheezes);
   - **Wheezes:** Continuous adventitious lung sounds, which are superimposed on the normal breath sounds. The definition of "continuous" is described by the American Thoracic Society (ATS) as being a wheeze of greater than 250 ms. The ATS also defines wheeze as high-pitched continuous sounds. Low-pitched continuous sounds with a dominant frequency of about 200 Hz or less are defined by ATS as being rhonchi sounds. There is some controversy associated with these definitions, amongst researchers, as wheezes have been reported to produce highly variable frequencies ranging from 80 to 1600 Hz, and according to other researchers 350 to 950 Hz. The new definitions of CORSA describe the dominant frequency of a wheeze as usually being > 100 Hz and a duration of > 100 ms;
   - **Snoring:** Snores are loud sounds with intensity above 50dB but mean energies as high as 85-90dB (A), subject to the recording technique. Snore sounds contain periodic components and are reported as having fundamental frequency between 30 and 250 Hz;
   - **Stridor:** Stridors are very loud wheezes. A trained examiner can recognize a stridor as supraglottic, or tracheal. Suctioning of ary-epiglottic folds onto the lumen of the airways during inspiration causes stridor. Stridor is characterized by a prominent spectral peak at about 1000 Hz;
   - **Cough:** Cough meter or holter device which recorded both breathing sounds of cough via a stethoscope microphone and cough activated abdominal EMG signals (via surface electrodes).
   - **Stethoscope:** The present example incorporates one or more contact sensors, accelerometers, attached to the chest wall with either adhesive rings or by way of rubber rings or belts, or microphones involving the direct recording of chest-wall movement, referred to as a "contact sensor" and/or acoustic approaches involving the recording of the movement of the diaphragm interfaced to the pressure wave created by the chest-wall movement. Due to the subtle nature of the chest-wall movement the present example enables breathing sound monitoring by way of coupling the diaphragm acoustically with the chest wall through a closed cavity. Breathing sound monitoring can be achieved by any of or any combination of piezoelectric contact sensors and/or condenser air-coupled microphones. The present example includes special mounting elements, including a coupling sound chamber between a chest-mounted health monitoring device sound sensor and an individual's chest.
   - Sound senor technologies can include acoustic and/or kinematic approaches such as:
      1) electromagnetic induction involving the movement of a coil in a magnetic field and thus inducing a current through the coil 2) condenser principal involving the changing distance between two plates of a charged capacitor and 3) piezoelectric effect being the generation of an electric charge charging with the distortion of the piezoelectric or polyvinylidene difluoride (PVDF) material.
   - Whereby one or more entire nights and/or days breathing parameters can be monitored online or offline via ***wireless or other network application service***
   - Whereby one or more entire nights (or sleep time) and/or entire days (or wake time) breathing sound parameters can be analysed online or offline in order to automatically scan and detect respiratory events of interest

### Acoustic noise cancellation system

The present example further comprising any of or any combination of:
- Acoustic noise cancellation system capable of distinguishing (using mechanical and/or electronic microphone focussing and/or unwanted sounds cancellation techniques) sounds of interest (i.e. directed or focussed towards breathing sounds versus unwanted or background sounds). One approach here is for a secondary microphone to be directed towards room or other unwanted surrounding environmental sounds in order to incorporate this signal in conjunction with establishing a sound cancellation signal capable of being combined with a primary microphone targeted towards a specific sound source interest - i.e. to produce a sound cancellation signal capable of filtering out unwanted background or environmental noise;
- Disordered and normal breathing can be classified breath by breath and associated with wake and/or sleep stages, individual's posture, cardiac functions and/or respiratory functions ;
- Sound sensor and monitoring bandwidth can span across a frequency range anywhere between DC to 20 kHz, enabling lower frequency breathing cycles and events to be distinguished along with the higher frequency wheezing and associated breathing physiological processes. Additionally, he dynamic range needs to be in the order of 100 dB or above in order to cover the softest breathing sounds through to the loudest snoring sounds, and/or
- Respiration rate monitoring capability.

### Motion detection and/or actigraphy

The present example further comprising any of or any combination of:
- One or more motion or movement detection sensors or systems capable of detecting the most subtle physiological tremors or cardiobalistogram vibration signals up more prominent body movement signals;
- Precision digital accelerometer or other sensor device, along with analysis capable of detecting movement disorders such as Hypokinesia (decreased bodily movement) such as Parkinson's disease, or hyperkinesia (exaggeration of unwanted motion), such as twitching or writhing in Huntington's disease or Tourette's Syndrome;
- Motion detection analysis incorporating any combination of ***linear (i.e. spectral analysis) and non-linear (i.e. spectral entropy or associated complexity analysis)*** for enhanced delineation and classification of motion, tremor, or other movements and vibrations signals including the automatic detection and recording of motion data or to assist in the diagnosis and treatment of movement disorders, including Parkinsonian Tremor, Restless Legs Syndrome, Dystonia, Wilson's Disease or Huntington's disease;
- Motion detection analysis incorporating the capability to detect movements and motion parameters associated with key disabling Parkinson's disease symptoms, including bradykinesia (i.e. slowness of movement) and dyskinesia (i.e. diminished voluntary movements; involuntary movements), or other muscular, neurological or nervous system disorders; and/or
- Capability of alerting or notifying user in terms of medication guidance;

### REM sleep behaviour disorder (RBD)

The present example further comprising any of or any combination of:
- Automatic detection of REM sleep behaviour disorder (RBD) defined by the incidence of REM sleep without atonia (suppressed muscle tone), where said "REM sleep without atonia" can be defined by comparing (electromyography) EMG signals indicative of muscle tonicity during non-rapid eye movement (REM) sleep stages and REM sleep stages, in order to determine periods when the normal reduction of EMG activity during REM sleep is not evident and the EMG characteristic associated with non-REM versus REM sleep EMG comply with pre-determined ranges, thresholds and variances. For example, where a certain number of or percentage of REM EMG periods or segments exceed a certain amplitude increase (i.e. 10 to 20%) compared to non-REM EMG amplitude outcomes.
- -Whereby said "REM sleep without atonia" can be detected by way of monitoring any combination of parameters and associated analysis including motion sensors, EMG signal monitoring, other **sleep parameters.**
- -Whereby said "Automatic detection of REM sleep behaviour disorder (RBD)" refers to monitoring, detection and/or recording of motion and/or sleep parameter data to assist doctors or individual's with the diagnosis and treatment of movement disorders, including Parkinsonian Tremor, Restless Legs Syndrome, Dystonia, Wilson's Disease or Huntington's disease or motion detection analysis incorporating the capability to detect movements and motion parameters associated with key disabling Parkinson's disease symptoms, including
- bradykinesia (i.e. slowness of movement) and dyskinesia (i.e. diminished voluntary movements; involuntary movements).
- Whereby monitoring of **sleep parameters** including any of or any combination of **EEG,** EMG and/or EOG or associated surrogate measures" can be by way of companion wireless linked single headband sensor system incorporating one or more sensors capable of monitoring one or more of any of or any combination of **EEG,** EMG and/or EOG signals
- Whereby said "single headband sensor" can include any of or any combination of sensors;

### Pulse sensor integrated watch of other wrist worn device (band or bangle)

The present example incorporates a means of measuring arterial blood pressure (pulse) via pressure sensor which can be incorporated as part of watch or watchband (i.e. buckle of watch band) which can incorporate a surface pressure sensor capable of monitoring detecting wrist vascular pulse changes via vascular (i.e. the ulnar artery or the radial artery) expansion and contraction, and corresponding vascular movements measureable at the subject's wrist skin-surface.

The present example further comprising any of or any combination of:
Said sensor can comprise of or additionally incorporate a Doppler blood-flow sensor capable of monitoring vascular blood-flow.

Said "measuring arterial blood pressure' can include ***"measures"*** of any of or any combination of:
➢ pulse pressure (PP; a marker of arterial stiffness - i.e. predicts cardiovascular risk);
➢ augmentation pressure (AP) derived from the aortic pressure waveform/ difference between the second and the first systolic peak);
➢ Pulse wave analysis (PWA);
➢ pulse wave velocity (PWV); and/or
➢ Augmentation index (Aix; i.e. defined as AP as a percentage of PP;

The said "measures" can be monitored via sensors forming part of a wearable wrist attached device, a sensor forming part of a wrist monitoring system (as demonstrated in Doppler watch example ***Figure 36******,*** or wrist tonometry example per figures ***Figure 37******,*** ***Figure 38******, or LHS photoplethysmography optical LED example of*** Figure 7, ***or RHS PVD Vascular detection example (tonometry) example of*** ***Figure 7******,*** or alternatively sensitive ballisogram vibrations can be detected from use of single or multi-axis accelerometer as part of a watch, wrist band, earplug monitoring, arm-band monitoring, or clip on or attachment of other wearable device (i.e. belt, clothing or body, head or body extremity);

### Pulse wave analysis (PWA) and pulse wave velocity (PWV) monitoring and analysis capability

The present example further comprising any of or any combination of:
- ***eLife Watch Pulse Wave Analysis (PWA) wrist-based pressure-pulse tonimetry version:*** Measurement of arterial stiffness by way of pulse tonimetry and determination subsequent pulse wave analysis (PWA).
- Incorporation of applanation tonometry with inflatable wrist sensor device (can be part of watch band or watch buckle region) capable of generating pressure in order to appropriately (using biofeedback the precise pressure can be determined by ensuring vessel is flattened but not occluded based on monitoring pulse -pressure variations at the point where the vessel is flattened (but not occluded) due to safely applied pressure against the artery against underlying bone.
- ECG signal for PWA and PWV can be derived from chest band ECG and/or wrist band or watch-integrated sensors.

### PWA and PWV sensors

The present example further comprising any of or any combination of:
- PWA an d PWV sensors can be based on pressure wave propagation/reflection characteristics, using any of or any combination of monitoring techniques of (1) tonometric, (2) oscillometric and/or (3) piezo-electronic. (i.e. PWV measured by tonometry coupled with electrocardiography (EKG).

### Pulse wave analysis (PWA) sensor measures

The present example further comprising any of or any combination of:
- Distance from sternal notch to carotid pulse site and distance from sternal notch to umbilicus and then to groin are measured. The probe captures the wave form at the carotid and femoral sites, and the time elapsed between the tip of the QRS complex and the onset, or "foot," of the pulse wave is calculated. Generally, 10 s worth of data are captured, for a number of beats (i.e. 6 - 11), which are averaged for the elapsed time at the carotid and femoral sites. The distance divided by time generates the velocity. Continuous PWV measure can be deployed using 1 but preferably 2 wireless or wire connected probes located at locations such as (but not limited to) carotid and femoral sites.
- ECG signal for PWA and PWV can be derived from chest band ECG and/or wrist band or watch-integrated sensors.

### Ballistocardiograph

The present example further comprising any of or any combination of:
- Ballistocardiograph comprising of the measurement of the cardiac output by monitoring the body movements caused by the contraction of the heart and ejection of blood into the aorta.

### Position, locational and movement sensing and monitoring

The present example further comprising any of or any combination of:
- Individual ***posture*/*position*** detection ;
- ***Step or movement*** detection;
- ***Fall-detection*** capability;
- ***Gait characteristics;***
- ***Gyrometer*** to assist determinations such as (but not limited to) for ***position,*** posture, fall-detection,;
- ***Global positioning system (GPS);***
- ***Altimeter sensor, and*/*or***
- **Associated** automatic analysis, classification , tracking, recording and reporting capabilities

### Gait or movement tracking and characterisation of events of interest

Patient wearable and/or mobile wireless ***Gait analysis*** - Gait analysis based on spectral FFT and spectral outcomes (few seconds to minutes) of arm-swing signal versus step signal and a measure of synchronicity with option of comparative analysis of change of any period of time and symptom determination applicable to movement, nervous system, muscular or neurological onset coincidence such as Parkinson's

Means of monitoring or sensing a subject/patient sensing (i.e. one or more axis accelerometer motions sensor) whereby monitored variables can be analysed by way of any of or any combination of:
- **spectral means including** but not limited to filtered bands of information and/or Fast Fourier Transform (FFT) in terms of phase, frequency, amplitude, bispectrum and other signal characteristics;
- ***segmentation of signal characteristics in terms of motion signal dynamics,*** where for example only, the regularity, the rhythm, the symmetry of movement with other physiological movement channels or variable can be assessed, and/or the orderly, predictability, repetition of movements versus randomised movements can be characterised (i.e. non-linear dynamic analysis; complexity analysis; entropy analysis etc.). In this way the trend of an individual's monitored and tracked characteristics in terms of ***detecting more fluid and rhythmic motion*** towards a less predictable, repetitive and symmetrical (i.e. in the context of a smooth or distinguishable underlying signal rhythm can be characterised and detected. Moreover, such tracking and trend analysis targeted at distinguishing early onset of movement, nerve, muscular or neurological motor control disorders such as Parkinson's onset can provide the most effective means for personalised health-assessment as well as professionally assisted health assessment in order to instigate the earliest possible therapy considerations and slow down or avert more serious and costly health consequences associated with developing chronic and more debilitating health conditions.

The present example incorporates the capability of distinguishing very early onset of such disorders is to combine any of or any ***combination of locational, motion*** (i.e. GPS, GSM, wireless signal mapping and other mobile wireless locational services or technologies), ***locational-movement and manoeuvre-ability and*/*or manoeuvring determination, with the subject's gait or walking characteristics*** in order to enable evaluation of more challenging movement scenarios (such as turning corners or manoeuvring) as a more poignant or more sensitive assessment periods in terms of ***distinguishing symptomatic gait or walking characteristics associated with the earliest onset and subtle trending of muscular, nerve, neural or other physiological motor movement disorder causations.***
- whereby such analyses can be deployed as a means of ***segmenting an individual motion in accordance to various typical movement categories such as (but not limited to) sitting, walking, running, sports activity determination, stride determination, jogging, sitting, general desk activity movements etc.***
   He present example ***enables one or more subject*/*patient wearable or attachable or applied devices containing movement sensor systems (s)*** (such as but not limited to one or more axis accelerometer sensors) to be monitored and analysed in a manner whereby the output of these one or more sensors can be combined in order to further analyse and characterise events or ***health conditions of interest and*/*or other prognostic or diagnostic motion characteristics of said subject*/*patient;***
   In ***contrast to the prior art¹⁸*** the present example incorporates the means of combining motions (i.e. via accelerometer sensor(s) with single or more axis of measurements from a plurality of location(s) (i.e. ***but not limited to wristband and*/*****or wrist-watch**,* and measures of any one or more body extremities and/or any one or more limbs of said subject/patient) in order to enable analysis of the interrelationship (patterns or signal morphology relating to movement between a plurality of motion sensor locations including coherence, correlation, symmetry analysis, concordance, etc.) or signal similarity (i.e. tracking and mutual rhythm or fluidity) between a between the variables of a plurality of motion sensor subject/patient sensing locations. In this manner (for example only) the symmetry or synchronisation between arm swinging and leg movements can be examined and ***measured at any point in time or over a period of time*** and in accordance to various standard or more difficult manoeuvers in order to determine trends or absolute indications of deterioration or change in gait (such as can be associated with ***onset of health conditions including (but not limited to Parkinson's);***

### Movement and locational information

The present example further comprising any of or any combination of:
Posture, position, fall-detection, gait characteristics, *step or movement detection,* locational (GPS; Gyro; altitude) information, movement sensing, along with automatic analysis, classification, tracking, recording and/or associated reporting capabilities.

### ECG sensor(s) and monitoring

The present example further comprising any of or any combination of:
- One or more integral ***electrocardiograph (ECG) sensors*** , such as carbonised rubber sensors integrated in chest-mounted device or associated attachment gear

### Light sensor(s) and monitoring

The present example further comprising any of or any combination of:
- ***One more light sensors***
- Optional ***smart watch light sensor with combining analysis function*** for enhanced environmental light determination

### Breathing band sensors and monitoring

The present example further comprising any of or any combination of:
- One or more ***respiratory*** inductive plethysmography and/or piezoelectric and/or polyvinylidene difluoride (PVDF) respiratory sensors or sensor bands

### EMG sensors and monitoring

The present example further comprising any of or any combination of:
- ***EMG sensor and monitoring*** via universal purpose ECG or separate EMG sensors such as carbonised rubber sensors integrated within chest-mounted device or associated attachment gear
- ***EMG monitoring*** enabling determination of breathing efforts for the purpose of delineating obstructive sleep apnoea versus central sleep apnoea

### GSR

The present example further comprising any of or any combination of:
- Galvonic skin resistance via existent chest-band sensors or via separate sensors.
- The skin resistance can provide a measure of perspiration and when taken in context with other signals such as (but not limited to) temperature and activity or movement (i.e. accelerometer) can provide a measure of energy exertion.
   - Whereby said means of GSR can include incorporation of a plurality of non-polarising electrodes applied to skin surface with a small constant current (i.e. 3 to 5 uV), whereby electrical resistance of palmar skin is proportional to the voltage potential developed between said electrodes.

### Cardiac function

### Cardiac function including sensors for continuous monitoring any combination of:

The present example further comprising any of or any combination of:
- ***Photo-plethysmography pulse sensor;***
- ***Pulse transient oscillatory amplitude, pulse wave amplitude (PWA)*** and/or ***pulse arterial tone (PAT)*** via any of or any combination of pressure pulse sensor, ultrasonic Doppler bloodflow, ECG, pulse signals, integrated photo plethysmography signal and/or reflective plethysmography oximeter;
- ***Obstructive versus central apnoea discrimination*** by way of ***correlating respiratory movements with respiratory effort,*** whereby respiratory effort can be determined by EMG and/or pulse transient oscillatory amplitude measures and/using thoracic and/or abdominal respiration circumference movement; and/or
- ***Ultrasonic and*/*or ultrasonic Doppler*** and/or duplex vascular blood flow, vascular and/or other cardiac monitoring.

### Heart rate

The present example further comprising any of or any combination of:
Heart rate variability monitoring capability

### Sleep training system

The present example further comprising any of or any combination of:
- Incorporation of an alarm or alert system such as integrated vibration capability whereby an individual position and/or sleep disordered breathing such as apnoea or snoring can automatically activate said alarm or alert in order to train sleeper to shift their sleep position (i.e. side versus back) in order to minimise or eradicate sleep disordered breathing or snoring symptoms.

### Monitoring, Determination and Tracking of Sleep, Wake and other Mental States, Events of Interest or Health Conditions of Interest

The present example enables deployment one or more said "subject worn ***system(s)"*** with ***device attributes*** as presented in this document incorporating one or more integrated (embedded or attached) sensors capable of sensing and monitoring one or more environmental parameters or physiological parameters, along with the determination, prediction, onset or incident of ***"**events*/*measures*/*states"*** or health conditions of interest, where said examples further comprise any of or any combination of analysis or monitoring capabilities including any of or any combination of "**sleep parameters**" and/or "**neurology parameters**", incorporating means of automatic online determination of consciousness or psychological states or associated events/markers including any of or any combination of events or conditions detailed under following headings (as further detailed elsewhere in this patent application document) including under sub-headings: **Whereby the present example** comprises **sensing, monitoring, data-acquisition, signal processing, analysis, storage, and information access including the online automatic characterisation of a subject/individual's physiological, neurological, nervous system, movement system, muscular system, psychological, pathological, states, events of interest and/or health conditions including any of or any combination of the following:** Rapid Eye Movement (REM) Sleep Characteristics; Sleep Disorder Classifications; Select Sleep disorders Dreaming States; Dissociated States; Hypnosis States,
**Whereby the present example** comprises **sensing, monitoring, data-acquisition, signal processing, analysis, storage, and information access including the online automatic characterisation of a subject/individual's physiological, neurological, nervous system, movement system, muscular system, psychological, pathological, states, events of interest and/or health conditions including any of or any combination of the following:**
Rapid Eye Movement (REM) Sleep Characteristics, Sleep Disorder ClassificationsSelect Sleep disordersDreaming StatesDissociated StatesHypnosis States, SCORING STAGE WAKE (Wakefulness), SCORING STAGE N1, SCORING STAGE N2, SCORING STAGE N3, SCORING STAGE R, (any of or any combination of these states or conditions and associated detail below are part of the *"**events of interest:**"* or *"**health conditions of interest**"* referred to throughout this document), as further outlined here :
**Whereby the present example comprises sensing, monitoring, data-acquisition, signal processing, analysis, storage, information access including the online automatic characterisation of a subject/individual's physiological, neurological, nervous system, movement system, muscular system, psychological, pathological, states, events of interest and/or health conditions including any of or any combination of the following:**
- determination of consciousness or psychological states including any of or any combination of **Whereby the present example comprises sensing, monitoring, data-acquisition, signal processing, analysis, storage, and information access including the online automatic characterisation of a subject/individual's physiological, neurological, nervous system, movement system, muscular system, psychological, pathological, states, events of interest and/or health conditions including any of or any combination of the following:**
   Rapid Eye Movement (REM) Sleep Characteristics; Sleep Disorder Classifications; Select Sleep disorders Dreaming States; Dissociated States; Hypnosis States,
   **Whereby the present example** comprises **sensing, monitoring, data-acquisition, signal processing, analysis, storage, and information access including the online automatic characterisation of a subject/individual's physiological, neurological, nervous system, movement system, muscular system, psychological, pathological, states, events of interest and/or health conditions including any of or any combination of the following:**
      Rapid Eye Movement (REM) Sleep CharacteristicsSleep Disorder ClassificationsSelect Sleep disordersDreaming States Dissociated StatesHypnosis States, SCORING STAGE WAKE (wakefulness), SCORING STAGE N1, SCORING STAGE N2, SCORING STAGE N3, and/or SCORING STAGE R (any of or any combination of these states or conditions and associated detail below are part of the ***"events of interest:"*** or *"**health conditions of interest**"* referred to throughout this document).

### Plethysmography Oximetry and/or photo-plethysmography (PPG)

The present example further comprising any of or any combination of:
- Pulse photo-plethysmography (PPG) and outputs ;
- Plethysmography oximetry (i.e. such as but not limited to ear, finger, and or forehead reflectance-based pulse oximetry) enabling to non-invasive measurement of oxygen saturation **(**SpO2) and pulse rate (PR), along with outputs and contribution to measures including pulse-wave amplitude (PWA), pulse arterial tone (PAT), pulse transient oscillation amplitude, pulse transient time (PTT; synchronised with ECG signals), PTT arousal, surrogate or qualitative blood-pressure measures, sleep stage confidence levels or probability based on vascular tonicity and autonomic disturbances; PTT can be computed using unique synchronisation with companion wearable device such finger or other limb attached oximeter capable of generating a pulse signal indicative of cardiac output
- Pulse transient time (PTT) monitoring capability incorporating means of synchronising with peripheral limb pulse measurement signal
   (***such* as *finger or pressure pulse measurement integrated within smart-watch or smart-watch-band system***)

### Pulse transient oscillation amplitude measures

The present example further comprising any of or any combination of:
- ***Pulse transient oscillation amplitude measures*** as a marker of ***inspiratory*** effort applicable to the discrimination of central versus obstructive sleep apnoea events (for example but not limited to) or ***investigation of autonomic and cardiac function***
- Thoracic ***impedance measurement*** via separate sensors or impedance measurement as or integrated within existent ECG or EMG sensors
- ***Heart output monitoring*** incorporating ***ultrasonic*** and/or ***Doppler blood flow*** sensor

### Temperature

The present example further comprising any of or any combination of:
- Embedded temperature sensor for monitoring body temperature;

### Energy-exertion/metabolism-monitoring (EM) as a surrogate calorie-burn measure

The present example further comprising any of or any combination of:

(See details in this document under sub-heading : **Energy-exertion/metabolism-monitoring (EM) as a surrogate calorie-burn measure;)**
- Energy dissipation by way of measuring the heat dissipated from the skin surface can be achieved by using one or more temperature sensor (i.e. polyvinylidene difluoride (PVDF) thermistor, thermocouple or other) placed in a manner whereby the heat or energy dissipated from the body can be distinguished from ambient temperature or weather elements. This process can include the separate measurement of ambient temperature of the environment or weather elements versus the heat generated via a subject's skin due to energy dissipation during sports training or normal day or night conditions. The final estimation of a subject's calorie burn can incorporate a number of measures comprising any of or any combination of skin galvanic resistance, movement or other measures of body exertion.
- Temperature of subject's skin surface, temperature of heat dissipated from the subject's skin surface (i.e. near or just above skin surface), ambient environmental temperature, cardiac parameters (i.e. oximetry, pulse, heart-rate), respiratory parameters (i.e. respiration rate, respiration volume, respiration pattern, respiration depth),amongst other measures including (but not limited to those covered herein (Sound monitoring; Stethoscope auscultation sensor, monitoring and automatic analysis, classification, tracking and detection capabilities; Acoustic noise cancellation system; Motion detection; REM sleep behaviour disorder (RBD); Pulse wave analysis (PWA) and pulse wave velocity (PWV) monitoring and analysis capability; PWA and PWV sensors; Pulse wave analysis (PWA) sensor measures; Ballistocardiograph; Position, locational and movement sensing and monitoring; Gait or movement tracking and characterisation of events of interest; Movement and locational information; ECG sensor(s) and monitoring;
   Light sensor(s) and monitoring; Breathing band sensors and monitoring;
   EMG sensors and monitoring; GSR; Cardiac function; Sleep training system;
   Plethysmography Oximetry; Pulse transient oscillation amplitude measures; along with psychological states (i.e. sleep, wake).
- The ultimate range of parameters enabling determination of a subject's ***calorie burn*** rate or absolute values requires extensive calibration of the ultimate algorithm in terms of a multivariate analysis combining of these said parameters.
- Additionally, heat dissipation can be measured using more sophisticated means such as infrared sensor enabling body-heat dispersion and emission characterisation enabling physiological temperature as well as temporal special dynamic heat imaging for ***more comprehensive and precise modelling of metabolism and associated calorie burn rates.***
- or imaging analysis whereby the heat pattern profiling associated with a subject's body's skin surface energy dissipation can be computed more accurately in terms of tracking actual calorie burn versus extenuating or confounding factors, including weather elements, localised skin surface temperature traits of a subject versus overall genuine or precision calorie burning measures, and the like.

### Wireless access

- Whereby one or more entire nights and/or days physiological parameters (including any of or any combination of those presented in this document) can be monitored online or offline ***via wireless or other network application service***
- Whereby one or more one or more entire nights and/or days physiological parameters (including any of or any combination of those presented in this document) can be ***analysed online or offline*** in order to automatically scan and detect respiratory events of interest
- ***Wireless gateway*** enabling ***expansion module(s)*** and including but not limited to smart-watch and/or chest-monitor based system
- ***Wireless expansion modules*** including unique wireless modules sensor modules suitable for other physiological parameters including (but not limited to) **sleep parameters**
- ***Expansion monitoring modules*** capable of enabling a range of sleep or respiratory monitoring ranging from screening functions to full polysomnography
- ***Unique distributed wireless monitoring sensors*** (DWMS)(see elsewhere wireless modules etc) .
- ***Unique distributed wireless monitoring network*** (DWMN)(see elsewhere body network etc.)
- ***Unique distributed wireless monitoring network*** (DWMN) with intelligent ***high-dependence automatic wireless network interconnectivity pathway(s) and cache meshing determination in*** accordance to health diagnostic factors and priorities such as available resources, communication path reliability, data-acquisition characteristics (i.e. bandwidth and capacity factors in accordance to signal bandwidth, signal sensitivity and dynamic range requirements, sample rate, sample resolution, filtering requirements and the like).

### Other System functionality

- Online monitoring, detection, classification, remote surveillance and/or intervention alerts, automatic report and/or alert distribution, and/or automatic medication delivery
- Capability of enabling health-carer, GP, family, specialist, or other trusted party to "opt-in" capability (i.e. means of a subject to enable specific information to be accessed by selected individuals) for possible intervention, monitoring or analysis or analysis interpretation oversight, remote surveillance, health management or control where necessary or appropriate according to regulatory requirements. Regulatory requirements can include FDA or CE or TGA compliance requirements, medical association/academy or insurance reimbursement and other legal or voluntary entities.
- Configuration capabilities for privacy, security & health-community "opt in capability" ⁹(RFM 14935) in order to abridge professional-level and consumer-level health personalised information access and distribution requirements, while maintaining the user's chosen level of information privacy and security
- Unique personalised companion smart watch display/alert management configuration
- Online personalised health-community application incorporating any of or any combination of: capability for establishment of personalised health monitoring, reporting, display and/or tracking program by way of individual configuration or adoption of any "public" or "private" health-community configuration examples; capability for establishment of personalised health outcomes goals, control their health information security and privacy, opt in or choose their health-support-community (GP, physician, specialist, friends, family, partners, services, HMO's, reimbursements, health insurance etc⁹(RFM 14935))
- Contact free EMF or other connector interfaced charging capability
- Automatic system configuration based on consumer-level or professional-level sensor kit deployed for particular monitoring session

### One example of Sleep monitoring

- In one example of the health platform any combination of a unique smart chest band, smart health watch and a distributed wireless sensor network system can be deployed to accommodate the simplest fitness training program to the most sophisticated health insurance reimbursed and American Academy of Sleep Medicine (AASM) recommendation for sleep studies (i.e. Type 2; type 3 or type 4).
- Moreover, the systems user can "opt in" his personalised community for oversight for a range of Government subsidised or pay-per-need online services. In this way regulatory and medically validated sleep monitoring can be an integral part of your daily healthcare anywhere management with the attachment of a unique fitness-sleep chest band device, the selection of your smart health watch and/or mobile wireless system and activation of your selected health-community and network application service.
- Even if you are travelling on an airplane your health platform can still provide offline monitoring (using monitor by wire mode where required), followed by more comprehensive services based on your personalised "opt-in" health-community and reporting requirements following NAS reconnection.
- Traditional consumer sleep monitoring systems, capable of monitoring and analysing "gold-standard" polysomnography **sleep parameters,** have not been successfully deployed at a consumer-level for a number of reasons.
- In particular, the well-established and broadly accepted scientific and clinical standards for effective and accurate sleep monitoring (RFM Rechtschaffen & Kales, 1968) (RFM Conrad et al., 2007).
- Specifically, and in contrast with touted claims of some health-tracker companies, effective scoring of human sleep requires appropriate monitoring of brain signals (electroencephalography), muscle tonicity (electromyography) and eye movements (electrooculography). In general, monitoring sleep or sleep quality is far more than simply detecting a reduction in movement using traditional motion-based consumer-level sleep monitoring systems.
- For example, sleep is classified into a number of stages comprising of Stage W (Wakefulness), Stage N1 (NREM 1), Stage N2 (NREM 2), Stage N3 (NREM 3), Stage R (REM). Note that Stage N3 represents slow wave sleep and replaces the Rechtschatten and Kales nomenclature of stage 3 and stage 4 sleep (Conrad et al., 2007). One of the essential aspects of sleep classification is the determination of rapid eye movement (REM) phases of sleep, as these REM phases contribute to brain recovery and restoration phases.
- These REM recovery and restoration phases contribute to daytime performance, memory and general brain function along with overall health and well-being status. Accurate determination of
- REM sleep, for example, is reliant upon determination of low EMG levels, rapid eye movements, and low level mixed frequency **EEG** signals. REM state **EEG** signals can be similar to wake **EEG** signals, which is one of the reasons that over simplistic health-trackers based on motion detection cannot effectively track sleep or sleep quality.
- The present example overcomes traditional limitation by using a combination of professional-level diagnostic monitoring and analysis services which can be seamlessly and automatically interfaced both on and offline.
- Additionally, the present example provides a highly integrated range of wireless linked and patient wearable modules including (but not limited to) a wrist worn watch device with built-in data modem, wireless capabilities, and special time-stamped data and synchronisation capabilities (i.e. to overcome traditional lost data packets and or wireless interruptions - see wireless body network system and high-dependent data NAS system described elsewhere (Burton, D (2014, March; eHealth provisional; system ⁹; RFM14935), companion wireless modules with automatic connectivity and system configuration capabilities enabling streamlined consumer-level deployment with sophisticated professional-level sleep and other medical health diagnostic and treatment outcomes.
- Additionally, the present example enables a consumer-based health-tracking or monitoring system comprising of one or more interconnected (i.e. but not limited to wireless) wearable health-tracking or fitness device to provide daytime cardiac, respiratory, ventilation and motions or movement tracking in conjunction with sleep-time breathing sound monitoring functions (sleep disordered breathing) in order to provide a true day/sleep health tracking system. This system in its minimal form is no less expensive, obtrusive or complex than the previous state of the art systems but capable of effective and accurate sleep disordered monitoring of common disorders such as snoring.
- In the more sophisticated but sill streamlined, sleek and non-obtrusive, consumer-based versions effective and accurate sleep monitoring, along with a substantial capability in terms of diagnosing and health tracking of sleep and associated sleep disorders is available as a consumer product offering, as further outlined in this document.

### Dynamically adaptive high-dependence connectivity management (HDCM) System Overview (Burton, D. 2014; RFM: Ref ID: 14935)

- The present HDCM system can be deployed to augment conventional mobile phone and computing technology network application services (NAS) in a manner where more crucial monitoring such as eHealth, industrial, certain consumer applications, and other applications where high-dependence and/or deterministic data interconnectivity is important or essential.
- The HDCM system can be configured to accommodate different levels of data prioritisation.
- HDCM system can be configured in an efficient user-interface manner whereby the data bandwidth allocation of one or more interconnectivity formations such as numeric blood-pressure, heart-rate, heart-rate variability, oxygen-saturation, C02 levels, respiration rates, temperature and the like can be segmented and not be overwhelmed (band-width-wise) from lower priority but higher bandwidth data (such as video, audio, or high sample rate physiological data.
- The HDCM system's context health analysis can compare a monitored individual's current health status as it relates to information including any of or any combination of: a) personalised database; b) population database; c) consumer/patient medical history.
- Additionally, the present example can simultaneously monitor and analyse environmental measures and accommodate adaptation of system resource allocation in order to counter issues such as wireless reception deterioration, during which time additional memory buffer resources are required.
- The HDCM system can "determine" whether negative or concerning trends occur in terms of health status.
- The present example provides High Dependence Connectivity Management (HDCM) capability to enable, for example, environmental conditions such as temperature and heating to be adjusted in accordance to an individual's requirements in order to achieve optimal sleeping conditions (predetermined or dynamically computed from monitored environmental and physiological conditions, for example).
- Normative Databases of standard or normal health, weather, hazard and other environmental conditions as well as current conditions, traffic reports and the like can be manually or automatically updated to provide both up to date reports on current or forecast conditions, but also enable comparison with normative data-base to establish important or relevant alerts, warnings, alarms, along with safe-margin determination & associated trend, alerts and alarms
- Map-linked views capable of associating health monitoring conditions or trends with environmental factors such as pollens, air-pollution or allergies.

### Description of Figures

***Figure 16*** eLifeCHEST; eLifeWRIST; Somfit;eLifeWATCH.
***Figure 17*** *ELifeCHEST with professional-level user interface option (including changeover watch module option)*
***Figure 31*** presents chest-band with embedded day-night breathing stethoscope (eLifeSCOPE) [3, 4] monitor for day-night breathing sounds, ECG, temperature, position and respiration [2], ECG sensors [5, 6].

### Title: Adaptive Physiological-Body Network (APM) & NAS GatewayOverview

- The **Adaptive Physiological-Body Monitoring-Network (APM)** sensor monitoring system enables a **group** or **cluster** of **companion monitoring sensors systems** to automatically adapt the corresponding **sensor system resources,** the associated configuration of these system resources, along with the wireless and data interconnectivity parameters based on the intercommunication factors evident at any point in time.
- Importantly, each sensor system, is configured in a manner where the appropriate minimal **data-acquisition characteristics** configuration based on the **sensor-specific monitoring function** of the said sensor system is predefined or dynamically determined. The said **data-acquisition characteristics** can, for example only, be defined in terms of **minimal, typical** and **maximal** configuration parameters.
- For example, an **EEG sensor system** could be based on a minimal configuration of 100Hz bandwidth with a 512 sample per second, 16 bit sample resolution and 8^{th} order low-pass Butterworth filter.
- However the **typical data-acquisition characteristics** may be configured for 200Hz bandwidth with a 1024 sample per second, 24 bit sample resolution and 8^{th} order low-pass Butterworth filter, while the maximal bandwidth could be configured for 800Hz bandwidth with a 4096 sample per second, 24 bit sample resolution and 8^{th} order low-pass Butterworth filter.
- The APM system can **automatically track degraded wireless transmission** together with **varying data bandwidth throughput capabilities** and associated **interconnectivity bandwidth capabilities to** determine the bandwidth transmission **constraints** in terms of achieving **consistent, continuous and reliable interconnectivity.**
- In the simplest APN deployment example the APN system could **revert from maximal** to **minimal or typical data-acquisition characteristics,** thereby greatly reducing the data interconnectivity bandwidth demands of the system so that peaks and dips in the **wireless interconnectivity can be dynamically compensated for online** by way of the **APN system.** In this way **moderate or minimal versus preferred or maximal data acquisition characteristics** can be deployed in order to extend the local sensor buffer and data preservation capabilities, while said communication failure or inhibition is evident.
- Additionally, the APM system can **model or simulate and continuously adapt or optimise wireless routing pathways** between any **combination** of **"local body network" groups or clusters of body applied sensor systems** (or associated monitoring systems/resources) along with body worn or independently separately located **wireless hubs or gateways. In this way** problematic interconnectivity issues such as degradation of wireless interconnectivity due to aspects such as body shrouding of wireless communication or wireless reflections and obstructions or interference can be mitigated by way of rerouting this combination of **local sensor system body network nodes** (can be configured as hub/gateway) and wider area communication hubs or gateways (can be configured as node).
- In some circumstances the **meshing of both local and wider area wireless and systems resources,** such as adapting the memory caching resources, is possible. For example, under extreme conditions, such as when an individual rolls on their back and shroud a back mounted sensor, it is possible of the APM systems to **locally cache** the monitored sensor data while the APM system **tests and/or simulates the local body network sensor system in concert with the combined with the wider area wireless gateway resources and interconnectivity capabilities** in order to reroute as required to minimise data loss risk. Furthermore, local **caching requirements** are **tracked to ensure local memory buffering** can be extended by **meshing memory buffering resources** across the local and wider network resources as required. All these considerations and **adaptations are conducted in context of the minimal physiological data acquisition characteristics** for each systems sensor and where required the need to adapt these said **data acquisition characteristics** in any manner whereby **the risk of data loss (i.e. minimise lost data packets) is most optimally mitigated in accordance to both the dynamics of the monitoring environment and the resources and interconnectivity options available on a combine local body sensor network and wider area network basis.**
- In this way the APN system continuously track, analyses, and determines the ongoing and current system resource and communication adaptation requirements while at the same time minimising the rate of changes and precisely logging any changes so that data is able to be precisely reconstructed and validated in terms of any **essential APM changes.** In this way system attributes and resources are determined and adapted online in accordance with the most appropriate system configurations based on minimal data loss and body monitoring information degradation.
- Importantly, any decisions or implementation in terms of **adaptation of wireless or monitoring attributes** is **deployed in concert with predetermined body monitoring minimal criteria** n order to ensure physiological monitoring or the integrity of associated data is not compromised.
- Moreover the APN system works both on a on a "local" body network basis" whereby one or more body monitoring sensor systems applied to an individual under monitoring can be adapted or **"meshed"** together in terms of **"sharing and adapting"** overall **local body network "systems resources"** along with the ability to similarly adapt wider communication network capabilities such **aggregating data interconnectivity** by way of deploying **multiple communication channels or mediums** (i.e. optical; satellite; Wifi , backup watch-dog (etc).

### Adaptive Physiological-Body Monitoring-Network (APM) System: update

- Wireless interconnectivity is **highly dependent** on many factors such as **electrical or electromagnetic interference, interference to transmission pathway** which can for example, **block, attenuate, reflect, deflect** or otherwise **degrade** transmission and reception signals. Additionally, in the case of an **wearable wireless monitoring** or **communication systems,** an **changes** in an individual's **movements, posture monitoring environment in general** or even environmental temperature and humidity conditions, can degrade and even block wireless intercommunication conditions.
- **For example,** during sleep monitoring an individual could roll over and **inadvertently degrade or block transmission** between wearable wireless sensors and a companion smart watch or bedside device with **in-build data modem.**
- The present Adaptive Physiological Monitoring example is able to dynamically track and **dynamically adapt wireless routing pathways** between two or more wireless hubs or gateways and/or nodes in a network of devices in order to compensate for **changing reception or transmission conditions** associated with an individual's movements; position; humidity, temperature other environmental conditions and environmental noise and other monitoring conditions, in order to ensure that pre-defined or dynamically determined minimal data communications associated with minimal sensor system data acquisition requirements, essential for consistent, continuous and reliable online real-time monitoring.
- The present example can adapt wireless routing pathways, wireless format (i.e. spread-spectrum, blue-tooth, Wifi, EMF coupling, in order to ensure the maintenance of minimal data conditions between two or more nodes or hubs in a local or wider area network of "companion devices" and associated gateways or backup devices per Dynamically adaptive high-dependence connectivity management (**HDCM**) System Overview (Burton, D. 2014; RFM: Ref ID: 14935). Whereby each said "companion device" is part of part of a network of **interconnecting devices.**
- The present example enables =sharing or **meshing of system resources** (i.e. **resources of any wireless devices in the monitoring network)** such as **memory buffering or caching** capabilities across a local **wearable device wireless network** so that data can be buffered across one or more **"companion devices".** Moreover the buffered data can be precisely time-stamped with channel and time information so that multiple channels of data can be reconstituted and synchronised at a later time, where required. Moreover the said reconstitution of the data can achieved by accessing data from any sensor system and associated resources in network of senor systems and/or related **HDCM system.**
- The present example enables **"adaptability and adjustment"** of data **acquisition parameters, signal conditioning parameters, wireless device resources, companion device resources, wireless interconnectivity characteristics, wireless interconnectivity formats and channels** in order to comply with **minimal predefined or dynamically determined data interconnectivity and monitoring requirements or standards and/or minimal data acquisition requirements,** whereby said "adaptability and adjustment" can include any of or any combination of parameters, systems resources, or other factors as detailed in "mobile high-dependence data management monitoring system with integral eHealth NAS and Atlas function (Burton, D. 2014; RFM: Ref ID: 14935).

### The present example further provides: Synclink

### Example

- One example involves **overcoming degradation of interconnectivity** associated with monitoring leg movements or finger oximetry via wireless sensors. If for example, wireless transmission is degraded due to shrouding caused by the monitored individual rolling over during sleep and smothering the wireless transmission pathways, this can be mitigated by way of the APM system. The APM system can **automatically compute,** by way of **simulation and modelling techniques, optimal interconnectivity strategies** comprised of rerouting the optimal transmission network and associated pathways. Said "strategies" could (for example only) deploy a less direct interconnection but more reliable transmission pathway. i.e. a wireless pathway via finger-sensor (i.e. oximeter probe) to head (such as **EEG** sensor system) or other body-located wireless sensor pathways (i.e. **Smart health watch or backup** high-dependence connectivity management (**HDCM**) system) exhibiting stronger data interconnection capabilities.
- Alternatively, under more **extreme conditions any interconnection and** combination of HDMC or sensor hubs or nodes, along with optimal sharing of resources (i.e. data acquisition memory buffering of network of sensor and hub systems) can be deployed so that risk of data loss is most effectively mitigated. In circumstances where signal transmission from any one or more sensors systems **adjustment of local and network buffering arrangements** can be deployed **until reliable wireless monitoring resumes.**
- **The present** example **further** enables consumer-level monitoring capabilities and sophisticated medical and scientific "gold-standard" physiological parameter monitoring, alike.
- The combination of APM and Dynamically adaptive high-dependence connectivity management (**HDCM**) System Overview (Burton, D. 2014; RFM: Ref ID: 14935) aspects enable traditional consumer mobile wireless network telecommunication network application services (NAS) to be deployed for reliable professional-level medical monitoring and diagnostic services.

### Example

- In one example per 4 step process and associated notes the present example has the capability of scanning the companion devices in order to automatically determine the study "format". For example, automatic "format" setting and can include any of or any combination of American Academy of Medicine (**AASM**) sleep study type 1 to 4 or Australian Sleep Association (ASA) level 1 to 4; SCOPER rating classification ; ECG event monitoring; holter ECG study monitoring; other cardiology studies; temperature monitoring; infrared monitoring; other health management monitoring; wireless-linked ear-bud health parameter monitoring (see also integrated ear-bud and eLifeCHEST monitoring capabilities system herein this document).
- Whereby "format" detection can be based on the detection of active companion devices the signal conditioning requirements and associated parameter settings (i.e. filters, sensitivity settings) , monitoring parameters, including data acquisition characteristics (sample rate, resolution, aliasing filters) can be automatically configured..
- Whereby "format" detection can be based on the detection of any "sensors and accessories", where said "sensors and accessories" have a means of generating a unique code or means of identification (i.e. each sensor and device attached or used by the system has an ability to generate an identification code (this identification code can be, for example, can be by way of superposition on input data or via a separate digital channel wireless or wire linked code system (for example only), in order to signal to monitoring system the total composition of electrode and sensors attached or connected as part of monitoring setup. In turn this electrode and sensor information can enable the system to determine what type of study has been configured and how this is classified in terms of scope of study (i.e. SCOPER classification) or medical organisation format (i.e. AASM type 1 to type 4 sleep study patient connection requirements). Additionally, the sensor or electrode determination codes signal to the system and enable automatic configuration of signal processing, data-acquisition analysis requirements.

### Other Adaptive Physiological-Body Monitoring-Network (APM) System Details ("Internet of Physiology") :

- The APM system in effect acts as a group or cluster of antennas capable of working together to compensate or override weaker signal transmission pathways, in circumstances whereby "shrouding effects", environmental IT conditions or environmental geographical aspects, patient changes in position in relation to surrounding walls or other structures effecting transmission, or other factors changing or impacting upon wireless transmission pathways.
- In particular, the APM uniquely deploys automatic or manual ***"adaptation"*** (i.e. ***system trade-offs according to "operating parameters"*** such as physiological signal conditioning, physiological signal acquisition, physiological signal processing, physiological signal data bandwidth, available data bandwidth and buffering capacity, and "system administrator" *ideal **versus typical versus minimal channel by channel physiological "operating parameters"***)*.* Whereby said system ***administrator can be defined or dynamically determined in terms of one or more authorised administrators assigned for example*** (but not limited to) assigned with "clinical", "technical", "scientific". "IT", "NAS", "patient", "doctor", "specialist", "consumer" or "Health management organisation", "health care provider or supplier" ***boundaries and access rights and associated type and levels of control applicable to different monitoring or study scenarios.*** And whereby said trade-offs enable a greater tolerance to both environmental interference and network connectivity factors applicable to physiological monitoring in hostile recording environments such as the typical consumer home, work or other uncontrolled monitoring locations.
- The APM incorporates automatic or manual *"**adaptation**"* whereby the most "appropriate" (highest overall and channel by channel signal frequency bandwidth, signal sensitivity, signal to noise ratio, and/or signal dynamic range can be achieved (based on study type, scenario and minimal, typical and ideal performance criteria and associated physiological sensor, physiological sensor-signal, physiological sensor signal conditioning, physiological sensor data acquisition properties, physiological data online transfer properties) .
- Said "physiological" can be substituted by industrial, consumer, medical, scientific, environmental or other online monitoring application or requirements.
- The APM incorporates automatic or manual ***"adaptation"*** whereby the most "appropriate" minimal data network or configuration of patient/consumer monitoring network resources (including most reliable wireless routing or pathways and the most effective buffering strategy and usage of available data memory buffers/caching facilities, companions wireless device resources (including memory buffering and wireless routing options) in order to maintain the pre-designated minimal criteria for each physiological monitoring channel.
- The APM incorporates automatic or manual ***"adaptation"*** whereby advanced strategies such as adaptable bandwidths and/or associated data acquisition and/or wireless data transmission channels and bandwidth requirements can be ***adjusted*/*adapted in accordance to minimal thresholds and operating ranges.*** These said minimal thresholds and operating ranges can be ***linked to system application*** (i.e. ***consumer-level versus professional medical level,*** for example only).
- In particular aspects effecting the ***reception and reliability of wireless monitoring such subject movements, shrouding,*** changing positions, clothing or blanket and other external conditions, changes in *location **and environmental conditions*** (ie electrical interference, magnetic interference, weather, clothing, furniture, building, etc.) ***that can otherwise impact upon continuous and reliable monitoring*** can in the main be countered and ***compensated*** for).

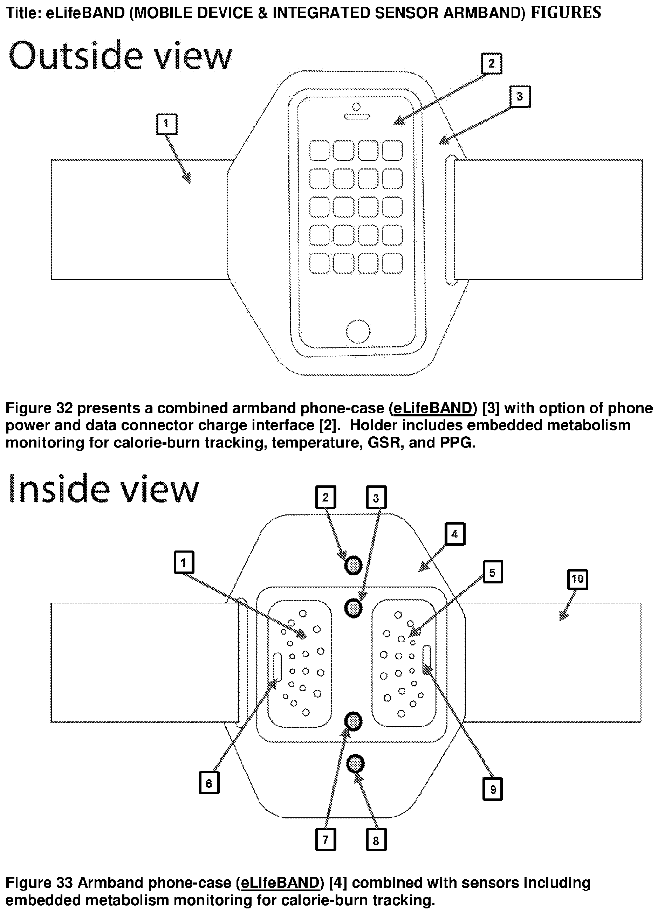

### Title: eLifeBAND (MOBILE DEVICE & INTEGRATED SENSOR ARMBAND) (Figure 32; Figure 33)

The present example can be incorporated into a patient wearable device such as (but not limited to) an arm band with one or more integrated (embedded or attached) sensors capable of monitoring one or more physiological parameters, enabling the present example to function as a physiological monitor and mobile wireless device holder ***(******Figure 32; Figure 33******)**,* further comprising any of or any combination of:
- integrated sensors including one or more physiological and/or movement or motion sensors and/or or GPS locational system.
- physiological sensors including those embedded within the patient wearable device (such as arm band) can include one or more infrared heating systems capable of monitoring the energy dissipation from a subject's skin surface.
- measurement capability comprising of the determination of "heat dissipation from the skin surface" can be by way of determination of any of or any combination of heat profile dissipated from a monitored subjects skin surface, hear of subjects skin surface, heat dissipation within said skin surface, heat dissipation of subject's blood and other organic matter, characteristics of energy ( hear) associated with subject's skin based on ambient conditions (i.e. control factor), characteristics of energy (heat) associated with subject's skin based on measurement device properties (i.e. determination of measurement method confounding factors);
- analysis capabilities including those associated with said "physiological monitor" can include the computation of a measure applicable to a subject's calories burning at any point in time;
- integrated (embedded or attached) sensors including one or more of any device(s) capable of measuring temperature including ( but not limited to) thermistor, thermo-coupler, polyvinylidene difluoride (PVDF) sensor(s);
- "physiological parameter" monitoring including combination of signals (physiological parameters) monitored from more than one subject wearable device;
- "physiological parameter" monitoring including combination of signals (physiological parameters) comprising any of or any combination of pulse, accelerometer, skin-surface heat dissipation, galvanic skin resistance, and/or temperature;
- one or more integrated (embedded or attached) sensors including any of or any combination of physiological parameters or determination of associated measures as covered elsewhere in this document (i.e. per below heading in this document ***"Energy-exertion*/*metabolism-monitoring (EM) as a surrogate calorie-burn measure;***
- one or more integrated (embedded or attached) can include any of or any combination of one or more physiological or other measures (i.e. weather elements, environmental conditions, location/GPS, and/or movement or motion);
- monitoring and measurement of "heat dissipation from the skin surface" including the capability for the determination of a monitored subject's heat profile associated with expiration of heat (i.e. resulting from physiological metabolic activity of the subject);
- measurement of "heat dissipation from the skin surface" can include the determination of a spatial dynamic temporal heat profile;
- measurement of "heat dissipation from the skin surface" can include the determination of a spatial dynamic temporal heat profile;
- physiological monitoring can include monitoring and determination of a calories burn computational algorithm whereby at least one input to this algorithm comprises of an infrared measurement;

### Energy-exertion/metabolism-monitoring (EM) as a surrogate calorie-burn measure;

- The present example enables a range of parameters enabling determination of a subject's calorie burn rate or absolute values requires extensive calibration of the ultimate algorithm in terms of a multivariate analysis combining of these said parameters, whereby said range of parameters can include any of or any combination of:
- Energy dissipation by way of measuring the heat dissipated form the skin surface can be achieved by using one or more temperature sensor (i.e. polyvinylidene difluoride (PVDF) thermistor, thermocouple or other) placed in a manner whereby the heat or energy dissipated from the body can be distinguished from ambient temperature or weather elements. This process can include the separate measurement of ambient temperature of the environment or weather elements versus the heat generated via a subject's skin due to energy dissipation during sports training or normal day or night conditions. The final estimation of a subject's calorie burn can incorporate a number of measures comprising any of or any combination of skin galvanic resistance, movement or other measures of body exertion.
- Temperature of subject's skin surface, temperature of heat dissipated from the subject's skin surface (i.e. near or just above skin surface), ambient environmental temperature, cardiac parameters (i.e. oximetry, pulse, heart-rate), respiratory parameters (i.e. respiration rate, respiration volume, respiration pattern, respiration depth),amongst other measures including (but not limited to those covered herein (Sound monitoring; Stethoscope auscultation sensor, monitoring and automatic analysis, classification, tracking and detection capabilities; Acoustic noise cancellation system; Motion detection; REM sleep behaviour disorder (RBD); Pulse wave analysis (PWA) and pulse wave velocity (PWV) monitoring and analysis capability; PWA and PWV sensors; Pulse wave analysis (PWA) sensor measures; Ballistocardiograph; Position, locational and movement sensing and monitoring; Movement and locational information; ECG sensor(s) and monitoring; Light sensor(s) and monitoring; Breathing band sensors and monitoring; EMG sensors and monitoring; GSR; Cardiac function; Sleep training system; Plethysmography Oximetry; Pulse transient oscillation amplitude measures; along with psychological states (i.e. sleep, wake).
- Additionally, the present example can measure heat dissipation by deploying infrared sensor or imaging along with analysis whereby the heat pattern or profile associated with the expulsion of heat (energy related to calorie burning) via the subject's body (skin surface) energy dissipation can be computed more accurately in terms of tracking actual calorie burn versus extenuating or confounding factors, including weather elements, localised skin surface temperature traits of a subject versus overall genuine or precision calorie burning measures, and the like.

### Summary (Figure 32; Figure 33)

***Armband-worn monitoring device*** with combined sleep, health and fitness incorporating a range of physiological parameter monitoring sensors including stethoscope monitoring, spatiotemporal dynamics of body-heat emissions (i.e. time-gated NFIR analysis) for enhanced calorie burn determination.

**Figure 30****, block [7]** Device: The ***calfit*** system integrates a metabolic or calorie burn measurement system within a wearable phone holder device.

The nub of this example is to enable a phone case (i.e. arm-band) to incorporate a series of measurement sensors and associated algorithms capable of superior determination of the effects of body energy generation as a more precise and personalised measure of an individual's energy exertion or associated estimation or determination of a subject's ***metabolism or related calorie burn**.*

The phone-case metabolic-measurement (**PCM**) comprises in one example of an armband phone-holder, incorporating measurement sensors for automatic online monitoring measurement and analysis of an individual's metabolism (i.e. ***calorie burn estimation)**.* Metabolism is the rate that your body burns calories, so tracking metabolism can be an important aid to weight loss.

The said measurement sensors in the simplest form a number of sensors embedded as part of the subject's ***phone-holder arm-band system**.* These arm-band phone-cases are commonly used by individuals and especially during exercise when one wishes to have phone access or playback music tracks during sports activities.

In one example, a phone arm-band holder can include one or more infrared temperature LED(s) and LDR(s) designed with ***time gate light activation*** of LED and associated receiver sensor (infrared LDR) in order to provide a degree of measurement-depth (i.e. able to be determined by way of ***time between gating on infrared LED*** and detection of return signals referenced to this time), enabling the ***characterisation of the heat transmission*** through and from the skin surface as a measure of body-heat expulsion. The infrared-LED transmitter and infra-red LRD receiver can comprise of ***near-field infrared (NFIR)*** monitoring sensors and/or associated systems.

In an example the pulse of the monitored subject can be measured using the said mobile ***phone's light generation and camera tracking*** capabilities as a means of tracking a subject's ***pulse rate via*** the measurement of the targeted vascular bed of the monitored subject.

In an example the ***internal accelerometer or movement sensor within the said mobile phone*** device can be deployed to measure the steps or motion of the said subject.

In an example the internal GPS within the said mobile phone device can be deployed to map the direction or geographical pathway and distanced covered by the monitored subject.

In an example ***temperature sensors*** (i.e. any combination or number of thermo-couplers, thermistors, and/or polyvinylidene difluoride (PVDF) etc.) can be embedded within the said arm-band phone holder and strategically positioned in order to ***delineate between*** factors such as background ***air-movement*** (i.e. resulting from subject movement or activity) or ***environmental*/*weather conditions*** (i.e. associated with a subjects movement or exercise and associated indoor or outdoor environment or weather elements) versus actual transmission of heat via the skin-surface resulting from energy dissipation of the body (associated with calorie burn).

In an example ***galvanic skin resistance (GSR)*** sensor(s) can be incorporated in the ***mobile-phone-holder*** in order to measure factors such as skin resistance as a measure of ***perspiration**,* energy exertion or effort or ***associated calorie burn factors*** of the monitored subject Whereby said means of GSR can include incorporation of a plurality of non-polarising electrodes applied to skin surface with a small constant current (i.e. 3 to 5 uV), whereby electrical resistance of palmar skin is proportional to the voltage potential developed between said electrodes.

In an example an ***inbuilt accelerometer*** or other movement sensor(s) can be incorporated in said ***mobile-phone holder.***

In an example an inbuilt ***GPS*** can be incorporated in said mobile-phone holder to ***map the direction or geographical pathway and distanced covered*** by the monitored subject.

In one example a ***companion wireless*** (i.e. Bluetooth, Wifi etc.) ***ear-phones*** (interconnected with mobile phone) enables the subject to have access to music and/or phone calls while on the move. Additionally, the said ear-**phones *can incorporate one or more health or fitness monitoring sensors*** as outlined further elsewhere in this document under the respective ear-buds sections.

In an example an inbuilt ***humidity sensor*** enabling the determination of surrounding weather conditions (i.e. also have an impact on effort or subject energy exertion as well as perspiration) can be incorporated in said mobile-phone holder to map the direction or geographical pathway and distanced covered by the monitored subject.

In an example an inbuilt ***airflow sensor*** (i.e. also have an impact on effort or subject energy exertion as well as perspiration) enabling the determination of surrounding weather and also airflow conditions associated with movement of activity of monitored subject.

In an example one or all of the above-mention sensors and associated measures relating to above-mentioned mobile-phone-holder and/or related to mobile-phone can be ***calibrated by way the monitored subject undergoing a defined or dynamically determined exercise sequence*** or circuit in order to determine various variables ***applicable to the specific subject's calorie burn estimation*** or determination.

In an example one or all of the above-mention sensors and associated measures relating to above-mentioned mobile-phone-holder and/or related to mobile-phone can be ***input to an algorithm*** (i.e. based as part of associated NAS, cloud-computing services, mobile-phone and/or armband holder device-based processing system) in order to enable the determination of a monitored subject's energy exertion (effort) and the associated estimation **or *determination of calorie burn.***

The present example further incorporates a means (i.e. EMF, connector interface for automatic phone system power interface and/or recharging of armband monitoring system) of self- contained power management or to avoid power or recharging considerations subject-monitoring ***phone-case** to **mobile-phone power-interface upon phone insertion, can be incorporated;***
Another example enables of an individual's ***medical or fitness or sports performance history*** or information to be incorporated as part the inputs to a health or fitness performance (such as metabolism or calorie burn determination/estimation), including factors such as body mass index and the like.

The present example enables any combination of any single or plurality of sensors and algorithm devices or functions described above and elsewhere in this document.

### Description of the Figures

***Figure 32*** presents a combined armband phone-case (eLifeBAND) [3] with option of phone power and data connector charge interface [2]. Holder includes embedded metabolism monitoring for calorie-burn tracking, temperature, GSR, and PPG.
***Figure 33*** Armband phone-case (eLifeBAND) [4] combined with sensors including embedded metabolism monitoring for calorie-burn tracking.

### Title: eLifeDOPPLER

### BACKGROUND

### Tittle: Doppler-watch 24/7 monitor

### BACKGROUND

Doppler ultrasonography and ultrasonography in general is an important vascular and cardiac measurement technique capable of assisting in the diagnosis or prognosis of a vast array of adverse and potentially fatal health sequaele.

However, the present state of the art monitoring systems have a number of limitations, particularly as it applies to ongoing monitoring in a range of real-world activities, circumstances and both psychological and physiological stress conditions.

The nub of the present example is to enable a ***means of enabling*** diagnostic, prognostic or pathological ***vascular and cardiac monitoring*** and analysis during wake or sleep based on a series of devices and methods including one or more ***wearable monitoring devices*** incorporating Doppler ultrasonography and/or ultrasonography applicable to continuous or selected periods of vascular and cardiac measurement and/or associated analysis characterisation for determination and/or prevention or mitigation of adverse or potentially fatal health sequelae, the present example further comprising any of or any combination of:
- incorporating system circumstances and thereby providing a comprehensive and accessible health tracking capability not available in the prior art;
- continuous and automatic analysis capability;
- ***means of enabling vascular and cardiac monitoring;***

### ULTRASOUND MODES

- Synchronisation or correlation between cardiac signals including any of or any combination of electro-cardiogram (ECG), -Doppler ultrasonic and/or ultrasound measures;
- B-scan ultrasound measures;
- Duplex ultrasound measures;

### ULTRASOUND PROBE ATTACHMENT MEANS

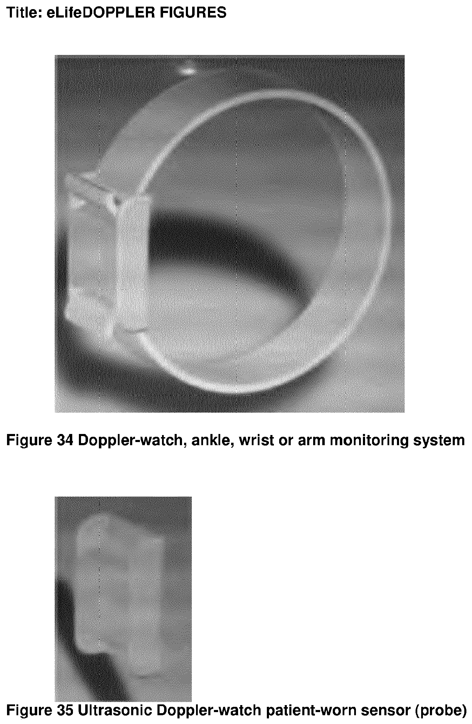

- the present example further comprises of a means of attaching an ultrasound probe (i.e. such means includes but not limited to ***ultrasound*/*piezoelectric sensor (probe)*** embedded or attached as part of a wearable watch, wrist or ankle (**Figure 34****)** or other device or parts or strap(s) or buckle associated with such device;
- whereby said ***ultrasound*/*piezoelectric sensor (probe)*** (**Figure 35**)is configured in order to apply appropriate or a predefined or dynamically determined pressure (i.e. via spring-loaded or motor controlled positioning and associated pressure-sensing arrangement) in order to establish ***low acoustic interface between said probe and monitored subject's skin surface*** to enable unimpeded transmission and reflection of ultrasound signals for further processing by the said monitoring device;
- whereby said device applies to wrist or ankle device or related strap, thus enabling suitable applied pressure, stability of attachment ultrasound sensor or probe, and also ***suitable coupling between ultrasound sensor or probe and monitored subject;***
- Said ***"suitable coupling between ultrasound sensor or probe and monitored subject"*** comprises of ensuring air-gap or other acoustic impedance disruption does not attenuate or overly distort signal ultrasound radiation and reflection pathway;
- whereby ***low coupling ultrasound acoustic impedance*** can be achieved by using ***pliable and low-acoustic impedance compound*** between the ultrasound probe and subject;
- whereby ***low coupling ultrasound acoustic impedance*** can be achieved by using a ***double-sided self*-*adhesive coupler pad*** which can ***incorporate ultrasonic imaging gel*** (low acoustic impedance) so that a durable ***long-term low acoustic impedance*** between monitored ***subject's skin surface and the ultrasound probe*** is achieved for good quality monitoring purposes;
- whereby ***low coupling ultrasound acoustic impedance*** can be achieved by using a reusable or disposable interface component comprising of ***coupler pad*** of material forming a low impedance coupling interface between the monitored ***subject's skin surface and the ultrasound probe*** is achieved for good quality (**low ultrasound acoustic coupling impedance**) monitoring purposes;

### ULTRASOUND WRIST VASCULAR MONITORING MEANS

- present example further comprises of a means of monitoring any of or any combination of subject's wrist, ankle, arm, and/or other subject limb or body extremity for any of or any combination of the following Doppler and/or ultrasonic vascular or cardiac characteristics, based on periodic or continuous monitoring of any of or any combination of:
   - Dual channel - radial artery and ulnar artery;
   - Single channel - radial artery and ulnar artery;
   - Single channel - ulnar artery;
   - Single channel - radial artery;

### ULTRASOUND ANKLE VASCULAR MONITORING MEANS

- the present example further comprises of a ***means of monitoring*** a subject's wrist and/or ankle and/or arm and/or other subject limb or body extremity for any of or any combination of the following Doppler and/or ultrasonic vascular characteristics and from said vascular imaging deriving any of or any combination of the following cardiac outcomes of a monitored subject:
   - whereby said ***means of monitoring*** comprises of one or more band(s) that can be worn by a subject and fitted to enable separate or simultaneous wrist and/or ankle locations in order to perform ***Doppler-pressure measurements (i.e. ankle-brachial-index-ABI);***
   - whereby said ***means of monitoring*** comprises of one or more band(s) that can be worn by a subject and fitted to enable separate or simultaneous wrist and/or ankle locations in order to perform ***Doppler-pressure measurements (i.e. ankle-brachial-index-ABI)*** in conjunction with blood pressure cuff;
   - whereby said ***means of monitoring*** enables a simple method for an easy and fast register of circulatory disturbances such as those associated with a subjects legs;
   - whereby said ***means of monitoring*** enables a simple method for an easy and fast register of circulatory disturbances such as those associated with a subjects legs whereby the bloodflow velocity at the A. tibialis posterior (ankle) and/or the A. tibialis anterior (instep) is measured with the Doppler, whereby a ***cuff at the shank*** can be inflated until the ***flow stops and the re-open*** until the flow returns; The current pressure at the cuff is the ***peripheral pressure*** of the measured artery. Then the same procedure is performed on the arm and the ***quotient between arm and leg pressure is an indication for circulatory disturbance, a stenosis or occlusion;***

### COMBINED APPLANATION TONOMETERY AND ULTRASOUND AND/OR DOPPLER ULTRASOUND MEASURES

- the present example further comprises of a means of ***monitoring a subject's wrist and*/*or ankle and*/*or arm and*/*or other subject limb or body extremity*** for any of or any combination of the following ***Doppler and*/*or ultrasonic vascular and*/*or cardiac characteristics*** and ***combining said characteristics*** with any of or any combination of the following monitoring and/or analysis outcomes of a monitored subject:
   - ***radial artery (******Figure 36******) applanation tomometery [1] comprising of applying a pressure sensor*** attached to the tip of a ***pressure-sensitive probe device,*** connected to a ***tonometery analysis system (i.e. smart watch device)*** which can record ***continuously*** or based on selected ***periods*** (i.e. 10 seconds for example) of ***arterial pressure*** in order to derive the associated ***ascending aortic pressure wave,*** from which a number ***cardiovascular measurements*** such as ***central aortic systolic pressure, aortic augmentation index, and the central pulse pressure can be derived;***

### PULSE TRANSIENT TIME (PTT) MEASUREMENT CAPABILITY

- the present example further enables pulse transient time measures based on determining the time between ***specifically selected points on the monitored subject's pulse wave*** as a measure of the time for the heart to pump blood to the point of monitoring, whereby the point of monitoring can be by way of using the ***ultrasound sensor or another pressure sensor*** to determine the pulse wave characteristics as a basis of such determination;
- whereby the said ***"specifically selected points on the monitored subject's pulse wave"*** are referenced to the subject's ECG signal;
- whereby any of or any combination of PTT and/or other ultrasound and/or tonometry and/or ECG measures and/or photo-plethysmography and/or cardio-ballistogram (i.e. motion etc.) and/or plethysmography oximetry measures or associated outputs can form the basis of a computation of estimated or surrogate blood pressure changes or values;
- whereby a ***plurality of movement sensors (***i.e. but not limited to accelerometer devices attached the subjects, body, head, body extremities, limbs etc.) can be analysed in terms of vascular or cardio-ballistogram pulse or pulse-shape characteristics in order to determine the accurate (i.e. using special time-synchronisation (i.e. ***multipoint time-synchronisation monitoring ; MTM** covered elsewhere in this document**)*** techniques as a means of determining with a common time-reference the time the body takes to pump the blood between two points (i.e. based on comparing similar vascular points on the pulse waveform) as a measure of pulse transient time with or without using ECG as a reference marker within such a determination; whereby said PPT measures can be used as a subject's surrogate or qualitative blood pressure marker;

### ULTRASOUND TO SUBJECT OPTIMAL INTERFACE COUPLING

- the present example further comprises of a means of ***monitoring*** whereby ultrasound and/or Doppler probe(s) can be applied to subject's skin with optimal pressure by way of any of or any combination of:
   - where said means of ***monitoring*** can be by way (but not limited to) the incorporation of a subject's ***surface-skin-applied pressure sensor*** to the wrist (or any other body limb, extremity, head or body part) of a subject whereby the ultrasound probe coverage of the wrist measurement sensor can ***provide the most reliable and robust measurement interface*** between a subject and the pressure or movement sensing device;
   - whereby the present example can incorporate one or more ***pressure-sensitive measurement sensor(s)* a*s part of a watch band*** (such as the buckle structure or region) or other wrist bangle device;
   - whereby the present example can incorporate one or more ***"pressure-sensitive measurement sensor(s)"*** to be applied in a manner where ***a spring-loaded mechanical probe (or other means such* as *inflatable or other adjustable part or motor driven mechanical part etc.) applies*** a suitable pressure (i.e. adequate pressure to allow and measure pulsations or relevant physical movement/pulsation of the subject's vessel under investigation, but not excessive to occlude vessel unless this is required as part of a specific measurement process) to enable the detection of vessel movement corresponding to the ***cardiac cycle*** and ***associated excursions of interest.***
   - whereby said optimal pressure refers to achieving low acoustic interface between ultrasound probe and skin surface in order to ***minimise artefact or ultrasound signal of interest attenuation or distortion;***

### WEARABLE TONOMETRY AND/OR ULTRASOUND AND/OR DOPPLER AND/OR PHOTO-PULSE AND/OR CARDIO-BALISTOGRAM PULSE AND/OR ELECTROCARDIOGRAPHY WEARABLE MONITORING SYSTEM

- the present example further comprises of a means of ***monitoring a subject's wrist and*/*or ankle and*/*or arm and*/*or other subject limb or body extremity*** for any of or any combination of the following ***Doppler and*/*or ultrasonic vascular and*/*or cardiac characteristics*** and ***combining said characteristics*** with any of or any combination of the following monitoring and/or analysis outcomes of a monitored subject:
   - electrocardiography signal;
   - ***radial artery (******Figure 36******) applanation tomometery [1] comprising of applying a pressure sensor*** attached to the tip of a ***pressure-sensitive probe device,*** connected to a ***tonometery analysis system (i.e. smart watch device)*** which can record ***continuously*** or based on selected ***periods*** (i.e. 10 seconds for example) of ***arterial pressure*** in order to derive the associated ***ascending aortic pressure wave,*** from which a number ***cardiovascular measurements*** such as ***central aortic systolic pressure, aortic augmentation index, and the central pulse pressure can be derived;***
   - a "tonometer- wearable" or attachable system **(i.e.** **Figure 37****)** where continuous and both wake and sleep tonometery can be monitored with a single device comprising of a pressure or movement sensor incorporating a means of ***monitoring*** the pulsations of any vessel of interest, applying the desired pressure or pressure sequence during measurement;
   - where said means of ***monitoring*** can be by way (but not limited to) the incorporation of a subject's ***surface-skin-applied pressure sensor*** to the wrist (or any other body limb, extremity, head or body part) of a subject whereby the pressure sensitive material flexibility and coverage of the wrist measurement sensor can ***provide the most reliable and robust measurement interface*** between a subject and the pressure or movement sensing device;
   - whereby the present example can further incorporate one or more ***pressure-sensitive measurement sensor(s)* as *part of* a *watch band*** (such as the buckle structure or region) or other wrist bangle device;
   - whereby the present example can further incorporate one be to enable the said ***"pressure-sensitive measurement sensor"*** to be applied in a manner where ***a spring-loaded mechanical probe (or other means such as inflatable or other adjustable part or motor driven mechanical part etc.) applies*** a suitable pressure (i.e. adequate pressure to allow and measure pulsations or relevant physical movement/pulsation of the subject's vessel under investigation, but not excessive to occlude vessel unless this is required as part of a specific measurement process) to enable the detection of vessel movement corresponding to the ***cardiac cycle*** and ***associated excursions of interest.***
   - the present example incorporates one or more ***wrist measurement device(s)*** whereby the radial artery is ***straddled via two outer supporting parts*** which allow a pressure sensitive probe which can be located between the two outer supports in a manner where the pressure to the subjects wrist is predetermined via the ***spring-loaded characteristics or via other controlled pressure means*** (i.e. via inflatable watch or other wrist cuff or motor driven positioning of tonometer measurement probe and associated pressure interface with a subject's wrist or vessel of interest). In this way the ***said pressure sensor probe (tonometery sensor)*** or a separate ***skin-surface-application-pressure sensor*** can provide ***feedback*** to ensure the optimal pressure (i.e. stenosis, occlusion or light pressure preventing vascular obtrusion, subject to measurement process of interest ) measurement on at any time is applied between the subjects wrist and tonometer pressure/movement sensor.
   - the present example further enables predefined or dynamically determined *sequences of vessel **of measurement non***-occluded, occluded, stenosis, or related degrees of occlusion to be metered out in a known manner, thus enabling a broad range of valuable blood-pressure, wave-shape cardiovascular measurements applicable to accurately deriving continuous every-day and nightly continuous or spontaneous measures relevant to the ***investigation of the cardiovascular and circulatory functions including (but not limited to) heart output, central aortic systolic pressure, aortic augmentation index, and central pulse pressure;***
   - the present example further comprises of monitoring **a *subject's wrist with annotated radial artery [1] and ulnar artery [2] applicable to measurement location in terms of "wearable-tonometer" for automatic continuous or spontaneous cardiovascular and circulatory system monitoring and functional performance and measurement determination (*****Figure 38*****)*.**
   - ***the present*** example further comprises of one or more ***wearable device(s)*** incorporating a ***ultrasound and*/*or Doppler monitoring system*** capable of monitoring the velocity of blood flow velocity in one or more arteries and/or veins applicable to the ***diagnosis or prognosis of peripheral vascular disease (PVD***)*,* including (but not limited to) artery occlusion and/or stenosis;
   - the present example further comprises of one or more ***wearable device(s)*** incorporating ***ultrasound and*/*or Doppler monitoring system*** whereby the said monitoring system incorporates an array sensor capable of electronic focus and/or beam steering capabilities;
   - the present example further comprises of one or more ***wearable device(s)*** incorporating ***ultrasound and*/*or Doppler monitoring system*** whereby the said monitoring system incorporates an array sensor capable of electronic focus and/or beam steering capabilities combined with a measurement scanning capability whereby any ***one or more ultrasound elements*** within an array of elements can be switched and activated, and/or whereby ***one or more ultrasound elements*** within an array of elements can ***automatically scan, detect and adjust and optimise ultrasound focus and beam steering to find and maximise ultrasound signal*** (i.e. from ulnar or radial artery);

### EXAMPLE OF THE PRESENT UL TRASOUNDIDOPPLER WATCH, WRIST BAND OR ANKLE BAND

In one example ***8 MHz piezoelectric "sensor probe"*** could be attached as part of a watch band in form of a ***wrist-watch ultrasound sensor monitoring buckle*** (Figure 13; ***[3]0.*** The location of the ***wrist-watch ultrasound sensor monitoring buckle*** can be positioned to measure vessels in the lower arm (peripheral) comprising of the radial artery [1] and ulnar artery [2] (**Figure 38**).

The said ***"sensor probe"*** can comprise of a single transducer part of approximately 5 x 5 mm, (i.e. 5mm circumference and 5mm depth) which can be attached or a part of a wrist or other limb, head, or other body extremity wearable device.

The said ***"sensor probe"*** can comprise of a single transducer part which can be configured to incorporate adjustment of the ***axis of the sensor probe of 30 degrees*** for example, in order to provide an appropriate ***insonation angle for precision measurement of blood velocity;***

The deployment of a ***fixed and known angle of insonation (i.e. 30 degrees)*** whereby a table of comparative values of blood velocity ***parameters can be referenced in order to confirm*** the likely location of vascular activity and detection of vessels of interest.

Alternatively the said ***"sensor probe"*** can comprise of an ***array probe,*** consisting of a number of piezoelectric (for example only) ultrasound sensors, such as a 4 array probe (i.e. 20 x 5mm).

Alternatively the said ***"sensor probe"*** can comprise of a ***rectangular piezoelectric probe*** (i.e. approximately 6 x 10 x 7mm).

Alternatively the said ***"sensor probe"*** can comprise of a ***Curved Array Probe*** using a small piezoelectric element (i.e. approximately 20 x 10 x 5mm).

The ultrasound processing, such as ***online FFT analysis can be supplemented*** by way of a telecommunication interface to a ***LAN, WAN, cloud-computing services or other network application services.***

The combination of local computer resources (i.e. smart watch) could enable any of or any combination of ***Doppler spectrum, M-mode, B-mode, Duplex, B-mode, colour display modes, and*/*or Doppler blood velocity modes of monitoring and display.***

***A Doppler-ultrasound-monitoring watch of approximately 50x50x15mm*** housing with display could be deployed. The local processing resources can deliver ***RAW Data (Doppler Quadrature) and a calculated envelope (max. velocities).***

### Description of Figures

***Figure* 7** eLifeWATCH with health monitoring sensor examples.
***Figure 9*** eLifeWATCH Doppler ultrasound example.
**Figure 34** Doppler-watch, ankle, wrist or arm monitoring system
**Figure 35** Ultrasonic Doppler-watch patient-worn sensor (probe)
**Figure 36** Subject wearing Doppler-watch

### Title: eLifePULSE

### Description of Figures

***Figure 7*** eLifeWATCH with health monitoring sensor examples.
   The **tonometry sensor [2]** is part of a wrist band or wristwatch band **[10].** The **tonometry sensor [2]** can comprise of a piezo-electric or PVDF sensor capable of tracking pulsations and pressure changes at the surface of an individual wrist due to the tracking of the expansion and contraction wrist-based vessels such as the radial or ulnar artery, and by way of tracking these vascular movements as pressure changes indicative or the cardiac blood-flow circulatory function.
***Figure 9*** eLifeWATCH Doppler ultrasound example.
***Figure 37*** An example of a eLifeWATCH or eLifeWRIST "tonometer- wearable" or attachable system.
***Figure 38*** presents a subject's wrist with annotated radial artery [1] and ulnar artery [2] applicable to measurement location in terms of "wearable-tonometer" for automatic continuous or spontaneous cardiovascular and circulatory system monitoring and functional performance and measurement determination.

### Title: eLifeMOTION

### PATIENT WEARABLE MOTION ANAYSER AND AUTOMATIC ANALYSIS

**The present examples enable one or more wearable devices (i.e. attached or applied to limbs, body, head or other body extremities but also applicable to implanted or physiologically attachable systems) with means of enabling diagnostic or prognostic monitoring applicable to *"monitoring relevant parameters"* and *"corresponding analysis determination and characterisation"* applicable to the onset or *"detection of events or health conditions of interest",* further comprising any of or any combination of:**
- Whereby said ***"monitoring relevant parameters"*** includes (but are not limited to) any of or any combination of motion, travel pathways, geographical locational pathways (i.e. GPS; GMS), accelerometer measures (i.e. range of different multi-axis accelerometer sensors), subject-posture or position measures, properties or patterns of movements, spectral nature of monitored movement signals, signal dynamic properties (i.e. non-linear dynamic, complexity, entropy analysis etc.) of monitored movement signals, coherence interrelationships between monitored movement signals, symmetry, multi-source (i.e. limbs, body, head and other extremities) movement symmetry and/or fluidity and/or synchronisation and/or patterns and/or gait and/or associated trends or changes over time;
- Whereby said ***"corresponding analysis determination and characterisation"*** include (but are not limited to) any of or any combination of motion, travel pathways, geographical locational pathways, patterns of movements, spectral nature of monitored movement signals, signal dynamic properties (i.e. non-linear dynamic, complexity, entropy analysis etc.) of monitored movement signals, coherence interrelationships between monitored movement signals, symmetry, multi-source (i.e. limbs, body, head and other extremities) ***movement symmetry and*/*or fluidity and*/*or synchronisation and*/*or patterns and*/*or gait*** and/or associated trends or changes over time;
- Whereby said ***"detection of events or health conditions of interest"*** include any of or any combinations of *"automated method of determining* **neurological prognosis or diagnosis"** *or personal-care management* of a *subject's* a nervous system, ***muscular or movement system,*** neurological disorders or highly prevalent neurological disorders including autism spectrum disorders (ASD, Asperger's), Epilepsy and other seizures, ***Parkinson's**,* attention deficit hyperactivity disorders (ADHD), Huntington's, traumatic brain injury, depression, anxiety and other subject states, transitions or events and health conditions of interest;

- the present example further enables a plurality of patent wearable devices or associated monitored outcomes (i.e. attached or applied to limbs (such as but not limited to ankle straps and wrist devices to characterise arm swing versus leg movement physiological motion characteristics along with interrelationships), body, head or other body extremities but also applicable to implanted or physiologically attachable systems)
- the present example ***further*** enables a ***combination of outcomes associated with motion analysis or a subject and*/*or with sleep-behaviour disorder*** outcomes and/or subject/patient information (i.e. but not limited to hereditary disease factors and/or idiopathic nature of SBD **(*****Figure 75***) section and other disorders applicable to prognostic, diagnostic but also early onset and hence treatment or therapy indicators applicable to early intervention, prevention, reversal or delay of full-blown said ***events or health conditions of interest" ;***
- an ***example*** includes the deployment as a useful physiotherapy measure and tracking system whereby automatic motion signal monitoring, motion characterisation and determination of events of interest can contribute to improved subject or patient outcomes;
- an ***example*** includes the deployment as a useful rehabilitation tool (i.e. therapy following stroke, accident of other causation of movement or other motor skill dysfunction) dysfunction) whereby automatic motion signal monitoring, motion characterisation and determination of events of interest can contribute to improved subject or patient outcomes;
- an example includes the ***deployment as a measure of breathing movement signals*** (i.e. sleep disordered breathing or activity respiration tracking) whereby anchoring, attaching or applying a motion monitoring sensor (i.e. accelerometer with one or more axis of sensing) to a physiological structure directly or indirectly coupled to the lung or associated breathing movements (i.e. but not limited to the sternum, collar-bone, ribs, other body extremities, etc.) enabling automatic motion signal monitoring, motion characterisation and determination of events of interest can contribute to improved subject or patient diagnostic, prognostic status or associated treatment outcome measures, Such a device can be a part of a head mounted (i.e. Somfit forehead sensor) monitoring sensor or system, or otherwise a wireless interconnected system capable of supplementing an existent sensor monitoring capabilities. The present example further has the option of deriving sleep disordered breathing information from such breathing movement detection.

### Title: eLifeALERT

### BACKGROUND

While the present state of the art provides reflective oculography as part of measuring eye movements indicative of drowsiness or changes in vigilance/attention state the present example incorporates high resolution and frame-rate video scanning techniques capable of accurately deducing changes in eye-lid muscle velocity, which in turn can be associated with drowsiness. Other combinations or monitoring and analysing a range of facial features, eye-gaze and head movements, coupled with steering wheel pressure, steering wheel movements, and various other subject/user/driver behavioural and operational characteristics can be combined to provide an driver attention/drowsiness tracking accuracy superior to conventional state of the art technology.

**The present examples incorporate a vehicle mounted and/or subject wearable diagnostic or prognostic monitoring system(s) incorporating the capability of automatic analysis applicable to the determination of the onset or incidence of *"health conditions*/*disorders or events of interest"* such as (but not limited to a subject's drowsiness or reduction of vigilance), and further comprise any of or any combination of:**
- **incorporation of communication interface(s) comprising and one or more WAN, LAN, IP, NAS, and/or Cloud-computing services services peer to peer information telecommunication mediums or systems to enable the transmission of physiological or environmental monitored data and/or subject/patient information exchange and/or access to online analysis capabilities capable of supplementing local monitoring, analysis, storage, and/or indications;**
- **Whereby determination of the onset or incidence of "health conditions/disorders or events of interest", include enabling an "automated method of determining neurological prognosis or diagnosis" or personal-care management of a subject's nervous system, muscular or movement system, neurological disorders including autism spectrum disorders (ASD, Asperger's), Epilepsy and other seizures, epileptiform discharges, bursts of muscle activity, other muscle activity, Parkinson's, attention deficit hyperactivity disorders (ADHD), Huntington's, traumatic brain injury, depression, brain cognitive functional or anatomical regions (i.e. defined or dynamically determined by standardised mapping or atlas definitions or special area of interest including but not limited to *suprachiasmatic region associated control or interconnectivity regions of the brain),* anxiety and other subject psychological states, *vigilance states, attention states, drowsiness states, micro-nap states, sleep or wake* states, associated transitions, and/or other events and health conditions of interest;**
- Vehicle control system(s);
- Vehicle video system(s);
- Subject/driver video monitoring system(s);
- Subject/driver infrared light emitting diode(s) transmission and corresponding light dependent resistor (LDR) sensor receiver sensor(s) monitoring system(s);
- vehicle attached and/or embedded subject/driver infrared light emitting diode(s) transmission and corresponding light dependent resistor (LDR) sensor receiver sensor(s) monitoring system(s);
- vehicle attached and/or embedded subject/driver infrared light emitting diode(s) transmission and corresponding light dependent resistor (LDR) sensor receiver sensor(s) monitoring system(s) applicable to light emitting diode (LED) with accompanying light dependent resistor (LDR) and/or infra-red light emitting diode (irLED) with accompanying infrared light dependent resistor (irLDR) applicable to Binocular or single eye video/and/or infrared oculography and/or combined video and Binocular (or single eye) infrared oculography;
- Subject/driver **EEG** monitoring system;
- Vehicle control or vehicle system sensor outputs; or
- Vehicle computer data;
- **The present examples may further comprise any of or any combination of: (per caption** **Figure 2****)**
- **VISION INDEX BLINK-SPEED / SPAN (VISE).**

The present examples enable conversion of any of or any combination of a video image(s) of a subject's eyes, eye-lid, face, head or other body extremities into a syntactic representation based on one or more tracked points such as visual video derived fiducial markers representative of subject movement region of interest (i.e. eye lid, eye-focus, etc.).
- In this manner a data file or representation of motion (i.e. eye-lid velocity or acceleration) can be derived in a non-obtrusive and non-contact or video format.
- An example of video measures indicative of driver drowsiness can comprise of the characterisation of eye-lid movements (blinks) based on the diagnosis or prognosis of drowsiness levels (i.e. indicative of tonic suppression with drowsiness supressing responsiveness or velocity of eye-lid blink rate) or drowsiness-risk based on Vision Index Blink-speed / span (VISE), whereby a ratio of the displacement of eye-lid span (i.e. linear measures or angular opening of eyes) versus the speed (velocity) of eyelid motions can be tracked. Moreover the video monitoring aspect can be integrated into a wearable device such as glasses or alternatively incorporated in a less obtrusive context such as the attachment or incorporation of one or more cameras as part of the subject's environment (i.e. occupational location, any transportation vehicle interior, traffic control environment, aircraft cockpit, ship control room or other).
- Additionally, the said video monitoring systems can be incorporated as part of an **EEG** monitoring system (i.e. embedded as part of video glasses or other wearable **EEG** monitoring device) and/or driver movement detections system;¹⁹⁻²¹
- Additionally, an the present example incorporates threshold level(s) as a marker of excessive drowsiness, whereby said threshold marker is indicative of an individual subject (i.e. via calibrated and personalised data means) or determined from any of or any combination of normative data. In this way the VISE measure can be configured to generate an alert or notification to the subject or other party when a predefined or dynamically determined threshold level is exceeded, or when the safe operational region transitions to a region of unacceptable drowsiness risk level.

The present examples may further incorporate means of *converting an image to a syntactic **representation comprising of a temporal sequence*** of events representative of the motion characteristics of a subjects eyelids;
- Whereby the said ***"converting an image to a syntactic representation comprising of a temporal sequence"*** further comprises of identifying at least one point of eye lid movement of a subject so that the precise motion and changes over time of the eye-lid motions can be tracked;
- -Whereby said "events of interest" can include (but are not limited to ***suprachiasmatic brain region changes associated control or interconnectivity regions of the brain;***

**EYE TRACKING APPLICABLE TO VIGILANCE, DROWSINESS, SMART CAR AND OTHER APPLICATIONS: monitoring and analysis associated with** eye-lid motion and and/or subject head-motion and/or forward road or travel pathway video and/or locational pathway (i.e. GPS and/or GMS) and/or rear road video and/or EOG signals and/or **EEG** signals including spectral road subject eyelid tracking for vigilance or drowsiness comprising of any of or any combination of or any combination of:
- ***high definition video based on updated scan rates*** greater than twice the time for any eye-closure or eye opening from totally open to totally closed and vice versa (i.e. typically 1/100 sec X ½ or at least 200 video scans of eye-lid region per second but up to 10 times the speed requirement or 1000 frames per second) preferably up to 10 times or greater speed of eye opening or closure span;
- ***high definition video based on pixel resolution*** capable of enabling accurate determination of eyelid velocity';
- ***high definition video based on pixel*** resolution capable of enabling accurate determination of eyelid acceleration (increase of speed or velocity) or deceleration (decrease of speed or velocity), whereby in one example a measurement of eye-closure or opening of 5mm (for example) would require in order to reduce calculation error of velocity to below 10% would demand a resolution of at least .5mm per pixel or based on Nyquist theorem limitation at least .25 mm (eye closure or eye opening measurement) per pixel. Whereby the acceleration of the eye movement could be required to detect not only 2.25 mm (based on 10% error) but preferable 10 time or more in terms of resolution to enable the subtle changes of acceleration associated with changes or early onset of drowsiness detection (i.e. .025 mm resolution based on example of 5mm real-world eye closure or eye opening span)) - therefore a pixel resolution of.025 or more can be achieved with a combination of multiple camera approaches and high pixel resolution;
- whereby ***said multiple camera*** can include one or more cameras designed to determine head position and a second camera with focus on a smaller area of the subject's head but particularly the subject eyes and eye-lid closure region of vision;
- ***said "focus"*** can include any of or any combination of ***electronically and*/*or mechanically and*/*or digitally steered*/*controlled*** camera (i.e. pan, zoom, focus etc.) arrangement (i.e. means includes linking said tracking to steering camera that looks to the locational position of head and eyes and then each eye-lid measurement region in order to control or guide primary eye-lid measurement camera i.e. a first camera locating head working in conjunction with a second camera able to measure accurately (i.e. 10 to 100 or more times pixel resolution of the eye-lid movement span) in order to accurately track values and changes in percentage of time the eye-lids are closed and/or eye-lid velocity and/or eye-lid acceleration at any point in time as a marker of drowsiness onset.

### BASELINE AND/OR SUBJECT/DRIVER SPECIFIC (PERSONALISED) REFERENCE-LEVEL CHARACTERISATION AND DETERMINATION ANALYSIS CAPABILITIES APPLICABLE TO MONITORIED PERIOD (I.E. PER ANY PRIOR NUMBER OF MONITORED BLINKS OR TIME-PERIOD) APPLICABLE TO VIDEO, INFRARED OCULOGRAPHY OR OTHER FACE AND/OR EYE AND/OR SUBJECT/DRIVER SENSED BEHAVIOUR OR PHYSIOLOGICAL PARAMETER MONITORING OR ANALYSIS);

The present example may further include the incorporation of a ***subject*/*driver profile within a pre-defined (i.e. during initial 10-20 minutes of driving) or dynamically determined,*** in order to establish a baseline determination in the context of determining how the driver/subject operates the vehicle, whereby an algorithm (i.e. any of or combination of analysis algorithms described in this provisional application) can constantly compare how the driver performs to the baseline or reference data (i.e. can be based on any of or combination of the monitored or derived parameters described in this provisional patent application);

The present example may further include the incorporation of an analysis process whereby **at the start of any or every diver/subject trip in a vehicle an analysis algorithm (based on any of the subject/driver monitored or derived parameters described in this provisional application) can compute a subject/driver's individual or specific driver-profile, whereby this said profile can then be** continuously compared with current monitored or sensed data. In this manner a transition from vigilance (i.e. satisfactory wakefulness as predefined or dynamically computed) to drowsiness (i.e. an unacceptable level or attention and/or alertness) can activate a driver/subject warning.

### -OTHER ELECTROENCEPHALOGRAPHY (EEG) CHARACTERISTICS APPLICABLE TO MONITORIED PERIOD (I.E. PER ANY PRIOR NUMBER OF MONITORED BLINKS OR TIME-PERIOD) APPLICABLE TO VIDEO, INFRARED OCULOGRAPHY OR OTHER FACE AND/OR EYE AND/OR SUBJECT/DRIVER SENSED BEHAVIOUR OR PHYSIOLOGICAL PARAMETER MONITORING OR ANALYSIS);

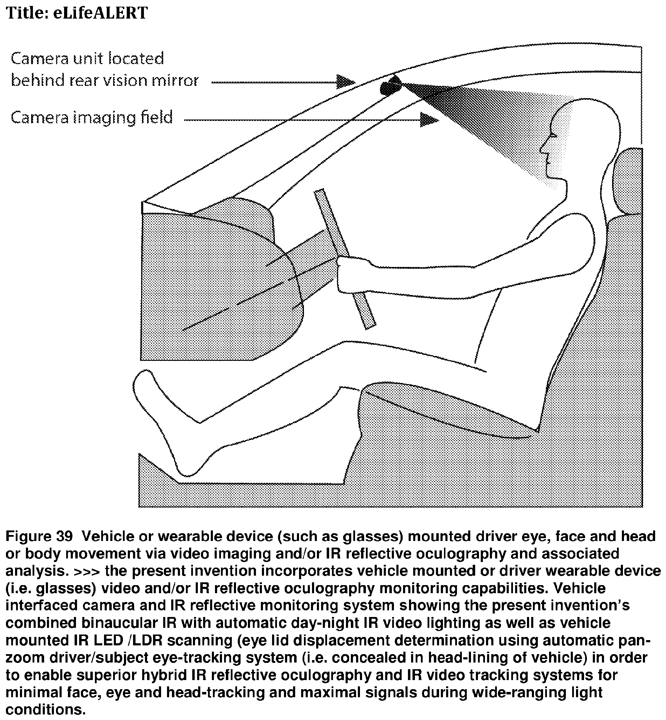

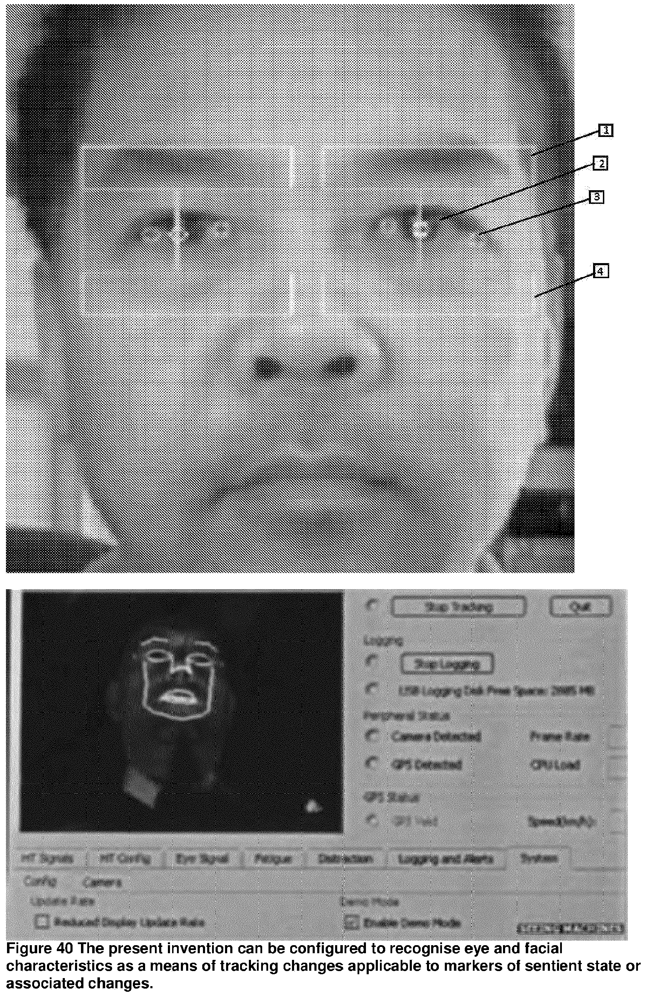

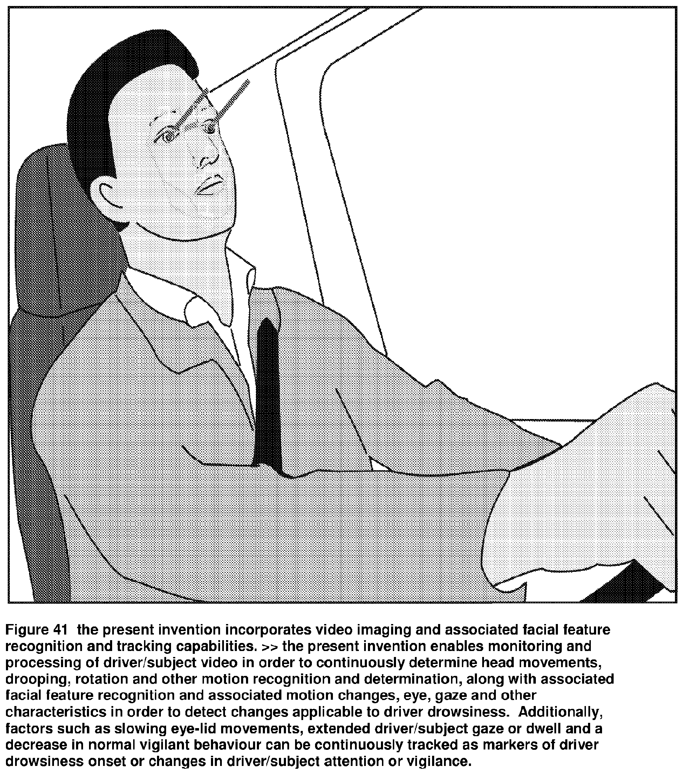

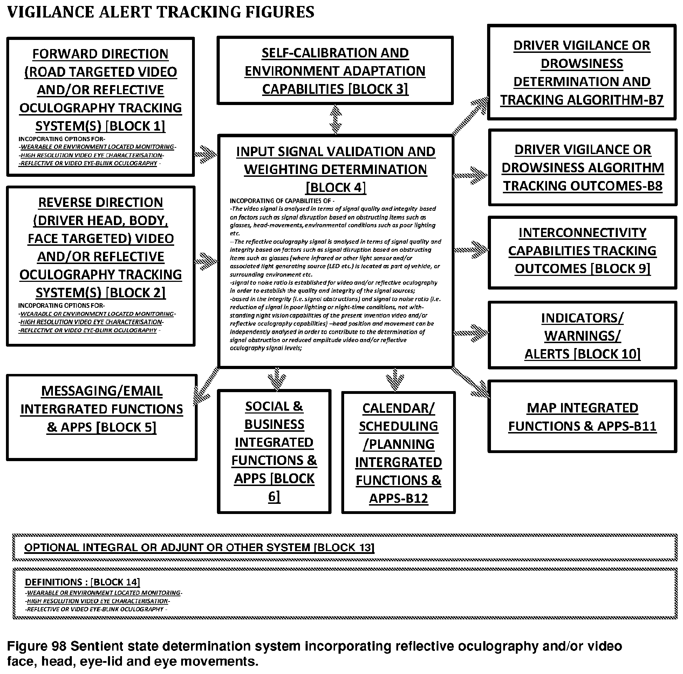

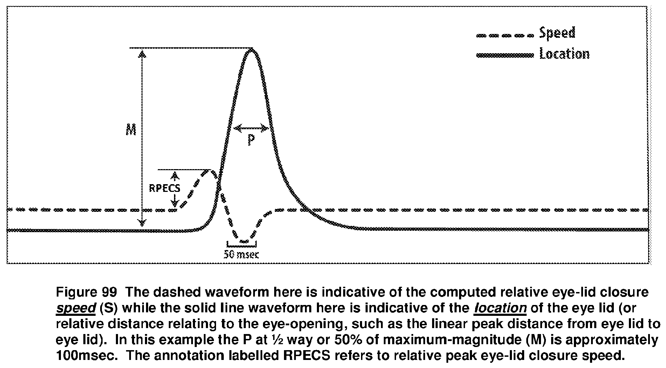

The present example may further comprise of the ***option for EEG monitoring whereby said EEG monitoring can be by way of sensors embedded in a subject wearable device*** and whereby associated automatic analysis can be deployed for the characterisation and determination of events of interest applicable to the incident or onset of driver drowsiness or sleep episodes, the present example further comprising any of wireless linked **EEG** sensor capable of being monitoring via vehicle-based monitoring system or via forehead glasses frame with attached **EEG** sensors and/or EG monitoring via tracking of infrared sensors capable of detecting changes in frontal lobe executive processing associated with an abatement of vigilance or attention (i.e. vehicle cabin mounted video or reflective oculography monitoring of a subject or driver face, eye, eye-lid, head or body movements or tracking of changes such as shown in ***Figure 39******)*** and/or determination of facial, body, eye and/or head features based on tracking changes related to a number of distinguishable points or locations of interest such as shown in example of ***Figure 40*** ***or*** ***Figure 41******;*** and/or tracking changes associated with road or driving environment as shown in ***Figure 42******;*** and/or incorporating a range of sensors within special driving glasses capable of incorporating any of **EEG** sensors for driver/pilot/subject forehead region, oculography sensors under eye region (i.e. marked as "transceiver units"), EOG or **EEG** or EMG or reflective oculography signals detected via frame of glasses, video eye gaze or movement changes via video mounted on glasses frames or via scanning reflection of eyes on glasses per example of glasses shown in ***Figure 43****; ;* and/or incorporation of vigilance driver tracking algorithm tracking capabilities presented in ***Figure 98*** ***[B7] AND [B8]*** and/or determination of eye-lid velocity measures as presented in ***Figure 99***)*.*
- whereby said ***wearable devices or associated monitoring system includes any of or any combination of ultrasonic and*/*or*** Doppler ultrasonic measures incorporated into a wearable device which can comprise of any of or any combination of vehicle cabin mounted video or reflective oculography monitoring of a subject or driver face, eye, eye-lid, head or body movements or tracking of changes such as shown in ***Figure 39******)*** and/or determination of facial, body, eye and/or head features based on tracking changes related to a number of distinguishable points or locations of interest such as shown in example of ***Figure 40*** ***or*** ***Figure 41****;* and/or tracking changes associated with road or driving environment as shown in ***Fiqure 42;*** and/or incorporating a range of sensors within special driving glasses capable of incorporating any of **EEG** sensors for driver/pilot/subject forehead region, oculography sensors under eye region (i.e. marked as "transceiver units"), EOG or **EEG** or EMG or reflective oculography signals detected via frame of glasses, video eye gaze or movement changes via video mounted on glasses frames or via scanning reflection of eyes on glasses per example of glasses shown in ***Figure 43******;*** and/or incorporation of vigilance driver tracking algorithm tracking capabilities presented in ***Figure 98*** ***[B7] AND [B8]*** and/or determination of eye-lid velocity measures as presented in ***Fiqure 99***).
   - ultrasonic and/or Doppler ultrasonic is incorporated into a ***wearable device*** can comprise any of or any combination of (i.e. as covered elsewhere in this document including but not limited to :; eLifeWATCH (Figure 5, [5]), eLifeWRIST (Figure 5, [8]); eLifeCHEST (Figure 5, [4]); eLifeEXG (Figure 23), a *wearable device (such* as *but not limited to Somfit headband* (Figure 25); *universal* - *bipolar sensor (Fiqure 23); oximeter (**Figure 22**); leaband (**Figure 24**); wristband (**Figure 2**), ankle-band (Fiqure 2), armband (Fiqure 2), earphone(s) (Fiqure* 14), chest-band *(**Figure 5**[3].[4];* *Figure 31**),* Figure 5, [2], Figure 43, Figure 46 to Figure 50, Figure 52, Figure 53, Figure 54, Figure 1 to Figure 31, Figure 37, Figure 55, Figure 45;
- whereby said ***wearable devices or associated monitoring system includes any of or any combination of*** MMT option; with APM option; with HDMC (Burton, 2014 eHealth patent appl.), the present example further comprising any of or any combination of ***Figure 39*** ***vehicle or environmental mounted monitoring system;*** determination of facial, body, eye and/or head features based on tracking changes related to a number of distinguishable points or locations of interest such as shown in example of ***Figure 40*** ***or*** ***Figure 41*** ***;*** and/or tracking changes associated with road or driving environment as shown in ***Fiqure 42;*** EOG or **EEG** or EMG or reflective oculography signals detected via frame of glasses, video eye gaze or movement changes via video mounted on glasses frames or via scanning reflection of eyes on glasses per example of glasses shown in ***Figure 43******;*** and/or incorporation of vigilance driver tracking algorithm tracking capabilities presented in ***Figure 98*** ***[B7]*** ***AND [B8]*** and/or determination of eye-lid velocity measures as presented in ***Figure 99***)*.*

- whereby said **EEG** monitoring or accompanying analysis can include characterisation or determination of ***spectral bands of interest* can** include (but are not limited to) **any combinations of spectral bands** including (but not limited to) any of or any combination of **delta/slow wave** (0.1-4 Hz), **theta** (4-8 Hz), **alpha** (8-12 Hz), **beta** (12-30 Hz), **low-gamma** (30-70 Hz), and/or **high-gamma** (70-180 Hz);
- whereby said ***spectral bands of interest* can** include (but are not limited to) **any combinations of spectral bands** including (but not limited to) **theta** (4-8 Hz), **alpha** (8-12 Hz);
- whereby the present example further incorporates the option of any of or any combination of above video measures combined with any of or any combination of **EEG** monitoring ***spectral bands of interest*** based on tracking changes related to the analysis of any one or combinations of ***spectral bands of interest;***
- whereby said combined **EEG** and video monitoring can be by way of a single device comprising of subject's glasses ***integrated with a head electrode attachment system*** (i.e. per Figure 3; Figure 4; Figure 25; Figure 46 to Figure 54, Figure 14);
- option of **EEG** monitoring spectral monitoring based on analysis of any one or combinations of ***spectral bands of interest;***
- whereby said **EEG** monitoring and/or binocular (or single-eye) infrared oculography and/or infrared-reflective-eye-lid monitoring and/or accompanying analysis can include characterisation or determination of (but are not limited to) along with the storage of behaviours of one or more subjects/drivers in order to distinguish movements from different drivers after the initial learning phase of any of or any combination of the said driver drowsiness algorithms (i.e. as outlined in this provisional patent application document);
- ***an example*** is to ***incorporate into glasses and*/*or EEG monitoring headset*** (with associated spectral analysis capability and/or combinational analysis incorporating **EEG** measures along with spectral outcomes and/or coherence measures and/or signal dynamics measures (i.e. non-linear dynamic, entropy and/or complexity analysis etc.) and/or brain symmetry analysis (i.e. hemisphere biased brain activation or coherence etc.) and/or combined with connectivity analysis (i.e. dipole determination and characterisation) and/or or the diver/subject's eye(s), incorporate the ***timing delay and the angle of the returned*/*received beam reflections (via LRD or other IR and*/*or or light sensitive sensor) to compute a range*** spectral power analysis **(**Figure 1 **to** Figure 5, Figure 14, Figure 21, Figure 25, Figure 27, Figure 43**)**;

### EYE OR FACIAL SUBJECT/DRIVER MONITORING AND CHARACTERISTICS APPLICABLE TO MONITORED PERIOD (I.E. PER ANY PRIOR NUMBER OF MONITORED BLINKS OR TIME-PERIOD) APPLICABLE TO VIDEO, INFRARED OCULOGRAPHY OR OTHER FACE AND/OR EYE AND/OR SUBJECT/DRIVER SENSED BEHAVIOUR OR PHYSIOLOGICAL PARAMETER MONITORING OR ANALYSIS)

- The present example may further include a transceiver unit which can firstly radiate a burst of infra-red beams (IR) transmitted in the direction ***of eye parameters including (but not limited to) eyelid closure speed, eyelid opening speed, span or linear measurement or angular factors related to eyelid opening or reopening movement,*** whereby such measures can be monitored via subject/driver worn device or vehicle attached or embedded device and/or whereby such measures can be monitored via one or more cameras attached or embedded as part of a subject/driver worn glasses and/or where by such measures can be monitored via one or more vehicle attached or embedded cameras and/or whereby such measures can be monitored via one or more IR transmission systems and associated receiver sensors (mounted on or part of vehicle or via subject/driver worn lasses or other devices;
- The present example may further comprise of a means of stereo vision-based monitoring (i.e. whereby such means can include monitoring video or infrared oculography via subject/driver worn devices such as glasses and/or vehicle attached or embedded systems which enable monitoring of both driver's eyes);

### EYE OPENING, CLOSURE AND BLINK CHARACTERISTICS APPLICABLE TO MONITORIED PERIOD (I.E. PER ANY PRIOR NUMBER OF MONITORED BLINKS OR TIME-PERIOD) APPLICABLE TO VIDEO, INFRARED OCULOGRAPHY OR OTHER FACE AND/OR EYE AND/OR SUBJECT/DRIVER SENSED BEHAVIOUR OR PHYSIOLOGICAL PARAMETER MONITORING OR ANALYSIS)

- measurement of ***blink closing period*** (BCP) during a pre-defined or a dynamically computed time segment (i.e. prior 10 to 100 sec);
- measurement of ***eye closure time*** (ECT) during a pre-defined or a dynamically computed time segment (i.e. prior 10 to 100 sec);
- measurement of ***eye opening time*** (EOT) during a during a pre-defined or a dynamically computed time segment (i.e. prior 10 to 100 sec);
- measurement of ***eye opening span*** (EOS; based on linear measurement (i.e. mm) or angular eye-opening maximum, during a pre-defined or a dynamically computed time segment (i.e. prior 10 to 100 sec);

### EYELID CLOSURE FREQUENCY AND DURATION CHARACTERISTICS APPLICABLE TO MONITORIED PERIOD (I.E. PER ANY PRIOR NUMBER OF MONITORED BLINKS OR TIME-PERIOD) APPLICABLE TO VIDEO, INFRARED OCULOGRAPHY OR OTHER FACE AND/OR EYE AND/OR SUBJECT/DRIVER SENSED BEHAVIOUR OR PHYSIOLOGICAL PARAMETER MONITORING OR ANALYSIS)

- measurement of ***eye-lid peak blink frequency*** during a pre-defined or a dynamically computed time segment (i.e. prior 10 to 100 sec) (EPBF);
- measurement of ***eye-lid minimum blink frequency*** during a pre-defined or a dynamically computed time segment (i.e. prior 10 to 100 sec) (EMBF);
- measurement of ***eye-lid average blink frequency*** during a pre-defined or a dynamically computed time segment (i.e. prior 10 to 100 sec) (EABF);
- determination of ***ratio*** of measurement of EOS / and any of or any combination of EPBF, EMBF, EABF;
- measurement of ***eye-lid blink frequency variance*** during a pre-defined or a dynamically computed time segment (i.e. prior 10 to 100 sec) (EBFV);
- measurement of ***eye-lid peak blink duration*** during a pre-defined or a dynamically computed time segment (i.e. prior 10 to 100 sec) (EPBD);
- measurement of ***eye-lid minimum blink duration*** during a pre-defined or a dynamically computed time segment (i.e. prior 10 to 100 sec) (EMBD)
- measurement of ***eye-lid average blink duration*** during a pre-defined or a dynamically computed time segment (i.e. prior 10 to 100 sec) (EABD);
- determination of ***ratio*** of measurement of EOS / and any of or any combination of EPBD, EMBD, EABD;
- measurement of ***eye-lid blink duration variance*** during a pre-defined or a dynamically computed time segment (i.e. prior 10 to 100 sec) (EBDV);

### EYELID SPAN CHARACTERISTICS APPLICABLE TO MONITORED PERIOD (I.E. PER ANY PRIOR NUMBER OF MONITORED BLINKS OR TIME-PERIOD) APPLICABLE TO VIDEO, INFRARED OCULOGRAPHY OR OTHER FACE AND/OR EYE AND/OR SUBJECT/DRIVER SENSED BEHAVIOUR OR PHYSIOLOGICAL PARAMETER MONITORING OR ANALYSIS)

_-measurement linear distance (mm) of eye-lid opening span (travel) (DEOS);
- measurement degrees of angular change corresponding to eye-lid opening span (travel) (AEOS);

### EYELID CLOSURE CHARACTERISTICS APPLICABLE TO MONITORIED PERIOD (I.E. PER ANY PRIOR NUMBER OF MONITORED BLINKS OR TIME-PERIOD) APPLICABLE TO VIDEO, INFRARED OCULOGRAPHY OR OTHER FACE AND/OR EYE AND/OR SUBJECT/DRIVER SENSED BEHAVIOUR OR PHYSIOLOGICAL PARAMETER MONITORING OR ANALYSIS)

- measurement (i.e. millimetres of linear distance per second or degrees of angular variation per second) of eye-lid peak **closure velocity** (EPCV);
- measurement of eye-lid minimum closure velocity (EMCV);
- measurement of eye-lid average closure velocity (EACV);
- determination of ***ratio*** of measurement of EOS / and any of or any combination of EPCV, EMCV, and/or EACV;
- measurement of eye-lid peak **closure velocity variance** (EPCVV);
- measurement of eye-lid minimum closure velocity variance (EMCVV);
- measurement of eye-lid average closure velocity variance (EACVV);
- determination of ***ratio*** of measurement of EOS / and any of or any combination of EPCVV, EMCVV, EACVV;
- measurement of eye-lid peak **closure acceleration** (EPCA);
- measurement of eye-lid minimum closure acceleration (EMCA);
- measurement of eye-lid average closure acceleration (EACA);
- determination of ***ratio*** of measurement of EOS / and any of or any combination of EPCA, EMCA, EACA;
- measurement of eye-lid peak **closure acceleration variance** (EPCAV);
- measurement of eye-lid minimum closure acceleration variance (EMCAV);
- measurement of eye-lid average closure acceleration variance (EACAV);
- determination of ***ratio*** of measurement of EOS / and any of or any combination of EPCAV, EMCAV, EACAV;

### EYELID OPENING CHARACTERISTICS APPLICABLE TO MONITORED PERIOD (I.E. PER ANY PRIOR NUMBER OF MONITORED BLINKS OR TIME-PERIOD) APPLICABLE TO VIDEO, INFRARED OCULOGRAPHY OR OTHER FACE AND/OR EYE AND/OR SUBJECT/DRIVER SENSED BEHAVIOUR OR PHYSIOLOGICAL PARAMETER MONITORING OR ANALYSIS)

- measurement (i.e. millimetres of linear distance per second or degrees of angular variation per second) of eye-lid peak **opening velocity** (EPOV);
- measurement of eye-lid minimum opening velocity (EMOV);
- measurement of eye-lid average opening velocity (EAOV);
- determination of ***ratio*** of measurement of EOS / and any of or any combination of EPOV, EMOV, EAOV;
- measurement of eye-lid peak **opening velocity variance** (EPOVV);
- measurement of eye-lid minimum opening velocity variance (EMOVV);
- measurement of eye-lid average opening velocity variance (EAOVV);
- determination of ***ratio*** of measurement of EOS / and any of or any combination of EPOVV, EMOVV, EAOVV;
- measurement of eye-lid peak **opening acceleration** (EPOA);
- measurement of eye-lid minimum opening acceleration (EMOA);
- measurement of eye-lid average opening acceleration (EAOA);
- determination of ***ratio*** of measurement of EOS / and any of or any combination of EPOA, EMOA, EAOA;
- measurement of eye-lid peak **opening acceleration variance** (EPOAV);
- measurement of eye-lid minimum opening acceleration variance (EMOAV);
- measurement of eye-lid average opening acceleration variance (EAOAV);
- determination of ***ratio*** of measurement of EOS / and any of or any combination of EPOAV, EMOAV, EAOAV;

### EYELID MEAN AND STANDARD DEVIATION CHARACTERISTICS APPLICABLE TO MONITORED PERIOD (I.E. PER ANY PRIOR NUMBER OF MONITORED BLINKS OR TIME-PERIOD) APPLICABLE TO VIDEO, INFRARED OCULOGRAPHY OR OTHER FACE AND/OR EYE AND/OR SUBJECT/DRIVER SENSED BEHAVIOUR OR PHYSIOLOGICAL PARAMETER MONITORING OR ANALYSIS)

- ***The present*** example may comprise of utilising a wearable device such as glasses with video monitoring capability to enable measurement of any of or any combination of the above listed video parameters;

### INFRARED TRACKING CHARACTERISTICS APPLICABLE TO MONITORIED PERIOD (I.E. PER ANY PRIOR NUMBER OF MONITORED BLINKS OR TIME-PERIOD) APPLICABLE TO VIDEO, INFRARED OCULOGRAPHY OR OTHER FACE AND/OR EYE AND/OR SUBJECT/DRIVER SENSED BEHAVIOUR OR PHYSIOLOGICAL PARAMETER MONITORING OR ANALYSIS)

- ***the present*** example comprises of utilising a wearable device such as ***glasses or vehicle mounted systems with infrared light emitting diode*** and light dependent resistor (LDR) as receiver for one or both of subjects eyes to enable measurement of any of or any combination of the ***above listed parameters*** (i.e. substitute infrared measures instead of video measures);
- whereby system can further monitor and track via ***infrared** any **of or** any **combination of the pupil centre*** to determine the ***eye's gaze, other eye related data such* as *pupil diameter, blinking and*/*or eye fixation status*** of subject/driver.

### OTHER FACIAL OR VIDEO MEASURES

The present example incorporates a vehicle mounted and/or subject wearable diagnostic or prognostic monitoring system(s) incorporating the capability of automatic analysis applicable to the determination of the onset or incidence of *"health conditions*/*disorders or events of interest"* such as (but not limited to a subject's drowsiness or reduction of vigilance), the example further comprising of vision monitoring and/or associated analysis comprising =of the characterisation of a subject's video and related determination any of or any combination of (i.e. ***Figure 39******;*** ***Figure 40******;*** ***Figure 41******;*** ***Figure 42******;*** ***Figure 43******;*** ***Figure 98******;Fiqure 99***).:
- Subject/driver's Blink status (true or false);
- Subject/driver's ***blink frequency*** (rate of blinking per second)
- Subject/driver's ***average blink duration*** (blink length);
- Subject/driver's **head position** (in the x, y, z axis)
- Head rotation: head ***angle vs starting position*** of head:
- Subject/driver's angular change of x-axis head position;
- Subject/driver's angular change of y-axis head position;
- Subject/driver's angular change of x-axis eye(s) position;
- Subject/driver's angular change of y-axis eye(s) position;
- Subject/driver's angular change of z-axis head position;
- Subject/driver's angular change of z-axis eye(s) position;
- Subject/driver's ***individual eye tracking*** (deviation of each eye from each respective centre position)
- Subject/driver's eyes and/or head ***gaze tracking*** (determines the part of the screen the driver is looking at);
- two cameras to provide ***stereo image of the subject's face and track head and face movements;***
- ***One or more cameras*** (help track each eye independently) to characterise subject video and determine any of or any combination of the ***eye(s) pupil size, measure the vergence*** (distance between eyes), and/or produce true blink analysis (i.e., ***detect eyelid position vs bright pupil occlusion***);
- whereby said video camera(s) can effectively operate under circumstances including any of or any combination of different subject/driver's environments ranging from bright sunlight (***using automatically switching day-light and night infrared modes***) to night time, from near to far positioning (i.e. use of its ***ultra-wide field of view mode***)*,* ***track subject's movement even when the eyes are obstructed by using other facial markers such* as *head pose, nose and mouth***;
- ***means (i.e. driver*/*subject specific recording and calibration during wake*/*drowsy*/*normal*/*states and*/*or means of associating different calibrations to different drivers automatically and*/*or manually) of calibrating system to compensate*** for circumstances in terms of ***eye-lid and*/*or eye function or behaviour*** such as (but not limited to) with older drivers who can have droopy eyelids, where it can be difficult to acquire a clear picture of the eye;
- whereby any ***combination*** of detailed (per this document) ***eye-lid, eye measures, vision sensed and determined driver*/*subject body positioning, hybrid system*** comprising of a plurality of ***pressure sensors per CMI*/*Burton (2012)*** or ***driver-specific automatic calibration capability using comparative reference data and vigilance threshold determination per CMI*/*Burton (2003),*** enables more precise (than any single approach) predictions and determination of driver drowsiness states or onset;
- whereby forward glasses mounted video camera(s) can location and characterise behaviour associated with where subject/driver is looking or where head is moving;

- ***whereby any combination of monitoring and measures including any of or any combination of pupil size, pupil response,*** eye-ball related ocular parameters such as a combination of eye gaze measures, dwell time, determination of brief reflexive eye blinks (i.e. <200-250msec) from those which are prolonged purposeful or abnormal eye blinks (i.e. >250 msec), discrimination between normal or drowsy reflexive as well as pathological eye-blinks or lid closures, identifying ocular signs of drowsiness such as the percentage of time one's eyes are opened or closed, seizures, or prolonged eye lid closure due to loss of consciousness or falling asleep.

### OTHER VEHICLE OR SUBJECT MONITORIED VARIABLES OR CAPABILITIES OR ASSOCIATED CHARACTERISTICS APPLICABLE TO MONITORIED PERIOD (I.E. PER ANY PRIOR NUMBER OF MONITORED BLINKS OR TIME-PERIOD) APPLICABLE TO VIDEO, INFRARED OCULOGRAPHY OR OTHER FACE AND/OR EYE AND/OR SUBJECT/DRIVER SENSED BEHAVIOUR OR PHYSIOLOGICAL PARAMETER MONITORING OR ANALYSIS)

**The present examples incorporate a vehicle mounted and/or subject wearable diagnostic or prognostic monitoring system(s) incorporating the capability of automatic analysis applicable to the determination of the onset or incidence of *"health conditions*/*disorders or events of interest"* such as (but not limited) to a subject's drowsiness or reduction of vigilance, the example further comprising any of or any combination of:**
- Incorporation of visual and/or sensor (i.e. reflective oculography, **EEG,** EOG, EMG) monitoring capability and/or a microprocessor with an ***algorithm for the characterisation*** of ***lane weaving*** based on vehicle inputs from driver (speed, steering, brakes, accelerator, indictors etc.) and other vehicle control outputs, whereby output measures of said algorithms can form part of decision matrix applicable to the determination of ***driver drowsiness;***
- Incorporation visual and/or sensor (i.e. reflective oculography, **EEG,** EOG, EMG) monitoring capability and/or a microprocessor with an ***algorithm for the characterisation*** of a subject/driver's ***eyelid and eye location behaviour*** based on video or infrared light emitting diode(s) ***(LED)*** transmission and corresponding light dependent resistance ***(LDR)*** reception of reflected signal;
- Incorporation of monitoring capability and/or a microprocessor with an ***algorithm for the characterisation*** of ***steering parameters*** based on vehicle inputs from driver (speed, steering, brakes, accelerator, indictors etc.) and other vehicle control outputs, whereby output measures of said algorithms can form part of decision matrix applicable to the determination of ***driver drowsiness or vigilance determination;***
- Incorporation of visual and/or sensor (i.e. reflective oculography, **EEG,** EOG, EMG) monitoring capability and/or a microprocessor with an ***algorithm for the characterisation*** of any of or any combination of ***driver steering behaviour, directional consistency; steering wheel movements, steering speed, steering wheel movement velocity, steering wheel acceleration, steering where change in position (i.e. linear measurement or angular displacement), driver activity*** based on vehicle inputs from driver (speed, steering, brakes, accelerator, indictors etc.) and/or other vehicle control outputs, whereby output measures of said algorithms can form part of decision matrix applicable to the determination of driver drowsiness or vigilance determination, whereby said characterisation parameters can be determined in ***different vehicle speed zones*** (i.e. zero to 40 Km/h; 40-60 km/h; 60-80 Km/h; 80-100 Km/h; 100-120 Km/h; 120-140 Km/h; 140-160 Km/h; 160-180 Km/h; 180-2000 Km/h; 200-220 Km/h; > 220 Km/h etc.), whereby steering ***patterns and parameters can be determined at the start of every trip*** within a predefined (i.e. 1 to 20 minutes) or dynamically determined period ***in order to establish a base-line level*** and then the ***current driving behaviour and associated monitored*** or vehicle parameters including steering and/or other parameters can then be ***continuously compared to this baseline level*** in order to enable the ***activation of countermeasures*** (i.e. steering wheel torque, steering wheel shaking or vibration; audible or visual alerts or alarms, winding down of windows, and/or reduction of vehicle cabin temperature etc.) of predicted danger such as resulting from the determination of subject/driver drowsiness onset;
- Incorporation of visual and/or sensor (i.e. reflective oculography, **EEG,** EOG, EMG) monitoring capability and/or a microprocessor with an ***algorithm for the characterisation*** of any of or any combination of ***driver steering behaviour, directional consistency; driver activity, steering parameters, steering deviation*** or other related information based on the deployment of ***one or more sensors attached or applied to steering*** wheel or steering system and/or related to outputs from existing vehicle steering measures, whereby said sensors or measures can be based on vehicle inputs from driver (speed, steering, brakes, accelerator, indictors etc.) whereby the said algorithms can form part of decision matrix applicable to the determination of ***driver drowsiness or vigilance determination;***
- Incorporation of visual and/or sensor (i.e. reflective oculography, **EEG,** EOG, EMG) monitoring capability and/or a microprocessor with an ***algorithm for the characterisation*** of any of or any combination of vehicle sensors on the steering wheel to ***monitor absence of corrective movements of the steering wheel,*** whereby the premise is to track the trend for corrective adjustments during normal driving, and where there is no such corrections during a pre-defined or dynamically computed **(i.e. said threshold period can be preconfigured or dynamically determined by characterising time in journey, driver's behavioural or motion characteristics etc.) threshold period** (i.e. after 5 sec of ***inactivity an alarm*** or other alert or notification or message can be activated) or other related information relayed based on the deployment of ***one or more sensors attached or applied to steering*** wheel or steering system and/or related to outputs from existing vehicle steering measures, whereby said sensors or measures can be based on vehicle inputs from driver (speed, steering, brakes, accelerator, indicators etc.) whereby the said algorithms can form part of decision matrix applicable to the determination of ***driver drowsiness or vigilance determination;***
- Incorporation of visual and/or sensor (i.e. reflective oculography, **EEG,** EOG, EMG) monitoring capability and/or a microprocessor with an ***algorithm for the determination*** of a ***driver alert or notification system and*/*or other message or alert or notification to driver*** or other **remote location** whereby the said algorithm output can form part of decision matrix applicable to the determination of driver drowsiness warnings (such as notification for driver to take break or rest; alert to control or remote centre where applicable)
- Incorporation of visual and/or sensor (i.e. reflective oculography, **EEG,** EOG, EMG) monitoring capability and/or a microprocessor with an ***algorithm for the determination*** of a ***driver alert, notification or feedback system and*/*or other message or alert or notification to driver*** or other **remote location** whereby the said algorithm output can form part of decision matrix applicable to the determination of driver drowsiness determination warnings such as (but not limited to) *lane **keeping torque applied to wheel*** and/or ***wheel shaker warning system;***
- Incorporation of visual and/or sensor (i.e. reflective oculography, **EEG,** EOG, EMG) monitoring capability and/or a microprocessor with ***an algorithm for the characterisation*** and related **camera enabling forward facing lane-tracking and other road or environmental tracking capabilities, whereby said camera(s) can be mounted behind rear-view mirror (or other suitable location),** whereby output measures of said algorithms can form part of decision matrix applicable to the ***determination of driver drowsiness or vigilance determination;***
   --Incorporation of visual and/or sensor (i.e. reflective oculography, **EEG,** EOG, EMG) monitoring capability and/or a microprocessor with ***an algorithm for the characterisation*** and related **camera monitoring enabling forward facing lane-tracking and other road or environmental tracking capabilities, whereby said camera(s) can be mounted behind rear-view mirror (or other suitable location),** whereby output measures of said algorithms can form part of decision matrix applicable to the ***determination of driver drowsiness or vigilance determination, further capabilities incorporating a means (i.e. video recognition processing) so that in cases of any of or any combination of detection of lane drifting, erratic driving or vehicle behaviour, and*/*or in circumstances when*** the ***lateral distance between the vehicle and the lane markings changes,*** the system can generate an audible signal or alarm or via stereo car speakers, or via seat, wheel or other vibrations feedback, in order to alert the driver to correct the course of the vehicle before more severe problems arise;
- Incorporation of visual and/or sensor (i.e. reflective oculography, **EEG,** EOG, EMG) monitoring capability and/or a microprocessor with ***an algorithm for the characterisation*** and related **camera and/or IR oculography monitoring enabling tracking and/or monitoring and/or analysis/characterisation during a** range of environments from bright sunlight to night time and/or from near to far positioning by way of tracking subject/driver's movement and also when the subject/driver's eyes are obstructed (i.e. video glasses, head movement etc.) by way of subject/driver head and/or facial and/or other body fiducial marker(s) such as (but not limited to) head-pose, nose, eyes, mouth etc.
- Incorporation of visual and/or sensor (i.e. reflective oculography, **EEG,** EOG, EMG) monitoring capability and/or a microprocessor with ***an algorithm for the characterisation*** and related **camera and/or IR oculography monitoring enabling tracking and/or monitoring and/or analysis/determination** of any of or combination of the subject/driver's pupil centre(s), pupil diameter(s), eye's gaze, blinking, tracking the pupil(s) centre to determine the eye's gaze, blinking and/or eye fixation , detection of microsleeps by determination and identifying head nodding characteristics, eye fixation and/or the cornea-reflex centres of the subject/driver's eye(s);
- Incorporation of visual and/or sensor (i.e. reflective oculography, **EEG,** EOG, EMG) monitoring capability and/or a microprocessor with ***an algorithm for the characterisation*** and related **camera and/or IR oculography monitoring enabling** compensation for one or both driver/subject's eye during special circumstances such as "lazy" eye function or other dual or single eye dysfunction or obstruction;
- Incorporation of visual and/or sensor (i.e. reflective oculography, **EEG,** EOG, EMG) monitoring capability and/or a microprocessor with ***an algorithm for the characterisation*** and related camera and/or IR oculography monitoring **enabling the determination of** the duration of **fixation of the eye** on specific objects on the road signs;
- Incorporation of visual and/or sensor (i.e. reflective oculography, **EEG,** EOG, EMG) monitoring capability and/or a microprocessor with ***an algorithm for the characterisation*** and related camera and/or IR oculography monitoring **enabling the determination of** detects **eyelid position vs bright pupil occlusion;**
- Incorporation of visual and/or sensor (i.e. reflective oculography, **EEG,** EOG, EMG) monitoring capability and/or processing capability enabling ***an algorithm for the characterisation*** and related camera and/or IR oculography monitoring **enabling the determination of subject/driver drowsiness even during head movements and/or visual or beam obstructions (such as glasses) by automatically switching between any or combination if IR oculography, video, head fiducial markers, body fiducial markers, facial fiducial markers or device worn fiducial markers and based on most effective (tracked or monitoring integrity of sensor or day) at any point in time applicable to drowsiness parameter monitoring and associated determination;**
- Incorporation of visual and/or sensor (i.e. reflective oculography, **EEG,** EOG, EMG) monitoring sensor capability and/or a microprocessor with a wearable device (such as glasses) or a vehicle mounted device such as near subject/drivers face, incorporating ***IR oculography*** comprising of high frame rate (IR transmission rate) ***infrared radiation at a frequency at least two but preferably more than ten times or more*** times the ***fastest possible blink rate (for one or two eyes) of*** a subject/driver's blink rate during the fastest blink rate circumstances, in order to sense the reflected IR pulse from the surface of the driver/subjects eye surface as a means of computing any of or any combination of the precise ***blink, gaze, location, and*/*or pupil characteristics associated to eye, head and*/*or facial characteristics;***
- Incorporation of visual and/or sensor (i.e. reflective oculography, **EEG,** EOG, EMG) monitoring device (i.e. driver cabin or wearable glasses) incorporating ***IR video monitoring*** comprising of ***high frequency video frame rate at a frequency at least two but preferably more than ten times*** or more times the fastest possible blink rate ***(for one or two eyes)*** of a subject/driver's blink rate during the fastest blink rate circumstances, in order to sense the reflected IR pulse from the surface of the driver/subjects eye surface as a means of computing any of or any combination of the precise ***blink, gaze, location, and*/*or pupil characteristics associated to eye, head and*/*or facial characteristics;***

### HYBRID VEHICLE OR SUBJECT MONITORIED VARIABLES OR CAPABILITIES OR ASSOCAITED CHARACTERISTICS APPLICABLE TO MONITORIED PERIOD (I.E. PER ANY PRIOR NUMBER OF MONITORED BLINKS OR TIME-PERIOD) APPLICABLE TO VIDEO, INFRARED OCULOGRAPHY OR OTHER FACE AND/OR EYE AND/OR SUBJECT/DRIVER SENSED BEHAVIOUR OR PHYSIOLOGICAL PARAMETER MONITORING OR ANALYSIS)

- Incorporation of a device with ***IR oculography*** monitoring comprising of high frame rate (IR transmission rate) ***infrared radiation at a frequency at least two but preferably more than ten times or more*** than the ***fastest possible blink rate (for one or two eyes) with an accompanying first algorithm and*/*or combined with***
   ***IR video monitoring*** comprising of ***high frequency video frame rate at a frequency at least two but preferably more than ten times*** or more times the fastest possible blink rate ***(for one or two eyes) with an accompanying second algorithm***
   of a subject/driver's blink rate during the fastest blink rate circumstances,
   in order to ***sense the reflected IR oculography signal*** and/or video associated with the driver/subjects eyes and/or eyelids as a means of computing any of or any combination of the precise ***blink, gaze, location, and*/*or pupil characteristics associated* to *eye, head and*/*or facial characteristics;***
- whereby an ***accompanying third algorithm processes the quality of the video and*/*or IR monitored*** signals (or video) or ***associated obstructions*** (of IR oculography signals or video) in order to weight or switch the emphasis of the driver/subject drowsiness determination based on the most optimal (reliable or confidence level or signals) and/or based on tracking head and/or gaze driver/subject characteristics;
- whereby the ***combination of any of IR oculography (attached to vehicle and*/*or wearable device such as glasses, said high frame rate IR (nigh-time or supressed light conditions) video monitoring,*** with said accompanying algorithms enables a reliable hybrid monitoring prognosis and diagnosis or driver vigilance or drowsiness;
- whereby the present example further comprises an option for said ***hybrid monitoring to incorporate*** a plurality of ***hand pressure sensors*** for continuously measuring hand pressure of the driver and sensor for detecting eye movement or driver head position, ***along with the driver's response to the prompting device***
- Incorporation of a device with ***IR oculography*** monitoring comprising of high frame rate (IR transmission rate) capable of accurately resolving velocity of any of eye-lid blinks, amplitude of reflected IR signal and also a means (***whereby means includes the ability to scan the distance between the upper and lower eye-lid or one or both eyes in order to more precisely measure changes with onset of drowsiness or changes in vigilance levels of driver*/*subject. Such scanning can include an array of IR-LEDs which can be switched in sequence and then have eye-reflected IR measured via one or more light dependent resistors in a manner whereby the IR-LEDS each scan* a *successive portion in an arc shape across the driver*/*subject's eye, thus enabling spatial versus conventional physiological measures of IR reflective oculography***) of continuously determining vertical or spatial characteristics or distance or angular displacement between the upper and lower eye-lid of a driver/subject as a marker of driver drowsiness or vigilance; In this manner prior measures of infra-red reflective oculography ratio of amplitude and velocity can be improved by way of the superior accuracy in terms of linear versus prior art surrogate amplitude eye-opening measures.
- the present example incorporates **measurement means** (i.e. ***Figure 39******;*** ***Figure 40******;*** ***Figure 41******;*** ***Figure 42******;*** ***Figure 43******;*** ***Figure 98******;Fiqure 99***) to objectively recognise characterise and measure drowsiness and/or sleep onset and/or vigilance and/or sleep urge (propensity) by way of incorporating at least one reflective oculography or video processing systems.

The present example can sense environment lighting and adjusting for additional weighting of night- vision monitoring (i.e. infrared video or infrared oculography versus daylight vision sensors or video drowsiness detection/onset algorithm inputs (i.e. ***Figure 39******;*** ***Figure 40******;*** ***Figure 41******;*** ***Figure 42******;*** ***Figure 43******;*** ***Figure 98******;Fiqure 99).***

The present example can enable an objective and **qualitative measure to objectively recognise characterise and measure drowsiness** and/or sleep onset and/or vigilance and/or sleep urge (propensity) by way of examining progressive change over time of the degree or magnitude or linear **distant separation between any points on the upper and lower eye-lid** or associated aspects (moving parts or muscle activity or related skin distortion or changes) compared to a measure related to eye-lid opening or eye-lid closing;

The present example provides **"sentient state" tracking** where the determination of said state comprises of any of or combination of reflective oculography and/or video measures, the present example further comprising any of or any combination of (i.e. ***Figure 39******; Fiqure 40;*** ***Figure 41******;*** ***Figure 42******;*** ***Figure 43******;*** ***Figure 98******;Fiqure 99***) ):
- **video "derived and associated measures and or tracking of same"** (i.e. based on day-light and/or night vision with automatically adjustable monitoring conditions such as but not limited infrared lighting and video monitoring combined with day-light monitoring) vision processing capable of determination and/or recognition of "facial characteristics" applicable to sentient state (cognition level or state and/or transition and/or vigilance state) of a subject/individual (such as but not limited to driver, occupational, sleep/wake or homeostatic sleep factors and/or **circadian** clock factors of a subject/individual);
- reflective **oculography "derived and associated measures and or tracking of same"** (i.e. based on day-light and/or night vision with automatically adjustable monitoring conditions such as but not limited infrared lighting and reflective oculography (measurement of steady state or pulsed light reflected from surface of subject/individual's eye-ball as an indicator of time of eye opening, velocity and/or acceleration factors (i.e. per figure showing wave-shape of reflected oculography signal - similar signal derivations in terms of deriving a signal indicative of eye-lid opening magnitude versus time of eye-opening can be derived via video measures);
- **the said "combination and or switching selection"** can be based on computing a measure of the oculography and/or video "signal quality determination" and/or " **oculography signal or video integrity determination"** associated with the weighting factor relevant to the combining process for one more or "sentient state" (or associated transitions) signals such as (but not limited to) oculography and/or video signals monitored and tracked from a subject/individual (i.e. driver or other occupation where drowsiness onset can be of issue or risk);
- **the said "quality determination" of signal can refer to accuracy and reliability of video and/or versus oculography signal(s).** i.e. signal to noise can impacted by environmental lightning conditions where video or reflective oculography signals, applicable to the determination of eye-lid motion and opening or closing velocity, can be degraded by poor lighting conditions where background noise competes significantly with signals of measurement relevance;
   (i.e. ***Figure 39******;*** ***Figure 40******; Fiqure 41; Fiqure 42;*** ***Figure 43******;*** ***Figure 98******;******Figure 99***) In the case of reflective oculography this resolution can be based on the minimal discernible measure of change in eye-opening or closure which can in turn be dependent on the number of, the distance of, the location and respective angle of reflected light sensor(s) as well as light source(s) (i.e. steady state and/or pulsed). In the case of video monitoring, the resolution determination can, for example, be based on the minimal discernible measure of pixels of video applicable to the measure of eye-opening. For example, 0 to 10 pixels change corresponding to eye-opening can result in a relatively poor accuracy as the eye-opening distance must change by 10% or more before a valid measure of change can be detected. Therefore this can adversely impact upon the resolution and consequential sensitivity of eye-opening or eye-lid closure changes. which can be tracked as a measure or sentient-state. The present example, to counter such limitations provides optional mechanical pan and zoom lens and camera targeting capabilities, as well as options for multiple cameras so that one camera can communicate the location of eyes to a tighter focus camera which focusses with higher resolution primarily on the eye and eye-lid regions. In this way driver head and face characteristics can be tracked whilst still enabling high resolution and accurate eye-lid velocity and linear or magnitude of eye-opening measures;
- whereby the said "oculography signal or video integrity determination" can be based on the disruption of the respective signals or video - i.e. due to glasses or other wearable face or head devices, or due to head movements etc. Additionally, the signal integrity can be influenced by misalignment of poor between subject/individuals eyes or eye-lid, or head or body or face and camera focus or lighting focus or infrared or other reflective oculography light transmitter or associated receiver;
- whereby said "sentient state" includes any of or any combination of (but not limited to) cognition level, awareness, alertness, attention-level, vigilance state, occupational, sleep/wake state, homeostatic sleep factors, and/or **circadian** clock factors of a subject/individual (such as but not limited to driver, occupational, sleep/wake or homeostatic sleep factors and/or **circadian** clock factors of a subject/individual);
- whereby **"derived and associated measures and or tracking of same"** include **running average measures** or trending variations applicable to drowsy diagnosis or prognosis of a subject/individual (i.e. decrease of eye-lid closure and/or opening measures (such as degree of linear magnitude measures, eye-lid movement opening or closure velocity or acceleration can a contributing factor indicative of drowsiness onset);
- whereby **"facial characteristics"** include tracking of any of or any **"combination and or switching selection"** of (but not limited to) eye-movements, eyelid opening time, eye-lid-closure time, eye-lid opening velocity, eye-lid closure velocity (whereby velocity can be computed by deriving linear or degree of eye-opening or closure and/or surrogate measures of eye opening as a qualitative measure of reflected oculography signal by way of measuring the infrared or daylight or blue-light or other light bandwidth signal reflected from one or both of subject/user's eye surface) ;

The present example converts any of or any combination of a **video image of a subject's eyes, eye-lid, face, head or other body extremities, from IR oculography and/or video monitoring, into a syntactic representation** based on one or more tracked points such as visual video derived fiducial markers representative subject movement region of interest (i.e. eye lid, eye-focus, etc.).

In this manner a data file or **representation of motion** (i.e. eye-lid velocity or acceleration) can be derived in a non-obtrusive and non-contact or video format. An example of video measures indicative of driver drowsiness can comprise of the **characterisation of eye-lid movements (blinks) based on the diagnosis or prognosis of drowsiness levels** (i.e. indicative of tonic suppression with drowsiness supressing responsiveness or velocity of eye-lid blink rate) or drowsiness-risk based on ***Vision Index Blink-speed* / *span (VISE),*** whereby a ratio of the displacement of eye-lid span (i.e. linear measures or angular opening of eyes) versus the speed (velocity) of eyelid motions can be tracked.

Moreover the video monitoring aspect can be **integrated into a wearable device such as glasses or alternatively incorporated in a less obtrusive context such as the attachment or incorporation of one or more cameras as part of the subject's environment** (i.e. occupational location, any transportation vehicle interior, traffic control environment, aircraft cockpit, ship control room or other).

Additionally, the said video monitoring systems can be incorporated as part of an **EEG** monitoring system (i.e. embedded as part of video glasses or other wearable **EEG** monitoring device) and/or driver movement detections system; ^{19, 21, 22}

Additionally, the present example further incorporates a **threshold level as a marker of excessive drowsiness,** whereby said threshold marker is indicative of an individual subject (i.e. via calibrated and personalised data means) or determined from any of or any combination of normative data. In this way the **VISE measure can be configured to generate an alert or notification** to the subject or other party when a pre-defined or dynamically computed threshold level is exceeded, or when the safe operational region transitions to a region of unacceptable drowsiness risk level.

### Description of Figures

***Figure 39*** Vehicle or wearable device (such as glasses) mounted driver eye, face and head or body movement via video imaging and/or IR reflective oculography and associated analysis. >>> the present example incorporates vehicle mounted or driver wearable device (i.e. glasses) video and/or IR reflective oculography monitoring capabilities. Vehicle interfaced camera and IR reflective monitoring system showing the combined binocular IR with automatic day-night IR video lighting as well as vehicle mounted IR LED /LDR scanning (eye lid displacement determination using automatic pan-zoom driver/subject eye-tracking system (i.e. concealed in head-lining of vehicle) in order to enable superior hybrid IR reflective oculography and IR video tracking systems for minimal face, eye and head-tracking and maximal signals during wide-ranging light conditions.
***Figure 40*** The present example can be configured to recognise eye and facial characteristics as a means of tracking changes applicable to markers of sentient state or associated changes.
***Figure 41*** the present example incorporates video imaging and associated facial feature recognition and tracking capabilities. >> the present example enables monitoring and processing of driver/subject video in order to continuously determine head movements, drooping, rotation and other motion recognition and determination, along with associated facial feature recognition and associated motion changes, eye, gaze and other characteristics in order to detect changes applicable to driver drowsiness. Additionally, factors such as slowing eye-lid movements, extended driver/subject gaze or dwell and a decrease in normal vigilant behaviour can be continuously tracked as markers of driver drowsiness onset or changes in driver/subject attention or vigilance.
***Figure 42*** The present example includes vehicle and/or driver mounted rear and/or front-projection video imaging and associated lane recognition and other visual analysis. >> The present example includes the incorporation of forward video as a measure of lane deviation or other steering properties that can be indicative of driver drowsiness or abatement of driver/subject vigilance.
***Figure 43*** Driver/subject wearable glasses incorporating any of or any combination of binocular or monocular IR reflective oculography and/or video imaging (with IR capability). >>>Driver/subject wearable glasses incorporating IR reflective oculography with option of the capability to enable new IR scanning whereby eye-opening can be more accurately determined via angular arc displacement of eye-lids or via the computing of linear displacement measure using IR scanning approach (versus physiological IR reflective signal (i.e. oculography IR Amplitude measures) enabling a more precise measurement than conventional surrogate reflective IR amplitude measures.
***Figure 98*** Sentient state determination system incorporating reflective oculography and/or video face, head, eye-lid and eye movements. incorporating any or any combination of:
   The present examples provides capability of monitoring a subject/drivers sentient state or related transition based on any of or any combination of capabilities including (but not limited to) ***BLOCK 1 forward direction vision tracking*** (road targeted video vision, wearable or environment located/mounted monitoring technology, high resolution video eye characterisation technology, reflective oculography, video eye-blink oculography, day or night vision capabilities, ***BLOCK 2 reverse direction vision tracking video or reflective oculography*** (subject/individual/vehicle driver or pilot etc.), ***[BLOCK 3] driver drowsiness*/*vigilance tracking without need for calibration based on the computation of the interrelationship of one or more measures of sentient state or associated transitions*** (i.e. continuous qualitative measure of any or any combination of continuous qualitative and/or quantitative measures or recognition or characterisation of, face position or movements, eye-gaze position or movements, head position or movements, body position or movements, body extremity position or movements, eye-lid motion, eye-motion, eye-lid closure speed, eye-lid opening speed, continuous qualitative measure of pupil size, continuous qualitative measure of eye open time, continuous qualitative measure of eye-closure time, continuous qualitative measure of eye opening degree or linear distance or magnitude etc.) that are ***independent of inter-subject*** (different drivers) or intra-subject_(different trips or driving sessions of the same subject),
   ***[BLOCK 4]- driver drowsiness*/*vigilance tracking algorithm determination incorporating the capability of weighting or switching a plurality of input sources*** (i.e. one or more of video and or reflective subject monitoring), in accordance to signal source quality (i.e. SNR) and/or integrity (i.e. signal disruption or disturbance), ***[BLOCK 5] messaging** can **be enabled, [BLOCK 6]*** wearable or mobile device user-configured displays, indicators and specialised programming functions can be shred via user networks based on "like" acceptance or other security and privacy conditions, ***[BLOCK 7]-*** DRIVER VIGILANCE OR DROWSINESS DETERMINATION AND TRACKING ALGORITHM, ***[BLOCK 8]-*** INTERCONNECTIVITY CAPABILITIES AND ASSOCAITED TRACKING AND HEALTH MANGEMENT OUTCOMES, ***[BLOCK 9]-*** INTERCONNECTIVITY CAPABILITIES TRACKING OUTCOMES, ***[BLOCK 10]-*** INDICATORS/WARNINGS/ALERTS, ***[BLOCK 11]-*** MAP INTEGRATED FUNCTIONS & APPS, as further outlined here in ***Figure 39******;*** ***Figure 40******;*** ***Figure 41******;*** ***Figure 42******;*** ***Figure 43******;*** ***Figure 98******;*** ***Figure 99*** along with the associated eLifeALERT section and other patent descriptions included elsewhere in this document.
   ***[BLOCK 1]-* FORWARD DIRECTION (ROAD TARGETED VIDEO AND/OR REFLECTIVE OCULOGRAPHY TRACKING SYSTEM(S) INCOPORATING OPTIONS FOR-** *-WEARABLE OR ENVIRONMENT LOCATED MONITORING- -HIGH RESOLUTION VIDEO EYE CHARACTERISATION- -REFLECTIVE OR VIDEO EYE-BLINK OCULOGRAPHY -*
   ***[BLOCK 2]-*** REVERSE DIRECTION (DRIVER TARGETED VIDEO AND/OR REFLECTIVE OCULOGRAPHY TRACKING SYSTEM(S) INCOPORATING OPTIONS FOR- -WEARABLE OR ENVIRONMENT LOCATED MONITORING- -HIGH RESOLUTION VIDEO EYE CHARACTERISATION- -REFLECTIVE OR VIDEO EYE-BLINK OCULOGRAPHY -
   ***[BLOCK 3]-*** SELF-CALIBRATION AND ENVIRONMENT ADAPTATION CAPABILITIES incorporating any or any combination of:
      - ***including driver drowsiness*/*vigilance tracking without need for calibration based on the computation of the interrelationship of one or more measures of sentient state or associated transitions*** (i.e. continuous qualitative measure of any or any combination of continuous qualitative and/or quantitative measures or recognition or characterisation of, face position or movements, eye-gaze position or movements, head position or movements, body position or movements, body extremity position or movements, eye-lid motion, eye-motion, eye-lid closure speed, eye-lid opening speed, continuous qualitative measure of pupil size, continuous qualitative measure of eye open time, continuous qualitative measure of eye-closure time, continuous qualitative measure of eye opening degree or linear distance or magnitude etc.) that are ***independent of inter-subject*** (different drivers) or intra-subject_(different trips or driving sessions of the same subject),
   ***[BLOCK 4]-*** INPUT SIGNAL VALIDATION AND WEIGHTING DETERMINATION INCOPORATING OF CAPABILITIES OF -
      incorporating any or any combination of:
      - The video signal is analysed in terms of signal quality and integrity based on factors such as signal disruption based on obstructing items such as glasses, head-movements, environmental conditions such as poor lighting etc.
      - The reflective oculography signal is analysed in terms of signal quality and integrity based on factors such as signal disruption based on obstructing items such as glasses (where infrared or other light sensor and/or associated light generating source (LED etc.) is located as part of vehicle, or surrounding environment etc.
      - signal to noise ratio is established for video and/or reflective oculography in order to establish the quality and integrity of the signal sources;
      - based in the integrity (i.e. signal obstructions) and signal to noise ratio (i.e. reduction of signal in poor lighting or night-time conditions, not with-standing night vision capabilities video and/or reflective oculography capabilities) -head position and movement can be independently analysed in order to contribute to the determination of signal obstruction or reduced amplitude video and/or reflective oculography signal levels;
   ***[BLOCK 5]-*** MESSAGING/EMAIL INTERGRATED FUNCTIONS & APPS
      incorporating any or any combination of:
      - ***messaging can be enabled*** whereby alertness/vigilance and/or drowsiness/fatigue levels can be transmitted, shared, or distributed to one or more duly authorised destinations (i.e. alertness/vigilance and/or drowsiness/fatigue related information can be accessed or sent/transferred to one or more software application(s) or related call centres, central information system, Cloud-computing services, NAS, LAW, WAN, WWW etc., enabling remote monitoring or coaching or analysis or guidance),
   ***[BLOCK 6]-*** SOCIAL & BUSINESS INTEGRATED FUNCTIONS & APPS
      incorporating any or any combination of:
      - wearable or mobile device user-configured displays, indicators and specialised programming functions can be shared via user networks based on "like" acceptance or other security and privacy conditions.
      User preferences of smart watches (i.e. programmable watch face formats), related information interfaces, vehicle on-board computer system(s) or interfaces (and/or other wearable devices on body or extremities, vibrating watch alerts or alarms, can be programmed or configured to respond to alertness/vigilance and/or drowsiness/fatigue thresholds or regions of interest based on user preference). The user preferences can be "liked" and/or "shared" as part of community, social, business networks amongst groups (i.e. similar occupations, similar action sports, similar travelling or adventures, similar hobbies, similar fields etc.) of similar concerns or interests. For example, students wishing to maximise study time efficiency and memory recall capabilities by tracking for appropriate or acceptable vigilance levels;
   ***[BLOCK 7]-*** DRIVER VIGILANCE OR DROWSINESS DETERMINATION AND TRACKING ALGORITHM
   ***[BLOCK 8]-*** DRIVER VIGILANCE OR DROWSINESS ALGORITHM TRACKING OUTCOMES incorporating any or any combination of:
      - including but not limited (also detailed in PER BLOCK 3) to ***driver drowsiness*/*vigilance tracking without need for calibration based on the computation of the interrelationship of one or more measures of sentient state or associated transitions*** (i.e. continuous qualitative measure of any or any combination of continuous qualitative and/or quantitative measures or recognition or characterisation of, face position or movements, eye-gaze position or movements, head position or movements, body position or movements, body extremity position or movements, eye-lid motion, eye-motion, eye-lid closure speed, eye-lid opening speed, continuous qualitative measure of pupil size, continuous qualitative measure of eye open time, continuous qualitative measure of eye-closure time, continuous qualitative measure of eye opening degree or linear distance or magnitude etc.) that are ***independent of inter-subject*** (different drivers) or intra-subject (different trips or driving sessions of the same subject), together with quantitative measures of these and other measures, whereby said quantitative measures can be based on associated (i.e. comparative occupation (i.e. driver, teacher, student, business manager etc.) , gender, age, BMI, comparative fitness levels, past homeostatic sleep/wake patterns, past and existent **circadian** clock factors, environmental clock factors such as social or work or travel or time-zone clock factors, user-personalised preference factors such as safety level or safety risk of activity or occupation, user-personalised preference factors such as work or leisure or sports or social schedule requirements, user-personalised preference factors such as wake/sleep cycle and associated clock alarm essentials, user-personalised preference factors such as determined by self-learning algorithm incorporating population comparatives as well as the monitoring factors (i.e. health management goals, monitoring, outcomes and/or wearable technology minimisation monitoring approach (i.e. see also ***Figure 45******;*** ***Figure 75******;*** ***Figure 76***) monitoring minimisation and information aggregation applicable to subject/user (i.e. ***Figure 77******;*** ***Figure 78*****;** ***Fiqure 79;*** ***Figure 80***).
   ***[BLOCK 9]-*** INTERCONNECTIVITY CAPABILITIES AND ASSOCIATED TRACKING AND HEALTH MANAGEMENT OUTCOMES
   ***[BLOCK 10]-*** INDICATORS/WARNINGS/ALERTS
      incorporating any or any combination of:
      - Enabling wearable device (i.e. watch or other device worn on head, body or body extremities, mobile devices, or other processing systems can be configured by user to display or provide alerts personalised to user's specific activity, field or occupation, alertness study measure, etc. in order to be relevant to users specific or personalised requirements.
   ***[BLOCK 11]-*** MAP INTEGRATED FUNCTIONS & APPS
      incorporating any or any combination of:
   **Block 11 integrated map functions**
      - The present example provides a an alphanumeric, graphic, tabular, visual or graphic means of presenting as part of map overlay or associated information (i.e. but not limited to directional routing, locational markers with related information, map icons with associated map key or other related information) any of or any combination of factory default/library based parameter configurations, automatically or part of user-preference of social or work or sports or professional or other network configurations or generic operator or user interface layouts and co configuration options, in accordance to the present information derived or accessible monitoring, analysis and/or information outcomes including (but not limited to):
         - sleep/wake homeostatic factors (i.e. indication of sleep-urge/propensity based on homeostatic sleep'/wake factors and/or **circadian** clock factors and/or activity factors);
         - **circadian** clock factors (i.e. indication of CC synchronisation or synchronisation with environmental (i.e. travel click, local time-zone, work-clock, social clock etc.) and/of homeostatic sleep/wake factors such as sleep/wake cycle and a measure of previous sleep quality (i.e. sleep structure including deep-sleep, REM sleep status or shortage etc.) along with associated measures of CC delay or advancement;
         - any of options or adjunct systems described elsewhere in this patent document but not limited to:
            >MULTIPOINT TIME-SYNCH MONITORING (MTM) SYSTEM : ***Figure 30***
            >IR reflective oculography and/or video imaging (with IR capability) and/or light therapy integrated and/or mobile CC HMS application interfaced systems ((i.e. glasses): ***Figure 43***
            **>Automatic diagnostic and prognostic EEG-monitoring and analysis system incorporating minimisation process enabling professional and consumer-level monitoring and automatic analysis determination (MINIMISATION SYSTEM) per example** ***Figure 45******;***
            >HMS WITH AUTOMATIC TRACKING OF EOI per example ***Figure 75****;*
            >HMS sensor or monitor device/system (SOMDI) interface per example ***Figure 76****;*
            >Driver/subject wearable glasses incorporating any of or any combination of binocular or monocular
            >Self-learning and Personalised Self-Adaptation System AI; ES per example ***Figure 77******;*** ***Figure 78******;*** ***Figure 79******; and*/*or***
            -Personalised subject/patient- health management system (HMS) [1] incorporating any of or any combination of monitoring goals per example ***Figure 80******.***
            - drowsiness/fatigue and/or vigilance/alertness factors, including (but not limited to) those described per eLifeALERT section and elsewhere in this patent application document;
   ***[BLOCK 12]-*** CALENDAR/SCHEDULING /PLANNING INTERGRATED FUNCTIONS & APPS-B13 -the present example enables, alerts, messages, warnings, scheduling, planning scenarios, sleep /wake and/or occupational (dive/rest) or coaching suggestions based on any of or any combination of a subject/individual's **circadian** clock cycle factors (i.e. monitored and/or determined by examples described elsewhere in this document), in accordance to optimal vigilance windows of time, based on any on one more **circadian** clock cycle factors (i.e. predicted peak energy and vigilance time for an individual) individual
   ***[BLOCK 13]-*** OPTIONAL INTEGRAL OR ADJUNCT OR OTHER SYSTEM [BLOCK 14] incorporating any or any combination of:
      - MULTIPOINT TIME-SYNCH MONITORING (MTM) SYSTEM : ***Figure 30******;***
      - IR reflective oculography and/or video imaging (with IR capability) and/or light therapy integrated and/or mobile CC HMS application interfaced systems ((i.e. glasses): ***Figure 43******;***
      - Automatic diagnostic and prognostic EEG-monitoring and analysis system incorporating minimisation process enabling professional and consumer-level monitoring and automatic analysis determination (MINIMISATION SYSTEM) per example ***Figure 45****;*
      - HMS WITH AUTOMATIC TRACKING OF EOI per example ***Figure 75****;*
      - HMS sensor or monitor device/system (SOMDI) interface per example ***Figure 76***
      - Driver/subject wearable glasses incorporating any of or any combination of binocular or monocular
      - Self-learning and Personalised Self-Adaptation System AI; ES per example ***Figure 77******;*** ***Figure 78******;*** ***Figure 79******;***
      - Personalised subject/patient- health management system (HMS) [1] incorporating any of or any combination of monitoring goals per example ***Figure 80******.***
   ***[BLOCK 14]-*** DEFINITIONS :
      - WEARABLE OR ENVIRONMENT LOCATED MONITORING- (i.e. any of or any combination of wearable technology (i.e. glasses etc.), vehicle mounted (i.e. behind rear mirror/display etc.), room mounted (i.e. display screen, desk, wall etc.) other environment location (pilot or traffic controller surrounding cabin, room, cockpit etc.)
      - ***HIGH RESOLUTION VIDEO EYE CHARACTERISATION-*** (can include any of or any combination or, but is not limited to):
         (i.e. option of pan, zoom electronic or mechanical capability to enable very high resolution required for accurate eye-blink or eye-movement characterisation (i.e. tracking of acceleration or velocity of eye-lids at any point in eye-lid closure or eye-lid opening or eye iris shape change, or eye movement tracking etc. , applicable to drowsiness or vigilance diagnosis or prognosis of an individual at any point in time.)
      - ***REFLECTIVE OR VIDEO EYE-BLINK OCULOGRAPHY**-* (can include any of or any combination or, but is not limited to):
         - one or more single light-dependent or sensitive sensor(s) (i.e. infrared and/or daylight) in conjunction with light emitting diodes or other indicators (i.e. infrared, daylight or other visual frequency band). The transmission or a light beam and detection of the reflected light beam (i.e. reflected of the shiny surface of the eye can be based on pulses of light and detection of reflected signal or steady state signals or any combinations of both.

The pulsed signals can be at a frequency where the eyelid obstruction to the reflective surface of the eye can be tracked in order to detect the most subtle changes applicable to eye-lid velocity or eye-lid acceleration changes (i.e. based on the average eye-lid blink taking about 300-400msec we could, for example assume a very fast blink to be say 30-40msec.

If variations of blink time are to be examined a factor of at least 10 times this fastest blink rate or say 3 to 4msec accuracy would likely be required at a minimum to establish accurate variations in blink times. Therefore a video frame rate of approximately 333 frames per second or say 400 or 500 frames per second would be required to ascertain discernible variations related to the onset of driver drowsiness changes.

Acceleration changes related to the eye-lid under such conditions could increase by a factor of 10 or require (for example only infrared pulse of 400 per second for standard measures of velocity changes of blink rates, to 4000 pulses per second with acceleration changes. Similarly video rates of 400 to 4000 frames per second may be required in order to resolve such variations from a video frame, assuming the pixel resolution from eye-lid to eye lid is sufficient to capture such variations (i.e. at least 10 pixels across the eye opening at all times but preferably 100 pixels resolution (for processing determination of eye-lid characteristics, including but not limited to eye-lid opening (magnitude, degree of opening or linear measure of opening), eye-lid closing (magnitude, degree of opening or linear measure of closing), from centre of top eye-lid to centre of lower eye-lid, in order to cover all subject/individual inter-study and intra-study (monitoring sessions) variances. In the context of video reflective oculography the video monitoring is capable of detecting the light reflected from the surface of the subject's eyes as well as distinguish via video processing facial features (i.e. tracking the upper and lower eye lids and determining a measure of opening degree or magnitude, as well as deriving measure of velocity (i.e. opening degree or magnitude divided by the time of eye-opening or closure.

The velocity can be measures at any period or section of the eye-opening. Additionally, the change of velocity at a select period or section of the eye-opening can be determined. These measures can be examined on an eye-opening by eye-opening basis or by way of a running and continuous average value based on a defined period (i.e. over the past 1, 2 o5 or 10 seconds for fast response measures, as well as over the past series of minutes for slower measures). These various derived measures can be examined in terms of variation correlated with drowsiness (i.e. slowing of velocity with reduction of tonicity implicated with deepening drowsiness, or faster speed blinks associated with vigilant state. Additionally, the rate of blinks can also change with onset of drowsiness, including slower blink rates but more pronounced eye-closure rates (are also measured continually).

***Figure 99*** The dashed waveform here is indicative of the computed relative eye-lid closure *speed* (S) while the solid line waveform here is indicative of the *location* of the eye lid (or relative distance relating to the eye-opening, such as the linear peak distance from eye lid to eye lid). In this example the P at ½ way or 50% of maximum-magnitude (M) is approximately 100msec. The annotation labelled RPECS refers to relative peak *eye-lid closure* speed.

The present example enables, in the case of reflective oculography, quantitative, self-calibrated measures relevant to each subject/individual in terms of reduction of blink speed ***(S)*** and/or degrees or percentages of reduction of blink speed over a period of time, along with associated measures of relative blink eye closing or opening relative (absolute measures not applicable with reflective oculography but reflective oculography pulse, per above example, tends to increase with the eye-opening period due to the increase in reflected light from the eye-during increases opening periods.

The relative duration of each blink period (P) can be measured at of a designated cross-sectional point (such as ¾ or ½ amplitude). The relative peak eye-lid closing ***speed (RPECS)*** can be measured for each reflective oculography pulse (corresponds to each eye blink) based on M (relative measure of peak eye-opening magnitude) divided by relative duration of eye-blink (P), whereby relativity is a progressive qualitative measure of blink by blink velocity. Additionally, graduated measures as well as thresholds and working ranges can be defined in order to determine measures or classifications of drowsy versus alert driving conditions.

Further capabilities of the present example enable normal and alert driving conditions to be determined manually or automatically based on a nominated initial driving period (i.e. first 5 to 10 minutes, for example only).

Objective video measurement and/or reflective oculography, via wearable devices such as glasses with infrared transmitter and receiver sensor monitoring or remotely (i.e. light transmitter and light sensitive receiver located within or as part of vehicle or other environment) as a means of deriving a waveform according to one or both eye-blinks of an individual, can be deployed as a means of tracking subject's/individual's sentient state.

The video measures can be computed in terms of estimated linear dimensions based on an approximation of the distance of the driver or individual from the camera based on recognition of other aspects of the video such as the location (backward or forward positioning) of seat etc. The above relative approach can also be applied to the video derivations.

The present example can use the reflective oculography and/or video monitoring and processing systems for the tracking and characterisation of a driver's eye(s) and eye-lid(s) as a measure or drowsiness or vigilance. >> The present example further enables monitoring and/or determination of driver/subject head or eye gaze, pupil, eye movement, eye-lid, eye-blink, for the determination of driver drowsiness onset or driver/subject vigilance/attention determination.
- The present example provides a means of **combining infrared oculography (IRO) measures with video eye measures** (i.e. eye-lid measures, face measures, head movement or position measures, body-movement or pressure measures such as via seat sensors or steering wheel interface to driver's body or limbs), **whereby in a first process a range of IRO measures** (such as those indicative of eye opening time, eye closure time, eye-lid opening velocity, eye-lid closure velocity, along with other measures further outlined in this section (ELifeALERT), together with any of or any combination of related measures) are computed along with confidence levels of such measures (i.e. Indication of signal quality via SNR or other signal verification processes).

In a **second process** are also able to be a range of **video measures** (such as those indicative of eye opening time, eye closure time, eye-lid opening velocity, eye-lid closure velocity, along with other measures further outlined in this section (ELifeALERT), together with any of or any combination of related measures) are computed along with confidence levels of such measures (i.e. Indication of signal quality via SNR or other signal verification processes).

In a **third process** based on **confidence levels associated with signal quality of said IRO and/or said video measures** along with a range of thresholds, limits and subject physiological (I.e. Eye-lid velocity and/or eye-open and/or eye-closure times etc.) and behavioural (I.e. Eye gaze, body movements, steering wheel movements, steering wheel pressure, steering adjustments etc.) conditions applicable to a range of driver or subject alertness and drowsiness conditions the present example can determine which measures of IRO and/or video can be most reliably utilised for determination of graduated levels or transitions associated with driver drowsiness or alertness, thereby my mitigating interference or errors associated with any singular IRO or video taking mode.
- A further example is to provide **"sentient state"** tracking whereby the determination of said state comprises of any of **or combination of reflective oculography** and/or **video measures,** the present example further comprising any of or any combination of:
   - **video "derived and associated measures and or tracking of same"** (i.e. based on day-light and/or night vision with automatically adjustable monitoring conditions such as but not limited infrared lighting and video monitoring combined with day-light monitoring) **vision processing capable of determination and/or recognition of** "facial characteristics" applicable to sentient state (cognition level or state and/or transition and/or vigilance state) of a subject/individual (such as but not limited to driver, occupational, sleep/wake or homeostatic sleep factors and/or **circadian** clock factors of a subject/individual);
   - **reflective oculography** "derived and associated measures and or tracking of same" (i.e. based on day-light and/or night vision with automatically adjustable monitoring conditions such as but not limited infrared lighting and reflective oculography;
   - (measurement of steady state or pulsed light reflected from surface of subject/individual's eye-ball as an indicator of time of eye opening, velocity and/or acceleration factors (i.e. per figure showing wave-shape of reflected oculography signal - similar signal derivations in terms of deriving a signal indicative of eye-lid opening magnitude versus time of eye-opening can be derived via video measures);
   - the said **"combination and/or switching selection"** can be based on computing a measure of the oculography and/or video "signal quality determination" and/or " oculography signal or video integrity determination" associated with the weighting factor relevant to the combining process for one more or "sentient state" (or associated transitions) signals such as (but not limited to) oculography and/or video signals monitored and tracked from a subject/individual (i.e. driver or other occupation where drowsiness onset can be of issue or risk);
   - the said **"quality determination"** of signal can refer to accuracy and reliability of video and/or versus oculography signal(s). i.e. signal to noise can impacted by environmental lightning conditions where video or reflective oculography signals, applicable to the determination of eye-lid motion and opening or closing velocity, can be degraded by poor lighting conditions where background noise competes significantly with signals of measurement relevance;
   - whereby the said **"signal quality determination"** can be based on **resolution.** In the case of reflective oculography this resolution can be based on the **minimal discernible measure of change in eye-opening or closure** which can in turn be dependent on the number of, the distance of, the location and respective angle of reflected light sensor(s) as well as light source(s) (i.e. steady state and/or pulsed). In the case of video monitoring, the resolution determination can, for example, resolution can be **based on the minimal discernible measure of pixels of video applicable to the measure of eye-opening.** For example, 0 to 10 pixels change corresponding to eye-opening can result in a relatively poor accuracy as the eye-opening distance must change by 10% or more before a valid measure of change can be detected. Therefore this can adversely impacts upon the resolution and consequential sensitivity of eye-opening or eye-lid closure changes, which can be tracked as a measure or sentient-state. The present example, to counter such limitations **provides optional mechanical pan and zoom lens and camera targeting capabilities,** as well as options for multiple cameras so that one camera can communicate the location of eyes to a tighter focus camera which focusses with higher resolution primarily on the eye and eye-lid regions. In this way **driver head and face characteristics can be tracked** whilst still enabling high resolution and accurate eye-lid velocity and linear or magnitude of eye-opening measures;
   - whereby the said **"oculography signal or video integrity determination"** can be based on the disruption of the respective signals or video - i.e. due to glasses or other wearable face or head devices, or due to head movements etc. Additionally, the signal integrity can be influenced by misalignment of poor between subject/individuals eyes or eye-lid, or head or body or face and camera focus or lighting focus or infrared or other reflective oculography light transmitter or associated receiver;
   - whereby said **"sentient state"** includes any of or any combination of (but not limited to) cognition level, awareness, alertness, attention-level, vigilance state, occupational, sleep/wake state, homeostatic sleep factors, and/or **circadian** clock factors of a subject/individual (such as but not limited to driver, occupational, sleep/wake or homeostatic sleep factors and/or **circadian** clock factors of a subject/individual);
   - whereby **"derived and associated measures and or tracking of same"** include **running average measures or trending variations applicable to drowsy diagnosis or prognosis** of a subject/individual (i.e. decrease of eye-lid closure and/or opening measures (such as degree of linear magnitude measures, eye-lid movement opening or closure velocity or acceleration can a contributing factor indicative of drowsiness onset);
   - whereby **"facial characteristics"** include tracking of any of or any **"combination and or switching selection"** of (but not limited to) eye-movements, eyelid opening time, eye-lid-closure time, eye-lid opening velocity, eye-lid closure velocity (whereby velocity can be computed by deriving linear or degree of eye-opening or closure and/or surrogate measures of eye opening as a qualitative measure of reflected oculography signal by way of measuring the infrared or daylight or blue-light or other light bandwidth signal reflected from one or both of subject/user's eye surface) ;

### SECTION B:

### eHealth&Life NEURO mobile wearable monitoring systems

### Title: eLifeNEURO/ EPILEPSY (incorporating Subject-Specific EEG-Monitoring Minimisation. Adaptation- Generic plus TBI, autism, depression/Stim feedback, epilepsy HFO; aura)

### BACKGROUND: PARKINSON'S

- In the USA Parkinson's affects up to 2 percent of the population and is the most common neurodegenerative disorder. In Australia the disease effects about 80,000 people and about one in five of these people are diagnosed under the age of 50. This degenerative disorder of the brain often affects body movements and speech.
- More than 1.5 million people in the United States are believed to have Parkinson disease, 70,000 new cases are diagnosed each year and the annual economic burden of PD in the United States alone is estimated to be $23 billion.
- The eHealthMEDICS health watch platform incorporates a range of essential health management systems capable of addressing the burgeoning and rapidly growing global disease burden in the most efficient and effective manner through the deployment of its consumer-professional healthcare everywhere model and platform for life.
- A key applicable to consumers and professional medical or scientific patients alike is the new Parkinson monitoring system which can be deployed as an integral function of the eHealthMEDICS healthcare everywhere platform.
- Early detection of Parkinson in concert with *early intervention such* as *medication; surgical intervention; deep brain stimulation* can potentially slow down and even prevent more serious Parkinson's disease progression.
- Idiopathic REM sleep behaviour disorder can enable early detection of Parkinson's.
- ***However, the diagnosis of Idiopathic REM behaviour disorder (RBD)*** or in other words detecting RBD in the absence of any other (other than the possibility of Parkinson's) can be a ***valuable marker of early onset Parkinson's*** and hence a valuable opportunity to avoid more severe development of this condition - i.e. life changing or even life-saving.

### BACKGROUND: AUTISM SPECTRUM DISORDERS (ASD)

Autism spectrum disorder (ASD) is a neurodevelopmental disorder of unknown etiology but characterised by a range of social and communication impairments. ASD has been reported to be highly heritable and affect 1 in 88 children and 1 in 54 males. Reports of increased prevalence amongst children demonstrate the need to better monitor, track and manage this disorder.

While existent diagnostic monitoring systems and services do play an important and essential role these prior art methods or devices are often overly expensive, cumbersome or restricted in terms of population access.

Apparatus comprising of more accessible and routinely available personal-assisted monitoring applicable to normal lives any-time and everywhere, can contribute to wider accessibility and improved management of some of debilitating or mild "disorders".
- Such "disorders" include ***"health conditions of interest"*** or "events of interest/EOI" (per "EOI DEFINITION" detailed in abbreviation table at rear of this document);

More accessible, affordable and ubiquitous health tracking and management technology as described in this document can contribute to improved prognosis, diagnosis, prevention, risk-mitigation and most importantly putting the control into the hands of the person who cares most about each specific individual's personal health.

### BACKGROUND: EPILEPSY

- Epilepsy or "seizure disorders" is the 4^{th} most common neurological disorder and affects people of all ages
- The human brain is the source of all human epilepsy.
- Seizures and epilepsy can also affect one's safety, relationships, work, driving and lifestyle.
- The perception (stigma) or how people with epilepsy are treated can be a bigger problem than the seizures.
- Worldwide 65 M people suffer epilepsy & 4 to 10 of 1,000 people live with active seizures at any one time.
- In USA >2M have epilepsy and 1 in 26 people will develop epilepsy at some point in their lifetime.
- 150,000 new cases of epilepsy in the United States each year
- 1/3 of people with epilepsy live with uncontrollable seizures as no available treatment works for them.
- In 6 of 10 epilepsy sufferers the cause remains unknown. ²³
- Epilepsy can be medicated and in more chronic surgical intervention or stimulations devices can help.

### BACKGROUND: Prior art consumer sleep monitoring:

Other examples of problems with the previous state of art consumer mobile wireless or wearable monitoring system are individuals wearing health tracking systems that claim to track sleep and even sleep quality yet these alleged sleep monitors are actually based on motion detection, often from simple watch or wrist-based devices. Given **that the most important stages of sleep are based on deep sleep stages and most importantly REM sleep,** and indeed these are sophisticated brain-controlled states, it is little wonder that in general the **user experience** as it **relates to consumer-sleep tracking systems are dissatisfied** and in essence often being misled. The concept of detecting REM or even deep sleep, an essential property of quality or restorative, via a wrist band is highly controversial in the best scenario and deleterious in terms of fitness, let-alone health tracking. **The premise that monitoring essential sleep parameters (such as EEG, EMG and EOG) can be bypassed using a wrist-mounted activity sensor is likely to discourage and bring into dispute the future of genuinely accurate or useful consumer-level monitoring and associated health management.**

### DESCRIPTION

This document described a series of examples designed to improve personal and mobile access to health-monitoring, analysis and ultimately improved quality of life and health outcomes using minimalisation monitoring techniques, unique automatic and optionally online analysis processes, and devices capable of providing combined routine sleep and health management tracking with enhanced data access, appropriate data access, along with controlled community sharing benefits, the examples further providing any of or any combination of (but not limited to) as outlined herein:

### PATENT DECRIPTION

Method(s) of **monitoring** and **"automatic determination"** online of continuously any combinations of a subject's **physiological, pathological** and/or **psychological** measures or states (i.e. consciousness, unconsciousness, sleep-stages, wake state, along with associated altered and transient states or stages) applicable to predefined criteria relating to **"events or health conditions of interest",**
***"events or health conditions of interest"*** or "events of interest/EOI" (per "EOI DEFINITION" detailed in abbreviation table at rear of this document) whereby **"preceding periods"** relate to any duration or combination or sequence of sleep-wake period(s), time(s) or date(s) and/or **events or health conditions of interest;**

Whereby said **"combinations"** can include (but are not limited to) distinguishing predefined **seizure** or **epilepsy** events of interest distinguished by way of any of or any combination of **physiological, pathological** and/or **psychological** measures **combined with predefined sleep, wake sleep stage(s), or other psychological** states **and/or** ***"health conditions of interest** "* or "events of interest/EOI" (per "EOI DEFINITION" detailed in abbreviation table at rear of this document);
a) Whereby said **"automatic determination" includes** any of or any combination of: a) **comparative** analysis of a **subject's** current **and/or** recent and/or **longer-term trended monitored parameters** with reference to **reference data** including any of or any combination of: a) subject-specific (i.e. base line or prior studies or monitoring data, b) normative population, c) **pathological** or **disease** or **disorder** data, as a means of distinguishing onset or incident of health conditions or associated biomarker events of interest;
b) **Method(s) of automatic delineation of idiopathic versus non-idiopathic** SBD comprising of incorporating pathological **subject-specific medical history** (i.e. myoclonus, myalgia) SBD causations or online automatic monitoring and diagnosing pathological SBD causations together with the determination of **sleep behaviour disorder** (SBD);
c) **Method(s)** comprising of analysing any **combination** of subject's muscle activity, **body or limb movement parameters, EMG signals, psychological states (i.e. sleep/wake states/stages),** and/or **idiopathic SBD** versus **SBD** with **causation determination,** as a means of assisting in the **delineating** between any of or any combination of **movement, nervous system, neurological disorders** (i.e. **Parkinson's), sleep disorders** (restless leg syndrome/myoclonus; sleep deprivation) and/or **muscle disorders** (i.e. **myalgia** or fibromyalgia);
d) **Method(s)** comprising of characterisation of **subject-specific parameters** (physiological, psychological and/or neurological) in order to **detect or predict** the earliest possible onset or incident of (.i.e. **"aura")** associated events disorders, biomarker events or periods or **health conditions of interest;**
   Whereby the criteria such as ranges, thresholds, changes and other properties of said **"subject-specific parameters"** can be **predefined or dynamically computed;**
e) **Method(s)** incorporating combinational (i.e. **multivariate analysis)** algorithms comprising any of or any combination of (but not limited to) **spectral** and/or wavelet and/or **dynamical** (non-linear such as **entropy** or **complexity** analysis versus linear) and/or **statistical characteristics** in order to distinguish said "events" or "health conditions of interest".
   **Method(s)** comprising of analysis based on **"trending"** and **comparative analysis** of **"preceding period" monitoring outcomes** including (but not limited to) regression analysis capable **delineating subtle longer term "trends of interest"** such as (but not limited to) ***"health conditions of interest** "* or "events of interest/EOI" (per "EOI DEFINITION" detailed in abbreviation table at rear of this document) , whereby **"preceding periods"** relate to any duration or combination or sequence of sleep-wake period(s), time(s) or date(s) and/or **events or health conditions of interest;**
f) Method(s) of **determining** combined events of interest such as characterisation of physiological **monitoring parameters** by way of **source localisation;**
g) Whereby **monitoring** can comprise of one or more **patient applied elements** to the **forehead** or other parts of the **head or subject's body** enabling **continuous monitoring** at least one neurological, sleep parameter, and/or other physiological parameter;
h) Whereby said **"automatic determination"** can include characterisation of any combination of subject's sleep state (including **deep-sleep or REM**) and/or biomarkers such as **spectral** (ISA; **HFO; FRs;** or other automatic **characterisation and detection** parameters detailed in this document) and/or associated phase **coupling/decoupling;**
i) Whereby said **"automatic determination"** can include remote wireless interconnectivity capable of **online or offline local or remote data interchange and/or adjustment** of parameters (per automatic **characterisation and detection parameters** detailed in this document) associated with said determination;
j) Whereby said **"biomarkers" or "events of interest"** can be referenced as a means of determining directional and characteristics and magnitude **applicable** to **therapeutic intervention** including medication, magnetic stimulation, implantable device(s) such as those incorporating implantable cortical electrode(s) monitoring and/or cortical electrode(s);
k) PHASE OR SPECTRAL COUPLING FACTORS (as detailed elsewhere in this document);

### Title: eLifeNeurojSubject-Specific EEG-Monitoring Minimisation. Adaptation- Generic plus TBI, autism, depression/Stim feedback, epilepsy HFO; aura,

### NEUROLOGY DISORDERS PROGNOSIS, DIAGNOSIS CHARACTERISATION AND DETERMINATION

**Prognosis or diagnosis of** events of interest including single events and/or ensemble(s) (cluster(s)) of **biomarkers** of interest and/or health conditions and/or health sequelae including any of or any combination of precursor or various degrees of severity of incident of disorders including those of the sleep disorders, cardiac disorders, respiratory disorders, nervous system disorders, muscular or movement system disorders, neurological disorders, neurobehavioral disorders, cognitive impairment disorders, autism spectrum disorders (ASD), Asperger's, Epilepsy and other seizures, dementia, dyslexia, Alzheimer's, Parkinson's, attention deficit hyperactivity disorders (ADHD), Huntington's, traumatic brain injury, depression, anxiety and other subject states, transitions or events and health conditions of interest;

**The present example further incorporates wearable diagnostic or prognostic monitoring system(s) incorporating the capability of automatic analysis capabilities applicable to the determination of the onset or incidence of *"health conditions*/*disorders or events of interest".***

The present example comprises of apparatus for monitoring one or more **EEG** signals from a subject and automatically characterising signals in terms of the (including but not limited to automatic analysis) determination of any of or any combination of analyses including:
- means of determination (i.e. FFT or measurement of filtered bands of **EEG** signals from one or more channels) of power in the higher frequency bands of or nearby the region of 8 Hz to 40 Hz as a measure related in a linear manner to the amount of injury to a subject's grey cortical matter;
- means of determination (i.e. FFT or measurement of filtered bands of **EEG** signals from one or monitored channels) slow waves in the delta frequency band of or nearby 1 Hz to 4 Hz, as a marker of more severe TBI with a linear relationship in accordance to the degree of injury to white cerebral matter; injury; and/or
- means of determination of changes in **EEG** signal coherence and/or phase (i.e. FFT and associated phase outputs values);
- means of determination of signal changes in **EEG** signal coherence and/or phase (i.e. FFT and associated phase outputs values) measured via **EEG** monitoring in frontal and temporal lobe regions;
- means of determination of changes in **EEG** coherence and/or phase (i.e. FFT and associated phase outputs values) measured via **EEG** monitoring in frontal and temporal lobe regions as a marker linearly related to the degree of injury to both grey and white matter;
- Whereby said monitoring method(s) and device(s) can incorporate communication interface(s) comprising any one or more wide area network (WAN), local area network (LAN), internet protocol (IP), network application services (NAS), and/or Cloud-computing services to enable the transmission of physiological or environmental monitored data and/or subject/patient information exchange and/or access to online analysis capabilities capable of supplementing local monitoring, analysis, storage, and/or indications;
- Whereby determination of the onset or incidence of "health conditions/disorders or events of interest", include enabling an "automated method of determining ***"health conditions of interest"*** or "events of interest" (per "DEFINITION" detailed elsewhere in this document);

- The present example can provide personalised or specially configured wearable monitoring (see figures minimisation; range of head wearable devises) combined with online analysis capabilities whereby previous diagnostic or standardised diagnostic populations study outcomes (i.e. see self-learning ***Figure 77 to Figure 80******)*** can be deployed to examine the connectivity factors of an individual who is subjected to a battery of interactive tests (i.e. **see** eLifeALERT section).
- **whereby a single or battery of interactive tests can incorporate eye and gaze tracking (i.e. per "DIM ENTIA /ALZHEIMER'S /ASD /ASP "** section sub-heading **"Cognition Impairment Assessment and Determination" detailed elsewhere in this document.) including audio-visual test with interactive requests, designed to test the lucid nature and cognitive performance of an individual at any time, but particularly as it applies to an instant prognosis or diagnostic related to a range of mild severe traumatic brain injury conditions, the said individual or battery of tests incorporating any of or any combination of:**
   - *a) **brain connectivity (i.e. coherence; Dipole outcomes),***
   - *b) **brain signal dynamics*** (i.e. associated with a plurality of course (i.e. lateralisation; anterioralisation; brain hemispherical symmetry; other brain or specific regional activation; brain Dipole activation; sequential Dipole activation, brain coherence activation; sequential brain coherence activation; thalamus-cortical activation or coherence or Dipole activation (see also eLifeNLDB section;
   - *c) **Spatiotemporal dynamics*** of associated with an individual and batteries of cognition tests;
   - *d) **MMN or oddball response AEP outcomes;***
   - *e)* ***correlation of visual (with option of audio) test using video and*/*or reflective electooculography eye-tracking outcomes (see also eLifeALERT section)***
   - f) clusters of events or "health conditions or symptoms of interest" "events of interest/EOI" (per "EOI DEFINITION" detailed in abbreviation table at rear of this document) associated with any of the said tests;
   - *g) **transcranial Doppler (TCD)*** measures using automatic scanning techniques to minimise application expertise required to track vessels is general areas of the brain in terms of suppressed vascular activity related to increased intercranial pressure. i.e. a head-mounted device capable of tracking small and major vessels within and associated with the ***brain's Circle of Willis cerebral*** (arteries of the brain an brain stem) can characterised in order to contribute to prognosis or diagnosis of TBI by way of associated vascular modification i.e. change in intercranial pressure associated with mild to serious traumatic brain injury. For example intercranial hypertension prediction can be measured based on TCD profile. The appropriate TCD profile of a measured subject/patient can be compared to the said subject/patient pre-diagnostic calibrated or normal or resting state or normal test state (vascular response test or steady state with exogenous or endogenous stimulation means) whereby previously validated subject/patient or population studies, along with the determination of associated threshold values for diastolic velocity as a diagnostic or prognostic markers distinguishing normal versus abnormal TCD measures associated with TBI (i.e. according to an acceptable diastolic flow threshold or range of acceptable values such as 20 to 30 cm/sec threshold or acceptable range versus TBI suspicious operating region, can be predetermined via subject/patient-specific pre-diagnostic data or population studies and information self-learning an diagnostic information aggregation and wearable system minimisation per examples in HMS and options or adjunct systems per ***Figure 45 to Figure 81***).
      A **pulsatility index can be determined and assessed automatically** (i.e. using means of with scan, find, target, lock-in target automatic TCD scanning method) as a ***diagnostic or prognostic marker of TBI*** also evaluation as a marker of TBI (i.e. an index threshold or acceptable versus TBI suspicious operating region can be predetermined via subject/patient-specific pre-diagnostic data or population studies and information self-learning an diagnostic information aggregation and wearable system minimisation per examples in HMS and options or adjunct systems per ***Figure 45 to Figure 81***).
      The present example can also detect and measure in terms of ***threshold or ranges of values suggestion suspicious diagnostic or prognostic markets of TBI measure of low or no back flow*** as acceptable or markers of interest in the context of TBI diagnosis or prognosis²⁴.
      Additionally, ***cerebral autorequlation following minor head injury*** can be assessed by way of TCD autoregulatory response examination of cerebral blood flow velocity response corresponding to rapid brief changes induced in the arterial blood pressure, providing markers of the autoregulation index (ARI; i.e. an index threshold or acceptable versus TBI suspicious diagnostic or prognostic operating region can be predetermined via subject/patient-specific pre-diagnostic data or population studies and information self-learning an diagnostic information aggregation and wearable system minimisation per examples in HMS and options or adjunct systems per ***Figure 45 to Figure 81***)²⁵.

### Description of Figures

***Figure 45*** Automatic diagnostic and prognostic **EEG**-monitoring and analysis system incorporating minimisation process enabling professional and consumer-level monitoring and automatic analysis determination.
- **Further comprising any of or any combination of:**
   - ***[1] HEALTH MANAGEMENT GOALS & RELATED AUTOMATIC SYSTEM CONFIGURATION OR PARAMETER ADJUSTMENT AND FINE-TUNING OR ALERTS-*** I.E. adjusts or recommends automatic analysis and monitor-headset minimisation parameter and formats to optimise system prognostic and diagnostic outcomes (based on appropriate privacy, security, authority access rights) to achieve optimal monitoring analysis for neurological disorder of earliest onset/aura, onset prediction, state or transitions of interest.
   - ***[2] COMPLEX EEG DIAGNOSTIC HEADSET DEVICE 1 (******Figure 45******):***
      - Monitored Subject with wearable Device 1: **EEG** comprehensive headset
   - ***[3] PROGNOSTIC OR DIAGNOSTIC-STUDY MONITORED SUBJECT WITH MOBILE WIRELESS DEVICE (******Figure 45******);***
   - ***[4] AUTOMATIC EEG HEALTH-CONDITION CHARACTERISATION & OPTIMAL PARAMETER; DETERMINATION ANALYSIS (******Figure 45******)* for determination neurological disorder of earliest onset/aura, onset prediction, state or transitions of interest.**
      - [1] Automatic **EEG** online monitoring analysis and monitor-headset minimisation parameter Monitoring **EEG** measures from a person's head;
      - **automatic analysis including EEG** analysis transformation including a means of determining the ***coherence analysis transformation*** of a plurality of monitored **EEG** signals are characterised in terms comparative and/or absolute coherence measures;
      - Said analysis including **EEG** source localisation, source coherence analysis **(****Figure 45****;** , **CENTRE: LEFT),** and measures representative of ongoing coherence changes **(****Figure 45****;** , **BOTTOM: RIGHT);**
      - Said ***"automated method of determining neurological prognosis or diagnosis"*** includes methods applicable to ***"health conditions*** or symptoms ***of interest** "* or "events of interest/EOI" (per "EOI DEFINITION" detailed in abbreviation table at rear of this document);
   - ***[5] ANALYSIS VARIANTS INCLUDING THIOSE APPLICABLE TO TBI; ADHD; AUTISM; EPILEPSY (******Figure 45******);***
      - The present example further comprises of methods of analysis for determination or characterisation applicable to ***"automated method of determining neurological prognosis or diagnosis"*** or ***"minimisation analysis"*** comprising any of or any combinations of:
         - analysis transformation including spatiotemporal analysis transformation(s) (i.e. **Figure 45****);**
         - analysis transformation including source localisation analysis transformation(s) (i.e. current density reconstruction (CDR) (***Figure 45*****)**;
         - analysis transformation including coherence analysis transformation(s) (i.e. **Figure 45****);**
         - analysis transformation including Dipole connectivity analysis (i.e. **Figure 45****);**
         - analysis transformation including coherence analysis transformation(s) (i.e. **Figure 45****);**
         - signal dynamic analysis (i.e. nonlinear dynamical analysis such as but not limited to entropy, complexity analysis transformation(s) (i.e. **Figure 45****);**
         - wavelet analysis (i.e. nonlinear dynamical analysis such as but not limited to entropy, complexity analysis transformation(s) transformation(s) (i.e. **Figure 45****);**
         - AEP analysis transformation(s);
         - AEP transformation(s) including **mismatched negativity** test paradigms and computational outcomes;
         - AEP transformation(s) including **non-linear dynamic signal analysis** (i.e. entropy analysis, complexity analysis, etc.);
         - **odd ball AEP test paradigms** including mismatched negativity test paradigms and computational outcomes;
         - fast online averaging techniques such as **autoregressive analysis with external input modelling;**
         - state determination (i.e. states, levels and/or transitions associated with any of attention, alertness, vigilance, drowsiness, concentration levels, wake, sleep, consciousness, unconsciousness, associated transitions, hallucinatory, hypnosis etc.);
         - A further object is to enable objective **cognitive measures of EEG outcomes** including evoked response measures;
         - A means (but not limited to) includes measures of **attention such as P300** or other brain responses to a stimulus (i.e. standard and deviant click or tone pips) in terms of the delay between the auditory evoked response (i.e. measured via **EEG** from subject) signal measured from a subject under investigation or routine monitoring (i.e. speaker or headphone or earphone click or tone pip generation) response and click signal;
         - A further object is the determination of a change (i.e. deterioration) in a subject's cognitive capabilities at any point in time (i.e. associated with **cognitive dysfunction or deterioration** and/or neurological disorder prediction and/or ***"health conditions*** or symptoms ***of interest***" or "events of interest/EOI" (per "EOI DEFINITION" detailed in abbreviation table at rear of this document).
         - The said AEP response measures can be by way of averaging in order to improve the **EEG** signal to noise measures, whereby the deviant and standard stimulus pulses can be averaged.
         - **The AEP test paradigm** can (but is not limited to) comprise of **mismatched negativity paradigm** whereby the response to standard and deviant stimuli can be examined in terms of the presence or delay of the delay of the P300 signal as a measure of an individual's attention to the said stimuli.
         - A further object is to combine a number of these measures such as the coherence
         - An further object is to enable ***helmet with accelerometer*** processing and download capability along with management tool to manage progressive trauma scale associated with biometrics associated with movement analysis, biometric monitored data, cognitive test data (i.e. via PC or mobile device Software Application enabling cognitive performance and subject-specific outcome tracking) applicable to cognition dysfunction or associated risk factors.
   - ***[5] ANALYSIS VARIANTS INCLUDING THOSE APPLICABLE TO ASPERGERS OR AUTISM PROGNOSIS OR DIAGNOSIS (******Figure 45******);***
      - artefact reduction or removal including electrode, eye-link, epileptiform discharges, bursts of muscle activity, other muscle activity, regression analysis for further eye movement and other unwanted signals or noise;
      - prognostic or diagnostic classification based on ***spectral coherence analysis transformation(s);***
      - prognosis or diagnosis of Autism Spectrum Disorder (ASD) or of Asperger's syndrome (ASP);
      - prognostic or diagnostic classification based on **EEG** spectral coherence analysis transformation(s);
      - successive periods (i.e. past 2 to 10 minutes of monitored **EEG)** transformed using Laplacian or current source density analysis;
      - greater ***definition of the source dimensionality*** using two stage approaches such as ***PCA followed by ICA;***
      - determination of classifier classification factors proven to be effective in the prognosis or diagnosis of ASD or ASP;
      - use of predefined ***ASP and ASD classification factors*** based on studies involving normal compared to ASD and ASP subjects or existent published data can be deployed;
      - determination of coherence values derived from **EEG** electrode pair combinations;
      - reduction of spectral components by way of deploying principal components analysis (PCA) as a means of reducing data set for subsequent coherence analysis transformation;
      - deployment of ***discriminant function analysis (DFA)*** as a means of classifying ASD and ASP subjects;
      - deployment of coherence based DFA rules to establish capability of distinguishing between ASP and ASD subjects;
   - ***[5] ANALYSIS VARIANTS INCLUDING analysis EEG spectral power bands*** across different **EEG** signal frequency bands such as increased ***low-frequency EEG power in bands such*** as Delta and theta, along with increased power in *high-**frequency EEG power in bands such*** Beta and Gamma (i.e. compared to alpha bands) typical amongst ASD sufferers, and visa-versa with healthy subjects; ²⁶
      - analysis of ***asymmetry characterisation of EEG signal including determination of left and right hemisphere, posterior and frontal brain activation*** (AEP and/or continuous **EEG** analysis transformations) along with the ratio of such brain activation regions;
      - analysis ***signal dynamics* EEG** signal characterisation including determination of ***non-linear dynamic analysis*** (I.e. entropy, complexity and the like);
      - ***enhanced power of the left brain region*** associated with ASD;
      - brain activation and/or ***coherence signal changes*** in terms of left and right hemisphere shifts in brain activation/energy and/or connectivity (i.e. Dipoles) including lateralisation and/or anteriorisation;
      - ***Multivariate or combinational analysis outcomes*** incorporating analysis transforms of any combination of **EEG** processing including the said : ***-analysis of EEG spectral power bands", "spectral coherence analysis transformation(s)", "asymmetry characterisation of EEG signal", "source localisation coherence values", "signal dynamics*** including ***non-linear dynamic analysis,***
      - ***Incorporation of any of or any combination of these analysis or multivariate*/*combinational analysis approaches to in order to minimise the quantity and simplify or streamline the application of the minimal configuration EEG monitoring*** requirements to achieve effective, reasonably accurate but less obtrusive routine monitoring configurations for any of or any combination of neurological, nervous system, muscle or movement disorders and/or other health conditions or events of interest (i.e. per Figure **45****);**
      - measures based on the ***coherence transformation*** analysis of ***one or more EEG spectral frequency bands of any combination of EEG electrode locations*** and/or ***source localisation locations of any brain activation regions;*** ²⁷;²⁸
      - analysis of ***epileptiform abnormalities*** and/or ***associated EEG events*** (i.e. any of or any combination of spikes, k-complexes, HFOs, ripples, high-frequency ripples, spindles, and/or other **EEG** wave analysis) as a potential marker of ASD; ²⁹;
   - ***[5] ANALYSIS VARIANTS INCLUDING HRV* / *EPILEPSY ONSET* / *DEPRESSION (******Figure 45******);***
      - characterisation of excessive neuronal activity known to be associated with increased autonomic nervous system activity corresponding to epilepsy affects;
      - tracking of R-R interval (RRI) or heart rate variability (HRV) of monitored subject's electrocardiogram (ECG) or pulse derivation as a marker of the autonomic nervous function;
      - derivation of RR via subject-worn motion (i.e. accelerometer) detection (i.e. earplug accelerometer, other limbs, head, or body extremities capable of sensing cardiac pulse);
      - derivation of RR via subject-worn photo-plethysmography (PPG) detection (i.e. earplug accelerometer, other limbs, head, or body extremities capable of sensing cardiac pulse);
      - derivation of RR via subject-worn plethysmography (i.e. earplug accelerometer, other limbs, head, or body extremities capable of sensing cardiac pulse);
      - HRV characterisation including low frequency (LF) determination (represents modulations of the sympathetic and the parasympathetic nervous system) including (but not limited to) the power of the low frequency band (0.04Hz -0.15Hz power spectrum density (PSD));
      - HRV characterisation including high frequency (HF) determination (represents parasympathetic nervous
      system activity) including (but not limited to) the power of the high frequency band (0.15Hz -0.4Hz PSD);
      30
   - ***[5] DEMENTIA ANALYSER (******Figure 45******);***
      - Consumer or patient wearable or companion wireless device (carried or attached) with any combination of the following attributes:
         Analyser includes a microprocessor programmed algorithms to determine:
      - **Dementia analyser incorporating** location, falls/gyro, has family/carer pre-determined parameters of normal location or travel & behavioural patterns versus actual mobile tracking - predictive algorithm based on normal or predefined behaviour or routines travelling compared to potential dementia episode onset or incident (i.e. you set travel and road crossing parameters and boundaries as a marker or danger with online carer intervention via associated locational carer services team - plus special watch that communicates with person and can route them home, call a taxi or request other intervention.
   - ***[5] ANALYSIS VARIANTS INCLUDING THOSE APPLCABLE TO HFO*/*RIPPLES PER EPILEPSY (******Figure 45******)**;*
      Any of or any combination of measure or clusters combination/multivariate analysis of measures and/or temporal changes and/or spatial changes including:
      - HFO'S
      - ripples;
      - High-frequency ripples;
      - including any of or any combination of analyses, events of interest or health conditions of interest outlined as part of **Figure 45** and associated descriptions;
      - signal dynamics;
      - source localisation;
      - **EEG** channel pairs coherence;
      - brain hemisphere symmetry;
      - brain spectral source-localised power;
      - combinational/multivariate analysis; and/or
      - source localised connectivity (Dipoles);
   - ***[5] ANALYSIS VARIANTS INCLUDING THOSE APPLICABLE TO GAIT OR MOTION ANALYSER INCUDING PARKINSON ONSET SYMTOMS AND*/*OR COMBINED SBD SLEEP BEHAVIOUR DISORDER DETERMINATION (******Figure 45*** ***[5]);******Figure 75*** ***[20];[22]);***
      - **Parkinson onset analyser:**
         - In one example **trending analysis** along with related graphical, numeric or other outcomes can be presented to highlight trends indicative of neurological (i.e. or walking variation, or ***synchrony between limbs*** (such as motion and degree of legs versus arms swinging or movement flow and-over time as a potential marker of Parkinson's onset due to muscular, and/or neurological dysfunction),
         - In one example measures and graduated changes of over time ***enabling characterisation of walking, running, stride*** and other movement-negotiating or manoeuvring factors (such as the motion pattern ns related to negotiating or turning corners or changing direction, and the like, whereby this monitored or sensed information can be tracked and the associated outcomes relayed to subject's or appropriate healthcare-workers tracking or interested in tacking and accessing such information;

         -- In one example these measures and tracking capabilities can also be used to assist in the progress of ***physiotherapy associated with stroke*** and other debilitating disorders or accidents;
         -- In one example indicators or presentation of information can include any of or any combination of ***visual, numeric, graphical and other indicators*** or warnings applicable to tracking the fluidity and associated changes of an individual's movements, motion, walking, interrelationship with arms movement and walking stride can be characterised using locational and/or movement detection systems;
         -- In one example movement detection systems can include deployment and associated analysis of any of or any combination of or combination of step/pedometer measurements system, walking/pacing/running detection systems, speed, location tracking sensors and system such as any of or any combination of ***GPS, GSM, wireless network based measures, gyro-measurements, altitude, accelerometer systems, posture-position and*/*or gravitation measurements systems etc*.,** applicable to prognostic, diagnostic or subject personal-care management associated with nervous system, muscle or movement disorders, neurological disorders, other disorders or health conditions of interest including more specifically Parkinson's symptoms such as tremor analysis indicative of Parkinson's onset or incidence or associated medication control or requirements), sleep behaviour disorder (causally-linked or idiopathic; i.e. per ***Figure 75*** ***[20];[**2**2])***) such as trends or increasing tonicity during REM versus other sleep stages, amongst other combinations of pathological and physiological events or interest potentially indicative of health conditions of interest.
      ***[5]*** The present example **further provides analysis variants** capable of monitoring, characterisation and determination (i.e. **EEG** source localisation and/or Dipole activity and/or coherence interrelationships) of any of or any combination of:
      - **Perceptual Awareness Correlates or brain activity** (i.e. source localisation and/or Dipole activity and/or coherence interrelationships) located within parietal and prefrontal regions from an anatomical perspective;
      - Consciousness-dependent measures or changes associated with variations in physical stimulus, related to: Rivalry; and/or **Bistable perception,** and/or associated with **stereo pop-out** and/or or **change detection;**
         Clustering of activated foci apparent in superior parietal and dorsolateral prefrontal cortex;
      - determination of **Information "feedback"** (recurrent processing) **between the frontal cortex and other cortex regions, such as** mediated in accordance to **consciousness experience or changes;**
      - Brain signal changes or activity such as frontal-to-parietal brain communication;
      - Mediate sensory processing and associated brain **activity or interconnectivity determination,** which can occur outside of consciousness, such as mediated by **"feedforward"** information flowing in the posterior to anterior direction;
      - whereby said **EEG can be measured as a response to auditory stimulation** (i.e. via speakers embedded into sports protection headgear and/or helmets, and or via ear-phones or ear-pugs etc.) headgear;
      - whereby said **EEG can be measured can be part of a traumatic brain injury assessment and include any of combinations of EEG signal dynamics (i.e. non-linear dynamic analysis and/or coherence connectivity or brain region interrelationship/connectivity; or other analysis types as outlined elsewhere in this patent application document);**
   - ***[6] COMPARATIVE DATABASE REFERENCE DATA (******Figure 45******)***
      - A table or number of ***predetermined person-specific comparative criteria*** including population database (i.e. normal versus TBI impacted individuals), whereby normal versus abnormal operating ranges applicable to the person are established;
      ***[6]*** Examples of analysis variants comprises of any of or any combination of:
      - **signal dynamic analysis** (such as but not limited to non-linear dynamic analysis or entropy) for the determination of cognitive impairment or other events of interest or health conditions of interest (including but not limited to TBI);
      - **AEP spectral analysis example spectral signal analysis** for the determination of cognitive impairment or other events of interest or health conditions of interest (including but not limited to TBI);
      - **MMN and other AEP or ER test paradigms such as stoop test by way of online attention/awareness determination, such as, but not limited to, outlined under sub-headings elsewhere in this patent document application including:** ***Figure 45******;*** ***Figure 69 to Figure 71******;*** ***Figure** 75**; Fiqure 77 to* ***Figure** 80**)* for the determination of cognitive impairment or other events of interest or health conditions of interest (including but not limited to TBI);
      - whereby events of interest include single events and/or ensemble(s) (cluster(s)) of biomarkers of interest and/or *"health conditions* or symptoms *of **interest***" or "events of interest/EOI" (per "EOI DEFINITION" detailed in abbreviation table at rear of this document).

The present example further provides a means of automatic testing of any of or any combination of testing as part of aggregation of a subjects information via subject/user interface or other means **(****Figure** 77, BLOCK [8]), via data base(s) information aggregation (***Figure*** 78) expert system or artificial intelligence processes **(****Figure** 77), via databased information intelligence aggregation (per ***Figure 78***) , **(****Figure** 80; ***Figure*** 80 block [1]) and associated statistical performance assessment determination **(****Figure 80; *Figure*** 80 block [13]), the testing capabilities comprising any of or any combinations of:
**Intelligence assessments**
   - National Adult Reading Test (NART);
   - Wechsler Adult Intelligence Scale (WAIS);
   - Wechsler Intelligence Scale for Children (WISC);
   - Wechsler Preschool and Primary Scale of Intelligence (WPPSI);
   - Wechsler Test of Adult Reading (WTAR);
**Memory assessments**
   - assessment of any of or any combination of semantic memory and episodic memory (i.e. declarative memory or explicit memory);
   - assessment of any of or any combination of long term memory systems including procedural memory and priming or perceptual learning (i.e. non-declarative memory or implicit memory;
   - working memory or short term memory comprising any of or any combination of semantic memory (i.e. memory for facts), episodic memory (i.e. autobiographical memory), procedural memory (i.e. memory for the performance of skills), priming (i.e. memory facilitated by prior exposure to a stimulus), working memory (i.e. a form or short term memory for information manipulation);
   - California Verbal Learning Test;
   - Cambridge Prospective Memory Test (CAMPROMPT);
   - Memory Assessment Scales (MAS);
   - Rey Auditory Verbal Learning Test;
   - Rivermead Behavioural Memory Test;
   - Test of Memory and Learning (TOMAL);
   - Wechsler Memory Scale (WMS);
   - Test of Memory Malingering (TOMM);
**Language assessments**
   - Boston Diagnostic Aphasia Examination;
   - Boston Naming Test;
   - Comprehensive Aphasia Test (CAT);
   - Multilingual Aphasia Examination;
**Executive function assessments**
   - executive functions assessment as an umbrella evaluation for a range of cognitive processes and sub-processes;
   - executive assessment functions evaluation comprising problem solving, planning, organizational skills, selective attention, inhibitory control and some aspects of short term memory;
   - Behavioural Assessment of Dysexecutive Syndrome (BADS);
   - CNS Vital Signs (Brief Core Battery);
   - (CPT);
   - Controlled Oral Word Association Test (COWAT);
   - d2 Test of Attention;
   - Delis-Kaplan Executive Function System (D-KEFS);
   - Digit Vigilance Test;
   - Figural Fluency Test;
   - Halstead Category Test;
   - Hayling and Brixton tests;
   - Kaplan Baycrest Neurocognitive Assessment (KBNA);
   - Kaufman Short Neuropsychological Assessment;
   - Paced Auditory Serial Addition Test (PASAT);
   - Rey-Osterrieth Complex Figure;
   - Ruff Figural Fluency Test;
   - Stroop task;
   - Test of Variables of Attention (T.O.V.A.);
   - Tower of London Test;
   - Trail-Making Test (TMT) or Trails A & B;
   - Wisconsin Card Sorting Test (WCST);
   - Symbol Digit Modalities Test;
   - Test of Everyday Attention (TEA);
**Visuospatial assessments**
   - assessment of visuospatial function covering areas of visual perception, visual integration; and visual construction;
   - Clock Test;
   - Hooper Visual Organisation Task (VOT);
   - Rey-Osterrieth Complex Figure;
**Dementia specific assessments**
   - assessment of dementia including most vulnerable function comprising of testing memory, language, orientation and problem solving;
   - Clinical Dementia Rating
   - Dementia Rating Scale
**Batteries of tests for the assessment of neuropsychological functions**
   - assessment of cognitive skills;
   - Barcelona Neuropsychological Test (BNT);
   - Cambridge Neuropsychological Test Automated Battery (CANTAB);
   - Cognistat (The Neurobehavioral Cognitive Status Examination);
   - Cognitive Assessment Screening Instrument (CASI);
   - Cognitive Function Scanner (CFS);
   - Dean-Woodcock Neuropsychology Assessment System (DWNAS);
   - General Practitioner Assessment Of Cognition (GPCOG);
   - Hooper Visual Organization Test;
   - Luria-Nebraska Neuropsychological battery;
   - MicroCog;
   - Mini mental state examination (MMSE);
   - NEPSY;
   - Repeatable Battery for the Assessment of Neuropsychological Status;
   - CDR Computerized Assessment System;

### [7] SUBJECT/PATIENT INFORMATION & MED. HISTORY ETC. (Figure 45)

- The present example further incorporates or enables consideration of the monitored ***subject*/*patient history* as *an indication of potential risk, or status and*/*or idiopathic status*** associated ***diagnostic, prognostic characterisation or determination;***
- Subject/patient information include (but is not limited to) intelligence quotient (IQ), status of non-idiopathic disorders such as (but not limited to) sleep behaviour disorders and/or autism; age gender; other disorders and/or ***"health conditions*** or symptoms ***of interest** "* or "events of interest/EOI" (per "EOI DEFINITION" detailed in abbreviation table at rear of this document).

### -[8] AUTOMATIC EEG ONLINE MONITORING ANALYSIS AND MONITOR-HEADSET MINIMISATION DETERMINATION (Figure 45)

-- The present example further incorporates or enables ***high-temporal resolution monitoring*** with a minimal set of electrode and a minimally obtrusive selection of **EEG** location to monitor which still achieving acceptable temporal resolution based on a special analysis of a ***larger or super-set of EEG electrodes*** using high resolution temporal analysis techniques and then follow this with ***a subset of electrodes*** capable of monitoring the most significant brain region of interest based on subject-specific prognosis and diagnostic outcomes;

- Automatic **EEG** analysis for the determination of minimal configuration-**EEG** monitoring head-set requirements and/or analysis requirements and associated parameter settings in order to achieve acceptable level of prognostic and diagnostic outcomes (i.e. via means of predetermined or specified criteria) analysis for neurological disorder of earliest onset/aura, onset prediction, state or transitions of interest.
- "determining a neurological state of a person" can including monitoring a plurality of **EEG** signals **(****Figure 45****);**
   -the present example further provides a method whereby a ***first more comprehensive EEG electrode headset*** or ***head-cap* (i.e.** **Figure 45****;**
      **: TOP LEFT;** **Figure 1** to Figure 31, Figure 37; **Figure 49****),** or other head-attached and subject applied electrode monitoring system is used to monitor a super set of **EEG** locations followed by an analysis process which can determine the essential monitoring electrode (based on neurological, nervous, muscular or other physiological disorders of target interest) in order to establish a minimised or more simplified and stream-lined configuration of a second device **EEG** monitoring electrode configuration (i.e. ***Figure 43******,*** ***Figure 46 to Figure 50******,*** Figure 52, ).
   - The present example further incorporates or enables a reconfigurable or adjustable **EEG** headset allowing electrodes can be detached or reconfigured in terms of the minimal configuration **(****Figure 52****)** established by a analysis minimisation process **(****Figure 45****).**

In this way the ***first EEG head set*** or head-cap monitoring device **(****Figure 45****)** can be deployed as a more complex and sophisticated monitoring format in terms of enabling a more detailed and higher resolution spatiotemporal analysis of the subject's brain **(****Figure 45****;**
**: TOP LEFT),** in order to establish the minimum configuration and associated sub-set of **EEG** electrodes **(****Figure 45****)** required to effectively provide diagnostic and prognostic outcomes of suitable precision (based on defining events of ***interest such* as *heightened states of neurological disorder events of interest versus less severe sample period of neurological disorders;* (****Figure 45****).**

The said "second monitoring device" comprises of the minimum **EEG** electrodes and optimal electrode position determination to enable a stream-lined or less complex monitoring configuration for less-obtrusive daily or more routine-based (i.e. consumer-based) tracking purposes (i.e. ***Figure 43******,*** ***Figure 46 to Figure 50******,*** Figure 52, ).

### -[9] SIMPLIFIED EEG DIAGNOSTIC HEADSET DEVICE 2 (Figure 45):

**Subject monitored with simplified routine wearable EEH headset configuration based on professional diagnostic level to consumer-level minimisation analysis processes.**

### -[10] EEG ROUTINE OR CONSUMER-LEVEL DAILY/NIGHTLY HEALTH TRACKING HEADSET DEVICE 2: (Figure 45)

- **Monitored Subject with wearable Device 2: EEG simplified headset**

### -[11] AUTOMATIC EEG ANALYSIS EEG-MONITOR-MINIMISATION (Figure 45)

Automatic **EEG** Analysis for determination of minimal configuration-**EEG** monitoring analysis for neurological disorder of earliest onset/aura, onset prediction, state or transitions of interest.

### [12] ONGOING HEALTH-CONDITION CHARACTERISATION & OPTIMAL PARAMETER DETERMINITION MONITORING AND OUTCOMES PERFROMANCE ASSESSMENT ANALYSIS (Figure 45);

### [131 EEG MONITORING AND ANALYSIS IMPROVEMENT OR FINE-TUNING OF PARAMETERS AND OTHER YSTEM RECOMMENDATIONS OR AUTOMATIC CHANGES; (Figure 45)

### Example of analysis: Coherence and Dipole connectivity analysis

In oneexample a comprehensive ***first device* EEG** electrode monitoring headset with a comprehensive plurality of **EEG** sensors **(****Figure 45****;**
**: TOP LEFT;** , capable in one processing with ***one processing phase*** to determine important and useful spatio-temporal brain activation progressions or changes applicable to characterising the onset, incidence applicable to diagnostic or prognostic neurological, nervous system and/or muscular system disordered states or transition of interest **(****Figure 45****).**

A second process phase involving further analysis to enable the minimal electrode locations and number of electrodes in terms of enabling the diagnostic and prognostic precision and reliability in order to effectively detect the onset, or incidence of states or associated transitions applicable to events of interest including ***"health conditions*** or symptoms ***of interest***" or "events of interest/EOI" (per "EOI DEFINITION" detailed in abbreviation table at rear of this document).

***Figure 46*** TOP: Simplified fashionable fore-head band **EEG** monitor system. >>

The headband in one simplified version can incorporate 3 forehead electrodes comprising Fp1 [2], Fp2 [4], Fpz [3]. The headband can be provided in a partial circumference version mainly covering the frontal forehead region, through to complete circumference version as shown in this figure. More extensive electrode formats can include additional electrode options such as any of AF7 [1], AF8 [5] and/or Iz [6] electrode as shown in the figure. The latter electrode is located on inside of rear of the headband enabling monitoring of **EEG** characteristics such as from frontal (anterior) to rear (posterior) or rear to frontal brain region energy shifts. BOTTOM: headband Somfit/eLifeNEURO with forehead **EEG**/EOG/EMG, LDR and/or motion health tracking capability

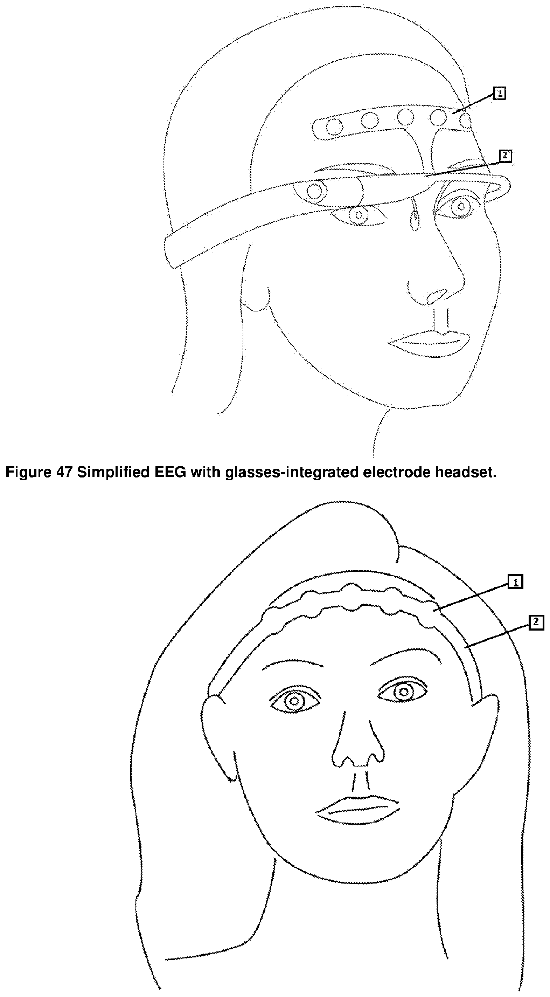

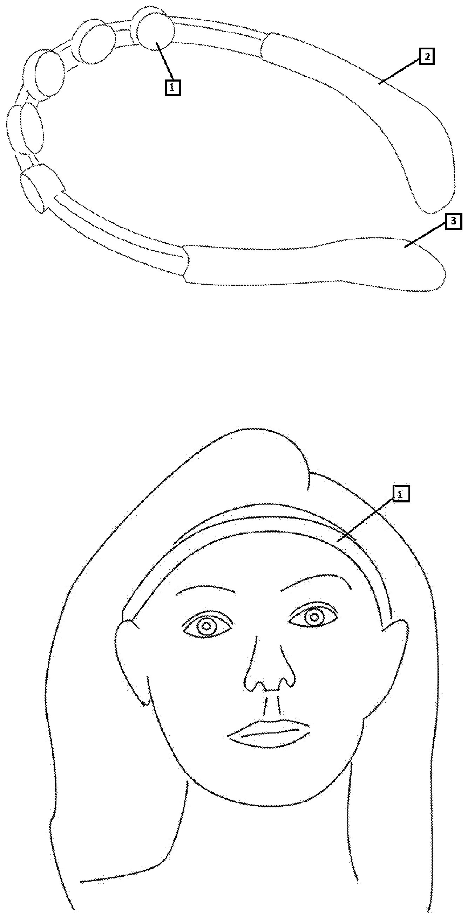

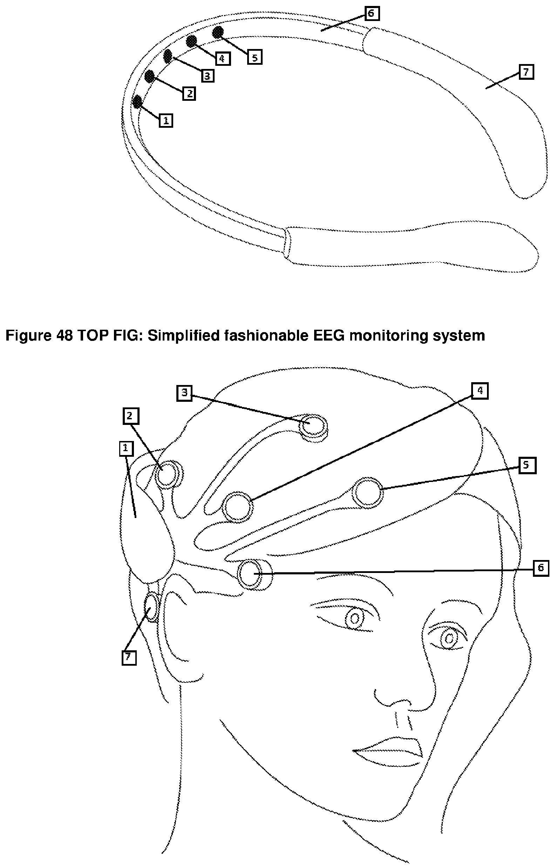

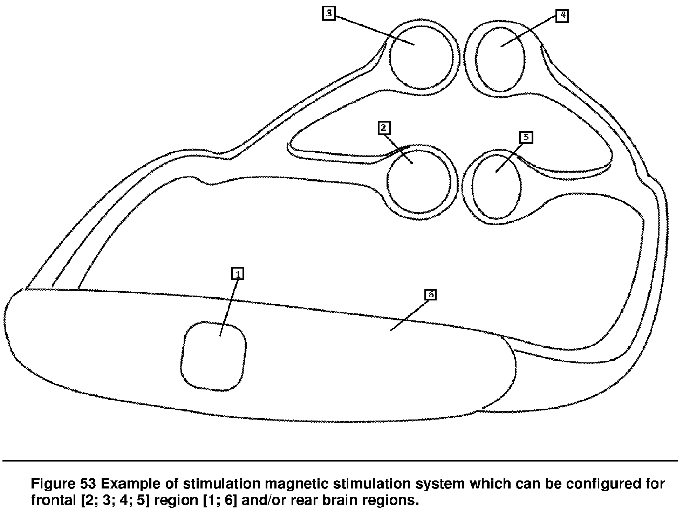

### Figure 53, Figure 48 TOP FIG: Simplified fashionable EEG monitoring system

>> BOTTOM FIG: electrophysiological sensors embedded on inside of sensor headband for **EEG** monitoring. "AUTOMATIC **EEG** ANALYSIS **EEG**-MONITOR-MINIMISATION" **(*****Figure 45*****)** can be deployed as a means of determination of **EEG** characteristics applicable to detecting **EEG** applicable to neurological disorder or health condition of interest. The specific analysis transformations and signal properties relevant to the simplified electrode headband (or other suitable **EEG** headband format, subject to particular subject/patient prior diagnostic studies or based on other studies outcomes or reference database parameters). In this way electrode minimal configurations (such as, but not limited to), multivariate analysis including any of or any combinations of two or more electrode monitoring locations and analysis transformations such as, but not limited to, coherence characteristics, signal dynamics characteristics (such as complexity or entropy analysis), spectral characteristics. The electrode, automatic online and/or spatiodynamic combination applicable to diagnostic and prognostic events of interest including single events and/or ensemble(s) (cluster(s)) of biomarkers of interest and/or health conditions and/or health sequelae including any of or any combination of precursor or various degrees of severity of incident of disorders including those of the sleep disorders, cardiac disorders, respiratory disorders, cardiac disorders, respiratory disorders, nervous system disorders, muscular or movement system disorders, bursts of muscle activity, other muscle activity, neurological disorders, neurobehavioral disorders, cognitive impairment disorders, autism spectrum disorders (ASD), Asperger's, Epilepsy and other seizures, epileptiform discharges, dementia, dyslexia, Alzheimer's, Parkinson's, attention deficit hyperactivity disorders (ADHD), Huntington's, traumatic brain injury, depression, anxiety, psychiatric and other subject states, transitions or events and health conditions of interest;

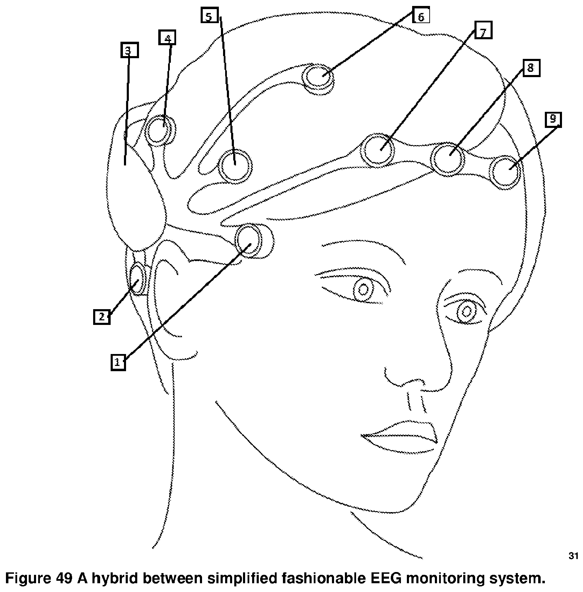

### Figure 49 A hybrid between simplified fashionable EEG monitoring system.

**>>A range of EEG headband configuration simplified or more complex electrode configurations can be configured in accordance to prior diagnostic studies (i.e. "AUTOMATIC EEG ANALYSIS EEG-MONITOR-MINIMISATION" (****Figure 45****)). The simplified studies can be derived via automatic statistical data mining (****Figure 80** **block [1; 2; 9; 13; 14]), analysis classification (****Figure 80** **block [8]), statistical analysis assessment (****Figure 80** **block [13])and ongoing expert systems/intelligent system algorithm knowledge aggregation and transfer to knowledge (****Figure *77*****)(*****Figure 78*****) via fine-tuned analysis variants (****Figure 80****) and related subject-patient physiological data and medical information (*****Figure 78*****) monitoring and analysis algorithms. Discriminate Function Analysis derived functions, to classify patient/subject-specific "events of interest" or "health conditions of interest" (any of those events of conditions outlined in this patent application document). - The present example further incorporates or enables any combination of "AUTOMATIC EEG ANALYSIS EEG-MONITOR-MINIMISATION" (****Figure 45****) or AUTOMATIC SUBJECT-SPECIFIC STATISTICAL ANALYSIS MULTIVARIATE AND FACTOR DETERMINATION. More complex monitoring formats for more sophisticated monitoring requirements , but still derived from complex analysis outcomes as a means of determining predefined (based on spatio-temporal and/or coherence characteristics subject-specific diagnostic studies or population healthy and disordered comparative data-base outcomes) as a means of detecting neurological states including autism, traumatic brain injury, epilepsy, autism, attention deficit hyperactive disorder (ADHD), Parkinson's, Huntington's, depression, cognitive dysfunction, or spatiotemporal source.**

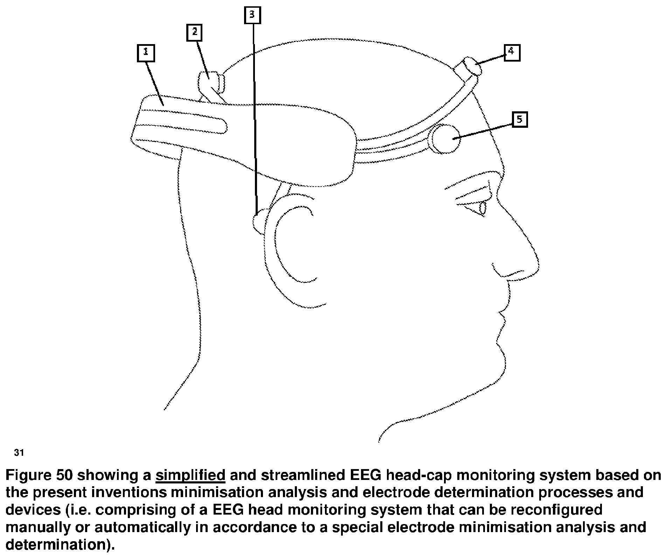

***Figure 50*** showing a simplified and streamlined **EEG** head-cap monitoring system based on the minimisation analysis and electrode determination processes and devices (i.e. comprising of a **EEG** head monitoring system that can be reconfigured manually or automatically in accordance to a special electrode minimisation analysis and determination). ³¹

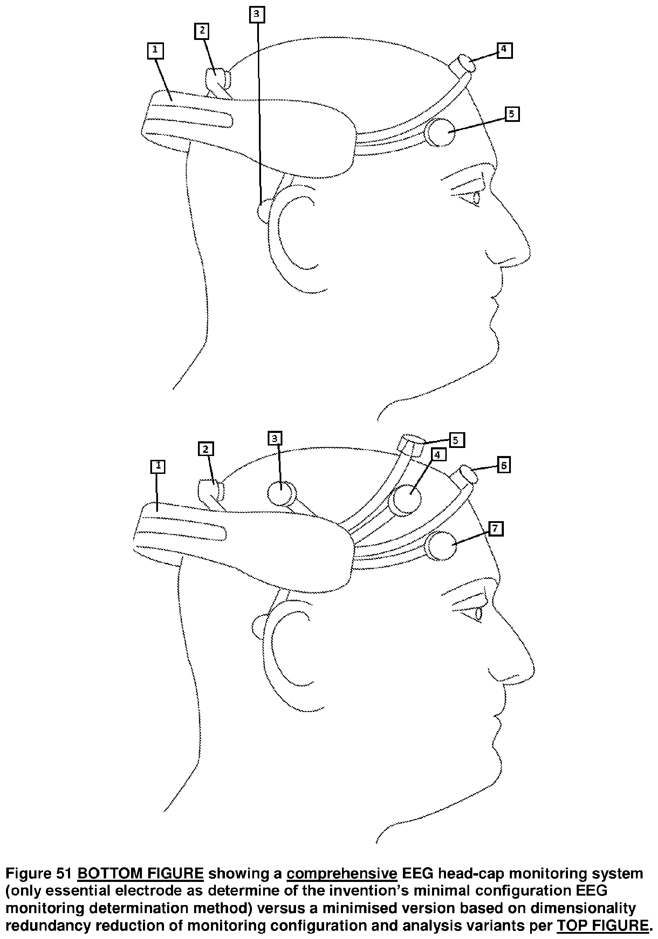

***Figure 51*** BOTTOM FIGURE showing a comprehensive **EEG** head-cap monitoring system (only essential electrode as determine of the minimal configuration **EEG** monitoring determination method) versus a minimised version based on dimensionality redundancy reduction of monitoring configuration and analysis variants per TOP FIGURE.

***Figure 52*** Example of electrode [5] minimisation system where a single device can have the non-essential electrodes [1;2] disabled or detached in order to provide a less obtrusive monitoring capability more suited to routine or event daily consumer level monitoring requirements.

***Figure 53*** Example of stimulation magnetic stimulation system which can be configured for frontal [2; 3; 4; 5] region [1; 6] and/or rear brain regions.

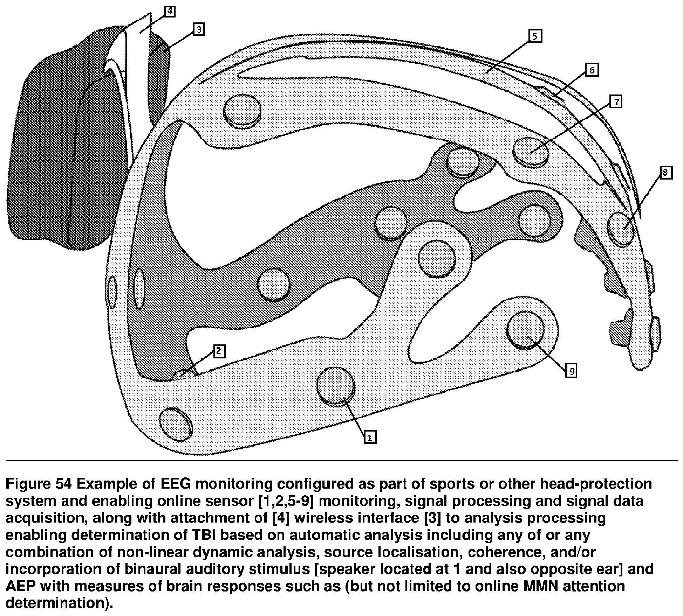

***Figure 54*** Example of **EEG** monitoring configured as part of sports or other head-protection system and enabling online sensor [1,2,5-9] monitoring, signal processing and signal data acquisition, along with attachment of [4] wireless interface [3] to analysis processing enabling determination of TBI based on automatic analysis including any of or any combination of non-linear dynamic analysis, source localisation, coherence, and/or incorporation of binaural auditory stimulus [speaker located at 1 and also opposite ear] and AEP with measures of brain responses such as (but not limited to online MMN attention determination). ³²

***Figure 55*** Example comprising of 133 channel high-density monitoring.
>>>TOP LEFT: 133 channel high-density monitoring head-cap; TOP RIGHT: Dipole source localisation accuracy and associated connectivity measures: Dipole location computed converged using standardised MRI image database, current density reconstruction (CDR). Most substantial Dipole (RED) appears to be centralised according to the Thalamus region; CENTRE LEFT: The left-lateral image view emphasises the central and occipital brain activation, typical during sleep; BOTTOM LEFT: brain top-view topographic MAPS, the predominance of the 41 msec (TP41) AEP component, located around temporal region is likely to be contributing to the emphasis of frontal region brain activation (i.e. anterior localisation of the 3 Dipoles per top right figure. BOTTOM RIGHT: Dipole computations and ARC output table present 3 Dipoles, with 1 located in the Anterior region and 2 in the Anterior-posterior/Superior-Inferior (upper rear brain) region 15.¹⁵.

**TOP RIGHT, DIPOLE ANALYSIS:** CHANGING MIN LAG (where lag refers to the presence of **EEG** coherence according to the time-range parameter configuration (typically time lag or lead factors can be configured for maximum shift of +/-50% of the selected time-range. In this way FFT's or originating **EEG** waveforms can be computed and the overlaps according to the lag or lead configurations are detected.).

TIMES TO ADJUST ARCS - 3 ARCS; RESULTS: SLEEP (EV2 3M GM AVE); STUDY 2; FD BP, MAPS, 3D View: bot; COH Obj clip < 50%; CDR Par clip
< 36%; Min Lag 7.4ms; 120ms Max Lag; CDR transp. 100%; Electr. Transp. 90%; SRC COH (fixed) for CDR (sLORETA) Object ON; Dipoles (3 fixed MUSIC) Object On; Dipoles Strength Icon Activated; Auto Noise Est

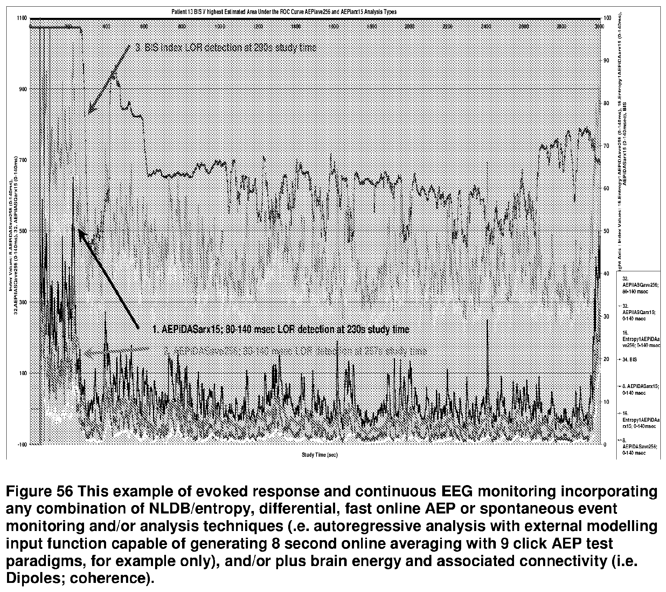

***Figure 56*** This example of evoked response and continuous **EEG** monitoring incorporating any combination of NLDB/entropy, differential, fast online AEP or spontaneous event monitoring and/or analysis techniques (.e. autoregressive analysis with external modelling input function capable of generating 8 second online averaging with 9 click AEP test paradigms, for example only), and/or plus brain energy and associated connectivity (i.e. Dipoles; coherence).
**>>>The graph here demonstrates the deployment of any of or any combination of automatic online "spontaneous event" and/or "evoked response" computational outcomes enabling any of or any combination of preconfigured and subject/patient-specific or generalised diagnostic and/or prognostic and/or biofeedback and/or treatment/therapeutic-linked outcomes or control capabilities. The said automatic online analyses processes can include, per this figure, example of analyses graphs (scatter-plots of AEP versus continuous EEG consciousness/mental monitoring and determination example, showing anaesthesia depth index values;** Burton D. A&CD (2009) Ref 13689 **³³) any combination of auditory evoked test paradigms applicable to processing contingent potential evaluation (i.e. MMN, odd-ball, AEP, using any of coherence, NLDB analyses, spectral signal analyses, morphological signal analyses) including as specifically shown here AEP entropy, AEP differential, and/or continuous EEG subject monitored parameters. >The present example further enables the incorporation of supplementary or "distributed processing" techniques, capable of being integrated via techniques such as "time-synchronisation" techniques (i.e. but not limited to "MTM" as described herein), such as (but not limited to) NAS, Cloud-computing services, LAN, WAN, IP, WEB intercommunication systems. The graph specifically presents scatter-plot overlays of consciousness versus unconsciousness dichotomised - conscious and unconscious surgical anaesthesia periods). >Best (AROC) BISTM values, AEParx15 (types 8;15,16;32) and AEPave256 (type 8; 32) values. The fastest patient-13 LOC transition was found to be arx 15-sweep AEP (Black-1), followed by AEP 256-sweep (Red- 2) and then BIS (Dark blue- 3). >This figure compares scatter-plots of BISTM data (blue) to the AEPi[arx15] analysis types (2.2 s; 15-sweep delay) corresponding to the 3 best overall total (TOT) 2-sample t-test p-values. BISTM data (type 33; with 3 LOC artifact values set to "0") resulted in a 2-sample t-test p-value of 8.8689E-05 (ranked 8th best). >The best 2-sample t-test p-value of 2.1983E-06 was computed from Entropy2AEPiDA [arx15] (red; type 17: 0-15 ms) results, followed by 6.7531E-06 computed from AEPiIASQ[arx15] (brown; type 25: 0-15 ms) results, followed by 1.2297E-05 computed from Entropy2AEPiDA[arx15] (black; type 23: 80-140 ms) values and the 4th best ranking of 1.1355E-05 computed from AEPi IASQ[arx15] (green; type 31: 80-140 ms) values. AEPilASQ[arx15] (type 31: 80-140 ms) corresponding to the green coordinate plots are referenced to the right y-axis scale while all other plots are referenced to left y-axis scale. >>>The present example enables any of or any combination of : a) wearable device monitoring (such as any of or any combination of** *a)* *Figure 48**;* *Figure 49**;* *Figure 50**;* *Figure 51**;* *Figure 52**;* *Figure 53**;* *Figure 54**;* **Figure 25*****;*** **Figure 3*****;*** **Figure 4*****;*** **Figure 5*****;*** **Figure 27****;_****Figure 3****,** **Figure 5****,** **Figure 16****,_****Figure 17*****,*** **Figure 21*****,*** **Figure 23*****,** b)* **analysis including any of or any of combinations of analysis as outlined in** *Figure 56* **to** *Figure 83* *c)* **AEP differential analysis (AEPiDAS),** *d*) **Fast autoregressive analysis modelling with external input function (arx 15-sweep AEP ; 2.2 s),** *e*) **slower AEP (38 s) latency-dependent averaging,** *f*) **non-linear dynamic (entropy) AEP indicator values;** *g*) **Evoked early-latency (0-28 ms) measures across the postauricular muscle (PAMR) region;** *h*) **Evoked PAMR measures as markers of intraoperative awareness during anaesthesia-induced muscle suppression;** *i)* **Fast (2.2 s) arx 15-sweep AEP detection of quick (0.25-3.0 s duration) body movements, quick arousals and noxious stimuli events were shown to be typical anaesthesia-specific events;** *j*) **online detection of these events as useful discriminators of sedation or anaesthesia-reversal, with the potential to reduce the incidence of intraoperative awareness** *k*) **measurements including the monitoring of hypnosis, amnesia, immobility, analgesia and anxiolysis, together with interrelated effects including awareness accompanied with elevated anxiolysis or anaesthetic-induced paralysis;** *l*) **optimal measurement combinations of slower trending EEG parameters, coupled with fast (2.2 s) and/or slow differential and non-linear dynamic (entropy) moving time averages (MTA), m) AEP measures computed across a wide-band of different latency intervals,** *m)* **determination of consciousness transitions, reaction and responsiveness to online events, and discrimination between peripheral and central nervous system signals;** *n*) *"health conditions* **or symptoms *of interest"* "events of interest/EOI" (per "EOI DEFINITION" detailed in abbreviation table at rear of this document);**

### ANALYSIS OPTIONS AVAILABLE AS PART OF ANY OF OR ANY COMBINATION OF AEP, ERP, AND/OR ANALYSIS FORMATS³³:

The present example enables a combination of AEP or ER analysis transfromations to be utilised in a single analysis context or combinational analysis context, where \by combinations of signal dynamics analysis processes (i.e. non-linear dynamic analysis methods such as entropy or complexity analysis) can be deployed as a means of determining consciousness and/or cognition status and/or levels and/or transitions as a diagnostic o prognostic tool applicable neurological or other brain, nervous or muscular system disorders (i.e. Alzheimer's, TBI, mTBI, dementia, spectrum of autism disorders, ADHD, Parkinson's, etc.).

### AEP/ERP Data Pre-processing

The present example enables ERP or AEP data to be analysed using a range of primary and secondary analysis transforms computed across one or more channels and also one or more progressive AEP or ERP signals.

Averaging or regression modelling techniques, including approaches capable of achieving faster averaging response times or less online computational delay, can be deployed where more responsive online measures are required.

### Fundamental Signal Processing and Data Formatting

Prior to primary or secondary transformations/analysis the present example deploys a number of fundamental signal and data formatting processes including (but not limited to) more advanced processing Secondary ERP analysis is preceded by a series of primary functions including pre-acquisition signal processing (amplification, signal-filtering, alias-filtering), and fundamental post-acquisition analysis (such as sorting out of ERP along with associated events).

### Primary AEP/ERP Data Transformations

The present example can deploy a range of **EEG** and/or ERP (including AEP waveform analysis) and waveform analysis (mathematical and statistical transforms) primary data transforms.

The primary transformations can include epoching the monitoring periods of interest, baseline, baseline adjustment to minimise wavering of data due to factors such as electrode DC **offset**s or body movement artifacts, artefact rejection of unwanted signal noise or distortion, averaging a sequence of ERP waveforms, regression techniques enabling more sophisticated averaging such as use of regression modelling with an external input in order to improve online responsiveness of measures and minimise conventional ERP data averaging computational delay factors.

### Secondary AEP/ERP Data Transformations

Moreover, to the marker of the diagnosis or prognosis entropy and **EEG** and/or ERP analysis (mathematical and statistical transforms) are deployed as part of the secondary analysis processes which include analysis.

### Combinational Analysis

In terms of waveform analysis the present example can deploy any of or any combination of analysis measures using a number of models ranging from minimal to maximal models. The analysis approach can incorporate a range of full or complex analysis types combined in a manner whereby AEP and **EEG** measures can provide higher cognition determination than any single **EEG** analysis type or single AEP analysis type or solely AEP or sole **EEG** measures³³.

### Statistically based joint probability measurement accuracy determination

Refer to section titled "NEUROCOGNITION MANAGEMENT SYSTEM", under sub-heading "STATISTICAL ASSESSMENT OF CUMULATIVE TESTS".

Mathematical *Primary Analysis of raw AEP or AER waveforms includes any of mathematically* ***derived and*/*or statistically derived outcomes.***
∘ Mathematically derived outcomes include morphological (i.e. time-series signal pattern analysis) analysis as a means of deriving ERP peaks, troughs, and associated latency delays, phase coherence or phase interrelationship (with related or other ERP waveforms).
∘ Statistical analysis includes computations such as (but not limited to) computation of skew, kurtosis, median frequency of the amplitude spectrum from 8-30 Hz (or other regions of interest in the context of consciousness or cognition depth) including higher frequency cognition-related spectral power, percentile spectral edge determinations or other statistical analyses.

### Secondary Neural Connectivity Sequencer (NCS) Analysis

(i.e. further described below) as a means of deriving a sequence of brain connectivity activations based on resting or special conditions such as cognitive and/or motor task challenges, or vidual, electrical, magnetic and also auditory stimuli (including oddball or MMN test paradigms).

### Secondary Neural Connectivity Tracking (NCS&T) Analysis,

comprising of automatically toggling through connectivity parameters in a manner whereby the maximum and minimum lag time between interconnecting regions can be toggled through in a sequential or other manner (successive approximation etc.) in order to determine the level of sensitivity in terms of activating interconnectivity across the brain at any time or corresponding to any stimulus or task challenge.

### Mathematical Primary Analysis of raw AEP or AER waveforms

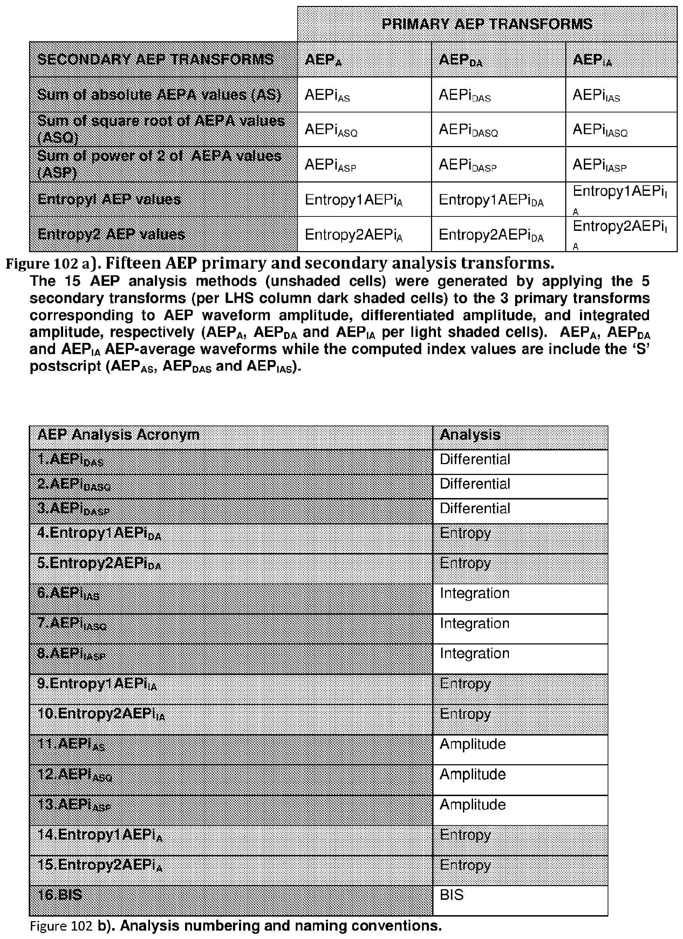

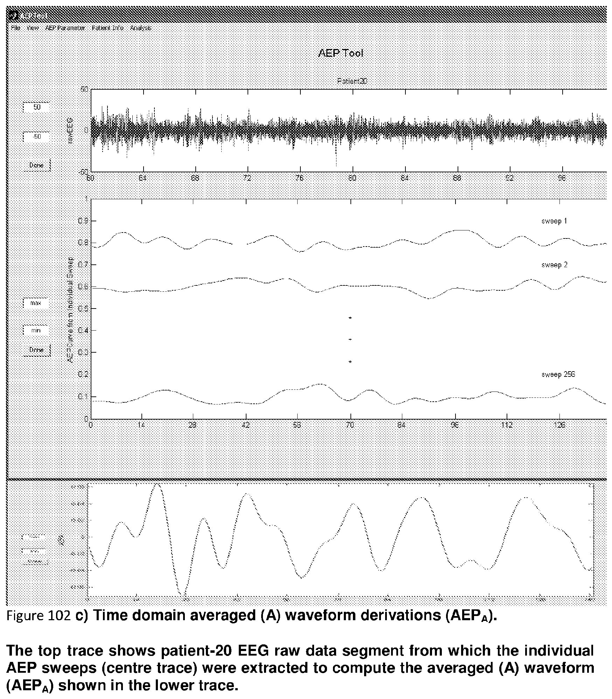

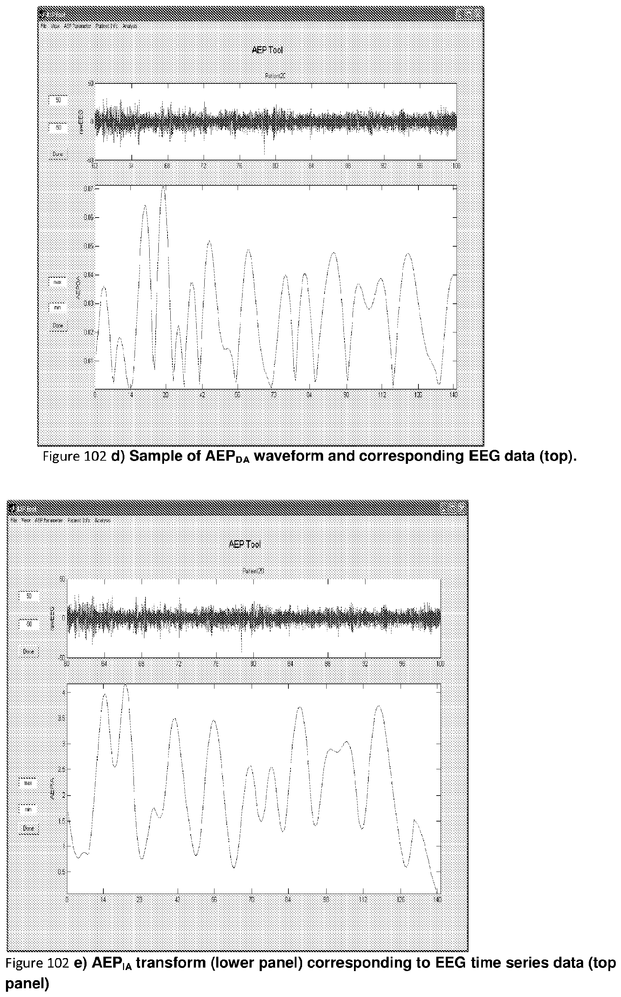

➢ Secondary ERP analysis is preceded by a series of primary functions including pre-acquisition signal processing (amplification, signal-filtering, alias-filtering), and fundamental post-acquisition analysis (such as sorting out of ERP along with associated events). At the For example, (but not limited to) the present example can compute any of or any combination of 5 secondary AEP methods with 3 primary AEP transforms in order to determine 15 AEP or ER analysis variants as presented in the table per Figure 102 **a).**
   A total of 16 sets of indicator values consisting of the 15 AEP index and BIS indicator values were generated for the 4 selected patient recordings and the analysis types were numbered and named according the table per Figure 102 b) conventions.
   The following section details background formulas used to compute the AEP_{A}; AEP_{DA}; and AEP_{IA} waveforms.

### AEP-Average (AEP_{A})

The AEP-average waveform was generated from the preceding 256 sweeps, except where greater than 75% of these sweeps were denoted as artifact. The AEP_{A} has also been described in earlier publications and referred to as the AEP-averaged or "grand mean waveform" (AEPgmw) 34

The AEP_{A} (256-sweep average) signal was computed in accordance to the formula: ${AEP}_{ave} \left[i\right] = \frac{{\sum }_{j = 0}^{255} AEPs \left[\left(i-j\right)*Δt\right]}{256}$ Equation 1
*AEPave*-represents the AEP-average waveform.
*AEPs*-single sweep auditory evoked potential.
AEP*_{[j]}-*raw AEP waveform at the instant *j*.
Δ*t* - time interval between the audio stimulus clicks ³⁵
The resulting AEP_{A} curve as is shown in Figure 102c) lower panel of Figure 102 **c).** right section. The upper panel represents the **EEG** raw data from which the lower panel of Figure 102 **c).** time-domain AEP-average was derived.

### First Order Derivative AEP (AEP_{DA})

The first order derivative or so called 'differential amplitude' is applied to the AEP-average (AEP_{A}), and the absolute value of the derivative AEP is defined as: $y \left(i\right) = \left|x\left(i\right)-x\left(i+1\right)\right| i = 1 , 2 , … 255$ Equation 2
Where y*(i)* is computed from the absolute amplitude difference between two successive points (total 256 points).
Where *x(i)* is the i^{th} data point of AEP_{A}, and y(*j*) is the *i*^{th} data point in the derivative of the AEP signal. The resulting first order derivative AEP curve as shown in the lower panel of Figure 102 **d)** in right section. The upper panel presents **EEG** raw data from which Figure 102 **d)** lower panel differential AEP waveform trace was derived.

### Integrated AEP (AEP_{IA})

The integrated (I) time-domain AEP-average signal (A) is designated 'AEP_{IA}' and is computed by:
i) Starting from the first data point, the mean value (DC component) of this 10 ms data is removed. The sum of the absolute value of the 10 ms DC-removed data is defined as the first value of the moving window integration AEP, i.e., AEP_{I}.
ii) Moving to next data point of repeating step (i). The resulting AEP_{IA} curve as shown in the lower panel of Figure 102 e) in right section. The upper panel represents the **EEG** raw data from which Figure 102 **e)** lower panel differential AEP waveform trace was derived.

### Secondary AEP Transforms

The five secondary AEP transforms were designated as the "sum of the absolute AEP_{A}, values" (S) ³⁶, sum of the square root of AEP_{A} values" (ASQ) ³⁷, "sum of the power of 2 of AEP_{A} values" (ASP) ³⁵, Entropy 1 AEP_{A} values" (Entropy1AEPi_{A}), and Entropy 2 (logarithmic version) AEP_{A} values" (Entropy2AEPi_{A}).

### Sum of absolute AEP amplitude values (AEPi_{AS})

The AEPi_{AS} values are defined by the following equation. $AEPiAS \left(t\right) = \frac{{\sum }_{i = 1}^{m} x \left(i\right)}{m}$ Equation 3 *AEPi_{AS}*
Where, *x(i)* is the *i*th data-point from the AEP waveform and *m* is the number of data point from the AEP waveform.

### Sum of square root of AEP amplitude values (AEPi_{ASQ})

The AEPi_{ASQ} values are defined by the following equation. $AEPiASQ \left(t\right) = \sqrt{\frac{{\sum }_{i = 1}^{m} x \left(i\right)}{m}}$ Equation 4 *AEPi_{ASQ}*

### Sum of power of 2 of AEP amplitude values (AEPi_{ASP})

The AEPi_{ASP} values are defined by the following equation. $AEPiIASP \left(t\right) = \frac{{\sum }_{i = 1}^{m} x \left(i\right) ∗ x \left(i\right)}{m}$ Equation 5 *AEPi_{ASP}*

### Entropy1 AEP_{A} values

The "Entropy1AEPi_{A}" entropy function^{38, 39} as outlined here.$Entropy 1 AEPi \left(t\right) = wnetropy \left(x\left(i\right), 'Shannon'\right)$ Equation 6 Entropy1

### Entropy2 AEP_{A} values

The "Entropy2AEPi_{A}" method was based on Shannon's mathematical theory of communication ⁴⁰ as outlined here. $Entropy 2 AEPi \left(t\right) = \left|x\left(i\right)\right| * log \left(\frac{1}{\left|x\left(i\right)\right|}\right)$ Equation 7 Entropy2 With three basic AEP waveforms, denoted as AEP_{A}, AEP_{D}, and AEP_{I}, fifteen (15) AEP indexes can be calculated by using five (5) methods.

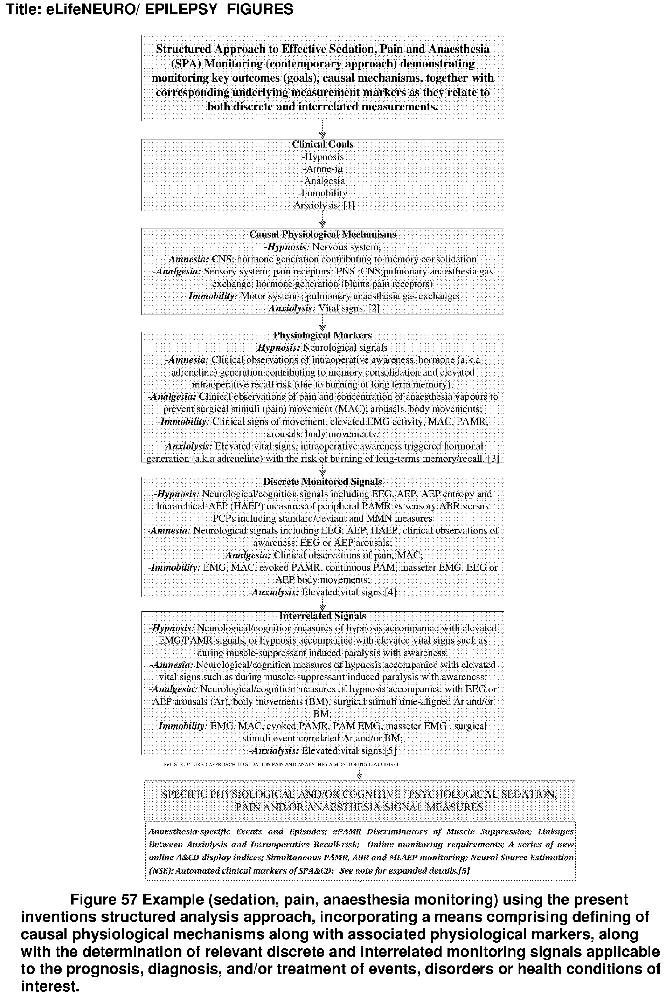

Figure 57 Example (sedation, pain, anaesthesia monitoring) using the structured analysis approach, incorporating a means comprising defining of causal physiological mechanisms along with associated physiological markers, along with the determination of relevant discrete and interrelated monitoring signals applicable to the prognosis, diagnosis, and/or treatment of events, disorders or health conditions of interest.
**>>> In this specific Sedation, Pain and Anaesthesia (SPA) Monitoring example this structured approach presents the capability to incorporate an overall subject/patient-specific or more generalised i.e. based on incorporation of other population normal or disease/disorder data bases) as a mean of analysing and/or wearable device monitoring and analysis configuration of any range of simplified to complex monitoring techniques based on any of physiological, psychological, pathological subject/specific or "broad-ranging" health management goals or monitoring goals for a subject /patient. >>>The present structured approach to enable subject/patient specific monitoring or health goal determination based on either existent patient history and symptomatic subject/patient health information (i.e. snoring plus daytime drowsiness, exhaustion or fatigue symptoms could be analysed as potential problematic sleep disordered breathing and consequently link via prompt and health management system hyperlink to a single "click and go" series of consumer-level or patient-level (professional medical options with further option of doctor, specialist or other level or suitably qualified and HMS system registered medical health-carer provider service) prognostic or diagnostic study coaching or monitoring solutions. Consequently, using the expert system/artificial intelligence capabilities (****Figure 45****;****Figure 75** **;****Figure 76****;** **Figure 77****;** ***Figure 78******;*** **Figure 79****;** **Figure 80****) with incorporation of further means of automatic determination of a range of minimalized to complex system monitoring configuration determinations (i.e. but not limited to per** **Figure 45****) the present example can automatically recommend, subject to means of determination of appropriate (i.e. on a country by country respectful basis, which can be determined by way of locational and internet protocol information at any time or in any place) regulatory and also means of determination of medical authority requirements, recommendations or restrictions and also subject to required or patient desired medical intervention (i.e. the present example enables the subject/patient to determine or "opt in" and "opt out" their specific health management system (HMS) community including private health community (i.e. the patient's chosen medical or other personal coaches, advisors, helpers or registered health experts as part of the community with rankings, recommendations, reports, similar experiences, qualifications, checks for authenticity and current medical or other authority board memberships and the like) or public health community (i.e. but not limited to similar experiences and solution shared by a broader health community) shared information or specific authorised information access or exchange based on subject/patient-specific preferences, privacy and security requirements. For example, if the subject/patient chooses to "opt in" or "authorise" a registered doctor, range of specialists, sleep specialist, scientist, technician , clinician, nurse, health coach, family or friend, or other selected organisation or individual the HMS system can determine the required or appropriate complexity level and automatically present the subject-patient with the range of specific health management options. >If, for example only the symptoms input to the HMS system appropriately conformed with and were appropriately supported (i.e. an appropriate questionnaire or survey plus some fundamental medical checks (i.e. as could be stipulated by government or medical insurance bodies or other similar authorities) and also if the symptoms such as snoring, with moderate sleep deprivation (based on both patient self-reporting but more importantly tracking of subject/patient's sleep patterns and outcomes via HMS compatible sleep tracking system) severe daytime exhaustion and minimal daily activity (based on both patient self-reporting but more importantly tracking of subject/patient's sleep patterns and outcomes via HMS compatible health and activity tracking system), coupled with the subject/patients option for including a registered sleep doctor and personal doctor (i.e. registered general practitioner or physician) could result in the presentation (i.e. per hyperlinked health monitoring options) to the subject of a range or more comprehensive monitoring solutions such as, but not limited to, American Academy of Sleep Medicine (AASM) or US Medicare type 2 or type 3 sleep study monitoring requirements, or Australia Sleep Association (ASA) or Australian Medicare unattended level 2 to 3 sleep monitoring requirements. >Alternatively, in the case with all things being equal except that there is not an appropriate level of compliance with medical oversight recommendations or medical reimbursement requirements, or regulatory requirements in general for a specific country or regions, then a more streamline or simplified consumer-level or health management coaching approach would be adopted by the HMS systems. Accordingly, in this more simplified approach the subject/user could be presented with more "simplified sleep or respiratory screening monitoring solution options" which could for example comprise of recommendations is terms of a subject/patient's sleep deficiency and likely sleep propensity, along with recommended sleep catch-up or sleep improvements suggestions, based on the HMS artificial intelligence or expert system analysis computational capabilities. In such as "simplified sleep or respiratory screening monitoring solution options" case the hyperlinked range of more simplified or consumer-level monitoring solutions could comprise (but are not limited to) of AASM type 4 guidelines or ASA level 4 sleep disorder breathing screening guidelines. >In this manner the present example can automatically determine the subject/patient's most relevant health management recommendations or coaching suggestions and enable the subject/patient to choose with as few as a single click a monitoring device, complete with configuration and analysis requirements the most appropriate health managements solution based on the subject/patient-specific health management requirements, HMS goals, HMS health community and oversight circumstances, and/or budget and/or complexity requirements of the HMS user. >The present example further enables biofeedback or therapeutic (i.e. ventilation or positive airway device intervention for automatic subject/patient-specific adaptation or reconfiguration purposes (i.e. adjustment and ultimate optimisation of arousal thresholds levels to maximise sleep quality and minimise sleep fragmentation, for example due to, daily or common variations in sleep propensity or deprivation, daily activeness (i.e. via information linked wearable activity monitoring system, etc.), changes in sleep to medication or sleep deprivation based on dynamic subject/patient-specific requirements (i.e. sleep deprivation and/or medication administration at any point in time for the specific subject/patients).**

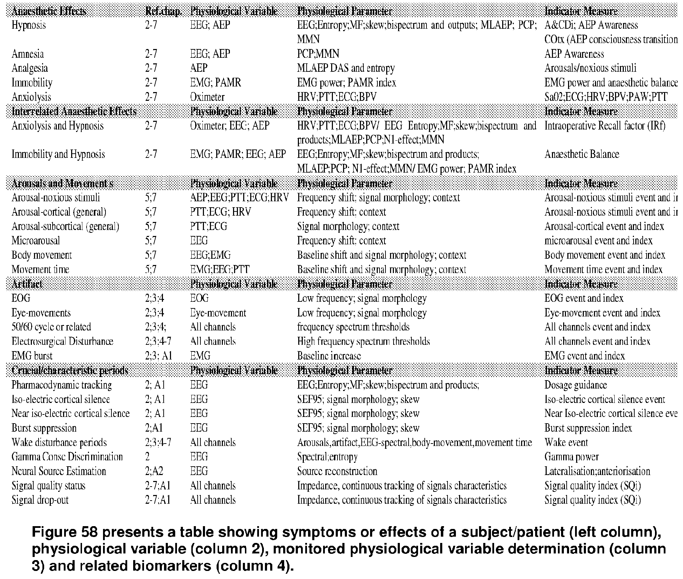

***Figure 58*** presents a table showing symptoms or effects of a subject/patient (left column), physiological variable (column 2), monitored physiological variable determination (column 3) and related biomarkers (column 4).
**>>>In conjunction with the structured approach per previous** **Figure 57** **to HMS determination of a range of minimalized to complex monitoring system configurations the table here (example only) presents an example of the capability to map out various events, symptoms or effects of a subject/patient (left column), which can be determined automatically or via HMS user (medical or user/patient access as appropriate under different circumstances). The physiological variable per column 2 can be mapped in accordance to the column 1 symptom or monitored events, in order that the most appropriately monitored physiological variable can be determined (per column 3), along with related biomarkers (column 4) capable characterising such symptoms, events or health conditions of interest.**

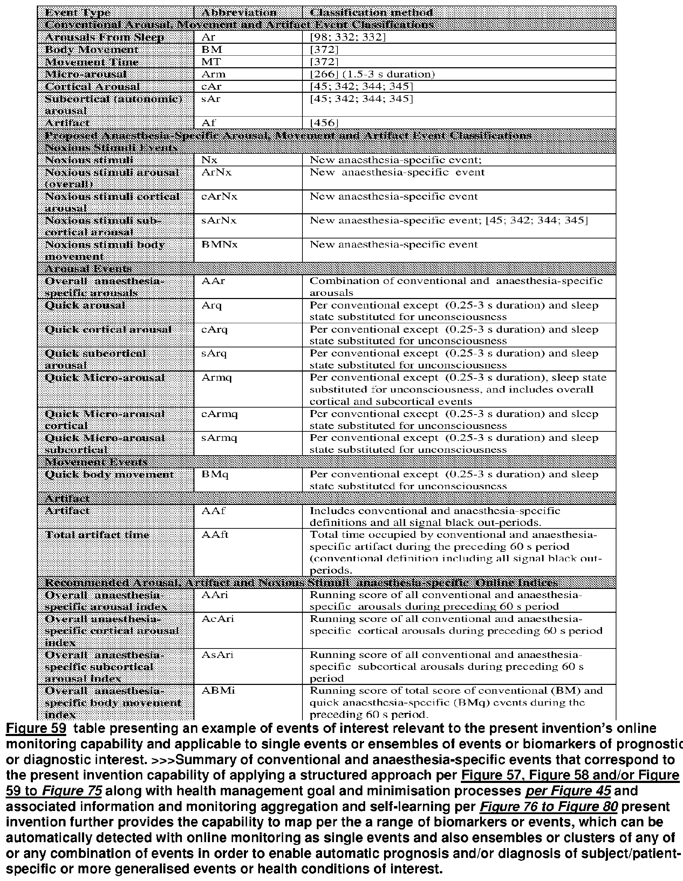

***Figure 59*** **table presenting an example of events of interest relevant to the online monitoring capability and applicable to** single events or ensembles of events or biomarkers of prognostic or diagnostic interest. >>>Summary of conventional and anaesthesia-specific events that correspond to the capability of applying a structured approach per Figure 57, Figure 58 and/or Figure 59 to Figure 75 along with health management goal and minimisation processes per Figure 45 and associated information and monitoring aggregation and self-learning per Figure 76 to Figure 80 present example further provides the capability to map per the a range of biomarkers or events, which can be automatically detected with online monitoring as single events and also ensembles or clusters of any of or any combination of events in order to enable automatic prognosis and/or diagnosis of subject/patient-specific or more generalised events or health conditions of interest.

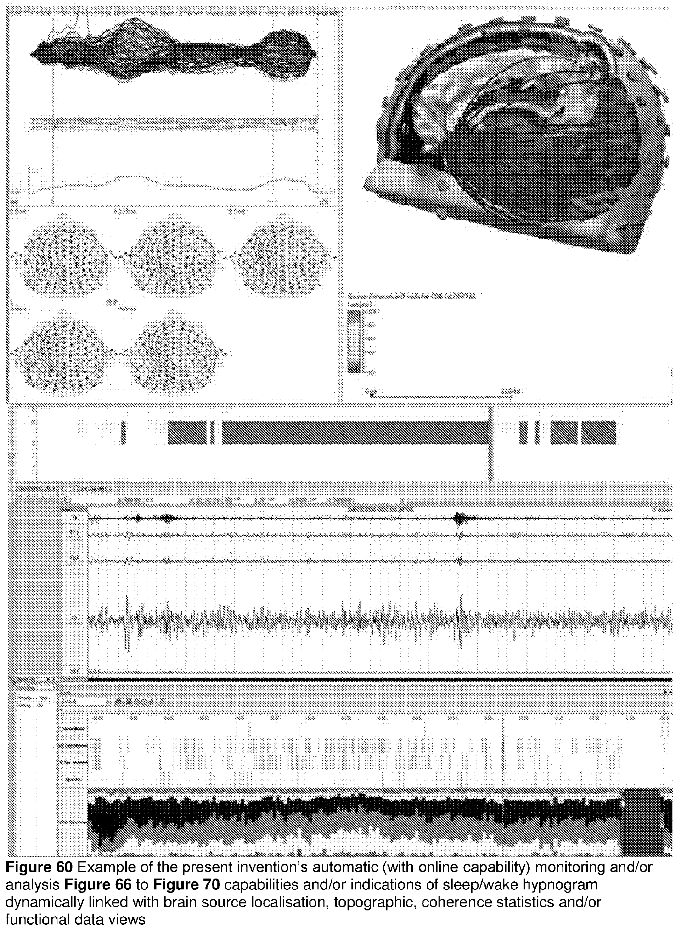

***Figure 60*** **Example of the automatic (with online capability) monitoring and/or analysis** **Figure 66 to Figure 70** **capabilities and/or indications of sleep/wake hypnogram dynamically linked with brain source localisation, topographic, coherence statistics and/or functional data views**

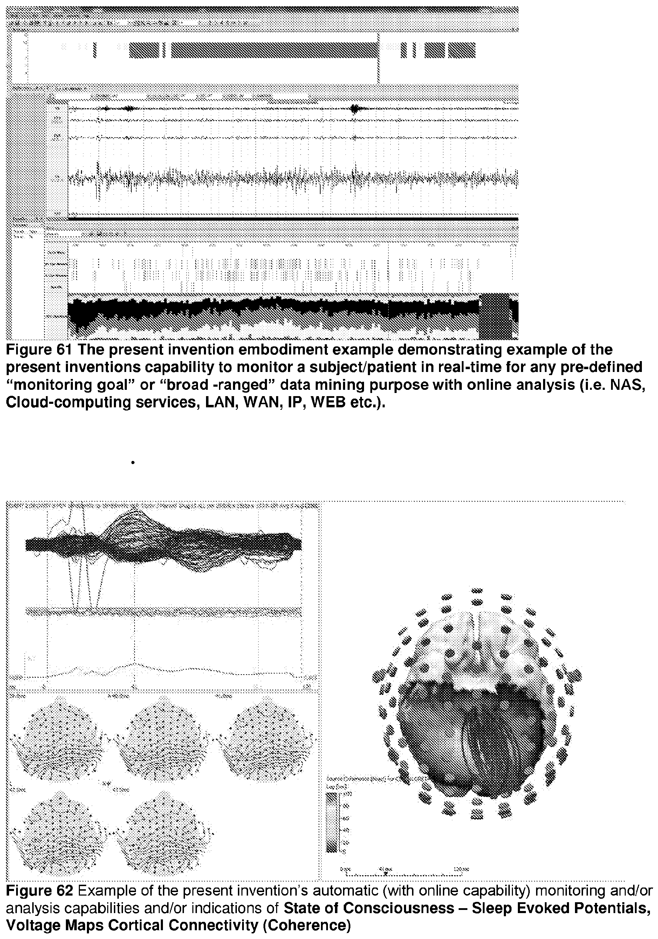

***Figure 61*** The present example demonstrating example of the capability to monitor a subject/patient in real-time for any pre-defined "monitoring goal" or "broad -ranged" data mining purpose with online analysis (i.e. NAS, Cloud-computing services, LAN, WAN, IP, WEB etc.***)*.**
**>>>>>In this example the subject/volunteer is configured with a more "complex " electrode cap but based on the wearable monitoring system "minimisation" process the subject-specific parameters can be automatically and/or with computer-assistance compute a "simplified" monitoring configuration (such as but not limited to) any of or any combination of analysis and/or wearable monitoring system configuration processes (i.e.** **Figure 45** **automatic wearable device configuration system ;** **Figure 45****;**
   **example of online physiological, psychological, pathological; diagnostic and/or prognostic monitoring system;** **Figure 56** **example of evoked response and continuous EEG monitoring incorporating any combination of NLDB/entropy, differential, fast online AEP or spontaneous event monitoring and/or analysis techniques (.e. autoregressive analysis with external modelling input function capable of generating 8 second online averaging with 9 click AEP test paradigms, for example only), and/or plus brain energy and associated connectivity (i.e. Dipoles; coherence);** **Figure 57** **structured approach example of the present example highlighting the incorporation an overall subject/patient-specific or more generalised i.e. based on incorporation of other population normal or disease/disorder data bases) as a mean of analysing and/or wearable device monitoring and analysis configuration of any range of simplified to complex monitoring techniques based on any of physiological, psychological, pathological subject/specific or "broad-ranging" health management goals or monitoring goals for a subject /patient. The Health Management System (HMS) enables a subject/patient's symptoms relating to the HMS goals or health management objectives to be automatically mapped in terms of the corresponding physiological parameters and associated biomarkers/measures (i.e.** **Figure 58****) or events (i.e.** **Figure 59****) of interest. The present example enables a range varying from minimalistic/streamlined to more complex (i.e. but not limited to** **Figure 43****;** **Figure 45****;** **Figure 43****,** **Figure 46 to Figure 50****,** **Figure 52****, ) monitoring configurations to be automatically determined using specialised artificial intelligence/expert-system processes (****Figure 76****;****Figure 77****;** ***Figure 78*****;** **Figure 79****;****Figure 80****) along with further unique analysis processes described herein (****Figure 45****;** **Figure 60****;** **Figure 61; Figure 62****;** **Figure 63; Figure 64****;** **Figure 65; Figure 66****;** **Figure 67****;** **Figure 68****;** **Figure 70****;** **Figure 71****;** **Figure 72****;** **Figure 73****;** **Figure 74****;** **Figure 75****;****Figure 76****;** **Figure 81; Figure 82****;** **Figure 83****) specific and configured in accordance to subject/patient prognostic or diagnostic or treatment requirements. More generalised data-mining approaches are possible subject to the monitoring and HMS goals configured by the system user and their associated health community. This particular figure demonstrates how even the most comprehensive evoked potential (EP) and spontaneous event monitoring is possible using fast averaging AEP techniques (i.e. but not limited to automatic regression modelling with external input modelling) and distributed or supplementary online analysis processes (i.e. Cloud-computing services, NAS, WAN, LAN, WEB, IP etc.).**

***Figure 62*** **Example of the automatic (with online capability) monitoring and/or analysis capabilities and/or indications of** State of Consciousness - Sleep Evoked Potentials, Voltage Maps Cortical Connectivity (Coherence)

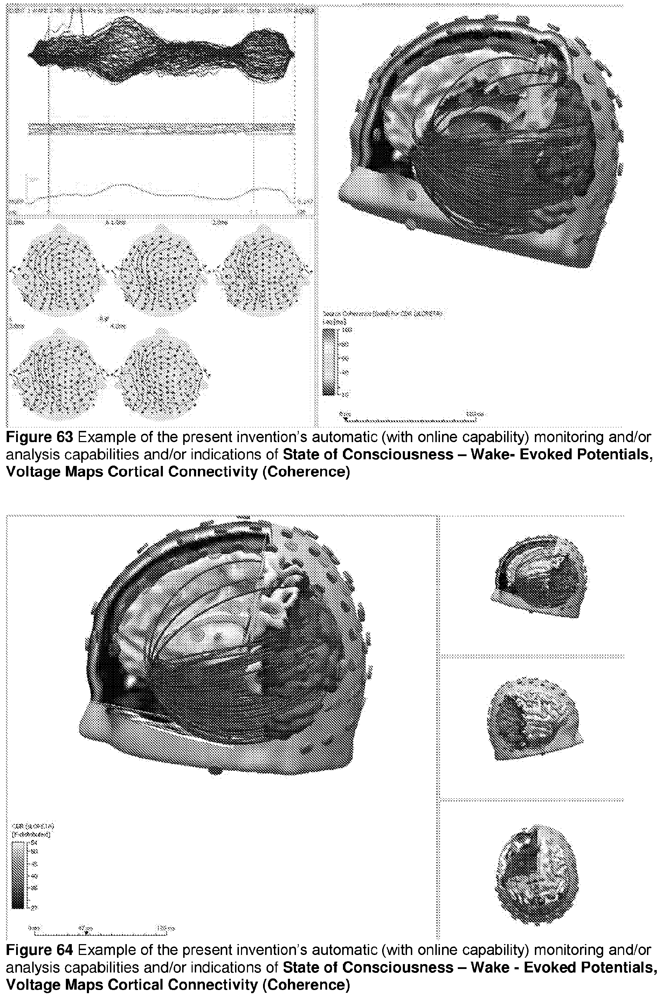

***Figure 63*** **Example of the automatic (with online capability) monitoring and/or analysis capabilities and/or indications of** State of Consciousness - Wake- Evoked Potentials, Voltage Maps Cortical Connectivity (Coherence)
***Figure 64*** **Example of the automatic (with online capability) monitoring and/or analysis capabilities and/or indications of** State of Consciousness - Wake - Evoked Potentials, Voltage Maps Cortical Connectivity (Coherence)
***Figure 65*** **Example of the automatic (with online capability) monitoring and/or analysis capabilities and/or indications of** state of Consciousness - Wake - Evoked Potentials, Voltage Maps Cortical Connectivity (Coherence) & Image Data.

***Figure 66*** **Example of the automatic (with online capability) monitoring and/or analysis capabilities with online source localisation and associated indications.**
>>>The indications and/or visualisations include **source localisation incorporates means of mapping brain activations regions of interest with cortical regions identified via system user predefined regions or interest or associated with existent or standardised brain atlas or other guides.** In this example of online, automatic averaging (AVE; including option of autoregressive analysis with an external input modelling), segmentation (SEG), current density reconstruction (CDR) and coherence (COH). The coherence arc activity based on the convergence of CDR f**EEG** and standardised MRI image data, computed during SLEEP (stage 2) period, presents a preponderance of neural interconnectivity between the thalamus and occipital cerebral cortex regions. In contrast, the coherence arc activity based on the convergence of CDR f**EEG** data and a standardised MRI image data, computed during WAKE period, presents a preponderance of neural interconnectivity between the thalamus and frontal cerebral cortex regions. Understanding the mechanisms of consciousness, including related regions of brain activation or inhibition, connectivity (coherence) and crucial functional aspects such as the *thalamus ("consciousness switch")* and *thalamus-cortical interrelationship* remain an important subjects for future research

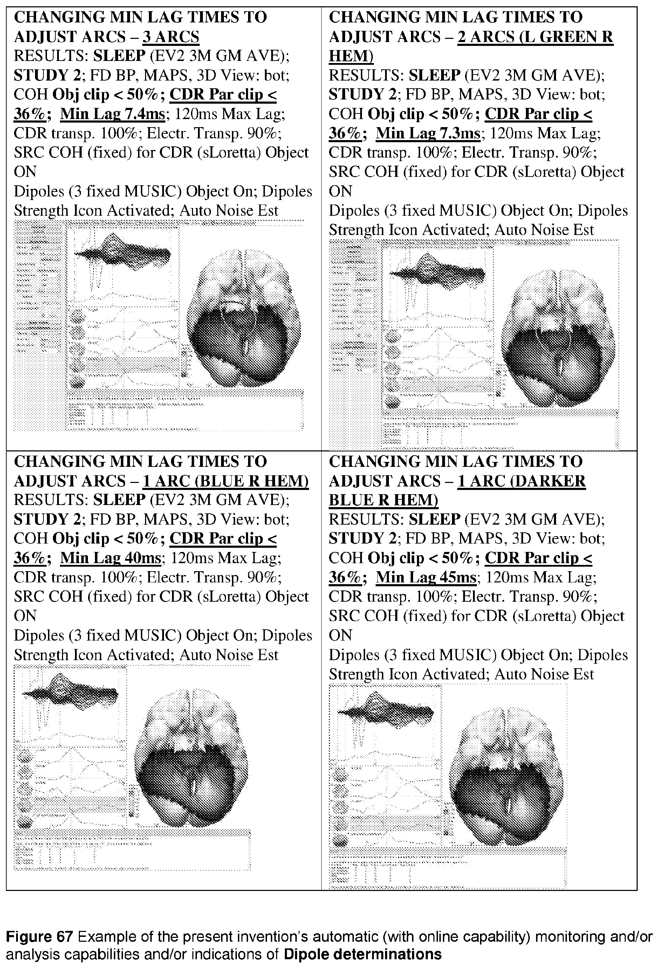

***Figure 67*** Example of the automatic (with online capability) monitoring and/or analysis capabilities and/or indications of Dipole determinations

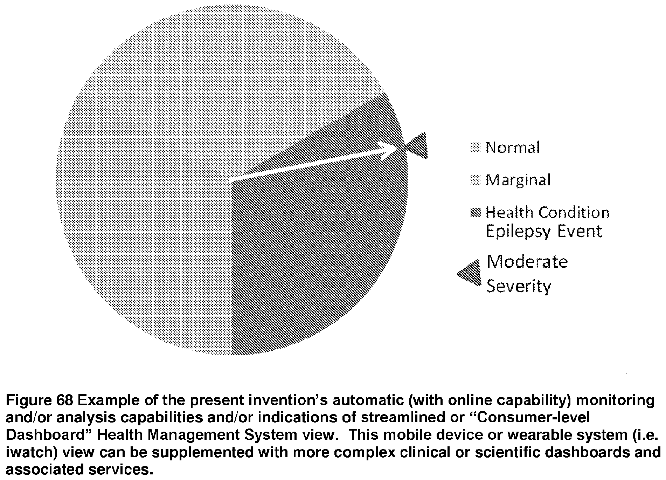

***Figure 68*** Example of the automatic (with online capability) monitoring and/or analysis capabilities and/or indications of streamlined or "Consumer-level Dashboard" Health Management System view. This mobile device or wearable system (i.e. iwatch) view can be supplemented with more complex clinical or scientific dashboards and associated services.

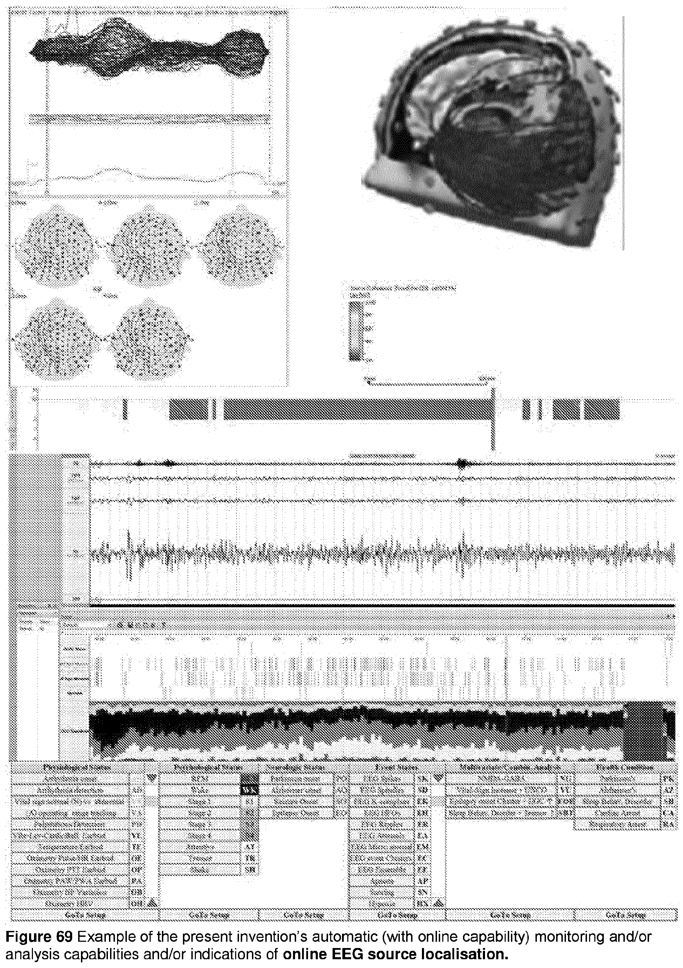

***Figure 69*** **Example of the automatic (with online capability) monitoring and/or analysis capabilities and/or indications of** online **EEG** source localisation.
**>>> online EEG source localisation includes coherence interconnectivity arcs (top right), EEG evoked responses (top left), EEG topographic maps (top left below averaged responses), sleep stage hypnogram (below topographic maps), EEG traces (below hypnogram), events including Deltawave, left eye movements, right eye movements, spindles, EEG frequency spectrum (can also include respiratory events, along with active events, event prediction, cluster analysis definitions, multivariate analysis detection criteria, and associated psychological, neurological and physiological conditions and states. The convergence of brain localisation, sleep monitoring and respiratory event detection enable unique views capable of revealing important associations such as sleep disordered breathing generated spatial dynamic cognitive disruptions (i.e. EEG arousals) relevant to early behaviour development (for example only).**

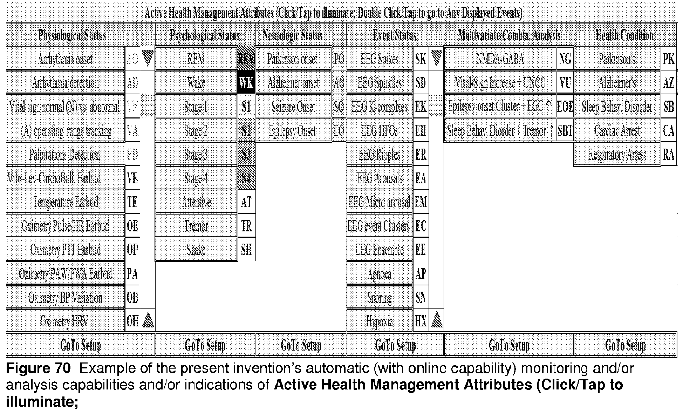

***Figure 70*** **Example of the automatic (with online capability) monitoring and/or analysis capabilities and/or indications of** Active Health Management Attributes (Click/Tap to illuminate;
**>>>Double Click/Tap to go to Any Displayed Events) and associated parameter configuration. >>Configuration attributed include any of or any combination of Physiological Status, Psychological Status, Neurologic Status, Event Status, Multivariate/Combinational Analysis and Health Condition markers. Per example configuration table here online/real-time monitoring can be configured for detection of a range of physiological, psychological, physiological health states or conditions applicable to: 1) physiological condition (i.e. epilepsy, Parkinson's Alzheimer's, autism etc.); 2) psychological state (sleep/wake; unconsciousness/consciousness depth, attention level, state high frequency, consciousness state); 3) event types (i.e. spindle, K-complex, arousal, micro-arousal, high frequency oscillation (HF), high frequency ripples, high frequency type A, high frequency type B, etc.), time context (i.e. early onset, late onset, actual incident); (temp -overview of figure layout- spindle (LH functional data), 3 D cut rotating image (RH image display) plus polysomnography or EEG display with colour coded conditions (i.e. epilepsy onset cluster indications via red markers; onset Parkinson's onset cluster via orange markers etc.) and codes for spindle, K-complex, arousal); psychological states for sleep/wake/consciousness depth; attentions-MMN etc.)**

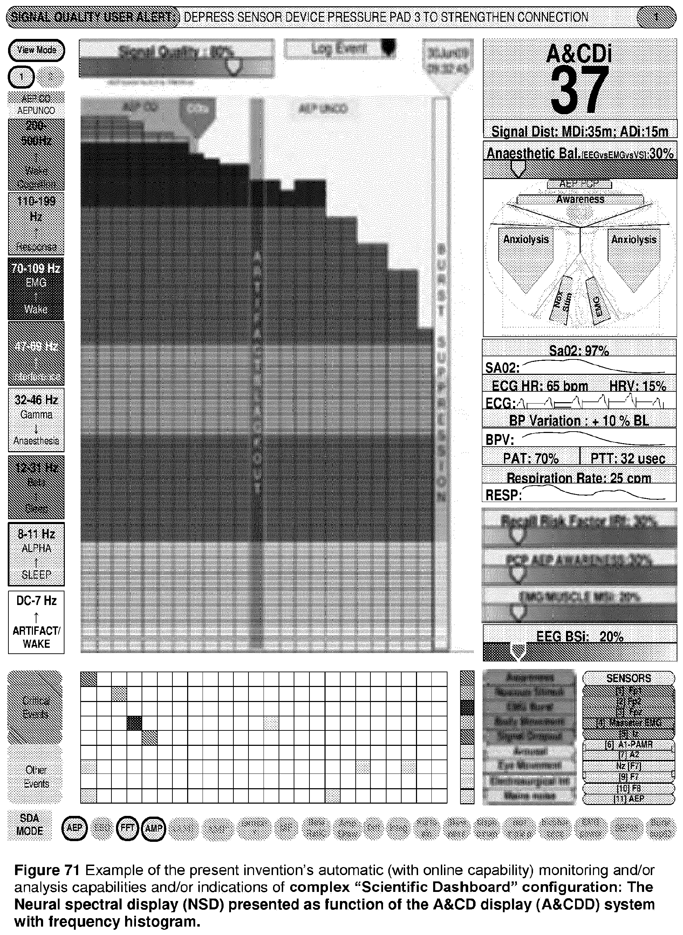

***Figure 71*** Example of the automatic (with online capability) monitoring and/or analysis capabilities and/or indications of complex "Scientific Dashboard" configuration: The Neural spectral display (NSD) presented as function of the A&CD display (A&CDD) system with frequency histogram.

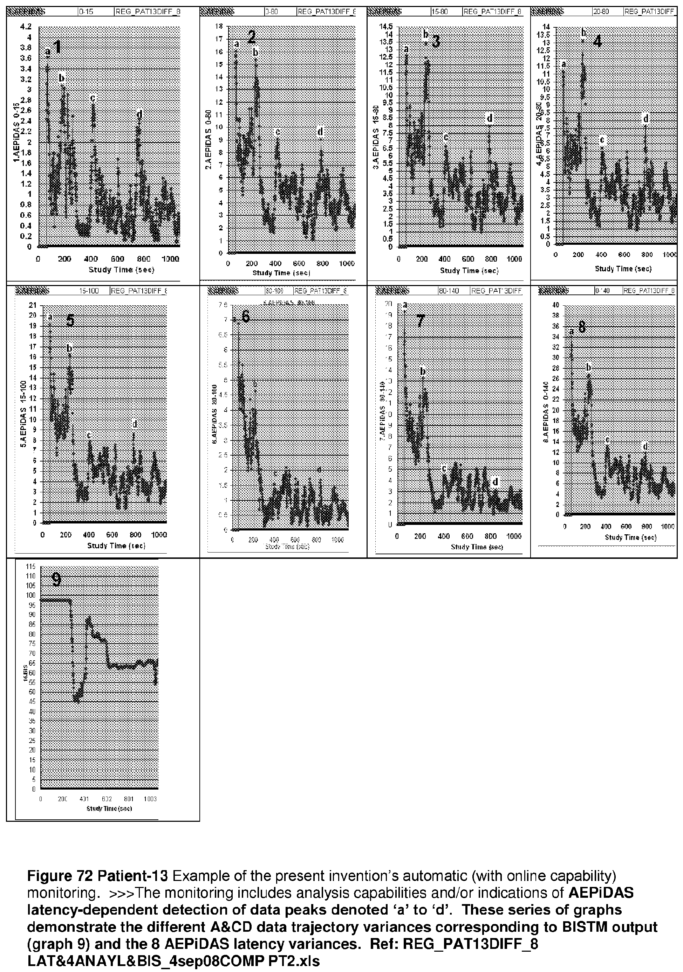

***Figure 72*** Patient-13 Example of the automatic (with online capability) monitoring. >>>The monitoring includes analysis capabilities and/or indications of AEPiDAS latency-dependent detection of data peaks denoted 'a' to 'd'. These series of graphs demonstrate the different A&CD data trajectory variances corresponding to BISTM output (graph 9) and the 8 AEPiDAS latency variances. Ref: REG_PAT13DIFF_8 LAT&4ANAYL&BIS_4sep08COMP PT2.xls

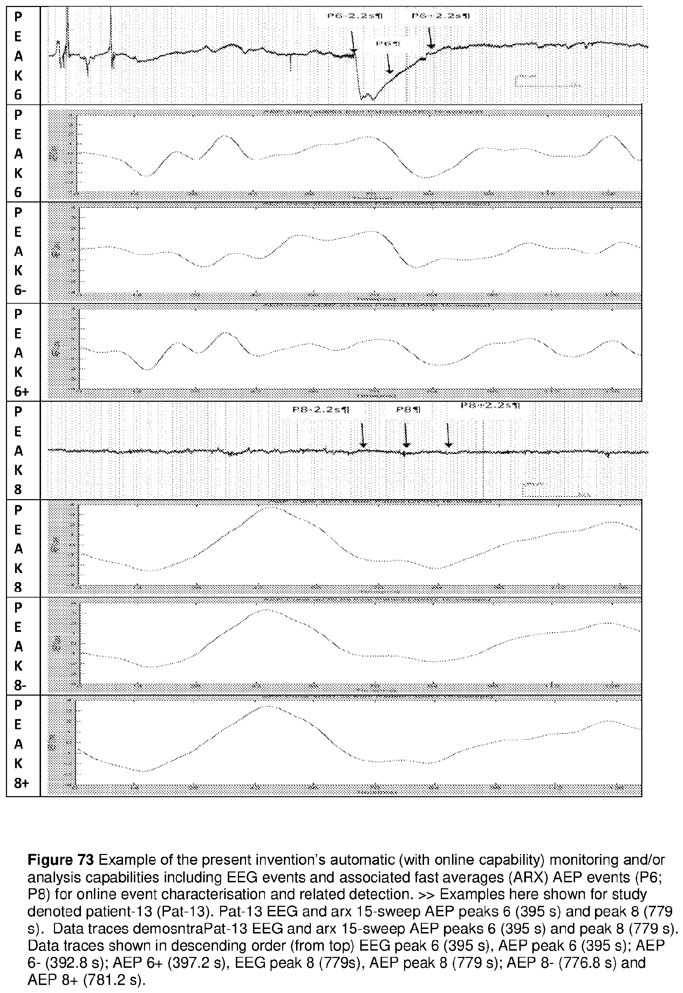

***Figure 73*** Example of the automatic (with online capability) monitoring and/or analysis capabilities including **EEG** events and associated fast averages (ARX) AEP events (P6; P8) for online event characterisation and related detection. >> Examples here shown for study denoted patient-13 (Pat-13). Pat-13 **EEG** and arx 15-sweep AEP peaks 6 (395 s) and peak 8 (779 s). Data traces demosntraPat-13 **EEG** and arx 15-sweep AEP peaks 6 (395 s) and peak 8 (779 s). Data traces shown in descending order (from top) **EEG** peak 6 (395 s), AEP peak 6 (395 s); AEP 6- (392.8 s); AEP 6+ (397.2 s), **EEG** peak 8 (779s), AEP peak 8 (779 s); AEP 8- (776.8 s) and AEP 8+ (781.2 s).

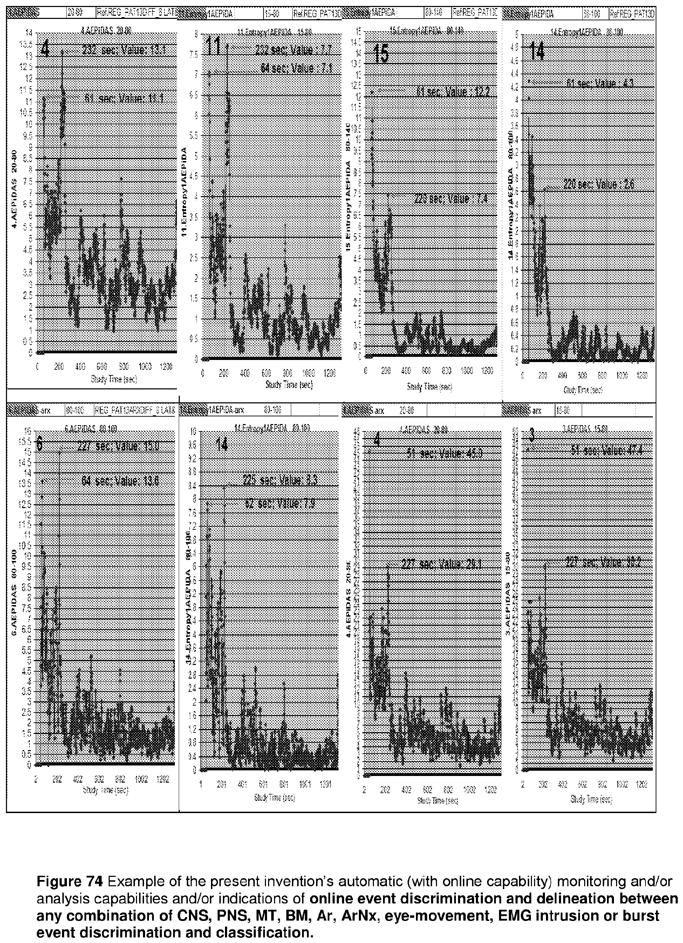

***Figure 74*** Example of the automatic (with online capability) monitoring and/or analysis capabilities and/or indications of online event discrimination and delineation between any combination of CNS, PNS, MT, BM, Ar, ArNx, eye-movement, EMG intrusion or burst event discrimination and classification.

***Figure 75*** Automatic online determination of events of interest including wake or sleep period biomarkers applicable to onset or incidence of neurological disorders such as Parkinson (including idiopathic SBD), epilepsy, Depression, Autism and/or ADHHD

***Figure 76*** eLifeMEDICS & eHealthMEDICS HEALTH MANAGEMENT SYSTEM (HMS). Highly secure and private data gateway with object layers capable of interfacing disparate information and monitoring systems while segregating clinical information and data from private information and encompassing private key and data segregation techniques designed to mitigate hacking or other security risk factors.

***Figure 77*** Health Management System (HMS) expert or artificial intelligence engine providing a means of continuous diagnostic and prognostic health tracking algorithm improvement

The **Inference Engine [D]** utilises the inputs from the **interface to the outside world [B]** to activate the relevant rules via the rule interpreter **[17].** The inference module can deploy data driven reasoning based on inputs **[B]** or forward "chaining", as well as the capability to provide a diagnosis or prognosis based on recognition of a specific or applicable group of symptoms (i.e. ***Figure 78*** [13]). Additionally, the present example incorporates a number of processes designed to resolve conflicts in order to determine the appropriate inference rules **[D].** Examples of these rules include prioritisations or rule, context limitations, meta-rules, "first-come first-served basis", timing context of information, generality ordering and Buggins' Turn or "round robin". The knowledge base **[B]** contains a range of simple to complex rules, along with sequences, facts, structured entities, attributes of such entities, attributes of these entities, relationships between entities, inferred facts, uncertainties (in rules), uncertain evidence, vague rules, and uncertainty between evidence and conclusion.

**The explanation module [14]** allows a user (human) readable form of the rules activated, the applicable conflict resolution, along with the selected rule which is subsequently generated, explaining the determination of the solution.

By way of this process of artificial intelligence or expert system self-learning information accumulation, and associated improvement of monitoring and ultimate knowledge base ***Figure 77*** **[A] Knowledge Base** (i.e. information accumulated based on the evaluation of inputs from the **Interface to the Real World [8]** and the associated of the rule interpreter which is based on standardised scoring rules applicable to disease, disorders or ***health conditions of interest) the prognostic and diagnostic accuracy continues to improve and "fine-tune" with continued usage.***

***Figure*** 78 Prognostic and Diagnostic Knowledge Data Bases [6;7;8;9;10;11;12] based on translation [2;5] and aggregation to system intelligence via internet/web-linked [3] expert and subject/patient applicable information sources [1;4].

***Figure* 79** Health Management System (HMS) Information to Knowledge Transformation Method
***Figure 80*** Personalised subject/patient- health management system (HMS) [1] incorporating any of or any combination of monitoring goals.
**>>>Monitoring goals include personalised subject/patient- health management system (HMS) [1] with any o for combination of monitoring goals [2], data-acquisition monitoring [3], artefact obliteration [4], dimensionality minimisation [5], monitoring sensor(s) dimensionality minimisation [6], monitoring device(s) dimensionality minimisation [7], up to date range of analysis variants [7], analysis classification [8], broad-based analysis variants determination [9], superset analysis variants determination [10], sub-set monitoring analysis variants determination [11], up to date range of analysis variants [12], statistical performance evaluation of complex to simplest monitoring algorithms models [13], expert system and/or artificial intelligence determination 14].**

***Figure 81*** Overview of ehealth (eLife) platform comprising of unique mobile devices, related applications, services and system control factors.
***Figure 82*** Integrated sensor attachment (ISA) with signal quality indicators and incorporating option of concertina expansion system ¹¹
***Figure 83*** Automatic mode determination (AMD) and the integration of affiliated sensor (ISA), patient interface (WEM; CEM; UIM) and signal quality management (SQE; SQI&C), and automatic identification and characterisation (AICC) systems 1;16

### Title: eLifeNEURO / EPILEPSY

### Background

**Ideally, sufferers of disorders or diseases** should be able to benefit from **early onset warning, notification,** early event or condition prediction, for early and remote intervention where required, online-remote or local sensor and/or monitoring system and/or signal conditioning intervention, biofeedback therapeutic control, guidance and/or automatically tailored medication and/or dosage and/or **drug-delivery** and/or drug dispensation, online intervention, prediction and warning an individual relating to events of interest include single events and/or ensemble(s) (cluster(s)) of **biomarkers** of interest and/or ***"health conditions*** or symptoms ***of interest"*** or "events of interest/EOI" (per "EOI DEFINITION" detailed in abbreviation table at rear of this document).

**Typically with prior state of the art ,** epilepsy sufferers require **clinical diagnosis** which can involve complicated and sophisticated monitoring for one or more days in order to enable medical specialists to diagnose the presence and if possible the particular source or type of epilepsy. Due to the expense and the sophistication of epilepsy monitoring and **source localisation systems,** comprehensive diagnosis is challenging and not freely accessible or affordable to all epilepsy sufferers. Furthermore, neurological, nervous, or movement "disorders" such as Epilepsy, Parkinson's, Alzheimer's, Autism, Depression often occur infrequently and spontaneously, so that a means of enabling individuals a consumer-based monitoring with diagnostic clinical outcomes and professional oversight or intervention can be the difference between sufferers of disorders being appropriately diagnosed and treated versus an ongoing lack of diagnosis or misdiagnosis.

***Traditional health and fitness tracking system*** or even professional health or scientific monitoring systems fail to enable simple but effective and sophisticated monitoring and health management of complex physiological aspects associated with improved health management in general but in particular the capability of early treatment intervention and early onset detection of conditions leading to more deleterious, severe or even potentially fatal health conditions. Moreover, previous state of the art fail to detect more complex phenomenon such as physiological aura.

**The present** example **enables early and effective diagnosis of these said "disorders"** can result in early medication or therapeutic intervention which can **slow-down and in some cases even avert more severe onset.**

The **present** example through its consumer-level affordability and professional **intervention online** information oversight and accessibility can enable longer-term heath studies within a subject's home or normal environment in a manner that otherwise would be too prohibitive cost-wise or impractical for many sufferers of disorders. The trending capabilities enable longer term study data to be analysed for more **subtle trends** indicative of disease onset that may otherwise not be possible.

The integrated **"opt-in"** function enables means to secure data (i.e. comprising of means to "opt" or establish levels of security (i.e. any of or any combination of encryption, biometrics, military level security, civilian level security; consumer level security; professional medical level security; etc.) along with associated privacy constraints along with accessibility rights of individual attempting to access any other health or personal data, while maintaining an individual's privacy and security rights right to **nominate** or **authorise** access by their selected professional health carers. In the way access to expert health care can be expedited and also improved in terms of efficiency and associated affordability.
- The present example further incorporates or enables a means of selecting user-access complexity level versus security and privacy level versus ***automatic recommended levels for various predefined*** roles or certified trust levels of certain individuals or organisations.

In terms of the **therapeutic application, more severe onset of disorders can slowed down and event averted** in certain cases. Such **therapeutic application** can include said monitoring linked to associated **biofeedback. Biofeedback** can comprise of surgical solutions such as brain stimulation. For example, onset-prediction of neurological, nervous system, sleep, or movement disorders could activate automatic formulation or rate of medication. Alternatively, automatic control of medication dispenser systems could be controlled in a manner where an individual is prompted and automatically issued the appropriate medication at any point via control of a medication dispensation system.
- **The present example further incorporates neurological therapy biofeedback; characterising and fine tuning medical condition determination;**
- The present example further incorporates or enables **therapeutic** intervention, whereby **neurological disorders** can be treated by way of biofeedback via neurological monitoring of an individual directly influencing any of or any combination of location, strength, duration, formulation of magnetic signal delivered to the said individual.

In terms of the **therapeutic** intervention, **neurological disorders** can be treated by way of biofeedback via neurological monitoring of an individual directly influencing any of or any combination of location, strength, duration, formulation of electrical stimulation which can be applied to subject via surgically implanted or surface applied electrical stimulation electrodes. In this way the severity, duration and number of incidents (such as epileptic seizures or Parkinson's shaking or uncontrolled movements) could be reduced and even averted. Moreover, the present example brings a consumer application level of access and affordability to everyday lives that otherwise could be stigmatised, lived in elevated fear, or otherwise result in degradation of the quality, safety and overall normality of life.

The **ability to more effectively characterise and manage** an individual's disorder based on a daily and nightly monitoring and professional **medical oversight and intervention** can provide a means for **progressively fine-tuning patient-specific monitoring** and **analysis parameters,** which in turn **progressively increases** the **early warning and therapeutic options** for an individual until their level of **health-management ultimately** approaches or even **exceeds that of a normal person.** The said **physiological monitoring** capability can involve one or more channels of **EEG** monitoring with associated analysis.

**The present example incorporates or enables communication interface (including one or more wearable devices, WAN, LAN, IP, NAS, and/or Cloud-computing services) with any of or any combination of the following disorder/health condition prediction, onset or incident characterisation and determination processing capabilities, further outlined here:**
- -Whereby said "monitoring and/or diagnostic and/or therapeutic system" includes any patient wearable system or device;
- -Whereby said "patient wearable system or device" include a single or plurality of any combination of :
   - **forehead mounted and/or attached devices or systems;**
   - wrist mounted and/or attached devices or systems;
   - chest mounted and/or attached devices or systems;
   - body mounted and/or attached devices or systems;
   - limb mounted and/or attached devices or systems;
   - head mounted and/or attached devices or systems;
   - neurology disorder precursor algorithm
   - -seizure forecasting algorithm;
   - **Seizure spectral precursors with option of clusters analysis of events of interest (combinations of spectral bands), and/or**
   - **Seizure precursor detection via clusters of events (combinations of spectral bands).** 41

**The present example further incorporates or enables communication interface (including one or more wearable devices, WAN, LAN, IP, NAS, and/or Cloud-computing services) with the capability of automatic determination of the onset or incidence of health conditions/disorders or events of interest by way of incorporating characterisation and determination processing capabilities HFO, further outlined here:**
- The present example further incorporates or enables **automatic characterisation** with the option of **online capabilities.** These **characterisation and detection** capabilities include high frequency oscillations (HFO) using criteria which can be predetermined, automatically adapted via artificial intelligence or other means, based on criteria that typically can comprise of HFO amplitude (i.e. 8-1200µV), duration (i.e. 10-200ms), cluster of a minimum number of sequential HFO events (i.e. 1 - 10) with minimal inter-event interval (i.e. 10 - 50ms)^{42, 42-45}.

**The present example further incorporates or enables communication interface (including one or more wearable devices, WAN, LAN, IP, NAS, and/or Cloud-computing services) with the capability of automatic determination of the onset or incidence of health conditions/disorders or events of interest by way of incorporating characterisation and determination processing capabilities such as automatic characterisation; automatic algorithm adaptation based on expanded data analysis outcomes associated with seizure and/or other analysis of disorder precursors, further outlined here:**
- The present example further incorporates or enables automatic online analysis comprising any of or any combination of:
   - prediction of seizure onset by way of characterising commonly occurring biomarkers, such as spectral events, from previously recorded seizure events;
   - statistically analysing these "biomarkers in a manner in order to progressively improve the seizure prediction capability and early warning detection with increased statistical probability power, as prediction capability increases with determination of said seizure precursor biomarkers, and/or
   - incorporation of said automatic online processes applicable to neurological, nervous system disorders or other adverse health sequelae.

**The present example further incorporates or enables communication interface (including one or more wearable devices, WAN, LAN, IP, NAS, and/or Cloud-computing services) with the capability of automatic determination of the onset or incidence of health conditions/disorders or events of interest by way of incorporating characterisation and determination processing capabilities such as spectral events of interest; algorithm training capabilities (based on cumulative event detection and associated precursor analysis) and/or clusters or events of interest as further outlined here:**
- The present example further incorporates or enables **online monitoring** of one or more neurology signals, combined with automatic online processing capable of detecting seizure precursors via singular or clusters of "spectral events of interest";
- whereby said monitoring can incorporate (but is not limited to) online monitoring via a wearable device
- whereby said **spectral events of interest** include (but are not limited to) **any combinations of spectral bands** including (but not limited to) any of or any combination of **delta/slow wave** (0.1-4 Hz), **theta** (4-8 Hz), **alpha** (8-12 Hz), **beta** (12-30 Hz), **low-gamma** (30-70 Hz), and/or **high-gamma** (70-180 Hz);
   logistic regression classifiers can be trained to discriminate labelled ***preictal*** or ***interictal or postictal or ictal epileptiform discharges (IEDs)*** data events or segments using any combination of said combinations of the band spectral power features;
- Additionally classifiers can trained for any combination or biomarkers or clusters including spectral bands, HFOs, ripples, and other spectral bands analysis characterisation and determination;
- Whereby said "spectral events of interest" include (but are not limited to) any combinations of the band spectral power features;
   seizure spectral cluster precursors
- means of "defining" prior to online monitoring session or dynamically during monitoring session seizure clusters;
- means of detecting and/or monitoring and/or tracking and/or recording, and/or reviewing clusters of said "events of interest" by way of defining properties of "events of interest in accordance to cluster analysis
- whereby cluster analysis properties can be (but are not limited to) definitions or properties learned or adapted during or prior to online monitoring;
- means of detecting and/or monitoring and/or tracking and/or recording, and/or reviewing clusters of said "events of interest" by way of defining properties of "events of interest in accordance to cluster analysis
- whereby cluster analysis properties can be (but are not limited to) definitions or properties previously defined prior to online monitoring;
- means of detecting and/or monitoring and/or tracking and/or recording, and/or reviewing clusters of said "events of interest" by way of defining properties of "events of interest in accordance to cluster analysis
- whereby cluster analysis properties can be (but are not limited to) definitions or properties learned or adapted during or prior to online monitoring;
- means of detecting and/or monitoring and/or tracking and/or recording, and/or reviewing clusters of said "events of interest" by way of defining properties of "events of interest in accordance to cluster analysis
- whereby cluster analysis properties can be (but are not limited to) definitions or properties previously defined via previous diagnostics studies or related data analysis;
- whereby seizure precursor detection via clusters of events (combinations of spectral bands). ⁴¹
- **The present example further incorporates or enables communication interface (including one or more wearable devices, WAN, LAN, IP, NAS, and/or Cloud-computing services) with the capability of automatic determination of the onset or incidence of health conditions/disorders or events of interest by way of incorporating characterisation and determination processing capabilities such as spectral; detection of events of interest including HFO; ripples; morphologic pattern determination; non-parametric statistical based determination; mixed events; ensembles or cluster analysis; spatial dynamic signal characterisation; k-complexes, and/or spindles, as further outlined here:**
   - The present example further incorporates or enables online "monitoring" of one or more "spectral events of interest" and/or "events of interest" and/or "biomarkers" and/or "neurology" signals, combined with automatic online processing capable of detecting seizure precursors via singular or clusters of "spectral events of interest" related to neurological or nervous system disorders such as (but not limited to) Parkinson's, epilepsy or seizures in general (generic);
   - Whereby said **"spectral events of interest"** can include (but are not limited to) any combination of **high-frequency** (in or nearby spectral regions of 100-500 Hz) **oscillations** (HFOs), **ripples** (in or nearby spectral regions of 100-250 Hz) and/or **fast ripples** (in or nearby spectral regions of 250-500 Hz);
   - Whereby said "events of interest" can include (but are not limited to) mixed events comprising any combination or sequences of **high-frequency** (in or nearby spectral regions of 100-500 Hz) **oscillations** (HFOs), **ripples** (in or nearby spectral regions of 100-250 Hz) and/or **fast ripples** (in or nearby spectral regions of 250-500 Hz);
   - Whereby said "events of interest" can include (but are not limited to) any combination of **HFOs** characterized by extracting **morphologic features;**
   - Whereby said "events of interest" can include (but are not limited to) any combination of **HFOs** characterized by extracting **quantitative morphologic features;**
   - Whereby said "events of interest" can include (but are not limited to) mixed events comprising any combination or sequences of high-frequency deployment of supervised classification and **nonparametric statistical tests** can be undertaken in order to examine quantitative changes associated with quantitative changes applicable or probable as it relates to precursors and/or the incidence of (but not limited to) seizures or epilepsy;
   - Whereby said "events of interest" can include (but are not limited to) HFO morphology linked to fluctuation in the relative rates of **ripples, fast ripples, and mixed** (ripples, fast ripples) frequency events, and/or
   - Examination of changes in HFO signal morphology or fluctuations in neurology signal associated with ripples, fast ripples, and mixed frequency events;
   - whereby said monitoring can incorporate (but is not limited to) online monitoring via a wearable device;
   46
- **The present example further incorporates or enables communication interface (including one or more wearable devices, WAN, LAN, IP, NAS, and/or Cloud-computing services) with the capability of automatic determination of the onset or incidence of health conditions/disorders or events of interest by way of incorporating characterisation and determination processing capabilities such as spectral events of interest; training classifiers; clustering analysis enabling the determination of ensembles of events of interest; clusters or groups ERs and/or SRs; high frequency oscillations; Combinational/multivariate analysis further outlined here:**
   - disease/disorder precursor characterisation and determination including (but not limited to) **clustering analysis** whereby predetermined or dynamically determined **ensembles or groups ER and/or SR** and/or other brain activation outcomes or "events of interest" can be examined in the context of a group of changes and/or brain activation and/or brain deactivation based on "any combination of analysis outcomes based on examining" **spatial dynamic properties** and/or spatial and/or temporal properties and/or NLDA properties ⁴⁷
   - Including but not limited to any type or variants of entropy and/or **complexity analysis)** ^{15, 32} and/or sleep events (such as any combination of **spindles, k-Complexes, arousals, micro-arousals, high frequency oscillations (HFOs)** ⁴⁸, **high frequency ripples, and or other events including (but not limited to) events, physiologic conditions or events, "predefined (or dynamic determination of) brain regions of interest" and/or psychological states, conditions or events per examples (but not limited to) attributes, parameters and measures presented in any of or any combination of** **Figure 57****;** **Figure 58****;** **Figure 59****;** **Figure 60****;** **Figure 61; Figure 62****;** **Figure 63; Figure 64****;** **Figure 65; Figure 66****;** **Figure 67****;** **Figure 68****;** **Figure 69****;** **Figure 70****;** **Figure 71****;** **Figure 72****;** **Figure 73****;** **Figure 74****;** **Figure 75****;****Figure 76****;** **Figure 77****;** ***Figure 78*****;** **Figure 79****;** **Figure 80****;** **Figure 81; Figure 82****;** **Figure 83****;**

### High frequency oscillations

- Whereby said **"high frequency oscillations"** or **"low frequency activity"** can include (but are not limited to) frequency regions or approximate frequency regions of any combinations of :
- Sustained, repetitive transient increases in high **gamma** (in or nearby spectral regions of 80-150 Hz) amplitude,
- Sustained, repetitive transient increases in high **gamma** (in or nearby spectral regions of 80-150 Hz) amplitude **phase-locked** to the **low-frequency** (in or nearby spectral regions of 1-25 Hz) ictal rhythm,
- multi-unit firing bursts synchronized across the core territory of the seizure;
- sustained high frequency oscillations (HFO) incorporating coherence or **cross-frequency between high gamma amplitude** and low-frequencies (in or nearby spectral regions of 1-25 Hz);
- low-level, **heterogeneous neural** firing correlated with a **lack** of high **frequency oscillations** in **adjacent monitoring regions;**
49

### Combinational/multivariate analysis

- **Whereby said** "any combination of analysis outcomes based on examining" includes (but is not limited to) any combinations or **multivariate** analysis incorporating combinations of analysis types comprising any combination of **source localisation** processing techniques or **time-series** processing techniques including in general (but not limited to) properties such as **linear versus non-linear, stationary versus non-stationary, and random versus deterministic.**
- **Whereby said** "events of interest" can include" (but are not limited to) automatic online and/or real-time detection or determination of precursors of seizures including epileptic seizures;
- a) Said **"Automatic online** diagnosis including combinational analysis" and/or neurology disorder and/or event and/or seizure occurrence or associated onset-prediction comprising any combination of :
- i) **Random versus deterministic, stationary versus non-stationary and linear versus non-linear signal properties ⁵⁰,** signal to noise, dynamical, and/or spatial characteristics of any single or multiple ensembles of time series;

- **The present example further incorporates or enables communication interface (including one or more wearable devices, WAN, LAN, IP, NAS, and/or Cloud-computing services) with the capability of automatic determination of the onset or incidence of health conditions/disorders or events of interest by way of incorporating characterisation and determination processing capabilities such as artefact reduction or elimination aided by adaptive filtering or stationary wavelet transform filtering; Lyspunov exponent and/or discrete wavelet packet and/or distinguishing feature set(s); non-linear dynamic (i.e. any of or any combination of Shannon's/wavelet/complexity/entropy transforms); discrete wavelet and/or assisted by pre-diagnostic studies and/or support vector machines (SVM) classification and/or epileptic seizure probability; SOZ characterisation and determination; fast ripples; linear regression analysis; high frequency oscillation; high frequency neural activity; artificial learning analysis; gamma bands analysis; along with associated systematic groups/ cluster characterisation and determination and/or associated multivariate analysis further outlined here:**
   - The present example further incorporates or enables online, automatic, patient wearable device incorporating the capability of any combination of (generic object intro):
   - Means of detecting and/or reducing and/or **eliminating artifact** and other unwanted signals and background noise;
   - Means of detecting and/or reducing and/or **eliminating artifact** and other unwanted signals and background noise, whereby said means includes (but is not limited to **adaptive filtering;**
   - Means of detecting and/or reducing and/or eliminating artifact and other unwanted signals and background noise, whereby said means includes (but is not limited to **stationary wavelet transform filtering;**
   - Means of incorporating supplementary wireless network processing capability to supplement on-board device processing capabilities;
   - Means of determination of control and/or control and /or therapeutic function associated with **biofeedback brain stimulation;**
   - Whereby said **"Biomarker detection"** includes (but is not limited to) the detection of any combination of **physiological** disorder/condition/state/status and/or **neurological** disorder/condition/state/status and/or **psychological** disorder/condition/state/status and/or detecting **precursor** to **epileptic seizures** in intracranial (i**EEG**) or electroencephalogram **(EEG)** (generic biomarker precursor or event);
   - Whereby said **"Biomarker detection"** detection can include (but is not limited to) **"Lyspunov exponent"** including but not limited to largest or larger of such components of a **discrete wavelet packet** transform derived based on the **EEG** signal segments,
   - Whereby said **"Lyspunov exponent"** can include (but is not limited to) largest or larger of such components" of a **discrete wavelet** packet transform derived based on the **EEG** signal segments and whereby said "components" can be (but is not limited to) being defined as a **distinguishing "feature set(s)";**
   - Whereby the said "feature set(s)" can be further processed by **support vector machines** (SVM) classification, in order to identify whether the segment under analysis is "a probable epilepsy or seizure precursor";
   - Whereby the said **"feature set(s)"** classified as being associated with "a probable epileptic seizure precursor" can be further processed in the context of the consecutive and/or **clustering nature** of one of more seizure precursor events as part of final determination of valid seizure precursor detection status;
   - Whereby said **"Biomarker detection"** detection can include (but is not limited to) "non-linear dynamic" processed period or event including but not limited to largest or larger of such components of a discrete wavelet packet transform derived based on the **EEG** signal periods or segments;
   - Whereby said "non-linear **dynamic"** can include (but is not limited to) largest or larger of such components of a discrete wavelet packet transform derived based on the **EEG** signal segments and whereby said "components" can be (but is not limited to) being defined a distinguishing "feature set(s)";
   - Whereby said "Biomarker" or "event(s) of interest" can include (but is not limited to) **complexity** analysis;
   - Whereby said "Biomarker" or "event(s) of interest" can include (but is not limited to) **Shannon** wavelet entropy;
   - Whereby said "Biomarker" or "event(s) of interest" can include (but is not limited to) **discrete wavelet** packet transform;
   - Additionally, said onset prediction can be **assisted by pre-diagnostic studies** designed to characterise each specific patient in terms of analyses types listed here or any combination of analyses listed in document herein.
   - Whereby the said "feature set(s)" can be further processed by support vector machines **(SVM) classification,** in order to identify whether the segment under analysis is **"a probable epileptic seizure precursor";**
   - Whereby the said **"feature set(s)"** classified as being associated with "a probable epileptic seizure precursor" can be further processed in the context of the consecutive and/or clustering nature of one of more seizure precursor events as part of final determination of "valid seizure or epilepsy precursor detection status";
   - Whereby **combinational or multivariate** analysis can combine the outcomes of a said "probable epileptic seizure precursor(s)" in order to determine greater accuracy in the determination of final or more advanced analysis of "valid seizure or epilepsy precursor detection status";
   - Whereby **combinational or multivariate analysis** can combine the outcomes of a said "probable epileptic seizure precursor(s)" and/or other analysis types presented herein (body; abstract; drawings; field; etc. ) in order to determine greater accuracy in the determination of final or more advanced analysis of "valid seizure or epilepsy precursor detection status" (generic combinational/multivariate patent-wide seizure or seizure precursor analysis);
   - Whereby **"combinational or multivariate analysis"** can combine the outcomes of a said "probable epileptic seizure precursor(s)" and/or other analysis types presented herein (body; abstract; drawings; field; etc. ) in order to determine greater accuracy in the determination of final or more advanced analysis of "valid seizure or epilepsy precursor detection status" (generic combinational/multivariate patent-wide seizure or seizure precursor analysis);
      ⁴⁷;⁵¹
   - -Whereby said **"combinational or multivariate analysis** can "Comprise of determination and decision/detection process incorporating any combination of **EEG** and/or other neurological signals including (but not limited to high frequency oscillations (in or nearby spectral regions of 80-500 Hz)
      51
   - ; determination of **seizure-onset zone** (SOZ) ; **ripples** (in or nearby spectral regions of 80-250 Hz); **fast ripples** (in or nearby spectral regions of 250-500 Hz); **Linear regression analysis** fitted to the power trends corresponding to any number or period of intervals including (but not limited to) 1, 5, and 15 min periods prior to seizure onset was determination; **High-frequency oscillations** (HFOs) referred to as ripples (in or nearby spectral regions of 80-250 Hz); or so-called **fast ripples** (in or nearby spectral regions of 250-500 Hz); determination of frequency (i.e. rate, incidence, clustering) of said **high frequency ripples** or **high frequency oscillations; delineation between clustering or systematically grouped** (clustered) or related events such as any combination of HFOs, **ripples, spikes and/or other neurological events** versus mainly **randomised occurrences,** grouped determination of frequency (i.e. rate, incidence, clustering, distribution and/or frequency or events) of said **high frequency ripples** or **high frequency oscillations;**
   - Whereby monitoring **EEG** must be sample high enough and filtered with adequate spectral bandwidth to limit the attenuation of said any **combination of HFOs, ripples, spikes and/or other neurological events** of interest;
   - -Whereby monitoring **EEG** must be sampled at a sample rate at least twice the upper frequency of interest;
      51
      52
   - -Whereby said **"events of interest"** or **"biomarkers"** include (but are not limited to) **high frequency neural activity** (in or nearby spectral regions of 40 Hz and 150 Hz);⁵²
   - -Whereby said "events of interest" or "biomarkers" include (but are not limited to) any online or dynamically determined and/or predefined (i.e. including but not limited to learning or artificial analysis techniques from prior or ongoing monitoring) and/or any combinations of anatomical **EEG** or other neurological signal properties;⁵²
   - -Whereby said "events of interest" or "biomarkers" include (but are not limited to. any online or **dynamically** determined and/or predefined (i.e. including but not limited to learning or **artificial learning analysis techniques** from prior or ongoing monitoring) any events or periods of interest related temporal **EEG** or other neurological signal properties;⁵²
   - -Whereby said "events of interest" or "biomarkers" include (but are not limited to. any online or **dynamically determined** and/or predefined (i.e. including but not limited to learning or artificial analysis techniques from prior or ongoing monitoring) any events or periods of interest related to **gamma-band** (in or nearby spectral regions of 40 Hz) **EEG** or other neurological signal properties ⁵²
   - Whereby said "Biomarker detection" includes (but is not limited to) the detection of any of or any combination of physiological disorder, condition, neurological disorder, state, status, psychological disorder, applicable to detecting the incidence of or precursors to epileptic seizures in intracranial (i**EEG**) or electroencephalogram (**EEG**) by way of **combinational** or **multivariate analysis** processes incorporating any combination of analyses per herein (generic combinational analysis biomarker precursor or event);
   ^{52, 53}
- **The present example further incorporates or enables communication interface (including one or more wearable devices, WAN, LAN, IP, NAS, and/or Cloud-computing services) with the capability of automatic determination of the onset or incidence of health conditions/disorders or events of interest by way of incorporating characterisation and determination processing capabilities such as tracking such brain activities with high spatiotemporal resolution; "events of interest" including hemodynamic responses with heterogeneous patterns; and associated multivariate and/or cluster analysis; EEG gamma power changes; Coupling or decoupling characteristics and determination between hemodynamic and neural activities; Bilateral cortex, nearby or related activity with hemodynamic changes region ;Thalamus nearby or related activity with hemodynamic changes region ;brain activation sequences; dipole activity and/or sequences coherence activity and/or sequences; Spatiotemporal dynamics of brain activity based on dynamical signal properties (linear & non-linear variations) along with associated cluster and multivariate analysis, further outlined here:**
   - The present example further incorporates or enables online "monitoring" of one or more "spectral events of interest" and/or "events of interest" and/or "biomarkers" and/or "neurology" signals, combined with automatic online processing capable of detecting seizure precursors via singular or clusters of "spectral events of interest" related to disorders such as (but not limited to) seizures or epilepsy and/or ***"health conditions*** or symptoms ***of interest** "* or "events of interest/EOI" (per "EOI DEFINITION" detailed in abbreviation table at rear of this document).

   - Monitoring via device recording continuous intracranial **EEG** (i**EEG**);
   - Monitoring noninvasively and continuously tracking such brain activities with high **spatiotemporal** resolution;
   - Whereby said "events of interest" can include (but are not limited to) hemodynamic responses with **heterogeneous** patterns;
   - Whereby said "events of interest" can include (but are not limited to) intracranial electroencephalogram **gamma** power changes;
   - Whereby said "events of interest" can include (but are not limited to) electroencephalogram or other neurology **gamma** power changes;
   - Whereby said "events of interest" can include (but are not limited to) the **decoupling** between local **hemodynamic** and neural activities;
   - Whereby said "events of interest" can include (but are not limited to) **hemodynamic** changes evolving from local regions of the **bilateral cortex;**
   - Whereby said "events of interest" can include (but are not limited to) **hemodynamic** changes evolving from local regions of the **thalamus;**
   - Whereby said "events of interest" can include (but are not limited to) **hemodynamic** changes evolving from local regions of the **thalamus** and **bilateral cortex** to the entire brain;
   - Whereby said "events of interest" can include (but are not limited to) **hemodynamic changes** evolving from any combination of local regions of the **thalamus, bilateral cortex** through to the **entire brain;**
   - Whereby said "events of interest" can include (but are not limited to) **hemodynamic changes** evolving from any regions of the **entire brain;**
   - Whereby said "events of interest" can include (but are not limited to) determination and/or detection of **"brain activation sequences"** applicable to the incidence or **onset of epilepsy and/or seizures in general;**
   - Whereby said "events of interest" can include (but are not limited to) **determination and/or detection** of **"brain activation sequences"** whereby said "brain activation sequences" can include (but are not limited to) **dipole activity;**
   - Whereby said "events of interest" can include (but are not limited to) determination and/or detection of **"brain activation sequences"** whereby said "brain activation sequences" can include (but are not limited to) **coherence** or associated **coherence interconnections;**
   - Whereby said "events of interest" "determination" can include (but are not limited to) **online dynamic determination** and/or prior analysis or previously monitored recordings;
   ⁵³
   ⁵⁴
- **The present example further incorporates or enables communication interface (including one or more wearable devices, WAN, LAN, IP, NAS, and/or Cloud-computing services) with the capability of automatic determination of the onset or incidence of health conditions/disorders or events of interest by way of incorporating characterisation and determination processing capabilities such as singular or clusters of spectral events of interest; slow modulation of high-frequency gamma activities; analysis of neural network dynamics; phase-coupling between any "events of interest", cross-frequency coupling between spectral bands; anatomical localization, spiking activity; event durations of HFOs, HFO rates; high frequency activity events or ensembles of events as a measure of cognition; gamma activity; high-frequency oscillations; pre-defined frequency band region's median amplitude band versus spectral power median frequency; relative power ratio of frequencies above a any nominated frequency and below any nominated frequency; spectral edge analysis; 95% quartile of the power spectrum and the relative power ratio of frequencies above 26 Hz and below 20 Hz; ripples, fast ripples, and mixed events; HFO clusters; tracking of early hemodynamic responses and/or other measures associated with heterogeneous patterns; decoupling between local hemodynamic and neural activities, and/or "physiological aura", along with associated cluster and multivariate analysis, further outlined here:**
   - The present example further incorporates or enables online "monitoring" and the "determination" of one or more "spectral events of interest" and/or "events of interest" and/or "biomarkers" and/or "neurology" signals, combined with automatic online processing capable of detecting seizure precursors via **singular or clusters** of **"spectral events of interest" related to** disorders such as (but not limited to) seizures or epilepsy and/or ***"health conditions*** or symptoms ***of interest"*** or "events of interest/EOI" (per "EOI DEFINITION" detailed in abbreviation table at rear of this document).

   - Whereby said "events of interest" "determination" can include (but are not limited to) measures of brain excitability based on the slow modulation of high-frequency **gamma** activities (in or nearby spectral regions of 40-140 Hz) in ensembles of **intracranial or EEG monitored contacts;**
   - Whereby said "events of interest" "determination" can include (but are not limited to) measure of **network dynamics;**
      54
   - Whereby said "events of interest" "determination" can include (but are not limited to) measures of brain excitability based on **80-150 Hz;**
   - Whereby said "events of interest" "determination" can include (but are not limited to) measures of brain excitability based on **1-125 Hz;**
   - Whereby said "events of interest" can include (but are not limited to) measures of brain excitability based on **40-140 Hz;**
   - Whereby said "events of interest" can include (but are not limited to) measures of **phase-coupling between any "events of interest"** and/or spectral events of interest" and/or biomarkers";
   - Whereby said "events of interest" can include (but are not limited to) measures of **cross-frequency coupling between spectral bands** listed herein phase coupling between any "events of interest" and/or spectral events of interest" and/or biomarkers";
   - Whereby said "events of interest" can include (but are not limited to) measures of **cross-frequency coupling between high gamma** amplitude and low-frequency brain signal spectral bands such as **1-25Hz** or around this spectral region;
   - Whereby said **"events of interest"** can include (but are not limited to) measures of any combination of **rates of HFOs** within and outside **spike events,** overlap between spike and HFOs events, event durations of spikes, **event durations of HFOs,** percentage of spikes incorporating HFOs calculated, **percentage of spikes** incorporating HFOs compared according to **anatomical localization, spiking activity,** and/or relationships to the **seizure onset zone;**
   - Whereby said "events of interest" can include (but are not limited to) measures of **HFO rates** as a measure of seizure onset prediction;
   - Whereby said "events of interest" can include (but are not limited to) measures of **high frequency activity events or ensembles of events as a measure of cognition;**
   - Whereby said "events of interest" can include (but are not limited to) measures of high frequency activity events or ensembles of events including activity between 40 Hz and 150 Hz;
   - Whereby said "events of interest" can include (but are not limited to) measures of high frequency activity including activity of high-frequency events or ensembles of events **gamma** (40-140 Hz);
   - Whereby said "events of interest" can include (but are not limited to) measures of **high-frequency oscillations** events or ensembles of events in region of 100-500 Hz or thereabouts;
   - Whereby said "events of interest" can include (but are not limited to) measures of high-frequency oscillations events or ensembles of events in region of 201 and 500 Hz; ⁵⁵
   - Whereby said "events of interest" can include (but are not limited to) measures of high-frequency oscillations events or ensembles of events in any **pre-defined frequency band region's median amplitude band versus spectral power median frequency** across any pre-defined frequency band;
   - Whereby said "events of interest" can include (but are not limited to) measures of high-frequency oscillations events or ensembles of events in region of 8-30 Hz median amplitude band versus spectral power median frequency;
   - Whereby said "events of interest" can include (but are not limited to) measures of ratio of Beta frequency range power versus overall power of broader frequency band;
   - Whereby said "events of interest" can include (but are not limited to) measures of high-frequency oscillations events or ensembles of events in region of 201 and 500 Hz as a marker of cognitive function; ⁵⁵
   - Whereby said "events of interest" can include (but are not limited to) measures of spectral based analysis techniques including percentage of the quartile of the power spectrum and the **relative power ratio of frequencies above a any nominated frequency and below any nominated frequency:** ⁵⁵
   - Whereby said "events of interest" can include (but are not limited to) measures of spectral based analysis techniques including, **spectral edge analysis, 95% quartile** of the **power spectrum** and the **relative power ratio of frequencies above 26 Hz and below 20 Hz;** ⁵⁵
   - Whereby said "events of interest" can include (but are not limited to) measures of series, sequences, clusters or groups of HFO cluster types (such as but not limited to **(ripples, fast ripples, and mixed events)** can be characterised, detected and classified in accordance to time epochs applicable to any combination of interictal (between seizures), preictal (prior to seizure), ictal (during seizure), and/or postictal (after seizure);
   - Whereby said "events of interest" can include (but are not limited to) measures of series, sequences or groups of **HFO cluster** types (such as but not limited to ripples, fast ripples, and mixed events) can be characterised, detected and classified in accordance to time epochs applicable to any combination of rates and/or series, sequences, clusters or groups of HFO and/or ripples according to interictal (between seizures), preictal (prior to seizure), ictal (during seizure), and/or postictal (after seizure);
   - Whereby said "events of interest" can include (but are not limited to) measures of patient-specific changes in HFO signal morphology associated with variations in the relative rates of ripples, fast ripples, and mixed frequency events;
   - Whereby said "events of interest" can include (but are not limited to) measures of distribution of HFOs and/or HFO cluster types (such as but not limited to ripples, fast ripples, and mixed events);
   - Whereby said "events of interest" can include (but are not limited to) measures of detection of early preictal in brain activity;
   - Whereby said "events of interest" can include (but are not limited to) measures of mapping of preictal, interictal, postictal and ictal brain activity as a marker of origin and evolution for possible intervention;
   - Whereby said "events of interest" can include (but are not limited to) measures of **tracking of early hemodynamic responses and/or other measures associated with heterogeneous patterns** as a marker of preictal, interictal, postictal and ictal activity or "physiological "aura" associated with ***preictal*** or ***interictal or postictal or ictal epileptiform discharges (IEDs)*** ictal onset;
   - Whereby said "events of interest" can include (but are not limited to) measures of tracking of **decoupling between local hemodynamic and neural activities** as a marker of preictal, interictal, postictal and ictal activity or **"physiological aura"** associated with early ictal onset;
- **The present example further incorporates or enables communication interface (including one or more wearable devices, WAN, LAN, IP, NAS, and/or Cloud-computing services) with the capability of automatic determination of the onset or incidence of health conditions/disorders or events of interest by way of incorporating characterisation and determination processing capabilities such as any of or any combination of coordinated ensembles of events determined via prior diagnostic processes; high-frequency neural activities predefined anatomical or functional brain regions; gamma-band neural synchronization; bilateral cortex and thalamus activity changes; hemodynamic" changes; combination of parameters; coherence interrelationship; linear dynamic computed changes, along with associated cluster and multivariate analysis, further outlined here:**
   - The present example further incorporates or enables online "monitoring" of one or more "spectral events of interest" and/or "events of interest" and/or "biomarkers" and/or "neurology" signals, combined with automatic online processing capable of detecting seizure precursors via **singular or clusters** of "spectral events of interest" related to neurological or nervous system disorders such as (but not limited to) Parkinson's, epilepsy or seizures in general (generic);
   - Whereby "events of interest" can include (but are not limited to) events or **coordinated ensembles of** events determined via **prior diagnostic processes;**
   - Whereby "events of interest" can include (but are not limited to) **high-frequency neural activities** (in vicinity of 40 Hz and/or 150 Hz) in conjunction with any of or any combination of **predefined anatomical, functional and/or temporal events;**
   - Whereby "events of interest" can include (but are not limited to) **high-frequency neural activities** (in vicinity of 40 Hz and/or 150 Hz);
   - Whereby "events of interest" can include (but are not limited to) any combination of events in conjunction with any of or any combination of predefined anatomical, functional and/or temporal events;
   - Whereby "events of interest" can include (but are not limited to) **gamma-band (in vicinity of 40 Hz) neural synchronization;**
      56
   - Whereby said "events of interest" can include (but are not limited to) measures of tracking of "hemodynamic" changes associated or evolving from local regions of the **bilateral cortex and thalamus** to the entire brain as an indicator of the onset of generalized seizures originating locally versus diffusely;
   - Whereby said "events of interest" can include (but are not limited to) measures of said **"hemodynamic" changes** can be measured using any **combination of parameters** including ultrasound, Doppler ultrasound, infrared, PPT, pulse signals, ECG signals, blood pressure, pulse wave amplitude, pulse arterial tone, oximetry;
   - Whereby said "events of interest" can include (but are not limited to) co-ordinated measures of coordinated brain activity corresponding to a plurality of spectral bands;
   - Whereby said "events of interest" can include (but are not limited to) co-ordinated measures of coordinated brain activity corresponding to a plurality of **coherence interrelationship;**
   - Whereby said "events of interest" can include (but are not limited to) co-ordinated measures of coordinated brain activity corresponding to a plurality of non-linear dynamic computed changes;
   - Whereby said "events of interest" can include (but are not limited to) co-ordinated measures of coordinated brain activity corresponding to a plurality of linear dynamic computed changes;
- **The present example further incorporates or enables communication interface (including one or more wearable devices, WAN, LAN, IP, NAS, and/or Cloud-computing services) with the capability of automatic determination of the onset or incidence of health conditions/disorders or events of interest by way of incorporating characterisation and determination processing capabilities such as non-linear analysis; linear analysis; progressive signal changes; entropy; complexity analysis; attractor dissimilarity; Lyapunov exponent; correlation dimension; linear prediction model(s), along with associated cluster and multivariate analysis, further outlined here:**
   - The present example further incorporates or enables online "monitoring" combined with the automatic "determination" and "tracking" of "events of interest" applicable to precursors or the incidence of neurological disorders including (but not limited to) epilepsy or other seizures;
   - Whereby "events of interest" can include (but are not limited to) "spectral events of interest", "biomarkers of interest", "neurology events", **"non-linear analysis", "linear analysis";**
   - Whereby "events of interest" can include any combination of "ensembles of events", "clusters of events", **"progressive signal** changes" and/or "signal transitions";
   - Whereby "non-linear analysis" can include any combination of **"entropy, "complexity analysis", attractor dissimilarity, Lyapunov exponent** and/or **correlation dimension;**
   - Whereby **"non-linear analysis"** can include any combination of **"entropy, "complexity analysis", attractor dissimilarity, Lyapunov exponent** and/or **correlation dimension;**
   - Whereby "events of interest" can be determined using **linear prediction model(s);**
   - Whereby "events of interest" can be determined using **autoregression model(s);**
   ⁵⁷

**The present example provide a wearable system for automated prognostic and/or diagnostic method or device subject-wearable system monitoring electrophysiological (EEG) with information data-networking and spectral characterisation applicable to neurological disorders prediction, onset, and/or incidence of attention deficit/hyperactive disorder (ADHD).**

An **automated prognostic and/or diagnostic method or subject-wearable system** (such as but not limited to watch, wristband, chest-band, headband, forehead sensor etc., earbuds) and further comprising of means of sensing signal(s), acquisitioning data, **monitoring,** storing, analysing, one or more **electrophysiological (EEG)** signals.

Said method or system further incorporates **information data-networking** (i.e. means including but not limited to LAN, WAN, IP, WWW etc.) or information telecommunication capabilities (i.e. means can be wireless mobile devices, other interconnectivity compatible wearable or companion devices, other wearable devices, wireless transmission, NAS, Cloud-computing services etc.).

***In a first process*** the present example comprises subject/patient monitoring capability with one or more physiological signal monitoring via a wearable monitoring device incorporating one or more **EEG** or other physiological monitoring sensors (i.e. **Figure 45****;** **Figure 43****,** **Figure 46 to Figure 50****,** **Figure 52****,** ; **Figure 48****;** **Figure 49****;** **Figure 50****;** **Figure 51****;** **Figure 52****;** **Figure 53****;** **Figure 54****;** **Figure 45****;**
);

***In* a *second process*** the present example enables sensor signal processing (i.e. amplification and/or filtering of accelerometer) of monitored signals;

***In a third process*** the present example provides signal or data acquisition of one or monitored signals;

***In a fourth process*** the present example comprises of any of or combination of analyses including (but not limited to) any of:
- -analyses processes including processes presented in any of the following figures or associated patent descriptions or body text including any of (but not limited to) **Figure 57****;** **Figure 58****;** **Figure 59****;** **Figure 60****;** **Figure 61; Figure 62****;** **Figure 63; Figure 64****;** **Figure 65; Figure 66****;** **Figure 67****;** **Figure 68****;** **Figure 69****;** **Figure 70****;** **Figure 71****;** **Figure 72****;** **Figure 73****;** **Figure 74****;** **Figure 75****;****Figure 76****;** **Figure 77****;** ***Figure 78*****;** **Figure 79****;** **Figure 80****;** **Figure 81;Figure 82****;** **Figure 83****;**
   - **means of automatic of analysis** including spectral characterisation, whereby **spectral characterisation** can include any or any combinations of:
      - FTT analysis;
      - FFT outputs including phase properties;

      - **median** amplitude band, **spectral power median frequency; relative power ratio, nominated frequency ranges, spectral edge** analysis; **95% quartile of the power spectrum** and the **relative power ratio** of frequencies;
      - **Ratio** of any two combinations of **spectral bands** (i.e. sub-Delta (i.e. >0.1 Hz) Delta (i.e. 0.1 - 3.5 Hz), Theta (i.e. 4.0 - 7.5 Hz), Alpha (i.e. 8.0 - 13.0 Hz), Beta (i.e. 14.0 - 30.0 Hz), Gamma (i.e. 30.0 - 100.0 Hz and above);
      - Computation of any **spectral bands** (i.e. sub-Delta (i.e. >0.1 Hz) Delta (i.e. 0.1 - 3.5 Hz), Theta (i.e. 4.0 - 7.5 Hz), Alpha (i.e. 8.0 - 13.0 Hz), Beta (i.e. 14.0 - 30.0 Hz), Gamma (i.e. 30.0 - 100.0 Hz and above);
      - Wavelet signal transfer;
      - ***Theta*/*Beta ratio*** (TBR);
      - characterisation of signal dynamic properties (such as but not limited to entropy, Shannon entropy, wavelet entropy, complexity analysis transforms etc.);
      - 3-dimensional source localisation and associated brain activation or suppression determination related processing (i.e. means such as but not limited to coherence, brain regional interrelationships or connectivity aspects such as Dipole, coherence, non-linear-dynamic/complexity/entropy-coherence monitored signal transformations etc.);
      - Spectral analysis segmentation capable of characterising specific biomarkers applicable to disorders, health conditions, and other events of interest;
      - Non-linear dynamic based (NLDB) signal analyses as described elsewhere in this document;
      - Coherence signal analysis as described elsewhere in this document;

***In a fifth process*** the present example provides prognosis or diagnosis of a subject's disorders, whereby said combination of any of or any combination of (but not limited to) analyses including those listed above and elsewhere in this document, enabling the characterisation of biomarkers applicable to a subject/patient's prognosis or diagnosis of the nervous system, movement or muscular system or **neurological disorders** prediction, **onset,** and/or **incidence** including of any of or any combination of Alzheimer's, **attention deficit/hyperactive disorder (ADHD),** Epilepsy, Parkinson's, Dementia, depression, Huntington's, seizure, autism, anxiety, and/or traumatic brain injury (TBI)^{58, 59} and/or ***"health conditions*** or symptoms ***of interest** "* or "events of interest/EOI" (per "EOI DEFINITION" detailed in abbreviation table at rear of this document).

***In a sixth process*** the present example enables information dissemination including messaging, event notification and processing, calendar notification and processing, health community management and associated information interchange, user opt-in or information access capabilities;

**The Present example provides a wearable system for automated prognostic and/or diagnostic method or device subject-wearable system monitoring electrophysiological (EEG) with information data-networking and spectral characterisation applicable to neurological disorders prediction, onset, and/or incidence of Alzheimer's**
An **automated prognostic and/or diagnostic method or subject-wearable system** (such as but not limited to watch, wristband, chest-band, headband, forehead sensor etc., earbuds) and further comprising of means of sensing signal(s), acquisitioning data, **monitoring,** storing, analysing, one or more **electrophysiological (EEG)** signals.

Said method or system further incorporates **information data-networking** (i.e. means including but not limited to LAN, WAN, IP, WWW etc.) or information telecommunication capabilities (i.e. means can be wireless mobile devices, other interconnectivity compatible wearable or companion devices, other wearable devices, wireless transmission, NAS, Cloud-computing services etc.).

***In a first process*** the present example comprises subject/patient monitoring capability with one or more physiological signal monitoring via a wearable monitoring device incorporating one or more **EEG** or other physiological monitoring sensors (i.**e.****Figure 45****;** **Figure 43****,** **Figure 46 to Figure 50****,** Figure 52, **;** **Figure 51****;** **Figure 52****;** **Figure 53****;** **Figure 54****;** **Figure 45****;**
), the present example further incorporating the option of wearable device minimisation ;

***In* a *second process*** the present example enables sensor signal processing (i.e. amplification and/or filtering of accelerometer) of monitored signals;

***In a third process*** the present example provides signal or data acquisition of one or monitored signals;

***In a fourth process*** the present example comprises of any of or combination of analyses including (but not limited to) any of:
Said method or system further incorporates means of automatic of analysis including **EEG signal characterisation** of one or more monitored channels , whereby **EEG** signal **characterisation** can include any or any combinations of:
- -analyses processes including processes presented in any of the following figures or associated patent descriptions or body text including any of (but not limited to) **Figure 57****;** **Figure 58****;** **Figure 59****;** **Figure 60****;** **Figure 61; Figure 62****;** **Figure 63; Figure 64****;** **Figure 65; Figure 66****;** **Figure 67****;** **Figure 68****;** **Figure 69****;** **Figure 70****;** **Figure 71****;** **Figure 72****;** **Figure 73****;** **Figure 74****;** **Figure 75****;****Figure 76****;** **Figure 77****;** ***Figure 78*****;** **Figure 79****;** **Figure 80****;** **Figure 81; Figure 82****;** **Figure 83****;**

- monitoring and analysis of one or more **EEG** channels;
- neuropsychological/cognitive interactive computer-based cognitive tests,
- genetic tests;
- scratch-and-sniff tests;
- FTT analysis;
- FFT outputs including phase properties;
- median amplitude band, spectral power median frequency; relative power ratio, nominated frequency ranges, spectral edge analysis; 95% quartile of the power spectrum and the relative power ratio of frequencies;
- Ratio of any two combinations of spectral bands (i.e. sub-Delta (i.e. >0.1 Hz) Delta (i.e. 0.1 - 3.5 Hz), Theta (i.e. 4.0 - 7.5 Hz), Alpha (i.e. 8.0 - 13.0 Hz), Beta (i.e. 14.0 - 30.0 Hz), Gamma (i.e. 30.0 - 100.0 Hz and above);
- Computation of any spectral bands (i.e. sub-Delta (i.e. >0.1 Hz) Delta (i.e. 0.1 - 3.5 Hz), Theta (i.e. 4.0 - 7.5 Hz), Alpha (i.e. 8.0 - 13.0 Hz), Beta (i.e. 14.0 - 30.0 Hz), Gamma (i.e. 30.0 - 100.0 Hz and above);
- Wavelet signal transfer;
- Theta/Beta ratio (TBR);
- characterisation of signal dynamic properties (such as but not limited to entropy, Shannon entropy, wavelet entropy, complexity analysis transforms etc.);
- 3-dimensional source localisation and associated brain activation or suppression determination related processing (i.e. means such as but not limited to coherence, brain regional interrelationships or connectivity aspects such as Dipole, coherence, non-linear-dynamic/complexity/entropy-coherence monitored signal transformations etc.);
- AEP assessment including but not limited to miss match negativity attention tests whereby responses to deviant versus standard AEP stimulus (via auditory stimuli) can be measures against progressive subject-specific outcomes over a period of time, and/or in comparison to previously calibrated reference outcomes and/or in reference to normative population comparative data bases.
- the present example further provides ***genetic tests*** can include but not limited to the determination of the presence of ***apolipo protein E4*** (protects brain cells and is governed by three genes being E2, E3 and E4). The inheritance of the E4 version of the gene from a single parent increases the risk of Alzheimer's to 3-fold, or in the case of inheritance from both parents 14-fold.
- the present example further provides an application located on a mobile device or computer program (such as but not limited to a wireless mobile device and/or WAN, LAN including NAS or cloud-computing services) can enable private or personalised health management (with professional healthcare "opt-in" oversight capabilities) ***with any of or any combination of EEG monitoring, genetic test outcomes, sniff-test outcomes, and*/*or cognitive interactive test outcomes.***
- the present example further provides an application located on a mobile device or computer program (such as but not limited to a wireless mobile device and/or ***WAN, LAN including NAS or cloud-computing services) can further include risk assessment modelling based on any of or any combination of EEG monitoring, genetic test outcomes, sniff test outcomes, and*/*or cognitive interactive test outcomes.***
- the present example further provides sniff-test outcomes can include a range of tests such as (but not limited to) quick scratch-and-sniff smell test of aromas for the loss of smell (potential predictive measure of Alzheimer's), including smoke, lilac, strawberry, leather and lemon.

***In a fifth process*** the present example provides prognosis or diagnosis of a subject's disorders, whereby said combination of any of or any combination of (but not limited to) analyses including those listed above and elsewhere in this document, enabling the characterisation of biomarkers applicable to a subject/patient's prognosis or diagnosis of the nervous system, movement or muscular system or **neurological disorders** prediction, **onset,** and/or **incidence** including of any of or any combination of Alzheimer's, **attention deficit/hyperactive disorder (ADHD),** Epilepsy, Parkinson's, Dementia, depression, Huntington's, seizure, autism, anxiety, and/or traumatic brain injury (TBI)^{60, 61}

***In a sixth process*** the present example enables information dissemination including messaging, event notification and processing, calendar notification and processing, health community management and associated information interchange, user opt-in or information access capabilities;

### Convergent Sleep & EEG Event Tracking System

- **The present example further incorporates or enables any of or any combination of:**
   - The present example further incorporates or enables convergent online monitoring of sleep and neurological events with cluster/or similar analysis and early detection of pending health conditions (such as epilepsy, Parkinson's, Alzheimer's) enabling professional and consumer-level health tracking, management and treatment
   - The present example further incorporates or enables automatic online, offline "algorithm" capable of "determination" and/or notification of homogeneous groups of brain activation or "health-conditions/state and/or events of interest" ("cluster analysis" or clustering), is the task of grouping a set of objects in such a way that objects in the same group (called a cluster) are more similar (in some sense or another) to each other than to those in other groups (clusters); the example comprising any of or any combination of:
      - Medication administration or dosage determination including drug dispenser control
      - Therapy biofeedback control including wireless or wire linked stimulation,
      - Therapy biofeedback control including magnetic stimulation control,
      - Biofeedback therapeutic control including (but not limited to) automatic or manual medication dispenser or other drug delivery;
      - **Whereby said "cluster analysis"** refers includes pre-defined criteria or properties characterises a group of objects in any (group) to be more alike (in any sense) those in other groups (clusters);
      - **Whereby said "determination"** includes (but is not limited to) statistical "data mining", such as "machine learning", bioinformatics, pattern recognition, information retrieval and image analysis;
      - **Whereby said "data mining"** algorithms can include (but is not limited to) the use of a pre-processing phase of analysis enabling pre-determination of said "data mining" algorithms in a manner whereby the ensemble of target "events" or "clusters" of interest can be can established based on prior data recordings or earlier monitoring data, including (but not limited to) a means of identifying multivariate data sets or characteristics "of interest";
      - **Whereby said "data mining"** algorithms can include (but are not limited to) the practice of analysing data without an a-priori hypothesis;
      - **Whereby said "machine learning"** algorithms can include (but is not limited to) approaches including :
         clustering, decision tree learning association rule learning, artificial neural networks, bayesian networks, inductive logic programming, support vector machines, reinforcement learning, representation learning, similarity and metric learning and/or sparse dictionary learning;
      - **Whereby said "machine learning"** algorithms (include but are not limited to) supervised learning algorithms, unsupervised learning algorithms, semi-supervised learning, transduction, or transductive inference, reinforcement learning, learning to learn learns, developmental learning, generative machine learning models and/or discriminative machine learning model;
      - **Whereby said "data mining"** algorithms can include (but are not limited to) processing and/or pre-processing techniques, along with examples (but not limited to) of approaches or uses of such analysis including games, business, science and engineering, medical data mining, spatial data mining, sensor data mining, visual data mining, music data mining, surveillance, pattern mining, subject-based data mining and/or knowledge grid data mining;
      - **Whereby said "notification"** includes (but is not limited to) numeric, tabular, graphic information and/or audible, visual and/or vibration alarms.
      - **Whereby said "determination"** includes (but is not limited to) predictions and/or onset determination and/or detection of the incidence of;
      - **Whereby said "health-conditions/state and/or events of interest"** include any of or any combination of psychological and/or psychological states;
      - **Whereby said "health-conditions and/or events of interest"** includes (but is not limited to) monitoring, tracking, treatment or overall management of health conditions, neurological disorders and/or health conditions applicable to neurological, nervous system and muscular degenerative, along with sentient state;
      - **Whereby said "health-conditions and/or events of interest"** includes (but is not limited to) monitoring, tracking, treatment or overall management of health conditions, neurological disorders and/or health conditions applicable to neurological, nervous system and muscular degenerative, along with sentient state and/or deleterious states applicable to epilepsy, seizure, Parkinson's, Alzheimer's, depression, and/or autism and/or per ***"health conditions*** or symptoms ***of interest"*** or "events of interest/EOI" (per "EOI DEFINITION" detailed in abbreviation table at rear of this document).
      - **Whereby said "health-conditions and/or events of interest"** includes (but is not limited to) monitoring, tracking, treatment or overall management of an individual's sleep, sleep behaviour disorder, neurology, cardiology, respiratory, neurology, nervous system, muscle system, physiological tremor, psychiatric or other physiological functional aspects;
      - **Whereby said "health-conditions and/or events of interest"** includes (but is not limited to) interrelationship or interaction between two or more physiological and/or psychological parameters or conditions or states;
         - **The present example further incorporates or enables:**
            - Remote system intervention, including the capability and means of manually or automatically adjusting sensors, associated signal conditioning and/or associated analysis algorithms.

The present example enables atlas definitions or regional mapping of the brain to be automatically associated with psychological and/or physiological in order to enable health management of applicable to onset or incident of health conditions in order to enable optimal personalised safety and/or preparation and/or minimise health or environmental risk and/or assist with related optimisation of therapy or medication of an individual and/or incorporate consumer-level system alerts, warnings and/or information assistance or guidance, while enabling remote consumer-level or professional level health-care assistance or intervention
- The present example further incorporates or enables a system whereby continuous monitoring at consumer-level or professional level whereby health conditions of interest can be detected or retrospectively determined in order to continuously increase the intelligence of the said system such that increased learning and capability to optimise an individual's health management
- The present example further incorporates or enables a system whereby continuous monitoring at consumer-level or professional level whereby health conditions of interest can be detected or retrospectively determined in order to continuously increase the intelligence of the said system such that increased learning and capability (such as but not limited to artificial intelligence) can be deployed to improve/optimise the detection, tracking, onset and/or treatment (health management) determination of an individual's general or specific health conditions of interest or concern with the course of time and continued or ongoing accumulation of personalised monitoring information and data.
- -Whereby the said determination of the said "detection and onset determination of an individual's specific health conditions of interest or concern" can include but are not limited to particular, most physiological mechanisms including those related to functions of cardiac, respiratory, nervous, muscle, blood, psychology, sleep, neurology and others are inherently interlinked and interrelated.
- **-Whereby the said information relating to "detection and onset determination** of an individual's specific health conditions of interest or concern" can include but are not limited to demonstrating online monitoring or detection of any of or any combination of:
   - 1) neurological and/or nervous system and/or muscular health conditions (i.e. epilepsy, Parkinson's Alzheimer's, autism etc.);
   - 2) physiological health condition (i.e. breathing or respiratory failure or function, cardiac failure or function),
   - 3) psychological state (sleep/wake state; unconsciousness/consciousness depth, attention level, high frequency, consciousness state;
      4) the present example enables AEP monitoring and analysis including odd-ball and MMN measures of cognition status and state **determination of level and/or sedation level and/or consciousness level or states or transitions and/or attention and/or cognition and/or cognitive impairment and/or awareness and/or associated responsiveness** based on related assessments utilising auditory evoked potential (AEP) stimulation (i.e. **MMN or odd ball auditory stimulus test paradigms** comprising of a standard and deviant auditory stimulus presentation to subject for example but not limited to) along with corresponding monitoring and analysis of evoked neurological responses in terms of **comparative waveform (i.e. MMN)** and/or evoked response delays, amplitude, spectral, **signal dynamics** (i.e. non-linear dynamic analysis such as entropy, complexity analysis etc.) characteristics and other AEP response characteristics. Whereby responsiveness and delay of AEP responses to an attention auditory stimulus (i.e. **odd ball change of stimulus** such as a sequence of standard tomes punctuated randomly with a deviant tone, followed by subject's measure of responsiveness or aware ness to change via assessments of AEP responses) paradigm (i.e. odd ball or change is an auditory stimulus chain).
   - 5) event types (i.e. spindle, K-complex, arousal, micro-arousal, high frequency oscillation (HF), high frequency ripples, high frequency type A, high frequency type B, etc.), time context (i.e. early onset, late onset, actual incident); 4) brain or other biological source localisation , and/or
   - 6) coherence , interrelationship, variance and/or association between any two physiological or neurological or imaged biological data in the context of time and/or anatomical location and/or spatial location or orientation and/or spatial dynamic aspects
   - In order to improve or assist therapy or diagnostic decisions and associated outcomes.
   - -Whereby the said "accumulation of personalised monitoring information and data" can be managed in a way whereby secure and restricted access is available only to the individual and/or only to delegated trusted entities determined or nominated by the said individual

- **The present example further incorporates or enables monitoring or intervention capabilities applicable to the onset, incident or prevention of health conditions or other events of interest including Parkinson's or Alzheimer's disease, comprising any of or any combination of:**
   - Consumer or patient wearable or companion wireless device (carried or attached) with any combination of the following attributes:
      ***Dementia analyser -*** location, falls/gyro, has family/carer pre-determined parameters of normal location or travel & behavioural patterns versus actual mobile tracking - predictive algorithm based on normal or predefined behaviour or routines travelling compared to potential dementia episode onset or incident (i.e. you set travel and road crossing parameters and boundaries as a marker or danger with online carer intervention via associated locational carer services team - plus special watch that communicates with person and can route them home, call a taxi or request other intervention.

In one exampletrending analysis along with related graphical, numeric or other outcomes can be presented to highlight trend indicative of neurological (i.e. git variation over time as a potential market of Parkinson's onset), muscular disorder such as fluidity changes of an individual's movements, motion, walking, interrelationship with arms movement and walking stride (i.e. utilisation of step, walk, speed, location (i.e. GPS and/or GSM and/or wireless network based measures), gyro-meter gravitational-direction-related, posture-position, and/or manoeuvres such as negotiating corners and the like, nervous system disorders neuromuscular disorders such as tremor analysis indicative of Parkinson's onset or incidence or associated medication control or requirements), sleep behaviour disorder (causally-linked or idiopathic) such as trends or increasing tonicity during REM versus other sleep stages, amongst other combinations of pathological and physiological events or interest potentially indicative of health conditions of interest.
- **The present example further incorporates or enables "Aura" (earliest possible health-condition onset prediction)**
- The present example further incorporates or enables the means of online (i.e. processing within or across a group of mobile or wearable devices and/or NAS or other LAN/WAN systems) or offline (i.e. off-line data- mining/predictive analysis) ***"**monitoring and tracking of* a *subject's physiological, psychological and*/*or pathological parameters (variables)"*** in order to identify the ***earliest possible onset or aura*** associated with the prediction (i.e. automatic or computer-assisted), onset, incident or prevention of health conditions or other events of interest including Parkinson's, Alzheimer's and other neurological, muscular, nervous-system, neuromuscular, and/or movement disorders/diseases, where said example further comprises any of or any combination of:
   ➢ Means of computing ***"base-line or running average values"* ;**
   ➢ whereby said "base-line or running average values" means can (for example) include regression, averaging, or running-average with predefined data periods upon which such ***"base-line or running average values"*** can be computed.
   ➢ Said ***""base-line or running average values"*** analysis can be by way of autoregressive analysis without or without modelling such regression. Further, ***"base-line or running average values"*** can include the provision to compute a plurality or only a single said **"*base-line or running average values".*** The plurality computational options can include the provision to compute "short-term" as well as "long-term" ***"base-line or running average values".***
   ➢ Whereby the time-period of the said ***"short-term" "base-line or running average values"*** can be as short as a fraction of a second depending on the nature of the physiological variable or event of interest and as long as required (typically seconds or minutes), while the said "long-term" ***"base-line or running average values"*** can be computed over the period as required.
   ➢ Whereby the "long-term" ***"base-line or running average values"*** could typically be configured in terms of hours, days, months and years applicable to the earliest possible trend detection applicable (for example) to longer-term health trends such as Parkinson's gait change over the period of time ( change in arm-swing synchronisation or overall pattern and behaviour as it related=s to movement or steps or stride (i.e. detected, for example with locational, positional, movement, sensors and monitoring covered elsewhere in this document).
   ➢ Whereby the "long-term" ***"base-line or running average values"*** could typically be configured in terms of days, months and years applicable to the earliest possible trend detection applicable (for example) to longer-term health trends such as Parkinson's sleep EMG amplitude variations during sleep based on longer periods of time such as those applicable to idiopathic sleep behaviour disorder (SBD) as covered elsewhere in this document.
   ➢ Whereby the "long-term" ***"base-line or running average values"*** could typically be configured in terms of days, months and years applicable to the earliest possible trend detection applicable (for example) to longer-term health trends such as the comparison between current monitoring and associated multivariate analysis means (i.e. combining analysis of : a) signal dynamics properties including linear versus non-linear dynamic signal characterisation), and b) characterisation of clusters or sequences of clusters of HFO's or ripples) to previously monitored and analysed outcomes based on said short-term or "long-term" ***"base-line or running average values".***
   ➢Whereby said ***"order to i**dentify* "** can include the determination of normal ranges associated with any monitored physiological parameter or any combination of parameters associated with two or more monitored physiological parameters;
   ➢Whereby said ***"order to identify* "** can include the determination of safety margins applicable to normal values of any one or combination of two or more of any monitored physiological parameters;
   ➢Where ongoing or most recent monitored physiological variables can be compared ***to previously monitored data or analysis*** of one or more channels of previously monitored data, in order to identify periods or incidents when a subject's physiological or psychological status varies from ***margin of safety*** of any one or more parameters;
   ➢ - The present example further incorporates or enables ***margin of safety ,*** normal or acceptable ranges of physiological function, to be defined and configured by automatic means,
   ➢ - The present example further incorporates or enables ***margin of safety ,*** normal or acceptable ranges of physiological function, thresholds applicable to the prediction or the onset or incidence of events or health conditions of interest to be configured by way of information access available to specialists, healthcare-workers, doctors or other appropriately qualified personnel authentication processes of ensuring ***various degrees or levels of by privacy and security,*** subject to professional or consumer level of the application or user associated requirement,
   ➢Where ***margin of safety*** , ***thresholds, and ranges of normal versus abnormal physiological parameter** measurement or related analysis,* can be by ***automatic means based on prior diagnostic data*** or other subject-specific ***settings*;**
   ➢Where these said **"*settings*"** and automatic means can further comprise of **"*learning or artificial intelligence"*** based on establishing the ***combination of physiological parameters and multivariate analysis most probable in terms of predicting the earliest onset of health condition or the related*** "aura";
   Whereby said ***""**monitoring and tracking of* a *subject's physiological, psychological and*/*or pathological parameters (variables)"* parameters** includes any events or health conditions of interest including (but not limited to) any of or any combination of the following (further detailed elsewhere in this document): Sound monitoring; Stethoscope auscultation sensor, monitoring and automatic analysis, classification, tracking and detection capabilities; Acoustic noise cancellation system; Motion detection; REM sleep behaviour disorder (RBD); Pulse wave analysis (PWA) and pulse wave velocity (PWV) monitoring and analysis capability; PWA and PWV sensors; Pulse wave analysis (PWA) sensor measures; Ballistocardiograph; Position, locational and movement sensing and monitoring; Gait or movement tracking and characterisation of events of interest; Movement and locational information; ECG sensor(s) and monitoring; Light sensor(s) and monitoring; Breathing band sensors and monitoring; EMG sensors and monitoring; GSR; Cardiac function; Sleep training system; Plethysmography Oximetry; Pulse transient oscillation amplitude measures; temperature, Energy-exertion/metabolism-monitoring (EM) as a surrogate calorie-burn measure; Physiological and/or Sleep and/or Wake measures or markers; along with other psychological states (i.e. sleep, wake)(as outlined in more detail under these respective heading in this document).
   ➢ ***In one example*** is where one or a combination of physiological parameters are measured along with associated analysis transforms deployed to distinguish predefined (via system user configurable means, via pre-diagnostic data of subject, via automatic learning capabilities able to evolve accuracy in terms of augmenting outcomes of onset events and conditions associated with events or incidents, such as seizures of interest, or via normative population data-base information, or via subject-specific data base information or any combination thereof) criteria applicable to likely onset or very early prediction of events or health conditions of interest, including the detection of specific clusters or other characteristics of ensembles of ***HFO's or ripples*** (including any of or any combination of parameters detailed in this document under headings ***Figure 45******.***

### Evolving and continually improving self-learning expert systems or artificial intelligence diagnostic or prognostic process

The ***"evolving and continually improving"*** (with ongoing or progressive detection of incidents of interest and greater specificity of probable combinations and associated multivariate analysis techniques, including MonteCarlo cross-coupling analysis with associated accuracy assessment measures (such as ordinal scaled measures including Pk probability statistical outcomes) of severity of epilepsy diagnosis or prognosis, and/or the option of associated ***"self-learning or artificial intelligence"*** ongoing automatic or computer assisted ***"learning"*** capability - i.e. the selection of monitoring channels and/or monitoring sensor locations and/or acquisition parameters and/or pre-acquisition signal processing (including artefact suppression, base-line adjustment, filtering, discrimination or separation between myogenic and neurogenic signals etc.)and/or post-acquisition processing (including artefact suppression, base-line adjustment, filtering, discrimination or separation between myogenic and neurogenic signals etc.) adapted in accordance to aims (i.e. diagnostic and prognostic goals) and requirements (i.e. constraints or monitoring environment or non-invasive preferences) associated with monitoring, can be progressively fine-tuned based on aggregated knowledge (i.e. per Figure 78 blocks 2 and 5 and per aggregation of knowledge per ***Figure 79*** )accuracy and relevant of diagnostic and prognostic outcomes (i.e. per ***Figure 45*** minimisation capability per ***block 8*** and ongoing assessment and improvement of monitoring and analysis parameters per ***block 13*);**
➢ The said ***"evolving and continually improving"*** multivariate analysis determination, in order to improve the prediction accuracy and earliest possible aura precursor determination, can be based on ranking of regression models and then cross-validation incorporating 1,000-fold statistical cross-validation was performed using the Monte Carlo technique⁶², followed by procedures to determine the best consciousness/unconsciousness prediction probability using minimal and full models (for example ***"evolving and continually improving"*** means only);
➢ In one example subtle physiological measures such as Ph (i.e. 24-hour pH impedance to evaluate reflux), body temperature, cardiac function (derived aortic pulse shape; heart rate variability, abnormal heart beats, blood pressure, etc.), oxygen saturation, gas-exchange parameters (C02) and/or any other measures including cardiac function such as those mentioned above (said ***""**monitoring and tracking of* a *subject's physiological, psychological and*/*or pathological parameters (variables)"* parameters)_could** be examined in the context of symptomatic precursors of events or health conditions of interest;
➢ In one example a detection of a precursor or incidence applicable to a health condition status or event of interest could automatically flag a remote surveillance monitoring centre or individual in order to provide ***"intervention as required by a monitored subject*";**
➢ Said ***"intervention as required by a monitored subject"*** means can be via triage processing whereby degrees of severity are determined from any combination of the online monitoring parameters, associated analysis outcomes, background patient history and information, background history of the severity and frequency of health conditions and events of interest, and/or ability of subject to respond to alerts designed to check subject's responsiveness and need for help (including automatic intercom access to remote surveillance centre or individual - such as via smart phone based watch). A Further means of ***"intervention as required by a monitored subject*"** can include automatic health-carer attendance, ambulance, paramedics or other carer alert and/or locational tracking system attached to the subject via bangle, watch, chest band or other to swiftly attend a distressed or at risk monitored subject;
➢ - The present example further incorporates or enables the ***"long-term"*** or ***"short-term"*** detection of depleted tonic suppression during sleep states (i.e. REM stage) as a potential marker of idiopathic sleep behaviour disorder (SBD) implicated as an early onset marker of Parkinson's disease.
➢ - The present example further incorporates or enables the ***"long-term"*** or ***"short-term"*** detection of asynchrony relationship and/or other abnormal (i.e. sloppy gait with and difficulty with corner manoeuvres) walking as a potential marker of idiopathic sleep behaviour disorder (SBD) implicated as an early onset marker of Parkinson's disease.
➢ - The present example further incorporates or enables the ***"long-term"*** or ***"short-term"*** detection of combinations of asynchrony relationship and/or other abnormal (i.e. sloppy gait with and difficulty with corner manoeuvres) walking, and detection of depleted tonic suppression during sleep states (i.e. REM stage) as a potential marker of idiopathic sleep behaviour disorder (SBD) as a potential markers of idiopathic sleep behaviour disorder (SBD) as an early onset marker of Parkinson's disease.

### Suprachiasmatic Region

- **The present example further incorporates or enables wearable diagnostic or prognostic monitoring system(s) incorporating the capability of automatic analysis applicable to the determination of the onset or incidence of *"health conditions*/*disorders or events of interest*" The example further comprising any of or any combination of:**
   - **monitoring method(s) and device(s) can incorporate communication interface(s) comprising and one or more WAN, LAN, IP, NAS, and/or Cloud-computing services peer to peer information telecommunication mediums or systems to enable the transmission of physiological or environmental monitored data and/or subject/patient information exchange and/or access to online analysis capabilities capable of supplementing local monitoring, analysis, storage, and/or indications;**
   - **determination of the onset or incidence of "health conditions/disorders or events of interest", include enabling an "automated method of determining neurological prognosis or diagnosis" or personal-care management of a subject's a nervous system, and/or *"health conditions* or symptoms *of interest"* or "events of interest/EOI" (per "EOI DEFINITION" detailed in abbreviation table at rear of this document).**
- **Further comprising any of or any combination of:**
   - -Whereby said "events of interest" can include (but are not limited to) brain regions including (but not limited to) the ***suprachiasmatic region associated control or interconnectivity regions of the brain*** (i.e. but not limited to ***Figure 66***);
   - -Whereby said "events of interest" can include (but are not limited to) brain regions including (but not limited to) any of or any combination of the ***regions associated control or interconnectivity regions of the brain*** (i.e. but not limited to ***Figure 66***);
      - Suprachiasmatic region;
      - LOCATION AND EFFECTS OF CONSCIOUSNES;
      - Hindbrain and midbrain : crucial for arousal and sleep;
      - Reticular formation damage : causes coma;
      - Prefrontal cortex : crucial to conscious control of signal processing;
      - Suprachiasmatic (SCN) nucleus **(circadian** clock);
      - Thalamus ("consciousness switch");
      - Posterior cingulate; and/or
      - Medial parietal cortical areas ; Medial basal forebrain areas;
   - -Whereby said "events of interest" can include (but are not limited to) ***brain regions including (but not limited to) those regions defined by standardised mapping or atlas definitions*** (i.e. ***Talairach Atlas***; Current atlas tools; Harvard Whole Brain Atlas, MNI Template, The standard template of SPM and International Consortium for Brain Mapping; Atlas of the Developing Human Brain; All Functional including Brodmann areas, Gyri, Sulci etc.);
   - -Whereby said "events of interest" can include (but are not limited to) brain regions include the ***suprachiasmatic region associated control or interconnectivity regions*** of the brain and/or diagnosis and/or treatment and/or medication of neurological or sleep disorders such as sleep delay syndrome;
   - ***Real-time detection of "Events of Interest"** ;*
   - Whereby "events of interest" ***can be detected online*/*real-time automatically*** and include (i.e. but not limited to) events, conditions, and/or attribute examples per **Figure 57****;** **Figure 58****;** **Figure 59****;** **Figure 60****;** **Figure 61; Figure 62****;** **Figure 63; Figure 64****;** **Figure 65; Figure 66****;** **Figure 67****;** **Figure 68****;** **Figure 69****;** **Figure 70****;** **Figure 71****;** **Figure 72****;** **Figure 73****;** **Figure 74****;** **Figure 75****;** **Figure 76****;** **Figure 77****;** ***Figure 78*****;** **Figure 79****;** **Figure 80****;** **Figure 81; Figure 82****;** **Figure 83****;**
   - -Whereby said ***"events of interest*" *can be defined and configured online for configuration*** by one or more pre-**defined authorises personnel including** (but not limited to) **scientific, technical, medical, specialist, consumer, patient, nurse** and other individuals with appropriate security authentication and access rights;
   - -Whereby said ***"events of interest***" can include (but are not limited to) ***health conditions, neurological disorders and*/*or health conditions applicable to neurological,*** nervous system and muscular degenerative, along with sentient states and/or deleterious states applicable to epilepsy, seizure, Parkinson's, Alzheimer's, depression, and/or autism and/or ***"health conditions*** or symptoms ***of interest***" or "events of interest/EOI" (per "EOI DEFINITION" detailed in abbreviation table at rear of this document).

**The present** example **further comprises of environmental monitoring comprising any of or any combination of:**
Sound monitoring; Stethoscope auscultation sensor, monitoring and automatic analysis, classification, tracking and detection capabilities; Acoustic noise cancellation system; Motion detection; REM sleep behaviour disorder (RBD); Pulse sensor integrated watch of other wrist worn device (band or bangle); Pulse wave analysis (PWA) and pulse wave velocity (PWV) monitoring and analysis capability; PWA and PWV sensors; Pulse wave analysis (PWA) sensor measures; Ballistocardiograph; Position, locational and movement sensing and monitoring; Gait or movement tracking and characterisation of events of interest; Movement and locational information; ECG sensor(s) and monitoring; Light sensor(s) and monitoring; Breathing band sensors and monitoring; EMG sensors and monitoring; GSR; Cardiac function; Heart rate; Sleep training system; Plethysmography Oximetry; Pulse transient oscillation amplitude measures; Temperature; Energy-exertion/metabolism-monitoring (EM) as a surrogate calorie-burn measure; **Sleep parameters;** Physiological and/or Sleep and/or Wake Markers; **Sleep parameters;** Sleep Architecture Measures; Environmental sensing; "dynamically-linked capabilities"; Psychological states; RAPID EYE MOVEMENT (REM) SLEEP CHARACTERISTICS; CLASSIFIED SLEEP DISORDERS; Select Sleep disorders:; DREAMING STATES; HALLUCINATION STATES; DISSOCIATED STATES; HYPNOSIS STATES; and as further outlined here and elsewhere in this patent application document:
**The present example further comprises of any of or any combination of physiological or environmental monitoring and/or associated analysis determination capabilities any environmental sensing comprising (but not limited to) comprising any of or any combination of:**
***Environmental sensing (with alarm*** or alert or indicator or interface to mobile device associated or messaging, email, phone automated voice message and other information or communication systems), weather elements, wind, humidity, temperature, ionisation monitoring, ionisation smoke alarm, methane monitoring, toxic gas monitoring, toxic chemical monitoring, Co2 gas monitoring, methane gas monitoring, other toxic gases, other toxic chemicals and/or thermometer and as further outlined here and elsewhere in this patent application document:
- The present example further incorporates or enables **any environmental sensing;**

**The present** example **further comprising any of or any combination of any events, health conditions including physiological, pathological, wake/sleep states, other psychological states, diagnostic, prognostic, neurological monitoring and/or associated analysis determination comprising(but not limited to) any of or any combination of:**
- ***"health conditions*** or symptoms ***of interest***" or "events of interest/EOI" (per "EOI DEFINITION" detailed in abbreviation table at rear of this document).

- -further Brain ***atlas or mapping*** ((i.e.Talairach Atlas; Current atlas tools; Harvard Whole Brain Atlas, MNI Template, The standard template of SPM and International Consortium for Brain Mapping; Atlas of the Developing Human Brain; All Functional including Brodmann areas, Gyri, Sulci etc) definitions (i.e. or regional functional/cognitive or anatomical mapping of the brain) can be ***automatically associated with wake*/*sleep states, psychological and*/*or physiological pathologic, diagnostic and*/*or prognostic markers* (****Figure 57****;** **Figure 58****;** **Figure 59****;** **Figure 60****;** **Figure 61; Figure 62****;** **Figure 63; Figure 64****;** **Figure 65; Figure 66****;** **Figure 67****;** **Figure 68****;** **Figure 69****;** **Figure 70****;** **Figure 71****;** **Figure 72****;** **Figure 73****;** **Figure 74****;** **Figure 75****;** **Figure 76****;** **Figure 77****;** ***Figure 78*****;** **Figure 79****;** **Figure 80****;** **Figure 81; ; Figure 82****;** **Figure 83****;**
   in order to ***enable health management applicable to onset or incident*** of health conditions in order to enable optimal personalised safety and/or preparation and/or minimise health or environmental risk and/or assist with related optimisation of therapy or medication of an individual and/***or incorporate consumer-level system alerts, warnings and*/*or information assistance*** or guidance, while enabling remote consumer-level or professional level health-care assistance or intervention

- The present example further incorporates or enables a system whereby continuous monitoring at consumer-level or professional level whereby health conditions of interest can be ***detected or retrospectively determined*** in order to ***continuously increase the intelligence*** of the said system such that ***increased learning and consequential effectiveness, precision and capability*** to optimise an individual's health management (including diagnosis, prognosis, pathogenesis, pathological marker determination, etc.);
- The present example further incorporates or enables a system whereby continuous monitoring at consumer-level or professional level whereby health conditions of interest can be ***detected or retrospectively*** determined in order to continuously increase the intelligence of the said system such that increased learning and capability (such as but not limited to artificial intelligence) can be ***deployed to improve*/*optimise the detection, tracking, onset and*/*or treatment (health management)*** determination of an individual's general or specific health conditions of interest or concern with the course of time and continued or ongoing accumulation of personalised monitoring information and data.
- ***Whereby the said determination*** of the said "detection and onset determination of an individual's specific health conditions of interest or concern" can include but are not limited to particular, ***physiological mechanisms*** including those related to functions of ***cardiac, respiratory, nervous, muscle, blood, psychology, sleep, neurology and others are inherently interlinked and interrelated;***
- ***Whereby the said information relating to "detection and onset determination of an individual's specific health conditions of interest or concern"*** can include but are not limited to demonstrating online monitoring or detection of any combination of:
   1) ***neurological*** and/or nervous system and/or muscular health conditions (i.e. epilepsy, Parkinson's Alzheimer's, autism etc.);
   2) ***physiological*** health condition (i.e. breathing or respiratory failure or function, cardiac failure or function),
   3) ***psychological state*** (sleep/wake state; unconsciousness/consciousness depth, attention level, high frequency, consciousness state; MMN; attention; sedation depth; anaesthesia depth; );
   4) ***event types*** (i.e. spindle, K-complex, arousal, micro-arousal, high frequency oscillation (HF), high frequency ripples, high frequency type A, high frequency type B, etc), time context (i.e. early onset, late onset, actual incident); 4) brain or other biological source localisation , and/or
   5) ***coherence, interrelationship, variance*** and/or association between any two physiological or neurological or imaged biological data in the context of time and/or anatomical location and/or spatial location or orientation and/or spatial dynamic aspects;
   in order to improve or assist therapy or diagnostic decisions and associated outcomes;
- ***Whereby the said "accumulation of personalised monitoring information and data"*** can be managed in a way whereby secure and restricted access is available only to the individual and/or only to delegated trusted entities determined or nominated by the said individual by way of computer system access rights allocation and/or associated computer system access security authentication means (i.e. biometrics, password, private-key, special biometric-data related key etc.);
- Whereby said "online monitoring" can incorporate any combination of:
   - a) synchronised parameter monitoring;
   - b) corresponding state determinations;
   - c) raw monitored physiological or psychological data and/or patient information and/or or other information;
   - d) wake/sleep raw monitored physiological or psychological data and/or patient information and/or or other information;
   - e) neurological event key with spindle, k-complexes, HFO's, entropy event, event status box, event status annotation;
   - f) physiological status such as physiological event key subject/patient-response, event status box indications, event status annotation indications;
   - i) Dynamic Interrelationships Link determination and indications;
   - j) ***neurological spatial dynamics defined by single events of interest or health conditions or interest and*/*or clusters of single events of interest or health conditions of interest; and*/*or***
      k) **The present** example **further comprises of any of or any combination of physiological or environmental monitoring or analysis determination capabilities comprising any of or any combination of:**
      - ***"health conditions*** or symptoms ***of interest** "* or "events of interest/EOI" (per "EOI DEFINITION" detailed in abbreviation table at rear of this document) ***including means of automatically configuring detection criteria (i.e. per automatically associated with wake*/*sleep states, psychological and*/*or physiological pathologic, diagnostic and*/*or prognostic markers* (****Figure 57****;** **Figure 58****;** **Figure 59****;** **Figure 60****;** **Figure 61; Figure 62****;** **Figure 63; Figure 64****;** **Figure 65; Figure 66****;** **Figure 67****;** **Figure 68****;** **Figure 69****;** **Figure 70****;** **Figure 71****;** **Figure 72****;** **Figure 73****;** **Figure 74****;** **Figure 75****;** **Figure 77****;** ***Figure 78*****;** **Figure 80** **;** **Figure 82****;** **Figure 83****;**
      - The present example further incorporates or enables consumer-level monitoring capabilities applicable to the most sophisticated medical and widely accepted ***scientific standards ("gold-standard ") parameter monitoring,*** augmented with unique high-dependence consumer-level network data-reliability capabilities, novel monitoring device technologies, improved body sensor technology, and analysis methodologies and processes in order to provide a means of ***enhanced professional-grade consumer-level monitoring.***
      - The present example further incorporates or enables a means (i.e. to enabling continuous monitoring combined with health condition event determination comprising any combination of any of or any combination of synchronised audio-visual, physiological, diagnostic, prognostic, pathologic properties, health conditions or events of interest (i.e. ***multipoint time-synchronisation monitoring; MTM), adaptive physiological-body monitoring (APM), adaptive physiological-body monitoring (APM), Somnisync "dynamic data-exchange" of sleep parameters" system.***
- **The present example further incorporates or comprises of a highly secure and private data gateway with incorporation of software object module capable of interfacing disparate information and monitoring systems, with a universal and defined interface manner, while segregating clinical information and data from private information and encompassing private key and data segregation techniques designed to mitigating security beaches whilst maintaining data accessibility in an effective manner with patient/subject-centric authorisation and privacy constraints but enabling personal emergency health specialists appropriate access, the present example further comprising any of or any combination of:**
   - ***Americana Academy of Sleep Medicine (AASM) or any SCOPER rating classification*** means (Figure 76), cardiology monitoring means, temperature monitoring, infrared monitoring means, health management wireless-linked integrated ear-bud monitoring (further detailed elsewhere in this document).
   - The present example further incorporates or enables the ability to ***target diagnostic conditions*** measures to be defined in terms of biological markers or clusters/ensembles of different biological markers applicable to the incident of onset of health of investigational, tracking or health management interest comprising any combination of :
   - ***A structured means of breaking down early onset*** or incident of health conditions of interest in terms of said health condition "related events";
   - whereby said health conditions can relate to any combination of ***cardiac, respiratory, neurological, ventilation physiological parameters;***
   - whereby said health conditions can relate to any ***combination of cardiac, respiratory, neurological, ventilation*** physiological "biomarkers" and/or "events";
   - whereby said health conditions can relate to any combination of ***cardiac, respiratory, neurological, ventilation sequences or patterns*** on one or more types or incidents of said "biomarkers" and/or "events";
   - whereby said health conditions can relate to any combination of cardiac, respiratory, neurological, ventilation sequences or patterns on one or more types or incidents of said "biomarkers" and/or "events";
   whereby said patterns of events includes ***clusters or different ensembles of*** events which can be reported or displayed in terms of a 3-dimensional brain view with visual identification or validation of the incident or early onset detection of different health conditions, of early onset or (i.e. **Figure 68****;** or more complex scientific, medical, or technical interfaces such as per ***Figure 45*****; *;*** ***Figure 56******;*** ***Figure 57******;*** ***Figure 58******;*** ***Figure 59******;*** ***Figure 60******;*** ***Figure 61; Figure 62******;*** ***Figure 63; Figure 64******Figure 65; Figure* 66****; *Figure 67******Figure* 69****;** ***Figure 70******;*** ***Figure 71******;*** ***Figure 72******;*** ***Figure 73******;*** ***Figure 74******)*.**
   - whereby said patterns of events includes clusters or different ensembles of events as presented in corresponding example figures **(****Figure 68****;** or more complex scientific, medical, or technical interfaces such as per ***Figure 45*****;**
   - ***;*** ***Figure 56******;*** ***Figure 57******;*** ***Figure 58******;*** ***Figure 59******;*** ***Figure 60******;*** ***Figure 61******;*** ***Figure 62******;*** ***Figure 63; Figure 64******Figure 65; Figure 66*****; *Figure 67******Figure 69******;*** ***Figure 70******;*** ***Figure 71******;*** ***Figure 72******;*** ***Figure 73******;*** ***Figure 74******)*** demonstrating online detection of any ***combination of*** :
      ***1) physiological condition*** (i.e. epilepsy, Parkinson's Alzheimer's, autism and/or ***"health conditions*** or symptoms ***of interest*"** or "events of interest/EOI" (per "EOI DEFINITION" detailed in abbreviation table at rear of this document).
   - ***2) psychological state*** (sleep/wake; unconsciousness/consciousness depth, attention level, state high frequency, consciousness state);
   - ***3)*** event types (i.e. spindle, K-complex, arousal, micro-arousal, high frequency oscillation (HF), high frequency ripples, high frequency type A, high frequency type B, etc.), time context (i.e. early onset, late onset, actual incident); (temp -overview of figure layout- spindle (LH functional data), 3 D cut rotating image (RH image display) plus polysomnography or **EEG** display with colour coded conditions (i.e. onset epilepsy cluster could be indicated via red markers; onset Parkinson's cluster via orange markers, etc.) and codes for spindle, K-complex, arousal); psychological states for sleep/wake/consciousness depth; attentions-MMN etc.);
   - Whereby said *"structured means"* includes the means of breaking down monitoring requirements in ***terms of properties such as*** (but not limited to) properties of system to monitor ***physiological parameters and detect and defining condition of structured approach*** in order to establish and continually improve the analysis model and combination of monitored physiological, neurological and/or psychological parameters (see also ***block [29]* Automatic analysis model Determination)** to most effectively detect events or health conditions of interest.

### Description of Figures

***Figure 75*** Automatic online determination of events of interest including wake or sleep period biomarkers applicable to onset or incidence of neurological disorders such as Parkinson (including idiopathic SBD), epilepsy, Depression, Autism and/or ADHHD

***Block [1]*** Subject/Patient/Consumer (***[1]***) is monitored by way of one or more device(s) or sensor(s) ***([2], [3], [4], [5], [6]*)** with associated outputs input to monitoring interface ***[7]*** (per below).
***[2]*** Integrated forehead sensor with Dynamic Data Synchronisation (DDS); incorporating any of **EEG,** EOG, EMG eye-movements, eye-lid movements
***[3]*** Conventional PSG sensor(s) /DDS ; incorporating any PSG parameters
***[4]*** Other patient worn system /DDS ; - i.e. slimline or sensor integrated smart-health-watch and/or other wrist mounted device
***[5]* Neuro-Sleep Dynamic Data Synchronisation** (NSDDS)
***[6]* Therapeutic links-** i.e. magnetic or electrical brain stimulation.; medication
***[7]*** Monitor signal, quality interface check, impedance, amplification, conditioning, data-acquisition, processing. The monitored physiological parameter(s) or sensor(s) are then input to blocks ***[9], [10], [11], [16], and block [20].***
***[8]* Study Sequence Analyser**-enables trending and other multi-study analysis techniques.
   - The present example further incorporates or enables any of or any combination of **comparative data base [12], parameter configuration [13], parameter configuration cluster analysis [13], comparative data base** cluster analysis [12] to be deployed in the context of **any time selected time period,** whereby said time period can refer to a sequence of time or series of previous studies (i.e. in terms of comparative reference data or comparative parameter configurations). Alternatively the study sequence analyser can be deployed in the context of mining data based on longer **trends such as changes in EMG amplitude during REM sleep** (i.e. applicable to sleep behaviour disorder; SBD) so that subtle increases in EMG amplitude which are heading or trending towards likely onset of Parkinson's symptom's (for example) can be identified at the earliest possible time in order to for medication or aversions of more severe Parkinson's onset.
***[9]* Automatic Movement/Nerve disorder Determination**
   i.e. incorporation of any of linear (i.e. spectral; regression; mean; area under curve and other analysis types), non-linear dynamic (i.e. entropy, Shannon), complexity, predictive) and/or statistical (i.e. skew, kurtosis) in order to distinguish a) tremor; b) shaking; c) bradykinesia (i.e. slowness of movement); and/or d) dyskinesia (i.e. diminished voluntary movements); versus artefact, background noise and other unwanted movement or motion related signals.
***[10]* Automatic Movement/ Nerve-disorder Detection.** i.e. accelerometer sensor to detect any of tremor; shaking; bradykinesia (i.e. slowness of movement); and/or dyskinesia (i.e. diminished voluntary movements); involuntary movements).
***[11]* Compute patient states including sleep/wake states (i.e. stages** including Stage W, Stage N1 (NREM 1), Stage N2 (NREM 2), Stage N3 (NREM 3), Stage R (REM)), **consciousness, sentient states, lucid states, associated transitions or other psychological states.**
***[12]* Comparative Data Base** -population normative -personalised
***[13]* Parameter Configuration**
***[14]* Spontaneous Response (SR) & Evoked Response (SR) Determination**
***[15]* Comparative Data Base Cluster Analysis**
   **TRACKING EVENTS OR HEALTH CONDITIONS OF INTERST**
***[16]* Tracking physiological processes;** health conditions, onset & incidence
***[17]* Parameter Configuration Cluster Analysis**
   - The present example further incorporates or enables automatic online diagnostic or prognostic determination of neural, nervous, sleep/wake, muscular, movement disorders whereby additional online or offline analysis can be conducted based on data-mining of any of currently monitored and previously monitored data in accordance to a subject/patient or health-workers authorised and specifically targeted health condition or events of interest events;

   - whereby said health conditions or events of interest can be monitored and analysed via one or more physiological signals (including but not limited to one or more **EEG** channels) which can be analysed in accordance to any of or any combination of **Parameter Configurations (****Figure 75****; [13]).**

Said **"Parameter configurations" (****Figure 75****; [13]) or "Comparative Data" Base (****Figure 75****; [12])** can include the detection or adjustment of parameters criteria to detect any of or any combination of parameters covered in this patent application document **(****Figure 75** **description)** or per sub-heading herein entitled: **Present Example of Comparative Data Base or Parameter Configuration Parameters.**

The **"Parameter configurations" (****Figure 75****; [13]) or "Comparative Data" Base (****Figure 75****; [12])** can be configured in terms of required online analysis and online event or health condition determination on an individual event or condition basis or in the context of a predefined cluster whereby any combination of different events or sequence/ensemble of different events can be tracked by the automatic analysis provision **(****Figure 75****; [16]).**
- The present example further incorporates or enables **EEG** monitoring of one or more **channels** with a patient/subject worn device with **online utilising multivariate or combinational analysis** comprising any sequence of combination of processing stages including Spatiotemporal dynamic neural source localisation analysis combined at least one of more other analysis processes including coherence, signal dynamic analysis (i.e. non-linear dynamic analysis including entropy, complexity analysis, etc.) entropy signal dynamics, spectral analysis (per **(****Figure 75****; description titled: Present Example of Comparative Data Base or Parameter Configuration Parameters.),** coherence, brain connectivity measures (i.e. Dipole analysis).

In one example spatiotemporal dynamics of subject/patients brain activation or deactivation can be tracked with ***minimal electrode or as few as 3 EEG*** electrodes (i.e. consumer or routine daily monitoring circumstances ) whereby broader trends such as shifts in neural energy can be tracked (including but not limited to) the ***brain lateralisation, anteriorisation, or hemispherical, hemispherical or front-real brain symmetry, etc.***
- The present example further incorporates or enables **EEG** monitoring of one or more channels with a patient/subject worn device with online ***cluster analysis* [15;17]** capability based on enabling a sequence or cluster of events or conditions to be characterised within a defined or dynamically determined time frame of interest. The said cluster analysis can be determined by way of **comparative** base-line states such as determined be a subject/patients personal data or broader based population normative or disease state data base references **[12]. Alternatively** the cluster analysis can be determined by way of parameter configurations **[17]** in accordance to (for example only) parameters or criteria defined here in under heading: " **Example of Comparative Data Base or Parameter Configuration Parameters".**
- The present example further incorporates or enables any of evoked responses and/or spontaneous events to be defined as a marker applicable to prognostic or diagnostic monitoring events or health conditions of interest.

- whereby ***evoked response*** can including any of events evoked via auditory, electrical, visual, pain, smell, or mental-process based stimuli. ***Alternatively spontaneous events*** such as (but not limited to) neural events including spindles spikes, HFOs, ripples, K-complexes, spectral bands of interest etc. can be characterised and tracked as markers of diagnostic or prognostic events or health conditions of interest **(Figure 75- [14];** **Figure 69****;****Figure 70****;** **Figure 71****).**

**HMS OUTCOME DETERMINATIONS AND INFORMATION ACCESS CAPABILITIES *Block [16]*** tracks events or health conditions of interest and ***Block [18]*** then analyses and determines when the properties of such events or conditions exceeds or complies (as the applicable case may be) with the established detection criteria (i.e. per **blocks [8]; [12], [13], [14] [15]; [16] and/or [17])** or predefined thresholds, working ranges or limits, in which case the respective system outputs are identified for further subject/patient or health carer notification or alert per **block [28].**

Accordingly, health management system (HMS) outcomes include any of or combination of the following outcomes as covered above in **blocks [10], [11], [18] and/or [24];**

In this way the tracking **[16]** and final diagnostic or prognostic determination of a subject/patient's health conditions or events of interest **(per criteria, database references or parameters outlined in Blocks [8], [9], [12], [13], [14], [15] and/or [17])** are determined **[block 26]** in order to enable notification or reporting to said subject/patient or associated health worker **(per block 28].**
- Whereby determination of the onset or incidence of "health conditions/disorders or events of interest", include enabling an "automated method of determining neurological prognosis or diagnosis" or personal-care management of a subject's a nervous system, muscular or movement system, and/or ***[18]*;**

***[19]* REM atonia determination:** All sleep states are analysed in terms of EMG means levels **(block [20]).** In circumstances where the MEG mean amplitude level exceeds the mean EMG measured during sleep states other than REM the percentage of this increase is noted (i.e. percentage of EMG amplitude exceeding the maximum average REM EMG level). If the percentage of increased EMG amplitude exceeds any previous mean REM EMG level **(i.e. does mean EMG amplitude exceed maximum EMG mean threshold for previous REM states; per block 21)** then the condition is flagged and determined **(per Block 26)** to be as "sleep behaviour disorder" (SBD).

If the flagged SBD is accompanied with a report verifying that no other condition related to increased EMG during REM stages (i.e. **restless leg syndrome, muscle pain** (i.e. myalgia), muscle processing disorders (i.e. fibromyalgia), nerve pain or other disorders known to increase EMG levels during REM sleep, then the condition will be flagged as **idiopathic SBD (per block [19], [22], [24])** and also noted as a potential symptom of Parkinson's onset. Similarly, if the said threshold is not exceeded **(per block [21])** then longer time base and data-mining analysis can still be applied in order to determine whether EMG amplitudes over a period of time or across a series or range of sleep studies appears to be positively trending towards such threshold levels (i.e. typically when EMG mean levels exceed the EMG men of other sleep stages by 10-20% (per **blocks [20], [23] and [21]),** then this conditions will also be flagged as a potential very early marker of ***Parkinson's onset*** or other muscle, nerve or neurological disorder incidence or onset.

***[20]* EMG level Determination:** for each sleep stage and/or active states versus non-active states (per worn or attached motion detection system such as accelerometer-based or other movement sensor-based system).

***[21]* REM sleep without atonia** - if so the degree of EMG mean above preceding mean EMG non-REM sleep stage levels is determined. i.e. percentage of REM mean EMG level above previous mean EMG sleep states. If the level of EMG is greater than threshold amount (i.e. typically set to 10-20% above preceding sleep state EMG mean levels)

### Idiopathic versus non-idiopathic RBD .

***[23]*** REM Itonia Threshold : EMG sleep behaviour disorder (SBD) threshold - i.e.10-20% REM EMG versus non-REM sleep stage EMG SBD determination versus other causes of REM sleep without atonia (such as restless leg syndrome and/or muscle pain (i.e. myalgia) or muscle processing disorders (i.e. fibromyalgia).

### Idiopathic REM Sleep Disorder Determination

***[25]*** The present example further comprising a range of compatible systems covered under the following sub-headings in this patent application document : 1)Ubiquitous Monitoring System Capabilities 2) Portable Ubiquitous Monitoring Device Capabilities
Determination of events of interest including wake or sleep period biomarkers applicable to onset or incidence of Parkinson, epilepsy, Depression, Autism, ADHD and/or ***"health conditions*** or symptoms ***of interest***" or "events of interest/EOI" (per "EOI DEFINITION" detailed in abbreviation table at rear of this document).

### Platform services, applications, devices, system

***[28]*** Message, Report, Event, Calendar, Alert, Notification. Information can be conveyed via simple user/subject wrist mounted bangle or smart-watch, or mobile wireless communication devices (i.e. **Figure 68****;** or more complex scientific, medical, or technical interfaces such as per ***Figure 45*****; *;*** ***Figure 56******;*** ***Figure 57******;*** ***Figure 58******;*** ***Figure 59******;*** ***Figure 60******;*** ***Figure 61; Figure 62******;*** ***Figure 63; Figure 64******Figure 65; Figure 66*****; *Figure 67******Figure 69*****;** ***Figure 70******;*** ***Figure 71******;*** ***Figure 72******;*** ***Figure 73******;*** ***Figure 74******)*.**

***Block [29]* Automatic analysis model Determination:** - The present example further incorporates or enables analysis capabilities in a manner whereby an automated algorithms incorporating a means (i.e. Use of **Monte-Carlo cross-analysis and Jack-knife analysis** techniques whereby a series or **recorded monitoring sessions or studies can be examined in terms of probability of determination** of defined or known outcomes (i.e. known seizure events or known periods of severe incidence of autism or other known events or health conditions of interest) based on a **minimal or maximal or moderate model** (set of monitored parameters or derived variables used to detect event or health condition or mental **neurological/psychological state of interest). Events of interest or analysis types** (or combination of analysis types) can include any of or any combination of analysis types and events covered in this Neurosleep patent application. In this manner the present example incorporates a **means of self- learning or fine-tuning of algorithm** by way of comparing **different models of monitored parameters with known diagnostic outcomes or events.**

Other defined or known outcomes including *"health conditions* or symptoms *of **interest"*** or "events of interest/EOI" (per "EOI DEFINITION" detailed in abbreviation table at rear of this document).

### [29] Automatic analysis model Determination: CONFIGURNG EVENTS OR HEALTH CONDITIONS TRACKING

### Present Example of Comparative Data Base or Parameter Configuration Parameters (per blocks [10], [11],18] and/or [24]; [16]; Blocks [8], [9], [12], [13], [14], [15] and/or [17];[28])

An ***example** of **the automatic** characterisation is presented here.*

**An example of the automatic online characterisation and associated diagnostic and prognostic tracking and detection capabilities** include any of or any combination of events or interest including infraslow, oscillation detection, gamma activity detection, Delta/slow-wave activity detection, Theta activity detection, phase spectral coupling factors, signal dynamics detection, artefact detection and reduction or eradication, physiological event detection, monitored signal baseline properties determination and threshold detection, detection, event detection, ECG event detection, health condition determination, health condition notification and multivariate analysis detection configuration as further outlined here under headings: **1.SPECTRAL BIOMARKER EVENTS, 2. SIGNAL DYNAMICS DETECTION** ; **3. ARTIFACT DETECTION AND REDUCTION OR ERADICATION** ; **4. PHYSIOLOGICAL EVENT DETECTION; 5. BASELINE DETECTION; 6. EVENT DETECTION; 7. ECG EVENT DETECTION; 8. HEALTH CONDITION DETEMINATION; 9. HEALTH CONDITION NOTIFICATION;** AND/OR 10. **MULTIVARIATE ANALYSIS DETECTION CONFIGURATION.**

### [29] Automatic analysis model Determination: 1.SPECTRAL BIOMARKER EVENTS:

### 1.1 SPECTRAGRAPH CONFIGURATION ⁶³

Delta wave - (0.1 - 3 Hz)
Theta wave - (4 - 7 Hz)
Alpha wave - (8 - 15 Hz)
Mu wave - (7.5 - 12.5 Hz)
SMR wave - (12.5 - 15.5 Hz)
Beta wave - (16 - 31 Hz)
Gamma wave - (32 - 100 Hz)

The **sensorimotor rhythm (SMR)** - oscillatory idle rhythm of synchronized electromagnetic brain activity. This signal appears in spindles in recordings of EEG, MEG, and ECoG over the sensorimotor cortex, and typically the frequency range is 13 to 15 Hz.

**Mu waves** are a sensorimotor rhythm, which is a synchronized pattern of electrical brain activity involving a large quantity of neurons. This signal is implicated with brain activity that controls voluntary movement, which typically appear in the range of 9-11 Hz is most prominent when the body is physically at rest. While the alpha wave occurs at a similar frequency over the resting visual cortex at the rear of the back of the head, the mu wave appears over the motor cortex, across a band between an individual's ears.

### 1.2 INFRASLOW ACTIVITY

- 1 or more events of this category can be configured for detection
- **Infraslow activity (ISA)** (i.e. typically <0.1 Hz or around this spectral region);

### 1.3 OSCILLATION DETECTION CONFIGURATION

1 or more events of this category can be configured for detection
- **High Frequency Oscillations (HFO)** (i.e. 70-250 Hz range and typically or around this spectral range); Ripples (100-200 Hz);
- Fast ripples (FRs: 250-600 Hz range; typically 250Hz to 500 Hz);
   Other cluster characteristics applicable to localizing one or more events relating to seizure onset zone (SOZ - correlate with underlying epileptogenicity or seizure event of interest);
- Selection of signal parameter settings based on 1) **Electrocorticography** (ECoG) or **intracranial EEG (iEEG);** 2) scalp **surface EEG electrodes;** 3) **EEG** cortical depth electrodes or depth microelectrodes;
- **Categorise above in terms of sleep states** (i.e. typically non-rem stage 1; non-REM stage 2; non-REM stage 3; REM sleep; wake; arousal);
- Detection and tracking of high-frequency activities including low-amplitude activities at or around spectral regions of 40 Hz (Alvarrado-Rojas et al., 2014) ⁶⁴
- Determination of HFO events of interest based on previous diagnostic studies and biomarker templates based on high-frequency activities, including any **fast ripples** at or around regions of 250-500 Hz;
- Determination of HFO events of interest based on previous diagnostic studies and biomarker templates based on high-frequency activities, including any **fast ripples** at or around spectral regions of 250-500, **associated with** previously determined the initiation of **epileptiform** potentials and seizures in individual's preceding temporal lobe and neocortical epilepsy or other relevant health condition determination diagnostic studies; ⁶⁵

### 1.4 GAMMA ACTIVITY DETECTION

- High-frequency activities, including **gamma oscillations** at 60-150 Hz; ⁶⁶
- **Spatially distributed** modulation of cortical high frequency oscillations in the gamma frequency band (40-120 Hz or around this spectral range); ⁶⁷

### 1.5 DELTA/SLOW WAVE ACTIVITY DETECTION

- Spatially **distributed** modulation of cortical frequency oscillations in the **delta/slow waves** band (0.5-3 Hz or around this spectral range); ⁶⁸

### 1.6 THETA ACTIVITY DETECTION

- Spatially **distributed** modulation of cortical frequency oscillations in the **theta** oscillations frequency band (4-8 Hz or around this spectral range);⁶⁹

### 1.7 PHASE OR SPECTRAL COUPLING FACTORS

- **Coupling between slow cortical** potentials and **high-frequency spectral activity** (including but not limited to HFO and HFO ripples or any other configuration criteria); ⁷⁰
- **Cross-frequency coupling or correlation or coherence** of **high-frequency** sub-bands in the gamma range to low-frequency; ⁷¹
- **Cross-frequency coupling or correlation or coherence** across the range of **high-frequency sub-bands to low-frequency** ;
- Detection of **interactions between the phase of low frequency rhythms** such as (but not limited to) **delta/slow waves** and theta frequency bands as they relate to the amplitude of different sub-bands of gamma frequency rhythms; ⁷²
- Prior analysis (to online monitoring of incidents or precursors to "events of interest") including examination of spatial fluctuations in the coupling phase of ensembles of neurological events;⁷³
- Cross-frequency coupling determination by way of statistical analysis of the interaction between different frequency bands; ⁷⁴
- Phase of a slow oscillation compared with fast oscillation the **amplitude;**⁷⁵
- Measurement of coupling between delta (0.5-3 Hz) or theta (3-8 Hz) for low frequency phases, and low gamma (LG: 40-70 Hz) or high gamma (HG: 70-140 Hz or 70-120 Hz) for high frequency amplitude envelopes; ⁷⁶
- Determination of said coupling whereby (but not limited to) raw signals can firstly be filtered in accordance to the frequency bands of interest, followed by extraction of phase and amplitude properties using Hilbert transform; ⁷⁷
- Instantaneous phase and instantaneous amplitude envelope values can be computed for delta bands and theta bands and for low gamma bands and high gamma bands.
- Sustained, repetitive transient increases in high gamma (in or nearby spectral regions of 80-150 Hz) amplitude phase-locked to the low-frequency (in or nearby spectral regions of 1-25 Hz) ictal rhythm,
   multi-unit firing bursts synchronized across the core territory of the seizure;
- Whereby said "events of interest" can include (but are not limited to) measures of cross-frequency coupling between spectral bands listed herein phase coupling between any "events of interest" and/or spectral events of interest" and/or biomarkers";
- Whereby said "data mining" algorithms can include (but is not limited to) the use of a preprocessing phase of analysis enabling pre-determination of said "data mining" algorithms in a manner whereby the ensemble of target "events" or "clusters" of interest can be can established based on prior data recordings or earlier monitoring data, including (but not limited to) a means of identifying multivariate data sets or characteristics "of interest".
- Whereby said "events of interest" can include (but are not limited to) measures of cross-frequency coupling between spectral bands listed herein and/or phase coupling between any spectral and/or biomarker events of interest;
- Whereby said "events of interest" can include (but are not limited to) measures of cross-frequency coupling between high gamma amplitude and low-frequency brain signal spectral bands such as 1-25Hz or around this spectral region;
- sustained high frequency oscillations (HFO) incorporating coherence or cross-frequency coupling between high gamma amplitude and low-frequencies (in or nearby spectral regions of 1-25 Hz); - Whereby said "events of interest" can include (but are not limited to) the decoupling between local hemodynamic and neural activities;
- Whereby said "events of interest" can include (but are not limited to) measures of tracking of decoupling between local hemodynamic and neural activities as a marker of preictal, interictal, postictal and ictal activity or "physiological "aura" associated with early ictal onset;

### [29] Automatic analysis model Determination: 2. SIGNAL DYNAMICS DETECTION

- Determination of biomarkers of interest can comprise of 2 processing stages comprising of the determination of a measures of non-linear dynamic signal assessment combined with another processing stage comprising of the determination of a measures of linear dynamic signal assessment in order to determine a final biological event or change of interest. For example, one or more **EEG** channels can be processed for changes in non-linear dynamic signal (i.e. entropy or complexity analysis) as the first step in and analysis process, followed by determination of spectral characteristics as part of a second process step. These process steps can be undertaken in the context of spatial dynamic source localisation or on the basis of analysing one or more **EEG** channels. [DB:14250;DB1Jly14]¹⁵ ;
- Determination of biomarkers of interest can comprise of 3 stages of the determination measures of non-linear dynamic signal assessment combined with another processing stage comprising of the determination of a measures of linear dynamic signal assessment and then a further stage of processing comprising of the determination of statistical measures in order to determine a final biological event or change of interest [DB:14250;DB1Jly14]¹⁵;

### [29] Automatic analysis model Determination: 3. ARTIFACT DETECTION AND REDUCTION OR ERADICATION

Eye movement or muscle artifacts reduction or elimination; ⁷⁸

### [29] Automatic analysis model Determination: 4. PHYSIOLOGICAL EVENT DETECTION

- Eye movement or muscle artifacts reduction or elimination; ⁷⁹

### [29] Automatic analysis model Determination: 5. BASELINE DETECTION

- 1 or more events of this category can be configured for detection
- 1 or more events of this category can be configured for detection
- Events of interest baseline shifts (IBS; or direct current shifts; 0-500uV); pre-event of interest baseline shifts (IBS; or direct current shifts; 0-500uV); conventional frequency activity (CFA - in order to help distinguish onset markers of events of interest);

### [29] Automatic analysis model Determination: 6. EVENT DETECTION

- 1 or more events of this category can be configured for detection
- **EEG** Spike detection;
- **EEG** spindle detection;

### [29] Automatic analysis model Determination: 7. ECG EVENT DETECTION

### [29] Automatic analysis model Determination: 8. HEALTH CONDITION DETEMINATION

- 1 or more health condition events of this category can be configured for detection
- Health condition events can be configured by selection any of or any combination of the above options in terms of very early onset, standard onset and incident of health condition event of interest.
   - Programmable health conditions can include *"health conditions* or symptoms of ***interest"*** or "events of interest/EOI" (per "EOI DEFINITION" detailed in abbreviation table at rear of this document).

### [29] Automatic analysis model Determination: 9. HEALTH CONDITION NOTIFICATION

- Notification by cellular phone, 3^{rd} party intervention, register update, report update, GP email/SMS/phone message; specialist GP email/SMS/phone message; family member GP email/SMS/phone message; nominated health carer; nominated points of contact GP email/SMS/phone message; ambulance service GP email/SMS/phone message; paramedics service GP email/SMS/phone message; smart watch 3^{rd} party phone intercom call help;

### [29] Automatic analysis model Determination: 10. MULTIVARIATE ANALYSIS DETECTION CONFIGURATION

- Selection of any of or any combination of above parameters and/or additional parameters can be selected and weighed using above configurations or separately configured parameters, in order to define combinational analysis events of interest determination.

Frequency bands of interest can be derived by way of 8-order Butterworth forward-backward IIR filters (avoid phase delays and improve frequency selectivity); ⁸⁰

**Data acquisition** sampling rate s should preferably be greater than 2000 samples per second and signal to noise monitoring capabilities should be capable of distinguishing the smallest possible HFOs, which can be as few of a few microvolts amplitude or less.

**Figure 76** eLifeMEDICS & eHealthMEDICS HEALTH MANAGEMENT SYSTEM (HMS). Highly secure and private data gateway with object layers capable of interfacing disparate information and monitoring systems while segregating clinical information and data from private information and encompassing private key and data segregation techniques designed to mitigate hacking or other security risk factors.
***[1]*** HMS sensor or monitor device/system (SOMDI) interface
***[2]*** HMS information interface (i.e. WAN, LAN, Cloud-computing services, NAS, peer to peer etc.)
***[3]*** Disparate SOMDI sensor and/or monitoring clinical/patient information system(s) or application(s)
***[4]*** Disparate SOMDI sensor and/or monitoring consumer/subject information system(s) or application(s) disparate medical monitoring clinical/consumer information system or application(s)
***[5]*** Physical and/or data conversion to standardised HMS compatible format (wireless or cable)
***[6]*** Security, privacy, authenticity firewall and interrogation layer interface/adaptor (wireless or cable)
***[7]*** Universal SOMDI data interface (software) object
***[8]*** MMT; APM, HDCM, eHealthATLAS, eHealthHDCM, eHealthCAPA, eHealthlOT, on-line dynamic data exchange; online data sychronisation system (i.e. Somfit sleep & health tracking); analysis and full-disclosure on-line analysis & monitoring/sensing (FDOA&M) system
***[9]*** AMD System: Regulatory and/or Medical Body Compliance Study Management: Conversion of standard and recommendations or requirements into a standardised format accessible by one or more patient/subject information exchange, patient record and information system, GP information systems, retrieval, repositories, REQUIREMENTS or regulatory International Academies, Associations, Government health insurance entities, private health insurance entities, regulatory and other distinguished bodies recommendations or standards in terms of patient information, diagnostic, prognostic, monitoring, security, information standards, information privacy - i.e. ASSM SCOPER; ASSM sleep study types 1 - iv; ASA sleep study levels 1 to 4; Medicare minimal requirements for each study type; Medicade sleep study requirements etc.
***[10]*** Automatic Mode Determination (AMD) System : Prognostic and/or Diagnostic Data Mining Authorisation & Program Implementation (i.e. registration, security authentication, clinical script, medical or health-carer or health organisation authorisation and coverage program)
***[11]*** Offline or online data mining diagnosis or prognosis - (i.e. any analysis examples, objectives or present patent capabilities covered in this patent application or accompanying patent (i.e. eHealthNEURO & eHealthSLEEP folios or earlier eHealthNAS&ATLAS patent folios (Burton 2014b))
***[12]*** Routine maintenance and independent security-barrier infringement enabling ongoing automatic and independent testing of system security, infringement or infringement attempts
***[13]*** Biometric authentication and clinical versus patient information data segmentation/layering (i.e. incorporating any of : a) means of accessing information via syntactic data representation (i.e. data pointers devoid of revealing private or confidential information); b) incorporating means of also means of separating (i.e. any of or combination of physical location and/or data location and/or geographical location) any of or any combination of the storage or location of data associated with clinical images and/or clinical data and/or physiological data and/or clinical diagnostic outcomes and/or prognostic outcomes and or patient information and/or patient history or other personal or confidential information. - The present example further incorporates or enables any of or any combination of data types (i.e. individual's physiological records, monitored data, medical information or data, patient information, patient medical history and or any of information relating to an individual) and or storage location of any of these data types to be stored separately in terms of physical location and/or computer and/or storage drive or computer location and also storage-wise separately to enhance data security or privacy. i.e. data types can be segmented, separated and/or layered in manner to minimise unauthorised access. The present example prevents of minimises the probability of any identifier information relating to an individual to be associated with corresponding medical information or data relating to the said individual. c) incorporation of biometrics data security with option of separating clinical data from patient information data (i.e. including patents (Burton 2005;Burton 2010;Burton 2013a).
***[14]*** Syntactic or data pointer management/finder system repository
***[15]*** Data identifier "zoo" (finder) and cross-conversions system. This system is capable of handling international eHealth record systems with required regulatory and government standards (or private or proprietary standards - i.e. private health insurers) applicable to varied and disparate subject/patient and data records, systems and other data or information
***[16]*** Patient/subject/consumer information data repository
***[17]*** Patient/subject/consumer imaging data repository
***[18]*** Patient/subject/consumer physiological and other clinical data repository

### PATENT TITLE: PARKINSON & OTHER NEUROLOGICAL, NERVE & MUSCLULAR DISORDER HEALTH management system

### PATENT DECRIPTION

### ABSTRACT

- While conventional health trackers incorporating motion sensors can be helpful in terms of managing medication and detecting the incidence of degenerative disorders such as Parkinson's, early onset detection of this disorder can contribute to early intervention including drug therapy which may ultimately delay or even prevent the onset of more advanced phases of Parkinsonism or dementia. - The present example further incorporates or enables or contributes to the determination of early onset detection and treatment of Parkinson. In particular, the present example incorporates a unique patient worn, wireless monitoring system capable of non-obtrusive, continuous routine monitoring and associated analysis techniques capable of early Parkinson's symptoms determination, including 'idiopathic' rapid eye movement (REM or dream sleep) behaviour disorder (RBD) combined with prominent motor activity as an early marker of Parkinson onset.

### Key features include:

- Patient worn, wireless monitoring system capable of non-obtrusive, continuous routine monitoring incorporating any combination of "sleep monitoring" and "motion" parameters
- Means of automatic "determination" of rem behaviour disorder, along with associated "context analysis" capable of deriving subtle early warning Parkinson's symptoms
- Means of utilising said "determinations" and context analysis as part of therapeutic decision, delivery, management or administration process
- *The nub **of the present*** example is to enable consumer-level or nightly tracking of RBD at a diagnostic level, which is capable of detecting other potential causes (such as restless leg syndrome) in order to provide a useful method and device for the *tracking of idiopathic SDB* applicable to the detection of early onset Parkinson's.
- The present example further incorporates or enables consecutive *RBD determination* in order to detect the *earliest possible trends or onset of RBD* by way of tracking a progressive or overall increase over 2 or more nights, as it relates to EMG level during REM stages, applicable to RBD.
- *The present* example *has incorporates* a means of *detecting EMG levels on* a *sleep study by sleep* study and also a sleep stage by sleep stage basis in order to detect changes in EMG applicable to idiopathic SBD (marker of early onset Parkinson's.
- Whereby said means of *detecting EMG level changes applicable to idiopathic SBD* (marker of early onset Parkinson's) can include (but is not limited to) determination of subtle increases of EMG levels during REM sleep compared other sleep stages;
- Whereby said means of *detecting EMG level changes* applicable to *idiopathic SBD* (marker of early onset Parkinson's) can include (but is not limited to) determination of subtle increases of EMG levels during REM sleep based on longer term *trend analysis of graduated SBD REM stage* EMG level's;
- Whereby said means of detecting *EMG level changes* applicable to *idiopathic SBD* (marker of early onset Parkinson's) can include (but is not limited to) determination of *idiopathic SBD;*
- Whereby said means of detecting *EMG level changes* applicable to idiopathic SBD (marker of early onset Parkinson's) can include (but is not limited to) includes analysis of *causations of RBD;*
- Whereby said means of *detecting EMG level changes* applicable to idiopathic SBD (marker of early onset Parkinson's) can include (but is not limited to) includes analysis of causations of RBD such as (but not limited to) *restless leg syndrome ;*
- The eHealthMEDICS PMS system can be discretely incorporated as a patient worn method or within the thin or "distributed sensor" (DS-eLifeWATCH) or the "integral-sensor" (IS-eLifeWATCH) range of health intelligent watch devices, coupled to a network application services (NAS) or other wireless communication or cloud-computing services to provide a number of ubiquitous health services.
- *One example of the **eHealthMEDICS** platform* with professional and personalised health solutions is to enable tracking of the onset or *incidence of neuro, nerve or muscular disorders* such as Parkinson's and Alzheimer's;
- *REM sleep behaviour disorder (RBD)* is a parasomnia and is a characterised by the loss of normal *skeletal muscle atonia* during REM sleep with prominent motor activity accompanying dreaming. Patients with 'idiopathic' RBD have been shown to exhibit an early clinical manifestation of an evolving neurodegenerative disorder;
- Therefore patients exhibiting *'idiopathic' RBD with prominent motor activity* as a *determinant of subject*/*consumer* as *a potential candidate (or biofeedback **marker;** medication **marker;** drug administration; drug disperser system; marker etc.) in order to enable control or information associated with early drug therapy* such as those effecting synuclein pathophysiology, in order to *delay or prevent the onset of neurodegenerative* disorders such as parkinsonism or dementia ⁸¹;
- The present example further incorporates or enables a personalised smart health watch system to enable routine, home-based determination and tracking of REM sleep behaviour disorder (RBD) by way of monitoring and scoring sleep stages, and in particular *REM sleep, accompanied with prominent motor activity,* whereby the sleep parameters including any combination of **EEG,** EOG and EMG signals can be monitored using one or more wireless sensors;
- The *present* example *provides reporting* capabilities enabling an individual to readily access their *REM sleep periods associated* with *tremor or vibration analysis* in order to track individual indices, occurrences, trends applicable to early onset or the incidence of neurodegenerative disorders such as *parkinsonism or dementia;*
- The present example further incorporates incorporate a sensor capable of detecting fine tremor movements ranging from cardio *ballistogram or tremors* to courser vibrations or movements. Such a sensor can comprise of any membrane or sensor capable of detecting movement or motion and generating a signal or measure associated with said "movement or motion";
- The present example further incorporates or enables access to said information related to *"signal or measure associated with said movement or motion"* in order to generate directly access "information" or further "transformations or transpositions *(linear or non-linear)"* of said "information" in order to associate such "information" with sleep states, including REM sleep state in order to identify possible phases of physiologically generated tremor associated with incidences of rem behaviour disorder.
- The present example further incorporates or enables said information relating to "signal or measure associated with *said movement or motion" or RBD* in the decision process or control process applicable to optimise the administration of appropriate drug therapy in order to achieve predetermined outcomes is terms of any of or any combination of minimising RBD and/or tremor conditions during pre-specified sleep or wake states.

*The present* example also provides programmable watch-face applications and configurable displays and alerts whereby users, for example, can display in numeric, graphic or tabular form and can also be alerted to response responses of interest or concern as it relates to excessive or incidence of idiopathic' RBD.

*The present* example enables automatic analysis, display, alert, reporting, report distribution or associated notifications to predetermined destinations based on *correlation of RBD with prominent motor activity* (i.e. walking fluidity and synchronisation between arms and leg movements (detectable via accelerometer or other movements tracking of legs and arms by way of spectral and other forms of analysis);

*The nub of the present* example is to enable the monitoring of rapid eye movement (REM) sleep by way of monitoring direct sleep parameters (i.e. **EEG,** EMG, EOG) or surrogate measures of muscle, brain and eye activity in conjunction with graded measures ranging from fine tremor to courser vibration during dream (REM) sleep, in order to diagnose the potential incidence or onset of at least one of neurological, nervous system, or muscular degenerative disorders.
- The present example further incorporates or enables a means of stepping through *ranges, thresholds and* grades of the said *fine tremor to courser vibration measures,* in order to provide subtle detection of even the most subtle and irregular/spontaneously occurring incidences of *simultaneous tremor an RBD,* as a means of enabling either personalised and private detection of the onset of at least of neurological, nervous system, *or muscular degenerative disorders.*
   *The present* example incorporates a means of *automatically or via computer assistance adjust ranges or threshold levels* of fine tremor to courser or more prominent motor activity in accordance to the use of both linear (i.e. spectral) or non-linear (i.e. entropy/complexity analysis) or combined approaches (.e spectral entropy) in order to *elicit from the monitored data underlying or otherwise indistinguishable signs of degenerative neural, nerve or muscular disorders* otherwise concealed due to the irregularity, or subtle appearance but life changing events (i.e. early treatment can save or revolutionise life health and quality).
- The present example further incorporates or enables automatic control or drug usage or enable *automatic medication administration* via drug delivery systems or drug dispenser access and indications in such a manner that the *optimal treatment can be achieved;*
- The present example further incorporates or enables the provision of automatic *earliest automatic notification or indications relating to automatic determination of events or health conditions of interest;*
- The present example further incorporates or enables the provision of automatic or *opted medical review* (i.e. subject can opt in or authorise access via medical oversight or health-care services) or second medical opinions as required, *relating to automatic determination of events or health conditions of interest;*
- The present example further incorporates or enables the provision of automatic intervention if required;
- The present example further incorporates or enables the provision of automatic intervention including automatic communication via a smart watch or other phone interface (i.e. GSM) capabilities relating to prevention of severity or high risk incident corresponding to *events or health conditions of concern (i.e. seizure);*

- The present example further incorporates or enables a means of enabling a ubiquitous health capabilities incorporating "system information integration means" (i.e. any of or any combination of monitoring, analysing, storing, reporting, indications, notifications, messaging, alerts, locality or geographic mapping or routing dynamic information exchange and/or calendar or other scheduling application dynamic information exchange interface) and elastic or expandable resources capable of being allocated in order to preserving means of uninterrupted, continuous and "deterministic data monitoring means", "monitoring means" (i.e. means including monitoring of any of or any combination of physiological, psychological, pathological states of a person to enable diagnostic and/or prognostic characterisation and pathological determination of disorders, diseases or other events of investigational interest or health concern, as well as sensing and monitoring of any environmental factors), the present example further comprising any of or any combination of:
- The present example further comprising of a "system information integration means" including (but not limited to) any of or any combination of monitoring, analysing, storing, reporting, indications, notifications, messaging, alerts, locality or geographic mapping or routing dynamic information exchange and/or calendar or other scheduling application dynamic information exchange interface;
- The present example further comprising of "monitoring means" including (but not limited to) any of or any combination of monitoring of any of or any combination of physiological, psychological, pathological states of a person to enable diagnostic and/or prognostic characterisation and pathological determination of disorders, diseases or other events of investigational interest or health concern, as well as sensing and monitoring of any environmental factors via a subject-wearable or other device in subject proximity providing sensing, monitoring, information access, indications or other said "system information integration means";
- The present example further comprising "deterministic data monitoring **means",**
   Including a series of examplescovered in this document or other patent applications as outlined here under subheadings "Ubiquitous Monitoring System Capabilities" and "Ubiquitous Monitoring Capabilities" the present example further comprising any of or any combination of:

### Ubiquitous Monitoring System Capabilities

- Automatic mode determination (AMD) system (Figure **83****);**¹⁰
- Adaptive Physiological-Body Network (APM) & NAS Gateway;
- Online Automatic Operator Complexity Level (OCL) system; ¹⁰
- *Remote surveillance monitoring, emergency surveillance and interventional monitoring* or support (ambulance, carers, paramedics, RACV or the like);

### Portable Ubiquitous Monitoring Device Capabilities

- Integrated sensor attachment (ISA) incorporating concertina expansion system; ¹⁰
- External Noise Sensing and Cancellation System; ¹⁰
- Pressure sensitive electrode activation (PSEA);¹⁰
- Online Automatic Signal Quality Estimation System (SQE) system;¹⁰
- Online Automatic Redundant Electrode Substitution (RES) System;
- Online Automatic Automatic Identification and Channel Characterisation (AICC) System;¹⁰
- Online Automatic Dynamically Linked Signal Conditioning (DLSC) System; ¹⁰
- Online Automatic Dynamically Linked Analysis Conditioning (DLAC) System;¹⁰
- Multipoint time-synchronisation monitoring (MTM);
- Adaptive physiological-body monitoring (APM) system;
   The present example further comprising "monitoring means" including (but not limited to) physiological or environmental monitoring;
   The present example further comprising "monitoring means" including (but not limited to) local device(s), network-based, community networks, wider networks (i.e. LAN, WAN etc.) or sharing/accessing resources applicable to local monitoring devices or systems as well devices or systems which are part of *defined* (i.e. distinguished in terms of privacy and security authorised access and also interconnectivity and communication compatibility) monitoring group of devices or systems.

- Where said **"sharing/accessing** resources" includes any local other system resources (such as data-acquisition storage, buffer or caching memory, wireless connectivity pathways or rerouting to overcome reception transmission constraints), in order to achieve the required online monitoring "minimal criteria";
- Where said "minimal criteria" includes both accessing and sharing resources with other systems or devices identified as forming part of a private-subject or wider network or community network, configured in accordance to data bandwidth requirements, data priority and predefined continuous monitoring/uninterrupted online monitoring or data reconstitution synchronisation, delay and deterministic criteria and associated local and network system requirements and availability at any point in time;

The present example further incorporates or enables enable automatic detection of REM sleep behaviour disorder (RBD) defined by the incidence of *REM sleep without atonia (suppressed muscle **tone),*** as applicable to determination of the incident or onset of disorders or adverse health sequelae *"health conditions* or symptoms *of interest" or* "events of interest/EOI" (per "EOI DEFINITION" detailed in abbreviation table at rear of this document), the present example comprising any of or any combination of (but not limited to):
Whereby said "events of interest" can include" (but are not limited to) automatic online and/or real-time detection or determination of precursors of Parkinson's disease including (but not limited to) "automatic detection of REM sleep behaviour disorder (RBD) defined by the incidence of REM sleep without atonia (suppressed muscle tone),
- Whereby said "REM sleep without atonia" can be defined by comparing (electromyography) EMG signals indicative of muscle tonicity during non-rapid eye movement (REM) sleep stages and REM sleep stages, in order to determine periods when the normal reduction of EMG activity during REM sleep is not evident and the EMG characteristic associated with non-REM versus REM sleep EMG comply with pre-determined ranges, thresholds and variances. For example, where REM EMG periods or segments exceed a certain amplitude increase compared to non-REM EMG amplitude outcomes.
- Whereby said "REM sleep without atonia" can be detected by way of monitoring any combination of parameters including motion sensors, EMG signal monitoring, other sleep parameters;
- Whereby said automatic detection of REM sleep behaviour disorder (RBD) refers to monitoring, detection and/or recording of motion and/or sleep parameter data to assist doctors or individuals to diagnosis and/or treat and/or automatically medicate and/or assist manual medication and/or assist medication administration or dispensation applicable to "movement disorders", including any combination of Parkinsonian Tremor, Restless Legs Syndrome, Dystonia, Wilson's Disease and/or Huntington's disease;
- whereby said movement disorders includes (but are not limited to) motion detection analysis capable of detecting the incidence of or the precursors to movements associated with key disabling Parkinson's disease symptoms, including bradykinesia (i.e. slowness of movement) and dyskinesia (i.e. diminished voluntary movements; involuntary movements).
   - - The present example further incorporates or enables linkage or interface to medication dispenser or vending system (i.e. search wireless drug management or dispenser system and/or medication vending system or administration system and/or other therapeutic treatment
   - - The present example further incorporates or enables \combinations of idiopathic SBD detection and motion detection of tremor, and other movements associated with key disabling Parkinson's disease symptoms, including bradykinesia (i.e. slowness of movement) and dyskinesia (i.e. diminished voluntary movements; involuntary movements) to be detected as markers for early onset or the incidence of Parkinson's;
   -- The present example further incorporates or enables said motion or idiopathic SBD markers to be incorporated as part of a medication notification or indication system;
   -- The present example further incorporates or enables said motion or idiopathic SBD markers to be incorporated in the determination of therapeutic control, including deep brain stimulation;
   -- The present example further incorporates or enables said motion or idiopathic SBD markers to be incorporated in the determination of therapeutic control, including magnetic brain stimulation;

### Movement, neurological or nervous disorder via automatic notification and/or video segment transmission of interest

A further example is to *enable automatic motion detection characteristics indicative of the onset or the incidence* of seizures or other movement or neurological disorders, based on any *combination or combinations of video **monitoring** or motion detection* (i.e. accelerometer sensor monitoring).
- whereby any combination or combinations of said motion and/or movement and/or other events of interest (EOI per glossary) can be analysed in terms of determination of configuring the analysis to track for said characteristics applicable to neurological motion characteristics applicable to the onset or incidence of epilepsy seizures and/or Parkinson's (i.e. Parkinson's-SBD; Parkinson's disorders, including bradykinesia (i.e. slowness of movement) and support services for dyskinesia (i.e. diminished voluntary movements; involuntary movements), or other neurological or nervous system disorders.

- A further example is to automatically send a notification (i.e. message, email, etc.) and/or *transmit video **segment*** (i.e. email to selected individual or individuals) or photo of the subject being monitored for movement of neurological or nervous disorders.

### PATENT TITLE: AUTISM SPECTRUM DISORDERS (ASD); ASPERGERS (ASP); DYSLEXIA;

### BACKGROUND (i.e. ASD, ASP and other neurodevelopmental or neurological disorders)

In recent times the prevalence of autism amongst children has been reported to have increased to 1 in 100 children.

The question as to whether Asperger's Syndrome (ASP) should be defined as a segment of overall Autism Spectrum Disorders (ASD) remains a subject of continued research. Moreover, the increasing prevalence of ASD and the need to enable non-invasive means of monitoring such as **EEG,** along with the capability of distinguishing neurophysiological variances between ASD and ASP remain a major prior art enigma.

It has been reported that children with autism spectrum disorders (ASD) without seizures exhibit a higher rate (10-50% higher) of epileptiform EEGs than individuals without ASD.

ASD is a neurodevelopmental disorder and the etiology remains an enigma. The behavioural dysfunction associated with this disorder are characterised by social and communication deficits along with the presence of restricted interests or repetitive behaviours. Amongst the ASD population group an increased prevalence of approximately 5% to 46% has been reported.
27
- The present example further incorporates or enables monitoring two or more **EEG** (scalp monitored electrical brain signals) and automatically deploy a means of analysing said signals in order to improve the accuracy of ***estimating the** underlying **regions** of the brain **generating** such **sources;***
   Whereby one means of improving the accuracy of ***"estimating the** underlying **regions** of the brain* ***generating such* sources"** includes the incorporation of ***clustering algorithm(s)*** enabling diagnostic and/or prognostic signal characterisation specific to an individual/patient/subject. Means of ***"estimating the** underlying **regions** of the brain **generating such sources"*** can further incorporate a process whereby an individual's monitored physiological data (including but not limited to ***"aura"*** conditions (outlined elsewhere in this patent application document) determination of ***"events** of **interest"*** and/or *"health conditions of **interest"*** can include defining or identifying (automatically or via expert or other interpretation assistance and/or validation or verification).
- The present example further incorporates or comprises of characterisation of an individual's ***"events** of **interest"*** and/or *"health conditions of **interest"*** can include a process where statistical analysis techniques (such as incorporation of *cross-validation, Monte-Carlo, Jackknife, factors* analysis determination, support vectors machine (SVM), deployed in either online or offline data-mining context to establish most accurate ***clustering algorithm comprising*** of most effective or precise detection of said events based on independent or automatic end-point events or health condition event determination of specific individual/patient/subject monitored (i.e. signal or multiple trail data of specific subject/patient/individual based on source estimations and a ***range** of analysis processes* (see examples per non-linear, **stochastic,** deterministic etc. (i.e. per any of or any combination of analysis formats, types variations, expert system, classification methods outlined in **Figure 80** ), *cluster **algorithm** determination* incorporation on and offline aspects including (but not limited to) such as incorporation of *cross-validation, **Monte-Carlo, Jackknife,** factors* analysis determination, or support vectors machine (SVM).
   Whereby ***clustered algorithms** can include **any** of or **any** combination of* signal dynamic transfer (non-linear dynamic analysis such as, but not limited to, complexity or entropy analysis), frequency domain analysis; coherence, correlation, Dipole analysis or other analysis indicative of brain connectivity; statistical analysis (such as, but not limited to):
   ***Whereby** cluster analysis composition **and** associated analysis processes* including any of or any combination of analyses:
   - stochastic spectral analysis comprising any of or any combination of stochastic, stationary, linear, non-linear (including non-linear dynamical analysis) types;
   - deterministic spectral analysis comprising any of or any combination of stationary, linear, non-linear (including non-linear dynamical analysis) types;
   - linear;
   - non-linear (including non-linear-dynamic analysis types such as but not limited to entropy, complexity analysis etc.);
   - stochastic;
   - deterministic;
   - stationary;
   - coherence;
   - pattern recognition including any of or any combination of linear, non-linear (including non-linear, dynamical analysis) types, stochastic, deterministic and/or stationary types;
   - Wigner transform (time-frequency analysis) including any of or any combination of linear, non-linear (including non-linear, dynamical analysis) types, stochastic, deterministic and/or stationary types;
   - wavelet composition including any of or any combination of linear, non-linear (including non-linear, dynamical analysis) types, stochastic, deterministic and/or stationary types;
   - statistical analysis;
      82 83
- The present example further incorporates or enables a means of *automatic* ***and*/*****or** computer-assisted* ***and*/*****or** online* ***and*/*****or** offline* ***and*/*****or** Data-mined **ASP** versus **ASD*** biomarker characterisation of signals and diagnostic or prognostic determination of "events of interest" or health conditions of interest, t the present example further **comprising of any of or any** combination **of the following** characterisation **or determination analyses:**
   Automatic PCA Analysis
      - *a first process* comprising of data reduction by way of focussing on most significant *Principal Component **Analysis (PCA)** outcomes* (i.e. highest signal to noise waveform outcomes) in order to generate a smaller data set based on indiscriminate nature of PCA output generation of coherence factors.
      - a ***second process*** comprising of the determination of data results derived by way of computing coherence outputs (frequency bands, associated coherence, for all electrode combinations, for a range of parameter variations such as but not limited neural source reconstruction and/or associated coherence measures; per any of or combination of parameters per *Figure **64******;*** *Figure **65******;*** *Figure **67******;*** *Figure* **69****)** from the complete range of **EEG** monitoring electrode locations .
   Automatic online discriminant function analysis (DFA)
      - *a third process in **order** to* review source dimensionality and ***reduce** the complexity or **number** of potential source locations* (i.e. improve identification precision of neural source localisations) probabilities comprising of identifying the most accurate factors found (i.e. based on defining validated ASD and/or ASP events periods of enabling reliable and significant differentiation between typical neurophysiological data of normal subjects/patient's (controls) versus from ASD subjects/patients by way of computing associated discriminant function analysis (DFA) outcomes.
   Automatic online Principal Component Analysis (PCA) and Independent Component Analysis (ICA)
      - *an optional fourth process in order to* review source dimensionality and *reduce the complexity or number of potential source locations* (i.e. improve identification precision of neural source localisations)
      comprising of deploying *PCA can be applied in the first place to identify signals with higher* SNR, followed by *Independent Component Analysis (ICA)* as a means of increasing accuracy of neural source localised Dipole localisation (dimensionality) determination and/or coherence determination;
   Online Source Localisation Analysis
      - means of Improving current density reconstruction (CDR; i.e. **EEG** source localisation) and Dipole Localisation/Dimensionality by way of implementing a first process of principal component analysis (PCA) followed by a second process of independent component analysis (ICA) followed by a third process comprising of CDR **EEG** source localisation;
   One technique designed to improve the CDR
      - means of providing blind source separation determination in cases where there are more sensors than available source signals whereby such mean can comprise of (but not limited to) undertaking a two stage process comprising firstly of applying Principal Component Analysis (PCA) followed by the application of Independent Component Analysis (ICA) of brain activation applicable to the incidence or precursors of disorders or other health sequelae including *"health conditions* or symptoms *of interest" or* "events of interest/EOI" (per "EOI DEFINITION" detailed in abbreviation table at rear of this document);
      - the incorporation of a PCA analysis stage is to increase the input SNR of the subsequent ICA stage, thereby reducing the sensor dimensionality;
      - whereby the ICA processing stage is used to separate the remaining composite signals into their respective independent components;
      - whereby signals with a signal to noise ratio of greater than one (>1 SNR) can be segregated within a more defined space using PCA in the first place before further specifying the number of signals of interest as part of the ICA process;: ^{32, 84}
         Establish wake/hypnogram and online CDR; COH tracking
      - the provision to analyse sleep monitored parameters (such as any of or any combination of **EEG,** EMG, EOG signals) in order to compute a sleep hypnogram;
      - the provision to undertake sleep or wake monitoring whereby an auditory stimulus is presented to subject/patient (such as but not limited to 7Hz binaural auditory stimulus);
      - the provision to monitor and determine AEP responses of subject/patient;
      - the provision to compute running average of a defined number of past AEP responses or a number of past AEP responses in order to compute a pre0defined minimal signal to noise of said AEP determination;
      - the provision to enable determination of AEP averages in order to compare, contrast and interpret SLEEP and WAKE GM average outcomes and differentiation of these wake and sleep AEP signal determinations;
      - the provision to utilise standardised (Colin; Montreal Institute) averaged MRI 3D brain image and segment out brain-stem and cerebellum regions, in order to converge actual f**EEG** outcomes to enable accentuation of deep-cortical brain views of interest;
      - the provision to utilise sLORETA current density reconstruction (CRD) with associated source coherence (COH) corresponding for each respective 3-min GM average of the SLEEP and WAKE period of interest;
      - the provision to analyse the activation of SLEEP (stage 2) versus WAKE as it relates to brain activation connectivity, coherence, networking and integration characteristics associated with both cortical (CDR; distributed) and also dipole (localised) coherence activation;
      - the provision to compare, contrast and interpret SLEEP versus WAKE CDR and COH outcomes; ³²
   Automatic Online Coherence (COH) Analysis
      - *A processing step based on utilisation* of *previously determined optimal diagnostic classifier(s)* determined via analysis of one or more of a specific subject/patient/individual's study (monitored **EEG)** along with related *EEG spectral coherence data* computed to provide the most precise classification outcomes (applicable to "events of interest" and/or "health conditions of interest");
      - whereby *greater definition of the source dimensionality* using *two stage approaches such* as *PCA followed by ICA* can be deployed any stage in the overall analysis processing as a means of *improving the dipole source localisation accuracy and associated connectivity;*
      - a process comprising of utilising any of *Discriminate Function analysis (DFA)* based on predetermined *EEG coherence based DFA rules* as part of *diagnosis and*/*or prognosis process* in order to characterise and determine classification of subjects in accordance to *ASD and*/*or ASP neurological disorder categories;*
      - whereby any of said characterisation and/or "events of interest" or "health conditions of interest" can be deployed by way of determination of reduced or increased coherence on a brain hemispherical symmetry or connectivity basis (i.e. *left hemisphere increased or decreased discernible activity compared to right hemisphere (i.e. lateralisation) and visa-versa);*
      - whereby any of said characterisation and/or "events of interest" or "health conditions of interest" can be deployed by way of determination of reduced or increased coherence on a brain anterior (front) to posterior (rear) brain energy or connectivity symmetry basis (i.e. rear of brain *increased or decreased discernible activity compared to front and visa-versa, including anteriorisation);*
      - whereby any of said characterisation and/or "events of interest" or "health conditions of interest" can be deployed by way of determination of reduced or increased coherence on a brain top (superior/dorsal) to bottom (inferior/ventral) brain energy or connectivity symmetry basis (i.e. rear of brain *increased or decreased discernible activity compared to front and visa-versa);*
      - whereby any of said characterisation and/or "events of interest" or "health conditions of interest" can be deployed by way of determination of *reduced or increased spectral coherence;*
      - whereby any of said characterisation and/or "events of interest" or "health conditions of interest" can be deployed by way of determination of reduced or *increased signal dynamic analysis* (non-linear dynamic analysis including (but not limited to) entropy and/or complexity analysis);
      - Whereby prognostic or diagnostic "events of interest" or "health conditions of interest" can be signal characterisation and/or determination applicable to any of or any combination of nerve disorders, muscle disorders, and/or *"health conditions* or symptoms *of interest "* or "events of interest/EOI" (per "EOI DEFINITION" detailed in abbreviation table at rear of this document).

### ²⁸ A stable pattern of EEG spectral coherence distinguishes children with autism from neuro-typical controls - a large case control study 84 27 ²⁹

### IED tracking

- The present example further comprising of the capability to *monitor at least 1or more channels of EEG* and track *preictal* or *interictal or postictal or ictal epileptiform discharges (IEDs)* with the further option of enabling hemispherical symmetry EEG characterisation (with plurality of EEG channel monitoring) via a patient/subject wearable EEG sensor and/ or monitoring device suitable for overnight and daytime studies as a prognostic or diagnostic marker of autism spectrum and other neurological, nervous system, muscular, movement disorders;

### IEDs Tracking

- a first process of comprising of the characterisation of one or more monitored EEG signals in terms of the detection of determination of *preictal* or *interictal or postictal or ictal epileptiform discharges (IEDs);*
- a *second process* comprising of the determination of sleep **stages** including wake, REM sleep and non-REM sleep;
- a third process comprising of the determination of an *index based* on the determination of number IEDs in accordance to the *classification of stage stages* (i.e. NREM versus REM stages);
- a third process comprising of the determination of an *index based* on any of or combination of:
   - the number of ***IED's*** for each period (i.e. 30 min period or 1hr period etc.) a monitored individual's *sleep stages;*
   - the number of ***IEDs_***for each period (i.e. 30 min period or 1hr period etc.) of wake stages;
   - the number of *IEDs* for each period (i.e. 30 min period or 1hr period etc.) of *non-REM sleep;*
   - the number of *IEDs* for each period (i.e. 30 min period or 1hr period etc.) of *REM sleep;*
- the present example provides a means of defining an event period in accordance to any event or clusters of events as outlined in this patent application document including *"health conditions* or symptoms *of **interest**"* or "events of interest/EOI" (per "EOI DEFINITION" detailed in abbreviation table at rear of this document).
- events of interest or health conditions of interest as further outlined in *Figure* **45** Automatic diagnostic and prognostic EEG-monitoring and analysis system incorporating minimisation process enabling professional and consumer-level monitoring and automatic analysis determination.; and/or per *"health conditions* or symptoms *of interest "* or "events of interest/EOI" (per "EOI DEFINITION" detailed in abbreviation table at rear of this document).
- the present example provides a means of monitoring a subject/patient and generating source localisation, segmentation, head modelling (such as boundary element models (BEM) or realistic models, current density reconstruction, coherence, Dipole modelled and other connectivity measurements outcomes .

*Title: eLifeNLDB Non-Linear Dynamical Based (NLDB) analysis with option of automatic determination and option of online distributed **processing** capable of spontaneous source localisation applicable to **diagnostic** or **prognostic** determination and*/*or biofeedback or treatment* of consumer/patient.") for both online and offline display or indication purposes, applicable to seel or wake or other "health conditions or symptoms of interest" or "events of interest/EOI" (per "EOI DEFINITION" detailed in abbreviation table at rear of this document). ³²

### Non-linear dynamic spatiotemporal neurological or neuro-behavioural diagnosis and prognosis system

- a further process comprising of the determination of an *index based* on any of or combination of:
   - excessive **EEG** frequency activity in delta or theta and/or beta or gamma versus alpha frequency bands;
   - an abnormally low connectivity across one or more regions of the brain;
   - where connectivity can include reduction in any of or any combination of coherence, correlation, phase-coupling, frequency-coupling, and or synchronisation between a plurality of brain regions;
   - a further process whereby brain activity increase or decrease is tracked in front, rear, top, bottom, left and/or right regions of the brain compared to normal brain behaviour based on normative population studies or disease/disordered population study groups;
   - a further process whereby brain activity increase or decrease is tracked in terms of spectral changes;
   - a further process whereby brain spectral and/or power activity increase or decrease is tracked in front, rear, top, bottom, left and/or right regions of the brain compared to normal brain behaviour based on normative population studies or disease/disordered population study groups;²⁶
   - whereby *detection of preictal* or *interictal or postictal or ictal epileptiform discharges (IEDs)* incorporates any of or any combination of:
      - detection of *sharp-wave* signals, by way of identifying transient signals, discernible from background;
      - detection of *spike wave signals,* which are the same as spike signals, except that they have duration of at least 20ms but under 70ms;
      - detection of *spike-and-slow-wave complex* signals, comprising of a pattern consisting of a spike signal followed with a *slow wave* (typically the slow wave has a higher amplitude compared to the spike signal);
      - detection of a plurality of *spike-and-slow-wave complex,* similar to spike-and-slow-wave complex, except that there are *at least 2 spikes* associated with at least one of the slow waves;
   - whereby detection of "events of interest" or "health conditions of interest" includes any of or any combination of:
      - detection of neurologic autism or *preictal* or *interictal or postictal or ictal epileptiform discharges (IEDs)* (physiologic state or event such as a seizure, other neurological events, or "events of interest" or "health conditions of interest") markers characterised by way of the detection of **EEG** monitored spike-waves or sharp-waves and detection of intermittent rhythmic delta activity;
      - detection of neurologic autism or *preictal* or *interictal or postictal or ictal epileptiform discharges (IEDs)* (physiologic state or event such as a seizure, other neurological events, or "events of interest" or "health conditions of interest") markers characterised by way of the detection of **EEG** monitored spike-waves or sharp-waves and detection of intermittent rhythmic delta activity, with a predominance of activity during predefined (can be configured as part of system adjustable parameters) sleep stages (such as but not limited to any of or any combination of sleep stages or drowsiness states);
      - determination of spike-waves in terms of synchronisation and/or independence of the temporal and bilateral temporal brain regions;
      - determination paroxysmal moderate to high-amplitude **EEG** rhythmic activity;
      - determination of brain region of determined activity; ***⁸⁵***

### Threshold of detection

- a further process comprising of the determination of threshold and/or normal function levels and/or ranges applicable to "events of interest" or "conditions of interest", whereby such parameter configurations and/or thresholds, working ranges, and/or limits can be defined in accordance to patient-specific or normative data (prior studies or data bases of study data) of any of or any combinations of:
   - cluster algorithm determination;
   - signal dynamic transfer (non-linear dynamic analysis such as, but not limited to, complexity or entropy analysis);
   - frequency domain analysis;
   - coherence, correlation, Dipole analysis or other analysis indicative of brain connectivity; statistical analysis;
   - indices defined by any of or any of combination of characterised, detected and/or determined events in terms of events per time period (any prior monitored period such as past events per hour and/or average events per past hour for any session or periods of monitoring study);
   - where indices can be computed in terms of any of or any combination of events per time, per sleep stage, and/or per wake stage;

### Abnormalities in overnight sleep EEGs in children with autistic spectrum disorders^{86 87 88}

- The present example further incorporates the ability to or deploy a computer implemented method comprising a diagnostic and/or prognostic platform with a means of enabling online monitoring via a mobile wireless connected or other computer network connected system interfacing to a subject/patient wearable or nearby proximity physiological or environmental monitoring device, the computer implemented method comprising any of or any combination of:
   A computer system incorporating one or more processes with memory storage capability involving one or more computer programs which can be executed by one or more processes, the one of more computer programs including instructions or applications enabling any of or any combination of:
   The present example further comprising *"health conditions* or symptoms *of **interest**"* or "events of interest/EOI" (per "EOI DEFINITION" detailed in abbreviation table at rear of this document).

- nonlinear variations or changes in one or more predefined (i.e. where data based as presented in ***Figure*** 78 including comparative data bases per blocks **[6]** to **[11])** using normative and/or disease/disorder population group databased reference of normal or baseline conditions from which to set an amount/degree of variation or associated thresholds, ranges and/or limits setting detection characterisation levels and/or severity of disease/disorder condition of interest or event of interest) regions of the brain;
- whereby changes or variations can include those identified by way of tracking changes in signal dynamic (including but not limited to non-linear dynamic analysis such as entropy or complexity analysis etc.) characteristics of any of or any combination of monitored **EEG** channels of subject/patient) including but not limited to more complex or higher density scalp and/or body electrode sensing placement locations;
- whereby changes or variations can include those identified by way of tracking changes in signal dynamic (including but not limited to) spectral analysis (i.e. any of or any combination of slowed mean frequency, one or more spectral bands) characteristics of any of or any combination of monitored **EEG** channels of subject/patient) including but not limited to more complex or higher density scalp and/or body electrode sensing placement (back flush higher density term);
- whereby changes or variations can include those identified by way of tracking changes in signal dynamic (including but not limited to) COHERENCE (i.e. any of or any combination of reduced coherence across any of or combination of one or more regions of the brain) characteristics of any of or any combination of monitored **EEG** channels of subject/patient) including but not limited to more complex or higher density scalp and/or body electrode sensing placement locations;
- whereby COHERENCE analysis incorporates a range of adjustable COHERENCE ANALYSIS PARAMETER CONFIGURATIONS can include the capability of setting a number of thresholds or acceptable ranges applicable to the COHERENCE determination;
- whereby the said COHERENCE determination can be derived from the cross-validation of COHERENCE COMPUTED data sets corresponding to any of or any combination of **EEG** sensor locations or associated source localisation determinations associated with **EEG** sensor electrode locations;
- whereby the dimensionality or large volumes of valid source localisation regions of brain activation can be reduced by way of deploying PCA and ICA analysis (see also as outlined under subheading Automatic online Principal Component Analysis (PCA) and Independent Component Analysis (ICA) elsewhere in this document) to minimise dimensionality of signals of interest based on signal to noise of most predominant components discernible based on **EEG** electrode signals.
- whereby said COHERENCE processing includes a means for enabling source analysis results capable of indicating if one **EEG** source waveform is similar in shape to another **EEG** source waveform, but shifted in time;
- whereby said COHERENCE processing includes a means for enabling source analysis includes the direction indication of the lag between two **EEG** sources, demonstrated by directional indicators (such as bit not limited to arrows - i.e. points to the later site);
- whereby said COHERENCE processing can be configured to fixed or rotating dipoles;
- -whereby COHERENCE analysis incorporates a range of adjustable parameters including (but not limited to COHERENCE computed with any of or any combinations of continuous, epoched, and averaged data;
- whereby COHERENCE analysis incorporates a range of adjustable parameters including (but not limited to COHERENCE computed across any of or any combination of two or more monitored signal waveforms;
- whereby COHERENCE analysis incorporates a range of adjustable parameters including (but not limited to COHERENCE computed across any of or any combination of two or more monitored signal waveforms whereby the time relationship between the said two or more waveforms can be adjusted or varied ;
- whereby COHERENCE analysis determination can comprise of computing the number of connections (arcs) that between all any of these 2
- whereby COHERENCE analysis incorporates certain frequency ranges (i.e. can be filtered in a preceding process to coherence analysis)
- whereby COHERENCE analysis enable the generation of output values that are the lags (time-shifts) of maximum overlap between any of or any combination of waveforms;
- whereby COHERENCE analysis enables coherence values greater than or equal to a defined Threshold value [%], and based on coherence values greater than or equal to the defined Threshold percentage being computed, stored or reported;
- whereby said changes or variations variation or changes can determined based on comparing a first state considered normal to a second state considered to be a prognostic or diagnostic event of interest or condition of interest;
- whereby events on interest can include *"health conditions* or symptoms *of **interest"*** or "events of interest/EOI" (per "EOI DEFINITION" detailed in abbreviation table at rear of this document). ⁸⁸

### Description of Figures

***Figure*** 80 Personalised subject/patient- health management system (HMS) [1] incorporating any of or any combination of monitoring goals.

### PATENT TITLE: Non Linear Dynamical Based Analysis (NDBA) with option of spontaneous automatic online analysis

### BACKGROUND

Abstract: In recent times it has become increasingly clear that subtle EEG biomarkers, some of which were previously only accessible via more invasive methods, are now potentially accessible. However, these subtle EEG measures are often deep-cortical or tiny signals of complex, diffused and often barely discernible using conventional monitoring processes.

Entropy analysis of neurological ER signals has been shown to be a marker of anaesthesia-depth and can provide a useful brain analysis tool (Burton et al. 2005-2010).

In particular non-linear dynamic based (NLDB) analysis such as Entropy source localisation techniques combined with fast averaging techniques (including autoregressive modelling with an exogenous input;ARX) provide a means of progressively tracking changes in a step-wise manner in order to discriminate deterministic or predicable neurological generators.

The present example provides means for enabling the brain or body or other biological sample to imaged (i.e. MRI, PET, CT, ultrasound, MEG etc.),visualised or measured, means including any of or any combination of (but not limited to) a) spontaneous EEG biomarkers (i.e. but not limited to transient events, phasic events, continuous events etc.), b) evoked response EEG measures resolved based on dynamic linking with "spontaneous events of interest" or resolved as running average or running auto-regression modelled values (without or with external input function as a means of producing faster averages), c) neurogenic measures (i.e. EEG or brain imaging measures), d) myogenic measures (i.e. EMG, muscle movement etc.) e) NLDB measures, f) source localised measures, g) sleep measures, h) circadian clock measures, i) epilepsy or other seizure measures, j) brain connectivity measures, k) spectral measures, **I)** infrared reflectance oculography measures, m) cluster analysis or ensembles/sequences of events, n) any of or any combination of (but not limited to cluster analysis or ensembles/sequences of events means, whereby said cluster analysis or ensembles/sequences of events means further can comprise of configuration or pre-definition by way of (but not limited to) any or any combination of the deployments (as also detailed more comprehensively elsewhere in this document) via the deployment of wearable-device minimisation (WM) system incorporating algorithm subject/patient-specific learning (ASL) system (i.e. but not limited to artificial intelligence and/or expert system analysis);
- the present example further incorporates the ability to or deploy a computer implemented method comprising a diagnostic and/or prognostic platform with a means of enabling online monitoring via a mobile wireless connected or other computer network connected system interfacing to a subject/patient wearable or nearby proximity physiological or environmental monitoring device;
- the present example further incorporates mobile-wireless distributed analysis (MDA) system enabling local wearable device or mobile device systems to have subject/patient analysis or monitoring systems to be supplemented via remote interconnected processing capabilities (i.e. Cloud-computing services, network application services, WAN, LAN other remotely located computer and processing resources);
- the present example further incorporating a apparatus system and/or computer system(s) with one or more processes with memory storage capability involving one or more computer programs which can be executed by one or more processes, the one of more computer programs including instructions or applications enabling any of or any combination of;

### (a. spontaneous EEG biomarkers)

- The present example further provides the capability of characterising and tracking spontaneous **EEG** measures representing neural sources along with neural source generation anatomical locations applicable to any events of interest further outlined elsewhere throughout this document and also (but not limited to) ***comprising defining** and **characterising*** events of interest (i.e. *"health conditions* or symptoms *of interest" or* "events of interest/EOI" (per "EOI DEFINITION" detailed in abbreviation table at rear of this document);

### (a.i. EEG Perfusion Markers)

- The present example further provides the capability of characterising and tracking **EEG** perfusion markers representative of cortical perfusion based on the interpretation of **EEG** spectral composition based on predefined characteristics applicable to the changes in **EEG** frequency composition based on the association (i.e. interpretation of perfusion levels or associated metabolism can be based on patient/subject-specific measures (i.e. ***Figure** 77**;* ***Figure** 78**;* ***Figure** 79**;* ***Figure** 80**)* or comparative patient population studies (i.e. Leuchter et al. (1999)) and/or related data bases (i.e. per ***Figure 78*****)** or measures between **EEG** spectral measures and the underlying metabolic activity of the human cerebral cortex. For example, the alpha spectral band is can be associated with the most significant negative partial correlation with perfusion.

### (a.ii. EEG biomarkers)

The present example further provides the capability of characterising and tracking **EEG** phasic activations such as K-complexes, spindles, high frequency oscillations (HFO), high frequency ripples, alpha bursts are important prognostic and diagnostic biomarkers (also detailed per biomarkers and events of interest covered in ***Figure** 45**, within patent description of* "eLifeNEURO/ EPILEPSY" *section* and elsewhere in this document). The present example provides a means of monitoring one or more physiological variables of a subject (including, but not limited to one or more **EEG** signals), the present example further enabling means of the determination the genesis (i.e. brain anatomical source localisation) of neural generators. The said neural generators can include signal changes within the brain beyond a certain signal to noise ratio threshold in order to distinguish events of interest from background noise or unwanted signals. Such signals of interest can include signal changes based on spectral composition of signal, coherence interrelationship between two or more signals, phase relationship between two or more signals, signal dynamics between two or more signals (such as but not limited to complexity, entropy or other non-linear dynamic based analysis/NLDB) (per examples, but not limited to, ***Figure** 45**;* ***Figure*** 66; ***Figure*** 69; ***Figure** 70**;* ***Figure** 84; **Figure** 85**;* ***Figure*** 86; ***Figure** 87**;* ***Figure** 88**;* ***Figure* 89****;** ***Figure 90; Figure 91******;*** ***Figure 92******;*** ***Figure 93******;*** ***Figure 94*****).**

### (a.iii. multi-dimensional source localisation (MSL) with HFOs, ripples and other spontaneous events)

The present example further provides the capability of characterising and tracking **EEG** phasic biomarkers (or so-called "spontaneous" events or "events of interest") enabling the identification of important information related to the neural sources, from where such spontaneous events are generated.

For example, precursors of frontal epilepsy can be marked with a presence of frontal lobe HFOs or ripple events of interest. However, epilepsy based on deep cortical origins, such as lesions, can be marked by HFO or ripple events that may not be easily discernible in **EEG** scalp surface recordings, due to the high attenuation of these deep cortical due to brain, skull skin and other physiological matter. Moreover, the brain generates many signals based on various body functions, thinking and other brain processing aspects, further disguising or overwhelming the presence of barely discernible signals of interest buried in surrounding signals and noise. Therefore, the present example further enables one or more analysis source localisation formats undertaken, and the outputs results of this plurality or any single analysis format of source localisation outcomes to be compared. The present example can further compute the probability or signal to noise thresholds of signals of interest based on source localisation based on any of or any combinations of spectral composition of signal, coherence interrelationship between two or more signals, phase relationship between two or more signals, signal dynamics between two or more signals (such as but not limited to complexity, entropy or other non-linear dynamic based analysis/NLDB) (per examples, but not limited to, *Fiqure **45***;
***; Fiqure* 66;** ***Figure* 69****;** ***Figure 70******;*** ***Figure 84; Figure 85******;*** ***Figure* 86****;** ***Figure 87******;*** ***Figure 88******;*** ***Figure 89******;*** ***Figure 90; Figure 91******;*** ***Figure 92******;*** ***Figure 93******;*** ***Figure 94****).* For example, subtle deep-cortical changes such as alpha bursts of spectral **EEG** power now evident during conventional sLORETA current density source localisation analysis may be evident when the results of spectral and/or NDB and or conventional sLORETA current density source localisation analysis results are compared and contrasted in a manner where the signal to noise ratio of any of these formats or a combination of these different formats can be combined (i.e. regression or average analysis).

In this manner the present example can, for example, substitute the value of any combination of values of the graduated values (suitably scaled) of spectral composition, or the graduated (suitably scaled) values of signal dynamics (i.e. such as but not limited to complexity, entropy or other non-linear dynamic based analysis/NLDB) with conventional current density (or voltage values) monitored at one or more **EEG** electrodes, as a means of computing any of or any combination of (but not limited to) source localisation outcomes based on **EEG** spectrum, signal dynamics and/or conventional means such as current n=density source localisation.

The MSL system can further provide a means of enabling signal to noise and/or thresholds adjustment capabilities, for example, to "tease" out or extract **EEG** signals of interest otherwise not discernible amidst other signal or noise. Such means of enabling signal to noise parameter adjustment capabilities includes attributing a graduated value to the NLDB transformed signal in accordance to the degree of linearity (i.e. entropy) as it relates to each monitored **EEG** electrode. For example, by allocating a continuously computed entropy or complexity value in the form or a running or continuous computation, based on the characterisation of the most recently monitored data (i.e. the past 10 sec, 30 sec, 60 sec, 3 min or other allocated time period; such past period can also be dynamically allocated or computed based on monitoring circumstances or requirements at an time). In this way a transformed NLDB time-series (NLDBT) values can be computed as a time series of values representative of each **EEG** electrode's NLDB transformed values. Similarly, a spectral dynamic time-series (SDT) values can be derived. The conventional signal to noise computational approach can be used for determining thresholds relevant to signals of interest. i.e. exceeding certain thresholds levels SDT can be indicative, for example, of alpha spectral burst of interest.

Similarly, exceeding certain thresholds levels NLDB can be indicative, for example, of alpha spectral or other continuous or spontaneous events of interest. Moreover, principal component analysis (PCA) and/or independent component analysis (ICA) as also covered in ***Figure** 56 to **Figure*** 78 *or* ***Figure*** 84 to ***Figure*** 94 can be computed whereby any of or any combination of SDT, NLDBT and/or conventional values such as current density measures can be computed as a means of teasing out or extracting "events of interest" *"health conditions* or symptoms *of **interest**"* or "events of interest/EOI" (per "EOI DEFINITION" detailed in abbreviation table at rear of this document).

Using the present approach these SDT or NLDB values can be substituted for conventional current, power or voltage magnitudes during source localisation computations (i.e. substituting SDT or NLDBT values for current values in sLORETA current density source localisation computation, for example), thereby enabling the various NLDBV views of the brain and/or SDT views of the brain. Such views can include identifying spontaneous or evoked events of interest, along with cluster analysis prognostic or diagnostic "health conditions or symptoms of interest" or "events of interest/EOI" (per "EOI DEFINITION" detailed in abbreviation table at rear of this document).

The MSL system can further provide a **means of MSL parameter adjustment capabilities, for example,** to "tease" out or extract **EEG** signals of interest otherwise not discernible amidst other signal or noise. Such **means of enabling means of MSL parameter adjustment capabilities, can** include for example, and in the case of **NLDBT** values determination can provide a means (.e. **NLDBV time series analysis epoch or time-period** upon which running values of NLDB values are computed. Other adjustable parameter values can include **NLDBV step time** representing the progressive steps in time after which each NLDBV is computed. Another NLDB parameter adjustment can include the coefficients in complexity or entropy analysis, for example, that determine the magnitude of the NLDBV values by way of the sensitivity adjustment of this NLDB analysis - i.e. in the case of complexity analysis a more sensitive NLDB analysis transformation could be based on "a synchronous factor". A "synchronous factor" could be based on, for example, the alignment of the alpha **EEG** signal cycles (peaks and troughs) in a manner whereby equal spacing between an **EEG** alpha cycles (for example) could represent a lower degree of complexity or NLDBV, while a moderate variance between the alignment of the alpha **EEG** signal cycles (peaks and troughs) could be represented by a moderate NLDBV, and a significant variation of the spacing or alignment (or synchronisation) of **EEG** alpha cycles (for example) could represent a greater magnitude (degree) of complexity or NLDBV.
**a.iv) Alpha busts, K-complexes, spindles-** The present example tracks and identifies **different phasic EEG patterns** including those appearing as discernible markers of different sleep stages. For example, by way of detecting changes (i.e. such as but not limited to any of or any combination of NLDB/entropy, spectral power, coherence, brain connectivity, Dipole modelling etc.) as a means of distinguishing appearance of and/or localisation of source of **alpha bursts, K-complexes, EEG spindles,** vertex-waves, **EEG** spectral characteristics or different respective sleep or wake or drowsiness stages or phases.

**(Dynamic Spatiotemporal EEG Connectivity (DSEC) characterisation); a.v.) Epileptiform discharges** - The present example tracks and identifies **epileptiform discharges such as EEG monitored spike-waves or sharp-waves and detection of intermittent rhythmic delta activity,** as a marker of epilepsy or other seizure onset or incidence (as further outlined elsewhere in this document).

***Dynamic Spatiotemporal** EEG Connectivity (DSEC) characterisation -* The present example tracks and identifies ***Dynamic Spatiotemporal** EEG Connectivity (DSEC) characterisation* of the human brain as a marker of the incidence or precursor to events of interest (i.e. but not limited to *"health conditions* or symptoms *of **interest" or*** "events of interest/EOI" (per "EOI DEFINITION" detailed in abbreviation table at rear of this document). For example, interconnectivity of the brain regions such as thalamocortical (TC) loops have been implicated as a marker of absence epilepsy. However, such measures have been in the prior art been reserved for research⁸⁹.

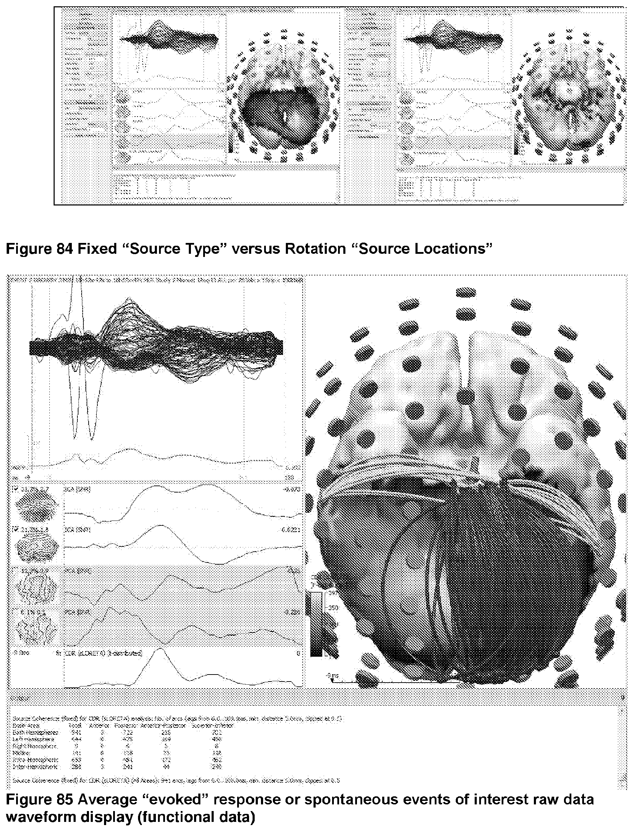

To overcome limitations of the prior art **the present** example **provides a means of enabling online analysis as part of a patient or consumer level wearable mobile wireless device,** whereby a **means of the monitoring, tracking and determination of ensembles or clusters of connectivity formations within the human brain, applicable to events of interest** (i.e. but not limited to *"health conditions* or symptoms *of **interest"*** or "events of interest/EOI" (per "EOI DEFINITION" detailed in abbreviation table at rear of this document) can be achieved. Such **means of the monitoring, tracking and determination of ensembles or clusters of connectivity formations within the human brain, applicable to events of interest** includes:
*1.* ***A first pre-diagnostic*/*****prognostic process step comprising** of **defining** and **characterising*** events of interest (i.e. *"health conditions* or symptoms *of **interest"*** or "events of interest/EOI" (per "EOI DEFINITION" detailed in abbreviation table at rear of this document). The said events of interest could (example only), comprise in the case of monitoring (i.e. see ***Figure*** 75 showing epilepsy monitoring and including epilepsy monitoring setup with HFOs, ripples, spectral etc. per **blocks 12-18** ) an epileptic subject prone to seizures or absence epilepsy (lapses of consciousness) consist of epileptiform discharges (i.e. spike and wave) and/or other means of ***Spatiotemporal EEG** connectivity dynamic determination applicable to **events** of **interest.*** Such ***Spatiotemporal EEG** connectivity **dynamic** determination applicable to **events** of **interest*** means includes the capability to monitor, acquire, source localise and generate coherence or Dipole modelling outcomes associated with spontaneous events of interest including **spike and waves, HFO's, spindles, K-complexes, alpha bursts** and other events of interest in a manner whereby the said ***coherence** and **Dipole measures** of brain connectivity **associated with these events** of **interest*** can be *quantified **in the context** of **spatiotemporal*** (3 dimensional source localised appearance of events of interest in terms of the time sequence of such occurrences). In this way the said characterisation of clusters or ensembles of coherence and/or Dipole modelled brain activity can be associated with events of interest. In this manner such examples or incidents of ensembles or clusters of brain connectivity can be deployed as a basis of comparison or reference as *part of the decision **process** involved **in the** identification and **ultimate** determination of* applicable or relevant (to subject) precursors of epilepsy in a more specific-manner and hence more subject-specific accurate and more sensitive (i.e. to enable the earliest possible onset detection time applicable to patient, thereby allowing patient to prepare themselves or make themselves safe or administer medication to avoid more serious health deterioration. This step incorporates a **monitoring minimisation processes (*****Figure*** 45) whereby simplified sensors specific to the most essential physiological channels and sensor locations is determined in order to enable a simplified and practical routine monitoring configuration suitable, as required, for daily or routine health monitoring and management.
2. *A second **processing** step **comprising** of the establishment of* a *reference database* (i.e. per ***Figure** 75**, block **12; also** per* ***Figure 78******, workspace)*** enabling **clusters or ensembles** of coherence to characterised based on patient-specific **pre-diagnostic studies** or other monitored patient data bases and information **aggregated to produce relevant knowledge** (i.e. *Fiqure 77;* ***Figure** 78**; Fiqure 79;* ***Figure** 80**)* showing data base information to knowledge aggregation), for enabling online monitoring algorithm comparative analysis in terms of tracking and characterising current and past monitored data to be examined for typical precursor or the incidence of events of interest (i.e. *"health conditions* or symptoms *of **interest"*** or "events of interest/EOI" (per "EOI DEFINITION" detailed in abbreviation table at rear of this document). The database of events of interest can include the various biomarker criteria applicable to detection of online events of interest for a specific subject. Criteria of such events of interest can be provides based on wake or sleep states. For example, epilepsy can be more predominant in certain sleep states so be enabling ***the present*** example ***to define events** of **interest by wake or sleep state (i.e. stage N1, N2, N3 or** REM) and biomarker **events or clusters** of biomarkers* can provide a more patient-specific and accurate prognostic or diagnostic capability.
3. *A **third processing step comprising** of* monitoring, signal processing, acquiring, and *online **diagnostic** or **prognostic** analysis of* a *subject,* whereby said *"online* ***diagnostic** or **prognostic** analysis of* a ***subject** "* (i.e. see ***Figure** 75**, block 1-7).*
*4. In **further process step the coherence** and **Dipole measures** of brain connectivity (i.e. **per*** ***Figure 84; Figure 85******;*** ***Figure* 86****;** ***Figure 87******;*** ***Figure* 88****;** ***Figure* 89****;** ***Figure 90; Figure 91******;*** ***Figure 92******;*** ***Figure 93******;*** ***Figure 94******) associated with events of interest*** can be determined and *quantified **in the context** of consciousness **states or** transitions of consciousness* ***states, including (but not limited to) wake or sleep* states** ;
*5. In further process step* the present example enables a means of ***diagnostic** or **prognostic monitoring** or tracking* online *Spatiotemporal **EEG** connectivity dynamic determination **applicable** to events of interest* (i.e. *"health conditions* or symptoms of *interest"* or "events of interest/EOI" (per "EOI DEFINITION" detailed in abbreviation table at rear of this document). Such means of the monitoring, tracking and determination of ensembles or clusters of connectivity formations within the human brain, applicable to events of interest (per first process step) includes comparing or referencing to typical events of interest relating to precursors or the incidence of *events of interest* (i.e. *"health conditions* or symptoms *of interest" or* "events of interest/EOI" (per "EOI DEFINITION" detailed in abbreviation table at rear of this document).
*6. In further process step* the present example enables a means ***diagnostic** or **prognostic** combined or separate sleep **monitoring** and*/*or analysis (means described elsewhere in this **document).***
*7. In further process step* the present example enables a means of ***diagnostic** and*/*or **prognostic** sleep **monitoring** and*/*or **analysis** with interlinked therapeutic and*/*or therapeutic and*/*or medication and*/*or medication dispenser system biofeedback* (Biofeedback and include any of the monitored or analysis events of interest including, but not limited to "health conditions or symptoms of interest" or "events of interest/EOI" (per "EOI DEFINITION" detailed in abbreviation table at rear of this document). *Means of therapeutic control* can incorporate information relating to these said events of interest in the decision or control process of therapeutic intervention to subject/patient including, but not limited to, positive airway pressure or non-invasive ventilation systems).
*8. In further process step* the present example enables subject or other parties warning and/or alerts and/or notifications of information or updates and access enabling diagnostic or prognostic notification or automatic indications (i.e. including watch or other health device alarm, vibration or visual notification) to any secured and authorised location, or subject/patient wearable or mobile device system;
*9. In further process step* the present example enables automatic indications or initiation of indications (i.e. conditional upon subject/patient acceptance or approval or authorisation) or activation of related (i.e. related to prognostic or diagnostic events or interest or as further described, but not limited to "health conditions or symptoms of interest" or "events of interest/EOI" (per "EOI DEFINITION" detailed in abbreviation table at rear of this document), messages, emails, other correspondence, information access to social or private health network, calendar appointment options, reminders etc., which related to health management or coaching or assistance (ambulance, nurse home assistance, prompting of intervention or contact of any sort from family or friends or carer etc.), health care supplier (i.e. therapeutic device supplier for spare parts or system attention, service or adjustment; health centre for diagnostic or therapeutic treatment or attention follow-up (i.e. adjustment of CPAP, APAP or NIPPV system to minimise sleep fragmentation, RERA, TERA, SDB, etc.);
*10. In further process step* the present example enables automatic indications comprising a means of 3^{rd} party information update and access enabling diagnostic or prognostic notification or automatic indication or reporting to any secured and authorised location;

### (b. evoked response EEG measures)

- The present example further enables the monitoring and tracking of a subject/patient's evoked response **EEG** measures capable of identifying neural sources along with neural source generation anatomical locations comprising any of or any combination of (but not limited to), as described elsewhere in this document under the SR explanations. Similar to SE evoked responses of evoked events can be resolved as a single event occurrence or by way of averaging a plurality of responses synchronised to a subject/patient stimulus presentation (i.e. audio, visual, vibration, temperature, electrical, magnetic stimulation etc.), whereby the averaging of multiple events can improve the signal to noise properties prior to further evoked response or evoked event processing;

### (c. neurogenic measures)

- The present example further enables the monitoring and tracking (including of a subject/patient's neurological measures, along with source generation or anatomical locations, associated with single and/or clusters of such events (including combinations of myogenic and/or neurogenic EOI) along with and/or comprising any of or any combination of (but not limited to) applicable to the prognosis or diagnosis or "health conditions or symptoms of interest" or "events of interest/EOI" (per "EOI DEFINITION" detailed in abbreviation table at rear of this document, whereby such events can include SR, ER and or CNT signal formats.

### (d. myogenic measures)

- The present example further enables the monitoring and tracking of a subject/patient's myogenic measures (i.e. EMG, atonia, etc.) along with source generation or anatomical locations, associated with single and/or clusters of such events (including combinations of myogenic and/or neurogenic EOI) along with and/or comprising any of or any combination of (but not limited to) those applicable to the prognosis or diagnosis or "health conditions or symptoms of interest" or "events of interest/EOI" (per "EOI DEFINITION" detailed in abbreviation table at rear of this document), whereby such events can include SR, ER and or CNT signal formats.

### (e. non-linear dynamic based (NLDB) measures (NLDB) measures)

- The present example further enables the monitoring and tracking of a subject/patient's NLDB measures along with source generation or anatomical locations, associated with single and/or clusters of such events (including combinations of myogenic and/or neurogenic EOI) along with and/or comprising any of or any combination of (but not limited to) those applicable to the prognosis or diagnosis or "health conditions or symptoms of interest" or "events of interest/EOI" (per "EOI DEFINITION" detailed in abbreviation table at rear of this document), whereby such events can include SR, ER and or CNT signal formats.

### NLDBV SOURCE LOCALISATION OVERVIEW

For point of clarification the present example incorporated NLDBV source localisation which can be computed online or offline by way of calculating the continuous (CNT) source of brain activation energy from a plurality of known **EEG** monitoring electrodes or via imaging data (i.e. any number or combination of voxels (image space) forming a total image of a subjects brain (or other body sections) . Alternatively evoked potentials (EP) whereby monitored data relates to responses related to an externally (exogenous) generated stimulus or evoked response (EP) also enabled.

Additionally, spontaneous responses (SR) such as spindles, HFOs, high frequency ripples, K -complex, alpha-bursts, spike and wave epileptiform discharges and other internally generated (endogenous) events via processing contingent potentials (PCP) or other events initiated by the human body in general or brain can also be distinguished, as further described elsewhere in this document. Furthermore the present example can source localise based on SR from a plurality of imaging voxel EP or CNT EEG.

The present example, when source localising online for a plurality of CNT **EEG** monitored electrode locations, can produce 2 or 3 dimensional maps of energy distribution, whereby various colour and superimposed event labelling annotations can cover a number of analyses formats, along with any of or any combination of energy formations and associated magnitudes.

For example, energy formations that can be monitored and source localised include spectral source localisation, NLDBV localisation and/or conventional current density reconstruction (CDR) such as using sLORETA modelling.

In the most simplistic first generation the present example can translate any of or any combination of said imaged voxels or monitored **EEG** locations (channels) into a similar context of representation as used in conventional CDR analysis except a new data set of neurology signal channels can be computed using similar data-acquisition resolution as original or raw data neurology image voxels or CNT **EEG** or EP **EEG** data sets. In the simplest manifestation of the present example a similar step time to the data-acquisition rate (i.e. for data acquired at 5000 samples per second the "step" time parameter configuration for generation of the spectral time-series values (STV) subject/patient data set or the non-linear dynamic database (NLDBV) subject/patient data set can be set to say 0.2ms). In this simple example the "epoch" time parameter for the STV or NLDBV data set generation could be set to 1 second providing a suitable time base to capture typical or predefined (including SR, ER CNT EOI, coherence connectivity, Dipole modelling, along with associated clusters, ensembles or sequences- i.e. but not limited to HFOs, high frequency ripples spindles, K-complexes, alpha-bursts, spike and wave epileptiform discharges, (i.e. subject to configuration as outlined elsewhere in this document, such as HFO period detection being configured to a duration of say 10-200ms, etc., for each respective SE or EOI).

In such a way the present example can generate a transformed 2 or 3 dimension data sets data sets. For example, NLDBV time-series values (NLDBTV) data sets comprise of time series magnitude of NLDBV, which can be represented by a first data dimension being time domain (i.e. temporal with some consideration based on time synchronisation with originating monitored image data (i.e. voxels) or **EEG** data (i.e. EP **EEG** and/or CNT EEG) data processing delays applicable to "step" and "epoch" parameter configurations, which impose corresponding time-delays) and a second dimension being computed magnitude of NLDB value (described elsewhere in this document).

In another example, NLDBV time-spectral time-series values (STV) data sets comprise of time series magnitude of spectral information, which can be represented by a first data dimension being time domain and a second dimension being computed magnitude of NLDB value (described elsewhere in this document), while a third dimension can represent the frequency band or spectrum of the monitored data for the applicable time period, in this latter STV example, the spectral content could be denoted in accordance to frequency band (i.e. any of or any combination of frequency band of or approximate to delta/slow wave (0.1-4 Hz), theta (4-8 Hz), alpha (8-12 Hz), beta (12-30 Hz), low-gamma (30-70 Hz), and/or high-gamma (70-180 Hz, ripple, HFO etc.), such as (but not limited to) examples per **(*****Figure** 45**;* ***Figure*** 69). The STV data set transformations can enable waveform data to be produced in a manner whereby y-axis display can be designated as magnitude and/or spectral content (single or dual y-axis formats possible), while x-axis represents time of monitored data.

Consequently, the present example enables, by way of transforming original monitored neurology or physiological source data to revised formats comprising any of or any combination of transformation variants (i.e. NLDBTV or STV and/or combined NLDBTV and STV).

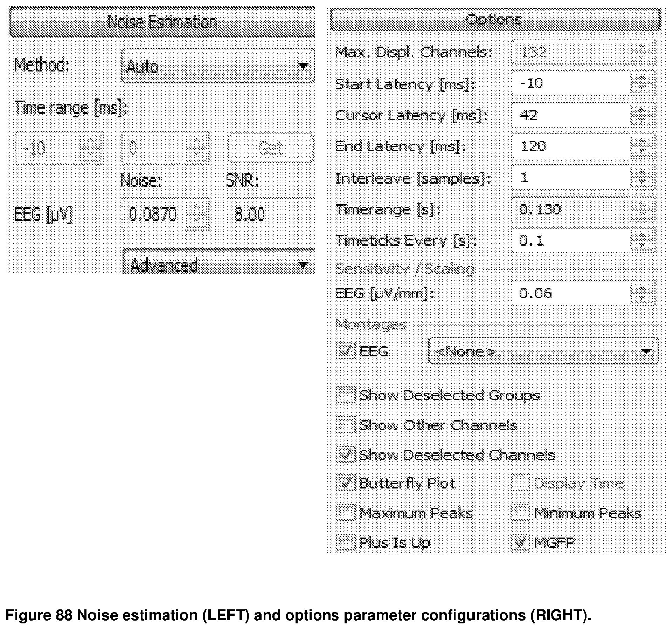

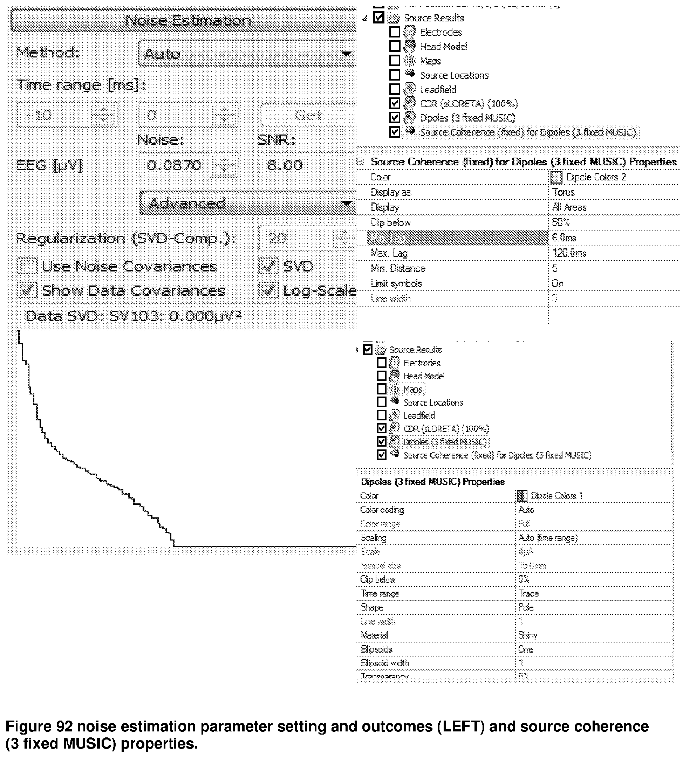

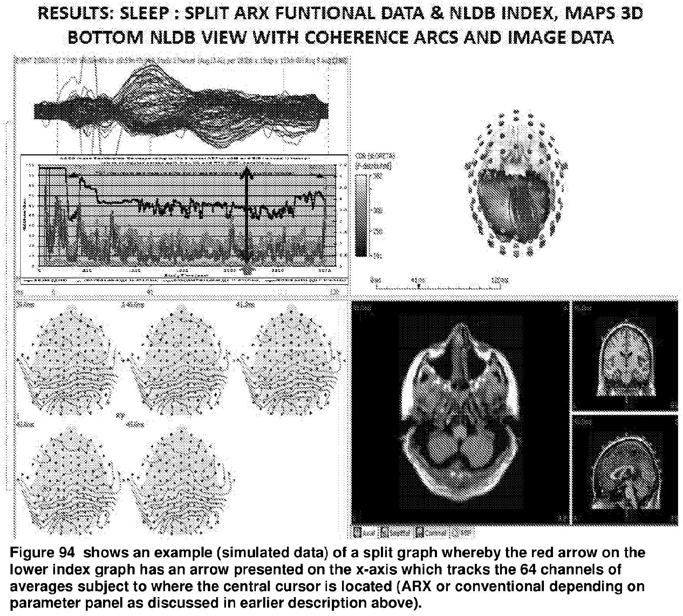

Moreover, the present example based on this approach can therefore deploy the substitution of transformed data, whereby analysis methodologies such as any of or any combination of (i.e. per example only of presentation format in ***Figure** 45, to Figure 94**)* image data views or computations (i.e. per example only of presentation format in ***Figure** 45**, **right** of row 2),* functional data views (i.e. per presentation format in ***Figure** 73**;* ***Figure** 61**, second trace row down),* ICA or PCA waveforms (i.e. per example only of presentation format in ***Figure** 85**, bottom left),* topographic map brain or body views (i.e. per example only of presentation format in ***Figure** 85**, bottom left),* butterfly plot data computations or views i.e. per example only of presentation format in ***Figure** 85**,* top ***left*),** , segmentation computation or views (i.e. per example only of presentation format in ***Figure** 63**, **right,** Fiqure 64,* ***Figure*** 66, ***Figure** 67*), head-modelling computation or views (i.e. per example only of presentation format in ***Figure** 63**, **right,** Fiqure 64,* ***Figure*** 66, ***Figure** 67*), source reconstruction such as CDR, sLORETA etc. (i.e. per example only of presentation format in ***Figure** 63**, **right,** Fiqure 64,* ***Figure*** 66, ***Figure** 67*), coherence computations or views (i.e. per example only of presentation format in ***Figure** 63**, **right,*** ***Figure** 64**,* ***Figure*** 66), Dipole modelling computations or views (i.e. per example only of presentation format in ***Figure 67*****),** graphic data views, flow or tabular views (i.e. per example only of presentation format in ***Figure 56******;*** ***Figure** 57**;* ***Figure** 58**;* ***Figure** 59**;* ***Figure** 65**;* ***Figure*** 68; ***Figure*** 69; ***Figure** 70**;* ***Figure** 71**;* ***Figure** 72**;* ***Figure 74***), noise estimations or signal to noise computations or views (i.e. per example only of presentation format in (i.e. per example only of presentation format in ***Figure*** 88; ***Figure** 92**),* combined neurology and/or sleep data views (i.e. per example only of presentation format in ***Figure** 45**;* ***Figure** 56**; Fiqure **57;** Fiqure 58;* ***Figure** 59**:* ***Figure** 60**Fiqure* 66; *Figure 68* *Figure **69*** ***Figure 70******Figure 71******Figure 94***), statistical coherence data computations or views (i.e. per example only of presentation format in ***Figure** 65**;* ***Figure*** 69; ***Figure*** 89; ***Figure** 90**).*

Additionally, any of the said **neurology** imaging or **EEG** processing (i.e. including but not limited to any of or any combination of computations or visual presentations relating to any of or any combination of, but not limited to, STV, NLDBV, or **EEG** time-series values) can be generated in synchronisation and biofeedback-wise (i.e. results of prognostic or diagnostic outcomes van be deployed as part of decision or control processes of therapeutic devices including magnetic stimulation systems, medication dispenser systems, medication administration system, APAP, CPAP, PAP, NIPPV systems, PAP systems etc. as a means of optimising treatment or setting or establishing treatment end-points.

### (NLDBV or STV butterfly plots)

In the example of NLDBV or STV butterfly plots, (i.e. per presentation format in top left box of ***Figure** 63**)* the present example can with the case of SE detection automatically detect any events of interest such as (for example only) an **EEG** sleep spindle (i.e. typically consists of 12-14 Hz waves lasting at least 0.5 seconds, and generated in the brain region of the reticular nucleus of the thalamus), whereby the start, end and centre point of the spindle event could manually marked or automatically be detected. In this manner a trace could be detected as online or offline both on a discrete event detection basis or part of a more comprehensive cluster analysis determination. The said cluster analysis determination can include any of or any combination of events (i.e. any of or any combination of sleep and/or neurological events including but not limited to spindles, K-complexes, HFOs, rippled, vertex waves, epileptiform discharge spike and wave events, alphas wave bursts, and wave discharges etc.) as precursors or based on the incident of any "health conditions or symptoms of interest" or "events of interest/EOI" (per "EOI DEFINITION" detailed in abbreviation table at rear of this document). In this example the automatic online source reconstruction or replay of these events could be time synchronise with the respective position of a current image 3D display visualisation of the monitored subject/patient's head. Moreover the *butterfly **plot*** could provide a plurality or any of the *NLDVB, STV* and/or conventional **EEG** amplitude versus time plots (i.e. x-axis for time representation of spindle event and y-axis for magnitude of spindle voltage). Additional or separate butterfly plots could be presented for spectral composition of event (i.e. a-axis representing duration of spindle event, y-axis representing amplitude/voltage of spindle event and z-axis representing frequency spectrum of spindle event. In the case of NLDBTV spindle event butterfly plot the x-axis could display the time of the spindle event while the y-axis could display the complexity or entropy magnitude of the spindle event (i.e. see elsewhere in this document where the "step" and epoch" transformation process enables the generation of substitute values for the conventional **EEG** time-series), for example only. Similarly, the source reconstructed event would appear within the 3D reconstructed visualisation of the subject/patient, but the said source localised event can be detected and displayed in the context of any of or any combination of NLDBTV, STV and/or **EEG** amplitude time-series. Moreover, probability or confidence-level (i.e. the spindle event may be discernible from only one of the three NLDVT, SVT or **EEG** amplitude time series transformations subject to cortical depth and composition of monitored ERG or image voxels. Moreover, in this way the present example can detect and track events previously undiscernible and hence provide a superior degree of prognostic and diagnostic precision.

Multiple sleep and neurological event dynamic data exchange (MS&NDDE) processing system:
The present example can incorporate MS&NDDE whereby a plurality or combination of analysis processing techniques (for example only) could enable a means of online or offline sleep analysis (i.e. per figures ***Figure** 60**;* ***Figure** 61**;* ***Figure*** 69; ***Figure 70***), such means enabling neurological or physiological events (i.e. sleep/wake stages, spindles, k-complexes, arousals, REM, EMG atonia, etc.) to be tracked and identified in terms of start and end times of event, along with sensor locations of monitored events. This event information can be dynamically exchanged with a second neurological processing system. This second neurological processing system, armed with the prior knowledge of specific neurological events can in this manner apply source reconstruction analysis which is both data and time synchronised with the first processing system. In this way any of or any combination of source localised neurology (i.e. CDR, SEG, CNT, EP, sLORETA source reconstruction model, BEM or actual patient/subject image head data or other head model, COH, and/or Dipole modelling) and/or **sleep parameters** or events (i.e. sleep/wake stages, spindles, vertex-waves, k-complexes, arousals, REM, EMG atonia, etc.) can be presented. Additionally, a third process or a part of the neurology or sleep analysis can compute neurology events of interest (i.e. HFOs, ripples, spike and wave epileptiform discharges, along with "health conditions or symptoms of interest" or "events of interest/EOI" (per "EOI DEFINITION" detailed in abbreviation table at rear of this document).

Alternatively one or any combination of a plurality of processing systems can be deployed to process the sleep EOI, neurology EOI and/or the said source localisation information of a subject/patient at any point in time. The present example further enables any of or any combination of "supplementary analysis", "minimisation analysis", "mobile wearable device capabilities", and/or "artificial analysis or expert system analysis" capabilities (as further detailed elsewhere).

**Cluster analysis** can be applied to combined any of or any combination or neurology and/or sleep EOI in order to enable the prognosis or diagnosis of "health conditions or symptoms of interest" or "events of interest/EOI" (per "EOI DEFINITION" detailed in abbreviation table at rear of this document); including (but not limited to) combined conditions such as sleep delay syndrome, sleep triggered epilepsy or SBD.

### NLDBV COH

In the present example of generating parameters applicable to generating statistical and/or visual (i.e. per ARCs with equivalent raw or originating data examples per ***Figure 45*****;**
*;* ***Figure 60******;*** ***Figure 62******;*** ***Figure 63; Figure*** 64 include (but are not limited to time interrelationship (between NLDBV COH applicable data set to be examined to be examined for the presence of COH - coherence applicable data parameters can be configured in accordance to cursor selected or automatically selected parameters. For example in the case of detected SR the coherence analysis can automatically be configured to be activated in accordance to plus and minus 25% from peak magnitude point of SR, whereby any other brain source localised signals falling within this amplitude range would be denoted as coherent linked data.

The present example further enables further enables, coherence brain signal analysis processes whereby temporal interrelationships are examine, whereby "COH time lead" and **"COH lag time"** parameters can be configured to be activated in accordance to plus and minus 30 % from **peak magnitude time point of SR,** whereby any other brain source localised signals falling within the selected **COH magnitude range** as well as plus 30% **(COH lead time)** of the total SR total time and minus 30% **(COH lag time)** of the SR total time period range can would be denoted as coherent linked data. Moreover, the visual coherent arcs can be annotated in accordance to **COH central magnitude** (i.e. centre cursor select), **COH minimum magnitude** (i.e. left cursor select of event of interest from which source reconstructions is being computed. This can be displayed as a butterfly plot to enable left, centre and right adjustable cursors to select actual start, end and peak or principal point of interest on said event of interest.

Separate cursors or interlocked cursors can also be configured for COH lead and lag times, and also for plus and minus **COH magnitude range selections** and **COH maximum magnitude** (i.e. right cursor) parameters. Alternatively COH time and magnitude parameter settings can be licked to one set of cursor or automatic settings versus independent parameter settings.

The present example further enables a direction of coherence ARC visualisation, tabular, graph, statistical measures, where by direction of coherence interconnection (ARC) can be determined in a manner whereby the representation of two interconnecting brain coherence arc locations can be configured in accordance to the parameter configuration of a **"forward COH arc direction"** parameter configuration or a "reverse COH arc direction".

In the case of the **"forward COH arc direction"** parameter configuration the brain activation qualifying in accordance to the "COH magnitude range" parameter configuration or "COH sensitivity parameter" configuration (i.e. COH interconnectivity arcs can be determined based on a spectral or NLDB (subject to COH analysis format) **"COH** sensitivity **parameter"** configuration, whereby for example any brain activation regions measured in accordance to the sensitivity parameter. From a temporal directivity COH ARC perspective the earliest brain activation region relating to COH interconnectivity would be could be designated as the originating location of the coherence interconnectivity (OLC), with the latent brain activation region designated as the destination of the coherence interconnectivity (DLC). The inverse situation applies in terms of ARC arrow COH directivity when the **"reverse COH arc direction"** parameter is selected.

Based on this example and from a visualisation perspective the COH ARC in this case could have the arrow-head or COH ARC directivity indication located at the OLC while the non-arrow end would be allocated to the DLC brain region.

From the example of STV the present example enables a similar approach whereby the leading or earlier COH STV brain activation region would be designated the OLC without arrow head and the DLC assigned the COH ARC arrow head.

This coherent linked data applies similarly to any of or any combination of transformation analysis formations including NLDBTV, STV and/or monitored neurology **EEG** (i.e. sLORETA, CDR etc. per figures ***Figure** 45**;*
*;* ***Figure** 60**;* ***Figure** 62**;* ***Figure** 63; **Figure*** 64) or voxel imaged signals.

### NLDBVT PROCESS EXAMPLES

### (SE or ER event of interest detection)

### (NLDBVs computed)

>In a further process (or next step) previously monitored time series data across a defined period (epoch) of time (based on parameter settings of epoch and step adjustment covered elsewhere) undergoes a complexity analysis transformation or entropy analysis transformation or spectral followed by entropy analysis transformation (i.e. Shannon's entropy analysis) . In particular entropy or complexity value (NLDBV) can be computed at consecutive steps in accordance NLDBV parameter settings including "NLDBV step" , "NLDBV epoch", "NLDBV sensitivity" (i.e. see elsewhere peaks and trough signal complexity regularity or variance).

### (Array generated for each consecutive step and epoch period)

> In a further process (or next step) consecutive entropy values computed for each "step" across the respective "epoch" time periods can be stored (i.e. time series of NLDB values stored in an array of computed values with each corresponding time period across which each value was computed).

### (Threshold magnitude/degree/level) value for ER event of interest & NLDB EOI detection)

> The present example in in a further process (or next step) the NLDBVs can be tracked and compared to a predefined (or optionally dynamically computed EOI threshold NLDBV value which marks the degree or magnitude of entropy or complexity corresponding to a signal period of interest. It is important to configure NLDBV appropriately or automatically. i.e. the present example can be configured to enable any of or any combination of "health conditions or symptoms of interest" or "events of interest/EOI" (per "EOI DEFINITION" detailed in abbreviation table at rear of this document). i.e. HFOs, ripples, spindles, epileptiform discharge spike and waves discharges, K-complexes, spindles, alpha bursts). The NLDBV EOI parameter configuration function ensures EOI are discernible and not excessively not skipped, or diminished.

### (SNR value for ER, SR, CNY EEG or other neurological or physiological NLDB EOI detection)

>The present example in a further process (or next step) the NLDBVs can be tracked and compared to a predefined (or optionally dynamically computed) EOI SNR NLDBV value which marks the degree or magnitude of entropy or complexity of the signal for each consecutive "step" and " period" that should be flagged as the start or end of a NLDBTV EOI.

The present example enables the EOI SNR NLDBTS parameters to be configured. For example (but not limited to), the most recently monitored epoch NLDBV (time-series of values or NLDBTV, as derived from **EEG** electrode signals or image data elements/voxels) can be analysed and compared or contrasted to any number of past NLDBV epoch values (i.e. noise denominator of signal to noise ratio equation (Signal/Noise equation could comprise of, for example, the average NLDBV of the past say "10" "epochs" (or as per configuration of NLDBV SNR parameters). In this case the NLDBV SNR threshold (being the determination thresholds between signals or EOI versus background noise or unwanted signals) level can be set (for example only) to a ratio of "2" " (or "6db") whereby the "threshold" SNR value would be configured to track signals of events of interest where the corresponding NLDBV is a magnitude twice that of the average of the past "10" NLDBV epochs).

Based on this scenario (as an example only), an **EEG** alpha burst of 1 second that falls within the NLDBV sensitivity setting (i.e. assuming "NLDBTV sensitivity parameter" is set to 10% - or in case of complexity analysis parameter configuration, any peak and trough values not varying in synchronisation or more than 10% in terms of 1/2 cycle time period or the peak to trough (alpha signal) inter-interval variation of less than 10% value from that of the average inter-interval peak to trough value computed for the respective EOI. Other NLDB EOI parameter configuration settings could be scale factors - i.e. NLDB scale factor or in one configuration setting the maximum complexity value (index value) at "0" when the EOI is a perfectly repetitious cyclic pattern such as a sine wave or sawtooth or triangular-wave generated by a signal generator with 100% deterministic or regularity or repetitious nature. Conversely, the lower end of the entropy or complexity index value scale could be set to "1" when the EOI or epoch under computation comprises of totally randomised data such as in the in the case of pink or white noise, for example.

### Epoch based NLDBV (E NLBV) versus spontaneous event EOI NLDBV (SE EOI NLDBV) versus evoked response EOI NLDBV (or ER EOI NLDBV).

The present example further enables continuous **EEG** monitored signal NLDB analysis transformations including any of or any combination of (but not limited to):
a) E NLBV refers to computation of entropy or complexity value over an epoch period, while
b) SP EOI NLDBV refers to computation of entropy or complexity value as it relates to a spontaneous event EOI, while c) ER EOI NLDBV refers to computation of entropy or complexity value as it relates to a evoked response EOI;

Additionally, evoked response (ER) **EEG** monitored signal NLDB analysis transformations include:
The present example further enables a) ER SP EOI NLDBV comprising of computation of entropy or complexity value as it relates to a spontaneous event EOI monitored during ER test paradigms (such as AEP or MMN etc.), and/or b) ER EOI NLDBV refers to computation of entropy or complexity value as it relates to a evoked response EOI monitored during ER test paradigms (such as AEP or MMN etc.),

### Spontaneous event (SE) EOI

The present example further enables spontaneous event (SE) determination whereby events can be identified by way of identifying the start and end of event (i.e. with start point of SR being point in time that NLDBV SNR exceeds threshold to point in time that NLDBV SNR no longer exceeds threshold or SNR parameter).

### NLDBV epoch classification parameter

The present example further enables "NLDBV epoch classification parameter" whereby for example, if this parameter is set to 50% then any epoch with 50% or greater of that epoch exceeding NLDBV SNR or threshold value will be classified as a NLDBV epoch.

### (NLDB CDR)

### Process - NLDBTV SNR threshold flagged source localised event of interest determination:

>> The present example further enables in a first process (or step) NLDB CDR can be computed by way of (for example only) configuring "step parameter" to 1 millisecond in order to enable NLDBV time series array of data to be computed for each monitored electrode array. An indexed and scaled NLDBV can be computed (per description in preceding text) each 1 millisecond step for the preceding epoch period.

### (f. source localised measures)

- The present example further enables source localised measures supporting the prognostic or diagnostic identification or determination of markers and/or physiological sources associated with EOI comprising any of or any combination of means of enabling any combination of NLDBTV, STV or conventional neurology imaging measures (i.e. voxel image sections) or **EEG** activation measures (i.e. **EEG** current density or voltage potentials) as further outlined in section titled "EXAMPLE OF AUTOMATIC ONLINE NLDB ANALYSIS AND SPONTANEOUS OR EVOKED EVENTS OF INTEREST DETERMINATION WITH SYNCHRONISED SLEEP/WAKE HYPNOGRAM AND SINGLE OR CLUSTER ANALYSIS OR ENSEMBLES OF EVENTS OF INTEREST INDICATIONS AND/OR BIOFEEDBACK TREATEMENT CAPABILITIES", and/or as detailed in other sections elsewhere in this document;

### (g. sleep measures)

- The present example further enables sleep measures supporting the prognostic or diagnostic identification or determination of markers and/or physiological sources associated with EOI comprising any of or any combination of means of enabling any combination of **NLDBTV, STV, ER, and/or SR, and/or conventional neurology imaging measures** (i.e. voxel image sections) and/or **EEG** activation measures (i.e. **EEG** current density or voltage potentials) as further outlined in section titled ***"******Physiological** and*/*or **Sleep** and*/*or **Wake Markers:"*** and/or as detailed in other sections elsewhere in this document;

### (h. circadian clock measures)

- The present example further **circadian clock measures** capable of identifying neural sources along with neural source generation anatomical locations comprising any of or any combination of (but not limited to): forward or reverse equation based source localisation (as covered elsewhere in this document), EOI determination, NLDBTV, STV, SR, ER, clusters of ERs, clusters of SRs, spectral EOI, interconnectivity of any of these listed EOI (i.e. coherence and /or Dipole sequences and/or associated clusters, sequences and/or ensembles) and/or neurological amplitude, power, morphological signals or values;

### (i. epilepsy or other seizure measures),

- The present example further enables **epilepsy or other neurological markers** supporting the prognostic or diagnostic identification of neural sources or other physiological sources and/or associated EOI along with source reconstruction, or related determination comprising any of or any combination of means of enabling any combination of **NLDBTV, STV or conventional neurology imaging measures** (i.e. voxel image sections) or **EEG** activation measures (i.e. **EEG** current density or voltage potentials) as further outlined in sections titled ***"****[29]** Automatic* **analysis model Determination:** 1.SPECTRAL BIOMARKER EVENTS:" or ***"Title: eLifeNEUROI EPILEPSY** ".*

PATENT TITLE: Non Linear Dynamical Based Analysis (NDBA) with option of spontaneous automatic online analysis", and/or other sections detailed elsewhere in this document;

### (j. brain connectivity measures)

- The present example further enables **brain connectivity measures** (i.e. **but not limited** to **Dipole modelling or coherence measures)** supporting the prognostic or diagnostic identification of neural sources or other physiological sources and/or associated EOI along with source reconstruction, or related determination comprising any of or any combination of means of enabling any combination of **NLDBTV, STV or conventional neurology imaging measures** (i.e. voxel image sections) or **EEG** activation measures (i.e. **EEG** current density or voltage potentials) as further outlined in sections titled ***"[29]** Automatic* **analysis model Determination:** 1.SPECTRAL BIOMARKER EVENTS:" or ***"Title: eLifeNEURO*/** *EPILEPSY ".*

PATENT TITLE: Non Linear Dynamical Based Analysis (NDBA) with option of spontaneous automatic online analysis".

### Title: eLifeNLDB Non-Linear Dynamical Based (NLDB) analysis with option of automatic determination and option of online distributed processing capable of spontaneous source localisation applicable to diagnostic or prognostic determination and/or biofeedback or treatment of consumer/patient." herein and/or other sections detailed elsewhere in this document;

### (k. spectral measures)

- The present example further enables **spectral measures** supporting the prognostic or diagnostic identification of neural sources or other physiological sources and/or associated EOI along with source reconstruction, or related determination comprising any of or any combination of means of enabling any combination of **NLDBTV, STV or conventional neurology imaging** measures (i.e. voxel image sections) or **EEG** activation measures (i.e. **EEG** current density or voltage potentials) as further outlined in sections titled ***"****[29]** Automatic* **analysis model Determination:** 1.SPECTRAL BIOMARKER EVENTS:" or ***"Title: eLifeNEURO*/*****EPILEPSY** ",*

PATENT TITLE: Non Linear Dynamical Based Analysis (NDBA) with option of spontaneous automatic online analysis",

### Title: eLifeNLDB Non-Linear Dynamical Based (NLDB) analysis with option of automatic determination and option of online distributed processing capable of spontaneous source localisation applicable to diagnostic or prognostic determination and/or biofeedback or treatment of consumer/patient." herein and/or other sections detailed elsewhere in this document;

### (I. infrared reflectance oculography measures)

- The present example further enables infrared reflectance oculography supporting the prognostic or diagnostic identification of neural sources or other physiological sources and/or associated EOI along with source reconstruction, or related determination comprising any of or any combination of means of enabling any combination of **NLDBTV, STV or conventional neurology imaging measures** (i.e. voxel image sections) or **EEG** activation measures (i.e. **EEG** current density or voltage potentials) as further outlined in sections titled *"Title: eLifeALERT"* and/or other sections detailed elsewhere in this document;

### (m. cluster analysis or ensembles/sequences of events)

***>>****clustered** (or **ensemble** or **sequence) analysis** of brain connectivity **(CABC;** including coherence, **Dipole),***

- spectral spontaneous event source localisation measures
- NLDB spontaneous event source localisation measures
- coupled with spatiotemporal dynamic analysis (SDA),
- NLDB source localisation measures coupled with spatiotemporal dynamic analysis (SDA),

*[i.e. spatiotemporal appearance biomarkers applicable to of discernible "events of interest" appearance based on cluster analysis means (i.e. incorporating any* of *or any combination of clusters* of *said* a) *to I) including, but not limited to, precursors determined* as *applicable to (but not limited to) "health conditions* or symptoms *of interest" or* "events of interest/EOI" (per "EOI DEFINITION" detailed in abbreviation table at rear of this document), *whereby such clusters can be determined based on comparative assessment with previously subject-patient monitored data (i.e. but not limited to pre-diagnostic studies) and*/*or or other subject*/*patient-specific information and*/*or via comparative reference to relevant population databases (see also figures with data based aggregation Fiqure 77;* ***Figure** 78**;* ***Figure** 79**;* ***Figure 80*****)],**

### n) (wearable-device minimisation (WM) system)

- The present example incorporates a means of determining a minimised monitoring configuration capable of effective monitoring of any subject/patient in accordance to the goals of monitoring and as applicable to the events of interest (EOI; per glossary);

### (algorithm subject/patient-specific learning (ASL) system)

- The present example incorporates a means of fine-tuning monitoring algorithm in accordance to specific patient monitoring goals *(Fiqure **45;*** ***Figure 75******;*** ***Figure 76******;*** ***Figure 77******;*** ***Figure 78******;*** ***Figure** 79**;* ***Figure** 80**)* and as applicable to the events of interest *"health conditions* or symptoms of ***interest"*** or "events of interest/EOI" (per "EOI DEFINITION" detailed in abbreviation table at rear of this document).

### (mobile-wireless distributed analysis (MDA) system)

- Whereby said monitoring method(s) and device(s) can incorporate communication interface(s) comprising any one or more wide area network (WAN), local area network (LAN), internet protocol (IP), network application services (NAS), and/or Cloud-computing services to enable the transmission of physiological or environmental monitored data and/or subject/patient information exchange and/or access to online analysis capabilities capable of supplementing local monitoring, analysis, storage, and/or indications;

### Title: eLifeNLDB Non-Linear Dynamical Based (NLDB) analysis with option of automatic determination and option of online distributed processing capable of spontaneous source localisation applicable to diagnostic or prognostic determination and/or biofeedback or treatment of consumer/patient.

The present example incorporates Non Linear Dynamical Based (NLDB) Analysis with option of spontaneous automatic online analysis including, but not limited to, analyses applicable to neurophysiological, cognitive, psychological//mental, and/or other physiological disorders, impairment or events or health conditions of interest, further comprising with option of automatic determination and option of online distributed processing capable of spontaneous source localisation applicable to diagnostic or prognostic determination, and further options of biofeedback and/or treatment of consumer/patient, the example further comprising any of or any combination of:
- means of applying analyse s transformation(s) one or more channels of **EEG** monitored data;
- whereby said analyses transformation includes (but is not limited to) to a **change** of signal **dynamics** properties including non-linear dynamic **signal analysis** outcomes and/or associated signal properties (i.e. but not limited to entropy, complexity analysis, bispectrum, FFT outputs etc.) during events such as (but not limited to), changes in depth of anaesthesia, consciousness states, altered states of consciousness, seizures, k-complexes, high-frequency oscillations, ripples, high-frequency ripples, spindles, **EEG** vertex waves, **EEG** seizure signals, **EEG** seizure onset signals, **EEG** spectral bursts or changes, **EEG** sleep staging signal property changes with sleep-related events or disturbances or events, arousals, body movements, sleep breathing disorders, sleep breathing disturbances, sleep disturbances or other events, sleep stages or states, different attention levels, sedation levels, awareness levels, drowsiness levels, vigilance levels, autism spectral disorders and different levels of severity, Parkinson's Huntington's, dementia, Alzheimer's, depression, and other events or health conditions of interest and/or psychological, mental or **physiological** disorders, impairment or disease (i.e see also Figure 75; to Figure 80; Figure 55 to Figure 74; Figure **84****;** Figure 94)
- means of fast averaging online averaging online monitored **EEG** data from a subject, whereby such means includes but is not limited to automatic **regression** modelling and averaged data with an external input function in order to minimise the delay associated with said averaging online **EEG** monitored and/or further processed data;
- means of applying NLDB **analysis** to any of or any combination of monitored **EEG** signals of a subject, whereby such means can be entropy or complexity analysis (for example but not limited to);
- whereby said monitored **EEG** data can comprise of evoked responses **generated** from an external stimulus **(such as** but not limited to auditory stimulus to one or both subject's ears);
- whereby said monitored **EEG** data can comprise of defining any event within **monitored EEG data or other physiological data,** whereby such events could be detected by way of characterisation of specific signal amplitude thresholds or characteristics and/or by way of definition of spectral properties of **EEG** signal and/or by way of exceeding predefined, ranges, levels or changes in **non-linear dynamic properties of analysed EEG signals;**
- whereby in the *first step* of a NLDB process a series of evoked response **EEG** waveforms (i.e. **synchronised** to **originating stimulus signal, such** as **auditory stimulus signal)** can be averaged by way of **averaging** a **number of** successive **evoked EEG responses** (artefact can be excluded), and whereby **said autoregressive** average **modelling with or without external input function can be deployed;**
- whereby in a *second process step* the determination of signal complexity (or entropy) value could be generated by analysing data sample values of an averaged value (for example but not limited to), whereby (in one example) an average comprising of 256 sample points (such as an evoked response covering 0 to 120ms post-latency region, for example only) **the computation of the subtraction of each successive sample point value from the previous sample point value could establish the "differential AEP averaged (i.e. based on computing values for an averaged sequence of AEP responses) the voltage value"** - i.e **the generation of** a **"differential amplitude version of the averaged waveform";**
- whereby in a *third step non-linear dynamic analysis* (i.e. such as but not limited to entropy or complexity analysis) transformation can be applied to the **"differential amplitude version of the averaged waveform"** as a means of generating a measure or index value corresponding to the said **"differential amplitude version of the averaged waveform"; (identified in second step of process).**
- whereby the deployment of these enables the generation of: 1) consecutive running AEP averages, per said process step 1, 2) **"differential amplitude version of the averaged waveform"** consecutive "running averages", per **said process step 2; 3)** non-linear dynamic index or measures associated per said *process step 3.*
- whereby in **process step 1** successive averages can comprise of bock of time **period and/or block of past evoked response signals** (including possibility of selecting any range of evoked response pre-stimulus or post stimulus signal values) of past monitored **EEG** data of any or any combination of subject monitored **EEG** or other physiological signals;
- whereby in process **step 1,** successive **averages can be compute in steps of any time period,** comprising of the time between successive past averages or past data computations (i.e. but not limited to 1 second, 2 seconds, 10 seconds etc.);
- whereby in this manner a non-linear dynamic value or continuously updated value/index can be computed for any monitored evoked response or averaged series of evoked responses, or consecutive steps of evoked response blocks of averages.

The present example further incorporates the ability to detect **outcomes of neural generators implicated** as **precursors and/or with the incident of neurological disorders** or changes in consciousness and/or altered consciousness states (epilepsy, vigilance, sedation and anaesthesia) and/or transitions of consciousness states in general, along with more specific transition and states by way of applying non-linear dynamic (NLD) analysis transformations, whereby computed NLD changes capable of **comparing and/or contrasting deterministic versus randomised signal properties** can serve as a prognostic or diagnostic tool for the determination or highlighting of events or health conditions of interest. For example, signal regularity measures applicable to brain sources (neural generators) such as those associated with **slow-wave (Delta) sleep, sleep spindles, onset and incident of seizures, deep-anaesthesia "saw tooth" neurological signals, high-frequency oscillations, high-frequency bursts, ripples, k-complexes, vertex** waves, spectral bursts or changes in **EEG** energy, along with other events or health conditions of interest can be detected using these new NLD-based principles but also be examined in terms of spatial dynamic NLD-based factors. These new NLDB measures can have profound implications when it comes to the investigation of consciousness, sleep, anaesthesia, epilepsy and many more of the most puzzling enigmas of modern science.

The present example further enables the said **non-linear dynamic measures** to **be** deployed as part of the decision matrix utilised in the determination of treatment or therapeutic system control, whereby said computer control can include but is not limited to administration, drug infusion, medication recommended administration, magnetic stimulation, electrical stimulation, visual or auditory biofeedback (linked to **EEG** analysis outcomes) or feedback, non-invasive positive ventilation or positive airway treatment, along with other treatment of biofeedback deployment;

### Spontaneous event detection with option of source localisation and/or NLDB determination

The present example can substitute a spontaneous event determination as a variation in the first process step (i.e. the present example can detect changes (i.e. such as but not limited to predefined thresholds or ranges (i.e. an **EEG** spindle, high frequency oscillation, or high frequency ripple event would, for example, would have a lower degree of complexity or lower entropy due to regularity or deterministic nature of signal (i.e. repetitious or cyclic nature of signal morphology of these events) versus irregularity of the spindle properties. Conversely, asymmetrical morphological signal properties of events such as k-complex **EEG** event or body movements could typically result in an increase in signal complexity and consequently higher degree of complexity or entropy value), whereby the said spontaneous event determination can be used in conjunction with other analysis (i.e. spectral, coherence etc.) or solely as a marker of brain activation event for additional non-linear dynamic **based** (NLDB) **processing steps;**

**Said** additional NLDB processing steps can be deployed in conjunction or in substitution of source localisation computations (i.e. for example but not limited to generating brain activation values and magnitude of values based on determination of NLDB analysis measures for one or more **EEG** monitored signals. Moreover, these NLDB measures and associated magnitudes or NLDB analysis measures can be computed using forward problem (for example, in the case of the forward problem the magnitude and location of values inside of the head are known, while monitored **EEG** (functional data) scalp data is unknown) and inverse problem (for example, in the case of the inverse problem the magnitude and location of monitored **EEG** (functional data) scalp data values are known, while the neural source or sources in the head are unknown.). The NLDB source localisation capability comprises of calculating two different classes of source models, based on distributed and local sources, where the distributed sources can be determined by utilising current density analysis methods, while local sources are computed by dipole analysis fitting models.

The NLDB analysis transform comprises of computing distributed sources by way of non-linear dynamic magnitude (NLDM), whereby conventional current density computational methods can be deployed except the NLD level/magnitude is substituted for the conventional current density factor, but whereby a source localisation visualisation (i.e. NLDM substituted for conventional **EEG** amplitude levels), topographic maps (i.e. NLDM substituted for conventional **EEG** amplitude levels and signal to noise thresholds are represented by signal NLD signal magnitude/levels), and where PCA or ICA analysis (i.e. NLD signal magnitude/levels (NLDSM) are compared to NLD noise/background-signals magnitude/levels (NLDNM) or NLDSM:NLDNM, compared to conventional signal to noise or S:N ratio).

The present example enables determination of non-linear dynamical coherence whereby conventional **EEG** signal amplitudes are substituted by **EEG** non-linear dynamic magnitude, and whereby statistical and/or visualisation of determination of non-linear dynamic arcs (NLDA) can be processed using similar lag and lead timing and range of levels (i.e. NLDM versus clip or amplitude levels in conventional **EEG** source localisation coherence computations); The present example can computed NLDB analysis transform comprising of computing distributed sources by way of non-linear dynamic magnitude (NLDM), in conjunction with conventional distributed sources determined by utilising current density analysis methods, along with local sources computed by dipole analysis fitting models; NLDBTV, STV, SR, ER, clusters of ERs, clusters of SRs, spectral EOI, interconnectivity of any of these listed EOI (i.e. coherence and /or Dipole sequences and/or associated clusters, sequences and/or ensembles) and/or neurological amplitude, power, morphological signals or values;

### EXAMPLES AND DESCRIPTIONS OF AUTOMATIC ONLINE NLDB ANALYSIS AND SPONTANEOUS OR EVOKED EVENTS OF INTEREST DETERMINATION WITH SYNCHRONISED SLEEP/WAKE HYPNOGRAM AND SINGLE OR CLUSTER ANALYSIS OR ENSEMBLES OF EVENTS OF INTEREST INDICATIONS AND/OR BIOFEEDBACK TREATEMENT CAPABILITIES

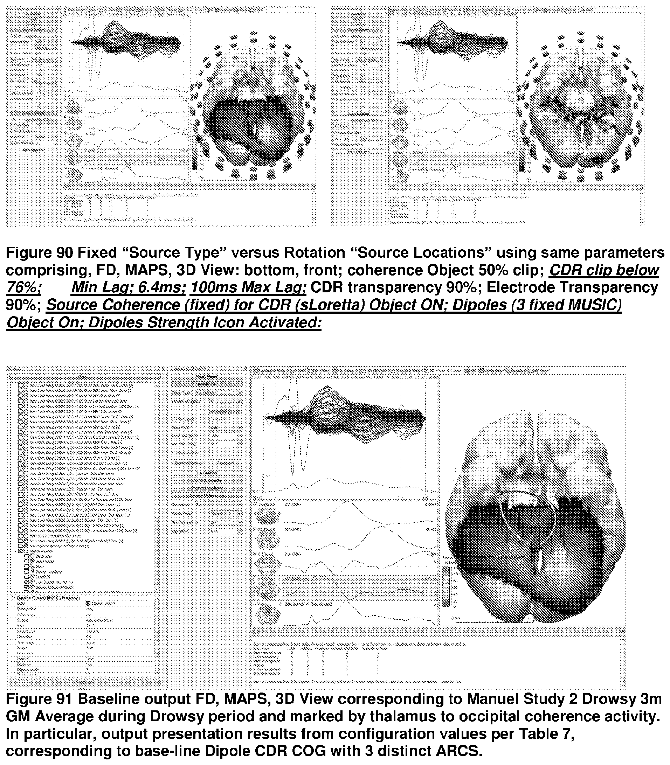

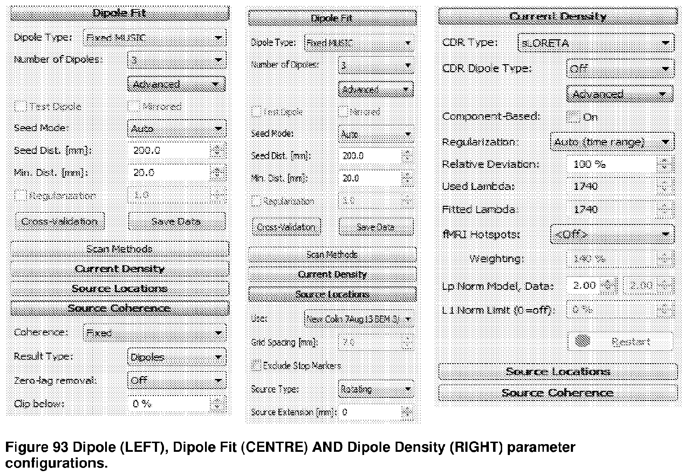

This application comprises of a number of example including a system with computer implemented method(s) comprising of a diagnostic and/or prognostic platform with a means of enabling online monitoring via a mobile wireless connected or other computer network connected system interfacing to a subject/patient wearable or nearby proximity physiological or environmental monitoring device, the computer implemented method comprising any of or any combination of:
- a computer system incorporating one or more processes with memory storage capability involving one or more computer programs which can be executed by one or more processes, the one of more computer programs including instructions or applications enabling any of or any combination of:
   - a means of monitoring a subject/patient with option of online mobile wireless monitoring incorporating a number of wearable sensor devices configured from the simpler/minimal (i.e. per ***Figure** 43**,* Figure 46 to Figure 50, Figure 52, *;* ***Figure** 53**;* ***Figure** 54**)* or more complex configurations (i.e. per ***Figure** 49**;* ***Figure** 51**;* ***Figure** 45**),* applicable to the "event(s) of interest or health condition(s) of interest" whereby determination of means of monitoring can be based on monitoring algorithms and device complexity or minimisation (i.e. figure 1 "monitoring goal" (i.e. figure 35), analysis variants (Figure 45), monitoring goals, monitoring parameter determination, monitoring device determination, monitoring algorithm and associated analysis variants determination **(*****Figure 77******;;*** ***Figure** 79**;* ***Figure** 80**;* ***Figure** 81**);*
   - a means of presenting stimulus to subject (i.e. auditory stimulus via earbuds, including conventional format entertainment earbuds, earphones or head-phones; magnetic stimulus via magnetic coils; electrical stimulus; vibration or other movement or pressure applied stimulus; temperature or other sensor or pain applied stimulus; whereby said stimulus can be attached or embedded within head-applied systems (i.e. such as but not limited to ***Figure** 43**,* Figure 46 to Figure 50, Figure 52, ***;** Fiqure **48;*** ***Figure 49******;*** ***Figure 50******;*** ***Figure 51******;*** ***Figure 52******;*** ***Figure 53******;*** ***Figure 54*****).**
   - **means** of signal processing (i.e. where means can include but is not limited to signal processing including filtering, de-noising and/or artefact reduction);
   - means of incorporating signal analysis (i.e. but not limited to averaging two or more responses as a means of increasing signal to noise of resultant outcomes, whereby such analysis means can include a signal averaging process comprising of time-alignment of response signals, in a manner where the start of each averaged response signal being averaged with other response(s) is time-synchronised with reference to the stimulus generation signal associated with generation of subject's evoked response;
   - means of analysing most recent averaged evoked response signals with earlier monitored and averaged evoked response signals, (whereby such means includes identifying time-series of monitored signal to be averaged in terms of a re-defined start and end time computed with respect to an evoked response stimulus signal presented to the subject under monitoring, and then averaging a plurality of the said time-series signals);
   - means of determining a deviation between the outcomes of the most recently monitored averaged signals with earlier monitored comparative base-line averaged signals, where means of determining deviation can be by way of computing mismatched negativity (MMN); or standard/deviant AEP test paradigms (i.e. such as comparing a standard train of evoked responses with a deviant or "odd-ball" evoked train of responses, or other waveform characterisation, spectral, linear and/or non-linear signal dynamics variation between most recently monitored averaged evoked responses and previously monitored base-line of averaged evoked responses;
   - means of associating MMN, odd-ball or other AEP test paradigm as a measure (i.e. but not limited to any of or any combination of indicators and/or report and/or alert and/or notification of awareness and/or attention and/or vigilance states etc.) of a subject's consciousness states, consciousness transitions, attention, awareness, vigilance, cognitive impairment, focus of tasks, drowsiness, and/or other psychological or physiological states and/or health conditions and/or events of interest;
      - means of identifying signals or evoked responses of interest and/or for purpose of analysis averaging by way of spontaneous or stimulus time-synchronised approaches;
   - means of comprising of means of determination of events of interest by way of signal to noise ratio **(SNR)** means (i.e. such as but not limited to averaged SNR, fixed point SNR, fixed single point SNR/Fsp/F-test Fsp, +- difference estimate SNR measures or other SNR methods, SNR means includes (but not limited to) amplitude methods such as determination of signals that exceed 2-fold or 3-fold, of signal amplitude versus background signals;
   - SNR measures deployed in the identification/determination of events of interest include any of or any combination of (but not limited to) determination of poor AEP signal quality will result in unreliable and inconsistent results; on-line SNR measure enabling the AEP recording quality to be assessed and/or compensated or improved during recording including compensation against poor AEP signal quality which results in unreliable and inconsistent results such as (but not limited to) the use of on-line SNR measure can enable the AEP recording quality to be assessed including single point F-test Fsp ⁹⁰; +- difference estimate ⁹¹ SNR measures;
   - where by means "spontaneous or stimulus approaches" of identifying **events** of interest, comprises identifying one or more or any combination of **EEG** channel signals in terms of predefined (i.e. via automatic levels or system user determined ranges, thresholds or normal versus abnormal operating signal regions or operation) according to signal morphology or pattern identification (i.e. events of interest can include any of or any combination of, but not limited to, elevated electromyography (EMG) rapid eye movement (REM) sleep (compared to other sleep stages; as in the case of sleep behaviour disorder (SBD); K-complex **EEG** events; **EEG** spindle events; **EEG** high frequency oscillation (HFO) events; **EEG** ripple events; **EEG** arousal events; sleep and wake states spectral levels or ratios of any of or any combination of frequency band of or approximate to delta/slow wave (0.1-4 Hz), theta (4-8 Hz), alpha (8-12 Hz), beta (12-30 Hz), low-gamma (30-70 Hz), and/or high-gamma (70-180 Hz);
   - whereby analysis to detect these said "events of interest" can include any of or any of or any combination of signal dynamic approached (i.e. including but not limited to non-linear dynamic analysis such as entropy, complexity and other non-linear dynamic analysis means), where non-linear dynamic means can incorporate identifying time-series data or spectral data derivations (i.e. FFT) of data segments or time-series determination means that exceed or fall outside certain specified limits -
   - where any of or any combination of events of interest can be determined by way of analyses comprising of any of or any combination of:
      ***a*)** detection of non-linear dynamic analysis **EEG** monitored signal analysis transformations of time series data that exceed or fall outside certain specified limits" include which fall outside of pre-defined or configured parameters (i.e. ranges, thresholds, changes etc.);
      ***b)*** detection of spectral analysis **EEG** monitored signal analysis transformations of time series data that exceed or fall outside certain specified limits" include which fall outside of pre-defined or configured parameters (i.e. ranges, thresholds, changes etc.);
      ***c)*** **detection** of coherence between signals of two or more **EEG** monitored signal analysis transformations of time series data that exceed or fall outside certain specified limits" include which fall outside of pre-defined or configured parameters (i.e. ranges, thresholds, changes etc.);
      d) detection of **signal morphology** or **signal** pattern characteristics of **EEG** monitored signal analysis transformations of time series data that exceed or fall outside certain specified limits" include which fall outside of pre-defined or configured parameters (i.e. ranges, thresholds, changes etc.);
   - means of defining cluster analysis characteristics of a plurality of "events of interest" as a means of detecting health conditions or ensembles of events of interest;
   - means of spatiotemporal dynamics characterisation comprising of automatically or computer-assistance means of identifying singular or averaged spontaneous and/or evoked responses of interest whereby the present example further provides a means of statistical (see **figures and associated descriptions, cross-references per neural source localisation, functional data, topographic maps, image data, sLORETA, Dipole, coherence outcomes, coherence statistical arcs and current density source localisation, neurology figures showing examples of Curry visual and statistical outcomes per examples** (i.e. **but not limited** to ***Figure 45******;***
      ***;*** ***Figure** 56**;* ***Figure** 57**;* ***Figure** 58**;* ***Figure** 59**;* ***Figure** 60**;* *Figure 61**;* ***Figure** 62**;* ***Figure 63******;** Fiqure **64:*** ***Figure** 65; **Figure*** 66; ***Figure 67******:*** ***Figure*** 68; ***Figure*** 69; ***Figure** 70**;* ***Figure** 71**;* ***Figure 72******;*** ***Figure** 73**;* ***Figure 74******;*** ***Figure 84; Figure** 85**;* ***Figure*** 86; ***Figure** 87**;* ***Figure*** 88; ***Figure** 89**;* ***Figure** 90; **Figure** 91**;* ***Figure** 92**;* ***Figure** 93**;* ***Figure 94*****).**
   - means of **spatiotemporal dynamics characterisation** comprising of **automatically** or computer-assistance means of identifying **singular or averaged spontaneous** and/or evoked responses of interest whereby the present example further provides a means **of statistical and/or visualisation means** (see **per** ***Figure** 84; **Figure** 85**;* ***Figure*** 86; ***Figure** 87**;* ***Figure*** 88; ***Figure** 89**; Fiqure **90;*** ***Figure** 91**; Fiqure 92;* ***Figure** 93**;* ***Figure 94*****).**
   - means of **spatiotemporal dynamics characterisation** comprising of **automatically** or computer-assistance means of identifying **singular or averaged spontaneous** and/or evoked responses of interest whereby the present example further provides a means **of statistical and/or visualisation means** including any of identifying events of interest and/or averaging events of interest and/or incorporating patient-specific or general head models as means of implementing **source localisation** of identified/determined averaged or singular spontaneous or evoked response events of interest and/or head model segmentation of source reconstruction analysis outcomes, and/or cross-sectional visualisation cuts or dissection of source localised outcomes and/or indicators highlighting clusters or single source localised events of interest (i.e. **sLORETA** current density reconstruction (CDR) source localisation analysis transformations ***Figure 84; Figure** 85**;* ***Figure*** 86; ***Figure** 87**;* ***Figure*** 88; ***Figure*** 89; ***Figure** 90; **Figure** 91**;* ***Figure** 92**;* ***Figure** 93**;* ***Figure 94*****).**

### AUTOMATIC ONLINE TIME-SYNCHRONISED HYPNOGRAM AND SPATIOTEMPOAL DNAMICS CHARACTERISATION

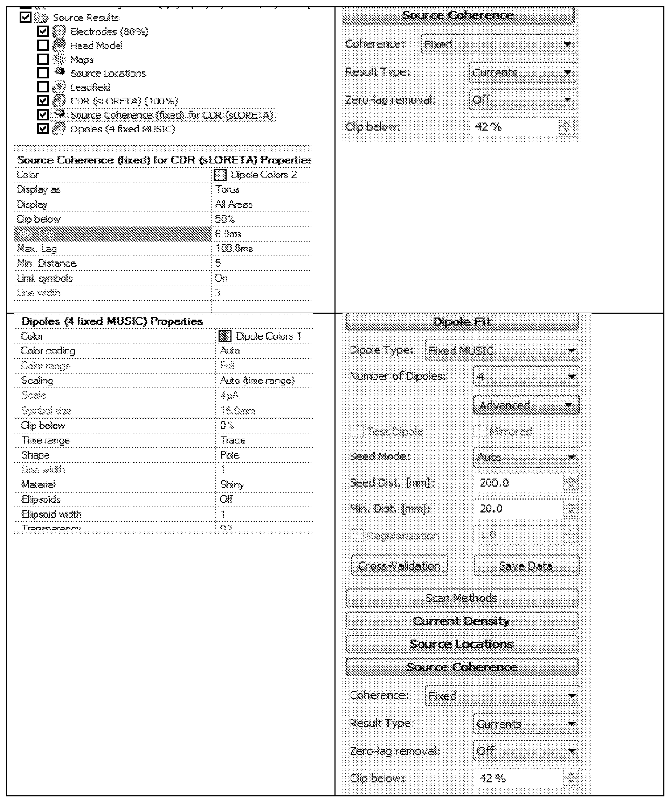

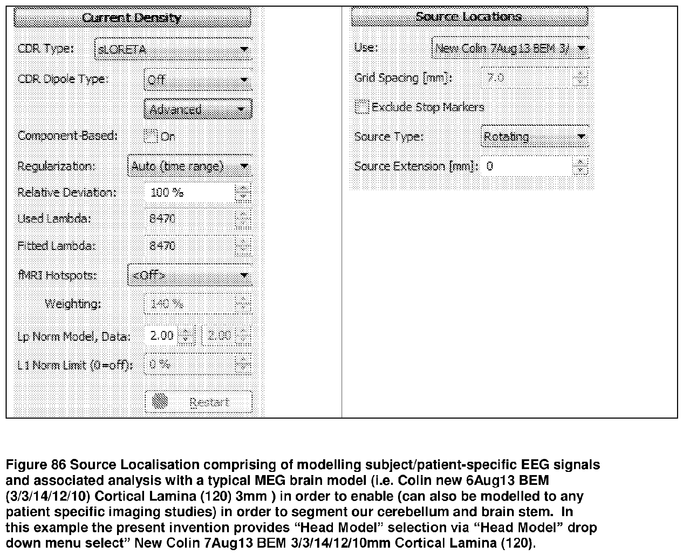

(i.e. **but not limited** to ***Figure** 45**;* ***Figure** 56**;* ***Figure** 57**;* ***Figure** 58**; Fiqure 59;* ***Figure** 60**;* ***Figure** 61**;* ***Figure** 62**;* ***Figure** 63**;* ***Figure 64******;*** ***Figure** 65**;* ***Figure*** 66; ***Figure** 67**;* ***Figure*** 68; ***Figure** 69**;* ***Figure** 70**;* ***Figure** 71**;* ***Figure 72******;*** ***Figure 73******;*** ***Figure 74******;*** ***Figure** 84**;* ***Figure** 85**;* ***Figure** 86**;* ***Figure** 87**;* ***Figure*** 88; ***Figure*** 89; ***Figure** 90**;* ***Figure** 91**;* ***Figure** 92**;* ***Figure** 93**;* ***Figure 94*****).**
- means of automatic determination of events of interest including any of spontaneous events of interest and/or evoked response events any of or any combination of means of automatic online time-synchronised determination of sleep/wake hypnogram (i.e. means can include analysis of monitored **EEG,** EOG, EMG sleep parameters); spatiotemporal dynamics physiological and neurological monitored signal parameter characterisation (i.e. ***Figure*** 69, ***Figure 84******;** Fiqure 85;* ***Figure*** 86; ***Figure** 87**;* ***Figure*** 88; ***Figure*** 89; ***Figure** 90; **Figure** 91**;* ***Figure** 92**;* ***Figure** 93**;* ***Figure 94*****);**
- means of **comparing different** states **such** as **generating Grand Mean (GM) averages representative of selected periods** (for example, but not limited to, running averages using overlayed segments with a step time parameter representing the time between each successive average computation and a block parameter representing the number of stimulus-generated evoked response periods averaged for each successive step averaging computation. i.e. a 1 second step and 256 block parameter could be deployed to average the past 256 stimulus evoked responses each consecutive 1 second of past/elapsed subject/patient monitoring time; alternatively a 3 minute block parameter could represent the number of evoked response applicable to the past 3 minutes of elapsed subject/patient monitoring time ) with **SLEEP** stages and/or **WAKE** periods using averaged sweep blocks (i.e. autoregressive fast averages or past 64, 128 or 256 or 512 sweeps) in consecutive steps (such as 15 sweeps/stimulus events);
- means of comparing and/or contrasting and/or interpreting **SLEEP, WAKE and other consciousness** sates **or** events **or health periods of interest in terms for the** averaged outcomes for any defined block or step period of subject/patient monitoring outcomes;
- means to deploy standardised (i.e. such as using any one or more averaged imaged from any imaging modality whereby multi-model imaging incorporating the actual structural and/or vascular and/or functional and/or anatomical and/or cognitive function of a **subject/patient** or a substitute **subject/patient's** brain data can be superimposed as part of the computational analysis or visualisation of the monitored subject/patient at any time) averaged MRI 3D brain image and segment out brain-stem and cerebellum regions, in order to converge actual fEEG outcomes to enable accentuation of deep-cortical brain views of interest;
- means of generating a computational current density reconstruction (CRD) sLORETA algorithm (i.e. means includes any of or any combination of automatic, online, or other via **supplementary** Cloud-computing services, network application **services/NAS,** LAN, WAN and other wireless linked information and/or processing and/or supplementary analysis systems, as a means of generating values with associated source coherence **(COH)** corresponding for each respective elapsed monitoring period (i.e. but not limited to 3-min period averages) of the SLEEP and WAKE periods of interest or any other states or events or health conditions of interest;
- means of analysing the activation of any sleep/drowsiness/unconsciousness/inattentiveness stage or state (i.e. per ***Figure 84; Figure** 85**;* ***Figure*** 86; ***Figure** 87**;* ***Figure*** 88; ***Figure*** 89; ***Figure** 90; **Figure** 91**;* ***Figure** 92**;* ***Figure** 93**;* ***Figure 94******)*** versus WAKE/alertness/viailance/consciousness/attentive/focus and other periods of interest or any other states or events or health conditions of interest as they relate to brain activation connectivity, coherence, networking and integration characteristics associated with both cortical **(CDR;** distributed) and also dipole (localised) coherence activation; -means of comparing, contrasting and interpreting SLEEP versus WAKE CDR and COH outcomes (i.e. per *Fiqure* 69; *Fiqure 84; Fiqure 85; Fiqure* 86; *Fiqure 87; Fiqure* 88; *Fiqure 89;* ***Figure** 90; **Figure** 91**;* ***Figure** 92**;* ***Figure** 93**;* ***Figure 94******)*;**
- means of monitoring, analysing, indicating, reporting, and/or storing brain intricacies such as those associated with any of or any combination of sleep, drowsiness, sedation, unconsciousness, inattentiveness, sedation, stage or state, state or stage (i.e. ***Figure** 84; **Figure** 85**;* ***Figure*** 86; ***Figure** 87**;* ***Figure*** 88; ***Figure*** 89; ***Figure** 90; **Figure** 91**;* ***Figure** 92**;* ***Figure** 93**;* ***Figure** 94**)* and other periods of interest or any other states or events or health conditions of interest as they relate to brain activation connectivity, associated with consciousness or altered states of consciousness;

The present example further provides *"health conditions* or symptoms *of interest" or* "events of interest/EOI" (per "EOI DEFINITION" detailed in abbreviation table at rear of this document).

The present example further enables a means of incorporating analysis as part of the decision and/or control process optimising control of magnetic stimulation applied to a subject's skull, whereby in one process measures of any of or any combination of source localisation, coherence analysis, Dipole analysis, non-linear dynamic based analysis, spectral analysis (where these said analyses are covered elsewhere in this document), whereby said treatment is applicable to and/or provides *"health conditions* or symptoms of *interest"* or "events of interest/EOI" (per "EOI DEFINITION" detailed in abbreviation table at rear of this document).

### EXAMPLE OF AUTOMATIC ONLINE NLDB ANALYSIS AND SPONTANEOUS OR EVOKED EVENTS OF INTEREST DETERMINATION WITH SYNCHRONISED SLEEP/WAKE HYPNOGRAM AND SINGLE OR CLUSTER ANALYSIS OR ENSEMBLES OF EVENTS OF INTEREST INDICATIONS AND/OR BIOFEEDBACK TREATEMENT CAPABILITIES

This application comprises of a number of examples including a system with computer implemented method(s) comprising of a diagnostic and/or prognostic platform with a means of enabling online monitoring via a mobile wireless connected or other computer network connected system interfacing to a subject/patient wearable or nearby proximity physiological or environmental monitoring device, the computer implemented method comprising any of or any combination of:
- a computer system incorporating one or more processes with memory storage capability involving one or more computer programs which can be executed by one or more processes, the one of more computer programs including instructions or applications enabling any of or any combination of:
   - the present example further comprises of a subject/patient wearable or applied device incorporating one or more physiological sensor and/or electrodes for monitoring and/or analysing a plurality of sleep parameters;
   - the said monitoring and/or analysing a plurality of sleep parameters includes (but is not limited to any of or any combinations of monitoring from a single forehead sensor one or more channels of **EEG,** EOG and/or EMG sleep parameter electrophysiological signals;
   - the said monitoring and/or analysing a plurality of sleep parameters includes (but is not limited to any of or any combinations of monitoring from a single forehead sensor (such as but not limited to Figure 21, Figure 25) one or more said sleep parameter electrophysiological signals in combinations with any of:
      - subject/patient posture/position monitoring sensor and determination (i.e. but not limited to 3-axis accelerometer sensor);
      - subject/patient environmental light monitoring sensor and status determination means (i.e. but not limited to light dependent resister);
      - subject/patient breathing sound monitoring sensor and status determination means(i.e. but not limited to microphone sensor);
      - subject/patient oximetry monitoring sensor and status determination means (i.e. plethysmography oximeter and related outputs);
      - subject/patient airflow sensor means monitoring sensor and status determination means;
      - subject/patient motion/accelerometer monitoring sensor and status determination means;
      - subject/patient ballistocardiograph monitoring sensor and status determination means;

      Where said "posture/position" monitoring sensor and determination means comprises (but not limited to) multi axis accelerometer monition and position sensor such as, but not limited to, 3-axis accelerometer with analyses of output values or detection of output information in order to determine patient position;
      Where said "environmental light" monitoring sensor and status determination means comprises (but not limited to) light detection means (i.e. but not limited to) light dependent resistor sending of environment light conditions;
      Where said "breathing sound" monitoring sensor and status determination means comprises (but not limited to) forehead monitoring wearable/applied sensor strip with attached or embedded infrared sensor directed to subject's nose and mouth for purpose of monitoring breathing airflow temperature changes;
      The present forehead sensor further comprising automatic and dynamic sleep parameter data exchange with a second wearable indicator device, enabling any of sleep measure or indices and/or fitness measures or indices and/or health measures or indices of the monitored subject/patient;
      Where "breathing sound" monitoring sensor and status determination means comprises (but not limited to) forehead monitoring wearable/applied sensor strip with attached or embedded microphone directed to subject's nose and mouth for purpose of monitoring breathing sounds;
      Where said "oximetry" monitoring sensor and status determination means comprises (but not limited to) forehead applied reflective plethysmography oximetry (as detailed under main section Title: eLifeCHEST/eLifeSCOPE and subheading "Plethysmography Oximetry");
      Where said "airflow" sensor monitoring sensor and status determination means comprises (but not limited to) drop down sensor connected to subject/patient nose and/or mouth airflow breathing inhalation and expiration breathing regions using sensor (i.e. polyvinylidene difluoride (PVDF), thermistor, thermocoupler sensor material or other temperature sensitive material;
      Where **"motion/accelerometer"** sensor means comprises (but is not limited to) one axis accelerometer sensor system;
      Where forehead wearable/applied sensor strip can incorporate the capability of patient movements and/or motion characterisation accelerometer (i.e. one or axis) ) signals monitoring and means of analysis (i.e. means including any of or any combination of, but not limited to a spectral band segmentation where specific spectral bands can be associated with one or more physiological signal sources such as, but not limited to any of or any combination of breathing and/or movement and/or sleep-disturbances, and/or sleep-restlessness such as body movements and rolling over and/or breathing disorders) and also waveform pattern or signal shape characterisation (i.e. means including any of or any combination of, but not limited to a spectral band segmentation where specific spectral bands can be associated with one or more physiological signal sources such as, but not limited to any of or any combination of breathing and/or movement and/or sleep-disturbances and/or breathing disorders) and/or other analyses derivations of breathing or breathing disorder characterisation;
      Where said "ballistocardiograph" monitoring sensor and status determination means comprises (but not limited **to)** the measurement of the cardiac output by monitoring the body movements caused by the contraction of the heart and ejection of blood into the aorta;
      Where the forehead applied or attached monitoring sensor device and/or wireless linked analysis processing system can enable optimisation and/or compensation of monitored **sleep parameters (EEG,** EOG and/or EMG signals) as a means of compensating forehead monitored **sleep parameters** in accordance to standardised human sleep staging scoring guidelines (such as those published by the American Academy of Sleep Medicine; AASM) and signal characteristics (i.e. spectral, phase and amplitude properties) of ideal or AASM recommended electrode locations. The required compensation factors associated with compensating forehead monitored **sleep parameters (EEG,** EOG and/or EMG signals) in order to emulate AASM recommended (i.e. F4 - M1; C4 - M1; O2 - M2; with backup including F3 - M2; C3 - M2; o1 - M2) **EEG** electrode placements by compensating frequency, phase and/or amplitude of the forehead applied sensor electrode locations (i.e. frontal forehead locations such as (but not limited to) minimalized (i.e. analysis minimalisation and monitoring, signal processing, data-acquisition, events of interest identification and tagging for source localisation, source localisation, coherence determinations, etc. per *Fiqure 45,* ***Figure** 75**,* ***Figure** 76**,* ***Figure** 77**,* ***Figure** 78**,* ***Figure** 79**,* ***Figure** 80**)* configuration such as but not limited to any combinations of **EEG** electrode (i.e. forehead strip sensor or forehead band (i.e. Figure 2, Figure 3, Figure 4, Figure 5, Figure 10, Figure 16, Figure 21, Figure 25) sensor location (see also (Fp1, Fp2, Fz, F6, F7, etc.);
      whereby such **EEG** signal compensation effects can be applicable to compensating differences in measurement pathway between characteristics of signals and analysis of interest, such as the most relevant to accurate determination of a subject's sleep stages and states, (i.e. compensating differences such as any of any combination of but not limited to white brain matter, grey brain matter, outer brain fluid, scalp skin, cranial bone, etc.) versus typical or subject/patient-specific signals monitored, including compensating for signal amplitude and/or phase and/or spectral variances (between standard **EEG** and/or EOG and/or EMG electrode monitored locations such as the optimal electrode locations;
      Where the forehead applied or attached monitoring sensor device and/or wireless linked analysis processing system can enable optimisation and/or compensation of monitored **sleep parameters (EEG,** EOG and/or EMG signals) as a means of compensating forehead monitored **sleep parameters** in based on compensating for most accurate estimated neural source localisation, regardless of how few electrodes are used, in order to most accurately determine the shifts in brain signal **energy** or activation;
      Where determination of "shifts in brain **signal energy** or activation" can include any of or any combination of *asymmetry characterisation of **EEG** signal including determination of left and right hemisphere, posterior and frontal brain activation* (AEP and/or continuous **EEG** analysis transformations) along with the ratio of such brain activation regions;
      Where determination of "shifts in brain **signal energy** or activation" can include any of or any combination of spatiotemporal dynamics of subject/patients brain activation or deactivation can be tracked with *minimal electrode or* as *few* as 3 ***EEG*** electrodes (i.e. consumer or routine daily monitoring circumstances ) whereby broader trends such as shifts in neural energy can be tracked (including but not limited to) the *brain lateralisation, anteriorisation, or hemispherical, hemispherical or front-real brain symmetry, etc.*
      Where the forehead applied or attached monitoring sensor device and/or wireless linked analysis processing system can include any of or any combination of:
         - analysis *signal dynamics* EEG signal characterisation including determination of *non-linear dynamic analysis* (l.e. entropy, complexity and the like);
         - *enhanced power of the left brain **region*** associated with ASD;
            - brain activation and/or *coherence signal changes* in terms of left and right hemisphere shifts in brain activation/energy and/or connectivity (i.e. Dipoles) including lateralisation and/or anteriorisation;
               The present example provides any of or any combination of diagnostic, prognostic monitoring or associated analysis or health management (i.e. event(s) of interest or health condition(s) of interest) comprising any of or any combination of:
            - Whereby prognostic or diagnostic "events of interest" or "health conditions of interest" can be signal characterisation and/or determination applicable to any of or any combination of nerve disorders, muscle disorders, movement disorders, psychological disorders, sleep or wake disorders, sleep disordered breathing, cardiac disorders, respiratory disorders, ventilator disorders, vascular disorders, neurobehavioral disorders, neurological disorders including (but not limited to TBI, ASD, ASP, Dyslexia, Huntington's Alzheimer's, dementia, epilepsy disorders, ADHD and/or Parkinson's disorders), along with associate aura's or onset health conditions (i.e. any combination of physiological changes identified in lead up to an event of interest or health condition of interest).

### EXAMPLE OF AUTOMATIC ONLINE NLDB ANALYSIS AND SPONTANEOUS OR EVOKED EVENTS OF INTEREST DETERMINATION WITH SYNCHRONISED SLEEP/WAKE HYPNOGRAM AND SINGLE OR CLUSTER ANALYSIS OR ENSEMBLES OF EVENTS OF INTEREST INDICATIONS AND/OR BIOFEEDBACK TREATEMENT CAPABILITIES

The present example provides means of enabling any combination of prognostic or diagnostic monitoring or tracking of NLDBTV, STV or conventional neurology imaging measures (i.e. voxel image sections) or EEG activation measures (i.e. EEG current density or voltage potentials) including GENERATING SEGMENTATION, BEM, CDR AND COHERENCE **OUTCOMES ³²(****Figure 84****;** **Figure 86****)³²** (i.e. ***Figure*** 69; example per: ***Figure 84; Figure** 85**;* ***Figure*** 86; ***Figure 87******:*** ***Figure*** 88; *Fiqure **89:** Fiqure 90; Fiqure 91; Fiqure **92;*** ***Figure** 93**; Fiqure* 94).

The present example provides means of enabling any combination of prognostic or diagnostic monitoring or tracking of NLDBTV, STV or conventional neurology imaging measures (i.e. voxel image sections) or EEG activation measures (i.e. EEG current density or voltage potentials) including SOURCE RECONSTRUCTION per example outlined here: HEAD MODEL **DETERMINATION** ⁹² (load New Colin 7Ag13 BEM (3/3/14/12/10) BEM 10/13/15mm [1] 9122x2195)
1. Select the "Source Reconstruction" ICON from the bottom of the parameter menu.
2. To setup head geometry, using mouse select "Head Model" and via "Head Model" drop down menu select" New Colin 7Aug13 BEM 3/3/14/12/10mm Cortical Lamina (120) 3
3. Select top image screen "FD, Maps, 3D View" (top display data menu tab)
4. Select "3D View" icon via from lower left "Object panel".
5. Shut off old cortical surface if selected.

The present example provides means of enabling any combination of NLDBTV, STV or conventional neurology imaging measures (i.e. voxel image sections) or EEG activation measures (i.e. EEG current density or voltage potentials) including MAPS / PCA DETERMINATION per example outlined here:
1. "Maps" icon at bottom of parameter panel was activated and then within "Maps / PCA" parameter panel "ICA" tick box was selected and the corresponding drop down-menu set to "4" followed by setting "Displayed Components" to "4".
2. The "Source Reconstruction" icon at bottom of parameter panel was then selected, "Dipole Fit" parameter bar was selected and then "Dipole Type" drop-down menu "Fixed MUSIC" (Multiple Signal Classification (MUSIC)) option was reselected and the "Number of Dipoles" drop-down menu was selected to "4" corresponding to the 4 distinctive peaks within the EVENT 4 Manuel Study 3, 3 MIN GM AVE butterfly plot functional data display.
3. "Contours" and "Map" were selected via respective tick boxes.
4. "Apply PCA/ICA-Filter" parameter.

The present example provides means of enabling any combination of prognostic or diagnostic monitoring or tracking of NLDBTV, STV or conventional neurology imaging measures (i.e. voxel image sections) or **EEG** activation measures (i.e. **EEG** current density or voltage potentials) including CURRENT DENSITY (sLORETA) DETERMINATION as outlined here: "Current Density" parameter panel is now selected.
1. Using mouse select "CDR Type" and via drop-down menu load "sLORETA" menu option. "CDR Dipole Type" can be selected to "Off".

The present example provides means of enabling any combination of prognostic or diagnostic monitoring or tracking of NLDBTV, STV or conventional neurology imaging measures (i.e. voxel image sections) or **EEG** activation measures (i.e. **EEG** current density or voltage potentials) including SOURCE LOCATIONS DETERMINATION as outlined here:
1. Select "Source Locations" parameter panel and then by activating "Use" dropdown menu select "New Colin 7Aug13 BEM 3/3/14/12/10m Cortical Lamina (120) 3mm" - this step eliminates cerebellum and brain step (per earlier segmentation) and also reverts from laminar source construction (blade affect) to cortex rendered format.
   This is a higher resolution cortical surface. It has been found that 3mm to 4mm is a critical point in getting from a smooth surface to a far from smooth surface as you can see.
2. Select "Source Type" and via drop down menu options select "Rotating".

### SOURCE COHERENCE (Fixed, Currents) DETERMINATION

1. Select "Source Coherence" parameter panel and then by activating "Coherence" dropdown menu select "Fixed" menu option. Coherent fixed dipoles comprise of spatio-temporal source models that integrate activity over a selected time range. This leads to improved SNR and consequently enables enhanced stability for reconstruction results, when compared to moving dipoles.
2. Fixed sources are there because they are basically orthogonal to the cortical surfaces and this creates a situation where if you are at different locations on the cortical surface you will get inverting source activity which is very important for coherence analysis.
3. Select "Results Type" dropdown menu select "Currents" menu option.
4. Select "Zero-lag removed" dropdown menu select "Off" menu option.
5. Enter "50%" value in "Clip Below" Dialogue box. "Clip below" function configures the threshold below which sources with strengths below the clipping threshold are excluded from coherence analysis. The threshold value is based on threshold levels based on a percentage of the absolute maximum source strength waveform applicable to the source coherence analysis.
6. The system may return a message here and notify with warning that computation will take a long time. System may warn of being "out of memory". This means that for this particular analysis I am going to have to change the model.
7. If this warning message is displayed an option is to return to source locations and select a slightly less dense model, such as selecting to 4/4. By doing this and checking after results are complete, you can see (from lower right hand green time bar) that it is taking a fair amount of time to do this Coherence calculation.
8. Select "3D View" per bottom of icon at lower LHS object panel.
9. Highlight "CDR" object selection (under "Source Results" main object).
10. Adjust CDR "Transparency" to "90%" (enables coherence arcs to be visualised as solid lines or various degrees of transparency until lines disappear).
11. Select "Time Range" object to "Movie" (shows a peak right in here which is good; points to right hand image screen per 3rd screen shot below).
12. Highlight "Source Coherence (fixed) for CDR sLoreta" menu LH object panel.
13. Configure "Clip Below" to "50%" (if adjusted from say 90% to 50% this will increase arc activity).
14. If required adjust "min lag" from "10.0ms" to "8.0ms" (if adjusted from say 10.0ms to 8ms this will increase arc activity).

The present example provides means of enabling any combination of prognostic or diagnostic monitoring or tracking of NLDBTV, STV or conventional neurology imaging measures (i.e. voxel image sections) or **EEG** activation measures (i.e. **EEG** current density or voltage potentials) including SOURCE RESULTS STATISTICS DETERMINATION & ADJUST SKIN OBJECT GRAPHICS DETERMINATION as outlined here:
1. Select "3D View" icon located at the bottom of the left panel.
2. Select the skin on which is graphically pleasing or in this example, the "tick box" for the higher resolution skin object file: "New Colin 7Aug13BEM 3/3/14/12/10 Skin (10) 3mm [1] (50%)".
3. Go to left "Object" parameter panel and highlight "Skin" parameter so that lower entry table changes.
4. Select the skin. Go to left "Object" parameter panel and highlight "Skin" parameter so that lower entry table changes. If the skin itself is already at "50%" and we make it "0%" transparent we cannot see through it. If we make it 100% transparent we see all the way through it. Optimal setting is likely to be "50%" transparent.
5. Select "3D view" (per top of data window tab selection ("Maps, 3D view") to display data, so that when image is rotated it actually spins around and shows the other part of the brain. You will now see the skin and image can be rotated.
6. So that you can slice into the skin highlight "skin" object, in left panel under main "Source results" objects.
7. Now adjust "cut plane" in lower objects table by selecting "triple plane".
8. If the cursors are not set quite right just go back to "ALL" (top display screen tab).
9. If the cursor is not right go to the lower right screen and adjust the crosshairs down and open up the coverage a little bit.
10. Select top data display tab "FD, Maps 3D" again and just flip so that image is more dedicated to the brain.

The present example provides means of enabling any combination of prognostic or diagnostic monitoring or tracking of NLDBTV, STV or conventional neurology imaging measures (i.e. voxel image sections) or **EEG** activation measures (i.e. **EEG** current density or voltage potentials) including ADJUST ELECTRODE OBJECT GRAPHICS DETERMINATION as outlined here:
1. The electrode will likely be too bright so go to the electrodes. In lower object panel change the "Electrode Properties" function labelled as "Transparency" from "0%" to "90%" so that the electrodes intensity is decreased.
2. Move mouse over to the "Source results" parameter panel and highlight the "electrodes" parameter file and change the "transparency" from "0%" to "90%" so that the electrodes intensity is decreased.
3. The electrode will likely be too bright so select "Source Results" main object directory highlight "Electrodes".
**4.** Now save parameter configuration to enable these settings to be recalled: i.e. save as a logical and memorable name such as: EVENT **4 ALL 15_59_14** to 17_13_50 MLR Study 2 Manuel 1Aug13 ALL per 265blk x 15stp x 133ch GM Avg 11Aug13 X Parameters.SEG BEM CDR COH.cfg

The present example provides means of enabling any combination of prognostic or diagnostic monitoring or tracking of NLDBTV, STV or conventional neurology imaging measures (i.e. voxel image sections) or **EEG** activation measures (i.e. **EEG** current density or voltage potentials) including GENERATION OF CDR AND DIPOLE BASED COHERENCE ACTIVATION (*Figure* 84) as outlined here:
(Dipole COH Review 24 Oct 13)
Current coherence is based on a distributed source localisation approach while a Dipole source localisation is based upon a more concentrated Dipole approach.

### Head Model

1. Select from dropdown menu: (i.e. use an image head model to enable more realistic superposition of functional subject-specific brain data, via the deployment of a series of pre-diagnostic image scans (can be averaged to produce a more typical representation) or a set of images or image from reference data base of other disease or normal patients).

### Dipole Parameters

1. Select Type: MUSIC
2. Experiment with 2, 3 or 4

### Current Density

1. Select sLORETA

### Source Locations

1. Use an image head model to enable more realistic superposition of functional subject-specific brain data, via the deployment of a series of pre-diagnostic image scans (can be averaged to produce a more typical representation) or a set of images or image from reference data base of other disease or normal patients).
   Cortical Lamina dimensions can (for example only) be configured to (120) 3mm dimensions.
2. Select Type: Rotating Source Locations were set to rotating with clear CDR results (left figure) versus fixed Source Locations where CDR results were concealed (right figure), as shown here.

The present example provides means of enabling any of or any combination of prognostic or diagnostic monitoring or tracking of **NLDBTV, STV or conventional neurology imaging** measures (i.e. voxel image sections) or **EEG** activation measures (i.e. **EEG** current density or voltage potentials) including **Source Coherence,** as **outlined here:**
Select Type: fixed
Select Results Type (Dipoles or currents) : Dipoles (will achieve tute switching of arcs between dipoles)
Note: MAPS (ICA) should not matter; noise estimation automatics OK (pre versus post stimulus variance SNR measure).

The present example provides means of enabling any of or any combination of prognostic or diagnostic monitoring or tracking of **NLDBTV, STV or conventional neurology imaging** measures (i.e. voxel image sections) or **EEG** activation measures (i.e. **EEG** current density or voltage potentials) including **CONFIGURATION DETAILS STUDY EVENT 2** *(i.e. per* ***Figure** 84; **Figure** 85**;* ***Figure*** 86; ***Figure** 87**;* ***Figure*** 88; ***Figure*** 89; ***Figure** 90; **Figure** 91**;* ***Figure** 92**;* ***Figure** 93**;* ***Figure** 94**);*

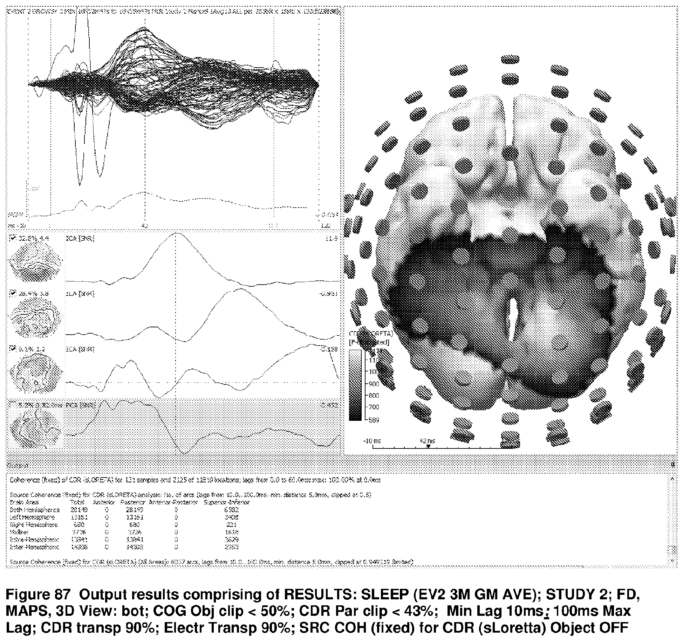

The present example provides means of enabling any of or any combination of prognostic or diagnostic monitoring or tracking of **NLDBTV, STV or conventional neurology imaging** measures (i.e. voxel image sections) or **EEG** activation measures (i.e. **EEG** current density or voltage potentials) **including CONFIGURATION DETAILS STUDY 2 EVT** 3 **(*****Figure** 87**;* ***Figure** 88**;* ***Figure 89******;* )** ***Figure 87*****);**

The present example provides means of enabling any of or any combination of prognostic or diagnostic monitoring or tracking of **NLDBTV, STV or conventional neurology imaging measures** (i.e. voxel image sections) or **EEG** activation measures (i.e. **EEG** current density or voltage potentials) including **CONFIGURATION DETAILS** : **DIPOLE ARC SWITCHING ANALYSIS STUDY 2 EVT** 3 **(*****Figure** 81;* ; **Figure 82****;** **Figure 83****;** ***Figure*** 84 to ***Figure 94******;** )*

**Current coherence** is **based on** a **distributed source localisation approach while** a **Dipole source localisation** is **based upon** a **more concentrated Dipole approach.**

### Head Model

Select from dropdown menu and image data base which can be utilised in order to form the base 3-dmensional model forming the basis image framework for functional **EEG** source localisation and associated analysis outcomes such as coherence arcs, Dipoles etc. (image reference data base can include a series of averaged MRI images, averaged PET images etc. to form a typical image basis for localising functional data analysis outcomes)

### Dipole Parameters

Select Type: MUSIC

### Current Density

### Select sLORETA

### Source Locations

Use: Use reference image data or subject-specific imaged data to enable more realistic superposition of subject-specific functional data and associated source localisation and coherence results to be modelled within. Cortical Lamina parameters can be configured to (120) 3mm Select Type: Rotating - Recommended fixed as shown in right figure here where Source Locations were set to rotating with clear CDR results **(left** **Figure** 90) versus fixed Source Locations where CDR results were concealed (right Figure **90**)**,** as shown here.

### Source Coherence

1. Select Type: fixed
2. Select Results Type (Dipoles or currents): Dipoles (will achieve tute switching of arcs between dipoles)
Note: MAPS (ICA) should not matter; noise estimation automatics OK (pre versus post stimulus variance SNR measure

VARIANT WITH 3 ARCS DISPLAYED : FD, MAPS, 3D View: bottom, front; coherence Object 50% clip; CDR clip below 0%; Min Lag; 6.ms; 120ms Max Lag; CDR transparency 90%; Electrode Transparency 90%; Source Coherence (fixed) for CDR (sLORETA; 100% transparent) Object ON; Dipoles (3 fixed MUSIC) Object On; Dipoles Strength Icon Activated; Automatic Noise Estimation
Determination of VARIANT WITH 3 ARCS DISPLAYED : FD, MAPS, 3D View: bottom, front; coherence Object 50% clip; CDR clip below 0%; Min Lag; 6.ms; 120ms Max Lag; CDR transparency 90%; Electrode Transparency 90%; Source Coherence (fixed) for CDR (sLORETA; 100% transparent) Object ON; Dipoles (3 fixed MUSIC) Object On; Dipoles Strength Icon Activated; Automatic Noise Estimation.

The present example provides means of enabling any of or any combination of prognostic or diagnostic monitoring or tracking of NLDBTV, STV or conventional neurology imaging measures (i.e. voxel image sections) or **EEG** activation measures (i.e. **EEG** current density or voltage potentials) including CONFIGURATION FOR ABOVE 3 ARC SERIES CDR COH (per ***Figure*** 84 to ***Figure 94;);***

The present example Non-Linear Dynamical Based (NLDA) Analysis with option of spontaneous automatic online analysis including, but not limited to, analyses applicable to neurophysiological, cognitive, psychological//mental, and/or other physiological disorders, impairment or events or health conditions of interest, further comprising with option of automatic determination and option of online distributed processing capable of spontaneous source localisation applicable to diagnostic or prognostic determination, and further options of biofeedback and/or treatment of consumer/patient, the example further comprising any of or any combination of:
- means of applying analyses transformation(s) one or more channels of **EEG** monitored data;
- whereby said analyses transformation includes (but is not limited to) to a change of signal dynamics properties including non-linear dynamic signal analysis outcomes and/or associated signal properties (i.e. but not limited to entropy, complexity analysis, bispectrum, FFT outputs etc.) during events such as (but not limited to), changes in depth of anaesthesia, consciousness states, altered states of consciousness, seizures, k-complexes, high-frequency oscillations, ripples, high-frequency ripples, spindles, **EEG** vertex waves, **EEG** seizure signals, **EEG** seizure onset signals, **EEG** spectral bursts or changes, **EEG** sleep staging signal property changes with sleep-related events or disturbances or events, arousals, body movements, sleep breathing disorders, sleep breathing disturbances, sleep disturbances or other events, sleep stages or states, different attention levels, sedation levels, awareness levels, drowsiness levels, vigilance levels, autism spectral disorders and different levels of severity, Parkinson's Huntington's, dementia, Alzheimer's, depression, EOI, and other events or health conditions of interest and/or psychological, mental or physiological disorders, impairment or disease (i.e. see also for example only, but not limited to *Fiqure 75; Fiqure* 77 to ***Figure 80***)
   - means of fast averaging online averaging online monitored **EEG** data from a subject, whereby such means includes but is not limited to automatic regression modelling and averaged data with an external input function in order to minimise the delay associated with said averaging online **EEG** monitored and/or further processed data;
   - means of applying NLDB analysis to any of or any combination of monitored **EEG** signals of a subject, whereby such means can be entropy or complexity analysis (for example but not limited to);
   - whereby said monitored **EEG** data can comprise of evoked responses generated from an external stimulus (such as but not limited to auditory stimulus to one or both subject's ears);
   - whereby said monitored **EEG** data can comprises of defining any event within monitored **EEG** data or other physiological data, whereby such events could be detected by way of characterisation of specific signal amplitude thresholds or characteristics and/or by way of definition of spectral properties of **EEG** signal and/or by way of exceeding predefined, ranges, levels or changes in non-linear dynamic properties of analysed **EEG** signals;
   - means of seizure monitoring via combined sensor and video system (i.e. applicable to monitoring EOI aura, onset and incidents via video camera attached or embedded or forming an element or part of said "forehead strip" or "forehead band" or other head applied or attached sensor(s) including any of or any combination of wearable monitoring devices or associated system, such as but not limited to ***Figure** 1* to ***Figure*** 94), whereby means includes time-synchronisation of video and physiological or **EEG** signals (including but not limited to MTM), whereby wireless data interconnectivity can provide means of transmitting video data to companion/compatible/interconnected device or network application services;

### FIRST STEP

- whereby in the first step of a NLDB process a series of evoked response **EEG** waveforms (i.e. synchronised to originating stimulus signal, such as auditory stimulus signal) can be averaged by way of averaging a number of successive evoked **EEG** responses (artefact can be excluded), and whereby said autoregressive average modelling with or without external input function can be deployed;

### SECOND STEP

- whereby in a second process step the determination of signal complexity (or entropy) value could be generated by analysing data sample values of an averaged value (for example but not limited to), whereby (in one example) an average comprising of 256 sample points (such as an evoked response covering 0 to 120ms post-latency region, for example only) the computation of the subtraction of each successive sample point value from the previous sample point value could establish the "differential AEP averaged (i.e. based on computing values for an averaged sequence of AEP responses) the voltage value" - i.e. the creation of a "differential amplitude version of the averaged waveform";

### THIRD STEP

- whereby in a *third step* non-linear dynamic analysis (i.e. such as but not limited to entropy or complexity analysis) transformation can be applied to the "differential amplitude version of the averaged waveform" as a means of generating a measure or index value corresponding to the said "differential amplitude version of the averaged waveform";
   (identified in second step of process).
- whereby the transformation of subject monitored spontaneous response (single spontaneous response or cluster or sequence of a plurality of spontaneous response) or evoked response (single evoked response or cluster or sequence of a plurality of evoked responses) measures includes the generation of: 1) consecutive running AEP averages, per said process step 1, 2) "differential amplitude version of the averaged waveform" consecutive "running averages", per said process step 2; 3) non-linear dynamic index or measures associated per said process step 3.
- *whereby in process step* 1 successive averages can comprise of bock of time period and/or block of past evoked response signals (including possibility of selecting any range of evoked response pre-stimulus or post stimulus signal values) of past monitored **EEG** data of any or any combination of subject monitored **EEG** or other physiological signals;
- *whereby in process step* 1, successive averages can be compute in steps of any time period, comprising of the time between successive past averages or past data computations (i.e. but not limited to 1 second, 2 seconds, 10 seconds etc.);
- whereby in this manner a non-linear dynamic value or continuously updated value/index can be computed for any monitored evoked response or averaged series of evoked responses, or consecutive steps of evoked response blocks of averages.

The present example further incorporates the ability to detect outcomes of neural generators implicated as precursors and/or with the incident of neurological disorders or changes in consciousness and/or altered consciousness states (epilepsy, vigilance, sedation and anaesthesia) and/or transitions of consciousness states in general, along with more specific transition and states by way of applying non-linear dynamic (NLD) analysis transformations, whereby computed NLD changes capable of comparing and/or contrasting deterministic versus randomised signal properties can serve as a prognostic or diagnostic tool for the determination or highlighting of events or health conditions of interest.

For example, signal regularity measures applicable to brain sources (neural generators) such as those associated with slow-wave (Delta) sleep, sleep spindles, onset and incident of seizures, deep-anaesthesia "saw tooth" neurological signals, high-frequency oscillations, high-frequency bursts, ripples, k-complexes, vertex waves, spectral bursts or changes in **EEG** energy, along with other events or health conditions of interest can be detected using these new NLD-based principles but also be examined in terms of spatial dynamic NLD-based factors. These new NLDB measures can have profound implications when it comes to the investigation of consciousness, sleep, anaesthesia, epilepsy and many more of the most puzzling enigmas of modern science.

The present example further enables the said non-linear dynamic measures to be deployed as part of the decision matrix utilised in the determination of treatment or therapeutic system control, whereby said computer control can include but is not limited to administration, drug infusion, medication recommended administration, magnetic stimulation, electrical stimulation, visual or auditory biofeedback (linked to **EEG** analysis outcomes) or feedback, non-invasive positive ventilation or positive airway treatment, along with other treatment of biofeedback deployment;
The present example can substitute a spontaneous event determination as a variation in the first process step (i.e. the present example can detect changes (i.e. such as but not limited to predefined thresholds or ranges (i.e. an **EEG** spindle, high frequency oscillation, or high frequency ripple event would, for example, would have a lower degree of complexity or lower entropy due to regularity or deterministic nature of signal (i.e. repetitious or cyclic nature of signal morphology of these events) versus irregularity of the spindle properties.

Conversely, asymmetrical morphological signal properties of events such as k-complex **EEG** event or body movements could typically result in an increase in signal complexity and consequently higher degree of complexity or entropy value), whereby the said spontaneous event determination can be used in conjunction with other analysis (i.e. spectral, coherence etc.) or solely as a marker of brain activation event for additional non-linear dynamic based (NLDB) processing steps;
Said additional *NLDB **processing** steps* can be deployed in conjunction or in substitution of source localisation computations (i.e. for example but not limited to generating brain activation values and magnitude of values based on determination of NLDB analysis measures for one or more **EEG** monitored signals.

Moreover, these NLDB measures and associated magnitudes or NLDB analysis measures can be computed using forward problem (for example, in the case of the forward problem the magnitude and location of values inside of the head are known, while monitored **EEG** (functional data) scalp data is unknown) and inverse problem (for example, in the case of the inverse problem the magnitude and location of monitored **EEG** (functional data) scalp data values are known, while the neural source or sources in the head are unknown.).

The present example NLDB source localisation capability comprises of calculating two different classes of source models, based on distributed and local sources, where the distributed sources can be determined by utilising current density analysis methods, while local sources are computed by dipole analysis fitting models.

The NLDB analysis transform comprises of computing distributed sources by way of non-linear dynamic magnitude (NLDM), whereby conventional current density computational methods can be deployed except the NLD level/magnitude is substituted for the conventional current density factor, but whereby a source localisation visualisation (i.e. NLDM substituted for conventional **EEG** amplitude levels), topographic maps (i.e. NLDM substituted for conventional **EEG** amplitude levels and signal to noise thresholds are represented by signal NLD signal magnitude/levels), and where PCA or ICA analysis (i.e. NLD signal magnitude/levels (NLDSM) are compared to NLD noise/background-signals magnitude/levels (NLDNM) or NLDSM:NLDNM, compared to conventional signal to noise or S:N ratio).

The present example enables determination of non-linear dynamical coherence whereby conventional **EEG** signal amplitudes are substituted by **EEG** non-linear dynamic magnitude, and whereby statistical and/or visualisation of determination of non-linear dynamic arcs (NLDA) can be processed using similar lag and lead timing and range of levels (i.e. NLDM versus clip or amplitude levels in conventional **EEG** source localisation coherence computations);
The present example can computed NLDB analysis transform comprising of computing distributed sources by way of non-linear dynamic magnitude (NLDM), in conjunction with conventional distributed sources determined by utilising current density analysis methods, along with local sources computed by dipole analysis fitting models;

### NLDBVT PROCESS EXAMPLES

### (SE or ER event of interest detection)

>In a further process (or next step) previously monitored time series data across a defined period (epoch) of time (based on parameter settings of epoch and step adjustment covered elsewhere) undergoes a complexity analysis transformation or entropy analysis transformation or spectral followed by entropy analysis transformation (i.e. Shannon's entropy analysis) . In particular entropy or complexity value (NLDBV) can be computed at consecutive steps in accordance NLDBV parameter settings including "NLDBV step" , "NLDBV epoch", "NLDBV sensitivity" (i.e. see elsewhere peaks and trough signal complexity regularity or variance).

### (Array generated for each consecutive step and epoch period)

>In a further process (or next step) consecutive entropy values computed for each "step" across the respective "epoch" time periods can be stored (i.e. time series of NLDB values stored in an array of computed values with each corresponding time period across which each value was computed).

### (Threshold magnitude/degree/level) value for ER event of interest & NLDB EOI detection)

>In a further process (or next step) the NLDBVs can be tracked and compared to a predefined (or optionally dynamically computed EOI threshold NLDBV value which marks the degree or magnitude of entropy or complexity corresponding to a signal period of interest. It is important to configure NLDBV appropriately or automatically. i.e. the present example can be configured to enable any of or any combination of "health conditions or symptoms of interest" or "events of interest/EOI" (per "EOI DEFINITION" detailed in abbreviation table at rear of this document). i.e. HFOs, ripples, spindles, epileptiform spike and waves discharges, K-complexes, spindles, alpha bursts). The NLDBV EOI parameter configuration function ensures EOI are discernible and not excessively not skipped, or diminished.

### SNR value for ER event of interest & NLDB EOI detection)

>In a **further process (or next step)** the NLDBVs can be tracked and compared to a predefined (or optionally dynamically computed) EOI SNR NLDBV value which marks the degree or magnitude of entropy or complexity of the signal for each consecutive "step" and " period" that should be flagged as the start or end of a NLDBTV EOI.

The EOI SNR NLDBTS parameters can be configured, for example only, in accordance to current epoch NLDBV (i.e. signal) versus any number of past NLDBV epoch values (i.e. noise denominator of signal to noise ration equation (Signal/Noise - for example the average of the past say "10" epochs (or as configured in NLDBV SNR parameters). In this case the NLDBV SNR can **be** set **(for example only)** to a **ratio of "2"** " **(or "6db")** then the SNR value where the current epoch NLDBV is in magnitude twice that of the average of the past "10" NLDBV epochs).

Based on this scenario, an **EEG alpha burst of 1 second** that falls within the **NLDBV** sensitivity **setting** (i.e. assuming sensitivity is set to 10% - or in case of complexity analysis parameter configuration, any peak and trough values not varying in synchronisation or more than 10% in terms of 1/2 cycle time period or the peak to trough (alpha signal) inter-interval variation of less than 10% value from that of the average inter-interval peak to trough value computed for the respective EOI.

Other NLDB EOI parameter configuration settings could be **scale factors** - i.e. NLDB scale factor or in one configuration setting the maximum complexity value (index value) at "0" when the EOI is a perfectly repetitious cyclic pattern such as a sine wave or sawtooth or triangular-wave generated by a signal generator with 100% deterministic or regularity or repetitious nature. Conversely, the lower end of the entropy or complexity index value scale could be set to "1" when the EOI or epoch under computation comprises of totally randomised data such as in the in the case of pink or white noise, for example.

**In this the present** example **provides** a **means of EOI monitoring and determination, relating (for example only but not limited to) epilepsy-sleep monitoring such** as **in the detection of sleep-stage changes or sleep-events** (i.e. **vertex waves, spikes, k-complexes, spindles, HFO's, ripples etc.) prone** to **trigger epileptic seizures. Additionally, single** events **or clusters, ensembles and/or sequences of events can be determined and detected. Such detection analysis can incorporate NLDB analysis along with combinations of spectral, amplitude, morphological and amplitude/power methods, in terms of single, clusters of, ensembles of or sequences of** events. **Due** to **the different signal properties deployed** via **each of these unique methods, the combination of one or more of these methods coupled with an associated "confidence level or probability factor determination has the potential** to **provide more precise and** sensitive **markers of the onset or incidence or disorder** states, **previously not discernible. Additionally, the combination with the wearable monitoring minimisation system enables such approaches** to **be deployed in routine and** even **out of monitoring centre on** a **routine basis.**

### Epoch based NLDBV (E NLBV) versus spontaneous event EOI NLDBV (SE EOI NLDBV) versus evoked response EOI NLDBV (or ER EOI NLDBV).

As the names suggest continuous **EEG** monitored signal NLDB analysis transformations include:
a) E NLDBV refers to computation of entropy or complexity value over an epoch period, while
b) SP EOI NLDBV refers to computation of entropy or complexity value as it relates to a spontaneous event EOI, while c) ER EOI NLDBV refers to computation of entropy or complexity value as it relates to a evoked response EOI.

**Additionally, evoked response (ER) EEG monitored signal NLDB analysis transformations include:**
The present example provides ER SP NLDBV refers to computation of entropy or complexity value as it relates to a spontaneous event EOI monitored during ER test paradigms (such as AEP or MMN etc.).
while b) ER EOI NLDBV refers to computation of entropy or complexity value as it relates to a evoked response EOI monitored during ER test paradigms (such as AEP or MMN etc.),

### Spontaneous event (SE) EOI

The present example provides spontaneous event (SE) event identification by way of identifying the start and end of event (i.e. with start point of SR being point in time that NLDBV SNR exceeds threshold to point in time that NLDBV SNR no longer exceeds threshold or SNR parameter).

### NLDBV epoch classification parameter

If the "NLDBV epoch classification parameter" is set to 50% then any epoch with 50% or greater of that epoch exceeding NLDBV SNR or threshold value will be classified as a NLDBV epoch.

### Brain Mental status or processing contingent determination

- *one process* step can compute coherence (per examples ***Figure*** 84 to ***Figure** 94**)* during a sequence of voluntary behavioural tests of a subject (i.e. in the case of testing TBI the subject may be requested to verbalise a sentence, undertake an equation, identify numbers, identify picture or other interactive cognitive tests capable of a range of sensitive but also very broad measures capable of enabling *behavioural and*/*or brain activation responses* (including source localisation and/or brain responsiveness or activation versus pre-diagnostic reference levels), such as pre-test or resting state or other states and/or in conjunction with evoked response synchronised outcomes (MMN, odd-ball or related to endogenous brain activation relating to cognition test paradigms);
- for example in one test of brain responsiveness relating to applying stimulus or thinking paradigms designed to activate any of or any combination of neuropsychological tests⁹³:
   >the frontal lobe activation associated with complex thought processes;
   >occipital lobe activation associated with visual processing;
   >parietal lobe activation associated with touch or pain sensory processes;
   >temporal lobe activation associated with auditory processing;
   >thalamus activation associated with "consciousness switch and also" the relaying of information from the brain to appropriate regions of the brain;
   >Broca's areas activation associated with speech production;
   >Wernicke's area activation associated with understanding or comprehension related to a subject's hearing;
   >hippocampus region activation associated with memory processes;
   >amygdala associated with regulation of fear;
   >cerebellum activation associated with motor movement and balance;
   ***>****cognitive** assessment tests (CAS)* such as (but not limited to) the CAS Battery of tests incorporating any of or any combination of Planning Scale, Attention Scale, Simultaneous Scale and/or Successive Scale;⁹⁴
   >Kaufman Assessment Battery for Children;
   ***>****Neurophysiologial** tests* any of or any combination of categories of testing including Intelligence, Memory, Language, Executive function, Visuospatial, Dementia specific, and/or Batteries assessing multiple neuropsychological functions;⁹³
- whereby "memory" function tests can include any of or any combination of (but not limited to) California Verbal Learning Test, Cambridge Prospective Memory Test (CAMPROMPT), Memory Assessment Scales (MAS), Rey Auditory Verbal Learning Test, Rivermead Behavioural Memory Test, Test of Memory and Learning (TOMAL), Wechsler Memory Scale (WMS), Test of Memory Malingering (TOMM);
- whereby "executive function tests can include any of or any combination of (but not limited to) Behavioural Assessment of Dysexecutive Syndrome (BADS), CNS Vital Signs (Brief Core Battery), (CPT), Controlled Oral Word Association Test (COWAT), d2 Test of Attention, Delis-Kaplan Executive Function System (D-KEFS), Digit Vigilance Test, Figural Fluency Test, Halstead Category Test, Hayling and Brixton tests, Kaplan Baycrest Neurocognitive Assessment (KBNA), Kaufman Short Neuropsychological Assessment, Paced Auditory Serial Addition Test (PASAT), Rey-Osterrieth Complex Figure, Ruff Figural Fluency Test, Stroop task, Test of Variables of Attention (T.O.V.A.), Tower of London Test, Trail-Making Test (TMT) or Trails A & B, Wisconsin Card Sorting Test (WCST), Symbol Digit Modalities Test, Test of Everyday Attention (TEA); > whereby "visuospatial tests" function tests can include any of or any combination of (but not limited to) Clock Test, Hooper Visual Organisation Task (VOT), Rey-Osterrieth Complex Figure; and/or
   >etc.;

***In one process*** he present example enables visual interaction and/ or response measurement via wearable devices such as glasses, protective head gear, auditory response microphone, video eye-response or infra-red reflective ocular responses, vision generating screens or displays or vision supervision (i.e. glasses, visors etc.);
***In one process*** the present example enables auditory interaction and/ or response measurement via wearable or environment measuring devices such as glasses, protective head gear, auditory response microphones;
***In one process*** the present example incorporates an auditory stimulus means such as earphone or speaker or other wearable device;
***In one process*** the present example incorporates a capability of measuring one or more **EEG** channels (i.e. per ***Figure 45 to Figure 55***);
***In one process*** the present example incorporates a capability of enabling any AEP test paradigms including mismatched negativity, odd ball response sequences or other AEP paradigms;
***In one process*** the present example can associate and indicate a degree or measure of association with a voluntary (i.e. visual test) subject or obligatory (i.e. ABR) test paradigm versus the corresponding brain activation or changes (i.e. source localisation, coherence, CDR, Dipoles etc.) typically (i.e. per ***Figure 60 to Figure 71*** and ***Figure* 84 to *Figure 94***) using for example subject/patient-specific or population data bases and learning analysis approaches (i.e. ***Figure 75 to Figure 80***) such as artificial intelligence or expert systems analysis) such implicated with such tests in order to equate the cognitive function or brain function of a subject;
***In one process*** the present example is capable of brain activation (i.e. per example of source localisation of brain activity demonstrated in ***Figure 84*** and in accordance to configuration of current density and source location parameter configurations demonstrated in lower panels of ***Figure 86***) and/or brain connectivity (i.e. Dipole parameter configuration in upper-right "Dipole Fit" section of ***Figure 86*** ***and image examples per*** upper right panel of ***Fiqure 91**; **or per coherence configuration per*** ***Figure 86***) and/or responsiveness based on any of or any combination of analyses for including signal dynamics (i.e. NLDB per non-linear dynamic analysis examples in ***Figure 94*** or ***Figure 74*** upper row panels numbered 11, 15, 14; being 3 right hand upper panels of waveforms) or lower panels labelled 14 being lower panel second to right), spectral (i.e. per example of ***neural spectral display in*** ***Figure 71*** or per spectral **EEG** brain signal graphic spectral sleep display in ***Figure 69***), detection of biomarkers and EOI (i.e. per example of source localisation of brain activity demonstrated in lower panel of ***Figure 69*** ***where events of detection can be selected per the lower panel configuration display or per display in*** ***Figure 71*** where "health conditions or symptoms of interest" or "events of interest/EOI" (as detailed in abbreviation section at rear od document) can be characterised followed by detection and indication of detection, connectivity analysis (including aforementioned coherence and/or Dipole analysis, along with statistical outcomes as presented in per example of source localisation of brain activity demonstrated in_lower panel of ***Figure 85*** showing arc evident across brain regions (i.e. both hemispheres, left-hemisphere, right-hemisphere, midline, intra-hemisphere, inter-hemisphere etc.).

### Figures Description

***Figure 84*** Fixed "Source Type" versus Rotation "Source Locations"
***Figure 85*** Average "evoked" response or spontaneous events of interest raw data waveform display (functional data)
***Figure 86*** Source Localisation comprising of modelling subject/patient-specific **EEG** signals and associated analysis with a typical MEG brain model (i.e. Colin new 6Aug13 BEM (3/3/14/12/10) Cortical Lamina (120) 3mm ) in order to enable (can also be modelled to any patient specific imaging studies) in order to segment our cerebellum and brain stem. The present example provides "Head Model" selection via "Head Model" drop down menu select" New Colin 7Aug13 BEM 3/3/14/12/10mm Cortical Lamina (120).
***Figure 87*** Output results comprising of RESULTS: SLEEP (EV2 3M GM AVE); STUDY 2; FD, MAPS, 3D View: bot; COG Obj clip < 50%; CDR Par clip < 43%; Min Lag 10ms*;* 100ms Max Lag; CDR transp 90%; Electr Transp 90%; SRC COH (fixed) for CDR (sLORETA) Object OFF
***Figure 88*** Noise estimation (LEFT) and options parameter configurations (RIGHT).

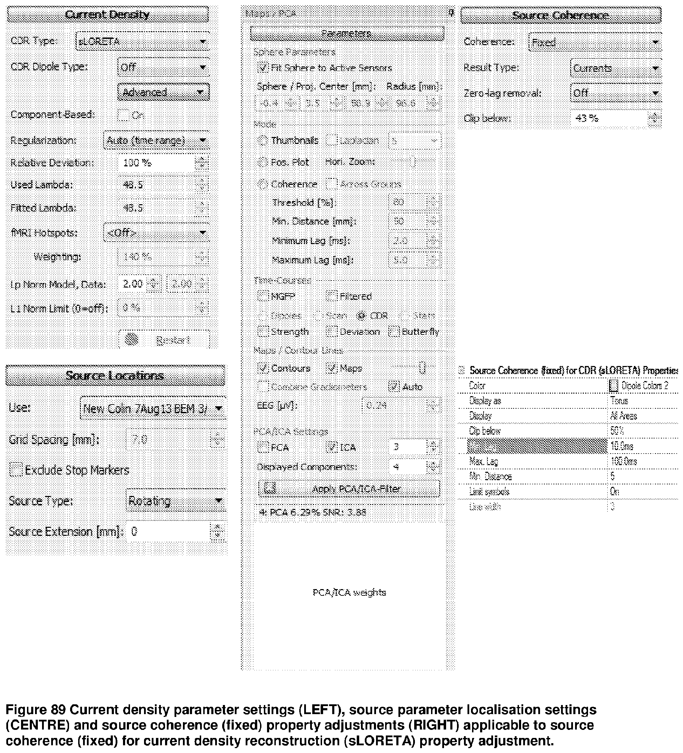

***Figure 89*** Current density parameter settings (LEFT), source parameter localisation settings (CENTRE) and source coherence (fixed) property adjustments (RIGHT) applicable to source coherence (fixed) for current density reconstruction (sLORETA) property adjustment.
***Figure* 90** Fixed "Source Type" versus Rotation "Source Locations" using same parameters comprising, FD, MAPS, 3D View: bottom, front; coherence Object 50% clip; *CDR clip below 76%; Min Lag; 6.4ms; 100ms Max Lag;* CDR transparency 90%; Electrode Transparency 90%; *Source Coherence (fixed) for CDR (sLORETA) Object ON; Dipoles (3 fixed MUSIC) Object On; Dipoles Strength Icon Activated:*
***Figure 91*** Baseline output FD, MAPS, 3D View corresponding to Manuel Study 2 Drowsy 3m GM Average during Drowsy period and marked by thalamus to occipital coherence activity. In particular, output presentation results from configuration values per Table 7, corresponding to base-line Dipole CDR COG with 3 distinct ARCS.
***Figure 92*** noise estimation parameter setting and outcomes (LEFT) and source coherence (3 fixed MUSIC) properties.
***Figure 93*** Dipole (LEFT), Dipole Fit (CENTRE) AND Dipole Density (RIGHT) parameter configurations.
***Figure 94*** shows an example (simulated data) of a split graph whereby the red arrow on the lower index graph has an arrow presented on the x-axis which tracks the 64 channels of averages subject to where the central cursor is located (ARX or conventional depending on parameter panel as discussed in earlier description above).

### PATENT TITLE: DIMENTIA /ALZHEIMER'S /ASD /ASP

### (incorporates neurological disorders such as but not limited to Dementia)⁹⁵

- The present example further incorporates or enables a computer implemented method comprising a diagnostic and/or prognostic platform with a means of enabling online monitoring via a mobile wireless connected or other computer network connected system interfacing to a subject/patient wearable or nearby proximity physiological or environmental monitoring device, the computer implemented method comprising any of or any combination of:
   A computer system incorporating one or more processes with memory storage capability involving one or more computer programs which can be executed by one or more processes, the one of more computer programs including instructions or applications enabling any of or any combination of:**⁹⁶**

- **MONITORING AND ANALYSIS GOAL(S) DETERMINATION (****Figure 80** **[8])**
- Whereby prognostic or diagnostic **"events of interest"** or **"health conditions of interest"** can be signal characterisation and/or determination applicable to ***"health conditions*** or symptoms ***o*f** ***interest**"* or "events of interest/EOI" (per "EOI DEFINITION" detailed in abbreviation table at rear of this document).
- **SYSTEM LEARNING AND AGGREGATED INTELLIGENCE (****Figure 80** **[14])**
   ***means of self-learning* *or aggregated knowledge*/*intelligence*** capability (whereby such means includes but is not limited to any of or any combination of AI, expert system, etc.)
- **DISCRIMINATE FUNCTION ANALYSIS FEATURE SET CLASSIFICATION (****Figure 80** **[8])**
   ***means of determination of and characterisation of most effective analyses*** (i.e. such as relevant to most accurate and reliable prognostic and diagnostic events of interest and health conditions of interest across a range of simple to complex sensor and/or wearable monitoring device(s) configurations)
   by way of (but not limited to incorporating any of or any combination of ***two-group discriminant function analysis (DFA)*** to produce a canonical variable, and whereby the discriminant variable by applying weighting/emphasis to the combination of input (to analysis model) variables can most effectively distinguish two separate groups (events of biomarkers of interest versus background signals not of interest), whereby such DFA analysis can thereby provide classification of features and associated analysis factors or types applicable to prospective diagnosis of events or health conditions of interest, and whereby accuracy of system can evolve or aggregate (**Figure 79**) in strength with increased subject/patient-specific and/or reference population data base studies or associated data and/or informational outcomes. Moreover the significance of the DFA analysis outcomes can be represented via statistical measures (i.e. but not limited to Wilks' lambda with approximation methods such as but not limited to Rao's). ***Whereby the prognostic value can then be estimated, displayed and*/*or reported,*** based on selected or all monitoring sessions of the said subject/patient to-date by way of leaving-one-out processes (such as the Jackknifing technique) ²⁷
- **TRIAGED BRAIN RISK TO SEVERE COGNITIVE IMAPAIRMENT (****Figure 80** **[8])**

The present example incorporates the capability of predicting in a quantitative manner the properties of the mental state such as (but not limited to on the diagnosis or prognosis of a range of mild to chronic brain disorders including (but not limited to) **brain at risk, mild cognitive impairment** (MCI), **disordered health condition** such as mental and/or neurobehavioral disorder and/or neurological disorder, **severe** state of mental and/or neurobehavioral disorder and/or neurological disorder, and cognitive impairment beyond **disordered health condition** (i.e. in case of dementia "cognitive impairment no dementia (CIND)) on a subject/patient at any time by way enabling quantitative effective ***feature extraction*** means by way of classifying factors and/or support vectors machine (SVM) classifiers and/or vectors ***means (cross-validation*** means (i.e. 1000-fold or any number of fold statistical cross-validation performed with analysis such as Monte-Carlo etc. **⁹⁷**), ***classification means*** (i.e. of determining most accurate and effective analyses parameters or analysis variants applicable to "monitoring goals", "subject/patient-specific personalised information", sensor dimensionality redundancy reduction, monitoring device minimisation and/or health conditions or events of interest);

The present example further provides the means of **determining the applicable analysis variants** (i.e. according to any of or any combination of determining most accurate and effective analyses parameters or analysis variants applicable to "monitoring goals", "subject/patient-specific personalised information", sensor dimensionality redundancy reduction, monitoring device minimisation and/or health conditions or events of interest) patient-specific or relevant biomarkers/events of interest/health conditions of interest ), whereby said means includes any or any combination of Monte-Carlo ***cross-validation, Jack-knife techniques, ) and*/*or machine vector, sensor complexity and redundancy dimension reduction means*** (i.e. as outlined per "-DIMENSIONALITY REDUCTION; -Monitoring sensor configuration (Figure 80 [6]); -Monitoring device configuration (Figure 80 [7]); -Monitored neuro parameters configuration (Figure 80 [12]);
- The present example further incorporates or enables monitoring of two or more **EEG** (scalp monitored electrical brain signals) and automatically deploy a means of analysing said signals in order to improve the accuracy of ***estimating the underlying regions of the brain generating such sources;***

- **triaged dementia assessment (****Figure 80** **[8])**
- a means of enabling a ***triaged dementia*** or other neural degenerative or neurological disorder capable of tracking, assessing, and/or reporting on the diagnosis or prognosis of a range of mild to chronic brain disorders including (but not limited to) brain at risk, mild cognitive impairment (MCI), dementia, severe dementia, and cognitive impairment no dementia (CIND), whereby said means of ***determination of triaged degree of dementia*** severity include the deployment of online and automatic analysis option of ***one process*** comprising the deployment of ***clinical*/*consumer assessment scales, another process comprising*** of clinical/consumer test paradigms ***and*/*or another process comprising*** of ***online consumer*/*patient prognostic and*/*or diagnostic monitoring accompanied with corresponding automatic analyses processes;***
   - whereby said automatic process analyses can include any of or any combinations of ***(i.e*.** ***Figure 76 to Figure 80******;*** ***Figure 45******;*** ***Figure* 69****;** ***Figure 71******;*** ***Figure 75******;*** ***Figure 45******),***
- **dementia features (****Figure 80** **[8])**
- Early onset dementia **EEG** characterisation by way of identifying ***slow wave activity*** (i.e. in delta or theta frequency bands or in the proximity of these spectral regions);
- Frontal lobe monitored (i.e. monitored via any ***frontal lobe EEG electrode locations*** including any of or any combination of (but not limited to) Fp1, Fp2, Fpz, AF7, AF8 and/or other brain regions) electrode locations along with detection of ***discernible intermittent rhythmic EEG delta activity*** ;
- **Clustering (****Figure 80** **[8])**
   Whereby one means of improving the accuracy of ***"estimating the underlying regions of the brain generating such sources*"** includes the incorporation of ***clustering algorithm(s)*** enabling diagnostic and/or prognostic signal characterisation specific to an individual/patient/subject. Means of ***"estimating the underlying regions of the brain generating such sources"*** can further incorporate a process whereby an individual's monitored physiological data (including but not limited to ***"aura"*** conditions (outlined elsewhere in this patent application document) determination of ***"events of interest"*** and/or ***"health conditions of interest**"* can include defining or identifying (automatically or via expert or other interpretation assistance and/or validation or verification).
- **AGGREGATE KNOWLEDGE/INTELLIGENCE (****Figure 80** **[14])**
   - ***intuitive or aggregate* knowledge/**intelligence as outlined in (***Figure 77******;*** ***Figure 78******;*** ***Figure 79******;*** ***Figure 80******)*** with option of automatic analyses (such as but not limited ***experts system analysis*** or ***artificial intelligence*);**
- **DIMENSIONALITY DETERMINATION**
- **Monitoring sensor configuration (****Figure 80** **[7])**
   - ***EEG monitoring device(s)*** (i.e. headset format or range of wearable or applied monitoring devices or sensor systems) required to enable **diagnostic or prognostic monitoring** session. i.e. based on clinical study standard or required consumer convenience level of monitoring study such as simplicity of monitoring in terms of the number of monitoring sensors or range of physiological channels to be monitored or complexity based on scientific or clinical nature of required monitoring study; i.e. per ***Figure 45*** comprising any of or any combination of COMPLEX **EEG** DIAGNOSTIC HEADSET DEVICE, comprehensive headset; AUTOMATIC **EEG** ONLINE MONITORING ANALYSIS AND MONITOR-HEADSET MINIMISATION DETERMINATION, SIMPLIFIED **EEG** DIAGNOSTIC HEADSET DEVICE and/or corresponding automatic analysis and associated determinations.
- **Monitoring device configuration (****Figure 80** **[7])**
   - **EEG** features or other neurological ***monitoring system(s) and*/*or wearable device(s) and*/*or* /*devices neurological parameter dimension determination*** and subsequent reduction with option of automatic analyses;
   - ***EEG features or other physiological* *monitoring system(s) and*/*or wearable device(s) and*/*or* /*devices neurological parameter dimension determination*** and subsequent reduction with option of automatic analyses;
- **Monitored neuro parameters configuration (****Figure 80** **[12])**
   - **EEG** features or other physiological ***monitored neurological parameter dimension determination*** and subsequent reduction with option of automatic analyses;
   - ***EEG features or other physiological monitored physiological parameters dimension determination*** and subsequent reduction;
- **Monitoring analysis feature configuration (****Figure 80** **[8])**
   - ***analyses or transformations or features or factors and vectors of derived outcomes based on selected analysis parameters dimension determination*** and subsequent reduction;
- **DIMENSIONALITY REDUCTION**
- **Monitoring sensor configuration (****Figure 80** **[6])**
   - ***Determination of minimal EEG monitoring sensor(s)*** by way of avoiding redundant, unnecessary or least relevant sensors in terms of achieving ***monitoring and analysis goals*** with key monitoring sensors, monitoring devices and associated configurations, classification of analysis types and associated factors for characterisation of key features applicable to events of interest or health conditions of interest; (i.e. headset format or range of wearable or applied monitoring devices or sensor systems) required ***in order to achieve predetermined required performance requirements*** (i.e., simplicity in terms of number of monitoring sensors or range of physiological channels to be monitored or complexity versus simplicity of sensor and/or monitoring device) and subsequent reduction (i.e. but not limited to example per ***Figure 45*** comprising any of or any combination of COMPLEX **EEG** DIAGNOSTIC HEADSET DEVICE, comprehensive headset; AUTOMATIC **EEG** ONLINE MONITORING ANALYSIS AND MONITOR-HEADSET MINIMISATION DETERMINATION, SIMPLIFIED **EEG** DIAGNOSTIC HEADSET DEVICE and/or corresponding automatic analysis and associated determinations).
- **Monitoring device configuration (****Figure 80** **[7])**
   - ***Determination of minimal* EEG** neurological ***monitoring system(s) and*/*or wearable device(s) and*/*or* /*devices neurological parameter dimension determination*** and subsequent reduction with option of automatic analyses;
   - ***Determination of minimal other physiological* *monitoring system(s) and*/*or wearable device(s) and*/*or* /*devices neurological parameter dimension determination*** and subsequent reduction with option of automatic analyses;
- **Monitored neuro parameters configuration (****Figure 80** **[12])**
   - ***Determination of minimal* EEG *monitored neurological parameter dimension determination*** and subsequent reduction with option of automatic analyses;
   - ***Determination of minimal other physiological monitored physiological parameters dimension determination*** and subsequent reduction;
- **Monitoring analysis feature configuration (****Figure 80** **[12])**
   - ***Determination of minimal analyses or transformations or features or factors and vectors of derived outcomes based on selected analysis parameters dimension determination*** and subsequent reduction;
- **Cognition Impairment Assessment and Determination**

The present example provides a means of the **assessment and determination of cognitive impairment severity** comprising of (but not limited to) the utilisation of any of or any combination of:
- the utilisation of the Clinical Dementia Rating (CDR) scale;
- Geriatric Depression Scale (GDS);
- Hachinski Ischemic Scale (HIS);

### Assessment and determination of Cognitive functional outcomes

The present example provides a means of the **assessment and determination of cognitive functional outcomes** comprising of (but not limited to) the utilisation of any of or any combination of:
- instrumental activity of daily living (IDAL);
- basic activity of daily living (BDAL);

### Assessment and evaluation the early dementia stages and/or severity of dementia

The present example provides a means of the **assessment and evaluation** the **early dementia stages and/or severity of dementia** in clinical practice comprising of (but not limited to) the utilisation of any of or any combination of:
- Montreal Cognitive Assessment (MoCA);
- Mini-Mental State Examination (MMSE);
- Addenbrooke's Cognitive Examination Revised (ACE-R);

### Cognitive performance assessment

The present example provides a means of **undertaking neuropsychological assessments** capable of evaluation of a **subject's cognitive performance** comprising of (but not limited to) the utilisation of any of or any combination of:
- Trail Making Test (TMT) for attention and executive function;
- Clock Drawing Test (CDT) for attention and executive function;
- Rey Osterrieth Figure Copy for construction praxis test,
- Phonological and Semantic fluency Token test for language test;⁹⁸

The present example further incorporates a computer implemented method whereby an expert system or machine learning capability (i.e. such as but not limited to example per ***Figure 45******;*** ***Figure 75 to Figure 80***) where by interaction with a consumer/patient can be by way assessment and/or classifier evaluation and/or data mining processes comprising of any of or any combination of
- **Rule learner (or Rule Induction) (Separate-And-Conquer method);**
   - OneR;
   - Ridor;
   - PART;
   - JRip (RIPPER);
   - DecisionTable;
   - ConjunctiveRule;
- **Decision Trees (Divide-And-Conquer method)**
   - J48 (C4.5);
   - ADTree;
   - J48;
      J48graft;
      JRip;
   - DecisionStump;
   - RandomTree;
   - REPTree;
   - CADtree;
   - LADtree;
   - BFtress;
   - SimpleCart;
   - OrdinalClassClassifier;
   - NNge;
   - Filtered Classifier;
   - Data Mining; and/
   - induction algorithms;
- **Attention and focus tracking**

The present example further enables clinical or data diagnostic evaluation of severity of event or health condition of interest ranging from minor to chronic impairment including cognitive dysfunction can be can be by way of assessing a subject or respondent (viewer/audience/user) in terms of response, attention or focus as it applies to **audio and/or visual stimulus means** whereby such means can include (for example but not limited to) displaying any audio visual sequence on a computer display screen and at the same time recording the viewer's visual and/or other sensory response in terms of any of or any combinations of:
- Means of characterising a subject's **eye focus, eye-gaze** parameters of the subject versus the actual association or relevance based on displayed audio or video, whereby mean includes any of or any combination of analyses or events of interest including face, eyes, eye-lids, head, body, pupil and/or gaze measures ;
- Means of characterising a subject's **attention or focus** by way of tracking their **head motion or head position** or associated response parameters of viewer versus the actual association or relevance based on displayed audio or video, whereby mean includes any of or any combination of analyses or events of interest including face, eyes, eye-lids, head, body, pupil and/or gaze measures;
- Means per eLifeCHEST/eLifeSCOPE and/or ElifeALERT sections;
- Means of characterising a subject's **attention or focus** by way of tracking their **eye-blinks** or associated response parameters versus the actual association or relevance based on displayed audio or video, whereby mean includes any of or any combination of analyses or events of interest including face, eyes, eye-lids, head, body, pupil and/or gaze measures as further outlined in eLifeALERT patent description section; x-link to sleep stethoscope ElifeALERT patent section here;
- Means of characterising a subject's **attention or focus** by way of tracking their **body movement or body position** or associated response parameters versus the actual association or relevance based on displayed audio or video, whereby mean includes any of or any combination of analyses or events of interest including face, eyes, eye-lids, head, body, pupil and/or gaze measures as further outlined in eLifeALERT patent description section; x-link to sleep stethoscope ElifeALERT patent section here;
- Means of characterising a subject's **AEP responses** versus the actual association or relevance based on displayed audio or video, whereby mean includes any of or any combination of (i.e. such as any of or any combination of but not limited to MMN, oddball, brain responses to sounds in audio-visual presentation and/or as also outlined in this document under sub-headings including: "
   "; ***Figure 46 to Figure 55******;*** and/or ***Figure 39 to Figure 43***);
- Means of characterising a subject's **interactive user-interface** versus the actual association or relevance based on displayed audio or video, whereby mean includes any of or any combination of gestures, touch screen interface, button activation, finger or any other body extremity pointing or responses, switch activation and other operator interface means;
- Means of playing an audio visual recording (2-dimenional or 3-dimensional) and/or video conference or other interactive communication means, and examining a subject/individual's responses, whereby said responses can be eye movements and means of examining responses can be by way of video and/or oculography measures (i.e. ***Figure 39 to Figure 43******;*** ***Figure 98 to Figure 99*****)** in accordance to responsiveness or synchronisation with said audio-visual recording or video conferencing, with the option of comparing or contrasting (i.e. ***Figure 77 to Figure 79***) such responses to population data bases for purposes of comparing and contrasting outcomes in terms of normal and abnormal outcomes;
   - The present example further incorporates or enables ***visual processing*** to any of or any combination of events such as the ***subject's eye, pupil, eye-lid, eye-closure, eye-blinks, head, eye-gaze**,* ***eye-attention, and*/*or AEP responses*** (i.e. such as but not limited to online MMN P300 or other EP responses as a measure of attention or alertness) in the context of the subject's ability or incidence of focus, concentration, alertness, cognitive engagement, normal characteristics of said events (i.e. based on control subject data bases or outcomes and/or brain risk, mild cognitive impairment, moderate cognitive impairment, severe cognitive impairment population groups of subject-patient informational or physiological/cognitive data references).
   - Pre-diagnostic data versus characteristics of any cognitive tests (per ***eLifeALERT*** section) via eye or gaze responsiveness or other outcomes ((i.e. ***Figure 39 to Figure 43******;*** ***Figure 98 to Figure 99******);***
- **Visual Assessment of Viewer Responsiveness to displayed information:**
   The present example enables a means of Visual Assessment of Viewer Responsiveness to displayed information, such means comprising any of or any combination of:
   - providing a means of **enabling a video or slide sequence** via a computer system display (i.e. computer tablet, wireless mobile device, PC etc.) **and the incorporation of a camera** (i.e. inbuilt into device or separate) to record the responses of the subject/viewer (i.e. facial responses, eye-blinks to displayed video, body response in terms of hyperactivity or distraction or calm focus (for example only) of viewer).
   - providing a means of assessing the responsiveness or interaction or behavioural characteristics (such as but not limited to ***subject's eye, pupil, eye-lid, eye-closure, eye-blinks, head, eye-gaze, eye-attention, and*/*or AEP responses***) of the viewer in relation to the audio-visual display.
   - providing means of (i.e. such means can include but is not limited to .....) time-synchronising between the audio or video stimulus or slide sequence with the recorded audio and/or video of the viewer.
   - providing further assessment means of determining subject's **performance outcomes** in terms of attention or focus as it relates to the audio or visual stimulus sequence (i.e. **such means** can include but is not limited to creating a visual, graphic (i.e. like a **heat map demonstrating** over periods of time greater intensity where gaze or subject focus is most frequently based), **statistical (such as numerical histogram representing subject's visual or auditory** or **other senses),** numerical or graphical overviews representative of the subject's viewing behaviour, responses, location or direction changes, viewing regions, etc. based on the subject's gaze, eye directions, face directions, face movements, head direction, head movements, body directions, body movements, head directions, head movements, at any point in time and corresponding to an audio visual sequence. These outcomes can then be compared to **database(s) of population groups of similar outcomes and statistical measures used to determine whether the subject is associated with results evident from brain risk population groups, mild cognitive impairment population groups, severe cognitive impairment population groups or chronic cognitive impairment groups,** for an example only) of evaluating the **association between any of subject responses** or body movements or facial responses or head position or eye position or eye movements or eye-lid movements or eye-opening status as it relates to the audio generated or display at any point in time;
   - the present example provides a means of **enabling the displayed video or audio** to be time-aligned or **time-synchronised** with the **responses of the viewer,** in order to establish the **comparative attention or attention deficit associated with each assessed subject.**
   - providing a means of **tracking eye gaze or focus** as it relates to the displayed video and mapping the visual eye focus or gaze projection) i.e. by way of tracking the engagement or responsiveness of each individual viewing the displayed video (i.e. in **terms of correlation or association between the screen activity** or visual movement regions versus the subjects experience and/or responses and/or interaction and/or other impact, including but not limited to **pupil changes, pupil gaze direction, head projection, body movements or positioning, body or eye,** or eye-lid or face or eye ball or EOG or other body extremities **movement tremor, jerkiness, rhythm stability, calmness, anxiousness,** nervousness and or other characteristics enabling determination and accommodation of subjects responses compared to normal, **low brain risk, mildly impaired, severely impaired or chronically impaired brain disorders/impact/disease and/or other events of interest of health conditions of interest.**
- **DIMENSIONALITY AND ANALYSIS VARIANT DETERMINATION:**
   **Comprising any of or any combination of sensor, monitoring, analysis transformations, BROAD-SCOPE, SUPERSET AND SUBSET (MINIMISED DIMENSIONALITY, REDUNDANCY ELIMINATION AND FINAL SUBJECT/PATIENT SPECIFIC ANALYSIS VARIANT COMPUTATION) ANALYSIS VARIANTS DETERMINATION, Hybrid Automatic Prognostic/Diagnostic Outcome-based (HAPDO) analysis determination (****Figure 80** **[9;10;11;12])-**
   - **The present** example **provides a computer implemented method of enabling any of monitoring, diagnosis and/or prognosis of** a **subject, the computer implemented method comprising at least one processor, at least one computer programs including computer instructions for:**
      - Hybrid Automatic Prognostic/Diagnostic **Outcome-based (HAPDO)** analysis determination;
      - **HAPDO** analysis determination comprising of at least one of any of or any combination of physiological and/or environmental **sensor(s);** physiological and/or environmental **sensor monitoring,** physiological and/or environmental **parameter monitoring,** physiological and/or environmental **analysis transformation** of any one of or any combination of **monitored parameters** to produce analysis **factors** and/or analysis **features** and/or analysis **vectors, classification** of **disorder/disease-specific analysis parameter superset (DAPS).**
      - The said of and/or parameters of analysis factors and/or features and/or vectors in terms of at least **2 or more physiological** and/or **2 or more mental states** and/or psychological states and/or behavioural states of subject (whereby one state can be defined as normal and the second state as diseased and/or disordered and/or impaired and/or events of interest and/or health conditions of interest).
      - **The present HAPDO** analysis further comprising of a means of **determining selected DAPS, comprising of the most effective or appropriate maximal set of analysis parameters.** This said **DAPS** comprises of analysis types and associated, variables, configurations and/or adjustment parameters (i.e. analysis variants) in accordance to factors including (but not limited to) any of or any combination of: 1) the **"MONITORING AND ANALYSIS GOAL(S) DETERMINATION", (as further detailed under this heading elsewhere in this patent application document).**
      - **One means of determining such** "selected analysis transformation formats, types and associated configuration or parameters" can be by way of a matrix or look-up table format or relational **data-base configured** with one column listing the range of disorders or health conditions of potential prognostic and diagnostic interest, with the most effective or relevant (based on **patient-specific data base** of **normal** clinical-data and/or patient information, and/or **patient-specific data base** of **disease/abnormal** clinical-data and/or patient information, and/or **population data bases of normal** and **disease/abnormal reference clinical-data** and/or patient information.
- **CLASSIFICATION AND DETERMINATION OF ANALYSIS VARIANTS (****Figure 80** **[8])** 47
   The present example provides a means of **aggregating information** (i.e. **translation from** broad-based **monitoring** information to **knowledge** applicable to subject/patient) applicable to the determination of the most effective and precise monitoring of a subject/patient's **diagnostic and/or diagnostic "analysis variants"** (as described elsewhere in this patent document) (see figure (***Figure 78***) applicable to relating to the subject/patient-specific events of interest or health conditions of interest comprising any of or any combination of analyses processes including:
   Means of characterising the ***period or segment of monitored signal(s) including*** (but not limited to **EEG**) providing the most accurate segment or period of monitored data (signal(s)) applicable to early onset "aura", "precursors" and/or events of interest or health sequelae of interest (i.e. any of or any combination of (but not limited to) condition; disorder; dysfunction; cognitive impairment; and/or behavioural disorder of interest; nervous system disorder, muscle disorder, neurological disorder; movement disorder; mental disorder, and/or psychological disorder);
- **Signal monitoring device type/configuration/number of devices dimensionality; analysis variant redundancy elimination of minimisation based on greatest sensitivity, specificity, minimal false-errors and false positives**
   Whereby means of characterising or classifying the ***period or segment of monitored signal(s)*** comprises any of or any combination of quality or effective feature extraction means such as pre-processing including prior to dimensionality pre-processing;
- **Classification techniques (****Figure 80** **[8])**
   Whereby means of characterising or classifying the ***period or segment of monitored signal(s)*** comprises any of or any combination of **artificial intelligence or expert system analysis** (**see** ***Figure* 77**), cross validation means **(for example but not limited to** **Figure 77****;** ***Figure 78*****;_****Figure 80****)**, or (for examples only) the determination of the largest Lyapunov exponent of the **discrete wavelet packet transform (DWPT),** and/or other quality or effective feature extraction means such as **support vector machine (SVM)** classifiers and/or **Linear discriminant analysis (LDA) techniques (for example but not limited to** **Figure 77****;** ***Figure 78*****;_****Figure 80****);**
- **Transition from information to knowledge data-bases (****Figure 80** **[14])**
   - These data bases of events of **patient-specific database references,** population **normal data-base references, population disease/disorder data-base references** normal interest can be established by way of any of or any combination of **expert system/intelligent analysis** means ( for example but not limited to ***Figure 78***; **Figure 80****), *aggregated* knowledge** means (for example but not limited to ***Figure 78***; **Figure 80****),** discriminant function analysis (for example but not limited to **Figure 80****), linear discriminate analysis (LDA)** (for example but not limited to **Figure 80****),** support vector machine (**SVM**) analysis (for example but not limited to **Figure 80****),** and/or largest Lyapunov exponent of the discrete wavelet packet transform **(DWPT),** and/or dimensionality minimisation/redundancy reduction ((for example but not limited to **Figure 80****).**
- **AEP, EEG, broad range of physiological sensors, monitoring, associated analysis variants (****Figure 80** **[9])**
   - whereby **"sensor monitoring**" can include any of or any combination of **EEG** electrode sensors;
   - whereby physiological **"parameter monitoring"** can include any of or any combination of continuous **EEG** and/or **auditory evoked potential (AEP)** monitoring;
   **whereby "analysis variants"** can include (but are not limited to) any of or any combination of entropy (including approximate entropy, Shannon's, wavelet entropy etc.) complexity, spectral analysis (including power ratios or any of or any two or more combination of spectral bands, bispectral index, FFT and associated outputs wavelet etc.), statistical signal analysis (.e. but not limited to kurtosis, skewness), chaos, morphology, fuzzy decision tree based selection procedures, and/or wavelet transform signal analysis, coherence, source localisation, source segmentation, statistical arc and other computational source localisation brain activity determination, **detection of events or events of interest by way of seizure precursors in EEG recordings that include the deployment of an adaptive Weiner algorithm on local field (EEG)potentials; analysis transformation using wavelet-based methods for similarity analysis across consecutive EEG signal segments using a reference segment of the same EEG**
   **signal, utilisation of non-linear processes for intracranial EEG analysis, the determination of changes in Lyapunov exponents (LEs) of the monitored EEG signals, investigation of spatio-temporal EEG dynamics, the combining of energy and EEG signal wavelet transformation outcomes, use of support vector machines (SVMs) and convolution networks on EEG intracranial signals along with SVM classifier usage with the corresponding autoregressive (AR) model(s) of EEG signals,** in conjunction with any of or any combination of analysis types detailed elsewhere in this patent application (including but not limited to example per **(for example but not limited to** **Figure 77****; *Fiqure 78*;_****Figure 80****)** and/or also as further detailed in this document per healthneuro and/or elifesleep stethoscope and/or analysis sub-headings outlined below; ⁴⁷ **vector analysis example**
- **DATA QUALITY, DENOISING ARTIFACT HANDING ANALYSIS (****Figure 80** **[4])**
   - the present example provides a means (described elsewhere under heading of **artefact, denoising** or other sections dealing with **background or unwanted signal** handling) or segmenting monitored data and/or gauging or assessing quality of monitored data (i.e. patient specific and/or other databases) in a manner whereby signals that confound or adversely impact the diagnostic or prognostic accurate can be marked and/or **eradicated** and/or reduced in terms of **adverse impact** to the **performance;**
   - whereby signal quality applicable to precision analysis can comprise any of or any condition of (but not limited to) determination (i.e. in order to establish thresholds and ranges of acceptable quality of signals applicable to acceptable or required (i.e. per "monitoring goals") precision analysis for the purpose of acceptable monitoring and associated analysis variant determination for ultimate events of interest or health conditions of interest) of estimated noise level with regard to the estimated value of the signal to noise ratio (SNR):
      - Sensor weighting;
      - Confidence Ellipsoids;
      - Regularized Dipole Fits;
      - Regularized Dipole Scans, and/or
      - Current Density Reconstructions;
- **Statistical Analysis Performance Assessment Determination (SAPD) enabling Superset and Minimal Analysis Variant Sets/features/vectors and/or Events and Health Conditions of Interest and/or Diagnostic or Prognostic knowledge Reference Data Bases (****Figure 80** **[13])**
   - **SAPD** enables super sets of **analysis variants** to be determined based on **aggregated intelligence (*****Figure 78******)**.* **Aggregated intelligence** here refers to determination of a large set of sensor, monitoring devices or configuration or wearable devices along with **associated analysis variants** based on a number of factors such as (but not limited to) any of or any combination of :
      ∘ User or expert selection of ***monitoring goals**;*
      ∘ User or expert selection **maximal monitoring device configuration;**
      ∘ User or expert **selection of preferred *monitoring* approach (SPMA).** i.e. can be structured in a series of priorities patient wearable formats, sensor configurations, monitoring sensor targets, physiological monitoring targets for initial, applicable to initial more Complex broad-based Investigatory Diagnostic Monitoring **(CIDM)** sessions versus subset (i.e. dimensionality redundancy reduction) Reduced Complexity Diagnostic Monitoring **(RCDM)** sessions (***per*** ***Figure 45***).
      ∘ **Aggregated patient specific knowledge.** i.e. based on patient-specific diagnostic monitoring outcomes, patient information, patient records, patient doctor or patient specialist input information (including validation of events or health conditions of interest or disorders or diseases of diagnostic relevance or interest (i.e. **means of scoping or establishing monitoring goals**);
      ∘ **Aggregated population-based knowledge.** i.e. based on **population normative** and **disease** clinical data and clinical data analysis variant outcomes (i.e. based on specific disorder or events or heath condition diagnosis or prognosis of interest);
      ∘ Determination of continuous analysis, review, update of most applicable analysis variants for a range of options of most complex (i.e. **CIDM)** through to series of monitoring and analysis combinations (i.e. monitoring parameters, monitoring devices, monitoring device configurations, and/or physiological targets) applicable to ***monitoring* goals**;
      ∘ Incorporation of any of or any combination of **statistical SAPD** or other analysis means comprising (but not limited to) multivariate logistic regression, bivariate correlation, determination of the receiver operating curve and classification computation in accordance to goodness of fit criterion, intelligent or expert system determination **(for example but not limited to** **Figure 77****;** ***Figure 78*****;** **Figure 80****),** minimisation determination **(for example but not limited to** **Figure 77****;** ***Figure 78***; **Figure 80****); DFA, LDA, SVM, DWPT,** cross-validation; entropy, complexity, spectral, statistical signal analysis, chaos, morphology, fuzzy decision tree based selection procedures, and/or wavelet transform signal analysis, coherence, source localisation, source segmentation, statistical arc and other computational source localisation brain activity determination; probability determination (including but not limited to Pk measures), standard error (SE); sensitivity, specificity, receiver operating characteristic (ROC), standard deviation (SD) measures; false positive detection rates; false negative detection rates **(for example but not limited to** **Figure 77****;** ***Figure 78*****;** **Figure 80****);**
      ∘ Determination of **best maximal** (i.e. **CIDM)** range of models through to data dimensionality minimised **EEG** (i.e. reduction of sensor and **analysis redundancy** based on relevance to monitoring goals etc.) and/or other **physiological parameter** monitoring and/or other **minimal analysis variant models** (i.e. **RCDM).**
   33

The present example provides any of or any combination of diagnostic, prognostic monitoring or associated analysis or health management of any of or any combination of:
- Whereby prognostic or diagnostic **"events of interest"** or **"health conditions of interest"** can be signal characterisation and/or determination applicable to ***"health conditions*** or symptoms ***of interest**"* or "events of interest/EOI" (per "EOI DEFINITION" detailed in abbreviation table at rear of this document) ⁹⁵

### Description of Figures

Figure 78 Prognostic and Diagnostic Knowledge Data Bases [6;7;8;9;10;11;12] based on translation [2;5] and aggregation to system intelligence via internet/web-linked [3] expert and subject/patient applicable information sources [1;4].

The combination of the present Health Management System's (HMS) aggregation of patient monitored and other patient information (i.e. records etc.) information **[4]** and expert information **[1]** (such as cross validation of events or health conditions of interest applicable to monitoring goals for specific subject/patient), accessible by way of web/internet/LAN/WAN **[3]**.

The combination of data-base, expert and patient information and/or clinical data enable the determination of the optimal prediction module **[5]** (analysis variants) and diagnostic module **[2]** (analysis variants).

The aggregated information (which can be translated to relevant knowledge (***Figure 77******;*** ***Figure 78***; ***Figure 79******)*** is stored and retrieved in the respective Subject/Patent-specific normative clinical/consumer monitoring Data Base **[6]**, Subject/Patent-specific disease/disorder/Health condition clinical/consumer monitoring Data Base **[7],**

Subject/Patent-specific normative and disease/disorder/Health condition clinical/consumer information Data Base **[8],** Population normative clinical/consumer monitoring Data Base **[9]**, Population disease/disorder/Health condition clinical/consumer monitoring Data Base **[10]**, Population normative and disease/disorder/Health condition clinical/consumer information Data Base **[11]**.

**[12] Data base** incorporating **subject/patient-specific and also generic or and population-based monitoring algorithms** with associated analysis variants (i.e. per **Figure 77 to Figure 80****)** disease/disorder groups) along with optimised monitoring models based on evolving **expert system/artificial intelligent transition** (i.e. from information to knowledge based analysis variants applicable to broad-scope data-mining, subject/patient specific monitoring goals and range of optimal minimal to full analysis variant models applicable to events or health conditions of interest.

Collectively the HMS system's "monitoring goals", followed by monitoring sensor dimensionality redundancy reduction and "analysis variant" "minimisation" using means of monitored signal feature extraction and associated "analysis variant" determination (i.e. such as but not limited to any of or any combination of expert system or artificial intelligent techniques **(****Figure 77**) , capable of evolving or continually improving health monitoring and management accuracy and effectiveness.

Means of "classification" of optimal signal feature set or "analysis variants" (i.e. including any of or any combination of, but not limited to DFA, LDA, SVM, DWPT) of from broad-based (per overall data-mining capabilities of remote/web-based database and information sources applicable to human disease or disorder or other psychological, physiological or mental conditions of risk, mild impairment, or chronic states.

**Figure 79** Health Management System (HMS) Information to Knowledge Transformation Method.

The present example comprising of a health management system incorporating intuitive (i.e. AI, or expert system) and aggregated information automatically and/or with expert assistance information (clinical and specialist intervention or oversight) translatable to knowledge (i.e. actual events or health conditions of interest cross-validated with expert/specialist and also patient specific diagnostic data/biomarker outcomes) to support consumer/patient-specific early onset (i.e. "aura" ) through to mild disorder and/or chronic diagnostic and prognostic health tracking, reporting, alerting, health data-mining and ultimately enhanced patient health outcomes.

***Figure 80*** Personalised subject/patient- health management system (HMS) *[*1] incorporating any of or any combination of monitoring goals.

### Personalised subject/patient-specific health management system (HMS) [1].

The present example provides any of or any combination of diagnostic, prognostic monitoring or associated analysis or health management applicable to health sequelae (per ***"health conditions*** or symptoms ***of interest" or* "**events of interest/EOI" (per "EOI DEFINITION" detailed in abbreviation table at rear of this document), ***the present*** example ***further* comprising *of or any combination of (but not limited to);***

### Incorporating monitoring goals [2].

- the present example further comprising of the capability of determining prognostic or diagnostic "events of interest" or "health conditions of interest" can be signal characterisation and/or determination applicable to ***"health conditions*** or symptoms ***of interest"*** or "events of interest/EOI" (per "EOI DEFINITION" detailed in abbreviation table at rear of this document);
- these goals can be defined by subject/user or with assistance (i.e. "opt-in") for health-workers or other carers;

### Data-acquisition monitoring [3]

- the present example further comprising of data-acquisition monitoring comprising of monitoring a subject's physiological variables and/or environmental conditions [3];

### Artefact obliteration [4].

- the present example further comprising of determination of artefact and/or noise or background signal contamination and associated reduction or obliteration [4].

### Dimensionality and redundancy reduction or minimisation of signals determination [5].

- General physiological dimensionality minimisation or more specific dimensionality minimisation can be deployed. For example, in the case of sleep related breathing disorders such as snoring accompanied by apnoea breathing sounds by way of a microphone, along with apnoea detection capable of determining cessation of breathing sounds could be adequate and also provide the most streamlined but effective monitoring and tracking solution for an individual.

In another more specific example of dimensionality minimisation in terms of determining the essential **EEG** locations required for monitoring and tracking specific neurological disorders (such as epilepsy monitoring localised to a specific individual), a pre-processing technique deploying principal component analysis (PCA) and ICA analysis steps could be involved. For example (per section titled: "AUTISM SPECTRUM DISORDERS (ASD); ASPERGERS (ASP); DYSLEXIA; ", detailed elsewhere in this document) , a process comprising of deploying ***PCA can be applied in the first place to identify signals with higher SNR,*** followed by ***Independent Component Analysis (ICA)*** as a means of increasing accuracy of neural source localised Dipole localisation (dimensionality) determination and/or coherence determination;

### Monitoring sensor(s) dimensionality minimisation [6]

### MONITORING SENSOR(S) REQUIREMENTS DETERMINATION - I.E. DIMENSIONALITY MINIMISATION AND REDUNDANCY [6].

The present example can determine the requirement of physiological monitoring parameters including any of or any combination of (but not limited to):
- ER parameters such monitored from one or more simultaneously recorded AEP physiological channels (including inline MMN for attention measures);
- somatosensory (SSEP), parameters such monitored from one or more simultaneously recorded pain threshold (i.e. heat probe etc.) or electrical stimulus electrode measures);
- **EEG** parameters such monitored from one or more simultaneously recorded **EEG** channels;
- Other physiological parameter monitoring options;

### DETERMINATION OF A RANGE OF MINIMAL SENSOR AND MONITORING MODELS ALONG WITH CORRESPONDING ANALYSIS PARAMETERS (SEE ALSO MONITORING MINIMISATION)

- See also Improving CDR and Dipole Localisation/Dimensionality
- i.e. One technique designed to improve the CDR blind source separation problem in cases where there are more sensors than available source signals is to undertake a two stage process comprising firstly of applying Principal Component Analysis (PCA) followed by the application of Independent Component Analysis (ICA);
- The application here of the PCA analysis stage is to increase the input SNR of the subsequent ICA stage, thereby reducing the sensor dimensionality
- In this approach the ICA stage is used to separate the remaining composite signals into their respective independent components
- i.e. segregate the signals with >1 SNR within a more defined space using PCA in the first place before further specifying the number of signals of interest as part of the ICA process data minimisation

### Monitoring device(s) dimensionality minimisation [7]

Monitoring device(s) dimensionality minimisation based on combination of monitoring parameters, sensors, and/or associated monitoring device(s) and/or environmental sensor(s) (i.e. air quality or pollen etc. pollution monitoring based on determination of probable asthma or other associated breathing disorders) device(s) [7].

The present example has the provision of enabling monitoring devices to be determined and/or configured including wearable consumer (eLifeMEDICS) or professional (eHealthMEDICS) or nearby monitoring sensors or physiological monitoring system(s) including (but not limited to) any of or any combination of:
- eLifeNeuro; eLifeNEURO/EPILEPSY; AUTISM SPECTRUM DISORDERS (ASD); -ASPERGERS (ASP); DYSLEXIA; DIMENTIA /ALZHEIMER'S; Somfit ; eLifeWATCH; eLifeBUDS; eLifeKIT; MULTIPOINT TIME-SYNCH MONITORING (MTM) SYSTEM; eLifeSLEEP; eLifeCHEST/eLifeSCOPE (chestband); Adaptive Physiological-Body Network (APM) & NAS Gateway; eLifeBAND (MOBILE DEVICE & INTEGRATED SENSOR ARMBAND); eLifeDOPPLER; eLifePULSE; eLifeMOTION; and/or eLifeALERT;

### Analysis classification [8],

The present example provides **any of or any combination of analysis classification techniques** capable of determining the highest probability in terms of detecting events of interest or health conditions of interest by way of establishing a series of algorithm models ranging from the simplest (minimal model) to the more complex models (i.e. full model) based on **classifying and ranking analysis types and variants** capable of achieving the required (i.e. such as but not limited to that specified as part of "subject/patient-specific personalised information" (per **Figure 80** **[1]**) "monitoring goals" (per **Figure 80** **[2]**) of health management (HMS) system, the example further comprising any of or any combination of:
- **Transition from information to knowledge data-bases (****Figure 80** **[14])**
- Comparative analysis based on data bases of events of **patient-specific database references,** population **normal data-base references, population disease/disorder data-base references** normal interest can be established by way of any of or any combination of **expert system/intelligent analysis** (such as but not limited to **Figure 77****;** ***Figure 78*****;** **Figure 80****;** **Figure 79****); aggregated knowledge** (such as but not limited to **Figure 77****;** ***Figure 78***; **Figure 80**; **Figure 79****);**
- cross validation means (such as but not limited to **Figure 77****;** ***Figure 78***; **Figure 80****;** **Figure 79**);
- discriminant function analysis **(DFA)** (such as but not limited to **Figure 77****;** ***Figure 78*****;** **Figure 80****;** **Figure 79****);**
- **linear discriminate analysis (LDA)** (such as but not limited to **Figure 77****;** ***Figure 78***; **Figure 80****;** **Figure 79****);**
- support vector machine (**SVM**) analysis (such as but not limited to **Figure 77****;** ***Figure 78****;* **Figure 80****;** **Figure 79****);**
- largest Lyapunov exponent of the discrete wavelet packet transform **(DWPT);**
- dimensionality minimisation/redundancy reduction (such as but not limited to **Figure 77****;** ***Figure 78***; **Figure 80****;** **Figure 79****);**
- Clustering analysis - as further outlined in this patent application document elsewhere (such as but not limited to **Figure 77****;** ***Figure 78***; **Figure 80****;** **Figure 79****);**
- Data mining - as further outlined in this patent application elsewhere (such as but not limited to **Figure 77****;** ***Figure 78***; **Figure 80****;** **Figure 79****);**

### Broad-based monitoring analysis variants determination [9].

The present example provides **any of or any combination of analysis types or variants (i.e. analysis parameter configurations)** capable of determining the highest probability in terms of detecting "events of interest or health conditions of interest" applicable to establishing a series of algorithm models ranging from the simplest (minimal model) to the more complex models (i.e. full model) based on **classifying and ranking analysis types and variants** capable of achieving the required (i.e. such as but not limited to that specified as part of "subject/patient-specific personalised information" (per **Figure 80** **[1])** "monitoring goals" (per **Figure 80** **[2])** of health management (HMS) system, the example further comprising any of or any combination of analysis types presented in this document or and associated figures and descriptions.

### Broad-based generic analysis categories [9]

The present example provides **broad-based analysis types and variants** comprising any of or any combination of **stochastic** and/or **stationary** and/or **linear** and/or **deterministic** and/or **non-stationary** and/or **non-linear** variants of **spectral analysis and/or;**

The present example provides **broad-based analysis types and variants** comprising any of or any combination of **stochastic** and/or **stationary** and/or **linear** and/or **deterministic** and/or **non-stationary** and/or **non-linear** variants of **pattern recognition analysis and/or;**

The present example provides **broad-based analysis types and variants** comprising any of or any combination of **stochastic** and/or **stationary** and/or **linear** and/or **deterministic** and/or **non-stationary** and/or **non-linear dynamic analysis** variants of **Wigner transform analysis and/or;**

The present example provides **broad-based analysis types and variants** comprising any of or any combination of **stochastic** and/or **stationary** and/or **linear** and/or **deterministic** and/or **non-stationary** and/or **non-linear dynamic analysis** variants of **wavelet decomposition analysis;**

The present example provides **broad-based analysis types and variants** comprising any of or any combination of **stochastic** and/or **stationary** and/or **linear** and/or **deterministic** and/or **non-stationary** and/or **non-linear dynamic analysis** variants of **coherence decomposition analysis;**
- **stochastic spectral analysis** comprising any of or any combination of stochastic, stationary, linear, non-linear (including non-linear dynamical analysis) types;
- **deterministic spectral analysis** comprising any of or any combination of stationary, linear, non-linear (including non-linear dynamical analysis) types;
- offline or online learning and continued algorithm improvement process;

### SUPERSET SUBJECT/PATIENT SPECIFIC MONITORING & HEALTH TRACKING ALGORITHM MODEL

[10] A large range of biomarker and events of interest are examined with a wide range of analysis types in accordance to the monitoring goals, in order to ensure relevant health conditions are able to be detected. Additionally the super-set of analysis variables provides a wider scope or complex monitoring conditions [prior to determination of a minimalized monitoring configuration more applicable to routine or consumer-level monitoring (i.e. ***Figure 45***).
- Can be based on predefined events or health conditions of interest; i.e. for anaesthesia monitoring - basic statistical, spectral, complexity, entropy, chaos and morphology signal analysis, signal analysis. AEP analysis such as wavelet transform, fuzzy decision tree based selection procedures.
- Can be based on broad-based online or offline data-mining for events or health conditions of interest.

Analyses superset options based on health condition and events of interest applicable to prognostic and/or diagnostic precursors or incidents applicable to targets registered (i.e. via consumer/patient or health carer or expert based on individual's patient history or requirements) or configured by patient-specific consumer self-care or professional health care requirements or events or health conditions of interest.

Alternatively this analyses super-set can be set via the clinical or consumer purpose or application (i.e. anaesthesia, health, fitness, sleep, epilepsy, TBI. Dementia, Parkinson's, Alzheimer's, Autism spectrum disorders, dyslexia, ADHD, ***physiological or psychological or pathological transitions,*** monitoring parameters open-data-mining health condition tracking or events of broad-based interest (i.e. to select diseases onset or possible emergent conditions to enable earliest possible intervention). i.e. for dementia **EEG** source localisation (such as but not limited) to current density reconstruction analysis, followed by source reconstruction model such as (but not limited to any of two different classes of source models including distributed and local sources (i.e.. distributed sources can be found using the current density methods, while local sources can be computed using by Dipole fitting analysis. Moreover the present example provides a range of different source models which can be deployed in order to enable different assumptions about the nature of the sources, whereby typically these said sources are assumed to be small (sLORETA, Minimum Norm, SWARM, eLORETA, Lp Norm, L1 Norm,), or of approximate strength as their neighbouring brain signals (sLORETA). Moreover, the present example can provide one or more or any combination of models in order to best achieve the statistical performance outcomes applicable to health conditions or events of interest, based on complexity or minimisation (i.e. "CIDM to RCDM options) and/or monitoring dimensionality applicable to desire monitoring format and/or "analysis variants" or application (i.e. "monitoring goals").
i.e. in order to identify brain activation regions of interest as applicable to patient-specific diagnostic ("MONITORING GOALS") outcomes or disease data bases or normative comparative data bases) in specific regions applicable to disease of interest (i.e. coherence analysis (i.e. in order to identify brain connectivity of interest, such as (but not limited to) cortico-cortico or cortico-subcortical connections typical of dementia)) activation or interconnectivity, **EEG** frequency slowing (such as but not limited to dementia) regions of interest as applicable to patient-specific diagnostic outcomes or disease data bases or normative comparative data bases), and/or non-linear dynamic analysis (i.e. reduced signal complexity as distinguished by analyses such as (but not limited to) entropy, multiscale entropy, complexity analysis, the first Lyapunov exponents (L1), fractal dimension (FD), zero-crossing interval (ZCI), Hojorth-Index etc.) providing additional methods to conventional linear methods (such as spectral, Lempel-Ziv complexity and/or central tendency measures, vector analysis⁴⁷;

### SUBSET SUBJECT/PATIENT SPECIFIC MONITORING & HEALTH TRACKING ALGORITHM MODEL

[11] The subset algorithm model refers to the determination of specific analysis types, associated parameter configurations, along with the relevant physiological variables and associated monitoring sensors or sensor locations, applicable to the most relevant health condition management for each specific person.

### ANALYSIS VARIANTS DETERMINATION, BASED ON SUBJECT/PATIENT-SPECIFIC REQUIREMENTS EXPERT INPUT, MONITORING GOALS, SIGNAL DIMENSIONALITY AND REDUNDANCY REDUCTION, MONITORING CONFIGURATION, ANALYSIS CLASIFICATION [12]

Up to date range of analysis variants for complex to simplest monitoring configuration algorithms models [12].

By way of aggregated information and self-learning processes (i.e. such as the ongoing improvement of the knowledge base per ***Figure 77 [A]*** by way of comparing expert scoring or analysis via concordance analysis outcomes assisting the fine-tuning of the automatic monitoring analysis in order to enhance prognostic and diagnostic outcomes based on ongoing aggregation of information to continually improve inference engine interpreter outcomes ***Figure 77 [D]**, **with successive monitoring studies.*** In this way the present example can continue to assess the most effective processes capable of accurate prognostic and diagnostic outcomes for reach individual person, along with their associated "health conditions or symptoms of interest" or "events of interest" (i.e. per definition in abbreviation section at rear of this document).

### STATISTICAL PERFORMANCE ASSESSMENT DETERMINATION [13] STATISTICAL ANALYSIS

- Statistical analysis comprised of bivariate correlation, multivariate logistic regression, classification computation in accordance to goodness of fit criterion, and determination of the receiver operating curve.

### RANKING AND REGRESSION MODELING

- Ranking of regression models and then cross-validation incorporating 1,000-fold statistical cross-validation can be performed using the Monte Carlo technique, followed by procedures to determine the best prediction models based on expert user and/or expert system validation of events or health conditions of interest based on applicable diagnosis or prognosis disorders/disease. probability using minimal and full models.

### EXPERT SYSTEM AND/OR ARTIFICIAL ANALYSIS DETERMINATION [14]

- Whereby said "events of interest or health conditions of interest" can include *"**health conditions*** or symptoms ***of interest"*** or "events of interest/EOI" (per "EOI DEFINITION" detailed in abbreviation table at rear of this document).
   - any of or any combination of (but not limited to) signal characterisation and/or determination applicable to any of or any combination of ***physiological or psychological or pathological transitions or** "**health conditions*** or symptoms ***of interest" or*** "events of interest/EOI" (per "EOI DEFINITION" detailed in abbreviation table at rear of this document)*:*

### Statistical performance evaluation of complex to simplest monitoring algorithms models [13],

Statistical performance evaluation as a means of establishing performance assessment of the range of complex to simplest monitoring configuration algorithms models along with associated monitoring device configurations, monitoring parameters and associated analysis variants [13].

### Expert system and/or artificial intelligence determination [14].

Expert system and/or artificial intelligence determination as a means of aggregating various information inputs in order to enable continued algorithm review, analysis and improvement with the combination of expert cross-validation of data, subject/patient-acquired data and/or environmental sensor monitoring, data bases and other sources (i.e. environmental, weather, health, disease centres, WHO and other independent reports applicable to subject/patients health or well-being) [14].

The present example incorporates a means of enabling **expert systems** or **artificial intelligence** whereby said means incorporates the capability of examining the **subject/patient personalised information [1]** together with **statistical performance outcomes [13]** of analysis variants for monitoring algorithm models ranging from **simplest** to more **complex models [12].** As presented in ***Figure 78*** Prognostic and Diagnostic Knowledge Data Bases [6;7;8] are established and evolved with increased **knowledge** based on translation [2;5] and aggregation to system intelligence via internet/web-linked [3] expert and subject/patient applicable information sources [1;4].

The present example further enables the **fine-tuning** and **continuous improvement of analysis variants** based for each respective monitoring analysis algorithm's (ranging from minimal to full models) based on continuously assessing and reassessing models per **Figure 80** **blocks [1] to [14]**.

### TITLE: NEUROCOGNITION MANAGEMENT SYSTEM

### BACKGROUND NEUROCOGNITION MANAGEMENT SYSTEM

### Brain connectivity sequence determination

The present example provides means of varying the connectivity parameters and sensitivity of these parameters in a manner to tease out the range of subtle to course or crude connections across any relevant time period (i.e. where a relevant time period can related to event related tasks or stimulus (i.e. the deviant butterfly plot averaged mean or ERP signal or an audio-visual or related behavioural cognitive task challenge). In this way the present example can provide a stepped or connectivity sequence covering the pre-stimulus or event period (base-line) through to the stimulus or event onset through to the end of stimulus or event period through to the post stimulus or event period.

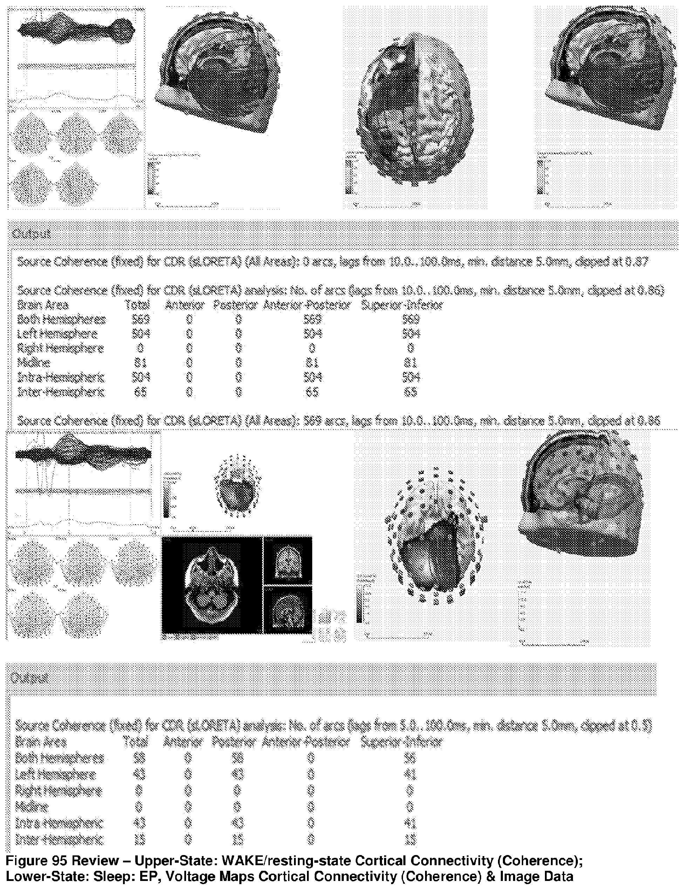

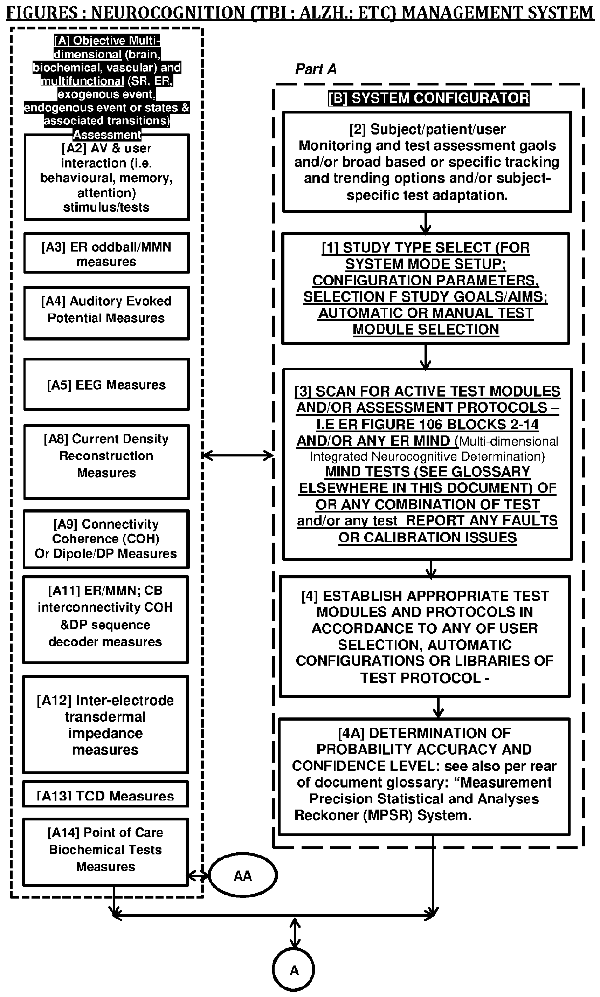

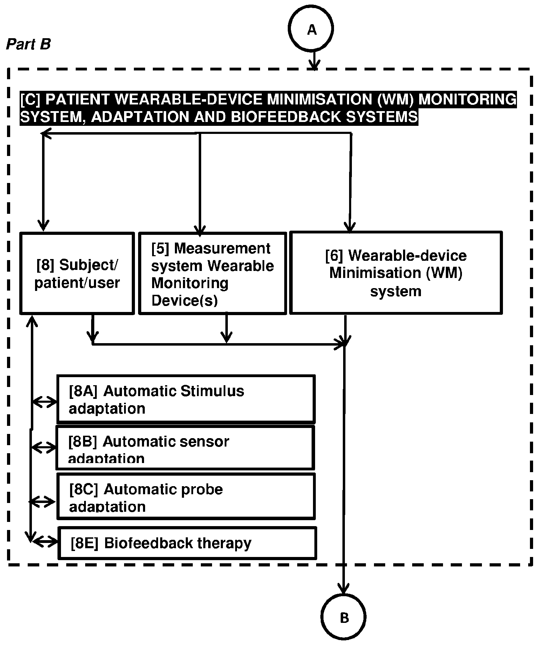

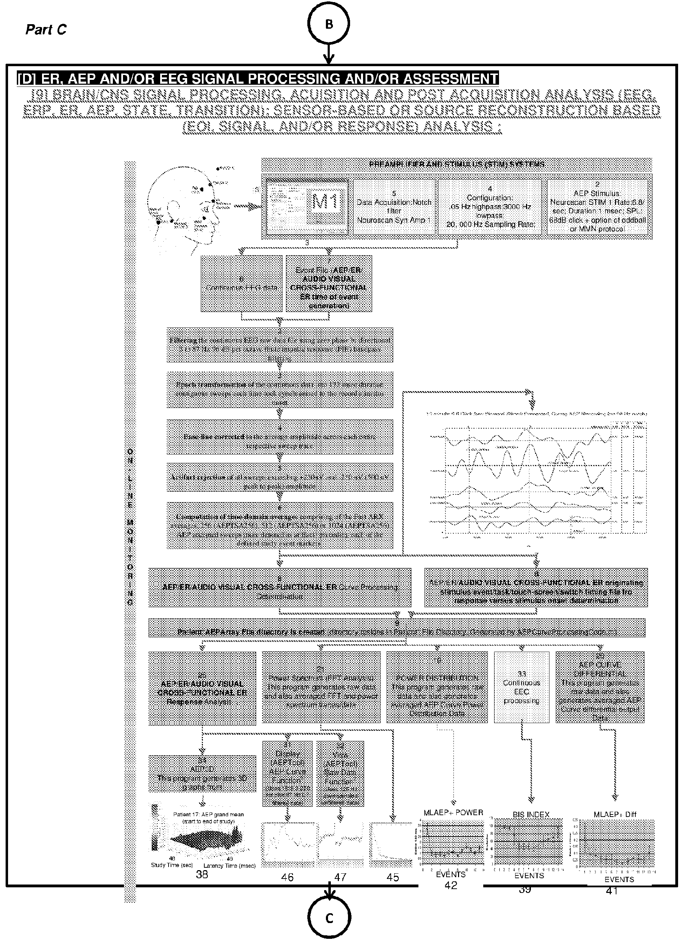

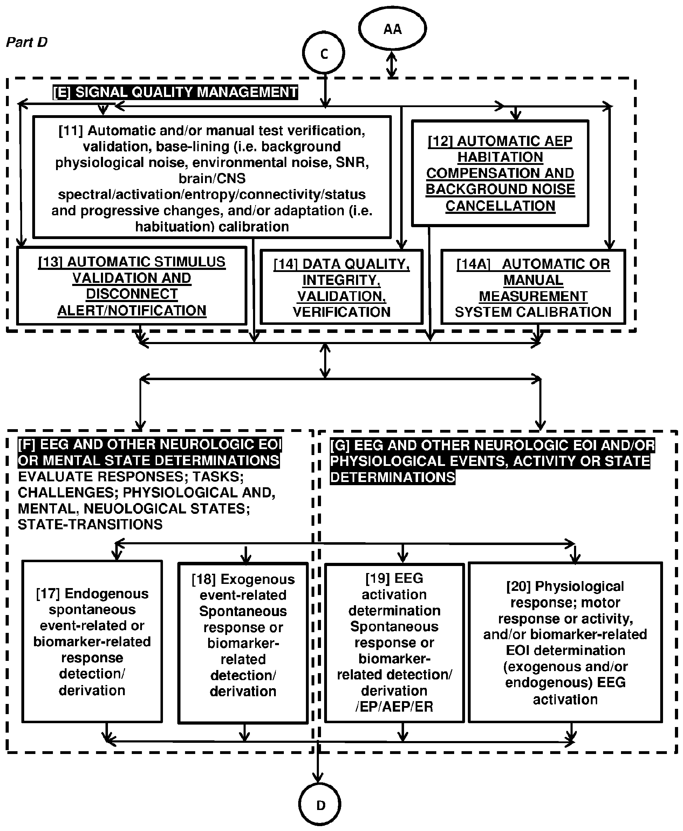

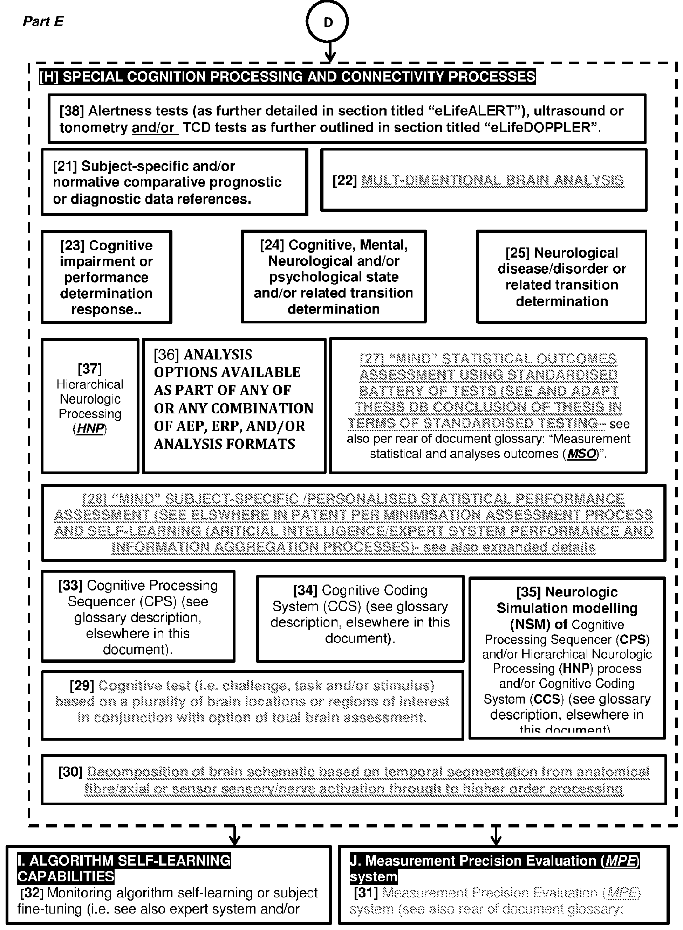

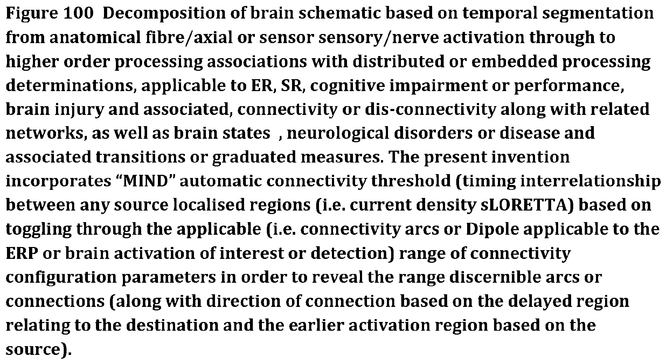

In this manner the automatic extraction of the ***Dipole*** (see description of example method covering processes from monitoring subject (***Figure 100******, BOX C)*** to processing of responses (***Figure 85******;*** ***Figure 88*****;** ***Figure 89*****;** (***Figure 100******, BOX D***), special response or endogenous processing of events and/or events of interest/EOI (see glossary) and/or MIND ER (see glossary) (***Figure 100******, BOX F**,* **G, *H);*** processes covering head-modelling **(*****Figure 86*****),** source reconstruction **(*****Figure 84******,*** ***Figure 86***), segmentation of modelled head (***Figure 86*****);** source reconstruction (SRC; ***Figure 87****)* coherence connectivity (***Figure 87****;* ***Figure 90***) and/or Dipole analysis (***Figure 91******;*** ***Figure 92******;*** ***Figure 93******);*** along with associated topographic, PCA and/or ICA outcomes or associated maps (***Figure 94******, lower left panel),*** source reconstructed image views (***Figure 94******, lower right panel),*** butterfly plot view of source reconstructed images (***Figure 94******, top-right panel***)*,* NLDB (entropy) AEP/ER brain activation analysis and/or AEP/ER differential brain activation and/or Integrated AEP/ER brain activation analysis and/or AEP/ER latency time-window analysis (***Figure 94******, centre left panel - i.e. cursor can define start, end and peak brain activation period of interest for source reconstruction and*/*or coherence connectivity and*/*****or Dipole modelling outputs)**,* coherence ARC connectivity visual outcomes (***Figure 94******, top left panel**)* or coherence ARC connectivity statistical outputs (***Figure 95******, lower panel**).*

Moreover the present example can aggregate health information, utilise self-learning (***Figure 100******, BOX I;*** ***Figure 77***), of fine-tuning and/or diagnostic processes using processes such as expert systems or artificial intelligence (***Figure 78******;*** ***Figure 77*****)** along with combining such aggregated information (***Figure 79*****),** across normative data bases or patient-specific data bases in order to produce a method of comparing any patient-specific data with nomative data bases (***Figure 78******)*** in order to achieve online comparative diagnostic or prognostic outcomes (***Figure 78*****),** (i.e. ***"******Shazam of healthcare tracking"***)*.*

The present example, can by way of these said ***"brain analysis signal processes and methods**"* (i.e. monitoring subject, response processing, source reconstruction, segmentation, coherence analysis, NLDB processing, Dipole modelling etc.) and methods analyse cognition states, examine cognition performance levels, examine levels of cognitive impairment, analyse transitions or levels of consciousness depth, examine mental and/or psychological and/or drowsiness (i.e. ***drowsy-subject example*** per ***Figure 67***) and/or sleep and/or wake states and/or altered states of consciousness (i.e. such as consciousness monitoring ( per ***Fiqure 94, centre left panel - i.e. cursor can define start, end and peak brain activation period of interest for source reconstruction and*/*or coherence connectivity and*/*or Dipole modelling outputs)*** in the context of any of or any combinations of SPECIAL COGNITION PROCESSING AND CONNECTIVITY PROCESSES per ***Figure 100******, BOX I including*** Cognitive Processing Sequencer (CPS) (see glossary description, elsewhere in this document); **[34]** Cognitive Coding System (CCS) (see glossary description, elsewhere in this document); **[35] Neurologic Simulation modelling (NSM) of** Cognitive Processing Sequencer **(CPS)** and/or Hierarchical Neurologic Processing **(HNP)** process and/or Cognitive Coding System **(CCS)** (see glossary description, elsewhere in this document), [36] ANALYSIS OPTIONS AVAILABLE AS PART OF ANY OF OR ANY COMBINATION OF AEP, ERP, AND/OR ANALYSIS FORMATS, and/or [22] MULT-DIMENTIONAL BRAIN ANALYSIS.

The present example, can by way of these said ***"brain analysis signal processes and methods"*** analyse cognition states, examine cognition performance levels, examine levels of cognitive impairment, analyse transitions or levels of consciousness depth, examine mental and/or psychological and/or drowsiness (i.e. ***drowsy-subject example*** per ***Figure 67*** ) and/or sleep and/or wake states and/or altered states of consciousness ( per ***Figure 94******, centre left panel** -* ***i.e. cursor can define start, end and peak brain activation period of interest for source reconstruction and*/*or coherence connectivity and*/*or Dipole modelling outputs)*** in the context of any of or any combinations of : a) **EEG** AND OTHER NEUROLOGIC EOI OR MENTAL STATE DETERMINATIONS (***Figure 100******, BOX F), and*/*or b) EEG AND OTHER NEUROLOGIC EOI AND*/*OR PHYSIOLOGICAL EVENTS, ACTIVITY OR STATE DETERMINATIONS ((******Figure 100******, BOX G),***

**A further example can determine brain connectivity and/or activation** sequencing (i.e. ***Figure 100*** **[33] ; [34] COGNITIVE PROCESSING SEQUENCER (CPS) AND COGNITIVE CODING SYSTEM (CCS) - see also glossary)** whereby sensor-based signal or spatiotemporal dynamic based (i.e. source reconstruction based) Dipole and/or coherence connectivity networks based on adjusting a sub-set of configuration parameters (i.e. associated with the peak signal average of a click ***AEP test paradigm)**.*

A more sophisticated rendition or so-called "reverse-engineering" of the brains wiring can be achieved using more sophisticated oddball or mismatched negativity test paradigms or relating special audio-visual tasks (i.e. prompting user to activate switch or touch screen in accordance to a specific challenge such a memory, mathematical, visual identification or awareness challenge).

By selecting a *sequence* of time intervals cascaded across the *pre event or* stimulus time to the *post event or stimulus* using the threshold teasing process (i.e. changing one variable at a time such as varying minimum lag time from 7.3ms to 45ms per example in (i.e. ***Figure 67*** demonstrates an example of an events based on AEP averages during a 3 minute DROWSY state whereby minimum lag time (or the time window of consideration in terms of computed brain activation coherence interrelationships), is shown for Min Lag of 7.4ms in top-let panel, 7.3ms in top-right panel, 40ms in lower-left panel and 45ms in lower-right panel). In this manner (as example only) the present example can step through the brain's Dipole connectivity arcs in order to establish a brain connectivity sequence.

Additionally, whilst the example shown here applies to a selected period or point in time relating to an event (shown in upper left hand box of each figure panel, and corresponding independent component analysis cursor indication, the present example can provide vary other parameters such as signal to noise ratio thresholds of ICA or PCA, COH clip values, CDR clip values, minimum lag times, maximum lag times, SCR type and parameter configurations, CDR type and parameter configurations, Dipole analysis type and parameter configurations, ICA or PCA parameter configurations and associated signal to noise threshold levels along with associated and underlying signal filtering parameters options, and other factors in order to "tease-out" or extract connectivity measures across range of fine to course settings. By way of examining the presence of Dipole and/or coherence incidences across the sequence representing a progressive sequence of time points from a pre-stimulus or event time to a post-stimulus or event time, the sequence of brain interconnectivity can be derived.

This said sequence can be presented for states such as resting or active cognition states or relating to various consciousness or altered states of consciousness or associated transitions. Moreover, the brain connectivity sequence determination can be compared to a subject's pre-diagnostic base-line or comparative values or associated databases relating to normative or disease control data bases. In this way ***brain connectivity sequencing*** determination can enable the diagnosis or prognosis of cognitive impairment severity or other neurological or cognitive disorders.

### Brain monitoring, source reconstruction, coherence and Dipole ERP analysis example

An example in terms of monitoring, signal processing, acquisition, and analysis of an AEP test and measurement protocol designed to assess consciousness states of an individual, is further described under "eLifeNLDB Non-Linear Dynamical Based (NLDB) analysis with option of automatic determination and option of online distributed processing capable of spontaneous source localisation applicable to diagnostic or prognostic determination and/or biofeedback or treatment of consumer/patient" section under sub-heading **"AUTOMATIC ONLINE TIME-SYNCHRONISED HYPNOGRAM AND SPATIOTEMPOAL DNAMICS CHARACTERISATION"** and referenced with **associated figures and related descriptions (but not limited to) any of or any combination of *Fiqure 45;*** ***Figure 56******;*** ***Figure 57******;*** ***Figure 58******;*** ***Figure 59******;*** ***Figure 60******;*** ***Figure 61******;*** ***Figure 62******;*** ***Figure 63******;*** ***Figure 64******;*** ***Figure 65******;*** ***Figure* 66****;** ***Figure 67******;*** ***Figure* 68****;** ***Figure* 69****;** ***Figure 70******;*** ***Figure 71******;*** ***Figure 72******;*** ***Figure 73******;*** ***Figure 74******;*** ***Figure 84******:*** ***Figure 85******;*** ***Figure* 86****;** ***Figure 87******;*** ***Figure* 88****;** ***Figure 89******;*** ***Figure 90******;*** ***Figure 91******;*** ***Figure 92******;*** ***Figure 93******;*** ***Figure 94******).***

The deployment of the interconnectivity sequence determination can be applied to a number of examples regarding the diagnosis and prognosis of neurological disorders or cognitive dysfunction.

The present example can further compare brain connectivity/interconnectivity across relatively straight forward click test and measurement protocols as described elsewhere in this section (i.e. per **""AUTOMATIC ONLINE TIME-SYNCHRONISED HYPNOGRAM AND SPATIOTEMPOAL DNAMICS CHARACTERISATION"** ") comparing states, transitions and levels of consciousness, unconsciousness, sleep, wake, drowsiness or resting states. Patient-specific pre-diagnostic baseline studies or broader-based population control and disordered or disease data bases enable a range of neurological or cognitive conditions or states or be identified.

### Brain connectivity sequence schizophrenia assessment example using neurocognitive test

In another application of the present example, such as in the case of schizophrenia where subjects/patient can benefit from personalised identification or guidance in terms of hallucinations versus genuine visual reception of an individual, ***the brain activation,*** the ***connectivity patterns*** and/or ***brain connectivity sequence*** associated with brain stem inputs indicative of ***authentic exogenous visual stimulus*** can be discriminate from hallucinations by way of a detecting an imbalance between brain stem related (exogenous information related to optical information reception of the eyes) versus mainly endogenous brain activation or connectivity information related to the occipital lobe.

### Brain connectivity sequence TBI assessment example using neurocognitive test (stroop test)

In one example a stroop (i.e. or other MIND ER tests per glossary at rear of this document) cognitive test could be conducted on a sports person who is suspected of a brain injury. The individual under test could have a simple or more complex wearable **EEG** monitoring head cap or monitoring device attached followed by being presented with a touch screen display challenge. The subject could be challenged to select the colour of a series of words (such as the name of a colour) displayed in the screen. In an attempt to test the cognitive function of the individual under test, both voluntary and obligatory ERP responses could be measures in conjunction with the user's task selections. ***Firstly,*** in terms of ***voluntary response cognitive assessment,*** the accuracy of the user's selections and capability of distinguishing ***deviant*** (i.e. mismatch between the word spelt out on screen and the colour of the displayed word) can be determined. ***Secondly,*** obligatory case the standard and deviant neurology responses corresponding to the users challenge to select the right colour, can be assessed by way of determining and examining any of or any combination of: a) ***ERP averaged ERP target or deviant responses,** b) **ERP brain activation,** c) **ERP coherence,** d) **ERP dipole connectivity,*** e) ***ERP coherence*** brain connectivity sequence determination, e) ERP Dipole brain connectivity sequence determination; f) ***comparison of pre-diagnostic or database*** of controls and cognitive impaired empirical data; g) ***objective statistical analysis*** of based on the progressive (i.e. if, for example, the tasks are spaced 1 second apart, 100 consecutive steps of 10ms with automatic thresholding comprising of toggling through configuration parameters (as described elsewhere), could be computed for ***relatively fine resolution brain connectivity sequencing*)** time sequence corresponding to the periods from ***pre-ERP event*** to ***post ERP event*** based on a series of tables of statistical measures (i.e. in the format of ***Figure 65***).

The present example provides the capability to measure the spatiodynamic (brain location within space and time) connectivity brain activity related to any ERP or AEP or ERP MIND (see glossary) in terms of fine or course intervals of categorised/non-parametric or parametric (infinite measures), whereby any of or any combination of brain analysis processes can be deployed including any or any combination of:
***a)***The present example, can by way of these said ***"brain analysis signal processes and methods"*** (i.e. monitoring subject, response processing, source reconstruction, segmentation, coherence analysis, NLDB processing, Dipole modelling etc.) and methods analyse cognition states, examine cognition performance levels, examine levels of cognitive impairment, analyse transitions or levels of consciousness depth, examine mental and/or psychological and/or drowsiness (i.e. ***drowsy-subject example*** per ***Fiqure 67***) and/or sleep and/or wake states and/or altered states of consciousness (i.e. such as consciousness monitoring ( per ***Fiqure 94, centre left panel - i.e. cursor can define start, end and peak brain activation period of interest for source reconstruction and*/*or coherence connectivity and*/*or Dipole modelling outputs)* in the** context of any of or any combinations of SPECIAL COGNITION PROCESSING AND CONNECTIVITY PROCESSES per ***Figure 100******, BOX I including*** Cognitive Processing Sequencer (CPS) (see glossary description, elsewhere in this document); **[34]** Cognitive Coding System (CCS) (see glossary description, elsewhere in this document); **[35] Neurologic Simulation modelling (NSM) of** Cognitive Processing Sequencer **(CPS)** and/or Hierarchical Neurologic Processing **(HNP)** process and/or Cognitive Coding System **(CCS)** (see glossary description, elsewhere in this document), [36] ANALYSIS OPTIONS AVAILABLE AS PART OF ANY OF OR ANY COMBINATION OF AEP, ERP, AND/OR ANALYSIS FORMATS, and/or [22] MULT-DIMENTIONAL BRAIN ANALYSIS.

The present example, can by way of these said ***"brain analysis signal processes and methods"*** analyse cognition states, examine cognition performance levels, examine levels of cognitive impairment, analyse transitions or levels of consciousness depth, examine mental and/or psychological and/or drowsiness (i.e. ***drowsy-subject example*** per ***Figure 67*** ) and/or sleep and/or wake states and/or altered states of consciousness ( per ***Figure 94******, centre left panel** -* ***i.e. cursor*** ***can define start, end and peak brain activation period of interest for source reconstruction and*/*or coherence connectivity and*/*or Dipole modelling outputs)*** in the context of any of or any combinations of : a) **EEG** AND OTHER NEUROLOGIC EOI OR MENTAL STATE DETERMINATIONS (***Figure 100******, BOX F), and*/*or b) EEG AND OTHER NEUROLOGIC EOI AND*/*OR PHYSIOLOGICAL EVENTS, ACTIVITY OR STATE DETERMINATIONS ((******Figure 100******, BOX G)**,*

**A further example can determine brain connectivity and/or activation** sequencing (i.e. ***Figure 100*** **[33] ; [34] COGNITIVE PROCESSING SEQUENCER (CPS) AND COGNITIVE CODING SYSTEM (CCS) - see also glossary)** whereby sensor-based signal or spatiotemporal dynamic based (i.e. source reconstruction based) Dipole and/or coherence connectivity networks based on adjusting a sub-set of configuration parameters (i.e. associated with the peak signal average of a click ***AEP test paradigm).***
***b)*** In a further example enables ***brain connectivity sequence scanning*** measures corresponding to connectivity related to ranges of interconnecting ***time delays between regions of coherence or Dipole connection,***
***c)*** ***brain connectivity sequence scanning*** measures corresponding to interconnectivity based on certain time windows or ranges of ***time delay between regions of brain activation, coherence or Dipole connections,***
***d) brain connectivity sequence scanning*** measures corresponding to interconnectivity based on certain windows or ranges of ***magnitudes between regions of brain activation, coherence or Dipole connections,***
***e)*** ***brain connectivity sequence scanning*** measures corresponding to interconnectivity based on certain time windows or ranges of ***NLD signal properties between regions of brain activation, coherence or Dipole connections,***
***f) brain connectivity sequence scanning*** measures corresponding to interconnectivity based on certain time windows or ranges of ***spectral signal properties between regions of brain activation, coherence or Dipole connections;***

### Automatic brain connectivity sequence scanner for diagnostic, prognostic and mental state determination

The present example provides the capability of analysing one of more neurology signals (i.e. including any of or any combination of PSECT, PET, MRI, CAT, XRAY, MEG, **EEG)** in order to determine the brain connectivity sequence relating to any of or any combination of states or levels of consciousness or altered consciousness or neurological disorders or related severity levels or cognitive impairment or related levels or cognitive performance or related levels, or behavioural disorders of related levels or personality disorders or related severity, levels or levels or states along with associated consciousness transitions consciousness or neurological states (per drowsy or sleep versus wake states as covered elsewhere in this document (i.e. see also expanded details of underlying analysis processes under sub-headings **"AUTOMATIC ONLINE TIME-SYNCHRONISED HYPNOGRAM AND SPATIOTEMPOAL DNAMICS CHARACTERISATION",** "Brain monitoring, source reconstruction, coherence and Dipole ERP analysis example", "Brain connectivity sequence schizophrenia assessment example using neurocognitive test", "Brain connectivity sequence TBI assessment example using neurocognitive test (stroop test)").

### Automatic brain connectivity sequence scanner therapy

The present example provides a means of enabling **"Automatic brain connectivity sequence scanner therapy"** - i.e. electrical Magstim forward and reverse equation options with simultaneous monitoring ((i.e. including any of or any combination of NFIR, SPECT, PET, MRI, CAT, XRAY, MEG, **EEG)** capable of targeting brain exogenous (stimulus) brain energy based on connectivity sequence modifications (i.e. enhancing behavioural, personality, mood, and other psychological or neurological changes - i.e. empathy regions of brain activated and logical regions deactivated; computational regions enhanced and emotional regions suppressed; conceptual or imaginary regions enhanced and logical or mathematical regions suppressed; for depression treatment serotonin biochemical generators activated and/or etc.;

### Example with underlying EEG monitoring, current density reconstruction, Dipole and coherence connectivity analysis, along with Automatic brain connectivity sequence scanner therapy

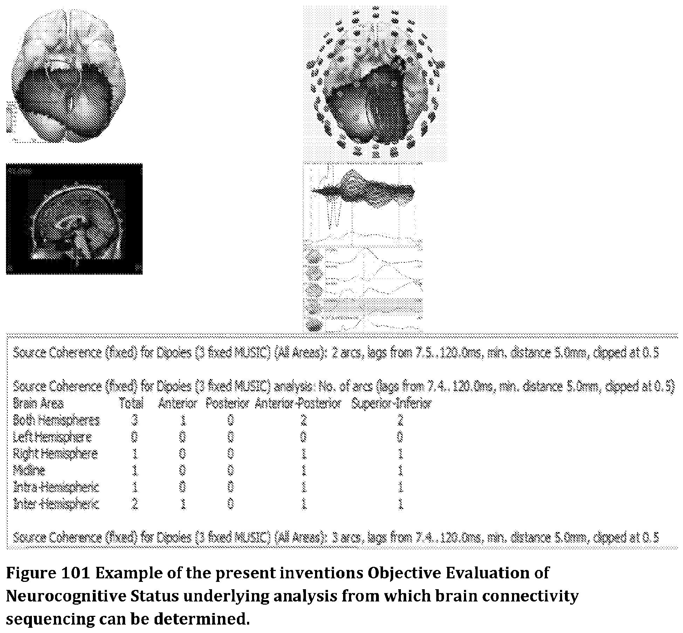

In one example in a first process phase enables computation of Dipole locations (***Figure 101*** **top left panel) based on** high density (133 electrode) function **EEG** recording converged with a standardised and averaged MRI image sleep corresponds current density reconstruction **(CDR)** outcomes (***Figure 101*** **top right panel).**

The present example can then determines **Dipole computations, along with the indication of related brain regions (i.e. in accordance to** *and*/*or associated control or **interconnectivity regions*** of the brain (i.e. as well as options for (but not limited to locations outlined in ***Figure 66***, ***Talairach Atlas;*** Current atlas tools; Harvard Whole Brain Atlas, MNI Template, The standard template of SPM and International Consortium for Brain Mapping; Atlas of the Developing Human Brain; All Functional including Brodmann areas, Gyri, Sulci etc.), which in this example appear to encompass the **Thalamus region** (***Figure 101*** **top left panel).** The most substantial Dipole **(RED)** also appears to be centralised according to the Thalamus region, which concurs with the literature associating Thalamus activation with deeper stages of sleep.

The present example can compute the image views, such as the left-lateral image view which in this example emphasises the central and occipital brain activation, typical during sleep (***Figure 101*** 2^{nd} **down left panel).**

Subsequently the present example can determine the Dipole locations and also ARC output table as shown with 3 Dipoles, with 1 located in the Anterior region and 2 in the Anterior-posterior/Superior-Inferior (upper rear brain) region (***Figure 101*** **lower panel)**

Additionally, the present example enables the computation of the coherence connectivity and associated output table of connections can be presented.

The present example can further automatically identify via any of coherence connectivity, Dipole connectivity or topographic views As demonstrated from the brain top-view topographic MAPS sequences or incidents which are relevant to comparative sequences or incidents of interest in terms of comparative base-line (or pre-diagnostic reference studies) applicable to diagnosis or prognosis of cognitive impairment or neurological disorders of interest. For example, ***Figure 101*** **2****^{nd} down right panel,** highlights the predominance of the 41 msec (TP41) AEOP component, located around temporal region is likely to be contributing to the emphasis of frontal region brain activation (i.e. anterior localisation of the 3 Dipoles per ***Figure 101*** **top left panel.**

The next phase or iteration of this analysis should review source dimensionality Given that there is such a large number of surface signals being mapped into so few Dipole outcomes, it may be worthwhile applying a multi-stage PCA to ICA approach to minimise dimensionality errors

For example, PCA can be applied in the first place to identify signals with higher SNR, followed by ICA analysis to improve the Dipole localisation (dimensionality)

As with all computational models, the assumptions and the calibration of the model can greatly influence outcome accuracy and model usefulness
Specific to this case study, greater definition of the source dimensionality using two stage approaches such as PCA followed by ICA is warranted in terms of improving the

Dipole source localisation accuracy and associated connectivity measures.

The present example can further compute the **Automatic brain connectivity sequence analysis,** as expanded upon elsewhere in this document under headings "Brain connectivity sequence schizophrenia assessment example using neurocognitive test", "Brain connectivity sequence TBI assessment example using neurocognitive test (stroop test)", "Automatic brain connectivity sequence scanner for diagnostic, prognostic and mental state determination".

Ain a further example simultaneously and/or subsequently computation of **Automatic brain connectivity sequence therapeutic analysis can be enabled,** as further expanded upon elsewhere in this document under heading ***"Automatic brain connectivity sequence scanner therapy".***

### TBI

Neurocognitive measurements for the assessment of cognitive impairment (i.e. Autism, TBI, Alzheimer's and other neurocognitive or behavioural disorders) can be confounded by factors such as habitation, the environment, the surroundings or incident(s) or the circumstances preceding or leading up to a cognitive assessment.

For example, the settling time related to the initial presentation of an AEP, ERP, task or other stimulus can be distorted by way of an individual's hypersensitive state, adrenaline hormone rush through the body prompted by environmental conditions such as a cheering or roaring crowd, or by the stimulation of the very event (i.e. a road accident, explosion etc. leading to the test.

In some cases the generation of a burst of endorphin neuro-transmitters brought on by the sensitive state or focus resulting from the novelty of a newly presented stimulus creates a period where the associated neural response magnitude is elevated while the subject under test settles or habituates to the test conditions, whilst in other conditions, subject to the rush and time required for such test results, the habituation may not be evident in the time required for such testing.

The present example provides a means of measuring the standard stimulus (baseline) versus deviant (oddball change in stimulus) response of an individual in terms of responsiveness time and magnitude of response so that the elevated habituation factor can to some degree be mitigated by way of using a measurement ***"ratio factors"*** (i.e. the ratio of any of or any combination of: 1) measure of ***magnitude*** of the test subject's response to deviant response, 2) measure of ***magnitude*** of the test subject's response to ***target*** response, 3) measure of time between stimulus onset and ***target*** response, 4) measure of time between stimulus onset and ***deviant*** response, 5) ***spectral markers*** associated with such ***"ratio factors",** 6) **signal dynamic markers*** (i.e. NLD measures such as entropy or signal complexity) associated with such ***"ratio factors",*** associated with such associated to the target response and/or the ratio of the individual magnitude of the test subject's response, 7) ***connectivity markers*** (i.e. Brain activation/i.e.CDR, Dipole or coherence as detailed elsewhere in this document), 8) ***brain connectivity sequence scanning*** measures corresponding to connectivity related to ranges of interconnecting ***time delays between regions of coherence or Dipole connection,** 9)* ***brain connectivity sequence scanning*** measures corresponding to interconnectivity based on certain time windows or ranges of ***time delay between regions of brain activation, coherence or Dipole connections,*** 10) ***brain connectivity sequence scanning*** measures corresponding to interconnectivity based on certain windows or ranges of ***magnitudes between regions of brain activation, coherence or Dipole connections,** 11)* ***brain connectivity sequence scanning*** measures corresponding to interconnectivity based on certain time windows or ranges of ***NLD signal properties between regions of brain activation, coherence or Dipole connections,*** 12) ***brain connectivity sequence scanning*** measures corresponding to interconnectivity based on certain time windows or ranges of ***spectral signal properties between regions of brain activation, coherence or Dipole connections;*** 13) Dipole or coherence brain connectivity sequence scanning measures as detailed elsewhere in this document), 13) by way of characterising target (deviant stimulus) response and comparing with spectral characterisation of standard stimulus response, 14) by way of characterising target (deviant stimulus) response and comparing signal dynamic factors (i.e. NLD measures such as entropy or signal complexity) with standard stimulus response signal dynamic factors (i.e. NLD measures such as entropy or signal complexity).

### TBI/COGNITIVE IMPAIRMENT MANAGEMENT

### Traumatic brain injury (TBI) prevalence and

The detection of concussion is crucial as even mild brain trauma can unleash a cascade of adverse neurological events, with the risk of manifesting significant short and long term neurocognitive impairment⁹⁹. Moreover, exposure to a subsequent concussion before recovery of the first injury can result in so-called "second-impact" syndrome, which can result in more serious longer term brain injury, and even fatalities. Therefore detection of the first brain injury is critical so that further injuries can be avoided.

Accurately identification of significant, but mild traumatic brain injury or concussion is not a trivial matter. For example, obvious signs of brain injury such as unconsciousness are reliable markers as large scale studies have already demonstrated that less than 10% of high school and college football players with concussion diagnosed injuries reported loss of consciousness¹⁰⁰.

The American Centre of Disease Control (CDC) reported that about 20% of individuals reporting mild TBI continue or concussion to experience post-concussion effects or cognitive defects one year after the original injury¹⁰¹. Based on these factors about 1 in 5 or about 60,000 of 300,000 student athletes are likely to exhibit prolonged cognitive defects as a consequence to brain injury incidence. Therefore the present example provides a ***personalised mobile and*/*or interconnected cognition therapy and*/*or assessment management options.***

### CONVENTIONAL NEUROIMAGING ASSESSMENT such as MRI, CT, or X-rays Electroencephalography (EEG) applicable to cognitive impairment (such as TBI, Alzheimer's & other dementia) measures and responsiveness testing

Whilst more macro-level brain injuries such as contusions, bleeds, and other obvious signs of more serious traumatic brain injuries can be detected using conventional neuroimaging techniques such as MRI, CT, or X-rays, mild TBI occurs on a neuronal cell body or axon micro-level ¹⁰²;¹⁰³;¹⁰⁴. Researchers have reported that mild traumatic head injury generates a cascade if events starting with the generation of excessive neurotransmitters¹⁰⁵.

***The present*** example ***can distinguish connections and*/*or disconnections*** at level of axons, dendrites, neurons, and/or synapse (such as can be damaged via brain injury including concussion which can lead to shearing of these fibre connections) by way of distinguishing connectivity such as coherence between any two or more brain activation (i.e. **EEG** sensor based or associated source reconstruction) locations within a point or period in time and 3-dimensional space (anatomical sources of said "brain activation") within a subject's CNS or brain, whereby such "distinguishing" can be determined by examining the time delay (i.e. lag or lead times between a plurality of brain activation signals, whereby said brain activation signals can refer to changes of brain signals including (sensor or source localised) power, voltage, current, signal dynamic changes (i.e. entropy and/or complexity analysis), spectral, EOI (see glossary), MIND ER (see glossary), and/or MIND ER. Additionally, the present example can distinguish longer

In this way the present example can distinguish any of or any combination of connectivity or disconnection aspects associated factors ranging from very fast fibre connectivity aspects through to sensory aspects (i.e. auditory brain stem, post auricular muscle responses, refractory responses, obligatory responses, inhibitory responses, afferent responses, middle latency auditory evoked potential signals (MLAEP), long latency AEP, late latency AEP LLAEP, LoLAEP, odd-ball responses, mis-matched negativity, and/or other AEP or ER signals.

### Laboratory based versus ambulatory or point of care mTBI measures

The present example is primarily targeted toward patient ***wearable devices or ambulatory measures*** of measures of mTBI in order to improve the ***accessibility, affordability, timeliness and ultimately life improvement and time-saving consequences of effective mTBI ambulatory assessment.***

Not-withstanding the ongoing controversy in terms of the effectiveness of mTBI cerebral blood flow measures, conventional cerebral blood flow measures include laboratory-based SPECT and PET imaging systems combined with the use of radioactive isotope injections. Additionally, specialised MRI with specialised DTI analysis can be deployed to assess the integrity of fine fibre tracks (i.e. vessels or nerves).

Other TBI laboratory neurology assessment approaches includes the analysis of via ***maanetoencephaloarapahy (MEG) activity*** recorded with superconductive quantum interference devices **[*SQUIDs]).***

However, these approaches are invasive, restrictive access wise and time-wise to be effective in routine day to day screening or for precautionary purposes.

### AUTOMATED OR MANUAL DETERMINATION AND/OR ADAPTATION AND/OR CONFIGURATION applicable to cognitive assessment test regime which is adapted manually or automatically

Therefore, the nub of the present example is to present a number or cognitive and brain assessment tests ***capable of being formulated in* a** ***test regime which is adapted manually** or **automatically*** in terms of special test circumstances and the key assessment aims in an ***objective manner based on test circumstances (i.e.*** required level of non-invasiveness, required time-for test, seriousness or type of suspected injury) and ***confounding assessment factors*** (i.e. means includes taking into account in terms of errors, measurement confidence levels, impact of confounding factors, such as external environment interference, external sounds etc.), whereby Measurement statistical and analyses outcomes **(*MSO*** per glossary at rear of this document) and also statistically determined confidence level or measurement probability can be computed (i.e. Measurement Precision Statistical and Analyses Reckoner ***(MPSR per*** glossary at rear of this document).

### EVOKED POTENTIAL AND CONTINUOUS EEG cognitive impairment (such as TBI, Alzheimer's & other dementia) measures and responsiveness testing

Moreover the said "number or cognitive and brain assessment tests" can include any of or any combination of means (i.e. **EEG** monitoring and associated source localisation (i.e. but not limited to) examples per Figure 84,Figure 85,Figure 86, Figure 89, Figure 85 and/or related connectivity analysis including (but not limited to) coherence or Dipole analysis (i.e. but not limited to Dipole examples in figures Figure 91 far right panel and lower right panel) along with related statistical outcomes (i.e. but not limited to examples per and/or related connectivity analysis including (but not limited to) Figure 91 lower right panel, Figure 87, lower panel), *means* of *axonal transport and other neuro-cellular related cognitive impairment based on ongoing follow-up tracking and monitoring subsequent to head-injury incident comprising* of *[A] Objective Multi-dimensional (brain, biochemical, vascular) and multifunctional (SR, ER, exogenous event, endogenous event or states & associated transitions) Assessment* *Figure 100**, [BOX A], including [A2] AV* & *user interaction (i.e. behavioural, memory, attention) stimulus*/*tests, [A3] ER oddball*/*MMN measures, [A4]* Auditory Evoked Potential Measures, [A4] Auditory Evoked Potential Measures, [A5] **EEG** Measures, [A8] Current Density Reconstruction Measures, [A9] Connectivity Coherence (COH) Or Dipole/DP Measures, [A11] ER/MMN; CB interconnectivity COH &DP sequence decoder measures, [A12] Inter-electrode transdermal impedance measures, [A13] TCD Measures, and/o[A14] Point of Care Biochemical Tests Measures, along with associated BLOCK [F] **EEG** AND OTHER NEUROLOGIC EOI OR MENTAL STATE DETERMINATIONS , BLOCK [G] **EEG** AND OTHER NEUROLOGIC EOI AND/OR PHYSIOLOGICAL EVENTS, ACTIVITY OR STATE DETERMINATIONS , BLOCK [H] SPECIAL COGNITION PROCESSING AND CONNECTIVITY PROCESSES.

Subjective cognitive assessment approaches including commercially deployed tests such as ImPACT and CogSport lack scientific validation¹⁰⁶, do have some merit when used as part of a more comprehensive test regime¹⁰⁷, but need to be used with caution based on reported concerns and limitations¹⁰⁸⁻¹¹¹ .

None-the-less as reflected in the present example subjective behavioural tests and/or objective neurocognitive tests can form an integral element of an overall adaptive test paradigm of

### CHEMICAL MICRO EVALUATION relating to cognitive impairment (including TBI, Alzheimer's & other dementia) measures and responsiveness

The present example provides a means of measurement and/or analysis of physiological or cognitive parameters applicable to the determination of biochemical changes implicated as a consequence of brain injury or cognitive deficit/impairment (i.e. caused by concussion or pressure waves or other incident applying excessive force to the brain of a subject) whereby such means comprises any of or any combination of assessment of biochemical measures or changes comprising any of or any combination of:
a) Assessment and determination of the alteration of the binding characteristics of excitatory transmitters including glutamate, to the N-methyl-D-aspartate (NMDA) receptors (i.e. further depolarization with efflux of potassium and influx of calcium) can result in, whereby such ionic changes can trigger a cascade of acute and sub-acute deleterious bio-cellular reactions.
b) Assessment and/or tracking and/or associated determination of ***changes (i.e. rapid or significant changes) in glucose or insulin levels;***
c) Assessment and/or tracking and/or associated determination of ***changes (i.e. rapid or significant changes) in subject's sodium-potassium (Na+-K+) levels;***
   i.e. a dramatic jump in glucose metabolism (i.e. detection of rapid or significant change in glucose or insulin levels) can be triggered as a consequence of the extra exertion of the subject's sodium-potassium (Na+-K+) physiological-pump mechanism, attempting to restore lost neural membrane (subsequent to brain injury) by boosting the generation of adenosine triphosphate (ATP).
***d)*** Assessment and/or tracking and/or associated determination of measures or related changes or associated reactions or responses of "hyper-metabolism" ***by way of any measures of or combination of measures associated with glucose, insulin, sodium-potassium (Na+-K+)* (*i.e. graduated, rapid or significant changes) in glucose or insulin levels and*/*or, excitatory transmitters including glutamate, to the N-methyl-D-aspartate (NMDA) receptors;***
   The corresponding "hyper-metabolism" can occur in circumstances of cerebral blood flow reduction when the difference between associated glucose demand versus supply drives a significant cellular energy predicament. Accordingly, the resulting brain energy deficit can result in the brains lack of preparedness and post-concussive vulnerability, particularly susceptible with second brain injury circumstances¹¹².
e) ***Whereby said measures can include point of care testing devices for directly (i.e. scanning fluid sample directly) or indirectly*** (i.e. via chemical impregnated test strips designed to react or highlight measure of interest, whereby said highlight can be colour change of test strip or other chemical reaction capable of being scanned/ detected/analysed/determined) sampling samples of body fluids including any of or any combination of urine, perspiration, blood, tears, interstitial fluid, saliva;

### VASCULAR/PERFUSION cognitive impairment (including TBI, Alzheimer's & other dementia) measures and responsiveness

The present example provides a means of measurement and/or analysis of physiological or cognitive parameters applicable to the determination of **cerebral blood flow or vascular** changes implicated as a consequence of brain injury or cognitive deficit/impairment (i.e. caused by concussion or pressure waves or other incident applying excessive force to the brain of a subject) whereby such **means (i.e. TCD measures per below)** comprises any of or any combination of assessment of reduction of **cerebral blood flow related to concussion vascular or cerebral blood flow measures** able to be implemented in an ambulatory manner (i.e. means including portable for ambulance, road-side, battle field, emergency, sports-field, bedside, and other ambulatory or wearable monitoring solutions avoiding dependence on more cumbersome or fixed infrastructure equipment-based studies to confirm the **diffuse effects of concussion and mTBI** on blood flow, such as with **Single Positron Emission Computed Tomography or SPECT¹¹³,** Positron Emission Tomography or PET¹¹⁴, or **arterial spin labelling perfusion functional Magnetic Resonance Imaging or fMRI**¹¹⁵), applicable to the measurement and/or assessment of cerebral blood flow or vascular measures or related changes comprising any of or any combination of:
- whereby said **"ambulatory manner means"** include (but are not limited to) any of or any combination of **TCD, TCCD,** 3-dimensional-view-reconstruction **(3D)** transcranial Doppler measures, determination and/or associated tracking;

### AXONAL TRANSPORT cognitive impairment assessment (including TBI, Alzheimer's & other dementia)

The present example provides a means of measurement and/or analysis of physiological or cognitive parameters applicable to the determination of ***axonal transport and other neuro-cellular related cognitive impairment*** or changes implicated as a consequence of brain injury or cognitive deficit/impairment (i.e. caused by concussion or pressure waves or other incident applying excessive force to the brain of a subject) whereby such **means (i.e. EEG monitoring and associated source localisation (i.e. but not limited to) examples per** ***Figure* 84,Figure 85****,****Figure 86****,** ***Figure* 89****,** ***Figure* 85** **and/or related connectivity analysis including (but not limited to) coherence or Dipole analysis (i.e. but not limited to Dipole examples in figures** **Figure 91** **far right panel and lower right panel) along with related statistical outcomes (i.e. but not limited to examples per and/or related connectivity analysis including (but not limited to)** ***Figure* 91** **lower right panel,** ***Figure* 87****, lower panel)** comprises any of or any combination of assessment of ***axonal transport and other neuro-cellular related cognitive impairment*** disruption able to be implemented in an ambulatory manner (i.e. means including portable for ambulance, road-side, battle field, emergency, sports-field, bedside, and other ambulatory or wearable monitoring solutions avoiding dependence on more cumbersome or fixed infrastructure equipment-based studies), applicable to the measurement and/or assessment of ***axonal transport and other neuro-cellular related cognitive impairment*** measures or related changes comprising any of or any combination of:
a) ***means axonal transport and other neuro-cellular related cognitive impairment surrogate measures (i.e. any of or any combination of cognitive deficit or brain injury assessment tests per*** ***Figure 84* to *Figure* 85** ***assessment tests per above);*** ¹¹⁶;
b) ***means of axonal transport and other neuro-cellular related cognitive impairment based on ongoing follow-up tracking and monitoring subsequent to head-injury incident** comprising* of *[A] Objective Multi-dimensional (brain, biochemical, vascular) and multifunctional (SR, ER, exogenous event, endogenous event or states & associated transitions) Assessment* *Figure 100**, [BOX A], including [A2] AV* & *user interaction (i.e. behavioural, memory, attention) stimulus*/*tests, [A3] ER oddball*/*MMN measures,* [A4] Auditory Evoked Potential Measures, [A4] Auditory Evoked Potential Measures, [A5] **EEG** Measures, [A8] Current Density Reconstruction Measures, [A9] Connectivity Coherence (COH) Or Dipole/DP Measures, [A11] ER/MMN; CB interconnectivity COH &DP sequence decoder measures, [A12] Inter-electrode transdermal impedance measures, [A13] TCD Measures, and/o[A14] Point of Care Biochemical Tests Measures, along with associated BLOCK [F] **EEG** AND OTHER NEUROLOGIC EOI OR MENTAL STATE DETERMINATIONS , BLOCK [G] **EEG** AND OTHER NEUROLOGIC EOI AND/OR PHYSIOLOGICAL EVENTS, ACTIVITY OR STATE DETERMINATIONS, BLOCK [H] SPECIAL COGNITION PROCESSING AND CONNECTIVITY PROCESSES.
**c) means of generating test & measurement paradigms applicable to assessment of spatial dynamic characteristic of corpus callosum and in the cingulum brain regions, whereby such means can include (but are not limited to) generating a stimulus and assessing the subsequent source localised brain activity, along with spatiotemporal brain analysis including (but not limited to) EEG monitoring and associated source localisation (i.e. but not limited to) examples per** **Figure 84,Figure 85****,****Figure 86****,** **Figure 89****,** **Figure 85** **and/or related connectivity analysis including (but not limited to) coherence or Dipole analysis (i.e. but not limited to Dipole examples in figures** **Figure 91** **far right panel and lower right panel) along with related statistical outcomes (i.e. but not limited to examples per and/or related connectivity analysis including (but not limited to)** **Figure 91** **lower right panel,** **Figure 87****, lower panel) AEP or magnetic stimulation targeted to brain activation assessment of any of various brain regions;**
**d) means of assessing damage to the axons, most noticeable in the corpus callosum and in the cingulum,** included **neurofilament dephosphorylation based on surrogate measures *(i.e*. *any of or any combination of cognitive deficit or brain injury assessment tests per*** ***Figure 84 to Figure 85*** ***assessment tests per above); ,***
e) means of assessing **deficits in axonal transport and conductance,** as well as the **accumulation of amyloid precursor protein (also applicable to testing for Alzheimer's and other neurological disorders exhibiting symptoms of cognitive deficit or impairment);**
**f) disruption of the axonal architecture necessary for distributed cortical connectivity and communication *(i.e. any of or any combination of cognitive deficit or brain injury assessment tests per*** ***Figure* 84 to *Figure 85*** ***assessment tests per above);***
**g) diffuse axonal injury (DAI)**¹¹⁷;¹¹⁸;¹¹⁹;
h) means of assessing **amyloid precursor protein associated with mild traumatic head injury based on chemical assessment (i.e. direct scanning of biochemistry composition of body fluid including chemical impregnated (with option of chemical reaction chemical dye coding or surrogate measures *(i.e. any of or any combination of cognitive deficit or brain injury assessment tests per*** ***Figure 84* to *Figure 85*** ***assessment tests per above);***
**i) means of assessing susceptibility of axonal fibre tracts to damage including surrogate measures(*(i.e*. *any of or any combination of cognitive deficit or brain injury assessment tests per*** ***Figure 84* to *Figure 85*** ***assessment tests per above)***¹²⁰ ;
**j) means of correlating axonal injury with compromised performance on neuropsychological tests including surrogate measures** (i.e. any of or any combination of cognitive deficit or brain injury assessment tests per ***Figure 84*** to ***Figure 85*** assessment tests per above).

### Subjective SYMPTOM-BASED SELF-ASSESSMENT and reports cognitive impairment applicable to neurological disorders such as Alzheimer's and other dementia, along with brain injury such as mTBI or TBI assessment or responsiveness testing

The present example incorporates a range of subjective assessment capabilities which in the main hinge relatedly heavily upon user/patient co-operation versus physiological tests or biomarkers that are primarily obligatory measures.

The said "subjective assessment " reporting capabilities include the acknowledgement and recording of a subject/patient's related brain injury or other potential cognitive deficit causations such as the intensity and frequency of headaches, dizziness, nausea, sleep problems, attention, visual focus, visual tracking etc.

In terms of assessing neurocognitive performance such as memory function or attention focus the present example incorporates a wide range of tests that can be manually or automatically **ADAPTED** (i.e. ***automatic determination of required assessment criteria such as*** assessment environment, circumstances, conditions, equipment and other applicable test requirements) test configured and adapted (i.e. test configuration can be modified and outcomes presented to user for verification. i.e. subject to the ***level of non-obtrusiveness*** and ***assessment speed requirements*** of test, any subsequent impact upon the assessment outcomes confidence levels or probability or accuracy can be automatically determined and displayed, thereby enabling user to automatically select from a ***pre-configured range of test-setups* or manually *configure and store the settings of special test requirements*** applicable to system user or subject or patient) in terms of ***TEST SEQUENCE, TEST COMPLEXITY, TEST SOPHISTICATION, TEST ACCURACY, TEST OUTCOMES CONFIDENCE LEVELS,* applicable to *neurological* disorders such as Alzheimer's and other dementia, along with brain injury such as mTBI or TBI assessment or responsiveness testing, the present** example **further comprising any of or any combination of:**
1) configuration and adaptation in accordance to ***environmental factors*** (i.e. noisy football field) ,
2) configuration and adaptation in accordance to ***test circumstances*** (i.e. at boundary or near football oval etc.),
3) configuration and adaptation in accordance to the degree of ***non-invasiveness*** (i.e. touch screen eye to hand tracking capability for immediate motor control and dexterity measures),
4) configuration and adaptation in accordance to ***accuracy versus speed*** of test,
5) configuration and adaptation in accordance to ***precision*** of tests based on low to high ***joint statistical probability*** (for example only) of test outcomes measures (i.e.joint probability analysis),
6) configuration and adaptation in accordance to ***multi-dimensional measurement requirements*** along with corresponding joint probability analysis or outcomes accuracy or associated probability factors (i.e. **EEG,** EP, TCD, self-reports, **EEG** and or EP objective neurocognitive performance or behavioural assessments, blood tests) time period of test,
7) configuration and adaptation in accordance to ***critical* *nature of test (i.e. subject dizzy, unconscious, wobbly on* feet *etc.*)** ,
8) configuration and adaptation in accordance to ***available test resources*** for test, 6) available analysis resources for test analysis and report,
9) configuration and adaptation in accordance to ***available test equipment*** for test report the neurocognitive task assessments tests can be undertaken that comprise of various degrees of vulnerability or susceptibility in terms of exposure to subjective self-interpretation or other errors related to dependence upon an individual's voluntary responsiveness to a test or battery of tests.
10) configuration and adaptation in accordance to ***sequence of tests,***
11) configuration and adaptation in accordance to ***difficulty level or each respective test*** with associated accuracy and related probability of outcomes,
12) configuration and adaptation in accordance to ***coarseness of test** -* i.e. degree or severity range of testing from MTBI to more severe TBI,
13) configuration and adaptation in accordance to ***short or long terms follow-up assessment regime*** (i.e. immediate TBI test following incident or suspected incident or longer or medium terms assessment tests designed to allow follow up and follow through cognitive management assessment
14) configuration and adaptation in accordance to ***Cognitive therapy modes*** (i.e. brain training or exercises, Mag stim treatment, audi-visual mood training (i.e. stress management, relation, sleep inducement etc.);
15) configuration and adaptation in accordance to ***Automated, personalised mobile and*/*or interconnected cognition therapy and*/*or assessment and associated health management options.***

### Neurocognitive task or stimulus response ASSESSMENT and reports cognitive impairment applicable to neurological disorders such as Alzheimer's and other dementia, along with brain injury such as mTBI or TBI assessment or responsiveness testing

The present example enables a range of self-reports and cognitive assessment tests, along with associated processes encompassing behavioural or cognitive performance outcomes assessments including **neurocognitive task assessment,** covering aspects such as **attention and memory tests, along with a range of tests such as any of or any combination of those further outlined in section titled "eLifeNeuro/Subject-Specific EEG-Monitoring Minimisation" under headings** "Intelligence assessments", "Memory assessments", "Language assessments", "Executive function assessments", "Visuospatial assessments", "Dementia specific assessments", "Batteries of tests for the assessment of neuropsychological functions"; **tests including those further outlined in section titled "DIMENTIA /ALZHEIMER'S /ASD /ASP" under headings** "-Cognition Impairment Assessment and Determination", "Assessment and determination of Cognitive functional outcomes", "Assessment and evaluation the early dementia stages and/or severity of dementia", "Cognitive performance assessment", "-Rule learner (or Rule Induction) (Separate-And-Conquer method);","-Decision Trees (Divide-And-Conquer method)", "-Attention and focus tracking", and/or "-Visual Assessment of Viewer Responsiveness to displayed information:".

### Electroencephalography (EEG) MONITORING cognitive impairment applicable to neurological disorders such as Alzheimer's and other dementia, along with brain injury such as mTBI or TBI assessment or responsiveness testing

### Electroencephalography (EEG) MONITORING cognitive impairment applicable to neurological disorders such as Alzheimer's and other dementia, along with brain injury such as mTBI or TBI assessment or responsiveness testing

The present example enables **EEG** monitoring ranging from a simplified streamlined wearable device monitoring options (i.e. ***Figure 1*** ***lower left;, Fiqure 46, Fiqure 47, Fiqure 48,*** ***Figure 49******,*** ***Figure 50******,*** ***Figure 52******,*** ***Figure 54***) through to more complex and sophisticated monitoring options (i.e. *Figure **55******,*** ***Figure 51***)*.*

Additionally, longer term or follow up neurocognitive monitoring assessments and ongoing monitoring is possible by way of minimisation of the subject wearable monitoring device (i.e. ***Figure 45*** minimisation capability per ***block 8*** and ongoing assessment and improvement of monitoring and analysis parameters per ***block 13***).

The present example further enables the ***"evolving and continually improving"* (with** ongoing or progressive detection of incidents of interest and greater specificity of probable combinations and associated multivariate analysis techniques, such as possible by way of online or offline automatic or manual analysis such as possible with comparative analysis of MonteCarlo cross-coupled analyses outcomes (i.e. combinations or any of analysis of monitored signals based on signal dynamics (such as NLD outcomes, spectral, spatial dynamic, source localisation activations trends and changes thereof, connectivity such as coherence and/or Dipoles and/or statistical measures, with associated accuracy assessment measures (such as ordinal scaled measures including Pk probability statistical outcomes) of severity of epilepsy diagnosis or prognosis, and/or the option of associated ***"self-learning or artificial intelligence"*** ongoing automatic or computer assisted ***"learning"*** capability - i.e. the selection of monitoring channels and/or monitoring sensor locations and/or acquisition parameters and/or pre-acquisition signal processing (including artefact suppression, base-line adjustment, filtering, discrimination or separation between myogenic and neurogenic signals etc.)and/or post-acquisition processing (including artefact suppression, base-line adjustment, filtering, discrimination or separation between myogenic and neurogenic signals etc.) adapted in accordance to aims (i.e. diagnostic and prognostic goals) and requirements (i.e. constraints or monitoring environment or non-invasive preferences) associated with monitoring, can be progressively fine-tuned based on aggregated knowledge (i.e. per Figure 78 blocks 2 and 5 and per aggregation of knowledge per ***Figure 79*** )accuracy and relevant of diagnostic and prognostic outcomes (i.e. per ***Figure 45*** minimisation capability per ***block 8*** and ongoing assessment and improvement of monitoring and analysis parameters per ***block 13);***

The present example incorporates a means of monitoring, processing analysing one or channels of continuous spontaneous **EEG** or auditory evoked potential (AEP) **EEG** signals, whereby evoked response potentials can be analysed in order to produce quantitative **EEG** (Q**EEG) EEG** measures, representative of brain injury¹²¹. Additionally the present example can compute measures indicative of cognitive deficit associated with TBI and neurological diseases such as dementia, whereby said measures can distinguish any of or any combination of markers of neurological dysfunction including cognitive unbinding ¹²² or reduction in neurological cohesion, and effects such as anteriorisation^{123, 124}. Such changes subsequently generate shifts in neural topographic foci, temporal sequences and more general alterations of the **EEG** spectral composition, which can be monitored and determined by way of source localisation, topographic brain activation power or amplitude analysis, coherence and/or Dipole analysis as provided in sample in figures along with associated descriptions (i.e. ***Figure 55*** lower left panel showing independent component analysis with topographic brain activation maps presenting peak signal brain activation foci; ***Figure 55*** lower right panel showing source coherence Dipole computations; ***Figure 69*** visualisation brain analysis along with ***Figure 70*** events of interest (EOI) determination and tracking as a measure of cognitive impairment biomarkers; ***Figure 71*** example of spectral analysis and other biomarker determination and tracking; ***Figure 85*** lower left panel principal component analysis and associated brain activation topographic maps for foci determination; ***Figure 85*** lower right panel showing source coherence (connectivity) analysis demonstrating connectivity deficits apparent across certain regions of the brain areas (i.e. both hemispheres, left hemisphere, right hemisphere, midline, intra-hemispheric, interhemispheric resulting from TBI or other causations of cognitive or brain connectivity dysfunction or injury; *Figure **56*** NLDB/entropy, differential, fast online AEP or spontaneous event monitoring and/or analysis techniques (.e. autoregressive analysis with external modelling input function capable of generating 8 second online averaging with 9 click AEP test paradigms, for example only),and/or plus brain energy and associated connectivity (i.e. Dipoles; coherence).

The present example provides spectral monitoring capabilities whereby higher **EEG** frequency spectrum, congruent with functional cognition ^{125, 126} can be tracked. Low frequency (1-15 Hz) spectrum proportion capable of indicating alertness, and high frequencies in the 201 to 500 Hz range can be monitored and tracked as markers of cognitive function and/or capacity ¹²⁷.

A battery of cognitive tasks can be undertaken by way of the accompanying mobile display whereby ¹²⁸ high frequency **EEG** (HFE) bandwidth of interest (i.e. ranged from 100 to 475 Hz using C3-C4 electrode placements) can be monitored during cognitive tasks. The monitored **EEG** can then be subjected to discrete Fourier Transform (DFT) in a manner to enable the outcomes be segmented into DFT spectral components. The said spectral components can include frequency bands such as low frequency band (LFB; 1-15 Hz), intermediate (IFB; 16-50 Hz), high frequency band 1 (HFB1; 51-100 Hz), high frequency band 2 (HFB2; 101-200 Hz), and high frequency band 3 (HFB3; 201-500 Hz). Markers indicative of cognitive function can be tracked including 0.25 spectral power proportion across the high frequency (HFB3; 201-500 Hz).

### ERP MONITORING cognitive impairment applicable to neurological disorders such as Alzheimer's and other dementia, along with brain injury such as mTBI or TBI assessment or responsiveness testing

The present example enables the monitoring and analysis of ERP applicable to markers of cognition and brain injury including synchronous activation of excitatory post-synaptic potentials (EPSPs) of pyramidal cells located in the grey matter¹²⁹.

The ERP monitoring and signal analysis can disassemble the underlying amplitude and associated signal morphology of the ERP waveform's peaks and troughs as well the latency (from stimulus onset) in order to mathematically and statistically compute measures indicative of the quantity of synchronously active neurons, the number of synaptic delays from the sensory receptor, the nerve fibre conduction times, as well as synaptic efficiency factors¹³⁰, whereby these ERP signal aspects can be compromised following head injury (such as concussion) or in association with neurological disorders (i.e. Dementia, ASD, etc.).

### EEG SPONTANEOUS TASK OR STIMULUS PERFORMANCE task cognitive impairment assessment applicable to neurological disorders such as Alzheimer's and other dementia, along with brain injury such as mTBI or TBI

- See eye tracking per eLifeMOTION section.

### EEG CONTINUOUS TASK OR STIMULUS PERFORMANCE and MONITORING of Task cognitive impairment assessment applicable to neurological disorders such as Alzheimer's and other dementia, along with brain injury such as mTBI or TBI

### ERP SPONTANEOUS TASK OR STIMULUS PERFORMANCE and MONITORING of task cognitive impairment assessment applicable to neurological disorders such as Alzheimer's and other dementia, along with brain injury such as mTBI or TBI

- See eye tracking per eLifeMOTION section.

### ERP CONTINUOUS TASK OR STIMULUS PERFORMANCE and MONITORING of cognitive impairment assessment applicable to neurological disorders such as Alzheimer's and other dementia, along with brain injury such as mTBI or TBI

The present example provides measures of ERP corresponding to cognitive performance tasks (such as touch screen interface with task challenges for user/patient - i.e. to enter a sequence of numbers via a key pad or select a sequence of colours by pressing appropriate coloured buttons on display screen etc.) along with the monitoring of the corresponding ERP waveform, thereby enabling the measurement of the related peaks, troughs, amplitudes and accompanying latencies with respect to stimuli onset. For example, the present example can prompt the user/patient's to respond to a task such as a request to activate a response button each time a red circle ***(deviant or target stimuli task)*** is displayed on the screen. In this example each 8^{th}, 9^{th} 10^{th} or 11^{th} (generally randomised to minimise anticipation of subject and consequently require a greater degree of attention for subject to complete tasks, thereby providing a means of enabling a more sensitive test of awareness related to computing the latency and amplitude of the corresponding ERP responses to the target versus standard stimuli tasks) could be displayed whilst every other circle could be coloured blue ***(standard stimuli task),*** thereby presenting a so-called ***oddball task sequence).*** In another example an auditory stimuli can be presented to a subject (i.e. via earbuds or earphones generating a sequence of sounds such as clicks or tone pips) in a manner that enables the corresponding ERP (in this case auditory evoked responses) can be monitored. Similar to the ***visual oddball task*** a standard and deviant stimulus (i.e. two different tones) can be presented and the corresponding ERP waveform amplitudes, troughs, peaks, along with associated latencies can then be analysed. This analysis can enable the standard and deviant AEP responses to be compared and the ***difference or ratio between the standard and deviant responses can represent the degree of awareness or attention*** of a subject at any point in time. The present example can also compare the negativity of the ***deviant* /*target ERP response*** compared to the standard ERP response (i.e. by subtracting the target from the deviant response or vice versa, or implementing a so-called ***mismatched negativity determination)*** as a ***measure of attention or awareness.*** Typically a number of standard and deviant responses will be averaged in order to compute a signal with improve the accuracy of the attention or awareness computation by ***improving the SNR of the ERP averages**.*

In order for a subject/patent to ***successfully complete the oddball tasks*** (i.e. either responding to the visual task or demonstrating awareness of the audio oddball stimuli) subject/patient must maintain constant attention or vigilance. During the test procedure the **EEG** is monitored in order to record the ERP signals corresponding to each task (i.e. in example of visual challenge) or stimuli (AEP). An accurate identification (i.e. ***touch screen activation or via button activation*** in response to visual standard versus deviant/target stimuli or attention and discrimination of auditory standard versus target/deviant AEP stimulus) in example of AEP stimuli) or response (visual stimuli) of the target stimulus elicits a cortical response referred to as the P300, which as the name suggests typically appears about 300ms following the onset of the stimulus, with a its positive polarity (based on scalp electrode recording montage¹³¹.

In this way the present example can detect by way of the P300 ERP response determination generate **a neuroelectrical response measures indicative of cognitive processes applicable to the allocation of attention and activation of immediate memory process¹³², providing a measure of a subject/patient's attention and discrimination between differing stimuli¹³³.**

In this way the present example can enable the deployment of a portable, robust, objective measure or attention and neurocognitive discrimination skills within a 10 minute test period following a suspected brain injury incident, virtually anywhere and anytime.

### ERP CONTINUOUS TASK OR STIMULUS PERFORMANCE and MONITORING of cognitive impairment assessment applicable to neurological disorders such as Alzheimer's and other dementia, along with brain injury such as mTBI or TBI - AUTOMATED ANALYSIS CAPABILITY

The present example overcomes the cumbersome and complex nature of ***previous state of the art*** automated ERP analysis methods (continue to be time-consuming, restricted to highly skilled and qualified personnel and beyond reach of daily usage such as elderly, children, sports or roadside accidents and associated ambulatory cognitive assessment requirements) along with limitations associated with depending upon ***previous state of the art*** manual and/or hand-scored and/or visual scoring techniques (i.e. such as identification of ERP waveform peaks or troughs **¹³⁴**, applicable to ERP evaluation) including the cumbersome, complex, costly, and highly inter-scorer dependent nature of assessments. In particular, the present example provides an advanced, automated and objective based analysis process which incorporates mathematical analysis of ERP waveforms in order to extract any of or any combination of information pertaining to the amplitudes, latency and morphology of ERP waveform peaks, troughs, along with the associated mismatched negativity, validation of ERP waveforms in terms of characterising changes in SNR and/or spectral and/or NLD characteristics of responses and/or target versus standard responses.

### ERP ONLINE SIGNAL QUALITY VALIDATION CAPABILITY

The present example can incorporate automatic ERP signal to noise ratio (SNR) measures of quality and signal validation in order to enable exclusion of ambiguous or erroneous data that could otherwise degrade associated measures (i.e. a minimal ERP signal of less than twice the magnitude of the background noise could typically be rejected).

Such measures can include (but are not limited to) single point F-test (Fsp) signal to noise measurement, providing an online quality estimation measure, with enhanced SNR derivations ¹³⁵. While no broadly accepted AEP signal quality standard exists, researchers have reported a minimal AEP signal of twice the magnitude of the noise level.

### EEG AND/OR ERP CONTINUOUS TASK OR STIMULUS PERFORMANCE and MONITORING WITH OPTION OF ASSOCAITED CONNECTIVITY DETERMINATION (I.E. COHERECNE AND/OR DIPOLE MODELLING) of cognitive impairment assessment applicable to neurological disorders such as Alzheimer's and other dementia, along with brain injury such as mTBI or TBI - AUTOMATED ANALYSIS CAPABILITY

The present example provides the capability of magnetic-electroencephalography and/or electroencephalography (and/or other brain imaging modalities including CAT, MRI, PET, etc.) imaging and/or evoked response potential continuous and/or task or stimulus oriented (i.e. responsiveness measures synchronised to stimulus onset) monitoring with a further option for the determination of associated (i.e. associated with continuous monitoring of connectivity trends or changes or associated with stimulus or task event), as a measure of changes in brain functions such as the those related to reduction in cerebral blood flow in response to the brain trauma¹³⁶, or **damage to axonal transport through the brain's white matter**¹³⁷;¹³⁸;¹³⁹;¹⁴⁰;¹⁴¹, along with commonly reported symptoms relating to disorientation, attention or focus deficits, or more specific memory functions.

In this way the present example incorporates a means of evaluating connectivity between cortical regions by determining the **EEG coherence. For example, the** example **in** Figure 67 **demonstrates the capability to automatically *location-wise search and then sensitivity-wise seek threshold status.***

***In terms of automatic characterisation of connectivity sensitivity wise* the present** example **can pan across a range of parameter configurations is order to determine the precise sensitivity and related parameters associated with brain connectivity including any or any combination of Dipole and/or coherence parameters including (for example only) coherence clipping levels, current density reconstruction clipping levels, minimum lag times, maximum lag time, source coherence type (i.e. fixed, moving, rotating etc.), current density reconstruction type (i.e. but not limited to sLORETA as in this example), number of Dipoles, number of ICA compoenents, number of CPA components, sequence or ICA and/or PCA analysis, associated ICA SRN parameters, associated SNR PCA parameters etc.**

***In terms of automatic characterisation of connectivity location-wise* the present example can pan across a range of parameter configurations is order to determine the precise sensitivity and related parameters associated with brain connectivity including any or any combination of monitored EEG and/or ERP sensor pairs.**

**In this way the present example can automatically characterise the thresholds and location of an individual's connectivity and compare these to comparative grades of dysfunctional and normal cognitive empirical study or pre-diagnostic subject-specific outcomes in order to compute a diagnosis or prognosis of cognitive impairment along with a graded measures of related severity.**

**An example of comparative study, or online monitoring of the subject/patient with cognitive impairment determination can deploy, for example, automatic or manual *coherence* parameter configurations (i.e. per *coherence* parameter examples of** ***Figure 86*** **top-left panel and top-right panel) for EEG and/or ERP monitored signals, and/or automatic or manual *Dipole* parameter configurations (i.e. per *Dipole* examples** ***Figure 86*** ***centre* -left panel and centre-right panel) for EEG and/or ERP monitored signals.**

**Examples include Dipole modelling such as of EEG or ERP activity (i.e.** ***Figure 84*** **shows in top-left panel a butterfly plot of ERP signals monitored from multiple electrodes, with related ICA and topographic maps shown in lower-left panel, Dipole locations along with coherence arcs presented visually in the right-panel together with objective associated statistical outcomes of coherence evident across the listed regions of the brain as presented in the lower table, and applicable per this example to sLORETA to current density reconstruction (CDR) analysis based on various threshold parameters such as clipping levels, minimum lag time, maximum lag time, minimum distance, along with independent options of ICA and related noise estimation or SNR parameters per example in** ***Figure 92******.* In this example 133 channels but butterfly plot and related computed averages can 1 or more pairs associated with 2 or more monitored EEG/ERP signal channels, for purpose of computing Dipole visual analysis (per** ***Figure* 55** **top-right panel) or Dipole statistical analysis (per** ***Figure 55*** **lower-right panel) and/or coherence modelling related to any or of any combinations of said pairs) detected between any combination of applied monitoring electrode pairs, thereby enabling a measure of phase synchrony based on shared brain activity between multiple, spatially separated neural sources¹⁴², presented in terms of visual modelling (i.e. per example in** ***Figure 85*** **showing a subject in drowsy state** marked by apparent thalamus to occipital coherence activity), **mathematical computations such as (but not limited to** ***Figure 56*** NLDB/entropy, differential, fast online AEP or spontaneous event monitoring and/or analysis techniques (.e. autoregressive analysis with external modelling input function capable of generating 8 second online averaging with 9 click AEP test paradigms, for example only, along with analysis options as presented is associated description section of this patent document), and/or plus brain energy **showing AEP entropy, differential analysis, EEG spectral and phase analysis) and/or statistical coherence (i.e. lower panel in** ***Figure 85*****) or Dipole modelling (i.e. per right hand panel** ***Figure 91*** **showing an example of 3 Dipoles interlinked with Dipole connectivity arcs coloured dark blue, light blue, green). The monitored EEG or ERP brain signals can be signal conditioned (i.e. filtering and amplification), acquisitioned and then undergo analysis transformation from the time domain (i.e. time series data) to the frequency domain in a manner that preserves both the amplitude (magnitude) and phase information relating to the originally monitored signals so that the present example can extract and detect discernible changes (i.e. applicable for evaluation mild to more severe grades of traumatic brain injury or cognitive impairment/deficit) related to changes in cortical connectivity subsequent to the incident of brain injury of a subject/patient under investigation.**

**The present example enables such ERP and/or EEG connectivity measures to be deployed as part of a wireless linked wearable EEG monitoring apparatus that can be incorporated within a head protection device (i.e.** *Figure 54* **shows a typical head protection system that could be worn in foot, hand or virtually any ball sports, with the incorporation of an attachable [4] wireless monitoring system [4] providing the online or remote analysis capabilities, with further option of attached EEG/ERP monitoring electrodes [5], [6], [7], [8] along with further option of in-build speakers or earphones (i.e. sensor location designed to press towards subject's ear(s), above electrodes and central to [1] and [9]) to present auditory stimulus or performance assessment tasks to subject/athlete any point in time) or deployed as a simplified (i.e.** *Figure 46, Figure 47**,* *Figure 48**,* *Figure 49**,* *Figure 50**,* *Figure 53*)**or minimised (i.e. per minimisation function** *Figure* 45 **[8]) or configurable** *(i.e.Fiqure **52***) **mobile, wireless interconnected (i.e. LAN, WAN, IP, WWW, cloud-based software services, NAS etc.) wearable monitoring systems or more complex (i.e.** *Figure 51**,* *Figure 55*) **and sophisticated wearable monitoring system including the option for in-built speaker or earplugs as a means of presenting ERP auditory stimulus as a basis of computing EEG and/or ERP and/or task oriented (i.e. verbal commands and or visor generated visual prompts to test subject/patient during or associated with sport or other occupation as a clinical or precautionary screening process described herein).**

**The present** example **enables the computation of ratios or other measures comparing coherence thresholds (i.e. the range or levels at which coherence activity such as visualised or statistically computed brain interconnectivity links/arcs are discernible), whereby said ratios can include (but are not limited to the determination of threshold coherence measures based on comparing the response to target stimulus or task responsiveness (i.e. thresholds parameter settings applicable to interconnectivity activity) thresholds applicable to non-target stimulus or task responses (oddball/deviant/distractor) for a subject/patient at any point in time and also optionally anywhere (i.e. by way of utilising wireless interconnectivity to wearable monitoring systems and software services such as Cloud-based services, NAS, WAN, LAN, IP, WWW computer interconnectivity services etc.).**

In order to enable the diagnosis or prognosis of suspected brain injury and/or the associated degree of severity the present example can, ***based on normative population data or established empirical data related to comparative non-concussed or cognitively impaired subjects*** (including various degrees of severity as well as sample population stratification formats, such as age, gender, medical history considerations, pre-diagnostic assessments, average population measures, graded population measures of cognitive impairment or injury etc.), monitor, analyse, measure, and evaluate an individual's brain connectivity factors (i.e. threshold at which a certain degree of interconnectivity linkages or activity are discernible compared to the comparative normal or graded against comparative disease/disorder severity data bases).
- The present example can incorporate **neural source localisation with combined therapeutic (targeting brain therapy)** neural targeted energy dispensation (i.e. magnetic, electrical and/or ultrasound stimulation or therapy targeted using for example forward equation source reconstruction techniques), with options of measuring endogenous and simultaneously (i.e. biofeedback mode indicating with online cause-effect management capability) stimulating (exogenous) brain states, transitions, neurocognitive brain interrogation/tasks/challenges (including AV, AEP, ERP), including associated interconnectivity sequences, and activation or suppression.

### TBI

Neurocognitive measurements for the assessment of cognitive impairment (i.e. Autism, TBI, Alzheimer's and other neurocognitive or behavioural disorders) can be confounded by factors such as habitation, the environment, the surroundings or incident(s) or the circumstances preceding or leading up to a cognitive assessment.

For example, the settling time related to the initial presentation of an AEP, ERP, task or other stimulus can be distorted by way of an individual's hypersensitive state, adrenaline hormone rush through the body prompted by environmental conditions such as a cheering or roaring crowd, or by the stimulation of the very event (i.e. a road accident, explosion etc. leading to the test.

In some cases the generation of a burst of endorphin neuro-transmitters brought on by the sensitive state or focus resulting from the novelty of a newly presented stimulus creates a period where the associated neural response magnitude is elevated while the subject under test settles or habituates to the test conditions, whilst in other conditions, subject to the rush and time required for such test results, the habituation may not be evident in the time required for such testing.

The present example provides a means of measuring the standard stimulus (baseline) versus deviant (oddball change in stimulus) response of an individual in terms of responsiveness time and magnitude of response so that the elevated habituation factor can to some degree be mitigated by way of using a measurement ***"ratio factors"*** (i.e. the ratio of any of or any combination of: 1) measure of ***magnitude*** of the test subject's response to deviant response, 2) measure of ***magnitude*** of the test subject's response to ***target*** response, 3) measure of time between stimulus onset and ***target*** response, 4) measure of time between stimulus onset and ***deviant*** response, 5) ***spectral markers*** associated with such ***"ratio factors",** 6) **signal dynamic markers*** (i.e. NLD measures such as entropy or signal complexity) associated with such ***"ratio factors",*** associated with such associated to the target response and/or the ratio of the individual magnitude of the test subject's response, 7) ***connectivity markers*** (i.e. Brain activation/i.e.CDR, Dipole or coherence as detailed elsewhere in this document), 8) ***brain connectivity sequence scanning*** measures corresponding to connectivity related to ranges of interconnecting ***time delays between regions of coherence or Dipole connection,** 9)* ***brain connectivity sequence scanning*** measures corresponding to interconnectivity based on certain time windows or ranges of ***time delay between regions of brain activation, coherence or Dipole connections,*** 10) ***brain connectivity sequence scanning*** measures corresponding to interconnectivity based on certain windows or ranges of ***magnitudes between regions of brain activation, coherence or Dipole connections,** 11)* ***brain connectivity sequence scanning*** measures corresponding to interconnectivity based on certain time windows or ranges of ***NLD signal properties between regions of brain activation, coherence or Dipole connections,*** 12) ***brain connectivity sequence scanning*** measures corresponding to interconnectivity based on certain time windows or ranges of ***spectral signal properties between regions of brain activation, coherence or Dipole connections;***

### FIGURE DESCRIPTIONS NEUROCOGNITION MANAGEMENT SYSTEM

In an example one or more test apparatus can be incorporated in order to enable objective assessment based on one or more test apparatus incorporating a range of dependent or independent multi-dimensional (brain, biochemical, vascular) and multifunctional (SR, ER, exogenous event, endogenous event or states & associated transitions) measurement capabilities, as shown in ***Figure 100*** ***BOX A, BOX A*** test protocol and parameter settings are determined and set-up either automatically via a range of preconfigured test arrangements in accordance to ***BOX*** ***B SYSTEM CONFIGURATOR. The CONFIGURATOR BOX B*** also and based on one or more ***BOX A*** measurement apparatus ranging from neurologic event-related behavioural tests, including memory challenges, mathematical tasks, visual tracking exercises, assessment of co-ordination between touch screen gestures and visual tracking and selection challenges and a number of cognitive assessments (i.e. see also example per glossary at rear section of this document under event-related multidimensional integrated neurocognitive determination (MIND)) applicable to both structural (i.e. source localised brain activation) and functional aspects of a subject's cognitions state. A further example is to enable the system user to determine the test constraints (i.e. speed versus accuracy, for example) and then select a cluster of tests according to such factors. IN this way a range of test clusters according to such factors can be presented to system user for selection, or the system user can select their preferred pre-configured test cluster or alternatively a default system test configuration (factory default or previously setup for particular test requirements or application deployment purposes). As denoted by ***(AA)*** an ***interlink*** with ***SIGNAL QUALITY MANAGEMENT BOX [E]*** enables adjustment of signal conditions, artefact suppression, electrode positioning guidance or automatic repositioning as required by monitoring environmental conditions and other factors impacting signal quality.

One the system is configured per ***BOX B*** the user can then elect (alternatively can be preconfigured is accordance to particular test requirements and specific application deployment purposes) to utilise the ***PATIENT WEARABLE DEVICE MINIMISATION (WM) MONITORING SYSTEM, OR ANY OF THE ADAPTATION AND BIOFEEDBACK SYSTEM OPTIONS** per **BOX C.*** Further examples provide wearable device minimisation capabilities whereby automatic modelling analysis functions based on monitoring performance outcome measures (i.e. using Pk and other statistical; measures capable of distinguishing levels or consciousness or cognition by way of correlating outcomes measures with markers indicative of level of cognition performance or consciousness depth) per ***Figure 45*** block [8];[12], to enable minimised wearable devoice formats specific to subject monitoring goals (***Figure 45******; block [11]**.* Further examples include adaptation capabilities ***(per blocks 8A, 8B,* 8C *and also biofeedback therapy capabilities per block 8E).*** Additionally, BOX B highlights the capability to incorporate Measurement Precision Statistical and Analyses Reckoner ***(MPSR) System [per box* B** ***block 4A]** .*

***BOX [D] ER, AEP AND*/*OR EEG SIGNAL PROCESSING AND*/*OR ASSESSMENT*** provides event-related response (ER) derivations as well as continuous **EEG** processing outcomes. Additionally, eye, head, face and body movement or direction or location details can be assessed in accordance to complete range of measures outlined further in this document **(per *section titled: eLifeALERT, for example only*), *along with combinational analysis outcomes*** such as ***eye-tracking and cognitive responses*** in order to determine an individual level of cognitive impairment, brain injury or performance capabilities.

***BOX [E] SIGNAL QUALITY MANAGEMENT BOX*** enables verification ***[block 11]*** signal quality to be continuously monitored and adapted online in order to detect sub-standard measurement conditions (i.e. deterioration or unacceptable signal to noise ratio characteristics) and automatically compensate for poor signal conditions where possible and appropriate or otherwise notify system user (together with hints or tips on problem and countermeasures), along with logging error conditions as a part of outcomes reporting and system error or confidence level determination. Automatic tracking of stimulus connection ***[block 13]*** via techniques including SNR determination along with spectral identification of stimulus characteristics can be deployed to correct or guide correction or notify system user when related errors are evident or attention is required.

***BOX [F]*** provide analysis capabilities **applicable** to **EEG AND OTHER NEUROLOGIC EOI OR MENTAL STATE DETERMINATIONS.**

***BOX [G]*** provide analysis capabilities **EEG AND OTHER NEUROLOGIC EOI AND/OR PHYSIOLOGICAL EVENTS, ACTIVITY OR STATE DETERMINATIONS. Including** Non-linear dynamic based ***(NLDB)*** evoked response or continuous **EEG** processing capabilities (as further described in section titled "Non Linear Dynamical Based Analysis (NDBA) with option of spontaneous automatic online analysis", under heading "EXAMPLE OF AUTOMATIC ONLINE NLDB ANALYSIS AND SPONTANEOUS OR EVOKED EVENTS OF INTEREST DETERMINATION WITH SYNCHRONISED SLEEP/WAKE HYPNOGRAM AND SINGLE OR CLUSTER ANALYSIS OR ENSEMBLES OF EVENTS OF INTEREST INDICATIONS AND/OR BIOFEEDBACK TREATEMENT CAPABILITIES".

In ***BOX [H]*** the present example provides **SPECIAL COGNITION PROCESSING AND CONNECTIVITY PROCESSES** including any of or any combination of **Subject-specific and/or normative comparative prognostic or diagnostic data references *BOX H, block* [21]; MULT-DIMENTIONAL BRAIN ANALYSIS including any of or any combination of (but not limited to) Cross-functional evoked response association, *physiological aspects, motor skills aspects, neurological connectivity (i.e. EEG and*/*or AEP, and*/*or ER signals or responses), brain energy activation (i.e. EEG and*/*or AEP, and*/*or ER signals or responses),* Spontaneous responses, Event-related responses,** ***awareness, memory and other cognitive tests or challenges** ,* ***behavioural and*/*or brain activation responses, cognitive assessment*** ***tests (CAS),*** **memory** function tests, **executive function tests, visuospatial tests** [BOX H, block 22] **along with other tests such as those outlined in glossary MIND ER definition, computation of Brain/CNS changes, or brain/CNS states, or brain/CNS transitions, *Determination, assessment and reporting short versus longer time and*/*or anatomically separated cognitive impairment or brain damage and*/*or delay aspects associated with brain function,* Comparative or Self-calibrated/reference Processes [BOX H, block 22];** Cognitive Coding System (***CCS***) per ***BOX H, block 23*** (see also glossary); **Cognitive, Mental, Neurological and/or psychological state and/or related transition determination** per ***BOX*** ***H,*** **block [24]; Neurological disease/disorder or related transition determination *BOX H,* block [25]** ; **"MIND" STATISTICAL OUTCOMES ASSESSMENT USING STANDARDISED BATTERY OF TESTS (SEE AND ADAPT THESIS DB CONCLUSION OF THESIS IN TERMS OF STANDARDISED TESTING *including* any of or any combination of (but not limited to)** Standardised Measurement Battery for Cognition/TBI Assessment [BOX H, block 27], Measurement statistical and analyses outcomes (***MSO***) per ***BOX H block* 27** (see also glossary); Hierarchical Brain Characterisation **[BOX H, block 271; "MIND" SUBJECT-SPECIFIC /PERSONALISED STATISTICAL PERFORMANCE ASSESSMENT [BOX H, block 28] including any combination of (but not limited to) Cognitive test (i.e. challenge, task and/or stimulus) based on a plurality of brain locations or regions of interest in conjunction with option of total brain assessment [BOX H, block 291; Cognitive test per** BOX H block **[29]; Decomposition of brain schematic block** BOX H block **[30]**; **Simulation** or **modelling** of Cognitive Processing Sequencer **(*CPS*)** per ***BOX H, block 35*** (see also glossary);**ANALYSIS OPTIONS AVAILABLE AS PART OF ANY OF OR ANY COMBINATION OF AEP, ERP, AND/OR ANALYSIS FORMATS** BOX H block **[36];** Hierarchical Neurologic Processing (***HNP***) (see also glossary description, elsewhere in this document) per BOX H block **[37] Alertness tests (as further detailed in section titled "eLifeALERT"), ultrasound or tonometry and/or TCD tests as further outlined in section titled "eLifeDOPPLER" per** BOX H block **[38]**.

A further example is the **ALGORITHM SELF-LEARNING CAPABILITIES** per ***BOX [I]**.*

**Another example is Measurement Precision Evaluation** (***MPE***) **system per per *BOX [J].***

### Figure 100

**Figure 100** Decomposition of brain schematic based on temporal segmentation from anatomical fibre/axial or sensor sensory/nerve activation through to higher order processing associations with distributed or embedded processing determinations, applicable to ER, SR, cognitive impairment or performance, brain injury and associated, connectivity or dis-connectivity along with related networks, as well as brain states , neurological disorders or disease and associated transitions or graduated measures. The present example incorporates "MIND" automatic connectivity threshold (timing interrelationship between any source localised regions (i.e. current density sLORETA) based on toggling through the applicable (i.e. connectivity arcs or Dipole applicable to the ERP or brain activation of interest or detection) range of connectivity configuration parameters in order to reveal the range discernible arcs or connections (along with direction of connection based on the delayed region relating to the destination and the earlier activation region based on the source).
**>>Adaptable (manual or auto) MIND assessment system test selection and sequence in accordance to test requirements (i.e. AIM, Accuracy, speed, devices, etc.)**
**>>Adaptable MIND assessment test selection and sequence in accordance to test AIMS, and by way of manual selection of preconfigured test regime or by way of automatic scan and detection of available test and measurements systems or via configurations of T&M system in accordance to test requirements.**
   ***Block [A2]**-* AV & user interaction (i.e. behavioural, memory, attention) stimulus/tests
   ***Block [A3]-*** ERP oddball challenge
   ***Block [A4]-*** Auditory Stimulus
   ***Block [A51-* EEG** Measures
   ***Block [A8]**-* Current Density Reconstruction Measures
   ***Block [A9}**-* Connectivity Coherence (COH) Measures
   ***Block [A11]-*** ERP/MMN interconnectivity COH &DP sequence decoder measures
   ***Block [A12]-*** ERP/MMN ; CB interconnectivity COH &DP sequence decoder measures as described elsewhere in this document under headings " **Brain** monitoring, source reconstruction, coherence and Dipole ERP analysis example", "Brain connectivity sequence schizophrenia assessment example using neurocognitive test", "Brain connectivity sequence TBI assessment example using neurocognitive test (stroop test)", "Automatic brain connectivity sequence scanner for diagnostic, prognostic and mental state determination", and/or "Automatic brain connectivity sequence scanner therapy".
   ***Block [A13]-*** TCD Measures
   ***Block [A14]-*** Point of Care Biochemical Tests Measures (i.e. body fluid including, interstitial fluid, urine, saliva, perspiration, tears etc.
>> The present example accommodates aspects such the inability for any single measure or measurement methodology to provide a thorough or adequate description of an individual cognitive impairment or performance state.

### Subject/patient/user

### Monitoring and test assessment gaols and/or broad based or specific tracking and trending options.

[2]
- **User interface for multidimensional integrated neurocognitive determination (MIND) system. Incorporates configuration function (i.e. Wizard) enabling simplified and intuitive establishment of test regimes based on user preferences, available devices etc.**

The present example provides ***an objective multi-dimensional assessment of cognitive status or impairment or transition*** whereby such means includes ***means of configuring the associated test paradigm in accordance to specific test circumstances*** either manually or automatically, whereby the example further includes any of or any combination of:
- Whereby *means of configuring in accordance to specific test circumstances* can be determined by any or any combination of factors such as required non-invasive or unobtrusive measurement requirements (i.e. if assessment is based at road side then the objective behavioural assessment may need to be limited to a series of verbal questions or challenges designed to grade the degree of cognitive impairment. Based on other injuries or the type of injuries at this stage the assessment may require neurological intervention such as the determination of excessive intracranial pressure by way of roadside transcranial Doppler measures of pulsatility index or Thrombolysis in Brain Ischemia (TIBI) transcranial Doppler flow grading to predict clinical severity (determination of persisting perfusion or residual flow around brain vessels which become suppressed or blunted in terms of flow characteristics as intracranial pressure rises after the incidence of concussion).
- If in another example a school child has received, or has a suspected bump or jolt to the head such as during a sports incident, may be warranted to complete the full range of tests ranging from ***[block A2]*** through to ***[block A14]*** due to the less critical nature of the environment and also the importance of ensuring the child does not have a secondary assault brain injury assault, latent reaction or related relapse.
- whereby said ***multi-dimensional assessment of cognitive status or impairment or transition*** can include any f or any combination of:
   - **Blocks [A2] to Block [A14];**
   - **Objective Behavioural and Performance Cognition Assessment [block A];**
      **AV and user interaction (Block A2];**
   - **Objective Neurophysiological Evoked Response Assessment (i.e. MMN or oddball AEP paradigm) *[block* A3];**
   - **Auditory stimulus *[block* A4];**
   - **Objective Neurophysiological Continuous EEG Assessment;**
      - i.e. signal dynamics, source reconstruction brain activity; spatiotemporal dynamics; connectivity (such as coherence or Dipole and/or associated dynamic sequencing and distribution brain function reconstruction; spectral characterisation; other events of interest/EOI determination and characterisation ***[block* 5];**
   - **Objective Transcranial Doppler Vascular Measure of Cognitive Impairment Assessment**
      - i.e. measurement of any of or any combination of: Transcranial Doppler intracranial hypertension in cases of traumatic brain injuries, cerebral autoregulation following minor head injury, pulsatility index, and/or vascular responses to neurological or cognitive test/challenges ***[block* A131;**
   - **Objective Assessment of Physiological Chemical Alteration or Transitions Associated with cognitive Impairment or more specifically TBI.**
      - i.e. Body fluid point of care testing whereby chemical reacting test strips applied to saliva, urine, tear-drops, interstitial fluid, perspiration, blood etc. can create a chemical response and associated reaction and colour change in chemical or via automatic, scanning, determination and diagnosis of body fluid sample under analysis.
      - The present example can incorporate a point-of-care test (such as chemical impregnated test strips or direct analysis of blood, urine, interstitial fluid, and/or perspiration (i.e. wrist watch or other wearable device) enabling the testing of elevated protein levels (i.e. associated with cerebrospinal fluid, for example) present in a subjects bloodstream, interstitial fluid, urine or other body fluids which can reveal whether a person has suffered a concussion ***[block* A14];**

### [1] STUDY TYPE SELECT (FOR SYSTEM MODE SETUP; CONFIGURATION PARAMETERS, SELECTION F STUDY GOALS/AIMS; AUTOMATIC OR MANUAL TEST MODULE SELECTION

- The present example enables **neurocognitive and neurologic test paradigms to be configured in accordance to specific test circumstances.**
- The present example incorporates model selection capable of determining selection of test modules (i.e. per *Figure 100**,* incorporating any of or any combination of test modules Blocks [2], [3], [4], [5], [8], [9], [10], [11], [12], [13], [14], which can be integrated in a single measurement device or interconnected via wireless of other means in order to product an aggregated measures of multi-dimensional including physiological and/or cognitive and/or neurologic and/or vascular measure applicable to the diagnosis or prognosis of neurologic or cognitive disorders.

In one example the system user can emphasis measurement protocol in accordance to aspects such as speed and/or accuracy and/or complexity, whereby properties of such aspects can be adjusted automatically in an iterative and integrated manner.

In one example of the **automatic and interactive mode selective** the present example can adjust measurement accuracy level, measurement complexity level, measurement modules or test-types, speed of test and the system will automatically respond with indication of the corresponding options test modules, sensor or wearable monitoring configurations, speed of tests (time required for each test and overall process), by way of a **measurement configurator module.**

In a further example the **measurement configurator module** can program a range of special purpose test configurations or provide a library of factory default test configuration options.

### -Manual or Automatic Test Configurator based on test requirements, test environment, test-circumstances, test speed or time limitations, test accuracy requirements etc.

Manual or Automatic Test Configurator based on test requirements, test environment, test-circumstances, test speed or time limitations, test accuracy requirements etc.
- The present example include provides a means of configuring a test protocol in accordance to users requirements subject top default or automatically configured (i.e. based on identification of active or deployed test devices and associated configuration parameters). In this way the present example can be configured manually (i.e. each parameter configuration selected in accordance to applicable options or ranges), can be automatically configured via a range or preconfigured or factory default test protocols In conjunction with scanning of available test devices and associated parameter ranges) or configured by way of a configuration tool (i.e. such as a software wizard designed to guide user through configuration stages).

A sample (but not limited to) of the setup options or configuration parameters include any of or any combination of user **test requirements, goals of test (i.e. specific type or range of cognitive or neurological disorder diagnosis or prognosis aims or requirements), test speed requirements or time limitations for assessment process and/or test outcomes/report, test accuracy and/or assessment confidence level requirements, test environment (i.e. ambulance test, intensive care tests, doctor general practice tests, football or ball game live verification for clearance to continue or cease play based on health factors), test-circumstances (i.e. on-field, field-side, court-side test, medical centre, hospital, ambulance, emergency, routine, etc.) , test devices available or required to be deployed (i.e. blood test, TCD, brain connectivity scan sequencer, test device configuration, test accuracy requirement, test speed requirements or test-timing limitations,** includes taking into account in terms of errors, measurement confidence levels, impact of confounding factors, such as external environment interference, external sounds etc.

### [3] SCAN FOR ACTIVE TEST MODULES AND/OR ASSESSMENT PROTOCOLS - I.E ER FIGURE 106 BLOCKS 2-14 AND/OR ANY ER MIND (Multi-dimensional Integrated Neurocognitive Determination) MIND TESTS (SEE GLOSSARY ELSEWHERE IN THIS DOCUMENT) OF OR ANY COMBINATION OF TEST and/or any test REPORT ANY FAULTS OR CALIBRATION ISSUES

### [4] ESTABLISH APPROPRIATE TEST MODULES AND PROTOCOLS IN ACCORDANCE TO ANY OF USER SELECTION, AUTOMATIC CONFIGURATIONS OR LIBRARIES OF TEST PROTOCOL OPTIONS

### Block [4]:

For example, the present example can enable testing **for evidence of impacted/altered brain function** with and without **an effective baseline data set specific to pre-diagnostic subject (under test) data.** In particular, based on there being no **available pre or post diagnostic measurement data** with many general patient populations or subject under test, the present example can produce a **number of cognitive and/or physiologic measurement ratios** (i.e. based on any "measures or analysis and detection or characterisation processes" or EOI (per glossary)" presented elsewhere in this patent document and /or any of or **any combinations of** "measures or analysis and detection or characterisation processes" or EOI (per glossary)" presented elsewhere in this patent document.
>> The present example can assess and measure compliance with tasks or challenges by way of a number of statistical approaches capable of combining one or more different ***"measurements"*** (i.e. any of and/or any variations of tests based on (example only but not limited to) blocks [2] to [14]. , Additionally, any combination of the said ***"measurements"*** can be computed as a combined outcome measure based on statistical approaches (including but not limited to) enabling any or more combinations of independent (i.e. ***probability statistics),*** dependent ***(joint probability statistics)*** and/or disparate or variant data set samples (i.e. using bootstrapping statistical techniques such as ***Monte Carlo analysis or Jackknife techniques),* as *outlined elsewhere in this document per sections detailing statistical approaches, joint probability, bootstrapping, Jacknife and Monte Carlo techniques.***

### I.E. IN ONE EXAMPLE TEST PATHWAYS FOR MULTIDIMENSIONAL ASSESSMENTS COULD INCLUDE ER (ODDBALL AND MMN); AEP (CLICK), EYE-TRACKING WITH VIDEO-VISUAL PRESENTATION TO SUBJECT; TRANSDERMAL IMPEDANCE ASSESSMENT; EYE AND HAND RESPONSE TRACKING; HEAD TRACKING; GAZE TRACKING; HAND-MOVEMENTS AND EYE MOVEMENTS (I.E. SYNCHRONISATION OR INTERRELATIONSHIP BETWEEN VISUAL PRESENTATION, USER'S INTERFACE TO TOUCH SCREEN OR SPECIAL SWITCH ACTIVATIONS AND EYE-GAZE OR MOVEMENT ANALYSIS; HEAD MOVEMENTS; TCD MEASURES USING AN ITEGRATED HEAD-SET WITH EEG/ER; TDC; COMBINED ELECTRDOE EEG AND TRASDERMAL IMPEDANCE MEASURES, BLOOD-TEST MEASURES, ALONG WITH HEAT-SET MOUNTED CAMERA FOR HEAD AND GAZE DETERMINATION AS WELL AS A SEPARATE EYE-TRACKING HEADSET MOUNTED CAMERA (I.E. EXTENDING FROM FOREHEAD TO TRACK EYE-MOVEMENTS)

### STATISTICAL DETERMINATION OF ACCURACY - PROBABILITY AND CONFIDENCE LEVEL DETERMINATION

- The present example can automatically compute the cumulative accuracy of one or more cognition assessment modules and associated configuration parameters (per example detailed under section titled "NEUROCOGNITION MANAGEMENT SYSTEM" under sub-heading "STATISTICAL ASSESSMENT OF CUMULATIVE TESTS"
- **[4A] DETERMINATION OF PROBABILITY ACCURACY AND CONFIDENCE LEVEL: -see also per rear of document glossary: "Measurement Precision Statistical and Analyses Reckoner (MPSR) System"**

### Statistical Assessment and Confidence or Probability Level of Cognitive Impairment Outcome Determination

- Based on determining the probability level of a cognitive impairment diagnosis, by way of computing the chance of one or more sequential physiologic or cognitive tests proving to be positive. In this way the permutations and combinations relating to one of more sequential tests can be computed based on the multi-dimensional test paradigms ranging from the simplest interactive touch screen challenge to the more sophisticated auditory evoked potential attention or awareness test paradigms or transcranial Doppler pulsatility index determination (i.e. to evaluate cerebral autoregulation following minor head injury cerebral autoregulation following minor head injury ) or incorporate a point-of-care test for elevated protein in blood relating to cerebrospinal fluid, associated with brain injury. ***[block* 4A]**;

### [block 4 and 4A];-Test Module Selection, Parameter Configuration, Test sequencer, and overall experimental controller

- **Integrated (to all test modules and associated parameter configurations) and automatic test accuracy determination.** The present example incorporates a ***"means"*** of automatically (with option of manual intervention) determining the optimal test modules.

***For example,*** any of or combinations of test modules such as (but not limited to) Blocks 2 to 14 per *Figure 100**,* can be configured as an integrated device or via a network or other interconnection between a plurality of devices, in a manner whereby the sequence and types of tests, together with factors such as configuration of parameters (i.e. complexity and number of EEG/ER monitoring sensors), can be determined in accordance to applicable test requirements, goals, test-circumstances and test accuracy requirements.

As it relates to the types of tests, *EEG*/*ER with simultaneous transcranial Doppler (TCD)* as part of the unique capability of incorporating TCD probe and auto-tracking (servo positioning or beam location of probe(s) in order to minimise support expertise and training required for accurate and effective deployment) within a single or plurality of wearable head-mounted device(s) incorporating any of or any combination of EEG/ER sensors and monitoring capability, and/or auditory stimulus capability, and/or visual display or stimulus capability. Additionally, the combined TCD and EEG/ER sensor, monitoring and assessment process could be supplemented with blood-test capability.

Whereby, in this example, the said *"means* of automatically (with option of manual intervention) determining the optimal test modules can include aggregating an amalgam of tests in a manner where the collective test outcomes are substantially more accurate and reliable than any singular or single dimension test (i.e. only **EEG** and/or ER response testing).

Such means of combining aggregating cognition test outcomes can incorporate means of combinational or permutation based statistic whereby any series and/or simultaneous number of test outcomes are aggregated to produce overall, superior measurement precisions.

Importantly no single physiological dimension of itself is likely to be able to describe or effectively asses the complex nature and disparate physiological impacts associated with micro traumatic brain injury (mTBI) or TBI.

The present example enables a means of aggregating a number of independent and/or dependent or interrelated tests in a manner to provide an overall superior outcome assessment. One means of joint probabilities of events that are not truly independent.

### [block 4 and 4A]; -MIND Test Combination & Permutation Determination and Configuration Module

The present example provides a means of measurement accuracy determination of assessment outcomes (i.e. projected confidence levels) of cognitive impairment based on test criteria as associated factors. i.e. based on:
- combinations and permutations of specific test measures
- nature of dependent versus independent relationship between multi-dimensional and multi-modality measures;
- specific test regimes,
- associated confidence levels;
- configuration parameters associated with each test and measurement process;
- signal quality associated with T&M (i.e. good, average, poor).

### [block 4 and 4A];- AUTOMATIC OR MANUALLY SELECTED TEST FORMATS AND DATASETS ORGANISED CONFIGURATION INCLUDING STATISTICAL ACCURACY CONFIDENCE

i.e. Test types, stimulus types and test sequence (i.e. test A. to J per blocks [5-14]; stimulus [1] to [3]) and as it related to independent test measures or dependent test measures.
**dependent Groups**
   **S2→B→D→E per blocks [3;6;8;9]**
   **S2→B→D→F per blocks [3;6;8;9]**
   **S2→B→D→G per blocks [3;6;8;11]**
   **S2→B→D→H per blocks [3;6;8;12]**
**>Independent Group 2**
**Test I per block [13]**
**>Independent Group 3**
**Test J per block [14]**

### [block 4 and 4A];-

### Neurocognitive Statistical Analysis Capabilities

### Multidimensional integrated neurocognitive determination system

The present example incorporates a means of integrating (i.e. wireless, data access, physical wired access between one or more measurement systems or other) of integrating one or more independent or dependent measurement device(s) (i.e. separate point of test chemical analysis of saliva, urine, tear drops, perspiration, interstitial fluid etc.) in a manner whereby the measurement data sets related to one or more marker of cognitive performance

The measurement precision statistical and analyses reckoner **(*MPSR*)** system (see also per glossary located in the later section of this document) enhanced ***accuracy probability neurocognitive assessment*** by way of ***permutation^{143, 144}*** and ***combinations¹⁴⁵*** factors¹⁴⁶⁻¹⁴⁸ along with capability of combining any of and combination of ***integrated multi-modality test systems*** (i.e. neurophysiological monitor combined with point of test blood biochemical analysis combined with TCD monitoring) monitoring, ***multi-dimensional*** monitoring aspects (i.e. TCD and neurological), ***multi-functional*** (i.e. different physiological and neurological functions) in order to generate automatically adaptable multivariate^{27, 149} and/or independent assessment test and regimes.

The present example can deploy ***jackknife*** estimations and bootstrap (generally useful for estimating the distribution of a statistic such as mean or variance without using normal theory, like z-statistic, t-statistic resampling statistical techniques), in order to deal with variance and bias estimations¹⁵⁰. Bootstrap **(bootstrapping does not make assumptions about distributions - i.e.** Any parametric statistical analysis assumes that the data being evaluated conforms to a normal distribution) method (i.e. Monte Carlo algorithm), which applies to **most random quantities including** the determination of the ***ratio of variance and mean,*** such as in the present example where a series of target and non-target event-related responses are measures and each of these two groups of responses these assigned randomly to the respective target and non-target response group, followed by analysing the difference or variance between these two groups of responses⁴³. In contrast to parametric statistical analysis, which are based on the premise that assessment data conforms to a normal distribution, the present example can analyse ER variance (i.e. non-target versus target responses) using bootstrapping approaches (i.e. do not assume "normal" statistical **distribution and provide exact probabilities for ER data comparisons. For example, and especially as** it applies to single subject/patient statistics, **the present** example **can incorporate randomization statistics, appropriate for ER measures.**
>> The present example can be deployed as a Class III (subject to FDA pre-approval) or Class II (FDA 510K equivalent approval, whereby the results of the test require interpretation by a skilled medical professional).
**(see also** Figure 45 **"wearable-device monitoring (WM) system).**

### [8] Subject/patient/user

### [5] Wearable Monitoring Device(s) requirements etc.

### [6] Minimisation Wearable Monitoring Device (s) Minimisation Methods requirements etc.

**[8A] Automatic Stimulus adaptation** - An example is to adapt stimulus in accordance to environmental conditions and/or stimulus generated responses (i.e. signal to noise ratio). For example, in circumstances where background noise is evident acoustic noise cancellation can be deployed to cancel out or minimise impact of background noise, which could otherwise distract subject from AEP stimulus. Similarly, stimulus parameters such as sound composition of stimulus signal can be adjusted in order "cut" through environmental sounds. "Cutting through" environmental sounds can be achieved, for example, by way of adjusting the spectral range of stimulus to be distinctive or distinguished from background noise. Additionally, the amplitude and spectral characteristics of the stimulus presented top subject can be adjusted in order to optimise the signal to noise characteristics of the measured subject response signals.

**[8B] Automatic monitoring sensor adaptation** - An example is to adapt sensor based on servo-controlled sensor or electrode location, whereby said sensor locations and/or angle of application to subject can be adjusted in accordance to optimal signal to noise ration from the monitored signal (i.e. small motors applying additional pressure between subject skin surface and sensor or electrode in order to improve signal; to noise characteristics automatically).

**[8C] Automatic monitoring probe adaptation** - An example is to adapt TCD probe, blood sample probes, electrical stimulators or other measurement system probes based on servo-controlled sensor or electrode location, whereby said sensor locations and/or angle of application to subject can be adjusted in accordance to optimal signal to noise ratio from the monitored signal (i.e. small motors applying additional pressure between subject skin surface and sensor or electrode in order to improve signal; to noise characteristics automatically).

**[8E] Biofeedback therapy** incorporating targeted brain stimulation or suppression capability, along with brain connectivity sequence stimulation in accordance to any of or any combination of analysis feedback or information including **EEG,** ERP, AEP, MMN, CDR, COH, Dipole, brain connectivity sequencing control etc.

**[8D] Multi-dimensional** (brain biochemical (i.e. blood test for spinal fluid etc.) , vascular, behavioural neurologic) and multifunctional (SR, ER, exogenous event, endogenous event or states & associated transitions per Block [2] to [14] per figure elsewhere titled "Adaptable (manual or auto) MIND assessment system test selection and sequence in accordance to test requirements (i.e. AIM, Accuracy, speed, devices, etc.)"

### [9] BRAIN/CNS SIGNAL PROCESSING, ACUISITION AND POST ACQUISITION ANALYSIS (EEG, ERP, ER, AEP, STATE, TRANSITION); SENSOR-BASED OR SOURCE RECONSTRUCTION BASED (EOI, SIGNAL, AND/OR RESPONSE) ANALYSIS ;

***BOX [D] ER, AEP AND*/*OR EEG SIGNAL PROCESSING AND*/*OR ASSESSMENT*** provides ***patient (E: top-left panel)*** monitored neurology signal pre-conditioning and post-acquisition filtering, data-acquistion, and stimulus test protocol configuration ***[BOX D, 9; block per preamplifier and stimulus (STIM) system, electrode channel configuration and location setup, boxes 5*; *4; 2],*** followed by monitoring **EEG *[BOX D; block 6],*** along with simultaneous monitoring and precise event timing determination for evoked response (ER) monitoring timing as it relates to recorded neurological data ***[BOX D; block 71. BOX[ D] then*** resolves evoked responses in the ***Filtering, Epoch Transformation, Base-line corrected, Artifact Rejection*** and ***Computation of time-domain*** (or frequency domain) averages, as denoted in the respective ***BOX D correspondingly labelled blocks.*** The ER resolution refers to resolving any event-related or evoked responses in terms of averaged or regressively modelled waveform characteristics **(per *BOX D centre-right panel examples showing averaged stimulus signals showing all brain wave deflections with reference to stimulus onset (0 ms x-axis marker))*** including oddball, audio-visual challenge or tasks, MMN and the like (as further expanded upon in glossary ***ER MIND*** examples). Other ER processing capabilities including the derivation of cross functional events, comprising of associating stimulus or audio-visual and other events or tasks/challenges can be determined per ***BOX D blocks* 8 *and* 9** in order to provide assessment of a subjects tracking or dexterity between hand (or finger gesture or selection) or other physiological motor requirements (based on tasks or challenges such as example (but not limited to) glossary ***MIND ER*** examples). Additionally, eye, head, face and body movement or direction or location details can be assessed in accordance to complete range of measures outlined further in this document (*per* ***section titled: eLifeALERT, for example only*), *along with combinational analysis outcomes*** such as **eye-*tracking and cognitive responses*** in order to determine an individual level of cognitive impairment, brain injury or performance capabilities. The present example can further compute a range of analyses outcomes based on the resolving or a subject's responses, whereby such outcomes include any of or any combination of: a) 3-dimentional (3D) views and results indicative of x-axis inter-stimulus response period, y-axis response amplitude and z-axis period over which test is conducted ***(per BOX D, blocks 25, 34 and graph outcomes per 38, 46 or 47 lower panel*)*,*** b) power or amplitude spectrum of spectral (i.e. FFT) monitored signals characteristics ***(per BOX D, blocks 21, 31 and lower graph 45*)*,*** c) ABR, MLAEP Long Latency AEP Late Latency AEP, MMN, oddball, special events (arousal, body movement, or other neurological or physiologic motor responses **(*per BOX D, block 19*)*,*** d) **AEP differential or integrated outcomes *(per BOX D, blocks 20, graph MLAEP+Diff),*** e) Bispectral and other continuous **EEG** signal processing outcomes such as spectral or NLD or coherence **(*graph* (*per BOX D, blocks 33, graph BIS INDEX).***

### Automatic and/or manual test verification, validation and signal compensation (i.e. automatic tracking and suppression of excessive artefact, base-line offset, suppressions of background electrical or acoustic other noise (i.e. background physiological noise, environmental noise).

### Signal Validation

[11] The present example provides the ability for determination and reduction and/or compensation and/or ignoring of data inflicted by poor (unacceptable) AEP or ER signal quality will result in unreliable and inconsistent AEP monitoring outcomes. The present example can deploy online ***signal to noise measures*** (i.e. such as F-value at a single point (Fsp)) in order to determine the appropriate or ***optimal number of averaged target or non-target stimulus responses in order to provide optimal signal to noise measures*** (i.e. greater than 6db) but at the same time ***retain time-series connectivity information by way of providing* as *many sequential progressive measures of brain*/*CNS change* as *possible across any given assessment periods.*** In this way the present example enables both measures of ***response changes*** (i.e. each progressive or running averaged response change) as well as ***progressive steps corresponding to coherence or Dipole connectivity measures*** in order to determine the related ***progressive connectivity sequencing*** (i.e. sequence of wiring or schematic of brain connections), during assessment test period.

### [12] AUTOMATIC AEP HABITATION COMPENSATION AND BACKGROUND NOISE CANCELLATION

### AUTOMATIC STIMULUS VALIDATION AND DISCOONNECT ALERTINOTIFICATION Block [13] STIMULUS DETECTION; UNWANTED BACKGROUND NOISE AND DISTORTION SUPPRESSION (INTERFACES WITH BLOCK 8A (I.E. STIMULUS ADAPTATION CAN MODIFY PARAMETERS OF STIMULUS TO IMPROVE RESPONSE SIGNAL TO NOISE WHERE REQUIRED)

**[13]** The present example provides a means (i.e. based on comparing the signal to noise ratio (SNR) of the originating stimulus presentation to that of competing or unwanted background noise or distortion. For example, the actual stimulus responses can be delineated from unwanted background noise or distortion signals by way of characterising the spectral associations between authentic stimulus and/or event related versus unwanted signal types. Additionally, authentic stimulus and/or event related versus unwanted signal types can be distinguished by way of associating the timing relationships between these two signal groups, based on the properties of stimulus or event responses being related to or synchronised with stimulus or event onset timing.

The present example provides a means (i.e. by monitoring background or unwanted or environmental noise such as noise from test environment or crowed sports locations and the like, in a manner where the unwanted noise, which can be recorded via a separate microphone for example, can be phase adjusted amplitude adjusted in a manner whereby the unwanted signals are out of phase (i.e. 180 degrees phase-shifted) from the wanted signals, so that signal cancellation or a "nulling-effect" can be achieved to suppress unwanted noise and accentuate wanted signals or responses). In this manner the most effective SNR can be achieved in order to enhance the delineation of the stimulus (i.e. sound) presentation and unwanted signal suppression (i.e. improve stimulus audio response versus unwanted background interference or noise), increasing the ***accuracy or effectiveness of AEP or ER neurocognitive assessment*** (i.e. determination of ER and/or AEP and/or deviant versus target responsiveness and/or MMN and/or P300) of subject under test.

The present example incorporates a means (i.e. novel stimulus presentation magnitudes can compensated in accordance to typical level increases during settling or habitation period when an individual tends to be more sensitive and thus responsive (neurocognitive) to AER or ER test protocols) of ***automatically compensating for habituation or settling effects related to AEP or ER test regimes.***

### DATA QUALITY, INTEGRITY, VALIDATION, VERIFICATION ASSESSMENT

[14] [14A] AUTOMATIC OR MANUAL CALIBRATION - An example is to enable stimulus and/or ER challenge or tasks to be adjusted in order to optimise reliability and accuracy of response measures such as detailed above under block diagram [8A], [8B] and [8C] descriptions. The present example calibration can include automatic or manual calibration capabilities (i.e. automatic challenge/task request along with signal characterisation, whereby task requests can include opening eyes; closing eyes; blinking; or scanning for eye movements and blinks; scanning for baseline eye, brain, physiological signal characteristics, whilst measuring corresponding responses and related physiological signal characteristics).

### EVALUATE RESPONSES; TASKS; CHALLENGES; PHYSIOLOGICAL AND, MENTAL, NEUOLOGICAL STATES; STATE-TRANSITIONS

- the present example can incorporate adaptive test assessment capable of establishing assessment protocol automatically based of detected brain functional areas of deficiencies; also sequencing tests to isolate disconnection and connections -

The present example can include any of or any combination of stimuli and/or challenge and/or continuous tracking of neurological state and/or disorders and/or injury (i.e. axonal assessment through to PCP brain function) based on any of spontaneous-event, spontaneous-state, evoked-response, task or challenge responsiveness or tracking (motor responses, neurological spatiotemporal dynamics along with segmented processing and/or connectivity distribution (i.e. sequence, brain information flow, brain [processing distribution, brain event synchronisation, etc. See also apparent patent page 8:1 reference

### -Cognitive, Physiological, and Motor Response Assessment Analysis and Outcome reference basis of measures

- A further example is to accommodate any of or any combination of intra-session subject-specific reference data base; inter-session subject-specific reference data base; normative subject-specific reference data base (i.e. based on subject age, gender, medical background etc.); control and/or disease and/or disorder data base; self-calibrated reference (i.e. ratio or two or more dependent or independent physiological and/or neurological factors such as percentage accuracy of eye-tracking to visual target relative to degree of responsiveness to deviant response as part of an odd-ball AEP test paradigm, for example only); adaptive or self-calibrated reference where (for example only) the initial test process can establish a baseline from which the cognitive or responsiveness measures can subsequently be based, in order to avoid the need from pre-diagnostic or subject-specific base-line data from prior assessment sessions (not always available and such reliance can limit the usefulness of the present example).
- The present example further enables analysis formats to be applied to the same data sets in order to extract disparate information relating to a subject's underlying neurologic signals. For example analysis capable of describing the underlying signal dynamics, including (but not limited to) non-linear dynamic analyses such as entropy, NLD, signal complexity can be applied. Additionally, more conventional linear signal dynamic analysis such as spectral analysis or time-domain frequency analysis, along with coherence or connectivity analysis can be applied in order to distil from the underlying signal specific characteristics pertaining to cognitive impairment, cognitive performance, neurologic disease and brain injury aspects. Such brain injury aspects can include events of interest biomarkers in general (EOI; such as expanded upon in glossary at the rear of this document) or analysis of event-related responses (i.e. visual or motor response or AEP or ER etc.). Further analyses include the deployment of time-series and/or pattern analyses and/or signal-morphology analyses which can expand the description of underlying neurologic signals. Moreover, a further example is enable sequence or progressive analyses changes over corresponding to a period of time and/or corresponding to graduated changes or transitions or absolute state definitions as it relates to any of or any combination of states including consciousness, cognition impairment, cognition performance, neurological disorders, responses relating to tasks or challenges, responses relating to stimulus of any type, and other mental states. Furthermore, an example is present such metal state or associated transitions in a manner whereby a schematic or series of progressive schematics (i.e. representing successive steps in time) can be produced to enable schematic overlay representation (i.e. sequences of coherence connectivity and/or Dipole connectivity) contrasting (i.e. whereby certain difference or variance from normative data sets can flag disorders or cognitive impairment of interest) or comparison (trending information can, for example provide indicative markers of neurological disease inset including indications for TBI, mTBI, Alzheimer's, Parkinson's, Dementia etc.). Additionally, the present example can analyse delayed responses in terms or AEP or ER responses and/or underlying waveform components of these responses (i.e. Na, P100, P300, MMN, etc.) in the context of cognitive impairment or consciousness state changes as further prognostic or diagnostic markers. In this way the present example can produce diagnostic and/or prognostic maps, characteristics signal "finger-prints", and/or biomarkers.

### [17] Endogenous spontaneous event-related or biomarker-related response detection/ derivation

### [18] Exogenous event-related Spontaneous response or biomarker-related detection/ derivation

### [19] EEG activation determination Spontaneous response or biomarker-related detection/ derivation /EP/AEP/ER Monitoring

For example of spontaneous response measures see elsewhere in this document per heading **"NLDBVT PROCESS EXAMPLES "**

For example of SPONTANEOUS OR EVOKED EVENTS OF INTEREST DETERMINATION see elsewhere in this document per heading "EXAMPLE OF AUTOMATIC ONLINE NLDB ANALYSIS AND SPONTANEOUS OR EVOKED EVENTS OF INTEREST DETERMINATION WITH SYNCHRONISED SLEEP/WAKE HYPNOGRAM AND SINGLE OR CLUSTER ANALYSIS OR ENSEMBLES OF EVENTS OF INTEREST INDICATIONS AND/OR BIOFEEDBACK TREATEMENT CAPABILITIES"

### [20] Physiological response determination (exogenous and/or endogenous) EEG activation

### [21] Subject-specific and/or normative comparative prognostic or diagnostic data references.

- The present example can incorporate data references for comparative assessment of current test outcomes in terms of cognitive performance, cognitive deficit, anatomic brain injury (i.e. the present example can categorise connectivity or dis-connectivity (i.e. based on comparison to previously monitored data, based on prior inter-session monitoring data, based in intra-session monitoring data, based on normative population data, based on disease data base or neural disorder data base, for example);
The present example can monitor and **compute brain/CNS status** relating to **periods or segments** of time corresponding to test sequence(s) (i.e. **oddball** and/or **MMN techniques** including one or more deviants interspersed with one or more deviant stimulus or challenges) including any of or any combinations of:
- **periodic** (i.e. based on grouping or averaging in any manner including averaging or statistical analysis approaches such as permutation and/or combinations combined with randomised or non-randomised sampling methods;
- **progressive** changes in accordance to computations based on information relating to the sequence or order of task/challenge or stimuli in a manner whereby the progressive sequence of brain connections associated with the progressive sequence of task/challenge and/or stimulus information can be determined as well as the overall combined or averaged changes relating to any series of task/challenge or stimulus target or non-target events;
- a further example is top allow trending for short to longer term data views, along with notifications or information distribution where certain thresholds or working ranges are out of safe operating range or acceptable limits;

### MULT-DIMENTIONAL BRAIN ANALYSIS CAN INCORPORATE MEASURES OF CONSCIOUSNESS STATES AND LEVELS, ALONG WITH ASSOCAITED TRANSITIONS, INCLUDING (FOR EXAMPLE) A BATTERY OF MEASURES CORRESPONDING TO AUDITORY EVOKED RESPONES (I.E. ODDBALL AND/OR MMN TEST PROTOCOLS CAPABLE OF ASSESSING ATTENTION OR AWARENESS), AEP (I.E. CONTINUOUS AUDITORY CLICK MEASURES), AND/OR INTERSPERSED OR TESTED SEPARARATELY WITH ANY OF OR ANY COMBINATION(S) OF ER MIND COGNITIVE ASSESSEMNT TESTS (PER GLOSSARY EXPANDED DETAIL COVERED ELSEWHERE IN THIS DOCUMENT).

[22] The present example provides the capability to monitor subject/patient and determine any of or any combination(s) of:
- **Cross-functional evoked response association** - whilst conventional systems typically assess a so-called spontaneous states, or specific stimuli responses, or states of measures of awareness or attention based on the relationship between a single stimulus format such as an auditory oddball stimulus test protocol, the present example provides a means of **assessing a plurality of physiological and/or cognitive responses to a plurality of stimulus and/or subject challenge or task events.** In this manner the present example can increase the probability that responses are directly related to measures of cognition or brain injury versus chance or co-incidental measures or confounding factors.

For example, a subject could be presented with a visual task (i.e. to touch computer pad display at the location and soon as possible after the appearance of orange squares. In this way the orange squares could be presented amongst different coloured and shaped objects at extreme locations of the screen. The degree of deciphering or tacking the orange squares can be increased in a graduated manner by way of introducing distractors or other non-target objects until a selection error rate of say 50% or more (equivalent to chance such as when one flips a coin) and such measures can then be compared to normative data or patient specific pre-calibration or base-line diagnostics data in order to provide an assessment of an individual's cognitive, status (for example only). Moreover the present example can systematically change parameters such as the speed target (i.e. orange squares) and distractor or non-target objects, in order to tease out or distil different anatomical regions of the brain in terms of cognitive function and thereby enabling the present example to zero in on anatomical as well as brain function region most in question in terms of determination of potential nerve disconnections (i.e. sheering due to concussion) or dis-connectivity, along with functional processing of the brain such as the alignment or tracking between the motor dexterity of hand and eye being able to synchronise in order to track, and activate visual targets within an acceptable or normal operable range. A further cognitive test could be to activate a switch or screen button after hearing a certain target sound against non-target or distractor sounds (using an AEP or MMN oddball test protocol. In this way the visual tracking, the motor response time of activating a switch or screen button, as well as synchronisation (i.e. the screen may display a target format so the individual's task can be to select the bull's eye or centre of the target as a measure of auditory attention, motor sychronisation between hand and eye and also various degrees of cognitive difficulty based on the type of target and related distractors presented during the test the accuracy of the system is enhanced probability factor of .

The present example, by way of combining (statistical outcome-based measures relating to the permutations and combinations of a plurality of measurement dimensions as well as conventional repetitive testing and randomisation statistical determinations, enables more subject specific determination of an individual's anatomical, brain functional, motor dexterity, and or physiological biological marker consequences applicable to the assessment associated with potential or known cognitive impairment or brain injury, the present example comprising any of or any combination(s) of combined and/or individual ***multi-dimensional*** measures such as (but not limited to):
*>* ***physiological aspects*-** i.e. pulse rate variations and/or pulse-transient time as a blood pressure surrogate measures and/or transdermal conductivity/galvanic skin resistance and/or motion tracking (such as accelerometer) and/or position tracking (such as gyrometer0 and/or location tracking (such as GSM or GPS) in response to high challenge or stressed/pressured tasks;
*>* ***motor skills aspects*** such as tracking and synchronsation or alignment between head position, eye-gaze and responsiveness, finger activation accuracy and responsiveness, hand position accuracy selection,
*>* ***neurological connectivity (i.e. EEG and*/*or AEP, and*/*****or ER signals or responses)** such* as *source localisation and coherence connectivity analysis associated with* source localisation and then coherence (i.e. ***short fibres connection lag timeltime interrelationship*** between a plurality of brain signal activations such as 0 to 8msec, along with longer ***contingent processing associations such as 8msec* to *500msec; or even refractory localised groups of neurons such* as *a subcategory of say 50 to 150ms; or even other connectivity categories such as inhibitory, ABR, fast latency, middle-latency, long-latency etc. thus enabling a multi-time mapping, sequencing (discussed elsewhere in this document) and connectivity brain assessment).*** By comparing brain region of activation with brain maps such as (but not limited to) functions and/or anatomical brain/CNS locations outlined in ***Figure 66*****, *Talairach Atlas;*** Current atlas tools; Harvard Whole Brain Atlas, MNI Template, The standard template of SPM and International Consortium for Brain Mapping; Atlas of the Developing Human Brain; All Functional including Brodmann areas, Gyri, Sulci etc., brain functional and anatomical associations can be determined, and thereby enabling connectivity and dis-connectivity determinations to be ascertained and reported upon based on normative and/or subject-specific and/or self-learning (discussed elsewhere based on expert and/or artificial systems using information knowledge versus information aggregation, rule interpretation etc. ).
*>* ***brain energy activation (i.e. EEG and*/*or AEP, and*/*or ER signals or responses)*** based on source localisation of with specific known brain regions activated on an anatomical or functional basis (similar to above mentioned neurological connectivity brain maps such as (but not limited to) functions and/or anatomical brain/CNS locations outlined in ***Figure 66******;***

In this manner the subject can then be tested with multiple different cognitive and physiological functions very clearly and quickly in order to assess the motor skills of the hands tracking the visual detection or awareness of brain and eyes, along with auditory responses (for example) to an oddball auditory sequence, in order to assess these interrelating cross-functional physiological and cognitive aspects that must be functioning within a certain acceptable range (based on pre-diagnostic or normative data, for example) in order to an individual to be assesses as acceptable in terms of risk from brain injury. Moreover, the present example can retest with a different sequence but incorporation of similar or a new combinations of physiological and/or cognitive functionality or responsiveness and/or co-ordination or interrelationship measures in order to swiftly provide a fool-proof and highly accurate assessment of TBI (beyond conventional singular approaches or approaches unable to assess multidimensional and cross-functional interrelationships).
> **Spontaneous responses** (i.e. endogenous types such as spindles, HFOs, ripples, K-complexes, vertex-waves, spikes, etc., as further detailed elsewhere in this document under heading, in section titled "Title: eLifeNLDB Non-Linear Dynamical Based (NLDB) analysis with option of automatic determination and option of online distributed processing capable of spontaneous source localisation applicable to diagnostic or prognostic determination and/or biofeedback or treatment of consumer/patient."... per section titled "Spontaneous event detection with option of source localisation and/or NLDB determination";
> **Event-related responses (i.e. such as task or challenge events as further detailed elsewhere in this document under heading, including any or any combination(s) of** Event Related Potential ERP (i.e. can include brain response measures to tasks such as ***awareness, memory and other cognitive tests or challenges*** (i.e. stroop and other tests covered elsewhere in this document under headings "Intelligence assessments", "Memory assessments", "Language assessments", "Executive function assessments", "Visuospatial assessments", "Dementia specific assessments", "Batteries of tests for the assessment of neuropsychological functions", ***"-Cognition Impairment Assessment and Determination",*** "Assessment and determination of Cognitive functional outcomes", "Assessment and evaluation the early dementia stages and/or severity of dementia", "Cognitive performance assessment", "-Rule learner (or Rule Induction) (Separate-And-Conquer method);", "-Decision Trees (Divide-And-Conquer method)-Attention and focus tracking", and/or
   "-Visual Assessment of Viewer Responsiveness to displayed information:";
   - the subject may be requested to verbalise a sentence, undertake an equation, identify numbers, identify picture or other interactive cognitive tests capable of a range of sensitive but also very broad measures capable of enabling ***behavioural and*/*or brain activation responses*** (including source localisation and/or brain responsiveness or activation versus pre-diagnostic or other data base normative reference levels), such as pre-test or resting state or other states and/or in conjunction with evoked response synchronised outcomes (MMN, odd-ball or related to endogenous brain activation relating to cognition test paradigms);
   - tests of brain responsiveness relating to applying stimulus or thinking paradigms can be designed to activate any of or any combination of neuropsychological tests including any o for any combination of⁹³:
      i) the frontal lobe activation associated with complex thought processes;
      ii) occipital lobe activation associated with visual processing;
      iii) parietal lobe activation associated with touch or pain sensory processes;
      iv) temporal lobe activation associated with auditory processing;
      v) thalamus activation associated with "consciousness switch and also" the relaying of information from the brain to appropriate regions of the brain;
      vi) Broca's areas activation associated with speech production;
      vii) Wernicke's area activation associated with understanding or comprehension related to a subject's hearing;
      viii) hippocampus region activation associated with memory processes;
      ix) amygdala associated with regulation of fear;
      x) cerebellum activation associated with motor movement and balance;
      xi) ***cognitive assessment tests (CAS)*** such as (but not limited to) the CAS Battery of tests incorporating any of or any combination of Planning Scale, Attention Scale, Simultaneous Scale and/or Successive Scale;⁹⁴
      xii) Kaufman Assessment Battery for Children;
      xiii) ***Neurophysiologial tests*** any of or any combination of categories of testing including Intelligence, Memory, Language, Executive function, Visuospatial, Dementia specific, and/or Batteries assessing multiple neuropsychological functions;⁹³
      xiv) **"memory"** function tests can include any of or any combination of (but not limited to) California Verbal Learning Test, Cambridge Prospective Memory Test (CAMPROMPT), Memory Assessment Scales (MAS), Rey Auditory Verbal Learning Test, Rivermead Behavioural Memory Test, Test of Memory and Learning (TOMAL), Wechsler Memory Scale (WMS), Test of Memory Malingering (TOMM);
      xv) **executive function tests** including any of or any combination of (but not limited to) Behavioural Assessment of Dysexecutive Syndrome (BADS), CNS Vital Signs (Brief Core Battery), (CPT), Controlled Oral Word Association Test (COWAT), d2 Test of Attention, Delis-Kaplan Executive Function System (D-KEFS), Digit Vigilance Test, Figural Fluency Test, Halstead Category Test, Hayling and Brixton tests, Kaplan Baycrest Neurocognitive Assessment (KBNA), Kaufman Short Neuropsychological Assessment, Paced Auditory Serial Addition Test (PASAT), Rey-Osterrieth Complex Figure, Ruff Figural Fluency Test, Stroop task, Test of Variables of Attention (T.O.V.A.), Tower of London Test, Trail-Making Test (TMT) or Trails A & B, Wisconsin Card Sorting Test (WCST), Symbol Digit Modalities Test, Test of Everyday Attention (TEA);
      xvi) **"visuospatial tests"** function tests can include any of or any combination of (but not limited to) Clock Test, Hooper Visual Organisation Task (VOT), Rey-Osterrieth Complex Figure; and/or other cognition assessments;
   - **the present** example **enables the determination of EOI (as further detailed in glossary section of this document) and/or including (but not limited to) stimulus events and/or ERs spindles, HFOs, ripples, K-complexes, vertex-waves, spikes;**
***>AUTOMATIC EEG HEALTH-CONDITION CHARACTERISATION & OPTIMAL PARAMETER; DETERMINATION ANALYSIS (******Figure 45******)***
   - **the present** example **enables the determination of neurological disorders of interest together with the earliest possible detection of these disorders by way of detection of the associated onset aura conditions, onset prediction, changes in physiological or neurological states or related transitions of interest, as further described in** ***Figure 45******, along with the corresponding fiqure description.***
   - the present example enables the determination of **consciousness or altered states of consciousness or related transitions (i.e. consciousness depth** as per example outlined with running fast ARX AEP (auditory click stimulus responses) averages and slower 256 sweep AEP averages ((auditory click stimulus responses) using one or combined AEP analysis types including differential AEP analysis, and/or NLDB or entropy AEP analysis (per example in **Figure 56** graph, or physiological and neurological interrelated variables or functions per structured breakdown analysis in **Figure 57****,** or example of **symptoms versus effects versus physiological variable versus physiological variable determination versus related biomarkers in** **Figure 58****, or analysis or events of interest relevant to the online monitoring capability and applicable to single events or ensembles of events or biomarkers of prognostic or diagnostic interest per** **Figure 59****);**
**> Brain/CNS changes, or brain/CNS states, or brain/CNS transitions are computed based on processes retaining information on brain/CNS changes based on conventional grouping or averaging techniques (i.e. targets and non-targets are traditionally averaged in order to compute changes between brain/CNS responses to targets versus non-targets or standard stimulus or task versus oddball or deviant response (i.e. often following 80:20 ration where by the ratio of presented standard stimuli versus the deviant stimuli is about 80/20 - or every 5^{th} stimuli (but often varies to minimise anticipation and create a more randomised measure versus anticipatory responsive measure. In particular conventional state of the art tends to mainly focus on or emphasis combining or grouping the different target or non-target or standard or deviant (i.e. oddball or MMN test regime) responses;**
**>brain/CNS changes *(while retaining appropriate signal to noise measurement* criteria (i.e. by way of ensuring signal to noise of measurement methods is always valid in terms of the signal measures being of substantially larger magnitude than the background noise or unwanted signals and distortion.**

### Determination, assessment and reporting short versus longer time and/or anatomically separated cognitive impairment or brain damage and/or delay aspects associated with brain function

- a further example is to assess ***longer distance versus short distant brain connectivity or disconnections*** such as those associated with sheering or axonal fibres as a consequence of brain injury. The present example can deploy specific ER (including MMN, oddball or visual and motor task/challenges) or AEP test regimes whereby specific test designed to test longer brain connections such as ***simultaneous visualisations implicating occipital regions of the brain coupled with mathematical calculations implicating the frontal lobe*** coupled with coherence connectivity assessments can be implemented in a manner whereby longer axon fibres sheered or broken ***axon via a brain injury incident*** would in this way show up in terms of diminished connectivity (coherence) or diminished connectivity between the frontal and rear part of the brain as well as intermediate connections between the frontal and occipital brain regions and memory functions regions such as the ***hippocampus or the thalamus*** which relays information from the body through to appropriate brain regions, and is also implicated with consciousness. An example ***is to provide a mean of segmenting examining different combinations of time delays between coherent brain signals and brain-function specific test regimes*** (i.e. ER, oddball, MMN, AEP audio visual tasks/challenges) capable of interrogating short time delays ***versus longer connectivity pathways.*** For example such means includes examining short time delays between coherent brain signal (i.e. 0 to 8ms could be set but any range can be configured) in conjunction with longer time delays between coherent brain signals (i.e. 8 to 50ms but any range can be configured). Moreover the present example can compare across any one or more combination(s) of brain signal coherence points within the brain connectivity values (i.e. coherence) associated with any plurality of coherent signal delay ranges. For example, in a first process the coherence between any combination(s) of connectivity points at a lower delay range (i.e. 0 to 8ms between brain coherent signals) can be determined, while in a second process step the coherence between any combination(s) of connectivity points at a higher delay range (i.e. 0 to 50ms between brain coherent signals) can be determined, ***and in a third process step the ratio*** between these two connectivity measures can be compared as a marker of long to short connectivity (i.e. ***after an injury where long axon fibres are typically sheered the ratio of short* to *long connectivity ratio measure could typically reduce in accordance to the severity of brain injury).***
   - another example is to examine the processing delays across the brain in the context of ER, AEP. Oddball, MMN and related processing peaks and troughs along spatiotemporal dynamic coherence across the brain (i.e. coherence analysis automatically or manually configured to identify the coherence between waveform peaks and troughs of brain responses in order to examine and describe the brain/CNS changes associated with cognitive deficit or brain injury).
   - a further example is to enable the generation of a sequence of ER challenges/tasks (i.e. such as computer pad with interactive challenges - such as commands to activate a switch or screen location in response to certain visual cues) and/or oddball or MMN ER stimulus tests whereby the sequence of tests are designed to ***test different functional aspects of the of the brain in a structured and sequenced manner*** and whereby a further option is to validate any particular test that generates substandard outcomes in order to purposely validate such outcomes. This approach provides ***an adaptable testing regime format enabling the accuracy of the assessment* to *be greatly enhanced*** in circumstances where crucial outcomes measures could otherwise potentially lead to marginally accurate or misleading outcomes. In this manner the present example not only based on multiple functional aspects, but also brain anatomical or locational regions (i.e. test can be structure to test lest versus right and vice versa as well as lateralisation, anteriorisation, frontal lobes versus occipital lobe activations, thalamus activity (consciousness switch), cortical-thalamus interrelations etc. In this manner the present example not only provides a battery of test capable of testing locational or regional interrelationships (connectivity), but also connectivity sequences, ***longer distance versus short distant brain connectivity or disconnections ,* *anatomical connectivity test* aspects** (i.e. detection of frontal to rear sheerer axial connections associated with brain injury, for example) during brain , (i.e. by examining axial or fibre connections using segmented connectivity interrelationships of less than 2 or 3ms factors such as sheered axional fibres can be detected by way of detecting the related absence of brain connectivity), different levels or coarseness of connectivity sequences (i.e. by layering analysis of spectral, coherence, signal dynamics, and/or spatiotemporal dynamics. Moreover the present example can utilise a ***battery of adaptable testing and*/*or sequence of different testing regimes is thus capable of providing a complete brain report* as *we'll* as *regional and interconnectivity or regions CNS*/*brain report along with conventional whole or overall brain*/*CNS approaches.***

### Comparative or Self-calibrated/reference Processes

- **Whereby the present** example **can reference said brain changes/CNS in accordance to any comparative or self-calibrated/references processes including any of or any combinations of:**
   - **Automatic monitoring and diagnostic or prognostic assessment algorithm wearable device determination and *minimisation* as further outlined in** **Figure 45** **bock 8** ;
      - **A further example is automatic monitoring and diagnostic or prognostic assessment algorithm accuracy refinement from maximal or superset model per** ***Figure 80*** ***block 10* to minimal or subset monitored parameters and associated analyses processes as further outlined in** ***Figure 80*** ***block 11.***
   - **A further example is automatic monitoring and diagnostic or prognostic *self-learning* (i.e. expert system per** **Figure 77** **inference engine block D or** **Figure 77** **workspace C) or artificial intelligence self-learning (i.e. as discussed elsewhere in this document under heading "Evolving and continually improving self-learning expert systems or artificial intelligence diagnostic or prognostic process") per** ***Figure 78*** ***prognostic module block 2 or*** ***Figure 78*** ***diagnostic module* block 5, or aggregation from knowledge to relevant knowledge per** **Figure 79****.**
      ➢ A further example provides **tailoring of the test protocols and assessment processes and reports in accordance to a questionnaire** completed by subject under assessment or system user on behalf of said subject, whereby said questionnaire information effecting test protocols and/or or measurements processes and/or report formats can include the previous concussion journal, diary or history, as well as prior neurological disorders, medication history and other factors that could impact the cognitive status and testing requirements of an individual under assessment;
      ➢ A further example is to enable short or longer term assessments **correlated with medication** or other therapy whereby the brain/CNS changes according to medication administration in accordance to one or more graduated or single dosage events;

### Cognitive impairment or performance determination response monitoring derivation ERP/EP/AEP/ER

[23]
- see also Cognitive Coding System (CCS) (see glossary description, elsewhere in this document).

### [24] Cognitive, Mental, Neurological and/or psychological state and/or related transition determination.

### [25] Neurological disease/disorder or related transition determination

### [27] "MIND" STATISTICAL OUTCOMES ASSESSMENT USING STANDARDISED BATTERY OF TESTS (SEE AND ADAPT THESIS DB CONCLUSION OF THESIS IN TERMS OF STANDARDISED TESTING

- see also per rear of document glossary: "Measurement statistical and analyses outcomes **(*MSO*)".**

### Standardised Measurement Battery for Cognition/TBI Assessment³³

>The present example incorporates a structured and hierarchical AEP measurement capability, enabling the ***discriminating response origins, classification (types of responses). along with associated characterisation in accordance to* aspects** such as ***sensory type, cognitive responses, ABRs, PAMR, habituation, refraction*** and ***afferent*** measures, together with ***MMN*** and other ***PCP-related measures,*** applicable to distinguishing relevant cognition/TBI measures.
>A further example is to incorporate a ***battery of steady state or graduated cognition change measures*** as well as transient or spontaneous neurological or physiological events along with transient cognition state change measures and/or performance measures, including (for example but not limited) measures of 2-sample t-test separation based on spanning the loss of consciousness or changes in brain/CNS states, transitions and/or performance measures, fast-slope brain/CNS states, transitions and/or performance measures, transitional time (TT) brain/CNS states, transitions and/or or performance measures, along with other measures such as average SD consciousness-state separation measures, classification and receiver operating characteristic (AROC) measures, a battery of PK correlation measures, event and cognition and/or consciousness state detection measures, quantal response curve assessments incorporating linear regression statistical analysis, univariate, multivariate and binary logistic statistics can be calculated as a means to describe associations between consciousness states and **EEG** or AEP/ER cognition measures.

The present example can incorporate any of or any combination(s) of fast-slope cognition or consciousness-state t-test TT separation measurement combined with absolute TT detection to effectively describe both the dynamical fluctuations associated with cognitions status, and as an indicator of responsiveness. ³³

In order to assess responsiveness to challenges or stimulus the present example can incorporate any combinations of contemporary (entropy) and classical (differentiation; integration) AEP analysis types in order to evaluate changes in brain/CNS at any time or specific to any assessment tests (such as any of or any combination of ER MIND tests as outlined in glossary).

### Hierarchical Brain Characterisation³³

>The present example Based on the ABR, MLAEP, SLAEP findings the possibility may exist to monitor a more comprehensive ensemble of response derivations arranged in a hierarchical manner from myogenic PAMR measures of peripheral activity, , sensory ABR changes representative of the cochlear receptors, neurogenic afferent and refractory amplitude/latency characteristics corresponding to stimulus changes, and ultimately to processing contingent potentials (such as MMN) representative of memory trace and decision processing, implicated during intraoperative recall.
*>The present* example *enables evoked potential hierarchical stimulus generation* to *enable simultaneous tracking of AEP responses during servo-controlled stimulus optimisation. Stimulus optimisation can incorporate adjustments of rate, intensity, standard*/*deviant ratio, stimuli shape, and stimulus spectral parameters.* In this manner the present example enables the generation of different stimulus formats based on the specific cognition status or associated response measures. Stimulus formats can include standard/deviant stimuli sequences, click, warble, chirp, tone, speech, and specialised audio sequences.

The evoked response test protocols can *range from evoked early latency responses (such* as *PAMR)* as *markers of peripheral activity, ABR signal quality and stimulus connection status, or cognition-specific sequences comprising of standard*/*deviant MMN test paradigms, enabling the determination of cognition impairment, cognition performance and*/*or related brain awareness or attention.*
>The *AEP hierarchical analysis* the disassembly of composite ER signals into separate channels of information relevant to cognition impairment, performance or associated brain injury, attention or awareness. The hierarchical analysis capabilities include the ability to classify and characterise *responses including ABR obligatory sensory responses, distinguish evoked peripheral measures (PAMR), and to capture higher level AEP processing contingent potential (PCP) measures, by way (for example) of delineating between N1-effect and those of higher brain MMN brain functional changes* which are implicated during long term intraoperative memory consolidation.

The present example can incorporate any of or any combination(s) of measures capable of ***delineating AEP, ER or SR responses*** according to the physiological origins and consist of the receptor potentials originating from the cochlear, neurogenic potentials originating from the acoustic nerve or neural populations within the CNS, and the myogenic potentials generated from the activation of neck and head muscles (including PAMR). i.e. a means of determining PAMR signals and/or associated muscle tonicity or responsiveness is to use active PAMR measures whereby the present example can purposely stimulate the PAMR using loud sounds and then gauge from the PAMR responsiveness a co-efficient or sensitivity factors which can be used in conjunction with subsequent AEP or ER measures to compensate for the actual stimulus impact during tests - in this way for example a test that is distracted by surrounding sounds such as during game day as part of on-field sports testing could benefit from a measure of ***ER, AEP or SR*** disruptive or distractions from background crowd or field noises, versus mainly or solely cognitive impairment factors.

Additionally, the present example can incorporate any of or any combination(s) of measures capable of delineating categories of auditory responses according to latency and comprise of the ***first, ABR, MLAEP, SLAEP*** and the ***LLAEP responses.*** These responses represent sensory or neural sources ranging from fast ***obligatory*** nerve and other ***sensory functions,*** to latent processing contingent potentials. The first cochlear and eighth nerve (0 to 5 ms) and ***fast brainstem* (*ABR***;2-20 ms) used to measure auditory pathway integrity indicative of the cochlear sensory functions, later responses such as the ***MLAEP*** (10-100 ms), ***SLAEP*** (50-300 ms) and ***LLAEP*** (150-1000 ms) responses are implicated in refractory and higher processing contingent potential, respectively ³³.

Another example is to deploy means (such as ***waveform analysis*** or latency-bin analysis) in order to tease out or delineate ER and/or AEP processing responses in accordance to determination of different groups or levels or brain/CNS connectivity or processing including (but not limited to) groups of obligatory responses, afferent, and contingent processing AEP responses and/or cognitively sensitive measures. By sorting out or categorising sensory versus axonial versus and cognitive responses apparent with by way of classifying ABRs, PAMR, habituation, refraction and afferent measures, together with MMN and other PCP-related measures, the present example can distinguish various layers of communication and/or processing within the brain and hence assess the changes in such measures associated with brain injury, medication, or cognitive performance and impairment. These said different layers can be deployed as part of the brain "sequencing" and "brain activation" determination processes covered elsewhere in this document.
>The present example enables the monitoring of the ***SLAEP group of responses*** (generated within the ***frontal cortex and associated areas),*** and these can be characterised and classified as ***P1, N1, P2, N2 and other SLAEP response classifications.*** The present example can produce SLAEP appropriate waveforms with as few as 30 to 50 stimulus presentations¹⁵¹. The present example can characterise all three N1 sub-components in response to a rapid change in stimulus level, from an otherwise quiescent condition as a marker of an individual's awareness or alertness and overall cognition impairment or performance ^{152, 153}.
>The present example can generate a stimulus onset from a quiet background (i.e. use of acoustic noise cancellation techniques) or generate a change in continuous stimulus tone in order to evoke an N1 response waveform from subject under test. i.e. ***least one of the N1 sub-components may be generated in response* to *attention triggering comprising of* a *process representing the initial readout relating to information from* sensory analysers** ^{153, 154}.
>The present example can track N1 responses as a marker of **a *form of memory trace,*** and the elicitation of this response and subsequent waveform subtraction can enable the measurements, corresponding to cortical processes involved in the determination of decision processes of a subject as it related to recognising stimulus changes;
>The present example can monitor and detect the presence of ***N1, corresponding to a stimulus presentation,*** providing ***physiological evidence of stimulus arrival at the auditory* cortex.** In this way ***"N1" measure can reflect the presence of an audible stimulus,*** and provide markers of ***auditory discrimination*** ^{153, 155, 156}. The present example can also deploy these measures as markers of the presence of stimulus in order to provide an online validation function (i.e. minimise confounding measurement factors such as background noise or unrelated spontaneous responses).
>The present example can track ***N1 changes*** can as markers for both: 1) ***refractoriness,*** or sensory change as shown by the ***Butler effect*** ¹⁵⁷, and 2) ***processing contingent potential or discriminatory stimulus change measures*** ^{153, 158, 159}. Butler effect can be measured in order to compensate confounding measures associated with repetitive stimulus whereby the ***N1 amplitude substantially recovers or re-emerges after* a *change in frequency or other stimulus parameter*** ^{160, 161}. i.e. an ***increase on the refractory period (inter-stimulus interval; ISI) can produce an increase in N1-P2 amplitudes.*** This effect has been referred to as ***"N1 effect" or "Butler effect"*** should ***not be mistaken for MMN,*** which have similar latency ranges but in contrast to the Butler effect ***correspond to processing contingent potential.*** The present example delineates of N1-effect (Butler) versus MMN **(*MMN can reflect PCP measures)*** as a means of distinguishing MMN awareness measures in the presence N1-effect responses (N1-effect relates to neural afferent changes not necessarily reflective of higher order cognition or awareness states). i.e. an increase in the ***inter-stimulus interval period*** or the so called refractory period has been shown to produce an increase in the N1-P2 amplitudes due to what is believed to be the recovery ***effect of the underlying neurons responsible for generating these responses.***
>The present example automatically tracks and compensates assessment measures of refractoriness and subsequent AEP response amplitude associated with changes in stimulus ***frequency*** ^{153, 162}, ***intensity*** ¹⁵³ ***and other stimulus parameters.*** Conversely, the present example tracks and compensates for N1 amplitudes that been shown to decrease due to greater ***refractoriness evident with repetitious stimuli*** ^{163, 164}. i.e. the ***second stimuli presentation has been attributed to the most significant response amplitude decrease, following a change in stimulus.***

### [28] "MIND" SUBJECT-SPECIFIC /PERSONALISED STATISTICAL PERFORMANCE ASSESSMENT (SEE ALSO ELSWHERE IN PATENT PER MINIMISATION ASSESSMENT PROCESS AND SELF-LEARNING (ARITICIAL INTELLIGENCE/EXPERT SYSTEM PERFORMANCE AND INFORMATION AGGREGATION PROCESSES)

The present example enables the system user to activate "automatic test assessment format, or manually select a specific test previously configured" or the ability to configure a test whereby at least one test or challenge associated with test or stimulus is linked and synchronised in time with location (i.e. the responses of the subject under test or more specifically the spatiotemporal brain dynamics corresponding to a stimulus, challenge or task test in a manner where the specific brain functional aspects being challenged by a test are noted and the brain source localisation or imaged area of the brain associated with such brain-functional responses are noted (i.e. as a region or point or series or points or regions of the brain, at a certain instance in time or instances in time) in order to enable the associated events to be investigated in the context of spatial locations with the brain as well as temporal relationship with the originating task, challenge or stimulus tests.

The dataset representing the acquisition of brain monitored or imaged neurological signals can be investigated in any or any combinations of: 1) individual event data set characterisation, 2) characterisation of a series of **"events",** 3) **"comparison** between a plurality of events";

Whereby said **"events"** refers to any response to stimulus, challenge or tasks (i.e. per Example (but not limited to) cognitive test include those listed in glossary at rear document under ***"ERP MIND" ).***

Whereby said **"comparison** between a plurality of events" includes any of or any combination of: 1) **any two or more events** or any combination of monitored events such as a **deviant or oddball response** (i.e. novel response resulting from a deviation from a standard or more routine stimulus or task challenge) **compared to a standard response** (i.e. the more repetitive or routine stimulus or challenge), whereby said events can be combined (i.e. any number of standard events can be combined and any number of deviant or oddball responses combined (i.e. combining includes different types or regression analysis or averaging, for example only) regression ; 2) **any 2 or more monitored events** or any combination of monitored events such as monitored from **signal channels** or electrode signals **(before source localisation,** for example, but not limited to); 3) **any 2 or more events** based on data sets based that been transformed based on source localisation analysis (i.e. **after source localisation,** for example, but not limited to); 4) the prior listed case 1) to 4) **incorporating at least one instance of base-line data** (i.e. base-line data can include any of or any combination of data-base references including: ***a) normative*** and/or control and/or disease and/or subject-specific pre-diagnostic data bases or data sets without stratification of samples according to subject/patient (i.e. gender, age, medical history, etc.); ***b*) *self-calibrated-data*** whereby the first part of data from a test session can be deployed as a basis of calibration for cognitive assessment outcomes; ***c***) any ratio or ***comparison between*** cognitive outcomes can be established as part of the testing to enable a **self-calibration** (i.e. a marker of ***relativity between* 2 *measures;*** i.e. **eye response tracking measures** (i.e. via in-build or separate camera tracking with associated eye measures as covered elsewhere in this document under section titled "eLifeALERT" and covering measures such as (but not limited to) eye, facia, body and head movements or positioning, location or direction and/or span of gaze, along with eyelid speed or velocity characteristics, eye-opening or eye-closing span, and other measures applicable to defining eye, eye-lid, head, face or body location or movements corresponding to stimulus, challenges, tasks or other driver or occupational behavioural and cognitive aspects.

In this way a plurality of cognitive and/or other physiological responses or measures can be compared in order to produce a third measure, being the outcomes measure based on a comparative or ratio value, thereby producing an index relevant to cognitive outcomes or other physiological outcomes measures, **not dependent on absolute or calibrated measures.**

Whereby said automatic or manual selection of a specific test previously configured test protocol is enabled. Test assessment criteria such as accuracy, speed of test, complexity of the test and other test requirements can be automatically computed based on the users selected criteria. In this way unknown parameters can automatically be adjusted in accordance to the user-selected criteria. i.e. if the highest degree of accuracy is required the multi-dimensional test format will be suggested and the data sets of cognitive assessments can be structured to interrogate the brains many functions and location across the brain in order to assess for both anatomical (i.e. ***fibre sheering after concussion, between frontal and rear of brain or between left and right hemispheres*** (for example only) can be tested by utilising cognitive tests incorporating mental challenges covering these brain functional regions. By configuring the present example to incorporate such tests, followed by source localisation, and then coherence or Dipole analysis, the present example can compare a subject's responsiveness to challenges (i.e. audio-visual display with touch screen interface challenges such as video game challenges) - if the coherence or connectivity across certain memory or alertness tests appear to be degraded then the present example can further focus on such areas or functional shortfalls of the brain and validate or cross-check such measures a number of times to increase the overall sensitivity of cognitive measures using this ***subject-specific adaptive process.***

**[29] Cognitive test (i.e. challenge, task and/or stimulus) based on a plurality of brain locations or regions of interest in conjunction with option of total brain assessment.** I.e. assessment test or challenge and/or stimulus protocol can be configured in order to activate specific brain functions and interrogate corresponding responses in accordance to brain locations or regions which are impacted or normally impacted by such tasks or challenges and/or stimulus protocols. In this manner the sensitivity and specificity of brain connectivity, coherence, Dipole, connectivity processing sequences, processing networks, process mapping, interconnectivity network mapping, connectivity schematics (i.e. as further outlined in glossary at rear of this document along with associated defences; Cognitive Processing Sequencer **(*BPS*);** Cognitive Coding System **(*CCS*);** Hierarchical Neurologic Processing (***HNP***)*;* Dipole Modelling, coherence analysis, coherence connectivity analysis) and other brain functional aspects can be classified in terms of specific connectivity or dis-connectivity in the case of brain injury (i.e. where brain connection fibres or axons can otherwise be disconnected and more effectively measured and diagnosed using any of or any combination of "total brain", "integrated brain", "interconnectivity brain", "sequenced brain", logic-flow brain", and/or "distributed processing brain" investigational approaches, applicable to prognostic or diagnostic (i.e. EOI including neurological disorders, injury, concussion, or disorders), cognitive (impairment or performance). In contrast, conventional single dimensional or specific connectivity versus a complex combinational approach risk oversimplifying or generalising the sophistication apparent with brain function along with associated effective measures.
- In one example the coherence arc activity ***Figure 101*** **(top-right panel)** can be computed based on the convergence of CDR **fEEG** and standardised MRI image data, (in this example computed during **SLEEP** (stage 2) period), in order highlight a preponderance of neural interconnectivity between the **thalamus and occipital cerebral cortex regions.**
- In one example the coherence arc activity can be computed based on the convergence of CDR **fEEG** data and a standardised MRI image data **(*****Figure 101******, centre-left panel*)*,*** (in this case computed during **WAKE period),** in order highlight a preponderance of neural interconnectivity between the **thalamus and frontal cerebral cortex regions¹⁶⁵.**
- In one example the **Dipole locations** ***Figure 101*** **(top-left panel)** can be computed based on the high density (133 electrode) function **EEG** recording (in this example converged with a standardised and averaged MRI image) whereby wake or sleep stages can be presented in terms of current density reconstruction **(CDR)** brain activation outcomes ***Figure 101*** **(top-right panel).**
- In one example the **Dipole computations can be computed, such as the here where the** computed Dipoles appear to encompass the **Thalamus region (*****Figure 101*** **(top-left panel).** In this example the most substantial Dipole **(*****Figure 101*** right-hand top Dipole) also appears to be centralised according to the Thalamus region, which concurs with the literature associating Thalamus activation with deeper stages of sleep. In this example the left-lateral image view emphasises the central and occipital brain activation, typical during sleep ***Figure 101*** **(*centre-left panel*)*.***
- In one example Dipoles can be computed along with ARC output table which in this example presents 3 Dipoles, with 1 located in the Anterior region and 2 in the Anterior-posterior/Superior-Inferior (upper rear brain) region ***Figure 101*** ***(lower panel).***
- As demonstrated in this example, the brain top-view topographic MAPS is shown in ***Figure 101*** ***(centre-right panel*)*,*** with a predominance of the 41 msec (TP41) AEOP component, located around temporal region is highlighted as a potential contributing factors as it relates to the emphasis of frontal region brain activation (i.e. anterior localisation of the 3 Dipoles per ***Figure 101*** **(top-left panel).**
- **The present** example **can source localise brain activation, Dipoles and coherence,** based on automatic (or manual adjust) and self-calibration techniques (i.e. previewing data using ICA and/or PCA techniques to identify main waveform or signal components of neurological interest) can determine the brain activation of interest in accordance to brain activity responses to corresponding to task, challenges or stimuli, as well as the option of correlation or associating such brain activation with eye-lid, eye-movement, eye-gaze, eye-lid acceleration and/or velocity characteristics, head motion, facial or head gaze and other eye or alert measures outlined in section titled "eLifeALERT" as part of this this patent description document.
- Specific to this case study, ***Figure 101*** greater definition of the source dimensionality using two stage approaches such as PCA followed by ICA can be deployed as part of the automatic (or optionally manual) pre-processing data phases in order to improve the Dipole source localisation accuracy and associated connectivity measures. For example, given that there can typically be a large number of surface signals being mapped into so few Dipole outcomes, the present example can automatically pre-process (prior to determination of brain source reconstruction, coherence, and/or Dipole analysis) data by applying multi-stage PCA to ICA approaches to minimise dimensionality errors. In this manner the present example can automatically apply PCA
- For example, PCA can be applied in the first place to identify signals with higher SNR, followed by ICA analysis to improve the Dipole localisation (dimensionality)¹⁶⁵

Another example versus conventional less-dimensional approaches (i.e. whole brain or general indices) is that while diagnosis or assessment of the brain may be possible under some conditions using only sensor-related signals (versus source reconstructed data) is the ability to analyse specific source-oriented brain activation which can otherwise be overwhelmed by surface signals. For example, some of the more critical brain structures such as the thalamus (also referred to as the "consciousness" switch) which can provide deeper cortical information relating to a subject's consciousness state are not as evident or at all evident when considered from the context of the "whole overall-brain" (averaged or broad-based analysis) approach, versus the capability of the present example which can consider the whole brain in terms of any structural or functional brain regions, but at the same time can localise special regions of bran activity not evident without source localised (even if coarsely source localised) analysis.

In this manner the **present** example **can propagate a series of brain tests, each interrogating the brain in a specific manner** so that it is possible to check connectivity across specific regions of the brain. For example, the present example can deploy a series of interactive steps (i.e. stimulus, tasks, challenges) whereby each step logged in terms of outcomes so that no single or small group of tests that can then be analysed by way of source localised brain activity tracking, using the knowledge that each test can be designed to alleviate or suppress certain structural and functional regions or aspects of the brain. Therefore, the present example incorporates a mean of whole-brain measurement, combined with the assessment of localised functional or structural regions of the brain.

This approach helps **prevent any course or generalised whole-brain or overall-brain assessment approaches missing or overwhelming deeper cortical or more subtle measures,** as missing such information can jeopardise determination of subtle but critical brain function or brain injury aspects crucial in the context of a comprehensive assessment of a subject's cognition and brain function states applicable to brain injury or neurological disorders.

An example of the monitoring and signal analysis processes, including brain source reconstruction analysis, inclusion or exclusion of certain brain segments as part of source reconstruction analysis, examples or current density and sLORETA source reconstruction analysis, along with coherence processing, Dipole modelling and determination of coherence connectivity arcs is presented in this document with ***Figure 84 to Figure 94******,*** along with the respective figure descriptions.

**[30] Decomposition of brain schematic based on temporal segmentation from anatomical fibre/axial or sensor sensory/nerve activation through to higher order processing associations with distributed or embedded processing determinations, applicable to ER, SR, cognitive impairment or performance, brain injury and associated, connectivity or dis-connectivity along with related networks, as well as brain states, neurological disorders or disease and associated transitions or graduated measures.**

***Figure 101*** **Example of the Objective Evaluation of Neurocognitive Status underlying analysis from which brain connectivity sequencing can be determined.**

**An example of Dipole locations (top left panel)** computed from this high density (133 electrode) function **EEG** recording converged with a standardised and averaged MRI image sleep corresponds current density reconstruction **(CDR)** outcomes **(top right panel)** brain activation, typical during sleep (2^{nd} **down left panel);** brain top-view topographic MAPS **(2^{nd} down right panel);** 3 Dipoles per **top left panel;** Dipole computations and ARC output table **(lower panel).**

**[33]** ; **[34] COGNITIVE PROCESSING SEQUENCER (CPS) AND COGNITIVE CODING SYSTEM (CCS)** - **see also glossary.** Top-left presents butterfly plot overlay of successive AEP waveforms corresponding of the 3 minute period representative of drowsy state (based on the online (or offline) simultaneous sleep/wake and/or consciousness state and/or altered consciousness state determination) combined with cognition impairment and/or performance assessment determinations.

**The overlayed blocks** represent the **connectivity sequence determination,** whereby successive points in time or periods or time can be examined visually, statistically, analytically, visually, graphically or numerically in order to describe the progressive sequence of changes applicable in this simulated data example (based on real experimental physiological recordings and analyses) on the top layer block showing the first process step being the start of the brain connectivity time sequence. **This first process step** example presents the current density reconstruction (CDR) view in **(****Figure 85** top right panel), applicable to the first 3 minute period with the corresponding functional AEP **EEG** waveforms overlayed as a butterfly **(****Figure 85** top-left panel), the current density source reconstruction with computation of coherence ARCS overlaid **(****Figure 85** right panel), along with corresponding table of coherence ARC outputs **(****Figure 85** lower panel, and the independent component analysis (ICA with topographic overlay maps presented in **Figure 85** lower left panel).

The **subsequent overlay boxes each represent a successive time period** (3 minute I this example) along with the associated psychological states (i.e. sleep, wake, consciousness or unconsciousness, consciousness level etc.). The sequence taken as a whole can then be represented in a **type of brain schematic overlay visually showing the progressive changes and sequence or flow of brain connected data as a function of time** (i.e. multiple layers and colour coding or line annotations can be deployed in order to denote different coherence delays representative of fibre interconnections such as 0-2 ms, through to Auditory brainstem response (ABR) and middle latency auditory evoked potential (MLAEP), slow latency AEP (SLAEP), or long latency auditory evoked potential (LLAEP)), in order to provide the brain connectivity sequencing capabilities.

A further example enables such sequences of brain connectivity to be determined based on **segmentation of brain connectivity time-windows** with time referencing or synchronisation with stimulus (or task, event or challenge as the case may be with visual or audio visual test protocols) onset (i.e. a) 0 - 3 msec to analyse coherence or other forms of connectivity associated with axon fibres that electrical brain signal travel down towards synaptic gap, as well as analysing signals that are transmitted across the synaptic gap to the next neuron via dendrite fibres that receive these signals from other neurons; thus the present example can examine connectivity and dis-connectivity associated with the sheering of such fibres in the case of micro-traumatic brain injury or more serious concussions events b) the first cochlear and eighth nerve (0 to 5 ms), c) fast brainstem (ABR;2-20 m) used to measure auditory pathway integrity indicative of the cochlear sensory functions, d) later responses such as the MLAEP (10-100 ms), e) SLAEP (50-300 ms), f) LLAEP (150-1000 ms) responses are implicated in refractory and higher processing contingent potential, respectively, g) afferent responses, h) N1-effect, i) special response measures such as P1/P50 midlatency AEP and the startle response (SR) which the present example can deploy as a measure of cognitive awareness of cognitive sensitivity, in addition to pre-pulse inhibition measures such as N40 potential; schizophrenia and other neurological disorder deficits in auditory P1 and N1 refractoriness etc.;
The present example incorporates a means of coding brain sequences in a manner whereby specific tasks, thought processes, stimulus responses, challenge or task responses or any spontaneous, event-related, exogenous or endogenous sequences (parallel and/or serial and/or simultaneous and/or successive) can be represented by a standardised coding system, thereby enabling normal or dysfunctional brain processes to be assessed and compared on an inter-subject and instar-subject study (monitoring session) basis.
In one example a course-brain sequence could be codes corresponding to 300ms stroop awareness ER protocol response to target comprising of Left-hemisphere Frontal lobe (10-20ms; 7 interconnecting coherence links/ARCS) to (central brain region (90-110ms; 20 ARCS) and Occipital-left lobe (130-150ms; 10 ARCS)) to frontal lobe to Parietal-left (150-170ms; 30 ARCS); This brain code sequencer of the present example could then be represented as a code sequence comprising of LF (10-20ms; 7 ARCS) to CB (90-110ms; 20 ARCS) & OL (130-150ms; 10 ARCS)) to frontal left-hemisphere lobe (250-350ms; 100 ARCS) to Parietal-left (350-400 ms; 50 ARCS). Moreover, the present example can compare this sequence with an acceptable ARC and coherence delay range in order to provide a degree of acceptable variation considered to equivalent in terms of the degree of cognitive performance or impairment. Cognitive brain code sequence comparisons can be based on changes of an individual under assessment with the course of time. Additionally, cognitive brain code sequence comparisons can be based on normative data references or pre-diagnostic data of subject under test.

Fine-brain sequence could be codes corresponding to 300ms stroop awareness ER protocol response could include more specific structural (anatomical ) brain regions such as hippocampus or thalamus or suprachiasmatic nucleus **(circadian** clock) regions (for example only) or any brain atlas or mapped regions.

In this manner the present example can generate events or stimulus for a range of tests (i.e. MIND ER per this document's glossary) in a manner whereby stimulus can be presented to evaluate regional brain connections, more distance brain connections and any brain connections across the whole brain in order to assess (for example) the degree and classification of brain injuries such as sheering of dendrite or axon fibres following concussion, TBI or micro-TBI. In the example here successive 3 minute periods of successive stimulus averages, followed by current density reconstruction analysis, followed by coherence analysis and then numeric representation of coherence ARCS **(****Figure 85** lower panel) CDR have been computed for 7 successive 3 minute periods, across a 21 minute monitoring session (i.e. n=3 minutes (m)).

Whilst this example is suited to tracking connectivity sequence across changes in wake to drowsy to sleep and various sleep stages, other shorter sequences capable of deciphering brain code sequences applicable to a single event is also possible. For example, the butterfly plot in **Figure 85** top left panel can be analysed in terms of the CDR, coherence and output ARCS for each consecutive 10ms (for example) in order to examine the brain connectivity and coding sequences applicable to the pre-stimulus to end of

In this example the 3 minute averaged period (such as 10 millisecond whereby the 10 seconds s minute successive periods) can be deployed across the averaged AEP waveform in order to examine the brain connectivity and coding sequence applicable to a single event (i.e. -10ms pre-stimulus t 120 ms post-stimulus in this example).

**As an example the following represents Lobes & other special brain regions of the brain Neurological-Schematic overlay examples;**

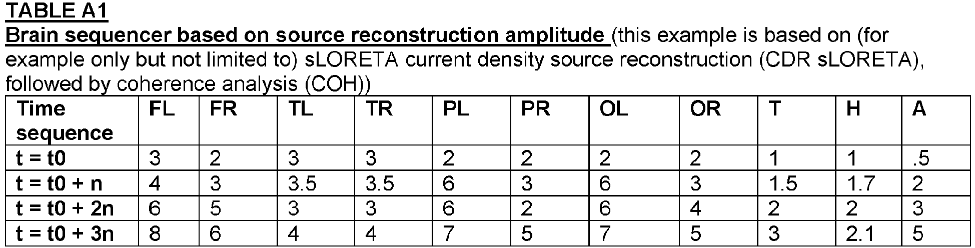

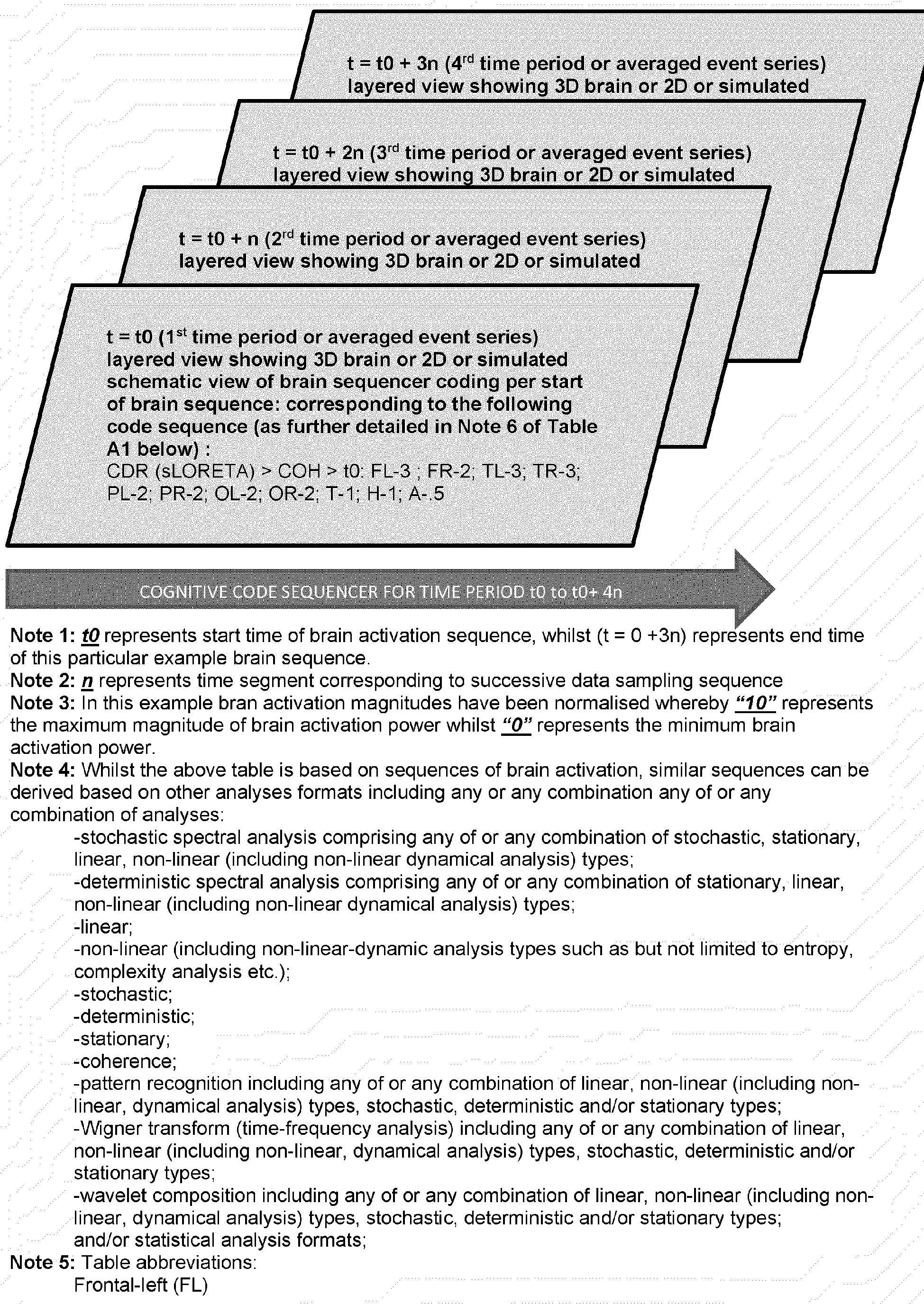

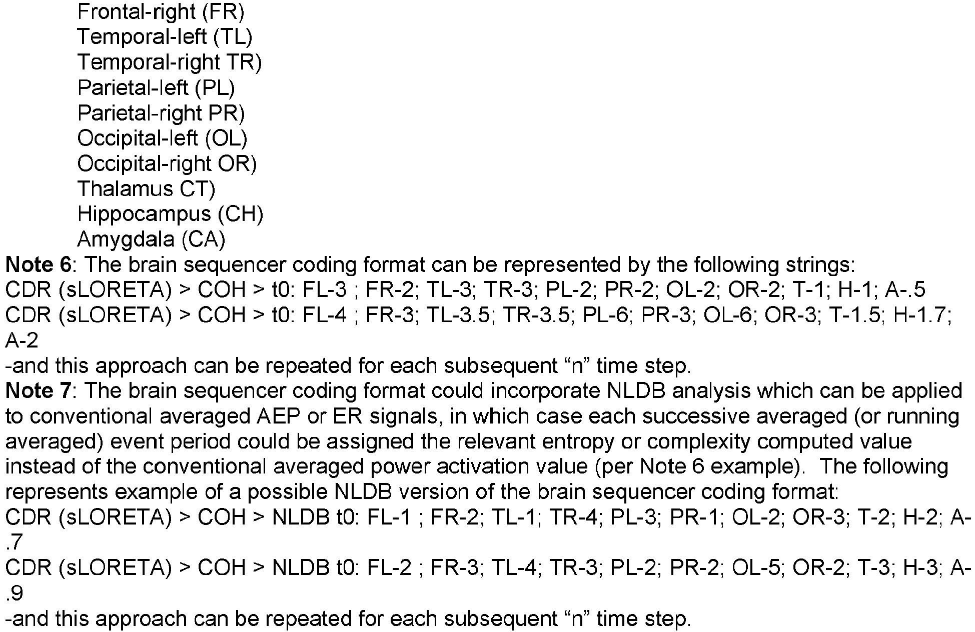

**[35] Neurologic Simulation modelling (NSM) of Cognitive Processing Sequencer (CPS) and/or Hierarchical Neurologic Processing (HNP) process and/or Cognitive Coding System (CCS) (see glossary description, elsewhere in this document).**

**THE PRESENT** EXAMPLE **INCOPORATES VISUALISATION SR; RS; NLDB; CPS; CCS;** (also referred to Cognitive Processing Sequencer (CPS; see also per glossary at rear of this document) -whereby any combination of **stimulus, challenge and/or challenge** (including but not limited to any of or any combination of examples per glossary MIND ER listing presented elsewhere in this document) can be assessed in terms of corresponding responses, in terms of the response times, interrelationship between any of the responses and the assessment of dexterity and/or responsiveness and/or tracking between any of the associated responses (I.e. Responses to any of or any combination of visual responses such as eye location, gaze location, eye-tracking, head tracking or interrelationship tracking including motor responses such as finger switch or touch screen or gestures; motor skills or responses such as hand, body, head, or limb movements (including those detected via non-contact or contact sensor systems).

In other words any time period or other **association between any responses and the originating event or stimulus** can be assessed for similar events or response types as well as disparate events or response types (I.e. Tracking of hand motor and dexterity skills in visually tracking and cognitively choosing the selection of a flashed or moving touch screen target). The present example can assess the correlation and interrelationship or association between a number of different neurological or physiological aspects (I.e. Multidimensional - such as tracking and measures of responsiveness of brain ER responses, motor hand movements, cognitive decision making and eye tracking according to single or related stimulus and/or tasks).

A example is to enable the **tracking and interrelationship associations between multidimensional neurological and physiological responses** to be represented in the context of graduated range of various degrees of stimulus deviation, brain sequencer process,
Neurologic Simulation modelling (NSM) of Cognitive Processing Sequencer (CPS) and/or Hierarchical Neurologic Processing (HNP) process and/or Cognitive Coding System (CCS) (see glossary description, elsewhere in this document).

**[36] ANALYSIS OPTIONS AVAILABLE AS PART OF ANY OF OR ANY COMBINATION OF AEP, ERP, AND/OR ANALYSIS FORMATS** per section titled "eLifeNeuro/Subject-Specific **EEG-**Monitoring Minimisation. Adaptation- Generic plus TBI, autism, depression/Stim feedback, epilepsy HFO; aura".
"ANALYSIS OPTIONS AVAILABLE AS PART OF ANY OF OR ANY COMBINATION OF AEP, ERP, AND/OR ANALYSIS FORMATS32:", covering any of or any combination of (but not limited to) aspects per sub-heading including AEP/ERP Data Pre-processing, Fundamental Signal Processing and Data Formatting, Primary AEP/ERP Data Transformations , Secondary AEP/ERP Data Transformations, Combinational Analysis , Statistically based joint probability measurement accuracy determination, and/or Mathematical Primary Analysis of raw AEP or AER waveforms includes any of mathematically derived and/or statistically derived outcomes.

[37] Hierarchical Neurologic Processing **(*HNP*)** (see also glossary description, elsewhere in this document).

**[38] Alertness tests (as further detailed in section titled "eLifeALERT"), ultrasound or tonometry and/or TCD tests as further outlined in section titled "eLifeDOPPLER".** Measurement statistical and analyses outcomes (***MSO***) per BOX H block 27 (see also glossary)
**Block 32** - **Monitoring algorithm self-learning or subject fine-tuning (i.e. see also expert system and/or artificial intelligence examples per figures** Figure 77, Figure 78 **and/or** Figure 79.

[31] Measurement Precision Evaluation (MPE) system (see also rear of document glossary: "MPE".

**[31]** Measurement Precision Evaluation *(MPE)* system (see also rear of document glossary: "MPE".
- MIND assessment outcomes measures/reports

### Figure 101

Figure 101 Example of the Objective Evaluation of Neurocognitive Status underlying analysis from which brain connectivity sequencing can be determined.

### TBI patent temp

**>>>In a first process,** a data set (I.e. responses corresponding to, but not limited to, target or deviant event responses) comprising of a singular or plurality of combined neurological and/or physiological responses) can be computed based on one or more data acquisition need neurological and/or physiological responses.
**>>>In a second process, a data set** can be computed based on a comparative data set (I.e. responses corresponding to, but not limited to, non-target or standard event responses) comprising of a singular or plurality of combined neurological and/or physiological responses.
**>>>in a subsequent process,** additional **data sets** (i.e. responses corresponding to, but not limited to, non-target or standard events with differing degrees of deviation, in order to evaluate a range of responses associated with a range of subtle to extreme target or deviant event responses, as a means of better understanding an individual's cognitive or neural status, performance or dysfunction).
**>>>in a further process, a series of target/deviant and/ non-target/standard events** corresponding to target/deviant and/ non-target/standard event responses (i.e. neurological/brain/CNS and/or other physiological types) can be analysed in a singular or combined (such as averaged or regression modelling) format in order to evaluate comparative physiologic and/or brain/CNS/neurological changes as a result of the respective stimuli and/or tasks and/or challenges.
**>>>in a further process, successive events** (i.e. physiologic and/or brain/CNS/neurological responses and associated changes resulting from one or more respective stimuli and/or tasks and/or challenges) can be analysed in the context of successive event representative in the context of corresponding time-series brain activation and connectivity progressions. Additionally, grouped or combined responses (i.e. averaged successive responses or averaged parallel or simultaneous response signals) and associated changes resulting from one or more respective stimuli and/or tasks and/or challenges can be analysed.
**>>>in a further process, single responses** can be analysed in the context of a series of events relating to stimuli, tasks or challenges and corresponding brain activation and/or connectivity changes.
**>>>in order to analyse the difference or characteristics of different data sets** (i.e. grouping of responses related to target stimuli versus grouping of responses related to non-target stimuli) and/or in order to characterise any specific data set of grouped (i.e. averaged or regressive modelling) responses a number of individual or combinational analyses approaches can be deployed, where by analyses types can include any of or any combination of ***Whereby cluster analysis composition and associated analysis processes*** including any of or any combination of analyses:
   - stochastic spectral analysis comprising any of or any combination of stochastic, stationary, linear, non-linear (including non-linear dynamical analysis) types;
   - deterministic spectral analysis comprising any of or any combination of stationary, linear, non-linear (including non-linear dynamical analysis) types;
   - linear;
   - non-linear (including non-linear-dynamic analysis types such as but not limited to entropy, complexity analysis etc.);
   - stochastic;
   - deterministic;
   - stationary;
   - coherence;
   - pattern recognition including any of or any combination of linear, non-linear (including non-linear, dynamical analysis) types, stochastic, deterministic and/or stationary types;
   - Wigner transform (time-frequency analysis) including any of or any combination of linear, non-linear (including non-linear, dynamical analysis) types, stochastic, deterministic and/or stationary types;
   - wavelet composition including any of or any combination of linear, non-linear (including non-linear, dynamical analysis) types, stochastic, deterministic and/or stationary types;
   - statistical analysis, and/or
   - NLDB;
>>>in order to analyse variance or difference between target (deviant) **and non-target (standard) responses** (i.e. including graduated or binary analysis methods of determining the degree of a subjects alertness of responsiveness to a stimuli) a number of analysis approaches can be applied as further outlined in this patent document including any of or any combination of ***BOX [F]*** provide analysis capabilities **applicable to EEG AND OTHER NEUROLOGIC EOI OR MENTAL STATE DETERMINATIONS.**

***BOX [G]*** provide analysis capabilities **EEG AND OTHER NEUROLOGIC EOI AND/OR PHYSIOLOGICAL EVENTS, ACTIVITY OR STATE DETERMINATIONS. Including** Non-linear dynamic based ***(NLDB)*** evoked response or continuous **EEG** processing capabilities (as further described in section titled "Non Linear Dynamical Based Analysis (NDBA) with option of spontaneous automatic online analysis", under heading "EXAMPLE OF AUTOMATIC ONLINE NLDB ANALYSIS AND SPONTANEOUS OR EVOKED EVENTS OF INTEREST DETERMINATION WITH SYNCHRONISED SLEEP/WAKE HYPNOGRAM AND SINGLE OR CLUSTER ANALYSIS OR ENSEMBLES OF EVENTS OF INTEREST INDICATIONS AND/OR BIOFEEDBACK TREATEMENT CAPABILITIES".

In ***BOX [H]*** the present example provides **SPECIAL COGNITION PROCESSING AND CONNECTIVITY PROCESSES** including any of or any combination of **Subject-specific and/or normative comparative prognostic or diagnostic data references *BOX H, block* [21]; MULT-DIMENTIONAL BRAIN ANALYSIS including any of or any combination of (but not limited to) Cross-functional evoked response association, *physiological* aspects, *motor skills* aspects, *neurological connectivity (i.e. EEG and*/*or AEP, and*/*or ER signals or responses), brain energy activation (i.e. EEG and*/*or AEP, and*/*or ER signals or responses),* Spontaneous responses, Event-related responses, *awareness, memory and other cognitive tests or challenges ,*** ***behavioural and*/*or brain activation responses, cognitive assessment tests (CAS),*** **memory function tests, executive function tests, visuospatial tests** [BOX H, block 22] **along with other tests such as those outlined in glossary MIND ER definition, computation of Brain/CNS changes, or brain/CNS states, or brain/CNS transitions, *Determination, assessment and reporting short versus longer time and*/*or anatomically separated cognitive impairment or brain damage and*/*or delay* aspects *associated with brain function.* Comparative or Self-calibrated/reference Processes [BOX H, block 22];** Cognitive Coding System **(*CCS*)** per ***BOX H, block 23*** (see also glossary); **Cognitive, Mental, Neurological and/or psychological state and/or related transition determination** per ***BOX H*, block [24]; Neurological disease/disorder or related transition determination *BOX H*, block [25]** ; **"MIND" STATISTICAL OUTCOMES ASSESSMENT USING STANDARDISED BATTERY OF TESTS (SEE AND ADAPT THESIS DB CONCLUSION OF THESIS IN TERMS OF STANDARDISED TESTING including any of or any combination of (but not limited to)** Standardised Measurement Battery for Cognition/TBI Assessment [BOX H, block 27], Measurement statistical and analyses outcomes **(*MSO*)** per ***BOX H block 27*** (see also glossary); Hierarchical Brain Characterisation **[BOX H, block 271; "MIND" SUBJECT-SPECIFIC /PERSONALISED STATISTICAL PERFORMANCE ASSESSMENT [BOX H, block 281 including any combination of (but not limited to) Cognitive test (i.e. challenge, task and/or stimulus) based on** a **plurality of brain locations or regions of interest in conjunction with option of total brain assessment [BOX H, block 29]; Cognitive test per** BOX H block **[29]; Decomposition of brain schematic block** BOX H block **[30]; Simulation** or **modelling** of Cognitive Processing Sequencer **(*CPS*)** per ***BOX* *H*, *block 35*** (see also glossary);**ANALYSIS OPTIONS AVAILABLE AS PART OF ANY OF OR ANY COMBINATION OF AEP, ERP, AND/OR ANALYSIS FORMATS** BOX H block **[36];** Hierarchical Neurologic Processing **(*HNP*)** (see also glossary description, elsewhere in this document) per BOX H block **[37] Alertness tests (as further detailed in section titled "eLifeALERT"), ultrasound or tonometry and/or TCD tests as further outlined in section titled "eLifeDOPPLER" per** BOX H block **[38];**

### Summary online TBI prognosis and or Diagnosis System

**The present TBI prognostic and/or diagnostic group of examples including:**
>at least one event generation aspect,
>monitoring aspect, analysis aspect,
>reporting or display aspect,
>along with a **series of apparatus** and/or methods for the **assessment of the sequence** and/or **steady state and or transitions** related to a series and/or **parallel (simultaneous) changes** (alterations) to the brain and/or central nervous system and or/ **cognition** and/or connectivity, and or inter-connectivity and/or sequencing and/or transmission (I.e. Atonal or sensory through to higher-order processing contingent), along with associated physiological, neurological, motor and/or other exogenous, endogenous activity or responses,
the examples further comprising any of:
**>event generation presented** to subject under test as stimulus (I.e. including any of or any combination of auditory, visual, electrical, magnetic, thermal, pressure or vibration and/or other stimulus) and/or task (I.e. activate switch, gesture, motion, touch screen selection or gesture, acknowledgement or response to audio, acknowledgement or response to video) and/or challenge generation capability, whereby timing information between said generation of such involves retaining the precise timing information of so that any subsequent physiological or neurological responses can be analysed in the context of the precise timing relationship with the originating stimulus or task or challenge events;
> **monitoring system comprising** of any of or combinations of wearable devices (such as figures ***Figure 46 to Figure 55*** showing a range of examples (but not limited to) simple to complex wearable monitoring device options, along with wearable-device minimisation capability per Figure 45 with minimisation determination block 8 and self-assessment and corresponding self-learning capabilities per ***blocks [11], [12] and [13],*** or stand-alone devices or iPad or computer touch pad etc., or sensor linked system (I.e. Tracks movement or dexterity of movements via sensors).
**>whereby the said event generation aspect incorporates** the capability of generating, and presenting to subject under test, at least one or any combination(s) of stimulus, task or challenge events;
**>whereby the said monitoring aspect incorporates** the capability of monitoring, signal processing, acquisitioning and analysing at least one physiological or neurological signal;
**>EEG, EP, imaging** (I.e. Voxel based analysis);
**>spontaneous event process;**
**>event-related process;**
**>numerical, parametric, non-parametric, graphical, visual,** and/or auditory outcome measures; and atlas combinations in terms of symmetrical or activation or suppression of brain activation and/or connectivity;
> ER
> AEP
> MIND
> MIND ER

### STATISTICAL ASSESSMENT OF CUMULATIVE TESTS

**>The present** example **incorporates** a means of combining a **plurality of independent or dependent cognition tests** in order to produce a more accurate measure than any one singular test.
   Moreover the present example can **deploy a number of multi-dimensional disparate physiological cognition tests** in an integrated manner whereby independent tests have a cumulative result in terms of improved outcome measurement precision.
**>An further example** is to enable a **multi-dimensional physiological and/or behavioural combined cognition assessment approach** including any of or any combination of (for example only but not limited to) ER, **EEG,** TCD, Behavioural tests. Moreover the outcomes of these tests if such as TCD and ER can be treated as independent and hence have a cumulative cognition test accuracy.
**>For example,** in the **case where the vaso-spasm test (test A)** is conducted in conjunction with a neurological test (test B- say 10 minute ER oddball audio stimulus and/or oddball audio-visual stimulus and challenge) then on the basis that test A and B are considered independent then the cumulative accuracy of this test sequence or simultaneous test paradigm.
>In the case where the **present** example **incorporates a head set with transcribing Doppler (TCD)** sensors combined with neurological test sensors and whereby the TCD test is considered to have a cognition deficit prediction accuracy of 40%, whilst the neurological test is considered to have an accuracy of 70% then the cumulative accuracy is 82% (I.e. Chance to be wrong is .3 x .6 = .18 and chance to be correct therefore .82) which is substantially greater accuracy than either isolated measure (I.e. TCD 40% or Neurologic 70%).
**>In a further example an integrated** (interconnected and/or same or **combined measurement devices and/or statistically cumulative measures** for enhance outcome precision measures of brain injury and/or cognitive impairment such as associated with neurology disorders including TBI, mTBI, Alzheimer's, dementia etc.), including any of or any combination of (but not limited to):
> **The present** example **can incorporate blood test measures** such as utilisation of chemical embedded test strips capable of detecting spinal fluid presence as a marker of brain injury. If (for example) this test yields a 60% accuracy in terms of brain-injury or cognition measures, then the resultant probability of a correct outcome accuracy when this test blood test is combine with above ER and TCD example could be considered as 92.8% (.6x.3x.4 = .072 or 7.2% chance for wrong result or 92.8 % chance of a correct result).
**>The present** example can **sequentially or simultaneously** (I.e. Measure with other tests such as TCD Vaso-spasm and/or ER oddball paradigms) **deploy vascular responsive measures.** For example, TCD ER "vascular response" measures are changes in circulatory measures associated with stimulus or challenge/task test paradigms. These said "vascular response" markers can be measured as ultrasonography and/or TCD responses to Neurologic ER response tests. In other words brain circulatory demands are enhanced with synaptic activity which is in turn activated by way or ER stimulus (auditory and/or visual and or physical such as hand squeeze or pain responses etc.) or cognitive challenge responses (I.e. such as but not limited to MIND ER examples per glossary at rear of this document). If (for example) this test yields a 70% accuracy in terms of brain-injury or cognition measures, then the resultant probability of a correct outcome accuracy when this test blood test is combine with above ER and TCD example could be considered as 97.84% (.6x.3x.4x.3 = .0216 or 2.16% chance for wrong result or 97.84 % chance of a correct result).
**>The present** example can **sequentially or simultaneously** (I.e. Measure with other tests such as **EEG spatiotemporal dynamic activation** (I.e. Measures of overall frontal lobe **EEG** energy increase during ER oddball test protocol (say 5 to 10 minute test) as well as measures of increased brain activation associate with one or more combined oddball deviant (target) responses - I.e. Responses corresponding to auditory and/or visual and or physical such as hand squeeze or pain responses etc., or resultant responses associated with cognitive challenge responses (I.e. such as but not limited to MIND ER examples per glossary at rear of this document). If (for example) after correcting for dependence via appropriate joint-probability statistical principles this **EEG** spatiotemporal dynamic test yields a 70% accuracy in terms of brain-injury or cognition measures, then the resultant probability of a correct outcome accuracy when this test is combined with above ER and TCD example could be considered as 97.84% (.6x.3x.4x.3x.3 = .00648% or .648% chance for wrong result or 99.352 % chance of a correct result).

### ENTROPY CONSCIOUSNESS LEVEL DETERMINATION

>The present example can **sequentially or simultaneously compute from event-related** (I.e. Corresponding to auditory and/or visual or other) stimulus NLDB measures from AEP (i.e. ***Figure 56***) as a measure of brain injury or cognition impairment based on sensor and/or source reconstruction measures, whereby spatiotemporal dynamic measures. For example, measures of overall frontal lobe **EEG** and/or AEP and/or ER NLDB analysis based measures change during ER oddball test protocol (say 5 to 10 minute test) as well as measures of changed NLDB Analysis associated with one or more combined oddball deviant (target) responses - i.e. **changes corresponding to NLDB analysis responses** to auditory and/or visual and or physical such as hand squeeze or pain responses etc., or resultant NLDB response changes associated with cognitive challenge responses (I.e. such as but not limited to MIND ER examples per glossary at rear of this document). If (for example) after **correcting for dependence via appropriate joint-probability statistical principles** the NLDB **EEG** and/or NLDB spatiotemporal dynamic test yields a 70% accuracy in terms of brain-injury or cognition measures, then the resultant probability of a correct outcome accuracy when this test is combined with above ER and TCD example could be considered as 99.81% (.6x.3x.4x.3x.3x.3) = .00194 % or .194% chance for wrong result or 99.81 % chance of a correct result).
**>Note:** as with all these accuracy determination outcome, the present example **automatically takes into account the desired measure type in terms or parametric or non-parametric** nature of accuracy determination measure. For example, if a non-parametric measure incorporating a 4 level **ordinal scale of normal cognition state, mild cognition impairment, moderate cognition impairment, severe cognition impairment** is deployed or required by user then the present example will compute individual and cumulative measurement precision (accuracy probability chance) taking into account such factors.
**>Neurologic test:** In this above example the **Neurologic test could comprise of comparing an auditory odd ball stimulus presentation** and comparing of target or odd-ball responses with non-target responses as a measure of cognition based on attention or alertness/awareness of odd-ball stimulus deviation;
**>TCD test: A further example** comprises of a **head-wearable mounting system incorporating one or more TCD probes** which can be **automatically or manually positioned** (with manual positioning option also where by head mount is attached for optimal positioning, location and attachment using a series of localised (i.e. Around anterior and posterior head vascular measurement regions) as a low-expertise or minimal training requirement aid of optimal and quality application, the present example further comprising any of (but not limited to);
>>as part of the said **integrated or TCD specific head-wearable mounting system for the inclusion of an auditory stimulus presentation via earphones,** headphones, speaker etc. along with a plurality of **EEG** electrodes to monitor resultant auditory stimulus Neurologic responses;
**>>>as part as of the said integrated** (I.e. Combined ER auditory and/or visual stimulus and/or resultant **EEG**/ER Neurologic responses and/or resultant vascular responses) **TCD head-wearable mounting system a local or remote guidance system** can be deployed to enable minimally skilled personnel to apply optimal TCD measures capable for example of accurate tracking of **anterior circulation vessels and/or posterior circulation vessels and/or medial circulation vessels and/or other Willis circle circulation vessels** and/or any head circulation vessels and/or the incidence of any blood vessels for mild, moderate, and severe vasospasm (VSP);
**>>>>An example** of such a guidance system can include (but not limited to) **head-wearable mounting system incorporating one or more TCD probes** which can be automatically or manually positioned with a manual guidance-positioning option whereby head mount is attached for optimal positioning, location and attachment using a series of localised (i.e. Around anterior and posterior head vascular measurement regions) using light emitting diodes (LED) (I.e. green illuminated diode for strong or acceptable TCD signal, orange LED illumination for marginal TCD signal, and red LED illumination for unacceptable TCD signal monitored). Additionally, visual display screens can achieve a similar function. In an automatic TCD head-wearable mounting system the TCD probes can incorporate servo (I.e. Biofeedback between TCD measurement signal to noise and position along with image idolisation angle of TCD measurement probe) control. For example, TCD probes can be beam controlled and/or physically repositioned via TCD probe or TCD beam/positioning and/or motor positioning of probe physical position and optimal imaging angle across and of "x-axis" (left to right and vice versa), "y-axis" (up and down and vice versa) and "z-axis" (back to front and vice versa) axis of complete rotation as well as courser longitudinal and latitudinal positioning as required for optimal TCD monitoring at any time.
>>In one example TCD measurement the **cerebral arterial vasospasm (VSP) and intracranial hypertension** (common outcomes of road-accident or combat-related traumatic brain injury (TBI)) can be measured ¹⁶⁶⁻¹⁶⁹.
>Reports show that almost a third of U.S. combat **soldiers with wartime traumatic brain injuries are at risk of developing significant complications, including constricted blood vessels as well as brain-swelling. TCD ultrasonography is a simpler** (than angiography, MRI or X-ray imaging) and less obtrusive procedure for monitoring vascular clinical outcomes such as TBI sufferers, where vascular conditions can be adversely impacted.
   The present example can measure and evaluate subjects, suspected of TBI for TCD-determined incidence of post-traumatic **cerebral vasospasm,** by way of tracking and characterising vascular velocity measures beyond a threshold (such when measures are greater than 200 cm/s) as a marker of brain injury and/or associated cognitive impairment.
>>>the present example can measure and or **track anterior circulation vessels and/or posterior circulation vessels and/or medial circulation vessels and/or other Willis circle circulation vessels** and/or any head circulation vessels and/or any blood body vessels for mild, moderate, and severe vasospasm (VSP).

### COMBINATIONAL STATISTICAL ANALYSIS OF DISPARATE MULTI-DIMENSIONAL OR DEPENDENT COGNITION MEASURES

the present example incorporates a means of **combining independent measures of cognition using joint probability** analysis techniques (such as example detailed elsewhere with combined TCD and Neurologic measures) as well as combining measures not considered truly independent using other approaches such as joint probability dependence correction methods ¹⁴⁷.

### RANDOMISED VERSUS ORDER-DEPENDENT (I.e. PERMUTATIONS) VERSUS COMBINATIONAL STATISTICAL DTERMINATION

**In another example multiple tests systems** can be integrated (i.e. per ***BOX A*** ***Figure 100*****)** and statistical culminated in terms of enhanced measurement outcome precision by way of utilising measurement outcome aggregation.
>>> **Permutation (AEP response. order specific) randomised sample** : in one example the response difference computed between target and non-target conditions can be calculated in one process, whereby a single subject test protocol can be designed based on a null-hypothesis that the difference between said target and non-target has no measurable outcome on corresponding responses (I.e. Neurologic response to auditory target versus non-target responses). The individual trails can be randomly assigned to two groups of outcome responses, comprising as non-target response group and target response group. In this manner the permitted difference between the permuted target and permuted non-target groups can be calculated as detailed below.

**In the case of 6.8 clicks per second auditory stimulus** (non-target- 147 ms inter-stimulus interval) versus oddball (target interspersed tone every say randomised 9, 10, 11 pulses or averaged at every 10th stimulus).

**In this example 250 trials** with a randomised but average 1 deviant tone target in 10 non-target clicks, encompassing 118 seconds can be deployed. In this case the total number of target trials is 25 (1 in 10 of 250 total trials) while the number of non-targets is (9 in 10 of 250 total trials). Therefor ore the corresponding number of permutations can be computed as : total number of trials permutation (250!) divided by non-target trials permutation (225!) multiplied by target trials permutation (25!) or 250!/ 225! X 25!;

**This can be represented by:**
2.567061100172e+59/1.5511210043331e+25= 1.6561684517239e+34 possible permutations, thus resulting in a maximum probability of 1.6561684517239e+34, or in other words the chance of an error being 1/1.6561684517239e+34.

***Note: repetition from Somfit overview at head of document* so *this section could be removed and replace with "refer to Somfit overview at head of document sections a) to dd)"***

### Title: Somfit

The application presents a number of methods and devices incorporating or enabling communication interface (including one or more wearable devices, WAN, LAN, IP, NAS, and/or Cloud-computing services) with the means of sensing, monitoring, tracking, storing and/or analysing any of or any combination of a subject's physiological parameters, pathological conditions, psychological states, wake, sleep, activity, fitness, health, other sentient states, associated transitions, and/or neurological parameters including option of automatic determination of the prediction, onset or incidence of health conditions/disorders or events of interest by way of incorporating characterisation and determination of processing capabilities or combinations (i.e. multivariate analysis) or clusters or ensembles applicable to any of or any combination of the following;
a) ***A patient-wearable sleep, fitness, health, sentient state monitoring device (Somfit);***
b) An electronic module ***(Somfit;*** ***Figure 1*** RHS, [1] and [7]) incorporating multi-device **(*****Figure 1*** RHS, [8], [5]) interchangeable electronic module and/or associated information with automatic dynamic online ***data-exchange enabling sleep, health and fitness measures*** and indicator display capabilities, comprising one or more light-detection and applied forehead electrophysiological sleep-parameter(s) **(EEG,** EOG and EMG) monitoring capability, along with measures or associated indices including (but not limited to) sleep efficiency (SE), wake after sleep onset (WASO);
c) The **sensing, monitoring and analysis** of any of or any combination of or any combination of wake and sleep events, measures, states or health and environmental conditions of interest,
d) **An electronic module *(Somfit;*** ***Figure 1*** **RHS, [1] and [7]),** comprising any of or any combination of or any combination of sensing, monitoring and analysis capabilities,
e) Said "electronic module" ***(Somfit)*** incorporating means (i.e. any of or any combination of or any combination of magnetic, mechanical, and/or interlocking) of being **interchanged between a plurality of wearable** health or environmental monitoring devices ;
f) Said electronic module which can be **attached or applied to a subject's forehead** or attached or worn on another wearable subject location (i.e. **wrist bangle or smart watch** device) **Figure 1** **RHS, [8], [5]);**
g) A "Forehead applied sensor" incorporating **at least one bipolar** forehead electrophysiological signal **(****Figure 4****; [8], [9], [10], [11], [12]);**
h) A "Forehead applied sensor" comprising of a **reusable sensor;**
i) A "Forehead applied sensor" comprising of a **disposable sensor;**
j) A "Forehead applied sensor" comprising of a sensor with or without a partial or total circumference headband **(****Figure 4****; [13]);**
k) A "Forehead applied sensor" which can comprise on one **side of a sensor with self-adhesive surface and embedded self-gelled electrophysiological electrodes,** whereby these said "electrode" can be exposed with the **removal of backing paper;**
l) A "Forehead applied sensor" which can comprise on **non-electrode side** of said "forehead applied sensor" a **means of interfacing** (i.e. self-adhesive, press-stud, magnetic, mechanical interlocking or other means) to said **"electronic module"** or a device containing or holding said "electronic module" **(****Figure 4****; [7]);**
m) A "Forehead applied sensor" The said "Forehead applied sensor" can monitor at least one forehead sleep-parameter signal including any of or any combination of or any combination of **EEG, EOG and EMG;**
n) The deployment of a subject **applied forehead-sensor capable of monitoring principle sleep-parameters *(EEG, EOG, EMG)*** combined with interconnectivity with a secondary wearable communication/indicator device enables a range of sleep and wake events or disorders to be tracked and managed, including the option of corresponding countermeasures by way of therapeutic device or other forms of intervention;
o) The ***deployment of compatible and interconnected companion patient worn devices*** (i.e. subject worn or related; placement of sound monitoring or environmental monitoring devices (Burton 2014 AU Prov. App. Mch14) further enables monitoring and automatic ***tracking of wake or sleep and associated breathing disorders.*** Information between a group of compatible (i.e. wireless communication and information) devices (i.e. as covered elsewhere in this document; eLife**WATCH** (Figure 5, **[5]),** eLife**WRIST** (Figure 5, **[8]);** eLife**CHEST** (Figure 5, **[4]);** eLife**EXG (****Figure 23****),** eLife**NEURO (****Figure 4****, [2])** which can automatically and dynamically interchange data between one of more of the said devices, enabling determination and associated indication of any combination of measures or associated indices;
p) Where said ***"measures or associated indices"*** can include any of or any combination of this for fitness, health and/or sleep-parameters ***(personalised sleep quality, efficiency*/*SE, wake after sleep onset*/*WASO,*** deep-sleep body recovery period, REM sleep brain restoration period, sleep-in-progress tracking, sleep-quality progress, sleep-journal, comparative population or personalised sleep function, sleep-debt, sleep-disturbance, respiratory-disturbance, sleep-disturbance causation along with corresponding sleep improvement hints or recommendations, sleep-architecture, sleep structure, sleep fragmentation etc.).
q) The deployment of a ***somnilink*** system comprising of an internet or other wireless or **interconnectivity means of interfacing signals or derived measures from the *Somfit* system with medical therapeutic or diagnostic devices (IOD) whereby the *Somfit* can form a biofeedback (closed loop or other control incorporating *Somfit* measures as part of decision making processes responsible for controlling therapy administration).** Where said therapy control can include sleep therapeutic devices such oral mouth adjustment systems, patient positioning devices or trainers, PAP, NIPPV and other devices. Where said therapy control can include relaxation or meditation vidual outputs or controls (i.e. massage chair control) or music or room lighting or video or 3D projections etc.). Where said therapy control can include magnetic or electrical stimulation devices.
r) The ***Somnisync*** system incorporates a means of enabling ***"dynamic data-exchange" of sleep parameters*** or other health or fitness parameters between two or more wearable devices or associated mobile wireless communication devices or computer systems.
s) Whereby said ***"dynamic data-exchange"*** can enable personalised management of wrist-bangle or other wearable device sleep and/or fitness and/or other health conditions or status;
t) This ***dynamic data-exchange*** can be via wireless interconnection between ***two or more wearable devices*** enabling a means for sleep monitored parameter data and/or associated sleep measures to be automatically displayed on a wearable display device, such as mobile phone **(****Figure 1****, LHS, [8]),** smart watch **(****Figure 1****, RHS, [5]),** wrist bangle **(****Figure 1** **RHS, [8])** or other wearable system;
u) This ***dynamic data-exchange*** can be via wireless interconnection between ***two or more wearable devices*** enabling a means for sleep monitored parameter data and/or associated sleep measures to be automatically displayed on a wearable display device, such as mobile phone **(****Figure 1****, LHS, [8]),** smart watch **(****Figure 1****, RHS, [5]),** wrist bangle **(****Figure 1****, RHS, [8])** or other wearable system;
v) An electronic monitoring transfer electronic module from (with applied forehead **EEG,** EOG, EMG monitoring part), for example, to remove forehead applied sensor and device and transfer electronic module to a wearable wrist device wrist augment conventional daytime wrist pedometer or motion-based fitness devices with sleep measures.
w) The ***Somnilink forehead applied, wrist device*** (i.e. watch or bangle) or ***other wearable or attachable devices*** can incorporate a means of detecting room or environmental light conditions (i.e. such ***means include light dependent resistor*** or other photo-sensors essential for internationally accepted standard sleep indices such as sleep efficiency or sleep after wake onset). The output information of the light sensors can be linked to online and automatic measures applicable to sleep measures such as sleep efficiency, sleep after wake onset, sleep time, REM sleep (during sleep time), deep-sleep (during sleep-time), NON-REM sleep etc. These said light detections sensor derived measures can be displayed as part of another wearable device to provide a subject instantaneous measures or indicators of sleep function and performance. These sleep measures can be displayed in conjunction with other fitness (such as accelerometer and/or motion sensor and/or pedometer sensor measures) in order to provide a sleep and fitness tracking capability applicable to the said subject via a wearable information indicator device (such as watch or bangle) or other subject wearable or attachable device.
x) A system automatically computing a subject's ***intra-sleep and inter sleep progress and associated outcomes*** and related information (disorders, measures, indices, quality or severity of sleep and associated disorders or concerns). The system is able to distil such information using an information ***triage means*** (i.e. ***triage means*** can include an information dissemination process, whereby such a process is in accordance to a predefined (i.e. but not limited to empirical prior data studies, normative or disease/disorder state populations data or studies) or dynamically (i.e. but not limited to determination and/or adaptation and/or adjustment based on preceding monitored or sensed information) computed user access ***authority*** and ***rights*** covering ***role, qualifications, security*** and ***privacy*** aspects as well as appropriate ***user interface and information access or information content complexity levels.** Said "information content" can include* sleep architecture, including REM sleep and deep-sleep amount, disruptions or arousals.
*y)* A system ***automatically computing and advising a subject when they should be having or need additional sleep, the quality of sleep, the improvement recommendations*** based on ongoing tracking and computation of a subject's ongoing sleep tracking outcomes, as well as these said outcomes comparative to a subject's normal sleep requirements or normative population data base comparatives. The present system incorporates a means of referencing said data bases.
z) A questionnaire or ***sleep survey such* as a *validated sleep-scale or validated drowsiness scale* to *be deployed in conjunction with self-assessment*** in order to **establish a set of criteria corresponding to a subject's normal, levels or indices** (i.e. REM sleep, sleep time, deep-sleep, arousal index, AH respiratory index, RERA index, overall sleep quality, and/or sleep deprived status, enabling a subject or subject's health carer ***enhanced information access for improved sleep management.** A* further ***means of comparing such information*** with current inter-sleep and intra-sleep progress reports or related trends in order to establish recommendations based on these outcomes and recommendations of hints for subject or associated health carer (means of ***means of comparing such information*** can include reference to a subject's personal model or sleep as established with said personal survey assessments and ongoing calibration of related "criteria" as well as comparison to patient normative data bases.
***aa)* *Sleep-stage-linked synchronisation of* a *clock or alarm* system** or other clock or alarm system to choose the more optimal alarm time for awakening based on users need to awaken versus sleep cycles of minimal adverse impact during awakening (i.e. avoid awakening during deep-sleep when longer sleep recovery may be evident, if prediction or eventuation of REM sleep stages are close-by that can enable less disruptive awakening without compromising overall sleep or awakening time requirement).
***bb**)A **minimal configuration comprising*** of a single headband sensor ***(Somfit)*** capable of monitoring brain signals capable continuous monitoring of **sleep *parameters (EEG, EOG, EMG)*** and automatic online processing to enable the determination of sleep stages (i.e. REM, non-REM stage 1, non-REM stage 2, non-REM stage 3 and stage 1).
**cc)** the options of ***monitoring room light detection (i.e. LDR)*** and/or ***breathing sounds (i.e. snoring)*** monitoring and/or sleep hypopnoea monitoring via a range of other optional sensors (including any of or any combination of RIP, PVDF, piezo or other thoracic and/or abdomen belt; nasal cannula sensor; airflow sensor)
**dd)**the option to have the single sensor strip incorporate an embedded ***reflective oximeter sensor (LED with LDR),*** whereby the oximeter sensor can be attached or embedded in the forehead sensor providing plethysmography and oximetry with associated outputs (including any of or any combination of or any combination of PTT, pulse-wave oscillatory amplitude autonomic markers of obstructive apnoea, pulse wave amplitude, pulse arterial tone). Whereby other sensors can include a "drop-down" (i.e. connects to ***Somfit)*** airflow sensor (i.e. PVDF, thermo-coupler, thermistor, nasal cannula etc.), whereby said sensor(s) can enable monitoring of sleep disordered breathing including apnoea, hypopnoea, mixed apnoea/hypopnoea.

### Somfit biofeedback therapy applied head sensor system

### ee) Patent:Somfit stim

The present invention incorporates a wearable applied sensor system comprising of an applied head or forehead sensor incorporating one or more monitoring electrophysiological electrodes (including combined or separate electrodes capable of monitoring and/or delivering stimulus signal), the present invention further comprising any of or any combination of:
- an attached or separate part comprising of a monitoring system;
- an attached or separate part comprising of a stimulus system capable of generating at least one stimulus signal;
- whereby the said monitoring system comprises of an "electronics module" capable of monitoring at least one electrophysiology signal;
- whereby the said monitoring system is capable of simultaneously emitting at least one stimulus signal;
- whereby the said monitoring system can incorporate analysis capabilities (I.e. on-board or integral processing to device and/or supplementary processing via wired network or cloud-based computing and/or other interconnected processing capability), - whereby the said stimulus signal can comprise of an electrical and/or magnetic and/or auditory signal;
- whereby the electrophysiological sensor(s) can incorporate the capability of generating an electrical stimulus to subject (I.e. forehead and/or face and/or head);
- whereby the electrophysiological sensor(s) and/or separate sensors can generate a stimulus signal to the subject (I.e. forehead and/or face and/or head);
- whereby the said stimulus signal can be for purposes of applying therapy to a subject:
   - whereby said therapy can be for the purpose of therapy associated with migraine and/or headache management and/or vascular management and/or brain activation and/or neural disorder management (including for example only anxiety, depression, Parkinson's, Alzheimer's etc.);
   - whereby said stimulation can be determined and generated by said monitoring system;
   - whereby the present invention can incorporate electrophysiological monitoring sensor(s) or separately attached have the capability of generating an electrical stimulus to subject (I.e. Forehead or head) for purpose of evoking a neurological response via such stimulation,
   - whereby present invention can incorporate vascular measurement sensor capability;
   - whereby vascular measurement capability can incorporate TCD sensor and monitoring capabilities and/or tonometry monitoring and/or vascular pressure or movement as further described elsewhere in this document;
   - whereby the present invention can incorporate electrical stimulus to subject (I.e.Forehead) for the purpose of ER stimulation, and whereby said resulting ER can be synchronised with originating electrical stimulation in order to evaluate responsiveness of stimulated region of brain (I.e. Frontal lobe responsiveness to stimulation) can be assessed;
   - incorporation of processing whereby source localisation of resultant electrical stimulation can be computed in order to assess source of brain stimulation or associated spatiotemporal dynamic measures;
   - incorporation of processing capacity enabling monitored responses to be synchronised with said electrical and/or auditory and/or magnetic stimulus to subject (I.e. Forehead) for purpose of ER stimulation" can be synchronised with electrical stimulation in order to evaluate responsiveness of stimulated region of brain in order to provide biofeedback between monitored and processed responses to stimulus and generation or properties of generated stimulus as a means of achieving desired the responsiveness outcomes (I.e. Brain signal activation change ; increased frontal left and/or right brain signal activation; suppression ; suppressed frontal left and/or right brain signal activation; suppression; Vascular signal activation change ; increased frontal left and/or right vascular signal activation; suppression ; suppressed frontal left and/or right vascular signal; changed source localisation characteristics towards preferred source localisation brain activity characteristics ; in order to achieve a certain state of focus, relaxation, meditation or sleep stage, etc.);

### Interconnection beam forming capabilities to optimise wireless interconnection capabilities

**ff)** A beam forming capability may be provided as it relates to wireless interconnectivity reception and/or transmission in a manner whereby the transmission pathway between the present system (i.e. Somfit) and interconnecting systems (i.e. but not limited to Cloud-based software services, LAN, Bluetooth, Wi-Fi, WAN etc.) are focussed in order to minimise interference from other unrelated (unrelated to specific Somfit interconnectivity requirements) or unwanted noise sources within the present system's monitoring environment at any point in time or anywhere.

### Integrated earphone ER assessment capabilities

gg) to the system may incorporate time synchronisation capabilities in order to utilise conventional mobile devices (phone, iPads, touchscreen devices, tablet PCs and the like) so that the system can superimpose auditory stimulus within earphone or earbuds in a manner where the said stimulus and resulting brain waves or other resulting responses can be measures and synchronise to original stimulus timing. In this way the system can either utilise existent entertainment or purposeful auditory signals as a stimulus signal and synchronise monitoring of resultant responses, followed by processing such responses (i.e. ER and/or AEP signals) in terms of the determination of an individual's responsiveness and/or alertness and/or awareness at any point in time. For example, the system can insert or superimpose as part of an existing music track or audio-visual experience, a special audio and/or visual stimulus designed to test an individual's responsiveness at any point in time. Additionally, the system can provide conventional AEP, MMN or oddball auditory or audio-visual tests (such as further exampled in glossary under examples per MIND ER).

### Somfit combined with envelope follower breathing and other sound processing capabilities

hh) The system may incorporate on-board (i.e. attached within or part of the Somfit monitoring system) processing capabilities designed to produce an envelope follower capability, in order to minimise storage and wireless capabilities otherwise required to accommodate monitoring or breathing and/or other sounds. The system may enable a calibrated sound function (manual or automatic procedure, such as factory calibration using a defined distance between subjects mouth or nose and a locational position associated with the monitoring system (i.e. Somfit), whereby a subject's snoring levels or other breathing sounds or other breathing sound characteristics such as asthma wheezing, cough, crackle (i.e. see also other breathing sound disorders listed elsewhere in this patent document).

### Somfit impedance monitoring for detecting change in health or mental conditions

ii) A means of measuring impedance between a plurality of electrodes may be provided, by means of emitting a small constant current signal applied to the subjects skin via a ***first electrophysiological electrode*** in a ***first step,*** and then in a ***second step*** measuring a voltage between a ***second electrophysiological electrode*** and therefore determining the impedance between the two said ***electrophysiological electrodes.*** Whereby the measured impedance between the first and the second said electrode can represent the galvanic skin resistance of a subject. Whereby tracking or monitoring the said impedance measures can be used as part to determine perspiration or anxiety of a subject. Whereby the monitoring of impedance can be used in the determination of sleep/wake cycle of a subject. Whereby the measurement of impedance can be used to determine a range of physiological or psychological conditions or status of a subject. Whereby the said constant current can be emitted from the first or second ***electrophysiological electrodes*** at any time or in any sequence. Whereby the said constant current can be switched between the said ***first and second electrophysiological electrodes*** in a manner that minimises polarisation of the said ***first and second electrophysiological electrodes*** or associated interface or connectivity associated with subject measures and the said ***first and second electrophysiological electrodes.*** Additionally, the present system can incorporate this manner of switching constant or other current or voltage or any energy factors between a plurality of electrodes or sensors in order to minimise electrode **offsets** or polarisation effects, which can otherwise deteriorate signal monitoring quality or signal monitoring conditions. Such galvanic skin resistance and/or skin applied impedance measures with other signals (i.e. EOI as outlined in glossary or other electrophysiological signals and associated measures such as any of or any combination of plethysmography oximetry including PWA, PAT, PTT, pulse outputs), **EEG,** EOG, EMG, body or surface skin temperature, ECG, tonometry, TCD and/or other physiological signals or conditions may be incorporated, as a means of determining onset conditions for seizures or other potential adverse health sequelae.

### GLOSSARY - ACCRONYMS, ABBREVIATIONS, DEFINITIONS, AS PART OF PATENT DESCRIPTION

| | |
|---|---|
| [1] | When preceded by a figure number this square bracket format refers to the relevant block number (listed in side of the brackets) as a specific example (but not limited to) as related to the text preceding the listed figure or figures. In this way the overall figure or figures are referenced as well as a more specific block number of the corresponding figure or figures. |
| A&CD | Anaesthesia and consciousness depth |
| AASM | American Academy of Sleep Medicine. |
| AASM Type 1, Type 2; type 3 or type 4 | American Academy of Sleep Medicine. Conrad, I, Ancoli-Israel, S, Chesson Jr, AL, and Quan, SF. The AASM Manual for The Scoring of Sleep and Associated Events, Rules, Terminology and Technical Specifications. 2007. American Academy of Sleep Medicine. And type (level) 1 to 4 as further outlined in N. A. Collop et al., "Obstructive Sleep Apnea Devices for Out-of-Center (OOC) Testing: Technology Evaluation," J.Clin.Sleep Med 7, no. 5 (2011): 531-548 ¹⁷⁰.¹⁷¹ |
| ABI | ankle-brachial-index |
| AC&R | Artifact Compensation and Rejection. |
| ADHD | Attention deficit hyperactive disorder |
| AEP | Auditory evoked potential |
| AEP | Auditory Evoked Potential (typically refers to the brain waveform covering the time period commencing or prior to an auditory stimulus onset through to the time of the onset or a time preceding the onset of the next auditory stimulus. |
| AEPi_{AS} | AEP index values based on Sum (S) of absolute AEP averaged (A) values |
| AEPiAS | Sum of absolute AEP amplitude values |
| AEPi_{ASQ} | Sum of square root of AEP amplitude values (AEPiASQ) |
| ALS | algorithm subject/patient-specific learning |
| AP | augmentation pressure |
| APAP | Automatic-titrated positive airway pressure |
| APM | (or APN) Adaptive Physiological-Body Monitoring-Network |
| APN | Adaptive Physiological-Body Network |
| ASA | Australian Sleep Association |
| ASA Level 1, 2, 3, 4 | Australian Sleep Association level recommended level 1 to 2 sleep study formats. |
| ASD | autism spectrum disorders |
| ASL | algorithm subject/patient-specific learning (ASL) system |
| ASPD | advanced sleep phase disorder |
| AU | Australia |
| AV | Audio Visual |
| AVE | Averaging or average (typically refers to averaging a sequence of evoked responses (evoked by and synchronised to a subject stimulus signal) |
| BCP | Blink closing period |
| BODY MASS INDEX | Body Mass Index |
| C02 | Carbon Dioxide |
| CAPA | Corrective action preventative action |
| CAT | Computer axial tomography or CT/computer tomography |
| CB | Cognitive behavioural (i.e. ERP and cognitive behavioural event related potential refers to the neurological response to an event along with the simultaneous associated ERP) |
| CC | **Circadian** Cycle |
| CDR | Current density reconstruction |
| CE | "Conformité Européene" which means "European Conformity" |
| CIDM | Complex broad-based Investigatory Diagnostic Monitoring |
| Clinical data or clinical information or diagnostic data or diagnostic information | Generally this applies to information that has been derived from measurement devices or monitoring systems such as wearable health monitoring or environmental monitoring data such as pollens, smog, smoke, pollution, temperature, humidity, toxic gases, toxic chemical and other environmental factors that can impact upon an individual's health. |
| CNS | Central Nervous System (where brain is mentioned CNS, which incorporates wider nervous system including spinal-cord and associated nerves can mainly be substituted) |
| CNT | Continuous data or continuously |
| CNT SR | Continuous **EEG** recording or monitoring (CNT) spontaneous response (SR) |
| Cognitive Coding System **(*CCS*)** | **Cognitive Coding System (CCS)** refers to the process whereby the **outcomes of the Cognitive Processing Sequencer (CPS) can be represented as a standardised code** which incorporates the BPS factors as well as a cognitive connectivity coding sequence applicable to assessment of an individual, comparison with normative data or trend analysis applicable to diagnostic or prognostic outcomes of an individual's mental/ psychological or neurological health. BCS enables, for example, the presentation of a stimulus such as video picture or auditory sound and then measures the brain, followed by step by step codification of connectivity before during stand after stimulus. In this way we can compare codification to challenges or tasks (such as MIND ER per glossary herein) or behaviour tasks for behavioural development status or progressive review via codification changes with time for a subject, along with normative codification comparisons. In other words a universal means of codifying brain cognition impairment, development, performance, deterioration in terms of objective status and trending changes with the course of time or in response to medication, therapy, environment and other factors such as neurological disorders impacting cognition is provided. |
| Cognitive Processing Sequencer **(*BPS*)** | **Cognitive Processing Sequencer (BPS)** enables a means of connectivity examining the sequence of brain connectivity, with the options for spatiotemporal dynamic BPS and also based on varying time windows applicable to singular events (i.e. examination of 10ms steps covering pre-stimulus to subsequent stimulus time period across a single brain response, or across a combination or permutation of brain responses (i.e. averaging or regressive modelling). In one example, such means can refer to one or more **EEG** channels being monitored, followed by current density source reconstruction, coherence analysis (with option of hierarchical segmentation applicable to fibre or ABR transmission connectivity through to higher order processing contingent potentials), and ultimately the sequence of overall or **hierarchical connectivity (i.e. time windows of lead or lag time between coherence connectivity regions of the brain)** based on a range of time windows or steps. "Steps" refers to the successive points in time examined in terms of changes in terms of progressive status and changes of brain connectivity. BPS can also be applied to spontaneous events, continuous status evaluation (i.e. across minutes or hours of monitored subject data) whereby cognition, consciousness, neurological disorders, and/or sleep/wake states and/or altered states of consciousness and/or therapeutic or medication induced changes can be examined in an objective manner and consistent or scientifically/clinically repeatable manner. |
| COH | Coherence |
| COH lag time | This parameters can be configured, for example, to say plus and minus 30 % of a point manually marked or automatically detected in a particular SR or EOI, where - 30% could denote 30% lag time with reference to the total SR or EOI represented by 100% and the lag factor relating to the centre cursor position location or the automatically detected centre point (time-wise) or for example peak point (magnitude-wise of the said SE or EOI under analysis. |
| COH lead time | Adjustment parameter for COH analysis |
| COH lead time | Similar to COH lag time example except +30% represents right hand cursor selection of 30% of total SR or EOI time being the end of range COH time period range of interest. |
| COH magnitude range setting | Parameter designating the magnitude range (i.e. applicable to NLDBV, STV, or conventional amplitude neurology **EEG** or voxel image element measures=) across which COG interconnectivity is detected |
| COH maximum magnitude | Parameter designating the maximum level across which COG interconnectivity is detected |
| COH minimum magnitude | Adjustment parameter for COH analysis |
| COH minimum magnitude | Parameter designating the minimum level across which COG interconnectivity is detected |
| COH time lag | Adjustment parameter for COH analysis |
| Coherence (noun) | the quality or state of cohering: as a : systematic or logical connection or consistency b : integration of diverse elements, relationships, or values (www.merriam-webster.com) |
| Connectivity (noun) | : the quality, state, or capability of being connective or connected <connectivity of a surface>; especially : the ability to connect to or communicate with another computer or computer system (www.merriam-webster.com) Note: in this text when presented in the context of the CNS or the brain can refer to neural interconnection or interconnectivity (i.e. can be shown via coherence analysis) |
| CPAP | Continuous positive airway pressure |
| CRX | **circadian-**automatic regressive analysis modelling with one or more external inputs |
| CRX | **Sircadian-automatic Regressive-analysis-modelling** with one or more external inputs |
| CT | Computer tomography |
| DA | Differential Analysis |
| DDE | Dynamic Data Exchange. Refers to exchange of data between two or more devices or systems with compatible data exchange interconnection capabilities. DDE **of sleep parameters** is the unique capability to exchange **sleep parameters** (i.e. **EEG,** EOG, EMG) in conjunctions with other conventional fitness or health tracking systems in order to enable a patient wearable or mobile (i.e. mobile wireless including phone, tablet, clock, watch, or other computing or display, notification, information, telecommunication or information access system or device capable or presenting ***genuine sleep*** (i.e. with **EEG** and/or EOG and/or EMG monitored or derived parameters for based REM or deep sleep based sleep quality, sleep-health, sleep-efficiency, sleep hypnogram, total sleep time, total REM and deep-sleep time or other sleep stages time or percentage, along with associated sleep deprivation, sleep surplus, **circadian** cycle **offset,** associated sleep propensity or sleep-urge indices,) versus conventional activity based surrogate and less accurate measures or health coaching approaches. |
| DEFINITIONS | -where a singular context or term is used plural context or term can be substituted and vice versa. |
| | -where sensor term is used an electrode or any type of physiological, environmental, neurological, |
| | -where analysis or supplementary analysis can be used including any interconnectivity options such as cloudcloud-computing services software services, network application services (NAS), LAN, WAN, WWW peer to peer and other connectivity options incorporating analysis capabilities. |
| | -where therapeutic term refers to administration of medication or devices. Devices can include respiratory assistance such as positive airway pressure (PAP), non-invasive positive airway pressure (NIPPV), magnetic stimulation etc. |
| | -whereby therapeutic devices can incorporate implantable devices such as (but not limited to) electrical stimulation devices for neurological disorders. |
| | For example eHealth neuro section can be applicable to eLife section and similarly for all other combinations of eHealth or eLife headings and subsequent sections, |
| Dipole moment (or so-called "Dipole" in this text) | In this text refers to the resultant synaptic energy represented at surface electrode as electrical signals, forming the basis of either **EEG** source localised or direct sensor processing. The genesis of this **EEG** signal or response is "the moment produced by a magnetic or electric dipole; *especially:* the product of the distance between the two poles and the magnitude of either pole (www.merriam-webster.com)" |
| DLC | destination of the coherence interconnectivity |
| Doppler watch modes | *Doppler spectrum, M-mode, B-mode, Duplex, B-mode, colour display modes, and*/*or Doppler blood velocity modes of monitoring and display* |
| DSEC | Dynamic Spatiotemporal **EEG** Connectivity |
| *DSP* | Digital signal processing |
| DSPD | delayed sleep phase disorder |
| DSPS | delayed sleep phase syndrome |
| DWMN | *distributed wireless monitoring network* |
| DWMS | *distributed wireless monitoring sensors* |
| E NLDBV | refers to computation of entropy or complexity value over an epoch period |
| EABD | Eye-lid average blink duration |
| EABF | Eye-lid average blink frequency |
| EACA | Eye-lid average closure acceleration |
| EACAV | Eye-lid average closure acceleration variance |
| EACV | Eye-lid average closure velocity |
| EACVV | Eye-lid average closure velocity variance |
| EACVV | Eye-lid average closure velocity variance |
| EAOA | Eye-lid average opening acceleration |
| EAOAV | Eye-lid average opening acceleration variance |
| EAOV | Eye-lid average opening velocity |
| EAOVV | Eye-lid average opening velocity variance |
| EBDV | Eye-lid blink duration variance |
| EBFV | Eye-lid blink frequency variance |
| eCAPA | eHealth based or linked Corrective action preventative action control or feedback (i.e. online PAP/NIPPV control adaptation for minimal sleep fragmentation, RERA arousals, and/or sleep disordered breathing and/or maximal sleep efficiency and/or maximal sleep quality and/or optimal **circadian** rhythm/clock synchronisation (i.e with local environment). |
| ECG | Electrocardiography |
| ECG | Electrocardiography |
| ECT | Eye closure time |
| **EEG** | Electroencephalography |
| **EEG** | Electroencephalography |
| eHealth or eHeallthMEDICS | eHealth (or eHeallthMEDICS) and associated pretext generally refers to professional healthcare applications. usage or oversight (i.e. professional diagnostic services, often covered by Government and/or private health organisations or health insurance organisation. Moreover the associated products and services are generally compliance with local market clearance requirements such as China or USA FDA, along with accredited medical bodies such as AASM or ASA) as opposed to eLife (or eLifeMEDICS) that generally refers to consumer health or consumer electronic product or services. |
| eLifeCHEST | Refers to the systems incorporating chest-wall or respiratory signal monitoring per (Figure 5Figure 5). |
| eLifeEXG | Refers to the systems incorporating extra (other) electrophysiological monitoring channels (Figure 23Figure 23) |
| ELifeNEURO | Refers to the systems incorporating neurology signal monitoring. |
| ELifePULSE | Refers to the systems incorporating pulse or cardiac or vascular signal monitoring. |
| eLifeWATCH | Refers to the systems incorporating watch-based or watch system/device-incorporated signal monitoring or analysis (i.e. Figure 5Figure 5). |
| eLifeWRIST | Refers to the systems incorporating wrist-based or wrist-system/device-incorporated signal monitoring or analysis (i.e. Figure 5Figure 5). |
| EM | Energy-exertion/metabolism-monitoring |
| EMBD | Eye-lid minimum blink duration |
| EMBF | Eye-lid minimum blink frequency |
| EMCA | Eye-lid minimum closure acceleration |
| EMCAV | Eye-lid minimum closure acceleration variance |
| EMCV | Eye-lid minimum closure velocity |
| EMCVV | Eye-lid minimum closure velocity variance |
| EMF | Electromagnetic field or electromagnetic frequency |
| EMG | Electromyography |
| EMG | Electromyography |
| EMOA | Eye-lid minimum opening acceleration |
| EMOAV | Eye-lid minimum opening acceleration variance |
| EMOV | Eye-lid minimum opening velocity |
| EMOVV | Eye-lid minimum opening velocity variance |
| EOG | Electrooculography |
| EOG | Electrooculography |
| EOI | Events of interest |
| EOI | Events of Interest (i.e. per "DEFINITION" described elsewhere in this document but not limited to. |
| EOI DEFINITION | Whereby DEFINITION of "health conditions of interest" or "events of interest/EOI" detailed throughout this patent application comprises: " any of or any combination of (but not limited to) prognostic, diagnostic, therapeutic/treatment, and/or biofeedback of events or biomarkers or health conditions or other and/or other health sequelae of interest, along with associated precursors, trends, combinations and/or ensembles (i.e. clusters) including any of or any combination of or degrees of severity or occurrence" (preceding underlined section referred hereafter as "events or health conditions of interest" including any of or any combination of (but not limited to) precursor or various degrees of severity applicable to disorders or biomarkers associated with altered states of consciousness, Alzheimer's, anxiety, Asperger's, auras associated with onset or very early onset of any of these conditions or events (including (i.e. any combination of physiological changes identified in lead up to an event of interest or health condition of interest. Such determination can be analysis including utilisation of artificial or expert system analysis described herein, whereby association between outcome events or health conditions and precursors enable continually improving patient-specific prognostic or diagnostic monitoring algorithms, attention deficit hyperactivity disorders (ADHD), autism spectrum disorders (ASD), brain cognitive functional or anatomical regions (i.e. defined by standardised mapping or atlas definitions or special area of interest including but not limited to suprachiasmatic region associated control or interconnectivity regions of the brain), brain connectivity, brain connectivity sequences, brain coherence, non-linear dynamic based (NLDB) analysis, predefined brain activation states or changes, predefined brain energy states or changes, changes or transitions from one state to another(i.e. related to any of or combinations of resting state, sleep, wake, alertness, sedation, anaesthesia attention, drowsiness, consciousness, altered states of consciousness, disorders, cognitive impairment etc.,), NLDB energy or sequences or activity, cardiac disorders, consciousness states, consciousness transitions, cognitive impairment, cognitive impairment disorders, dementia, delayed sleep disorder or syndrome depression, dyslexia, **EEG** power and/or spectral activation and/or localisation, Partial Seizures, Epilepsy and other seizures, EMG amplitude changes (i.e. REM or SBD), epileptiform discharges, Huntington's, interrelationship between any of these "events or health conditions of interest " muscular or movement system disorders, mental states, mental disorders, nervous system disorders, neurobehavioral disorders, neurological disorders, other muscle activity, motor neuron disorders, Parkinson's and associated movement or neurological disorders or symptoms, Korsakoff Syndrome, cerebral palsy, motor neurone disease, multiple sclerosis, central nervous system disease, myelin disease, muscular impairments, local sensory losses, visual disturbances or hallucinations, pathological markers, physiological markers, psychological markers, physiological states, physiological or psychological or pathological transitions, Psychological states, Psychiatric disorders, respiratory disorders, traumatic brain injury, sleep disorders, sleep behaviour disorders, sleep disordered breathing, residual sleepiness disorder, sleep/wake states, transitions or events and health conditions of interest, traumatic brain injury (TBI),vascular, wake disorders, NLDBTV, STV, SR, ER, clusters of ERs, clusters of SRs, spectral EOI, interconnectivity of any of these listed EOI (i.e. coherence and /or Dipole |
| | sequences and/or associated clusters, sequences and/or ensembles) and/or neurological amplitude, power, morphological signals or values whereby preceding italic and underlined section can be referred hereafter as **"events or health conditions of interest";** |
| EOI NLDBV | Events of interest non-linear dynamic based values |
| EOI SNR NLDBV | Events of interest signal to noise ratio non-linear dynamic based values |
| EOS | Eye-opening span |
| EOT | Eye opening time |
| EP | Evoked potential |
| EP | Similar to ER except relates specifically to an Evoked Potential (i.e. voltage) generated as a results of a stimulus presentation to subject/patient. |
| EPBD | Eye-lid peak blink duration |
| EPBF | Eye-lid peak blink frequency |
| EPCA | Eye-lid peak closure acceleration |
| EPCAV | Eye-lid peak closure acceleration variance |
| EPCV | Eye-lid peak closure velocity |
| EPCVV | Eye-lid peak closure velocity variance |
| EPOA | Eye-lid peak opening acceleration |
| EPOAV | Eye-lid peak opening acceleration variance |
| Epoch classification parameter | i.e. "NLDBV epoch classification parameter" whereby for example, if this parameter is set to 50% then any epoch with 50% or greater of that epoch exceeding NLDBV SNR or threshold value will be classified as a NLDBV epoch. |
| Epoch parameter | Per epoch parameter NLDBTV except as it applies to STV |
| Epoch parameter NLDBTV | Represents the period of data on which the transformation analysis (i.e. but not limited to conventional **EEG** voltage, current or power measures; NLDTV measures, STV measures, neurological measures etc.) are applied |
| EPOV | Eye-lid peak opening velocity |
| EPOVV | Eye-lid peak opening velocity variance |
| ER | Evoked response |
| ER | Evoked response or event-related response. ER evoked response comprising per name sake of an event evoked, whereby typically results from a stimulus presented to subject such as an auditory, visual, electrical, magnetic, vibration, temperature, movement, pressure or other stimulus presented to subject/patient. In this document ER can refer to electroencephalographic, neurological, electrophysiological, physiological or image voxel responses. |
| ER EOI NLDBV | evoked response EOI NLDBV |
| ER MIND | Event Related ER (i.e. can include brain response measures to tasks such as ***awareness, memory and other cognitive tests or challenges*** (i.e. stroop and other tests covered elsewhere in this document under headings "Intelligence assessments", "Memory assessments", "Language assessments", "Executive function assessments", "Visuospatial assessments", "Dementia specific assessments", "Batteries of tests for the assessment of neuropsychological functions", ***"-Cognition Impairment Assessment and Determination",*** "Assessment and determination of Cognitive functional outcomes", "Assessment and evaluation the early dementia stages and/or severity of dementia", "Cognitive performance assessment", "-Rule learner (or Rule Induction) (Separate-And-Conquer method);", "-Decision Trees (Divide-And-Conquer method)-Attention and focus tracking", and/or "-Visual Assessment of Viewer Responsiveness to displayed information:"; |
| | >the subject may be requested to verbalise a sentence, undertake an equation, identify numbers, identify picture or other interactive cognitive tests capable of a range of sensitive but also very broad measures capable of enabling ***behavioural* *and*/*or brain activation responses*** (including source localisation and/or brain responsiveness or activation versus pre-diagnostic or other data base normative reference levels), such as pre-test or resting state or other states and/or in conjunction with evoked response synchronised outcomes (MMN, odd-ball or related to endogenous brain activation relating to cognition test paradigms); |
| | - tests of brain responsiveness relating to applying stimulus or thinking paradigms can be designed to activate any of or any combination of neuropsychological tests including any o for any combination of⁹³: |
| | >the frontal lobe activation associated with complex thought processes; |
| | >occipital lobe activation associated with visual processing; |
| | >parietal lobe activation associated with touch or pain sensory processes; |
| | >temporal lobe activation associated with auditory processing; |
| | >thalamus activation associated with "consciousness switch and also" the relaying of information from the brain to appropriate regions of the brain; |
| | >Broca's areas activation associated with speech production; |
| | >Wernicke's area activation associated with understanding or comprehension related to a subject's hearing; |
| | >hippocampus region activation associated with memory processes; |
| | >amygdala associated with regulation of fear; |
| | >cerebellum activation associated with motor movement and balance; |
| | *>**cognitive assessment tests (CAS)*** such as (but not limited to) the CAS Battery of tests incorporating any of or any combination of Planning Scale, Attention Scale, Simultaneous Scale and/or Successive Scale;⁹⁴ |
| | >Kaufman Assessment Battery for Children; |
| | *>**Neurophysiologial tests*** any of or any combination of categories of testing including Intelligence, Memory, Language, Executive function, Visuospatial, Dementia specific, and/or Batteries assessing multiple neuropsychological functions;⁹³ |
| | -whereby "memory" function tests can include any of or any combination of (but not limited to) California Verbal Learning Test, Cambridge Prospective Memory Test (CAMPROMPT), Memory Assessment Scales (MAS), Rey Auditory Verbal Learning Test, Rivermead Behavioural Memory Test, Test of Memory and Learning (TOMAL), Wechsler Memory Scale (WMS), Test of Memory Malingering (TOMM); |
| | -whereby "executive function tests can include any of or any combination of (but not limited to) Behavioural Assessment of Dysexecutive Syndrome (BADS), CNS Vital Signs (Brief Core Battery), (CPT), Controlled Oral Word Association Test (COWAT), d2 Test of Attention, Delis-Kaplan Executive Function System (D-KEFS), Digit Vigilance Test, Figural Fluency Test, Halstead Category Test, Hayling and Brixton tests, Kaplan Baycrest Neurocognitive Assessment (KBNA), Kaufman Short Neuropsychological Assessment, Paced Auditory Serial Addition Test (PASAT), Rey-Osterrieth Complex Figure, Ruff Figural Fluency Test, Stroop task, Test of Variables of Attention (T.O.V.A.), Tower of London Test, Trail-Making Test (TMT) or Trails A & B, Wisconsin Card Sorting Test (WCST), Symbol Digit Modalities Test, Test of Everyday Attention (TEA); > whereby "visuospatial tests" function tests can include any of or any combination of (but not limited to) Clock Test, Hooper Visual Organisation Task (VOT), and/or Rey-Osterrieth Complex Figure; etc.; (see also expanded details under title "eLifeNeuro/Subject-Specific **EEG**-Monitoring Minimisation. Adaptation-Generic plus TBI, autism, depression/Stim feedback, epilepsy HFO; aura". |
| ERC | Event-Related Connectivity |
| ERE | Event-Related Entropy |
| ERF | Event-related magnetic fields |
| EROS | Event-related optical signals |
| ERP | Event related potential |
| ERP AEP | Event-Related Potentials or Evoked Response Potential Auditory evoked potential typically refers to the brain waveform (a segment of the corresponding monitored **EEG** signal) covering the time period commencing or prior to a stimulus, which can be a visual or auditory command, or other physical stimuli such as auditory sounds, visual presentations, electrical stimulation, or magnetic stimulation, for example only. An ERP signal can also be, for example, the responses monitored from a subject's eye via video scanning, head movement detection, facial movements, eye-lid motions and/or velocity, eye-lid opening and closure characteristics, eye-gaze direction, responsiveness or status (i.e. speed, location, changes, direction etc.), whereby the said event could be a response to a commend and/or related task such as activation or a screen or button, cognitive or visual or motor task or challenge. |
| ERP CB MIND | Ever related response cognitive behavioural MIND (measures) |
| EXG | Extra (other) electrophysiological signal |
| FD | fractal dimension |
| FDA | Food and Drug Administration |
| FFT | Fast Fourier transform |
| forward COH arc direction | Parameter configuration for COH direction being designated with origination at the earliest time of COH brain activation |
| GP | General practitioner |
| GPS | *Global positioning system (GPS)* |
| GSM | Global System for Mobile |
| GSR | galvanic skin resistance |
| HDCMS | high-dependence connectivity management system |
| HFO | High frequency oscillations |
| Hierarchical Neurologic Processing (***HNP***) | Hierarchical Neurologic Processing (***HNP***) refers to the establishment of a schematic diagram or associated information representation, that can associate the presence of monitored (i.e. **EEG** brain signal activation) or imaged (i.e. voxel activation) interrelationships in accordance a spontaneous event or with respect to an external task/challenge (i.e. per glossary ER MIND or stimulus (i.e. per visual display or auditory sound or electrical stimulus or magnetic stimulus or touch stimulus (such as pressure, heat, cold etc.). ***HNP*** can be organised in accordance to signal or voxel morphological relationships (such as signal phase, peaks, troughs, latency delay etc.) or in relationship to a time delay between stimulus or task/challenge onset and a time-window of interest (such as 300ms to 500ms after a visual stimulus or 120ms after a click auditory stimulus or within a window of 500ms after an oddball auditory or MMN auditory stimulus protocol where attention or awareness in the case of investigating attention or awareness for example. In this way the brain or CNS energy activation predominantly influenced by anatomical aspects (such as axon or dendrite brain fibres transmitting synaptic signals across the brain or subject to sheering or disconnection after TBI, for example, can be investigated), versus sensory, inhibitory, obligatory, afferent, and other higher order processing contingent related brain or CNS energy distribution. Importantly it may be possible to distil or disentangle the various layers of brain energy relating to exogenous events (i.e. stimulus, task, challenge) or spontaneous (i.e. endogenous events such as spindles, K-complex, |
| | It may be possible to **incorporate** any of or any combination(s) of measures capable of delineating AEP or ER responses according to the physiological origins and consist of the receptor potentials originating from the cochlear, neurogenic potentials originating from the acoustic nerve or neural populations within the CNS, and the myogenic potentials generated from the activation of neck and head muscles (including PAMR). i.e. a means of determining PAMR signals and/or associated muscle tonicity or responsiveness is to use active PAMR measures whereby it may be possible topurposely stimulate the PAMR using loud sounds and then gauge from the PAMR responsiveness a co-efficient or sensitivity factors which can be used in conjunction with subsequent AEP or ER measures to compensate for the actual stimulus impact during tests - in this way for example a test that is distracted by surrounding sounds such as during game day as part of on-field sports testing could benefit from a measure of ER or AEP disruptive or distractions from background crowd or field noises, versus mainly or solely cognitive impairment factors. |
| | **Additionally, it may be possible** toincorporate any of or any combination(s) of measures capable of **delineating categories** of auditory responses according to latency and comprise of the **first, ABR, MLAEP, SLAEP and the LLAEP responses.** These responses represent sensory or neural sources ranging from fast obligatory nerve and other sensory functions, to latent processing contingent potentials. The **first cochlear and eighth nerve (0 to 5 ms) and fast brainstem (ABR;2-20 ms) used to measure auditory pathway integrity indicative of the cochlear sensory functions, later responses such** as **the MLAEP (10-100 ms), SLAEP (50-300 ms) and LLAEP (150-1000 ms)** responses are implicated in refractory and higher processing contingent potential, respectively ³³. |
| | In this way it may be possible to segment brain activity in accordance to ***cognitive function, anatomical function.*** For example, any combination of ***cognitive functions, anatomical functions,*** can be analysed based on axon or dendrite neural fibre connectivity, auditory nerve responses, afferent neural responses (embedded groups of neurons responding in an obligatory manner, for |
| | example, to stimulus presentation), higher order processing contingent potentials (PCP) etc.). As described elsewhere these hierarchical functions can be assessed by way of examining time windows following a stimulus or ER task onset or by way of time window associated between a plurality of coherence corresponding to brain activation (i.e. very early time windows of <2ms can relate to brain fibre connectivity; implicated following stimulus onset, whilst other time windows include first cochlear and eighth nerve (0 to 5 ms), fast brainstem (ABR;2-20 ms), MLAEP (10-100 ms), SLAEP (50-300 ms) and LLAEP (150-1000 ms). |
| | It may be possible to distinguish any of the three N1 sub-components (respond to a rapid change in stimulus level from an otherwise quiescent condition) ^{153, 172}. |
| | The ***HNP*** function can also track and segment ***N1 changes*** as markers for both: 1) ***refractoriness**,* or sensory change as shown by the ***Butler effect*** ¹⁷³, and 2) ***processing contingent potential or discriminatory stimulus change measures*** ^{153, 174, 175}. |
| HMO's | Health management organisations |
| HMS | Refers to the Health Management System covered in more details throughout this patent application description and associated abstract and figures. |
| Hz | Hertz or reference to cycles per second of signal |
| I | Index |
| IASQ | Integrated analysis |
| ICA | Independent component analysis |
| IDscan | Identity scan, referring to capability to automatically scan for identification of a monitoring sensor |
| IED | interictal epileptiform discharges |
| IOMD | Internet Of Medical Devices |
| IP | Internet protocol |
| irLDR | infrared light dependent resistor (light detection sensor) |
| IRO | infrared reflectance oculography |
| IT" | Information technology |
| L1 | Lyapunov exponents |
| LAN | Local area network |
| LCOIM | Low-disturbance Continuous On-line Impedance Measurement |
| LDR | Light dependent resistor (light detection sensor) |
| LHS | Left Hand Side |
| M | Maximum-magnitude (i.e. maximum amplitude) |
| MDA | mobile-wireless distributed analysis (MDA) *system* |
| Measurement Precision Evaluation **(*MPE*)** system | The Measurement Precision Evaluation **(*MPE*)** system comprises evaluating outcome measures based on empirical data base studies or normative data related to broader population studies of subjects or subject-specific studies. These empirical studies can be examined using. In one example (but not limited to) the ***MPE capability*** logistic regression can be computed in order to ascertain models appropriate for the separation between consciousness and unconsciousness states, cognition states, along with associated measure of depth or levels. 1,000-fold cross-validation can be applied for the top performing models, coupled with backward variable selection as a means of categorising a minimal model or models, followed by computation of related prediction probability. In this manner the ***MPE*** determination in terms of evaluating preferred combinational or multivariate analyses formats can be computed, as well ***MPSR*** determinations in terms of feeding back to system user various prediction probability measures applicable to any combination of test measurement formats along with associated parameter configurations (i.e. as applicable (but not limited to) the combining of say ER, **EEG,** AEP, TCD, blood-sample testing measures as part of an overall cognition and/or brain injury and/or neurological disorder test regime). |
| | Further examples of the ***MPE*** is detailed under section titled "eLifeNEURO/ EPILEPSY", under subheading "Evolving and continually improving self-learning expert systems or artificial intelligence diagnostic or prognostic process |
| | ***" : "evolving and continually improving"*** (with ongoing or progressive detection of incidents of interest and greater specificity of probable |
| | combinations and associated multivariate analysis techniques, including MonteCarlo cross-coupling analysis with associated accuracy assessment measures (such as ordinal scaled measures including Pk probability statistical outcomes) of severity of epilepsy diagnosis or prognosis, and/or the option of associated ***"self-learning or artificial intelligence"*** ongoing automatic or computer assisted ***"learning"*** associated with monitoring, which can be progressively fine-tuned based on aggregated knowledge (i.e. per Figure 78 blocks 2 and 5 and per aggregation of knowledge per ***Figure 79*** )accuracy and relevant of diagnostic and prognostic outcomes (i.e. per ***Figure 45*** minimisation capability per ***block 8*** and ongoing assessment and improvement of monitoring and analysis parameters per *block 13);* |
| | Further examples of the ***MPE*** (i.e. ***""evolving and continually improving"*** multivariate analysis determination .......) is outlined in section titled "Title: eLifeNEURO/ EPILEPSY", : |
| | Further examples of the ***MPE*** is outlined in section titled "Title: eLifeNEURO/ EPILEPSY", under sib-heading "Description of Figures" as described in ***Figure 75*** Automatic online determination of events of interest including wake or sleep period biomarkers applicable to onset or incidence of neurological disorders such as Parkinson (including idiopathic SBD), epilepsy, Depression, Autism and/or ADHHD. |
| | Further examples of the ***MPE*** (i.e. The further incorporation or comprising of characterisation of an individual's ***"events of interest"*** and/or ***"health conditions of interest"*** can include a process where statistical analysis techniques (such as incorporation of ***cross-validation, Monte-Carlo, Jackknife, factors*** analysis determination, support vectors machine (SVM), deployed in either online or offline data-mining context to establish most accurate ***clustering algorithm.........**)* section titled "PATENT TITLE: AUTISM SPECTRUM DISORDERS (ASD); ASPERGERS (ASP); DYSLEXIA;. |
| | Further examples of the ***MPE*** (i.e. The incorporation of the capability of predicting in a quantitative manner the properties of the mental state such as (but not limited to on the diagnosis or prognosis of a range of mild to chronic brain disorders including (but not limited to) brain **at risk**, **mild cognitive impairment** (MCI), **disordered health condition**)" : The capability of predicting in a quantitative manner the properties of the mental state such as (but not limited to on the diagnosis or prognosis of a range of mild to chronic brain disorders including (but not limited to) brain **at risk**, **mild cognitive impairment** (MCI), **disordered health condition** such as mental and/or neurobehavioral disorder and/or neurological disorders....) may be incorporated as further detailed under section titled " "PATENT TITLE: DIMENTIA /ALZHEIMER'S /ASD /ASP", "under sub-heading "-TRIAGED BRAIN RISK TO SEVERE COGNITIVE IMAPAIRMENT (Figure 80 [8])). |
| | Further examples of the ***MPE** are described under section titled* "PATENT TITLE: DIMENTIA /ALZHEIMER'S /ASD /ASP", under sub-heading "STATISTICAL PERFORMANCE ASSESSMENT DETERMINATION [13]" (covering "STATISTICAL ANALYSIS"; "RANKING AND REGRESSION MODELING"). |
| Measurement Precision Statistical and Analyses Reckoner **(*MPSR*)** System | The Measurement Precision Statistical Reckoner (MPSR) System incorporate the means to aggregate and an amalgam of multi-dimensional dependent and/or independent measures in a manner whereby the overall precision of the measurement in enhanced compared to any singular or subset of measures. Examples of MPSR include (but not limited to any of or any combination of figures and associated blocks with descriptions per this documents ***Figure 45*** including Block [12] CONDITION CHARACTERISATION & OPTIMAL PARAMETER DETERMINATION MONITORING AND OUTCOMES PERFORMANCE ASSESSMENT ANALYSIS ; Block [13] **EEG** MONITORING AND ANALYSIS IMPROVEMENT OR |
| | FINE-TUNING OF PARAMETERS AND OTHER YSTEM RECOMMENDATIONS OR AUTOMATIC CHANGES, ALONG WITH ASSOCIATED MAXIMAL TO MINIMAL ALGORITHM MODELLING ASPECTS. See also example of determining prediction accuracy and confidence levels per example under section titled "NEUROCOGNITIVE MANAGEMENT SYSTEM" under sub-heading "STATISTICAL ASSESSMENT OF CUMULATIVE TESTS". |
| Measurement statistical and analyses outcomes **(*MSO*)** | The Measurement statistical outcomes (***MSO***). See also example of measurement outcomes determination under section titled "NEUROCOGNITIVE MANAGEMENT SYSTEM" under sub-heading "STATISTICAL ASSESSMENT OF CUMULATIVE TESTS". |
| MEG | Magnetic encephalography |
| MIND | Multi-dimensional Integrated Neurocognitive Determination; see expanded descriptions under section titled "NEUROCOGNITION MANAGEMENT SYSTEM" and associated figures and related descriptions for Figure 100. |
| MISD | Mind Interconnectivity Sequence Determination |
| MMN | Mismatched negativity |
| MMN MIND | Mismatched Negativity MIND Interconnectivity Sequence Determination |
| MRI | Magnetic resonance imaging |
| MS&NDDE | Multiple sleep and *neurological* event dynamic data exchange processing |
| MS&NDDE | Multiple sleep and neurological event dynamic data exchange |
| MSL | multi-dimensional source localisation (MSL) |
| MTM | multipoint time-synchronisation monitoring |
| MUSIC | Multiple Signal Classification |
| N1 or NREM 1 | (non-REM sleep stage 1) |
| N2 or NREM 2 | (non-REM sleep stage 1) |
| N3 or NREM 3 | (non-REM sleep stage 1) |
| NAS | Network application services |
| NAS, HDCMS, CPAP, eHealthATLAS | These systems have been described in other patent application documents (Burton, D. 2014; : Ref ID: 14935). |
| **Neurologic Simulation modelling** (**NSM**) of Cognitive Processing Sequencer (***CPS***) and/or Cognitive Hierarchical Neurologic Processing (***HNP***) process and/or Cognitive Coding System (**CCS**) | The **CPS, CHPS,** and/or **CCS Simulation or Modelling** aspect comprises of simulating brain functional sequences (imitation or enactment of the expected or anticipated outcomes for a specific subject and under certain subject test protocol conditions and as related to spontaneous event or event-related stimulus/task) based on determination of the in accordance to subject-specific or normative data in order to predict cognitive processing sequences applicable to certain event-related or task/challenge (i.e. such as auditory and/or visual and/or other tasks or challenges per MIND ER, listed in glossary herein). Utilisation of **CPS, CHPS, and/or CCS** may be enabled along with normative data from high cognition, average and impaired cognition subject-specific or normative data in order to model and simulate data, providing a basis of comparison or cross-reference in terms of a subject or group of subject cognitive prognostic or diagnostic assessment at any point in time. |
| NFIR | Near field infrared |
| NFIR | Near field infrared |
| NIPPV | Non-invasive positive pressure ventilation |
| NIRS | Near-field infrared spectroscopy |
| NLDB | Non-linear dynamics based (signal analysis) |
| NLDBM | non-linear dynamic based magnitude |
| NLDBT | NLDB time-series |
| NLDBTV | NLDB time-series values |
| NLDBTV | NLDB time-series (NLDBT) values |
| NLDBTV sensitivity parameter | If NLDBTV sensitivity parameter is set to 10% (in simple example of complexity analysis) any peak and trough values not varying in synchronisation or more than 10% (i.e. 1/2 period/cycle inter-interval variation of < 10% of corresponding SE or EOI inter-interval value. |
| NLDM | Non-linear dynamic magnitude |
| NLDSM:NLDNM | Non-linear dynamic signal magnitude/levels (*NLDSM*) are compared to non-linear dynamic noise/background-signals magnitude/levels (*NLDNM*) or NLDSM:NLDNM, compared to conventional signal to noise or S:N ratio). |
| OIM | On-line impedance measurement functions. |
| OLC | Originating location of the coherence interconnectivity |
| opt in or opt-in | "Opt in" Refers to the capability to allow access by other parties subject to special medical authorisation (such as verification with official medical practitioner register in order to confirm the legitimacy and qualifications, registration etc. of a medical practitioner's current and acceptable registration status with the official relevant register(s). Based security, [privacy, qualification medical registration and other verifications subject/patient can elect to accept a request for medical "op-in" or "Link-in" status (i.e. "health-network" "opt-in" or "Link-in" status. In this way general practitioners, dentists, chiropractors, osteopath, chiropodist etc.). One subject/patient/user has "opted" in selected health-network , messages, calendar appointments, scheduling, personal electronic health record (PEHR). personally controlled electronic health record (PCEHR), messages, alerts and other management systems (mobile phone, smart-watch etc.) can be accessed or configured as part of the health management *system* (HMS). |
| P | Period |
| PAP | Positive airway pressure |
| PAT | pulse arterial tone |
| Patient information | Generally this applies to information such as patient health survey's, body mass index, age, gender, past medical history, allergies, reactions to medication, blood type, available register of medical diagnostic data or record and other aspects that can contribute to an individual's routine or emergency health benefit. |
| PCA | Principle component analysis |
| PCM | phone-case metabolic-measurement |
| PCP | processing contingent potentials |
| PCP | processing contingent potentials |
| PET | Positive emission tomography |
| PP | pulse pressure |
| PPG | photo-plethysmography |
| PR or HR | pulse rate or heart rate |
| PTT | pulse transient time |
| PVD | *peripheral vascular disease* |
| PVDF | polyvinylidene difluoride |
| PWA | Pulse wave analysis |
| PWA | pulse *wave* amplitude *(PWA)* |
| PWV | Pulse wave velocity (PWV) |
| QRS | Q waves - depolarization of the interventricular septum, and can also relate to breathing. R wave - depolarization of the main mass of the ventricles. S wave-final depolarization of the ventricles. |
| R&K | A. Rechtschaffen and A. Kales, A Manual of Standardized Terminology, Techniques and Scoring System for Sleep Stages of Human Subjects (Bethesda,MD.: U.S. Dept. Health, Education and Welfare, Public Health Service., 1968). |
| RCDM | Reduced Complexity Diagnostic Monitoring |
| REM | Rapid Eye Movement |
| reverse COH arc direction | Parameter configuration for COH direction being designated with origination at the latest time of COH brain activation |
| RHS | Right Hand Side |
| RIP | Respiratory inductive plethysmography |
| RPECS | Relative peak eye-lid closure speed |
| S | Speed |
| SAAS | Software As A Service (such as cloud-computing services or network application services/NAS) |
| SBD | Sleep Behaviour Disorder |
| Schematic (adj) | showing the main parts of something usually in the form of a simple drawing or diagram : of or relating to a scheme or schema (www.merriam-webster.com) |
| SCOPER | AASM's classification system to specific and inclusive method for classifying and evaluating sleep testing devices for the diagnosis of obstructive sleep apnea in the |
| | out-of-center setting. Intended to provide guidance to sleep medicine clinicians in determine which out-of-center testing devices are appropriate for diagnosing obstructive sleep apnoea. Categorization of the **SCOPER system for out-of-center monitoring devices is based on measurements of Sleep, Cardiovascular, Oximetry, Position, Effort, and Respiratory parameters.** |
| SDA | spatiotemporal dynamic analysis |
| SDE | Spatial Dynamic Entropy |
| SDT | spectral dynamic time-series |
| SDTV | spectral dynamic time-series values |
| SE | sleep efficiency |
| SEG | Segmentation |
| SEG | Segmentation |
| SEM | slow eye movements |
| SEM | slow eye movements |
| SleepFit | SleepFit Wristband with novel SomniLink ^{®} & Real Sleep & fitness, along with actual versus goal/target measures for any time or date (***Figure 26******Figure 26***). Active measurement module can be physically or data-wise interchanged with a plurality of wearable devices including sleep monitoring head applied device and/or wrist and/or belt and/or necklace and/or any other subject/individual body, head, body extremity wearable device chosen location (i.e. but not limited to ***Figure 1******Figure 1***). |
| SNR | Signal to Noise Ratio |
| *SomnifitSomfit* | ***A patient-wearable sleep, fitness, health, sentient state monitoring device* (*somnifitSomfit*)** incorporates an electronic module (***SomnifitSomfit***; Figure 1Figure 1 RHS, [1] and [7]) incorporating multi-device (Figure 1 Figure 1 RHS, [8], [5]) interchangeable electronic module and/or associated information with automatic dynamic online ***data-exchange enabling sleep, health and fitness measures*** and indicator display capabilities, comprising one or more light-detection and applied forehead electrophysiological sleep-parameter(s) (**EEG**, EOG and EMG) monitoring capability, along with measures or associated indices including (but not limited to) sleep efficiency (SE), wake after sleep onset (WASO); |
| Somnilink^{®} | Refers to the linkage of sleep variables or monitored parameters as part foof a connectivity or control process. |
| *Somnisync* | The ***Somnisync*** system incorporates a means of enabling ***"dynamic data-exchange" of sleep parameters*** or other health or fitness parameters between two or more wearable devices or associated mobile wireless communication devices or computer systems. |
| SPECT | Single photon emission computed tomography |
| SpO2 | oxygen saturation |
| SQE | Signal Quality Estimation |
| SQI&C | Sensor/Electrode Quality Indicator and Control |
| SR | spontaneous response (or spontaneous event/SE) comprising of a neurological or physiologically generated event such as spindle, k-complex, spike, alpha-burst, HFO, high frequency ripple, vertex-wave, spike and wave epileptifiorm discharge event etc, |
| Step parameter NLDBTV | Represents the inter-interval time period relating to the computation of consecutive analysis transformations |
| Step parameter STV | Per "Step parameter NLDBTV" except as it applies to STV |
| STV | time-spectral time-series values |
| STV | spectral time-series values |
| SWD | shift work disorder |
| T&M | Test and Measurement |
| TBI | Traumatic brain injury |
| TC | Thalamocortical |
| TCCD | Transcranial Colour Doppler |
| TCCD-3D | Transcranial Colour Doppler 3-dimensional-view-reconstruction |
| TCD | Transcranial Doppler Thalamocortical |
| TGA | Therapeutic Goods Administration |
| TOT | Total |
| VISE | Vision Index Blink-speed / span |
| WAN | Wide area network |
| WASO | wake after sleep onset |
| WM system | **wearable-device minimisation (WM) system. An example of this system is outlined in section titled :" " under sub-heading " " and detailed per** **Figure 45** **"Automatic diagnostic and prognostic EEG-monitoring and analysis system incorporating minimisation process enabling professional and consumer-level monitoring and automatic analysis determination".** |
| WWW | World Wide Web |
| ZCI | zero-crossing interval |

A wearable monitoring system located adjacent to one or more body locations of a subject including at least one electrode or sensor adjacent the head to monitor and/or analyse via on-board or supplementary processing of any brain variables or muscle signals or eye movement signals, said system including any one of or any combination of:
a) capability to be universal and/or interchangeable or exchangeable wherein the parts of or the complete system are distributed between at least two locations for the deployment as any or a combination of a fitness or health tracking or environmental tracking system and a sleep monitoring system; or
b) capability to be applied as a subject part without any need for pressure attachment to the said subject's head or reliance upon a partial or total circumference band or other attachment method in order to achieve appropriate connection interface between said electrode and/or sensor; or
c) capability to accommodate or provide sleep staging analysis whereby sleep parameters can be analysed in progressive time epochs of at least 20 or 30 seconds duration so that correlation of brain signals, muscle signals and/or eye movement signals across said epoch periods can provide sufficient information and effective and meaningful responsiveness to changes in sleep and wake states of an individual, thereby enabling meaningful online (in near or relative real-time) sleep progression status or reports or offline reports; or
d) capability to enable both fitness or health tracking or environmental system as well as a sleep monitoring system actual status or status to date or at any time for an individual against any comparatives, in terms of progressive outcomes, defined period outcomes, and/or trended period outcomes; or
e) incorporation of information monitoring and analysis capabilities for automatic dynamic online data-exchange between one or more display or remotely located or other wearable device, enabling sleep, health and fitness progressive or other time period measures or analytic summary or tracking capabilities, thereby enabling seamless or continuous or uninterrupted short-term or long-term monitoring and analysis, across days and nights and for data trending purposes.

The monitoring system may incorporate any of progressive or online or offline analysis or reports which include any of or any combination of :
continuous EEG monitoring with associated analysis capable of the determination of epoching and epoch-based sleep stages or sleep hypnogram or sleep events, or sleep quality or sleep measures or indices, wake after sleep onset (WASO), respiratory event related arousals (RERA), therapeutic event related arousals (TERA), apnoea/hypopnoea index (AHI), sleep disturbance index (SDI), respiratory disturbance index (RDI), sleep fragmentation, percentage and amount of each sleep stage, total sleep time (TST), sleep hypopnoea, sleep delay syndrome delay factors, residual daytime sleepiness (RDS), arousal index (Al);
whereby said determination of epoching comprises of segmenting data in blocks of time in the range of 20 to 30 seconds.

The monitoring system may further comprise of supplementary processing capabilities whereby said epoch-based sleep stages determination comprises of local (micro-processing device or DSP system forming part of monitoring sensor or device) and/or available communication mediums, networks or other interconnectivity options (including simultaneously interconnecting with additional or supplementary communication networks, systems or other interconnectivity options such as WWW, IP, LAN, WAN, supplementary/companion monitoring/sensing or computing systems, SAAS including cloud-computing services or NAS, or peer-to-peer connections.

The system may comprise of any of or any combination of magnetic, mechanical, or other interlocking means enabling the interchange of the system or a part or parts thereof between a plurality of wearable health or environmental monitoring devices.

Said monitoring system may be located adjacent a subject's forehead or worn on another body part, including any of:
a) a forehead applied sensor incorporating at least one bipolar forehead electrophysiological signal (Error! Reference source not found.; [8], [9], [10], [11], [12]);
b) a forehead applied sensor comprising of a reusable sensor;
c) a forehead applied sensor comprising of a disposable sensor;
d) a forehead applied sensor comprising of a sensor with or without a partial or total circumference headband (Error! Reference source not found.; [13]); e) a forehead applied sensor comprised on one side of a sensor with self-adhesive surface and embedded self-gelled electrophysiological electrodes, whereby the said electrode can be exposed with the removal of backing paper; or f) a forehead applied sensor comprised of non-electrode side of said forehead applied sensor including a means of interfacing to said electronic module or a device containing or holding said electronic module (Error! Reference source not found.; [7]);

The monitoring system's "universal and/or interchangeable or exchangeable" capability may comprise of the said monitoring system or part of the said monitoring system able to be fitted adjacent to a subject's head for monitoring sleep periods as well as placed elsewhere in any space or worn on the subject's wrist, arm or other region of the subject's body for wake or non-sleep monitoring periods;

The monitoring system's capability "to be applied as a subject part without any need for pressure attachment" may comprise of a self-adhesive sensor device, for attaching the monitoring system and interface as well as monitoring electrodes for attachment to subject in order to enable monitoring of any of EEG, EMG, and/or EOG signals.

The monitoring system's capability "to accommodate or provide sleep staging analysis" may comprise of enabling continuous monitoring of EEG, EMG, and/or EOG signals in a manner whereby each 20 or 30 second epoch period can be analysed in order to correlate the EEG signal(s) characteristics, (such as EEG spectral distribution, along with amplitudes or power of frequency amplitudes, and/or half period amplitude analysis, wake, non-REM (NR) stage 1 , NR stage 2, NR stage 3 rapid eye movement (REM) sleep), along with EOG signal(s) characteristics (such as eye movements indicative of wake versus REM), and EMG characteristics (such as muscle tonicity indicate of lowest REM levels or progressively decreasing levels through stages of wake, non-REM (NR) stage 1 , NR stage 2, NR stage 3 rapid eye movement (REM) sleep),
along with assignment of the various wake and sleep stages in accordance to established guidelines as this of the American Academy of Sleep Medicine (AASM);

The monitoring system's capability "to enable both fitness or health tracking or environmental system as well as a sleep monitoring system" may comprise of enabling monitoring of sleep signals, signals applicable to circadian clock determination and circadian rhythm health management, as well as signals indicative or activity or exercise or fitness training, based on signal monitoring and analysis of variables including any of or any combination of brain signal electrodes (electroencephalography; EEG); muscle tone electrodes (electromyography; EMG) ; REM detection and sleep stage determination; self-adhesive easy to reposition and comfortable to wear applied sensor; oximeter measures including blood oxygen saturation levels and/or associated outcomes (i.e. any of heart rate, plethysmography, PTT determination, pulse arterial tone, pulse wave amplitude etc.); high sensitivity 3- axis accelerometer and 3-axis gyroscope capable of detecting position, steps, run, walk, bike, falls, walking or stride fluidity, walking or stride symmetry, neuro-muscular tremors or other associated motion or movement characteristics, gait or walking stability, trips, gait, walking and other movement characteristics; jogging, running, sitting, leaning, toppling, unsteady motion, falling, tripping, sudden movements or jerks, unusual movements, unusually hesitant or slow or unusual motion characteristics (i.e. indicative of danger or incidents or locations of concern), ambient light detection, microphone for breathing sounds, snore detection and room sound; room temperature; geographical satellite positioning system, along with analysis enabling the determination of distinguishing aspects outside acceptable or pre-determined thresholds or safe operating ranges;
The monitoring system's "incorporation of information monitoring and analysis capabilities for automatic dynamic online data-exchange" may comprise of enabling interconnectivity and interface between the said monitoring system and other mobile or wearable system(s) or interconnected systems in order to interface monitored data or associated analysis outcomes for the deployment of compatible systems and applications capable of analysing and/or displaying and/or presenting and/or dissemination longer-term (days, week, months, years) or shorter-term (seconds, hours) monitoring periods of subject's information including sleep, wake, circadian rhythm, health, activity tracking and/or management;
The said "circadian rhythm health management" may incorporate the determination of the biological cycle (circadian rhythm) by way of accommodating multi varsity analysis enabling one or more monitored channels of physiological data (I.e. any of or any combination of body temperature , environmental temperature, environmental light, EEG, EMG, pulse, ECG, HRV, PTT, blood pressure, body position, breathing, oximetry, motion, etc.) and/or incorporating into circadian rhythm determination of a subject's sleep-staging and other events of interest or analytical outcomes, and/or other information such as work, social, travel and other schedules or agendas, and/or sleep times, clock times, alarm times, schedules, notification and associated goals or coaching or guidance relating to optimal alignment between sleep propensity and sleep times in order to maximise sleep efficiency and quality (such as minimising sleep delay syndrome, insomnia, sleep deprivation).

## Claims

1. An applied sensor system comprising:
an applied head or forehead sensor comprising one or more electrophysiological monitoring electrodes for monitoring and for delivering at least one stimulus signal to a subject, the stimulus signal comprising a magnetic signal;
a stimulus system configured to generate the at least one stimulus signal;
a monitoring system comprising an electronics module configured to monitor at least one physiological signal, the monitoring system further configured to simultaneously emit the at least one stimulus signal; and
a processor configured to enable monitored responses to be synchronised with the delivered stimulus signal,
**characterised in that**:
the applied sensor system is wearable; and
the processor is additionally configured to compute source localisation of resultant stimulated electrical brain activity in order to assess a source of the stimulated electrical brain activity.

2. The system of claim 1 further comprising one or more vascular measurement sensors.

## Patentansprüche

1. Appliziertes Sensorsystem, umfassend:
einen applizierten Kopf- oder Stirnsensor, der eine oder mehrere elektrophysiologische Überwachungselektroden zur Überwachung und zur Abgabe mindestens eines Stimulussignals an ein Subjekt umfasst, wobei das Stimulussignal ein magnetisches Signal umfasst;
ein Stimulationssystem, das so konfiguriert ist, dass es das mindestens eine Stimulussignal erzeugt;
ein Überwachungssystem, das ein Elektronikmodul umfasst, das so konfiguriert ist, dass es mindestens ein physiologisches Signal überwacht, wobei das Überwachungssystem ferner so konfiguriert ist, dass es gleichzeitig das mindestens eine Stimulussignal aussendet; und
einen Prozessor, der so konfiguriert ist, dass er es ermöglicht, überwachte Reaktionen mit dem abgegebenen Stimulussignal zu synchronisieren,
**dadurch gekennzeichnet, dass**:
das applizierte Sensorsystem tragbar ist; und
der Prozessor zusätzlich so konfiguriert ist, dass er eine Quellenlokalisierung der resultierenden stimulierten elektrischen Gehirnaktivität berechnet, um eine Quelle der stimulierten elektrischen Gehirnaktivität zu beurteilen.

2. System nach Anspruch 1, ferner umfassend einen oder mehrere vaskuläre Messsensoren.

## Revendications

1. - Système de capteur appliqué comprenant :
un capteur appliqué sur la tête ou le front comprenant une ou plusieurs électrodes de surveillance électrophysiologique destinées à surveiller et à délivrer au moins un signal de stimulation à un sujet, le signal de stimulation comprenant un signal magnétique ;
un système de stimulation configuré pour générer ledit au moins un signal de stimulation ;
un système de surveillance comprenant un module électronique configuré pour surveiller au moins un signal physiologique, le système de surveillance étant en outre configuré pour émettre simultanément ledit au moins un signal de stimulation ; et
un processeur configuré pour permettre aux réponses surveillées d'être synchronisées avec le signal de stimulation délivré,
**caractérisé par le fait que** :
le système de capteur appliqué est portable ; et
le processeur est en outre configuré pour calculer une localisation source de l'activité cérébrale électrique stimulée résultante afin d'évaluer une source de l'activité cérébrale électrique stimulée.

2. - Système selon la revendication 1, comprenant en outre un ou plusieurs capteurs de mesure vasculaire.
